# EUROPEAN PATENT SPECIFICATION

(11) **EP 0 951 466 B1**
(45) Date of publication and mention of the grant of the patent: **21.01.2009**
(21) Application number: 97953208.2
(22) Date of filing: 22.12.1997
(51) Int. Cl.: C07D 243/16, C07D 243/24, C07D 401/04, C07D 223/18, A61K 31/55, C07D 409/12, C07D 407/12, C07D 401/14, C07D 217/24, C07D 223/10, C07D 225/02, C07D 211/76, C07D 207/273, C07D 311/76, C07D 307/22

(54) **CYCLOALKYL, LACTAM, LACTONE AND RELATED COMPOUNDS, PHARMACEUTICAL COMPOSITIONS COMPRISING SAME, AND METHODS FOR INHIBITING BETA-AMYLOID PEPTIDE RELEASE AND/OR ITS SYNTHESIS BY USE OF SUCH COMPOUNDS**
CYCLOALKYL-, LACTAM-, LACTON- UND VERWANDTE VERBINDUNGEN ALS HEMMER DER BETA-AMYLOIDPEPTIDFREISETZUNG
CYCLOALKYLE, LACTAME, LACTONE ET COMPOSES ASSOCIES, COMPOSITIONS PHARMACEUTIQUES CONTENANT CES COMPOSES, ET PROCEDES D'INHIBITION DE LA LIBERATION DU PEPTIDE BETA-AMYLOIDE ET/OU DE SA SYNTHESE AU MOYEN DE TELS COMPOSES

(30) Priority: 23.12.1996 US 780025
(43) Date of publication of application: 27.10.1999
(73) Proprietor: ELAN PHARMACEUTICALS, INC., South San Francisco, CA 94080 (US); ELI LILLY AND COMPANY, Indianapolis IN 46285 (US)
(72) Inventor: WU, Jing, San Mateo, CA 94402 (US); Tung, Jay S., Belmont, CA 94002 (US); Thorsett, Eugene D., Moss Beach, CA 94038 (US); Pleiss, Michael A., Sunnyvale, CA 94087 (US); Nissen, Jeffrey S., Indianapolis, IN 46268 (US); Neitz, Jeffrey, San Francisco, CA 94121 (US); Latimer, Lee H., Oakland, CA 94618 (US); John, Varghese, San Francisco, CA 94122 (US); Freedman, Stephen, Walnut Creek, CA 94596 (US); Britton, Thomas C., Carmel, IN 46033 (US); Audia, James E., Indianapolis, IN 46278 (US); Reel, Jon K., Carmel, IN 46032 (US); Mabry, Thomas E., Indianapolis, IN 46227 (US); Dressman, Bruce A., Indianapolis, IN 46202 (US); Cwi, Cynthia L., Indianapolis, IN 46256 (US); Droste, James J., Indianapolis, IN 46254 (US); Henry, Steven S., New Palestine, IN 46163 (US); McDaniel, Stacey L., Bloomington, IN 47403 (US); SCOTT, William L., Indianapolis, IN 46208 (US); Stucky, Russell D., Indianapolis, IN 46227 (US); Porter, Warren J., Indianapolis, IN 46269 (US)
(74) Representative: Suarez-Miles, Ana Sanchiz
(86) International application number: PCT/US1997/022986
(87) International publication number: WO 1998/028268

(56) References cited:
- EP-A- 0 652 009
- EP-A- 0 677 517
- WO-A-95/25118
- WO-A-97/30072
- FR-A- 2 278 335

## Description

### CROSS-REFERENCE TO RELATED APPLICATIONS

This application claims the benefit of U.S. Provisional Application No. 60/064,851 which was converted pursuant to 37 C.F.R. § 1.53(b)(2)(ii) from U.S. Patent Application No. 08/780,025, filed December 23, 1996.

### Field of the Invention

This invention relates to compounds which inhibit β-amyloid peptide release and/or its synthesis, and, accordingly, have utility in treating Alzheimer's disease.

### References

The following publications, patents and patent applications are cited in this application as superscript numbers:
- ¹: Glenner, et al., Biochem. Biophys. Res. Commun., 120:885-890 (1984)
- ²: U.S. Patent No. 4,666,829
- ³: Selkoe, Neuron, 6:487-498 (1991)
- ⁴: Goate, et al., Nature, 349:704-706 (1990)
- ⁵: Chartier Harlan, et al., Nature, 353:844-846 (1989)
- ⁶: Murrell, et al., Science, 254:97-99 (1991)
- ⁷: Mullan, et al., Nature Genet., 1:345-347 (1992)
- ⁸: Schenk, et al., International Patent Application Publication No. WO 94/10569, "*Methods and Compositions for the Detection of Soluble* β-*Amyloid Peptide*", published 11 May 1994
- ⁹: Selkoe, Scientific American, "Amyloid Protein and Alzheimer's Disease", pp. 2-8, November, 1991
- ¹⁰: Tetrahedron Letters, 34(48), 7685 (1993)
- ¹¹: Losse, et al., Tetrahedron, 27:1423-1434 (1971)
- ¹²: Citron, et al., Nature, 360:672-674 (1992)
- ¹³: Hansen, et al., J. Immun. Meth., 119:203-210 (1989)
- ¹⁴: U.S. Patent No. 3,598,859
- ¹⁵: Ogliaruso and Wolfe, Synthesis of Lactones and Lactams, Patai, et al. Editor, J. Wiley & Sons, New York, New York, USA, pp. 1085 et seq. (1993).
- ¹⁶: Ugi, et al., Tetrahedron, 52(35):11657-11664 (1996)
- ¹⁷: Blade-Font, Tetrahedron Lett., 21:2443 (1980).
- ¹⁸: Freidinger, et al., J. Org. Chem., 47:104-109 (1982)
- ¹⁹: Semple, et al., J. Med. Chem., 39:4531-4536 (1996).
- ²⁰: Holladay, et al., J. Org. Chem., 56:3900-3905 (1991).
- ²¹: Donaruma, et al., Organic Reactions, 11:1-156 (1960)
- ²²: Wolff, Organic Reactions, 3:307-336 (1946)
- ²³: Krow, et al., J. Org. Chem., 61:5574-5580 (1996)
- ²⁴: Tetrahedron, 35:2433 (1979)
- ²⁵: Gracias, et al., J. Am. Chem. Soc., 117:8047-8048 (1995)
- ²⁶: Milligan, et al., J. Am. Chem. Soc., 117:10449-10459 (1995)
- ²⁷: Miller, et al., J. Am. Chem. Soc., 118:9606-9614 (1996)
- ²⁸: March, Advanced Organic Chemistry, Reaction Mechanisms and Structure, 2nd Edition, McGraw-Hill Book Company, New York, New York, USA (1977)
- ²⁹: Colombo, et al., Tetrahedron Lett., 35(23):4031-4034 (1994)
- ³⁰: Rogriguez, et al., Tetrahedron, 52:7727-7736 (1996)
- ³¹: Parsons, et al., Biochem. Biophys. Res. Comm., 117:108-113 (1983)
- ³²: Watthey, et al., J. Med. Chem., 28:1511-1516 (1985)
- ³³: Armstrong, et al., Tetrahedron Lett., 35:3239 (1994)
- ³⁴: King, et al., J. Org. Chem., 58:3384 (1993).
- ³⁵: Hu, et al., Tetrahedron Lett., 36(21):3659-3662 (1995).
- ³⁶: Wada, et al., Bull. Chem. Soc. Japan, 46:2833-2835 (1973)
- ³⁷: Gaetzi, Chem. Abs., 66:28690m
- ³⁸: Wheeler, et al., Organic Syntheses, Coll. Vol. VI, p. 840
- ³⁹: J. Med. Chem., 28(12):1886 (1985)
- ⁴⁰: Brenner, et al., U.S. Patent No. 2,938,029
- ⁴¹: Evans, et al., J. Am. Chem. Soc., 112:4011-4030 (1990)
- ⁴²: Micouin, et al., Tetrahedron, 52:7719-7726 (1996)
- ⁴³: Butcher, et al., Tetrahedron Lett., 37(37):6685-6688 (1996)
- ⁴⁴: M.L. Reupple, et al., J. Am. Chem. Soc., 93:7021 et seq. (1971)
- ⁴⁵: P.A.S. Smith, Organic Reactions, 3:337-449 (1946)
- ⁴⁶: K. Orito, et al., Tetrahedron, 36:1017-1021 (1980)
- ⁴⁷: Krimm, Chem. Ber. , 91:1057 (1958)
- ⁴⁸: Suda, et al., J. Chem. Soc. Chem Comm., 949-950, (1994)
- ⁴⁹: Barton, et al., J. Chem. Soc., 1764-1767 (1975)

- ⁵⁰: Kitagawa, et al., J. Am. Chem. Soc., 117:5169-5178 (1975)
- ⁵¹: Akhatar, et al., J. Org. Chem., 55:5222-5225 (1990)
- ⁵²: Nedenskov, et al., Acta Chem. Scand., 12:1405-1410 (1958)
- ⁵³: Sakakida, et al., Bull. Chem. Soc. Japan, 44:478-480 (1971)
- ⁵⁴: Hoffman, et al., Tet. Lett., 30:4207-4210 (1989)
- ⁵⁵: Vedejs, et al., Tet. Lett., 33:3261-3264 (1992)
- ⁵⁶: van der Steen, et al., Tetrahedron, 47, 7503-7524 (1991)
- ⁵⁷: Hart, et al., Chem Rev., 89:1447-1465 (1989)
- ⁵⁸: Lowe, et al., Bioorg. Med. Chem. Lett., 4:2877-2882 (1994)
- ⁵⁹: McKennis, Jr., et al., J. Org. Chem., 28:383-387 (1963)
- ⁶⁰: Shirota, et al., J. Med. Chem., 20:1623-1627 (1977)
- ⁶¹: Overberger, et al., J. Am. Chem. Soc., 85:3431 (1963)
- ⁶²: Herschmann, Helv. Chim. Acta, 32:2537 (1949)
- ⁶³: Overberger, et al., Macromolecules, 1:1 (1968)
- ⁶⁴: Busacca, et al., Tet. Lett., 33:165-168 (1992)
- ⁶⁵: Croisier, et al., U.S. Patent No. 4,080,449
- ⁶⁶: J.A. Robl, et al., Tetrahedron Lett., 36(10):1593-1596 (1995)
- ⁶⁷: Flynn, et al., J. Med. Chem., 36:2420-2423 (1993)
- ⁶⁸: Orito, et al., Tetrahedron, 36:1017-1021 (1980)
- ⁶⁹: Kawase, et al., J. Org. Chem., 54:3394-3403 (1989)
- ⁷⁰: Lowe, et al., J. Med. Chem., 37:3789-3811 (1994)
- ⁷¹: Robl, et al., Bioorg. Med. Chem. Lett., 4:1789-1794 (1994)
- ⁷²: Skiles, et al., Bioorg. Med. Chem. Lett., 3:773-778 (1993)
- ⁷³: Grunewald, et al., J. Med. Chem., 39(18):3539 (1996)
- ⁷⁴: Thomas, et al., J. Chem. Soc., Perkin II, 747 (1986)
- ⁷⁵: Warshawsky, et al., Bioorg. Med. Chem. Lett., 6:957-962 (1996)
- ⁷⁶: Ben-Ishai, et al., Tetrahedron, 43:439-450 (1987)
- ⁷⁷: van Niel et al., Bioorg. Med. Chem. Lett., 5:1421-1426 (1995)
- ⁷⁸: Kawase, et al., J. Org. Chem., 54:3394-3403 (1989)
- ⁷⁹: Edwards, et al., Can. J. Chem., 49:1648-1658 (1971)
- ⁸⁰: Milligan, et al., J. Am. Chem. Soc., 117:10449-10459 (1995)
- ⁸¹: Curran et al., Tet. Lett., 36:191-194 (1995)
- ⁸²: Slusarchyk, et al. , Bioorg. Med. Chem. Lett., 5:753-758 (1995)
- ⁸³: Wyvratt, et al., Eur. Pat. Appl. 61187 (1982)
- ⁸⁴: Cornille, et al., J. Am. Chem. Soc., 117:909-917 (1995)
- ⁸⁵: Kolc, Coll. Czech. Chem. Comm., 34:630 (1969)
- ⁸⁶: Dickerman, et al., J. Org. Chem., 14:530 (1949)
- ⁸⁷: Dickerman, et al., J. Org. Chem., 20:206 (1955).
- ⁸⁸: Dickerman, et al., J. Org. Chem., 19:1855 (1954)
- ⁸⁹: Hoffman, et al. , J. Org. Chem., 27:3565 (1962)
- ⁹⁰: Wasserman, et al., J. Am. Chem. Soc., 103:461-2 (1981)
- ⁹¹: Crombie, et al., Tetrahedron Lett., 27(42):5151-5154 (1986)
- ⁹²: Yokoo, et al., Bull, Chem. Soc. Jap., 29:631 (1956)
- ⁹³: Burkholder, et al., Biog. Med. Chem. Lett., 2:231 (1993)
- ⁹⁴: Karanewsky, U.S. Patent No. 4,460,579.
- ⁹⁵: Kametani, et al., Heterocycles, 9:831-840 (1978)
- ⁹⁶: Yanganasawa, et al., J. Med. Chem., 30:1984-1991 (1987)
- ⁹⁷: J. Das et al. , Biorg. Med. Chem. Lett., 4:2193-2198 (1994)

All of the above publications, patents and patent applications are herein incorporated by reference in their entirety to the same extent as if each individual publication, patent or patent application was specifically and individually indicated to be incorporated by reference in its entirety.

### State of the Art

Alzheimer's Disease (AD) is a degenerative brain disorder characterized clinically by progressive loss of memory, cognition, reasoning, judgment and emotional stability that gradually leads to profound mental deterioration and ultimately death. AD is a very common cause of progressive mental failure (dementia) in aged humans and is believed to represent the fourth most common medical cause of death in the United States. AD has been observed in races and ethnic groups worldwide and presents a major present and future public health problem. The disease is currently estimated to affect about two to three million individuals in the United States alone. AD is at present incurable. No treatment that effectively prevents AD or reverses its symptoms and course is currently known.

The brains of individuals with AD exhibit characteristic lesions termed senile (or amyloid) plaques, amyloid angiopathy (amyloid deposits in blood vessels) and neurofibrillary tangles. Large numbers of these lesions, particularly amyloid plaques and neurofibrillary tangles, are generally found in several areas of the human brain important for memory and cognitive function in patients with AD. Smaller numbers of these lesions in a more restrictive anatomical distribution are also found in the brains of most aged humans who do not have clinical AD. Amyloid plaques and amyloid angiopathy also characterize the brains of individuals with Trisomy 21 (Down's Syndrome) and Hereditary Cerebral Hemorrhage with Amyloidosis of the Dutch Type (HCHWA-D). At present, a definitive diagnosis of AD usually requires observing the aforementioned lesions in the brain tissue of patients who have died with the disease or, rarely, in small biopsied samples of brain tissue taken during an invasive neurosurgical procedure.

The principal chemical constituent of the amyloid plaques and vascular amyloid deposits (amyloid angiopathy) characteristic of AD and the other disorders mentioned above is an approximately 4.2 kilodalton (kD) protein of about 39-43 amino acids designated the β-amyloid peptide (βAP) or sometimes Aβ, AβP or β/A4. β-Amyloid peptide was first purified and a partial amino acid sequence was provided by Glenner, et al.¹ The isolation procedure and the sequence data for the first 28 amino acids are described in U.S. Patent No. 4,666,829².

Molecular biological and protein chemical analyses have shown that the β-amyloid peptide is a small fragment of a much larger precursor protein termed the amyloid precursor protein (APP), that is normally produced by cells in many tissues of various animals, including humans. Knowledge of the structure of the gene encoding APP has demonstrated that β-amyloid peptide arises as a peptide fragment that is cleaved from APP by protease enzyme(s). The precise biochemical mechanism by which the β-amyloid peptide fragment is cleaved from APP and subsequently deposited as amyloid plaques in the cerebral tissue and in the walls of the cerebral and meningeal blood vessels is currently unknown.

Several lines of evidence indicate that progressive cerebral deposition of β-amyloid peptide plays a seminal role in the pathogenesis of AD and can precede cognitive symptoms by years or decades. See, for example, Selkoe³. The most important line of evidence is the discovery that missense DNA mutations at amino acid 717 of the 770-amino acid isoform of APP can be found in affected members but not unaffected members of several families with a genetically determined (familial) form of AD (Goate, et al.⁴; Chartier Harlan, et al.⁵; and Murrell, et al.⁶) and is referred to as the Swedish variant. A double mutation changing lysine⁵⁹⁵-methionine⁵⁹⁶ to asparagine⁵⁹⁵-leucine⁵⁹⁶ (with reference to the 695 isoform) found in a Swedish family was reported in 1992 (Mullan, et al.⁷). Genetic linkage analyses have demonstrated that these mutations, as well as certain other mutations in the APP gene, are the specific molecular cause of AD in the affected members of such families. In addition, a mutation at amino acid 693 of the 770-amino acid isoform of APP has been identified as the cause of the β-amyloid peptide deposition disease, HCHWA-D, and a change from alanine to glycine at amino acid 692 appears to cause a phenotype that resembles AD is some patients but HCHWA-D in others. The discovery of these and other mutations in APP in genetically based cases of AD prove that alteration of APP and subsequent deposition of its β-amyloid peptide fragment can cause AD.

Despite the progress which has been made in understanding the underlying mechanisms of AD and other β-amyloid peptide related diseases, there remains a need to develop methods and compositions for treatment of the disease(s). Ideally, the treatment methods would advantageously be based on drugs which are capable of inhibiting β-amyloid peptide release and/or its synthesis *in vivo*.

### SUMMARY OF THE INVENTION

This invention is directed to the discovery of a class of compounds which inhibit β-amyloid peptide release and/or its synthesis and, therefore, are useful in the prevention of AD in patients susceptible to AD and/or in the treatment of patients with AD in order to inhibit further deterioration in their condition. The class of compounds having the described properties are defined by any one of claims 1 to 4.

Accordingly, in one of its use aspects, this invention is directed to a use of a compound according to any one of claims 1 to 4 for the manufacture of a medicament for the treatment of Alzheimer's disease (AD).

Because the *in vivo* generation of β-amyloid peptide is associated with the pathogenesis of AD^{8,9}, the compounds of formula I can also be employed in conjunction with a pharmaceutical composition to prophylactically and/or therapeutically prevent and/or treat AD. Accordingly, in another of its use aspects, this invention is directed to a prophylactic a use of a compound according to any one of claims 1 to 4 for the manufacture of a medicament for preventing the onset of AD in a patient at risk for developing AD which method comprises administering to said patient a pharmaceutical composition comprising a pharmaceutically inert carrier and an effective amount of a compound or a mixture of compounds according to any one of claims 1 to 4.

In yet another of its use aspects, this invention is directed to a use of a compound according to any one of claims 1 to 4 for the manufacture of a medicament for treating a patient with AD in order to inhibit further deterioration in the condition of that patient which comprises administering to said patient a pharmaceutical composition comprising a pharmaceutically inert carrier and an effective amount of a compound or a mixture of compounds according to any one of claims 1 to 4.

This invention also provides for novel pharmaceutical compositions comprising a pharmaceutically inert carrier and a compound according to any one of claims 1 to 4.

Still further, this invention provides for novel compounds according to any one of claims 1 to 4.

The products of this invention include mixtures of R,S enantiomers at any stereochemical center. Preferably, however, when a chiral product is desired, the chiral product corresponds to the L-amino acid derivative. In the formulas set forth herein, a mixture of R,S enantiomers at the stereochemical center is sometimes indicated by a squiggly line as per convention. Othertimes, no stereochemical designation is made at the stereochemical center and this also infers that a mixture of enantiomers is present.

Preferred compounds described herein include those set forth in the tables below:

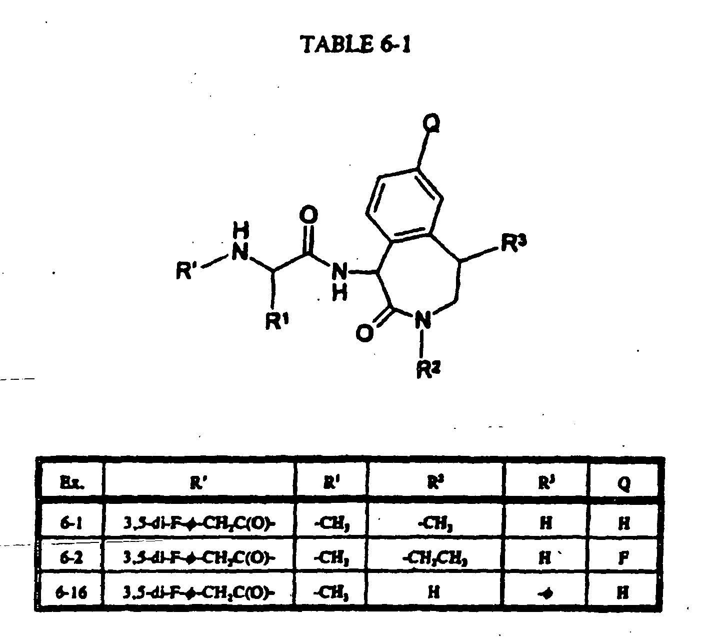

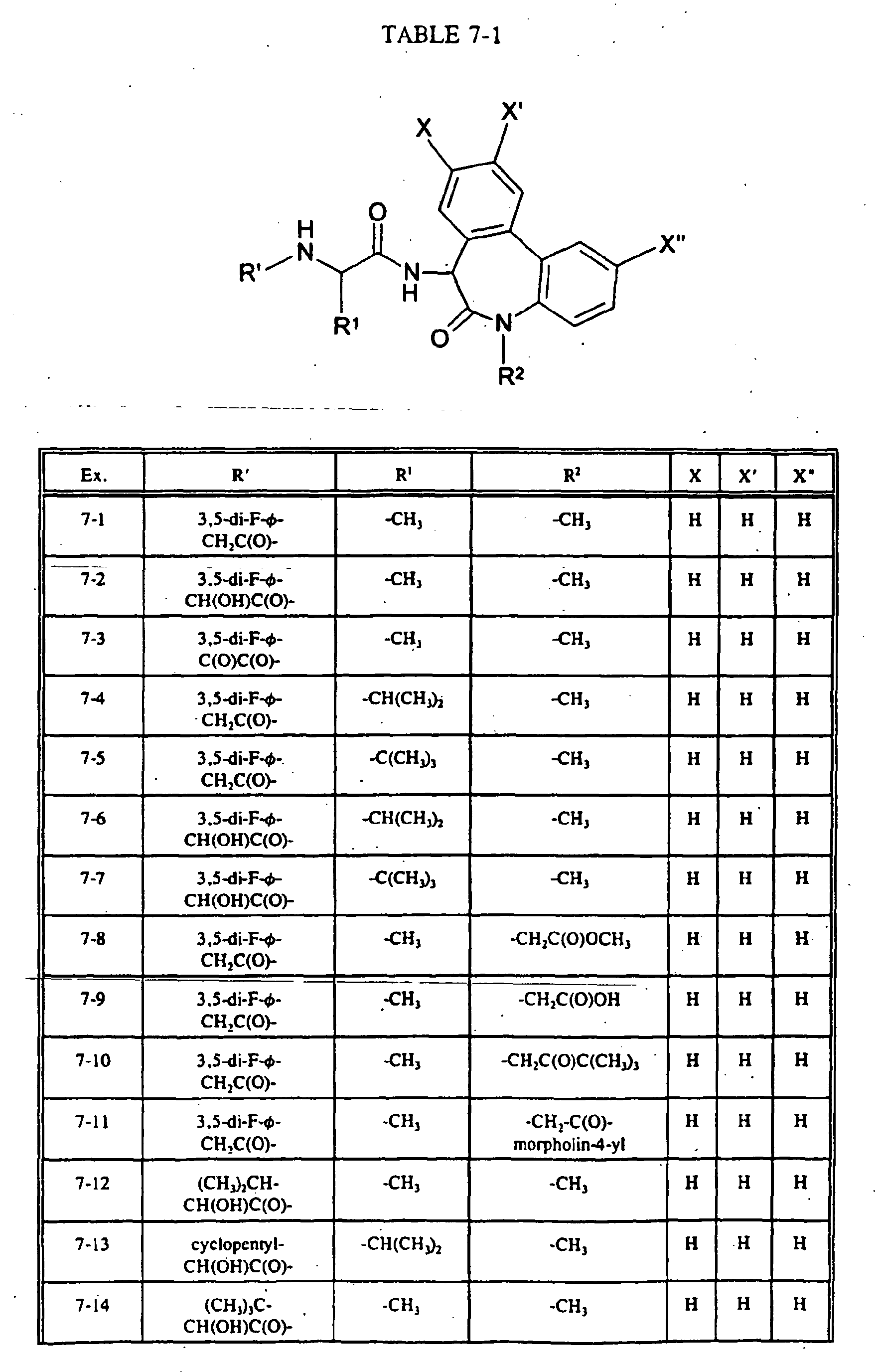

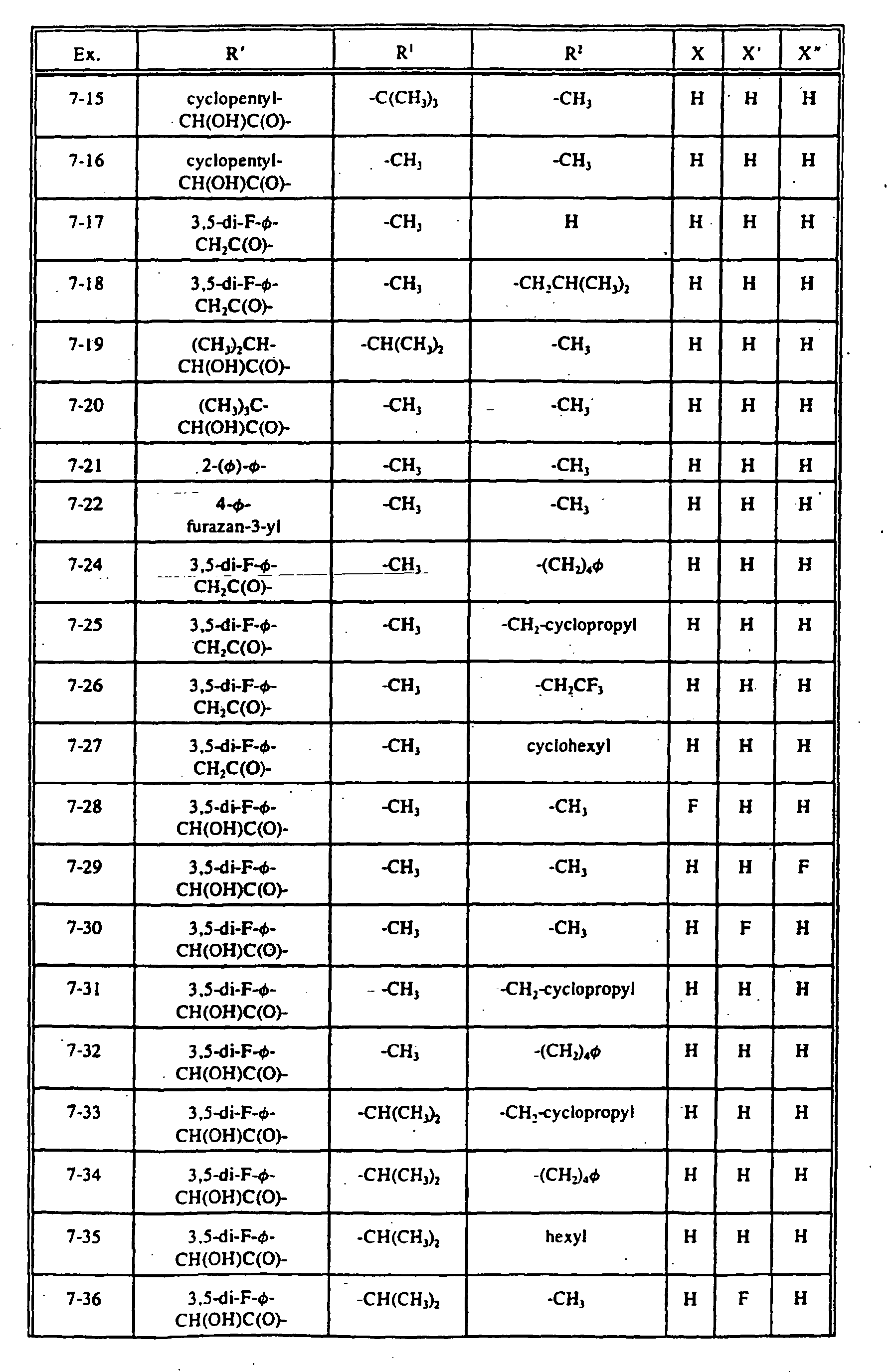

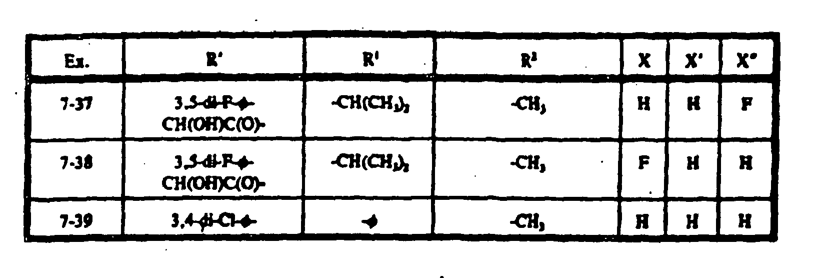

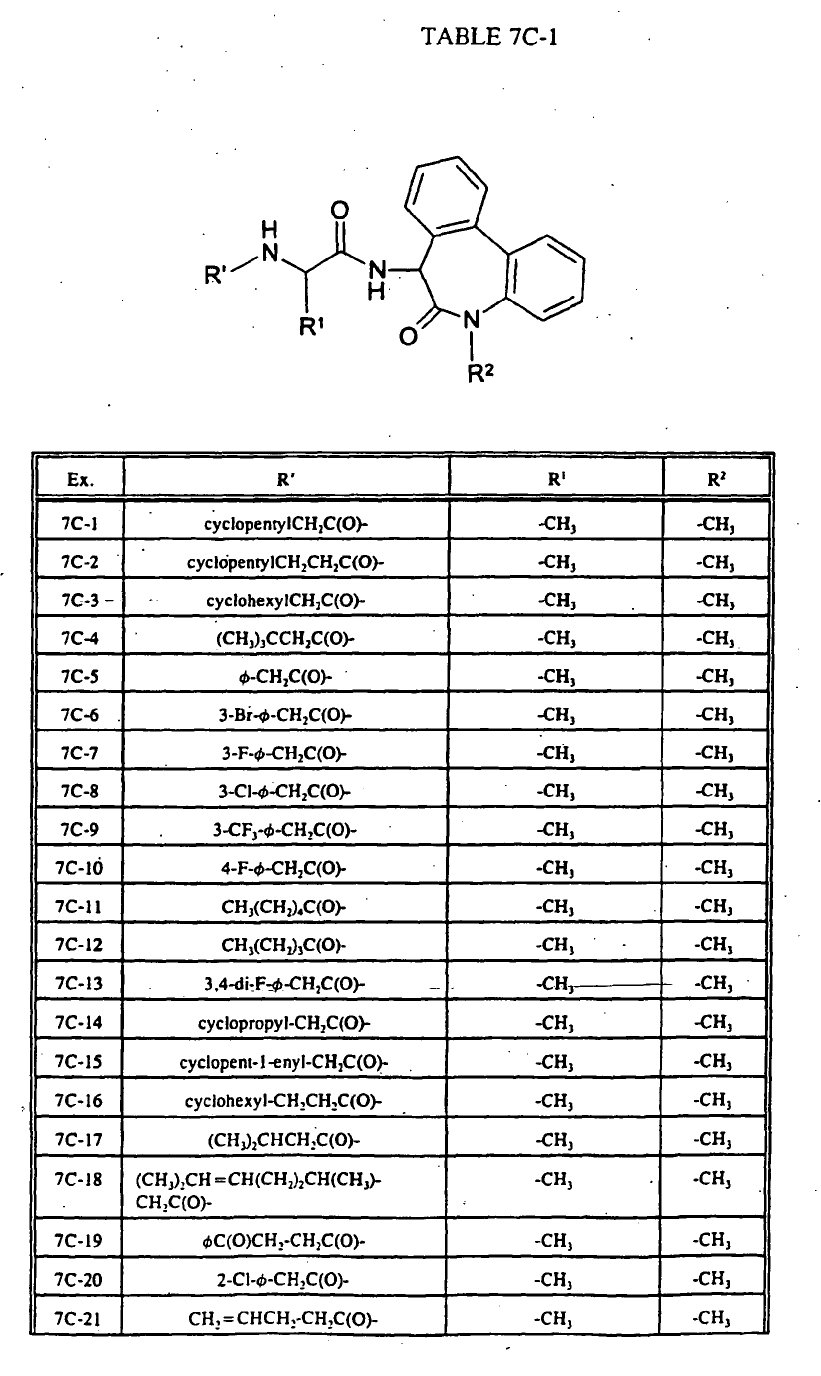

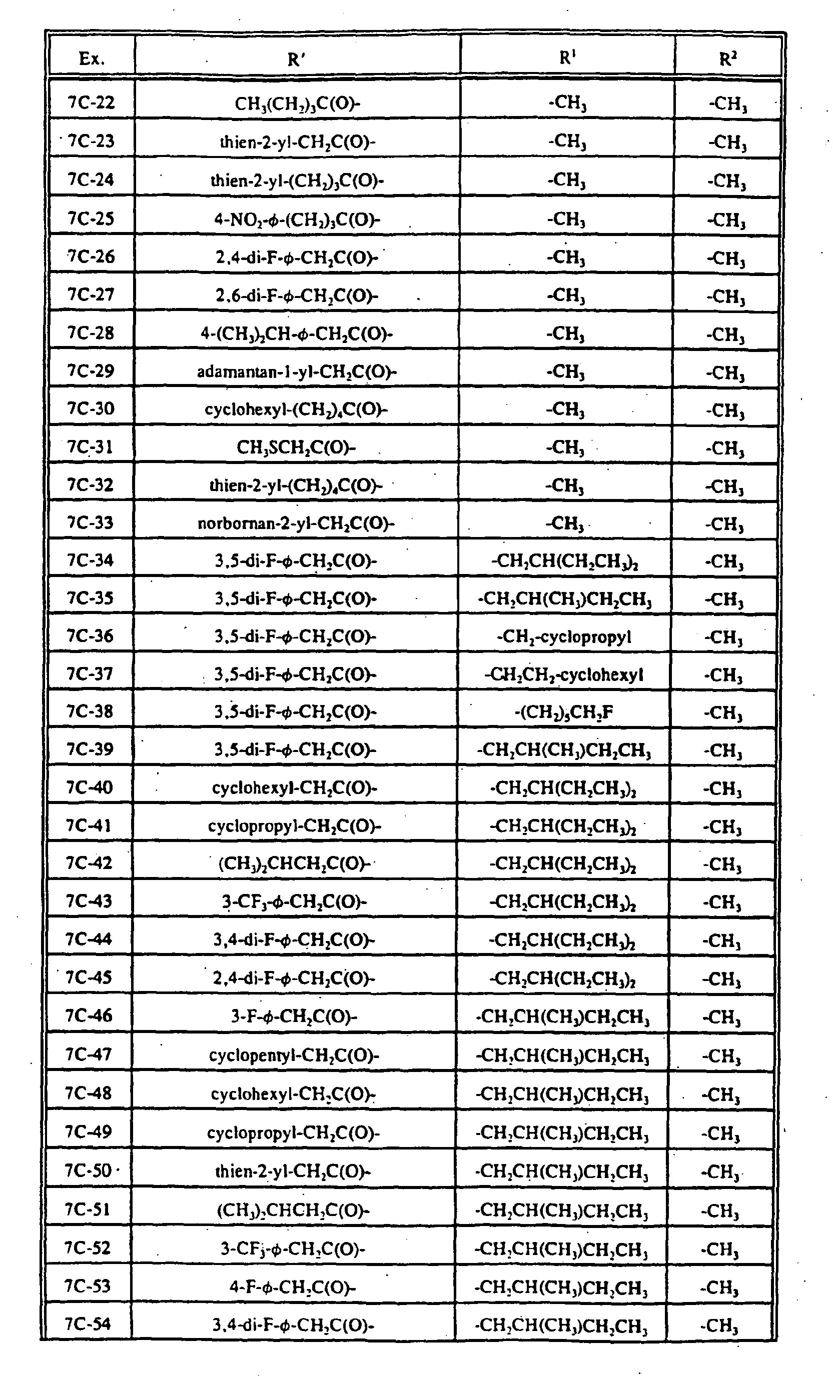

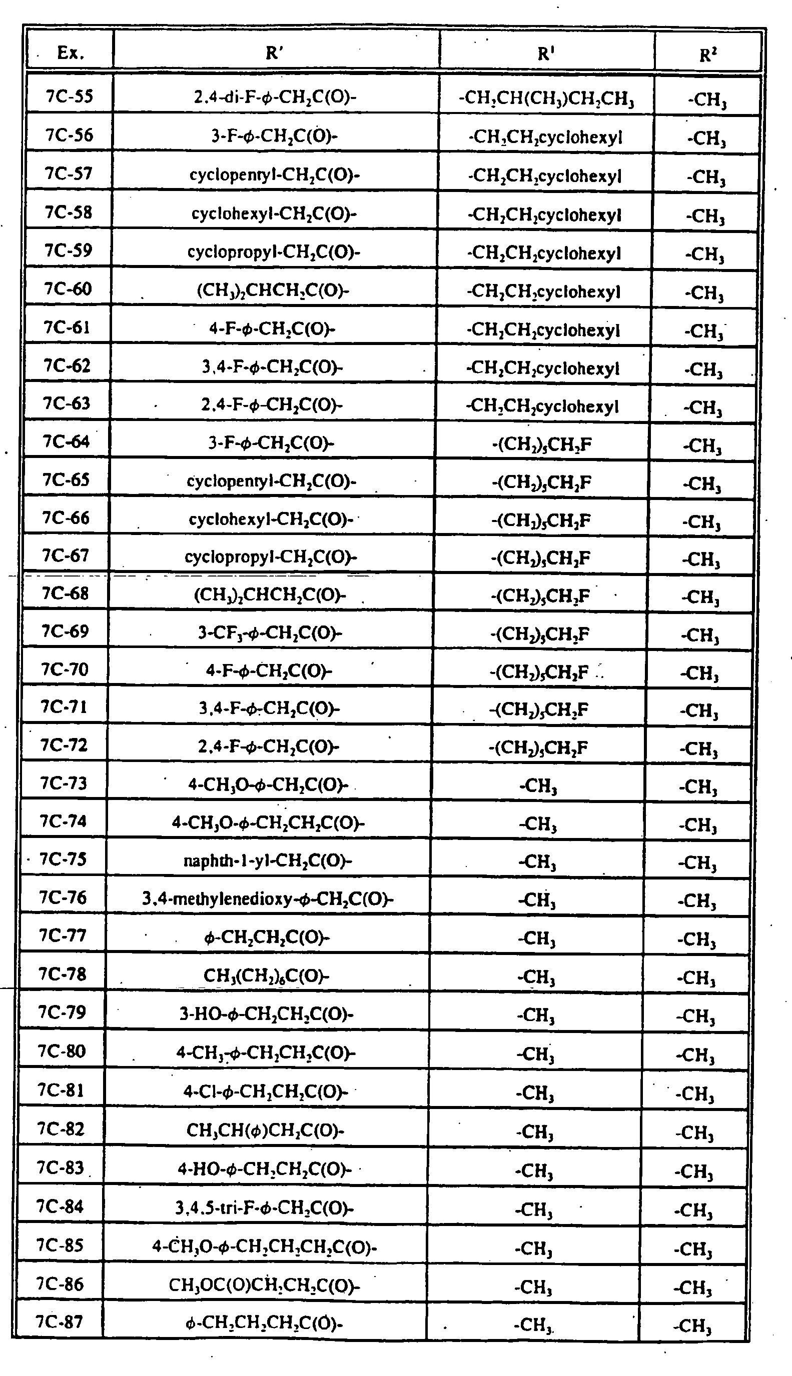

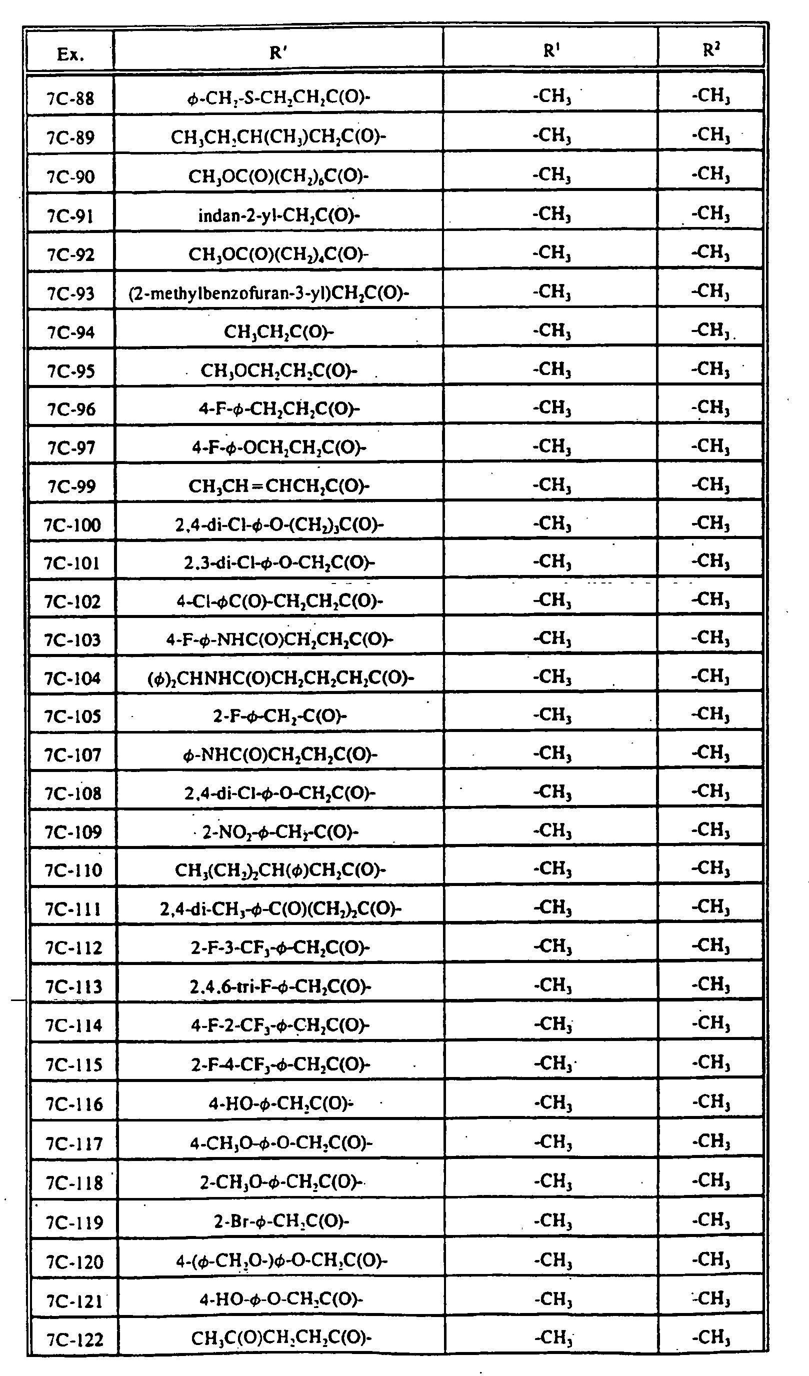

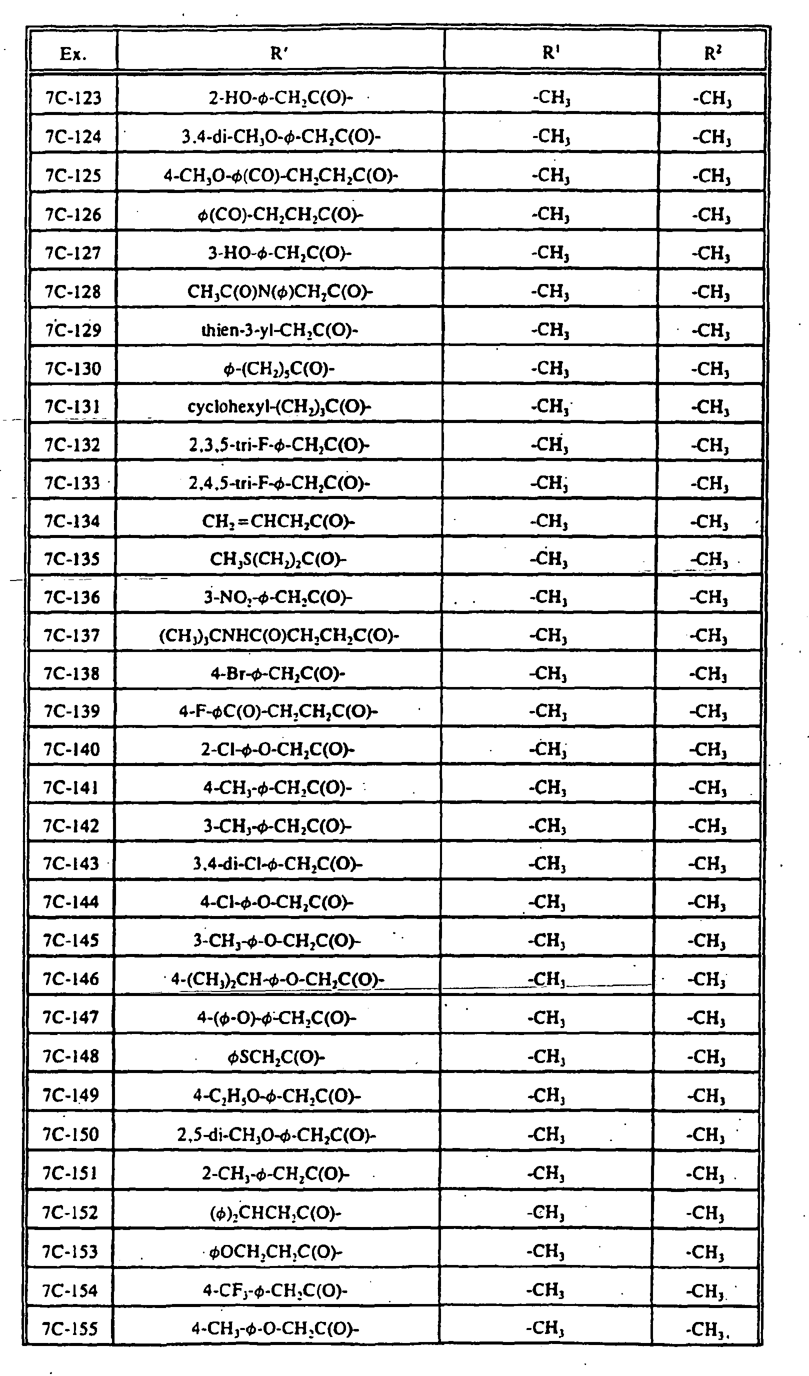

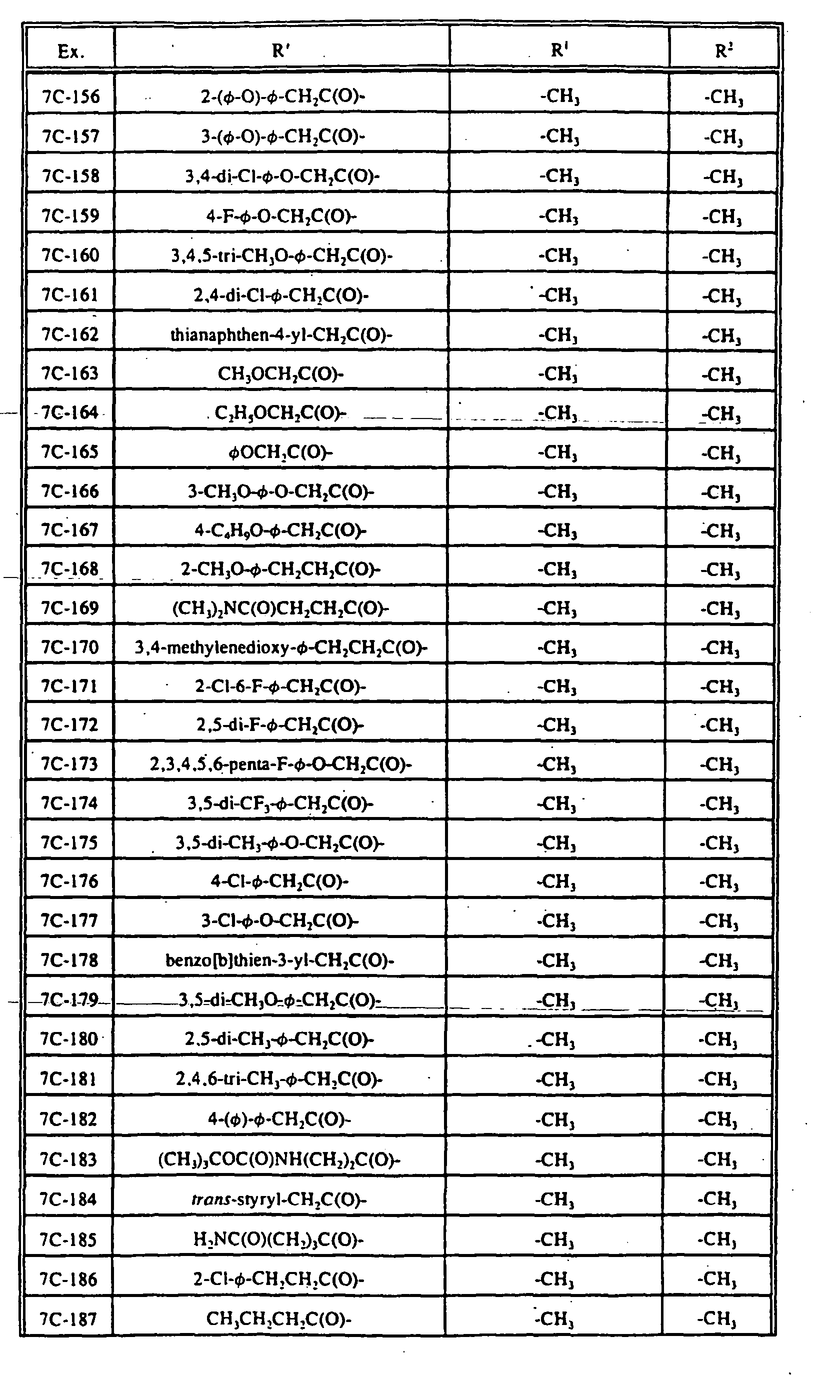

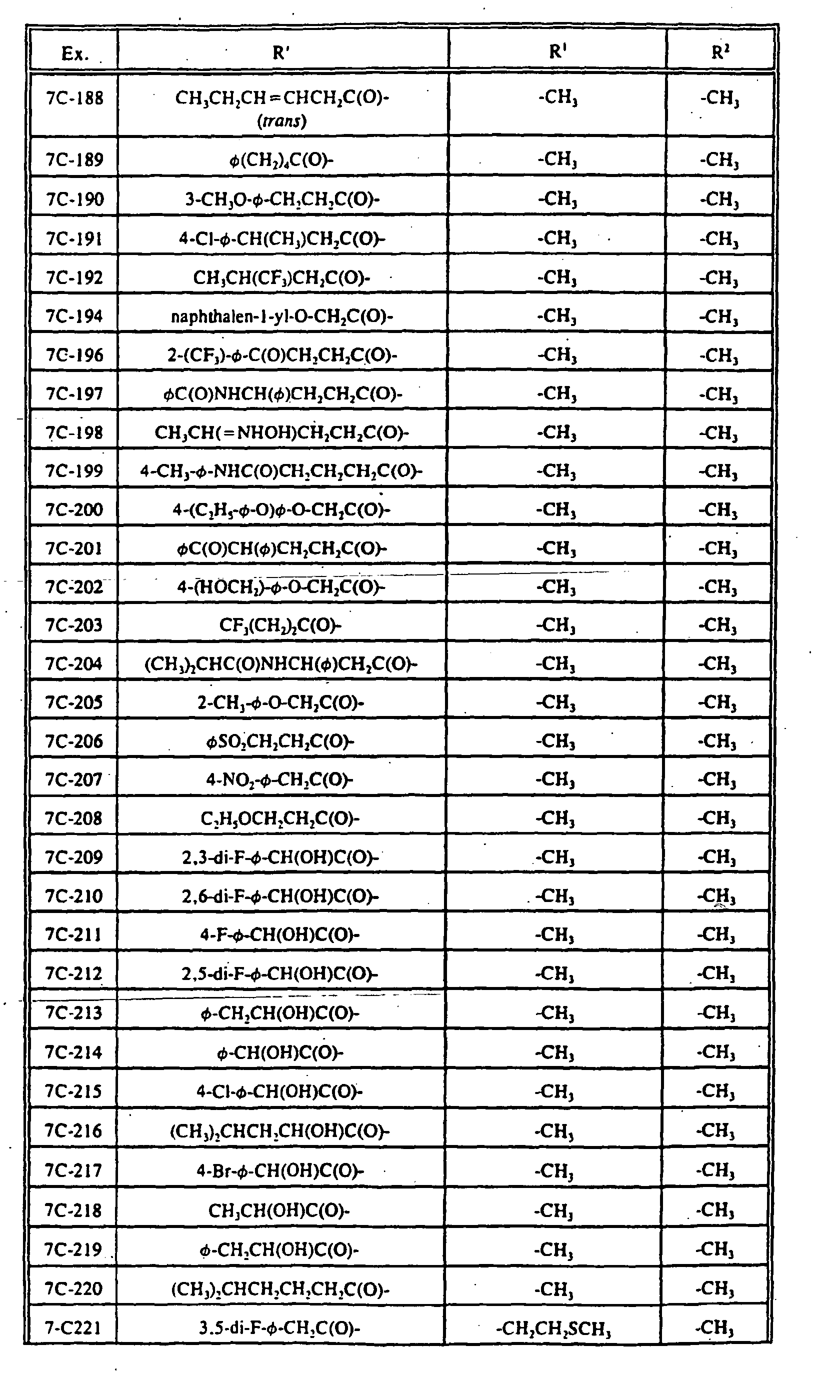

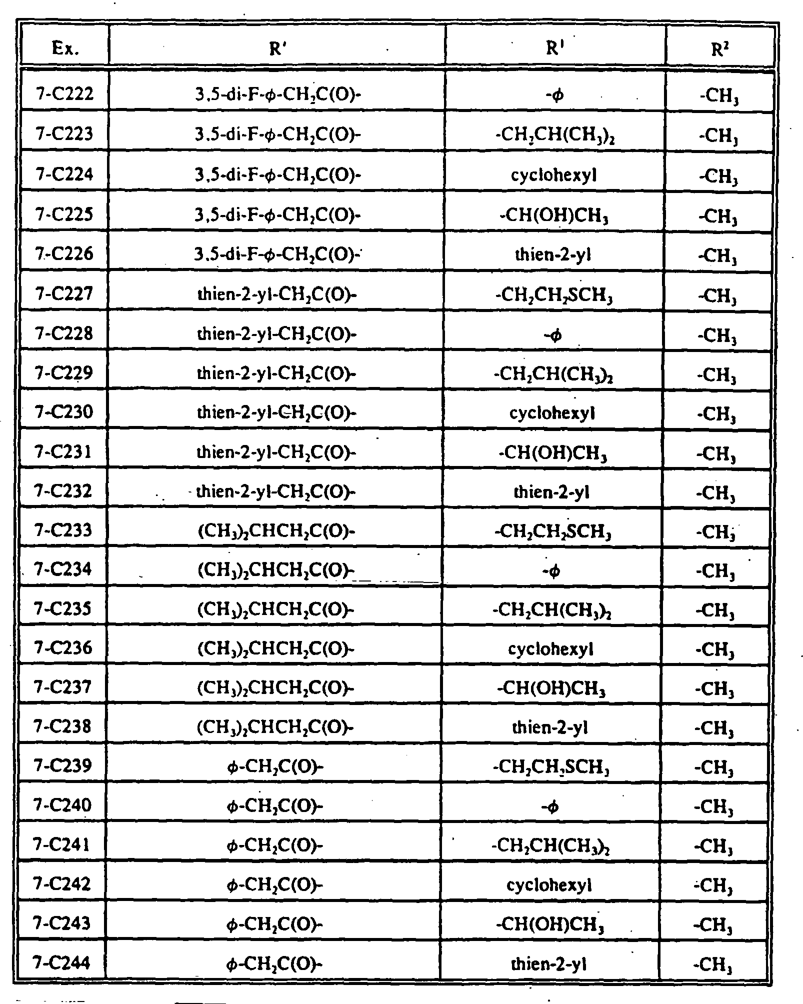

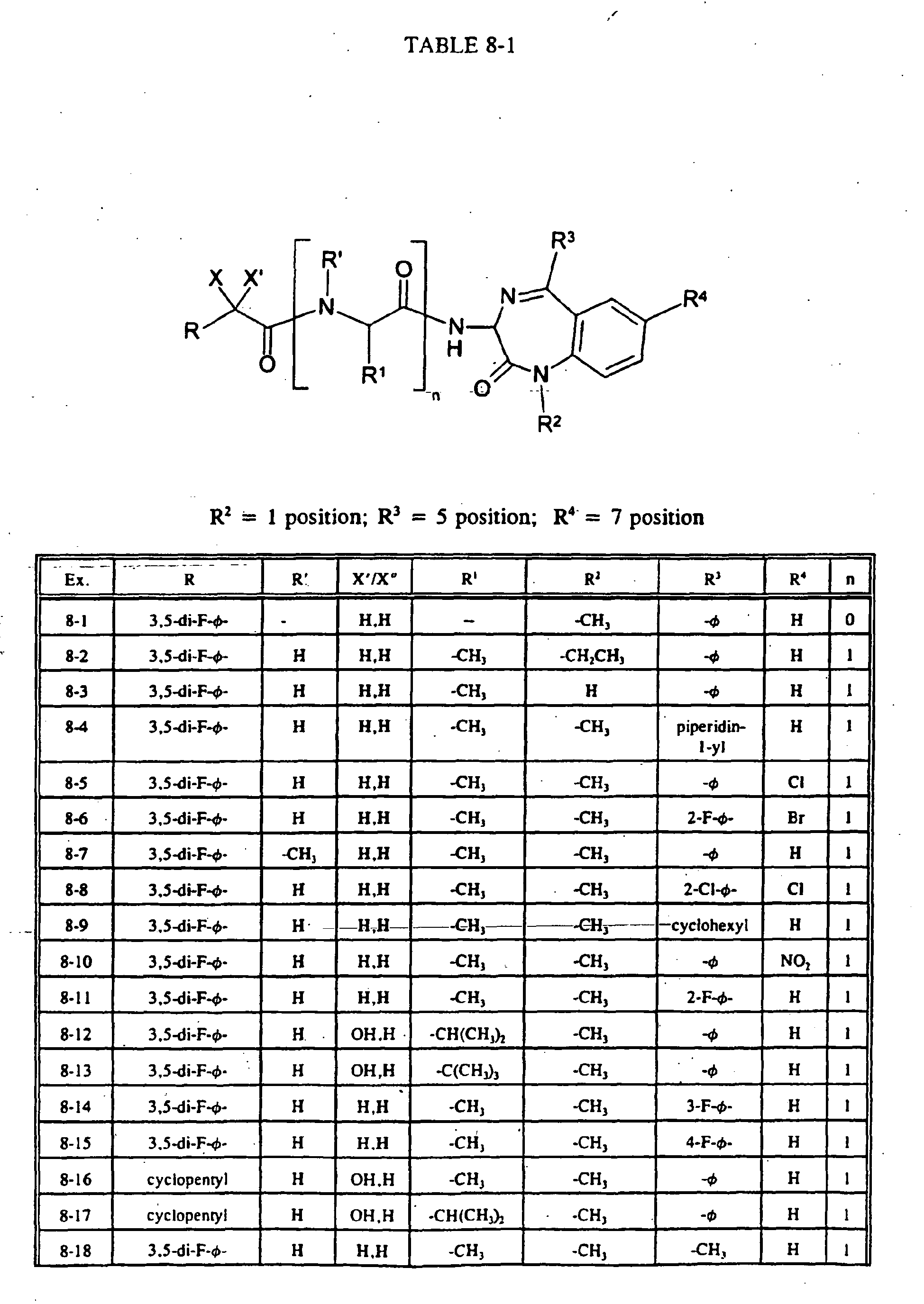

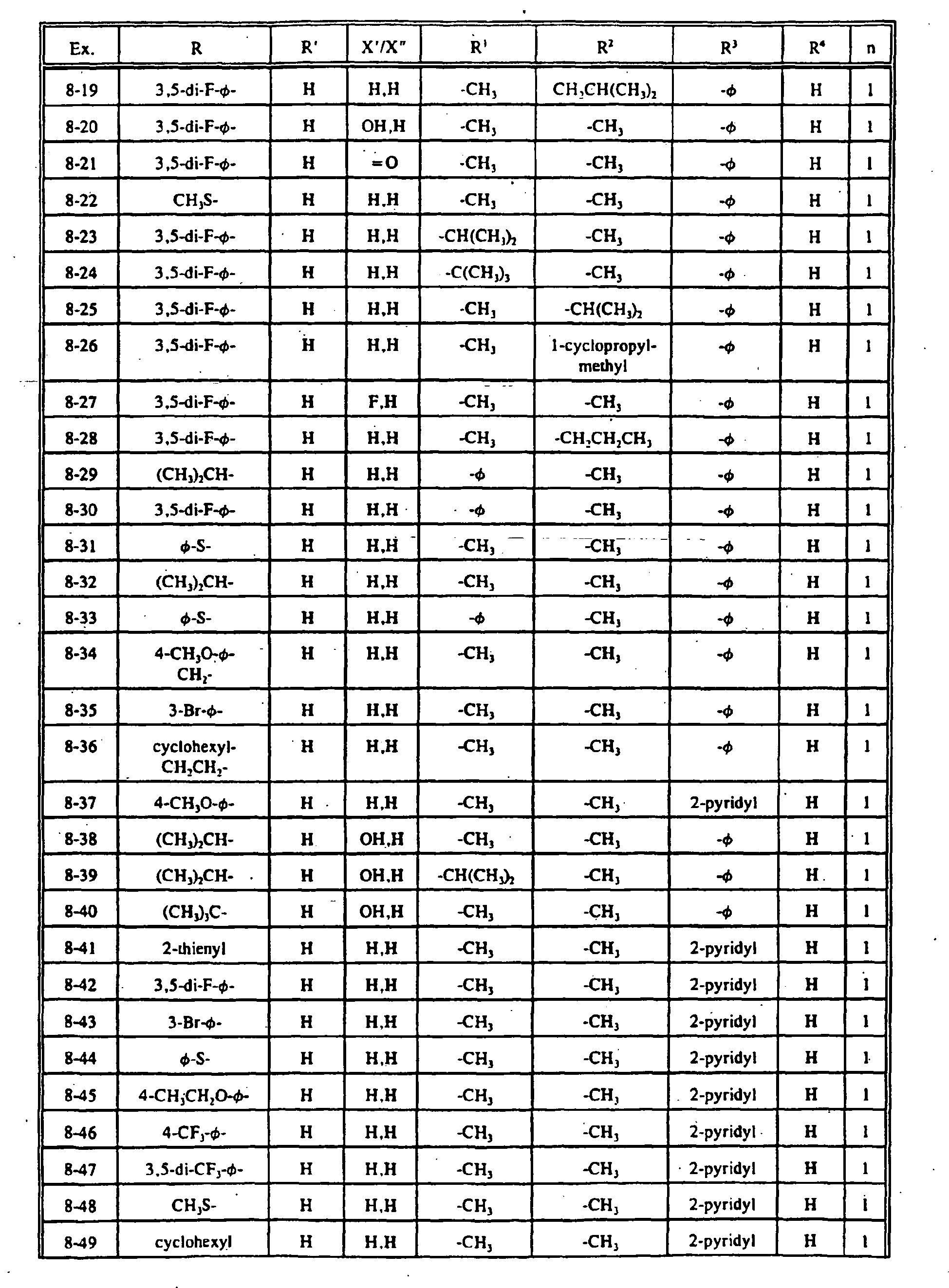

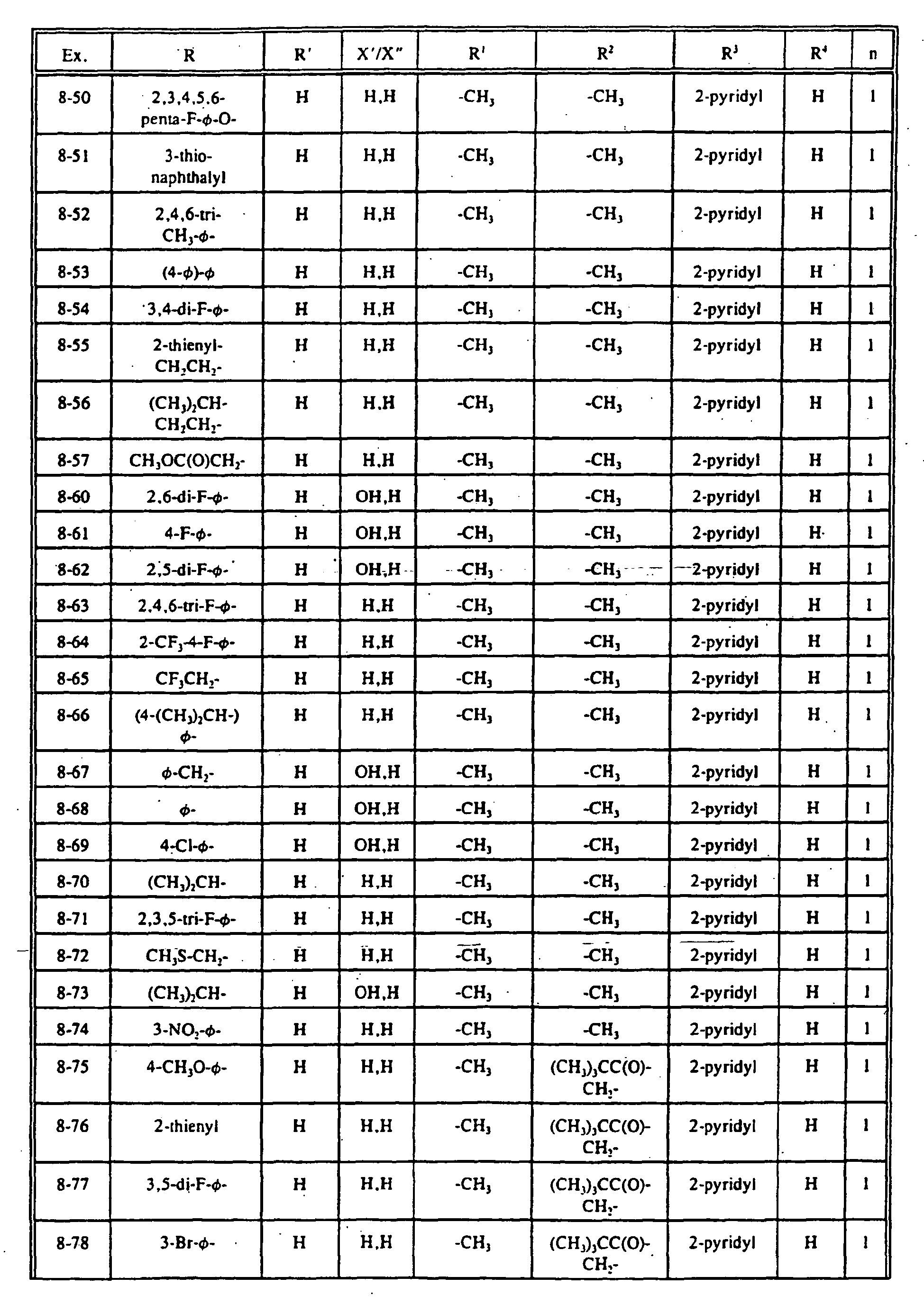

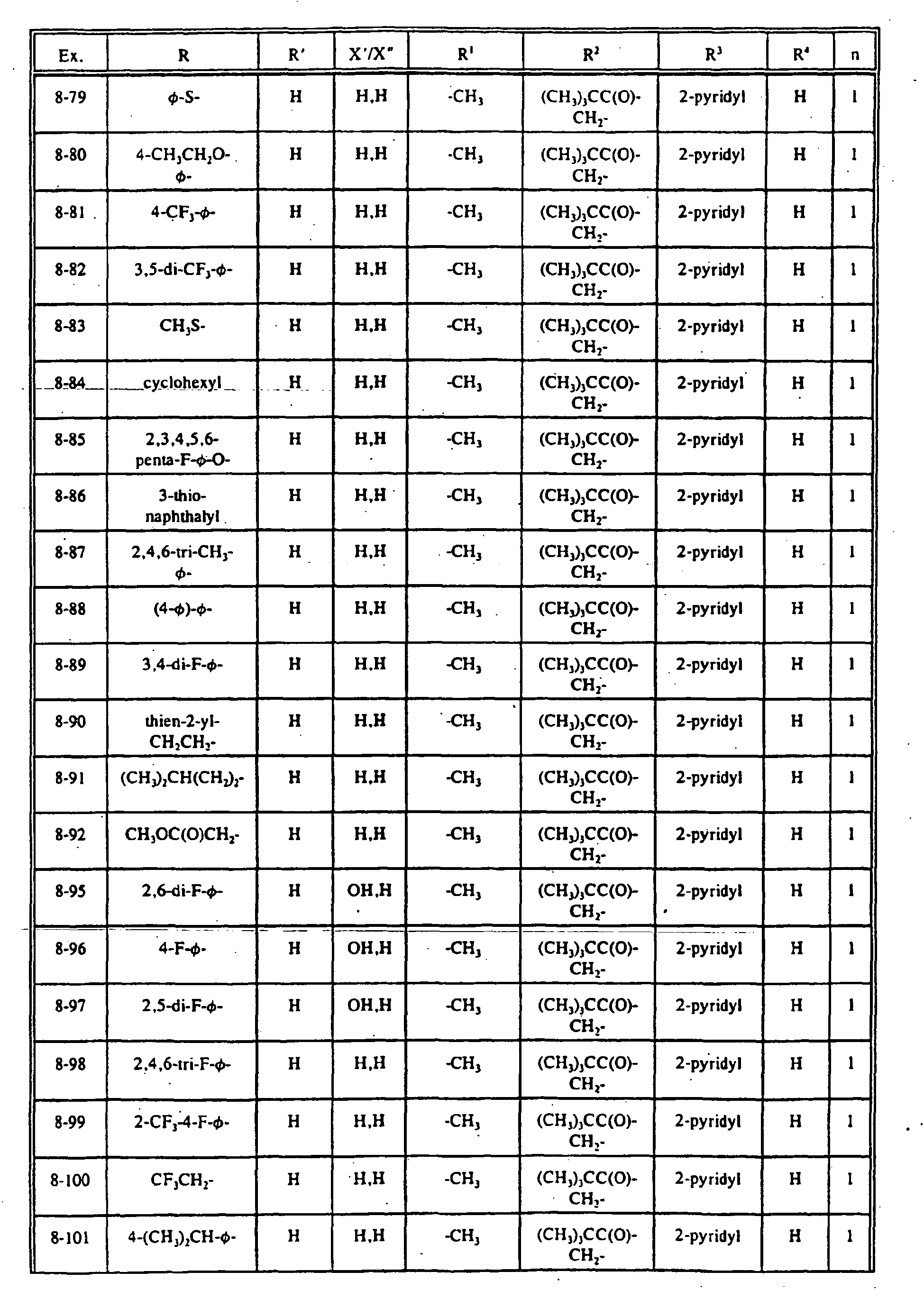

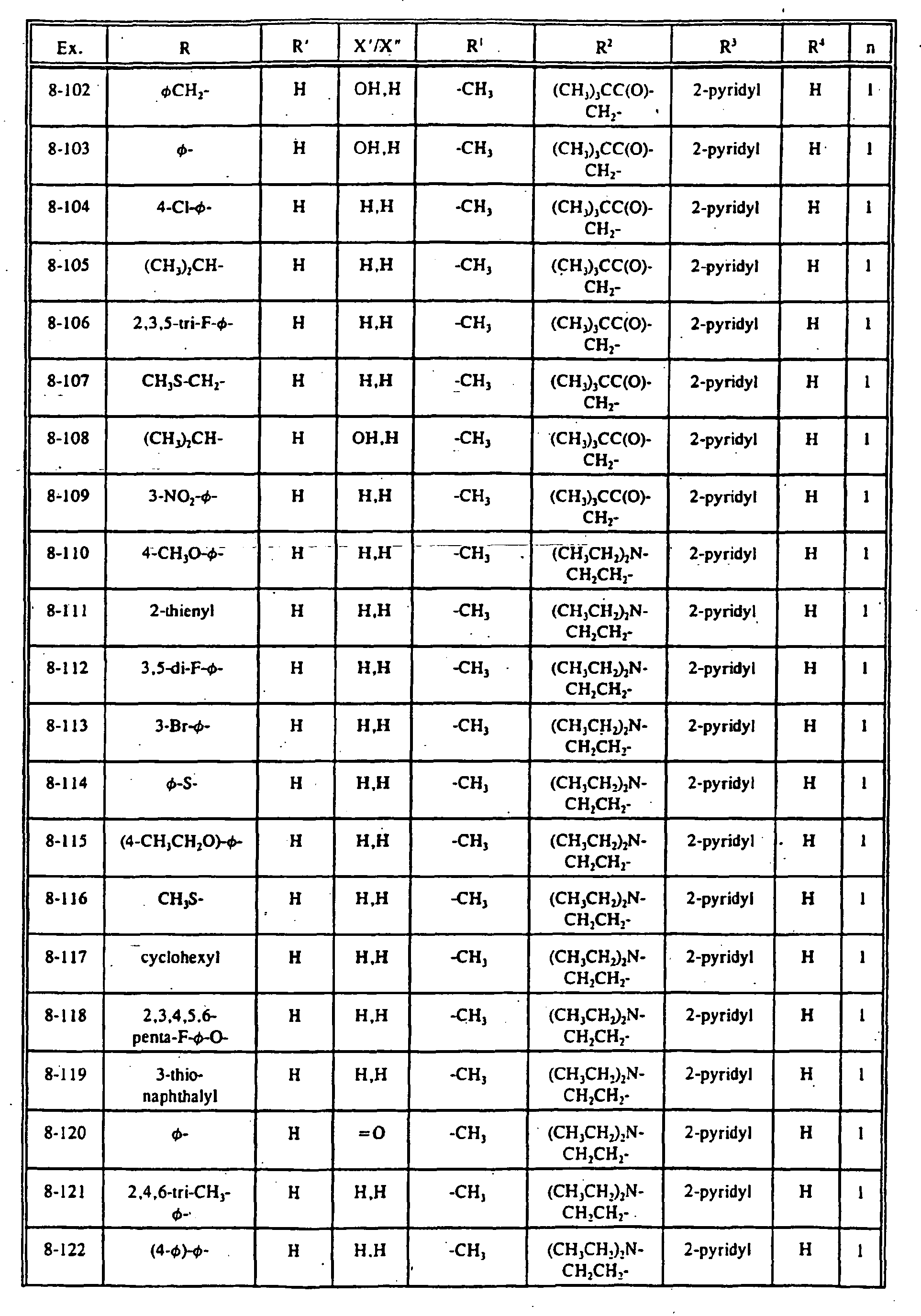

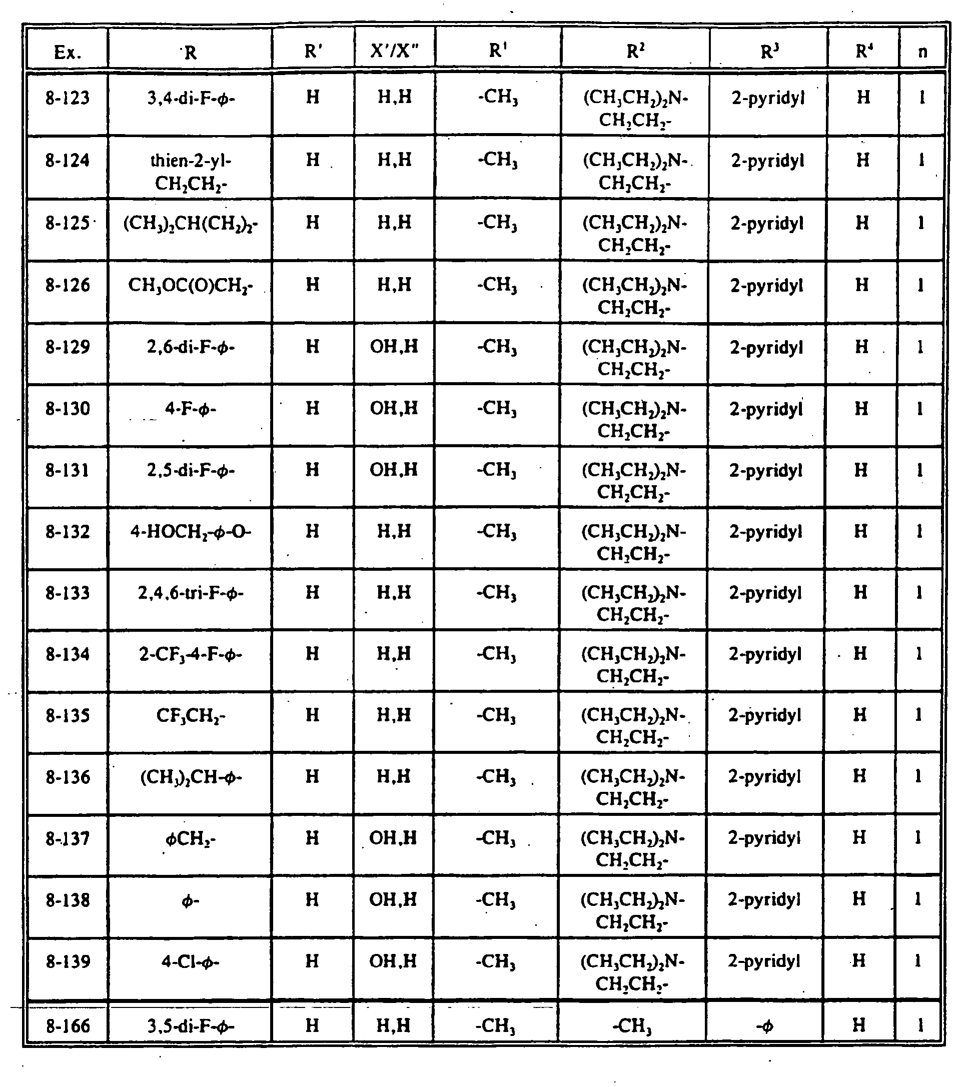

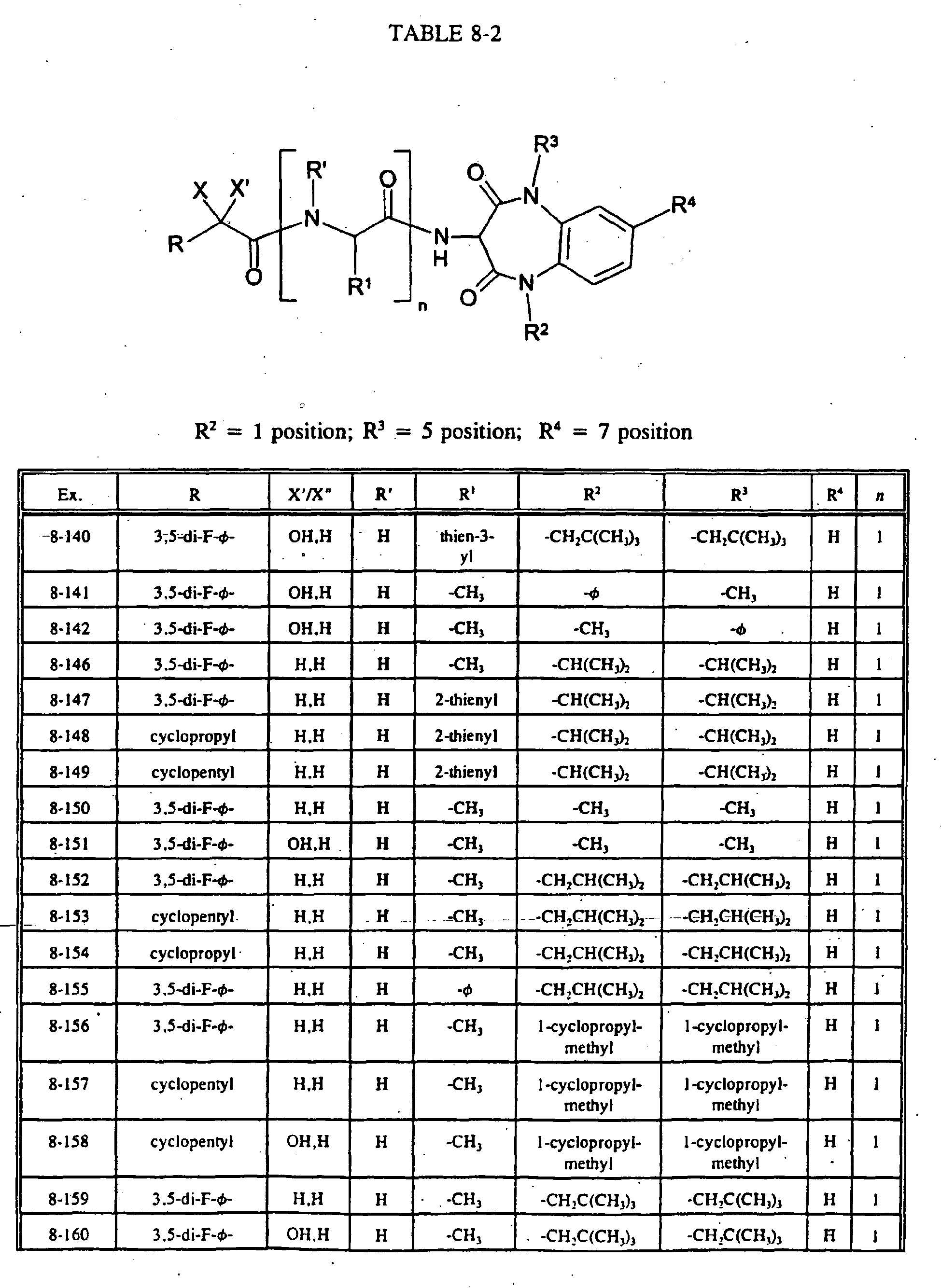

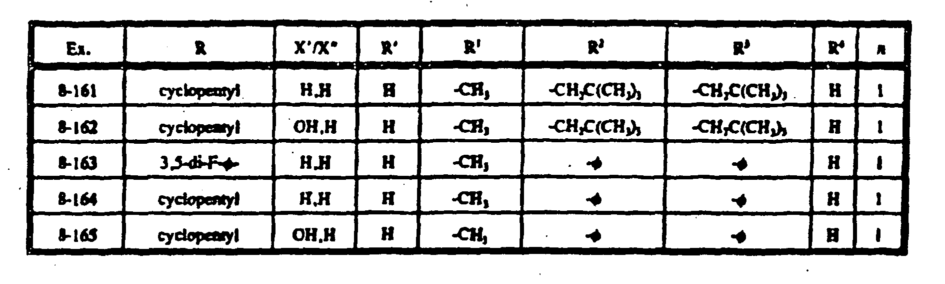

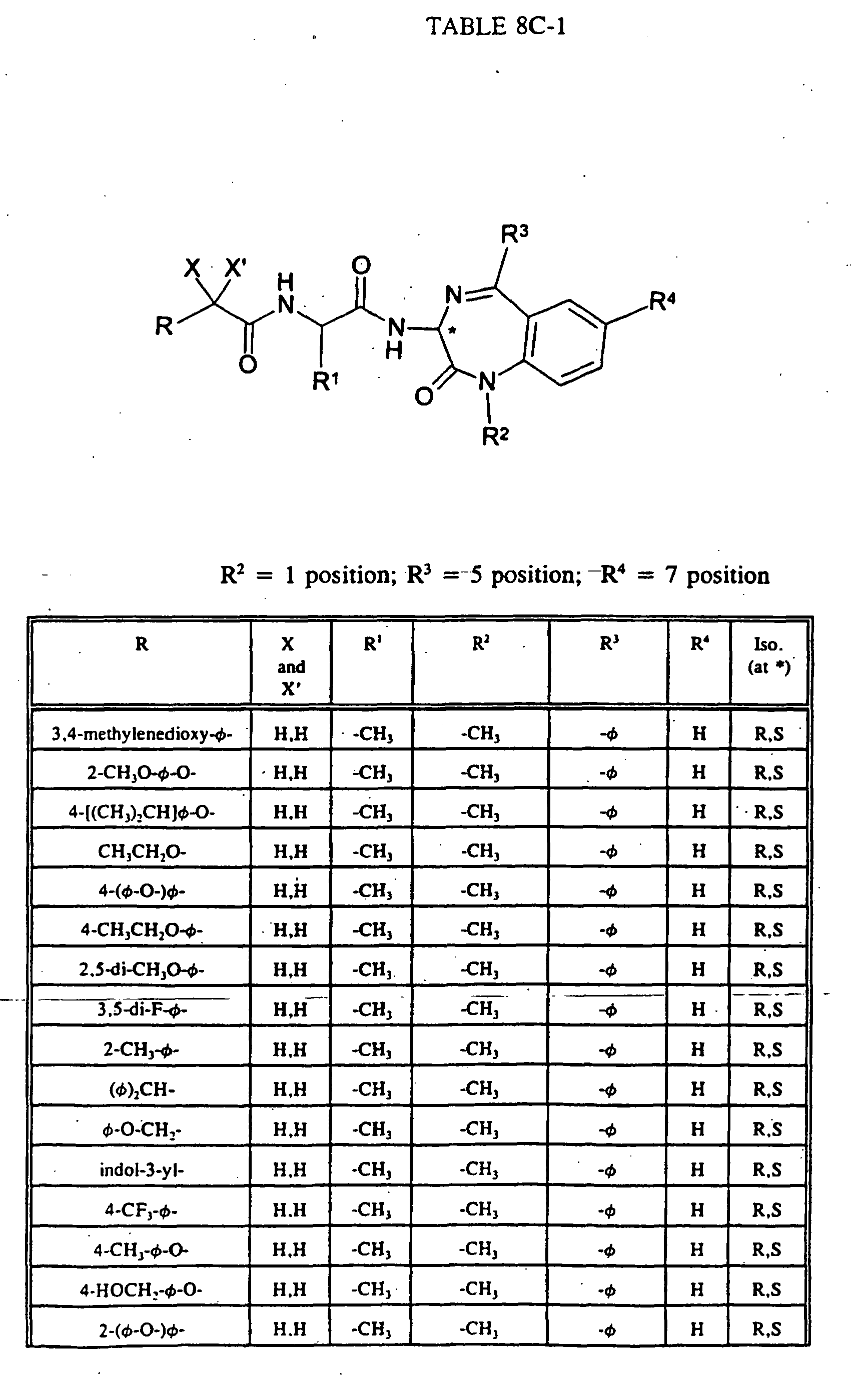

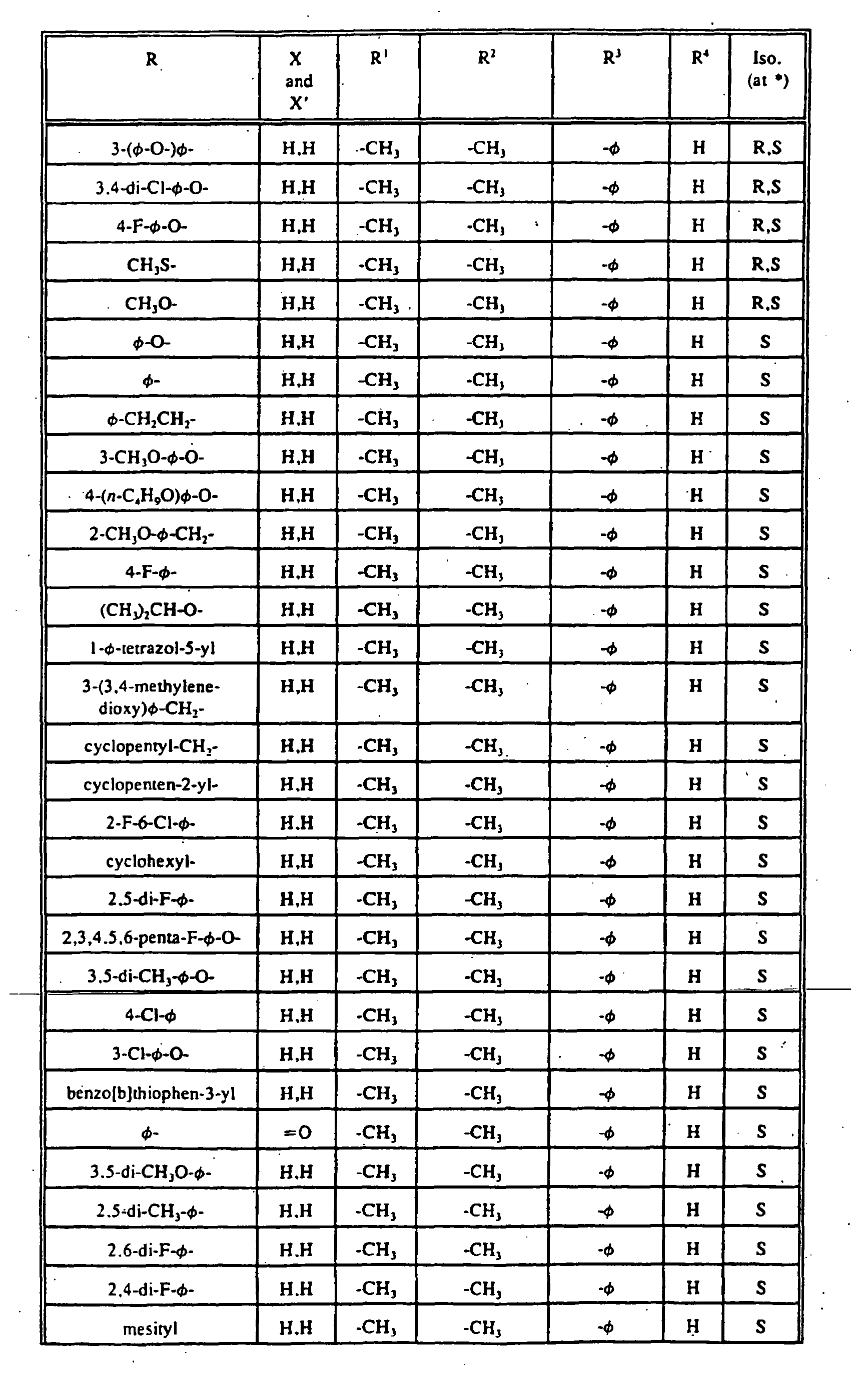

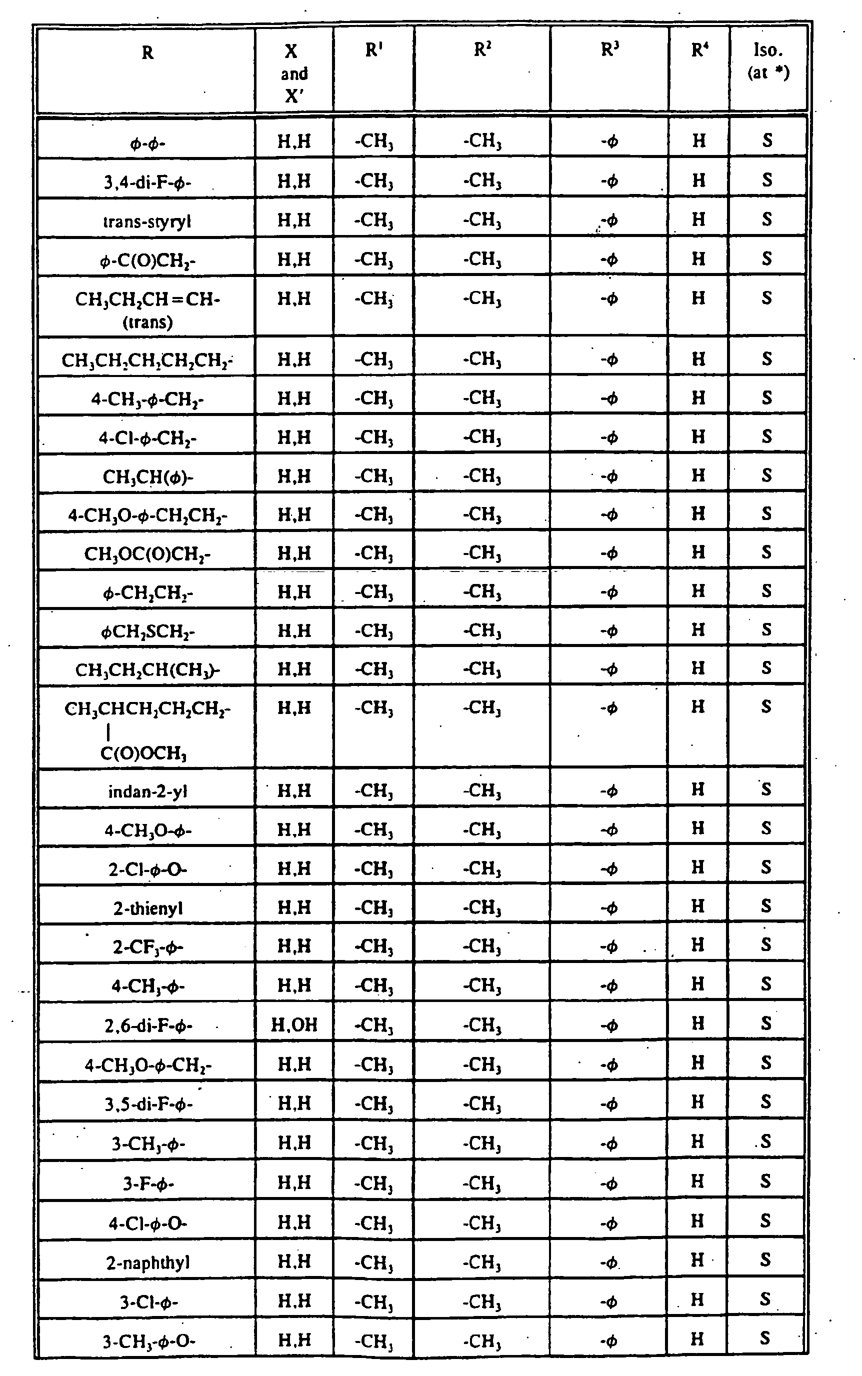

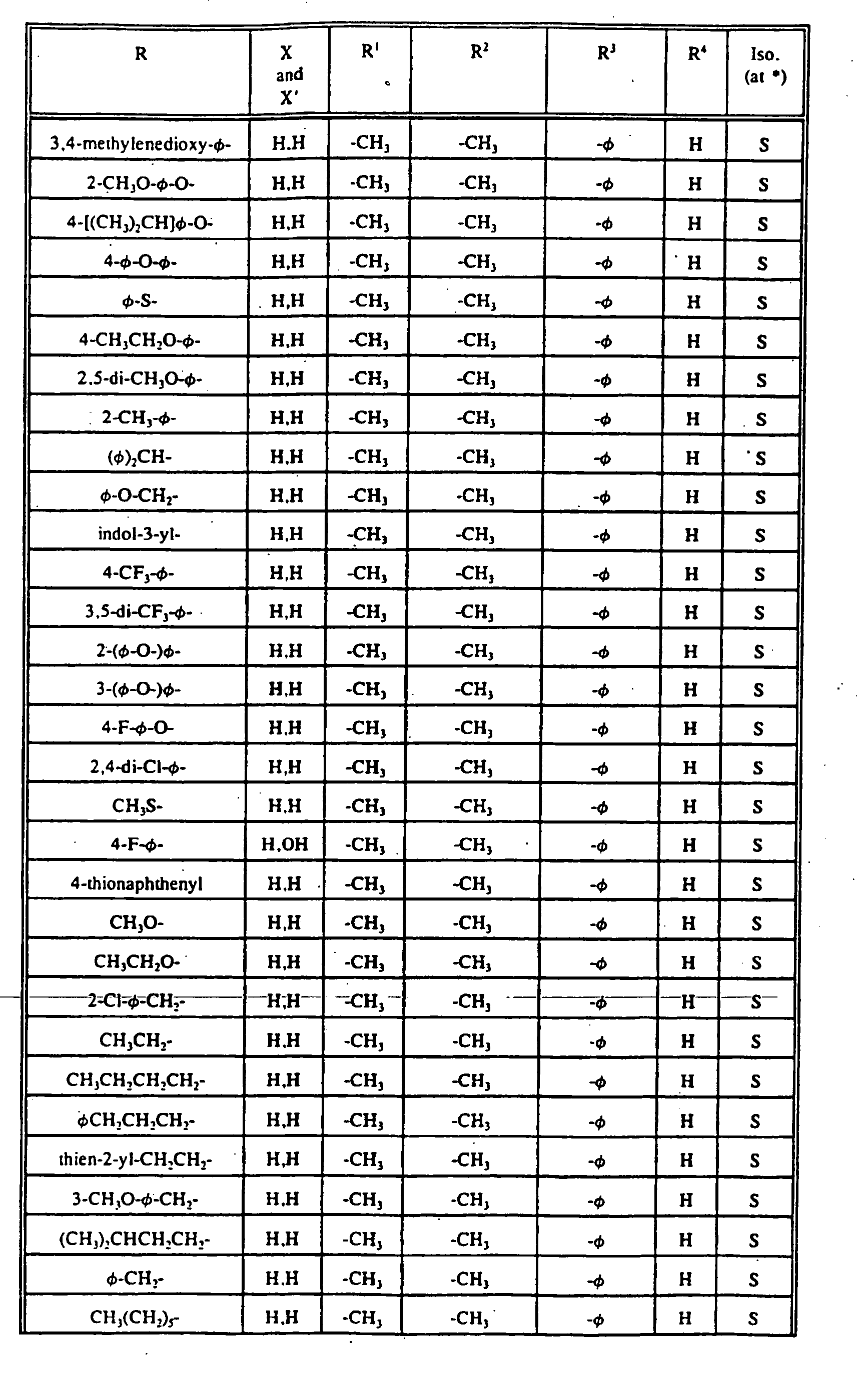

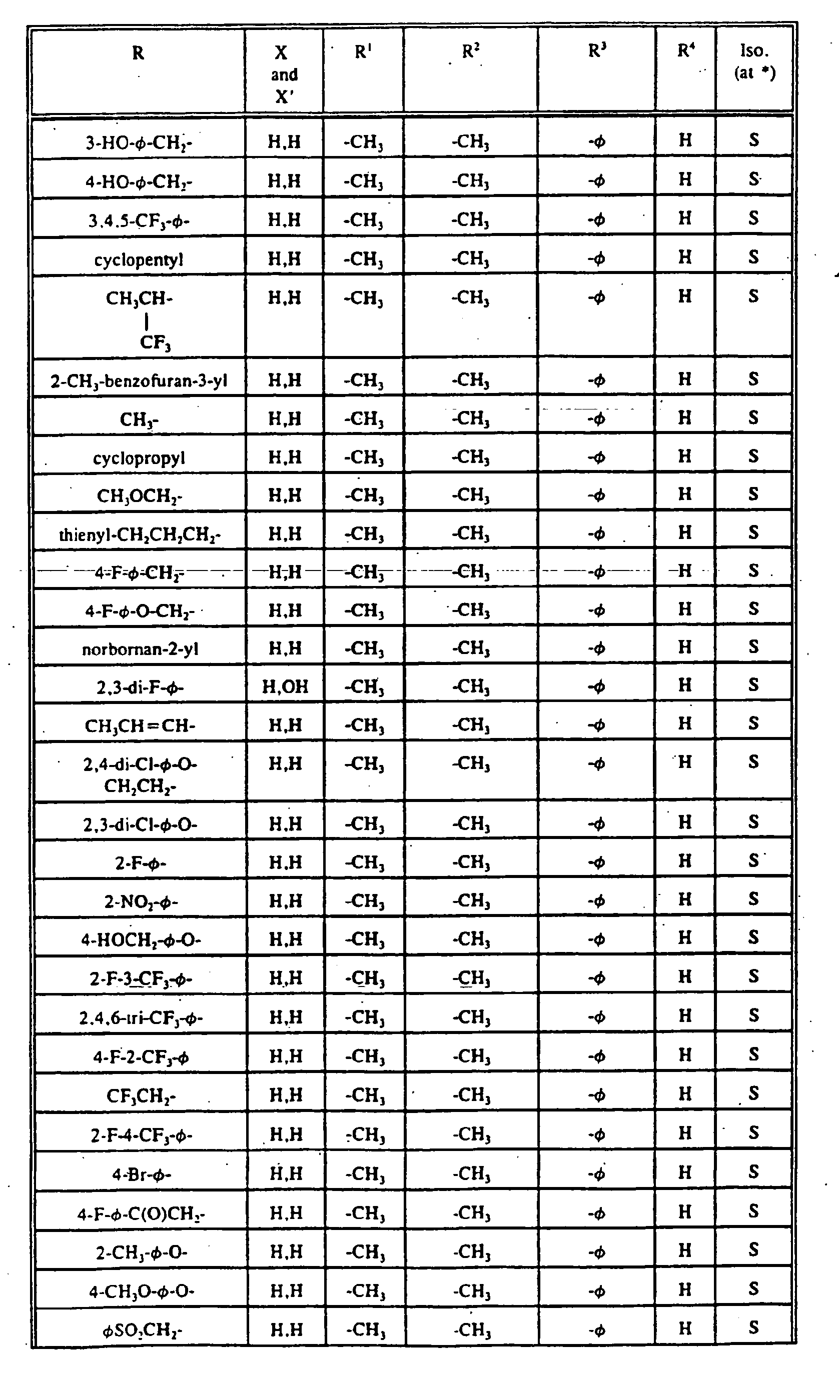

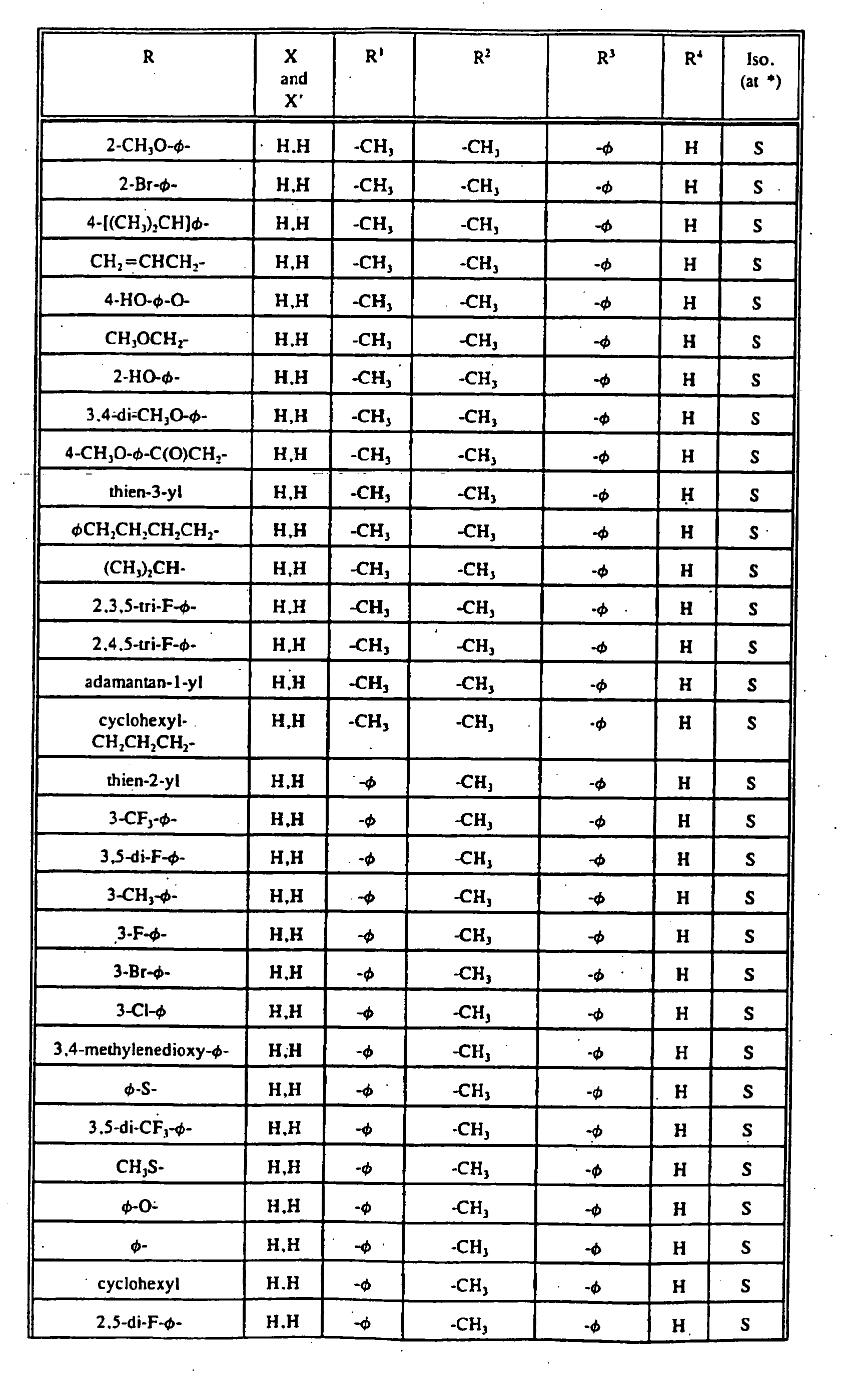

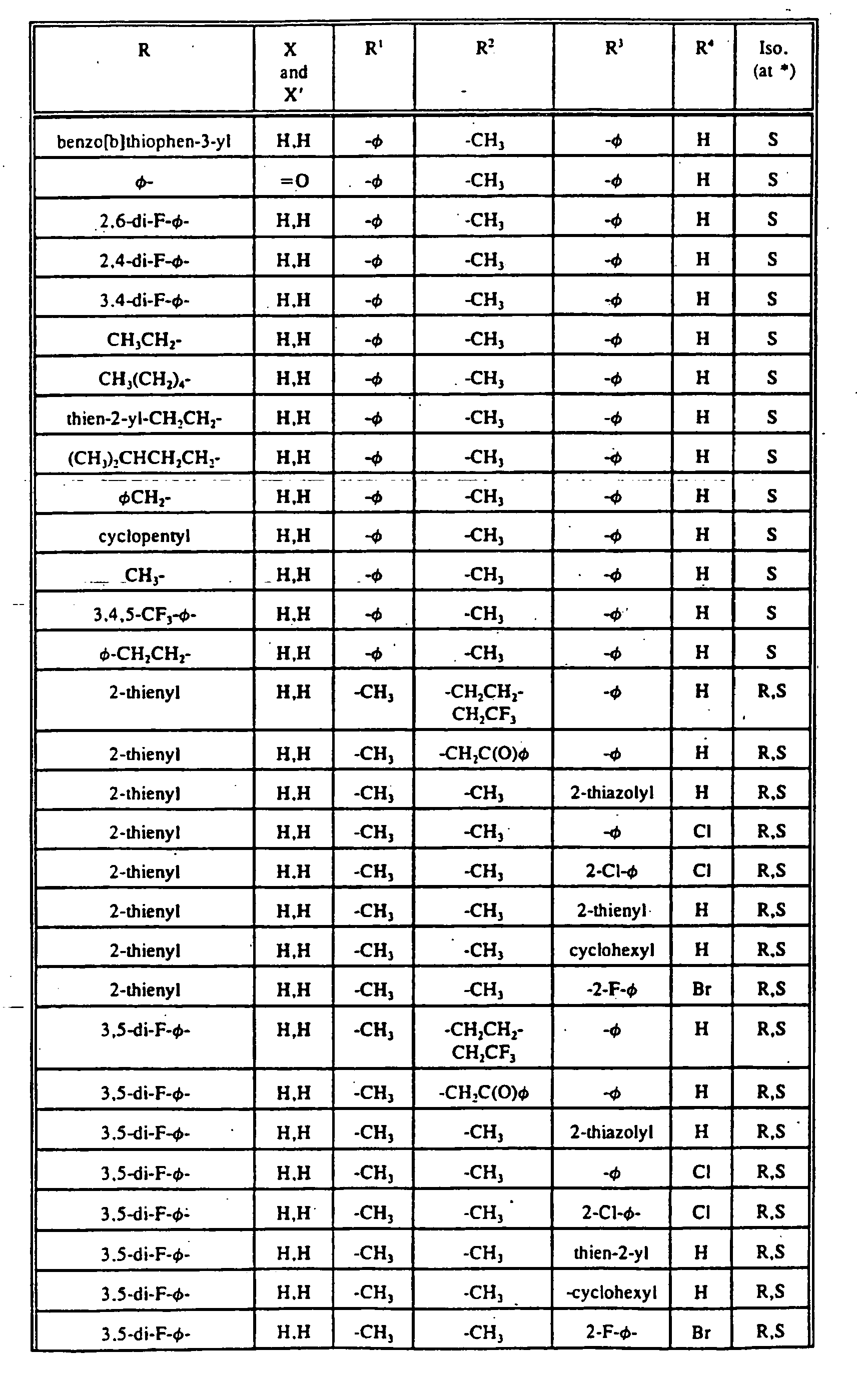

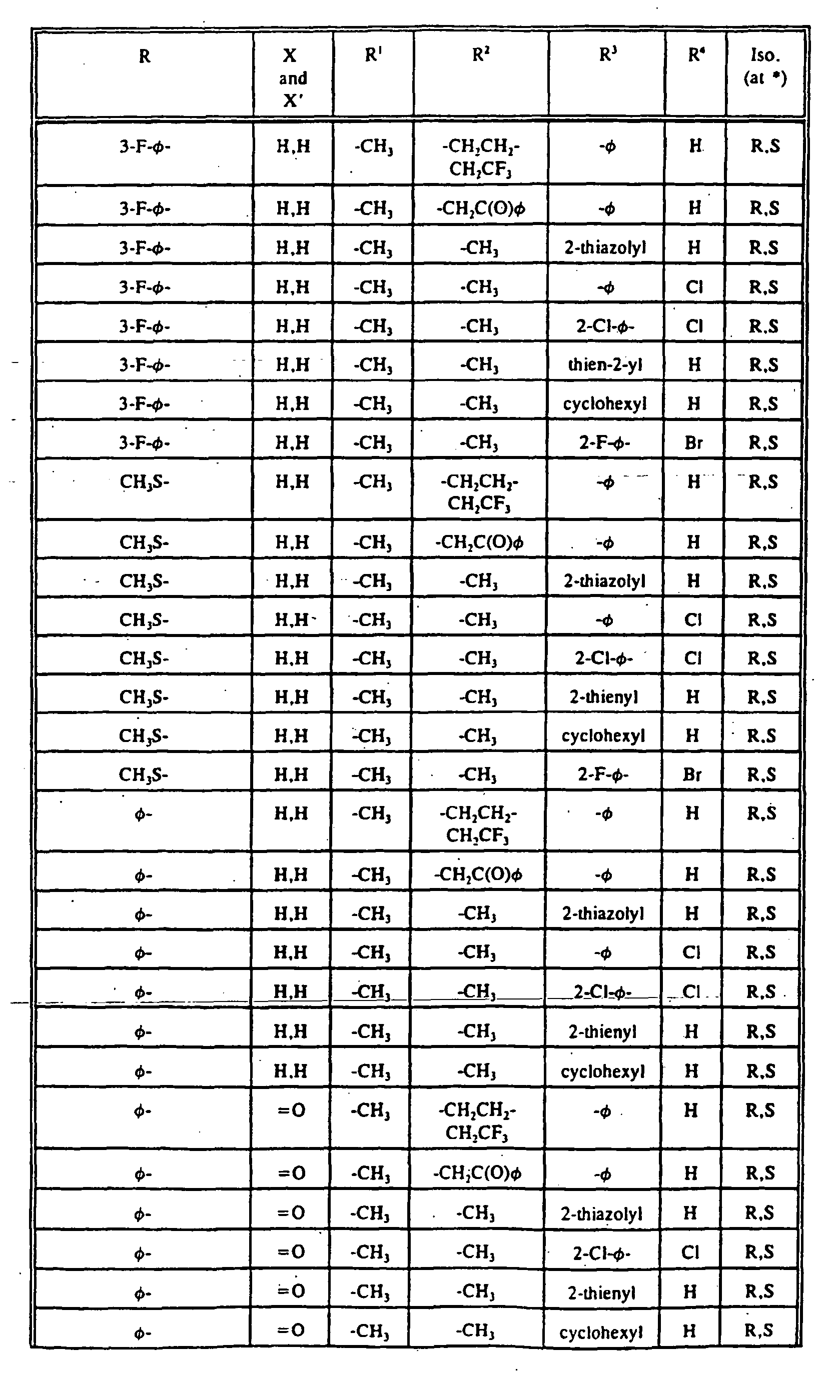

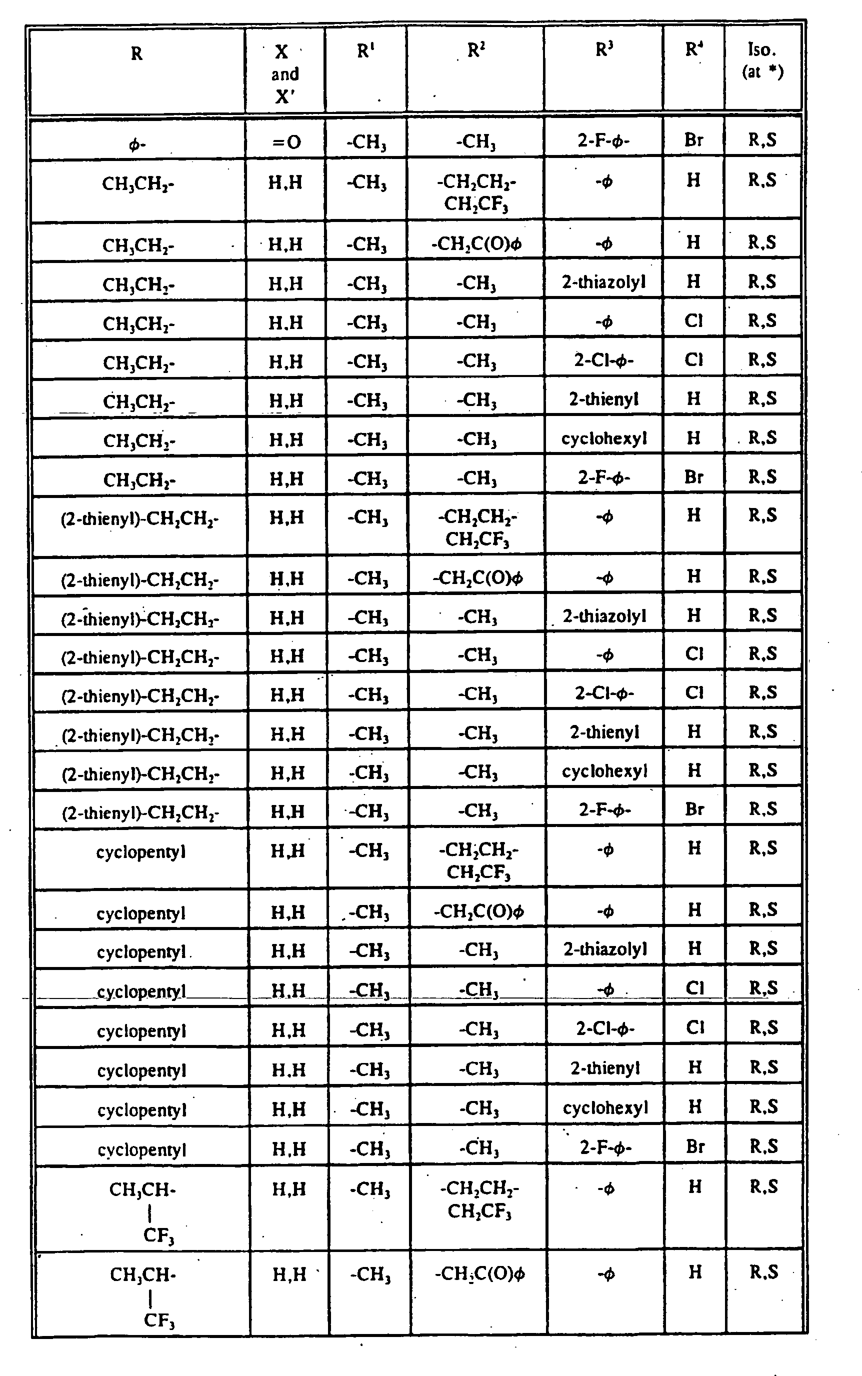

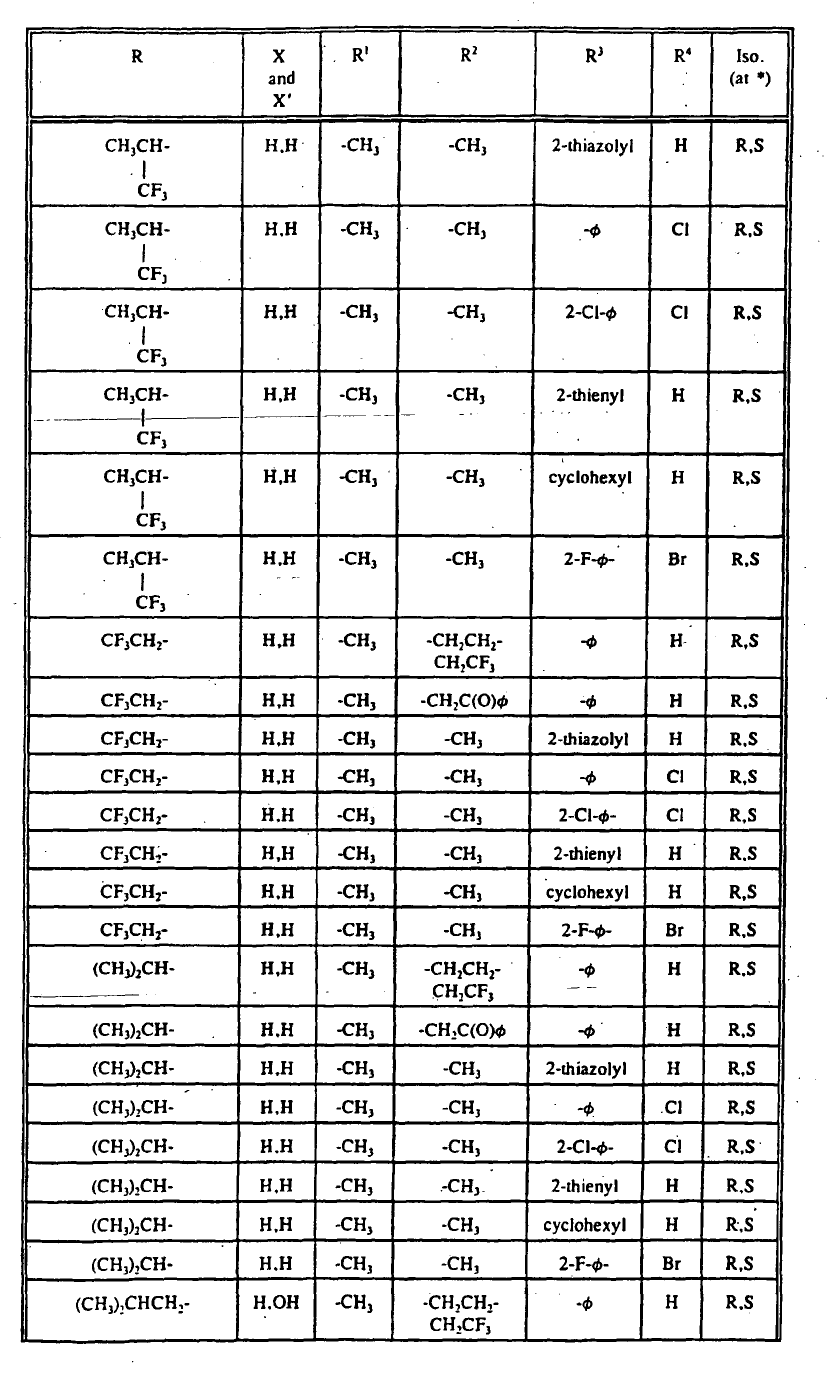

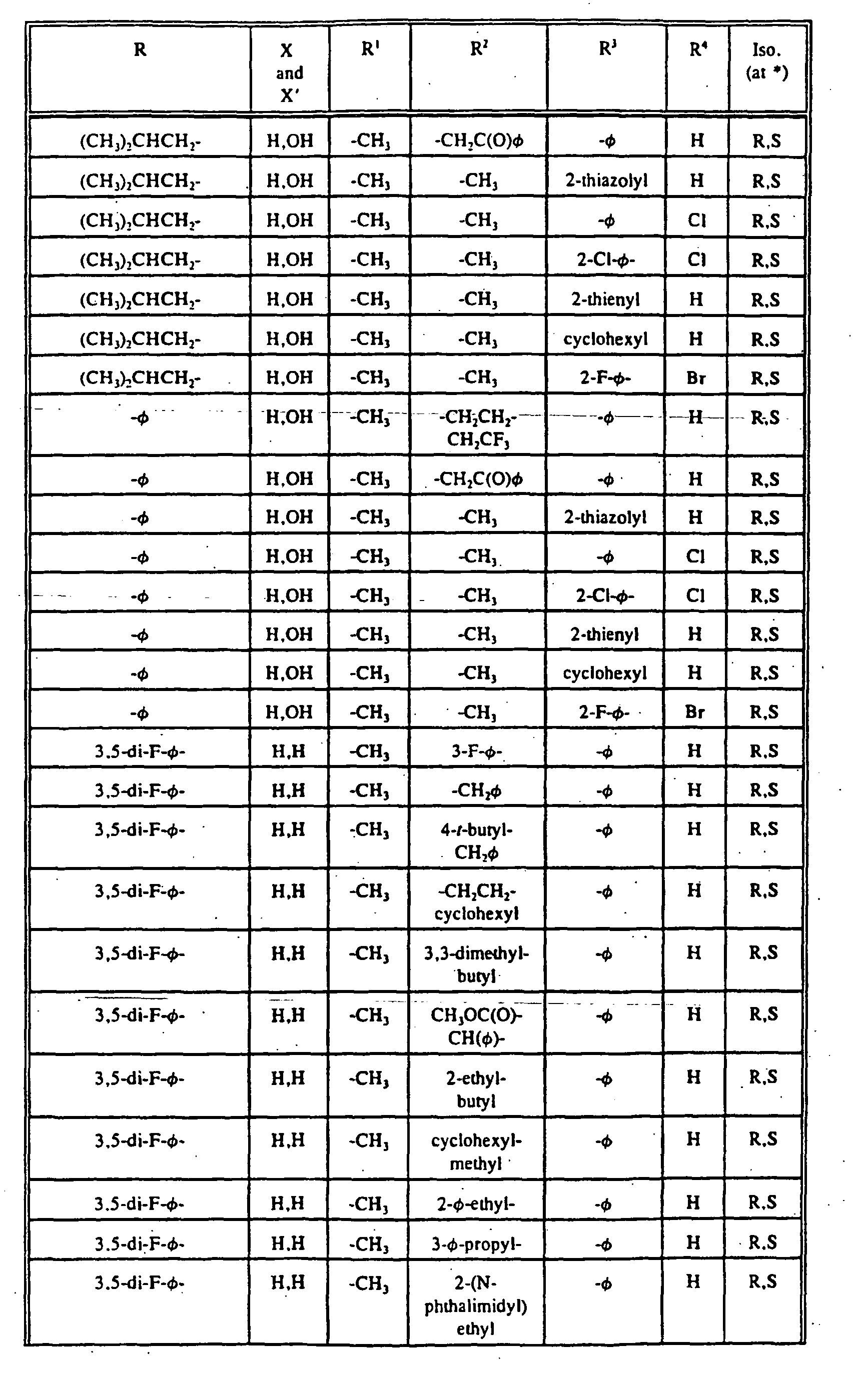

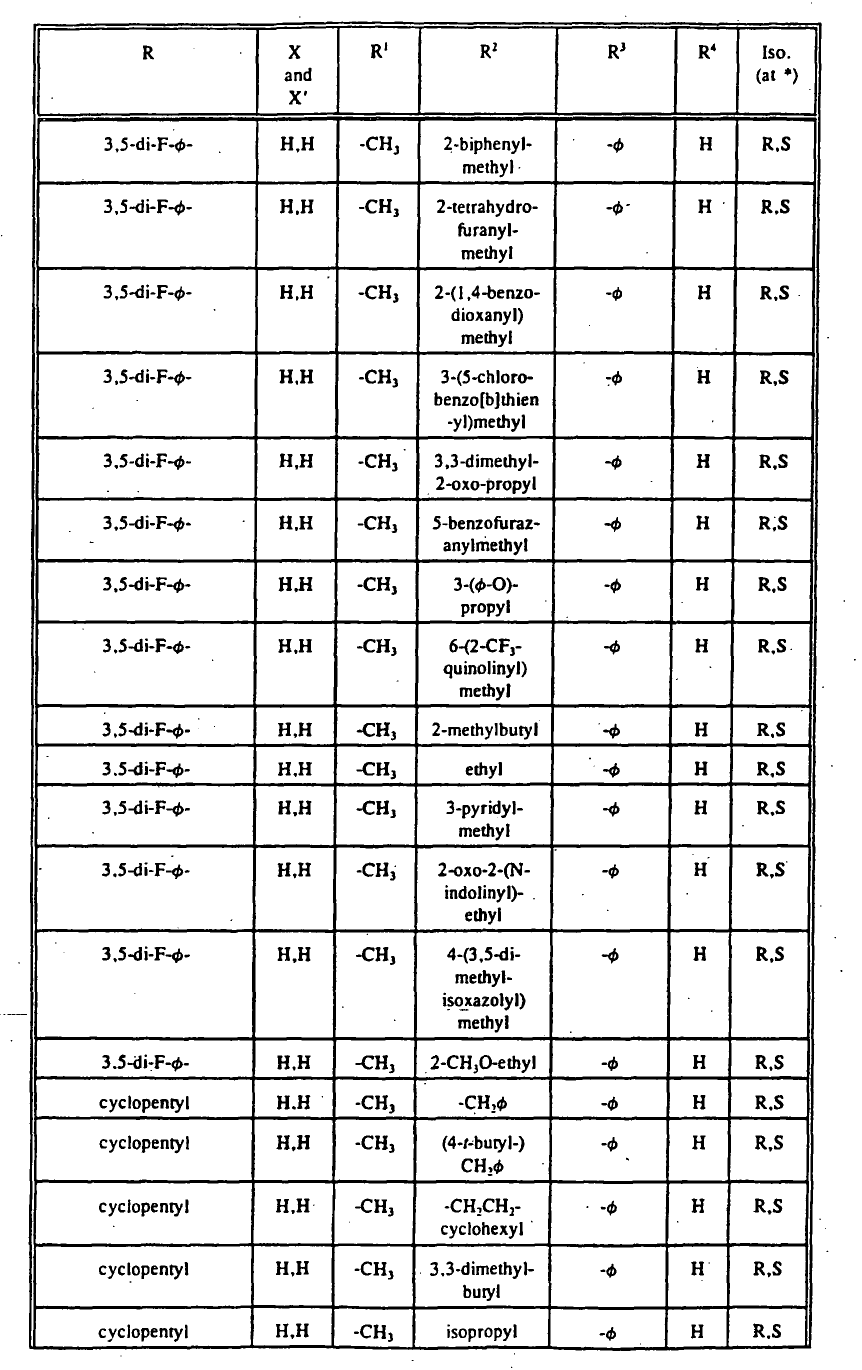

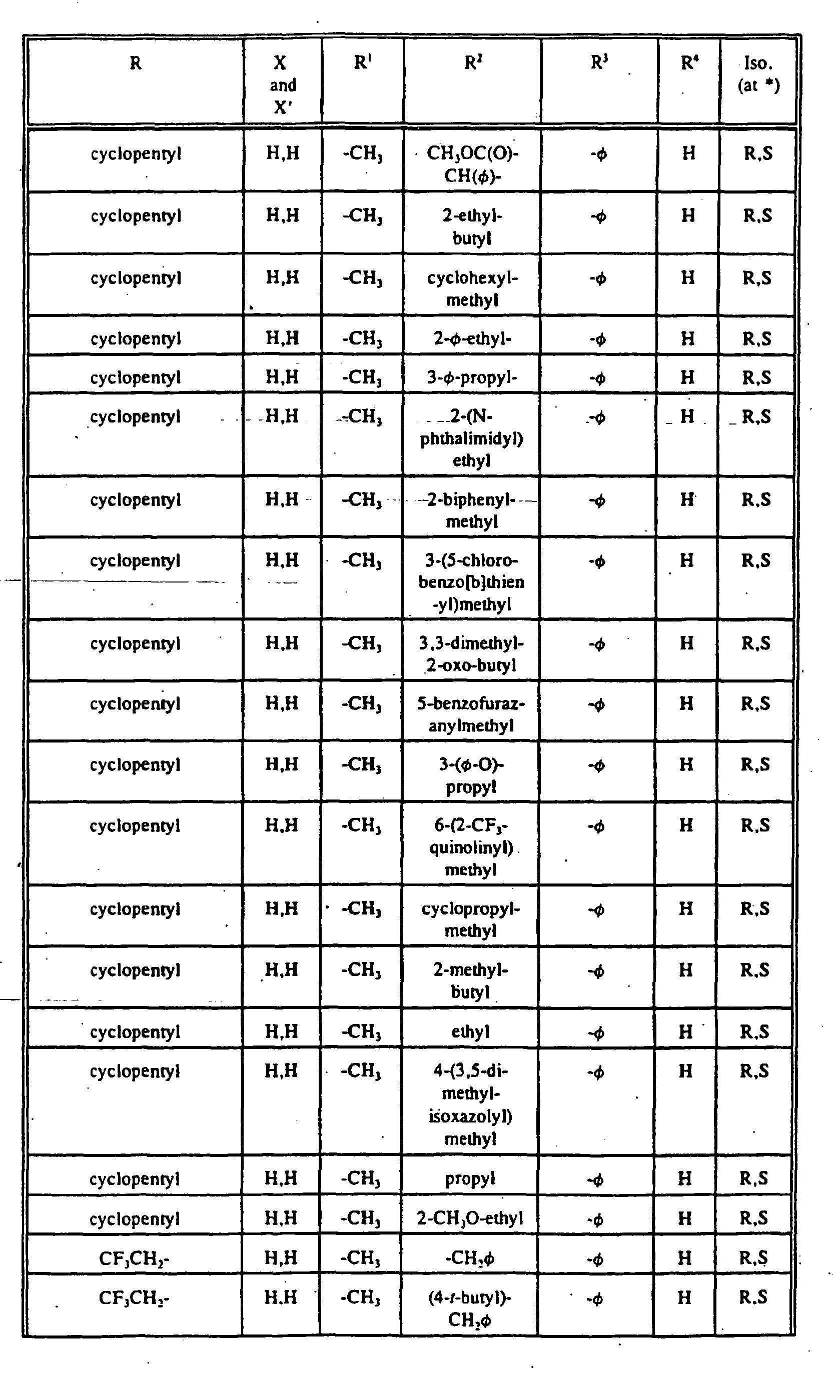

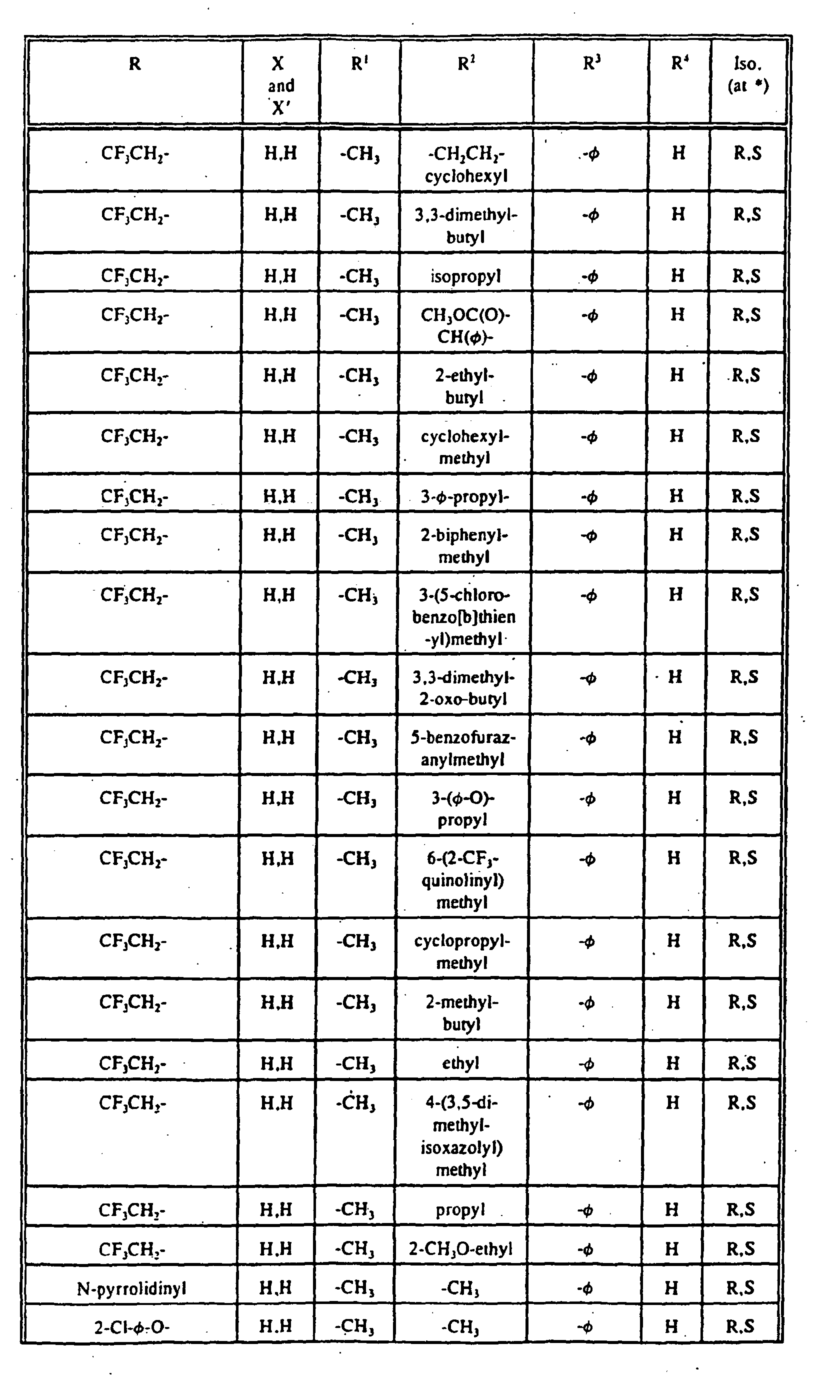

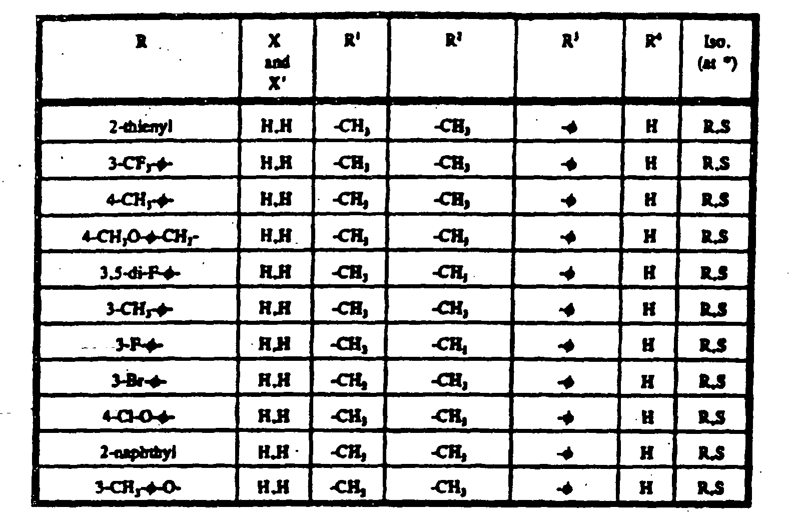

Also included within the scope of this invention are prodrugs of the compounds of formula I above including acylated forms of alcohols and thiols, aminals of one or more amines.

### DETAILED DESCRIPTION OF THE INVENTION

As above, this invention relates to compounds which inhibit β-amyloid peptide release and/or its synthesis, and, accordingly, have utility in treating Alzheimer's disease. However, prior to describing this invention in further detail, the following terms will first be defined.

### Definitions

The term "β-amyloid peptide" refers to a 39-43 amino acid peptide having a molecular weight of about 4.2 kD, which pepdde is substantially homologous to the form of the protein described by Glenner, et al.¹ including mutations and post-translational modifications of the normal β-amyloid peptide. In whatever form, the β-amyloid peptide is an approximate 39-43 amino acid fragment of a large membrane-spanning glycoprotein, referred to as the β-amyloid precursor protein (APP). Its 43-amino acid sequence is: 
1
Asp Ala Glu Phe Arg His Asp Ser Gly Tyr

11
Glu Val His His Gln Lys Leu Val Phe Phe

21
Ala Glu Asp Val Gly Ser Asn Lys Gly Ala

31
Ile Ile Gly Leu Met Val Gly Gly Val Val

41
Ile Ala Thr (SEQ ID NO: 1)
 or a sequence which is substantially homologous thereto.

"Alkyl" refers to monovalent alkyl groups preferably having from 1 to 10 carbon atoms and more preferably 1 to 6 carbon atoms. This term is exemplified by groups such as methyl, ethyl, *n*-propyl, *iso*-propyl, *n*-butyl, *iso*-butyl and *n*-hexyl.

"Alkaryl" refers to -alkylene-aryl groups preferably having from 1 to 8 carbon atoms in the alkylene moiety and from 6 to 10 carbon atoms in the aryl moiety. Such alkaryl groups are exemplified by benzyl and phenethyl.

"Alkoxy" refers to the group "alkyl-O-". Preferred alkoxy groups include, by way of example, methoxy, ethoxy, n-propoxy, iso-propoxy, *n*-butoxy, tert-butoxy, *sec*-butoxy, *n*-pentoxy, n-hexoxy and 1,2-dimethylbutoxy.

"Alkenyl" refers to alkenyl groups preferably having from 2 to 10 carbon atoms and more preferably 2 to 6 carbon atoms and having at least 1 and preferably from 1-2 sites of alkenyl unsaturation. Preferred alkenyl groups include ethenyl (-CH=CH₂), n-propenyl (-CH₂CH=CH₂), *iso*-propenyl and (-C(CH₃)=CH₂).

"Alkynyl" refers to alkynyl groups preferably having from 2 to 10 carbon atoms and more preferably 2 to 6 carbon atoms and having at least 1 and preferably from 1-2 sites of alkynyl unsaturation. Preferred alkynyl groups include ethynyl (-CH≡CH₂), and propargyl (-CH₂C≡CH).

"Aryl" refers to phenyl or naphthyl wherein such aryl groups can optionally be substituted with from 1 to 5 substituents selected from the group consisting of alkyl, alkoxy, halo, cyano, nitro, trihalomethyl, and thioalkoxy.

"Aryloxy" refers to the group aryl-O- wherein the aryl group is as defined above including optionally substituted aryl groups as also defined above.

"Carboxyalkyl" refers to the group "-C(O)Oalkyl" where alkyl is as defined above.

"Cycloalkyl" refers to cyclic alkyl groups of from 3 to 12 carbon atoms having a single cyclic ring or multiple condensed rings. Such cycloalkyl groups include, by way of example, single ring structures such as cyclopropyl, cyclobutyl, cyclopentyl, cyclooctyl, and the like, or multiple ring structures such as adamantanyl.

"Cycloalkenyl" refers to cyclic alkenyl groups of from 4 to 8 carbon atoms having a single cyclic ring and at least one point of internal unsaturation. Examples of suitable cycloalkenyl groups include, for instance, cyclobut-2-enyl, cyclopent-3-enyl and cyclooct-3-enyl.

"Halo" or "halogen" refers to fluoro, chloro, bromo and iodo and preferably is either fluoro or chloro.

"Heteroaryl" refers to an aromatic carbocyclic group of from 1 to 15 carbon atoms and 1 to 4 heteroatoms selected from oxygen, nitrogen and sulfur within at least one ring (if there is more than one ring).

Unless otherwise constrained by the definition for the heteroaryl substituent, such heteroaryl groups can be optionally substituted with I to 5 substituents selected from the group consisting of alkyl, alkoxy, aryl, aryloxy, halo, nitro, heteroaryl, thiol, thioalkoxy, thioaryloxy and trihalomethyl . Such heteroaryl groups can have a single ring (e.g., pyridyl or furyl) or multiple condensed rings (e.g., indolizinyl or benzothienyl). Preferred heteroaryls include pyridyl, pyrrolyl and furyl.

"Heterocycle" or "heterocyclic" refers to a monovalent saturated or unsaturated group having a single ring or multiple condensed rings, from 1 to 15 carbon atoms and from 1 to 4 hetero atoms selected from nitrogen, sulfur or oxygen within the ring.

Unless otherwise constrained by the definition for the heterocyclic substituent, such heterocyclic groups can be optionally substituted with 1 to 5 substituents selected from the group consisting of alkyl, alkoxy, aryl, aryloxy, halo, nitro, heteroaryl, thiol, thioalkoxy, thioaryloxy and trihalomethyl. Such heterocyclic groups can have a single ring or multiple condensed rings. Preferred heterocyclics include morpholino and piperidinyl.

Examples of nitrogen heterocycles and heteroaryls include, but are not limited to, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, morpholino, piperidinyl and tetrahydrofuranyl, as well as N-alkoxy-nitrogen containing heterocycles.

"Thiol" refers to the group -SH.

"Thioalkoxy" refers to the group -S-alkyl.

"Thioaryloxy" refers to the group aryl-S- wherein the aryl group is as defined above including optionally substituted aryl groups also defined above.

As to any of the above groups which contain I or more substituents, it is understood, of course, that such groups do not contain any substitution or substitution patterns which are sterically impractical and/or synthetically non-feasible.

"Pharmaceutically acceptable salt" refers to pharmaceutically acceptable salts of a compound of Formula I which salts are derived from a variety of organic and inorganic counter ions well known in the art and include, by way of example only, sodium, potassium, calcium, magnesium and ammonium tetraalkylammonium; and when the molecule contains a basic functionality, salts of organic or inorganic acids, such as hydrochloride, hydrobromide, tartrate, mesylate, acetate, maleate and oxalate can be used as the pharmaceutically acceptable salt.

The term "protecting group" or "blocking group" refers to any group which when bound to one or more hydroxyl, amino or carboxyl groups of the compounds (including intermediates thereof such as the aminolactams, aminolactones, etc.) prevents reactions from occurring at these groups and which protecting group can be removed by conventional chemical or enzymatic steps to reestablish the hydroxyl, amino or carboxyl group. The particular removable blocking group employed is not critical and preferred removable hydroxyl blocking groups include conventional substituents such as allyl, benzyl, acetyl, chloroacetyl, thiobenzyl, benzylidine, phenacyl, t-butyl-diphenylsilyl and any other group that can be introduced chemically onto a hydroxyl functionality and later selectively removed either by chemical or enzymatic methods in mild conditions compatible with the nature of the product.

Preferred removable amino blocking groups include conventional substituents such as t-butyoxycarbonyl (t-BOC), benzyloxycarbonyl (CBZ), and the like which can be removed by conventional conditions compatible with the nature of the product.

Preferred carboxyl protecting groups include esters such as methyl, ethyl, propyl, *t*-butyl etc. which can be removed by mild hydrolysis conditions compatible with the nature of the product.

### Compound Preparation

When *n* is one or two, the compounds according to any one of claims 1 to 4 are readily prepared by conventional amidation of a carboxyl acid as shown in reaction (1) below where, for the sake of illustration, n is one: 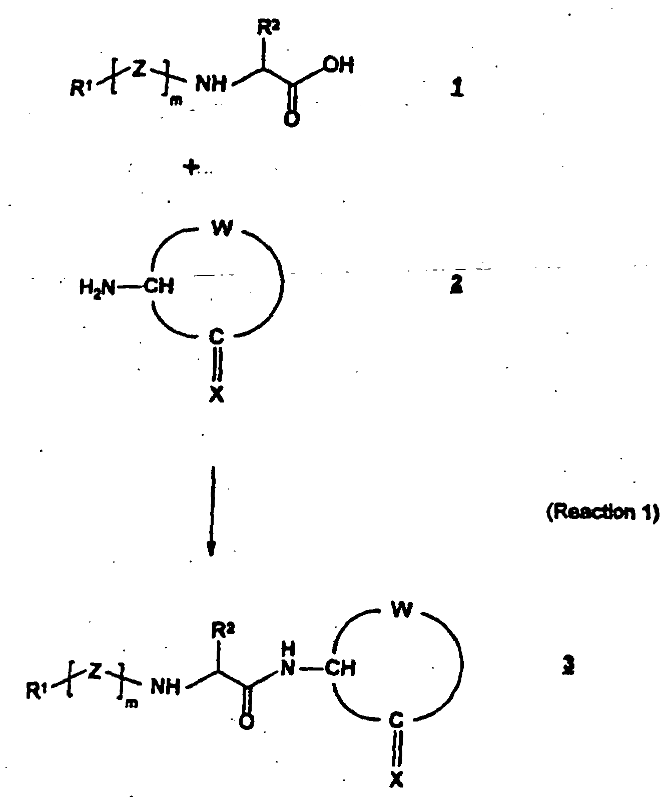 wherein R¹, R², Z and *m* are as defined above and wherein the lactam rings of the compounds of the invention according to any one of claims 1 to 4 are illustrated by the ring:

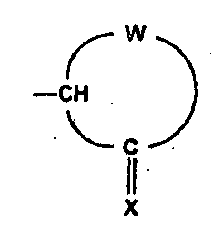

The reaction is conventionally conducted by using at least a stoichiometric amount of carboxylic acid **1** and amine **2.** This reaction is conventionally conducted for peptide synthesis and synthetic methods used therein can also be employed to prepare compound **3** which is a compound of formula I above. For example, well known coupling reagents such as carbodiimides with or without the use of well known additives such as N-hydroxysuccinimide, 1-hydroxybenzotriazole. etc. can be used to facilitate coupling. The reaction is conventionally conducted in an inert aprotic polar diluent such as dimethylformamide, dichloromethane, chloroform, acetonitrile and tetrahydrofuran. Alternatively, the acid halide of compound **1** can be employed in reaction (1) and, when so employed, it is typically employed in the presence of a suitable base to scavenge the acid generated during the reaction. Suitable bases include, by way of example, triethylamine, diisopropylethylamine and N-methylmorpholine.

When *n* is zero, the compounds of formula I can be prepared by N-substitution reactions of compound **2**. For example, when *m* = 0 and *n* = 0, N-arylation reactions on compound **2** lead to compounds of formula I. When *m* = 1 and *n* = 0,) reaction of compound **2** with an acetic acid derivative represented by the formula R¹-T-CH₂-COOH also lead to compounds of formula I. Both reactions are described below.

### Synthesis of Carboxylic Acid Starting materials

Carboxylic acids **1** can be prepared by several divergent synthetic routes with the particular route selected relative to the ease of compound preparation, commercial availability of starting materials, whether *m* is zero or one, whether *n* is one or two, etc.

### A. Synthesis of Carboxylic Acids

When *m* is zero and *n* is one, a first synthetic method involves the introduction of the R¹ group to the amino acid NH₂CH(R²)COOH or ester thereof.

The introduction of the R¹ group onto the amino acid NH₂CH(R²)COOH or ester thereof can be accomplished in several method. For example, conventional coupling of a halo acetic acid with a primary amine forms an amino acid as shown in reaction (2) below: 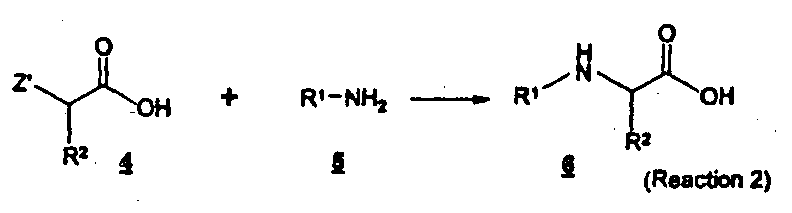 wherein R¹ and R² are as defined above and Z' is a halo group such as chloro for bromo. Alternatively, leaving groups other than halo may be employed such as triflate Additionally, suitable esters of **4** may be employed in this reaction.

As above, reaction (2) involves coupling of a suitable haloacetic acid derivative **4** with a primary amine **5** under conditions which provide for amino acid **6**. This reaction is described by, for example, Yates, et al.¹⁴ and proceeds by combining approximately stoichiometric equivalents of haloacetic acid **4** with primary amine **5** in a suitable inert diluent such as water and dimethylsulfoxide (DMSO), The reaction employs an excess of a suitable base such as sodium bicarbonate, sodium hydroxide, etc. to scavenge the acid generated by the reaction. The reaction is preferably conducted at from about 23°C to about 100°C until reaction completion which typically occurs within 1 to about 24 hours. This reaction is further described in U.S. Patent No. 3,598,859, which is incorporated herein by reference in its entirety. Upon reaction completion, N-substituted amino acid **6** is recovered by conventional methods including precipitation, chromatography and filtration.

In reaction (2), each of the reagents (haloacetic acid **4,** primary amine **5** and alcohol **6**) are well known in the art with a plurality of each being commercially available.

In an alternative embodiment, the R¹ group can be coupled to an alanine ester (or other suitable amino acid ester) by conventional N-arylation. For example, a stoichiometric equivalent or slight excess of the amino acid ester can be dissolved in a suitable diluent such as DMSO and coupled with a halo-R¹ compound, Z'-R¹ where Z' is a halo group such as chloro or bromo and R¹ is as defined above. The reaction is conducted in the presence of an excess of base such as sodium hydroxide to scavenge the acid generated by the reaction. The reaction typically proceeds at from 15°C to about 250°C and is complete in about 1 to 24 hours. Upon reaction completion, N-substituted amino acid ester is recovered by conventional methods including chromatography, filtration and the like. This ester is then hydrolyzed by conventional methods to provide for carboxylic acid **1** for use in reaction (1).

In still another alternative embodiment, the esterified amino acids of formula I above can be prepared by reductive amination of a suitable pyruvate ester in the manner illustrated in reaction (3) below: 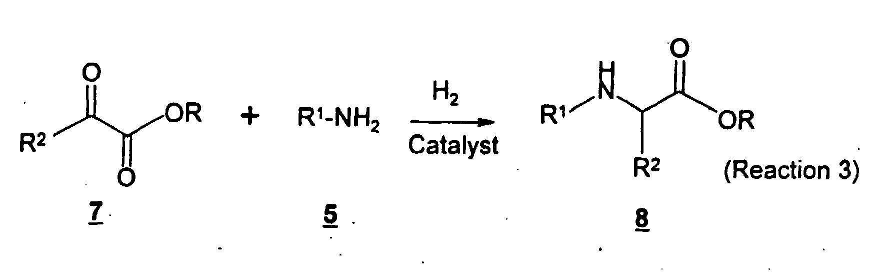 wherein R is typically an alkyl group and R¹ and R² are as defined above.

In reaction (3), approximately stoichiometric equivalents of pyruvate ester **7** and amine **5** are combined in an inert diluent such as methanol, ethanol and the like and the reaction solution treated under conditions which provide for imine formation (not shown). The imine formed is then reduced under conventional conditions by a suitable reducing agent such as sodium cyanoborohydride, H₂/palladium on carbon and the like to form the /N-substituted amino acid ester **8.** In a particularly preferred embodiment, the reducing agent is H₂/palladium on carbon which is incorporated into the initial reaction medium which permits imine reduction *in situ* in a one pot procedure to provide for the N-substituted amino acid ester **8**.

The reaction is preferably conducted at from about 20°C to about 80°C at a pressure of from 1 to 10 atmospheres until reaction completion which typically occurs within 1 to about 24 hours. Upon reaction completion, N-substituted amino acid ester **8** is recovered by conventional methods including chromatography, filtration and the like.

Subsequent hydrolysis of the ester **8** leads to the corresponding carboxylic acid derivative **1** which can be employed in reaction (1) above.

For compounds where *m* is zero and n is two, conventional coupling of a second amino acid (e.g., NH₂CH(R²)C(O)OR where R is typically an alkyl group) to the amino acid produced above (i.e., R¹NHCH(R²)COOH) provides for esters of an analogue of carboxylic acid **1** which are then conventionally deesterified to provide for an analogue of compound **1.**

Alternatively, an ester such as H₂NCH(R²)C(O)NHCH(R²)COOR where each R² is independently as defined above and R is typically an alkyl group can first be formed by conventional peptide synthetic procedures, N-substitution can be conducted in the manner described above followed by de-esterification to provide for analogues of carboxylic acids **1** where *n* is two.

When *m* is one and *n* is one, a first synthetic method involves conventional coupling of an acetic acid derivative with a primary amine of an esterified amino acid as shown in reaction (4) below: 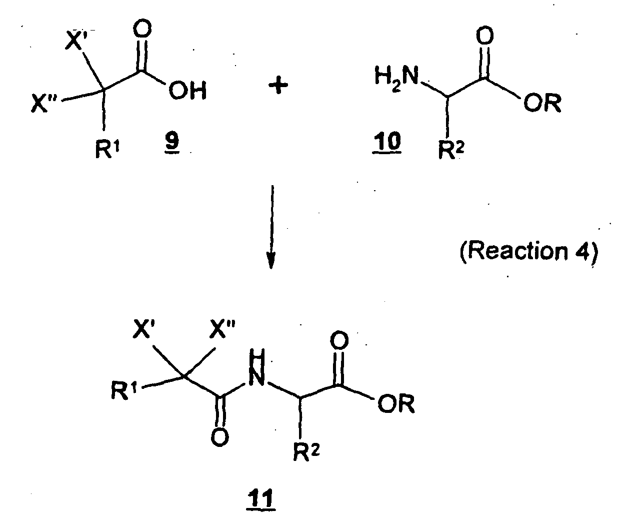 wherein R is typically an alkyl group and R¹, R², X' and X" are as defined above.

Reaction (4) merely involves coupling of a suitable acetic acid derivative **9** with the primary amine of amino acid ester **10** under conditions which provide for the N-acetyl derivative **11.** This reaction is conventionally conducted for peptide synthesis and synthetic methods used therein can also be employed to prepare the N-acetyl amino acid esters **11** of this invention. For example, well known coupling reagents such as carbodiimides with or without the use of well known additives such as N-hydroxysuccinimide, 1-hydroxybenzotriazole, etc. can be used to facilitate coupling. The reaction is conventionally conducted in an inert aprotic polar diluent such as dimethylformamide, dichloromethane, chloroform, acetonitrile, tetrahydrofuran and the like. Alternatively, the acid halide of compound **9** can be employed in reaction (4) and, when so employed, it is typically employed in the presence of a suitable base to scavenge the acid generated during the reaction. Suitable bases include, by way of example, triethylamine, diisopropylethylamine, N-methylmorpholine and the like.

Reaction (4) is preferably conducted at from about 0°C to about 60°C until reaction completion which typically occurs within 1 to about 24 hours. Upon reaction completion, N-acetyl amino acid ester **11** is recovered by conventional methods including precipitation, chromatography, filtration and the like or alternatively is hydrolyzed to the corresponding acid without purification and/or isolation other than conventional work-up (e.g., aqueous extraction, etc.).

In reaction (4), each of the reagents (acetic acid derivative **9** and amino acid ester **10**) are well known in the art with a plurality of each being commercially available.

When *m* is one and *n* is two, a further amino acid ester is coupled to the amino acid ester **11** by first de-esterifying **11** and then using well known peptide coupling chemistry with well known coupling reagents such as carbodiimides with or without the use of well known additives such as N-hydroxysuccinimide, 1-hydroxybenzotriazole, etc. which can be used to facilitate coupling. The reaction is conventionally conducted in an inert aprotic polar diluent such as dimethylformamide, dichloromethane, chloroform, acetonitrile, tetrahydrofuran and the like. De-esterification of the resulting ester provides for carboxylic acids **1** having *n* equal to 2.

Alternatively, carboxylic acids **1** having *n* equal to 2 can be prepared by first forming the ester, N-acetylating these esters and then de-esterifying the resulting product.

Carboxylic acids **1** having *m* equal to 1 and *n* equal to 1 or 2 can also be prepared by use of polymer supported forms of carbodiimide peptide coupling reagents. A polymer supported form of EDC, for example, has been described (Tetrahedron Letters, 34(48), 7685 (1993))¹⁰. Additionally, a new carbodiimide coupling reagent, PEPC, and its corresponding polymer supported forms have been discovered and are very useful for the preparation of such compounds.

Polymers suitable for use in making a polymer supported coupling reagent are either commercially available or may be prepared by methods well known to the artisan skilled in the polymer arts. A suitable polymer must possess pendant sidechains bearing moieties reactive with the terminal amine of the carbodiimide. Such reactive moieties include chloro, bromo, iodo and methanesulfonyl. Preferably, the reactive moiety is a chloromethyl group. Additionally, the polymer's backbone must be inert to both the carbodiimide and reaction conditions under which the ultimate polymer bound coupling reagents will be used.

Certain hydroxymethylated resins may be converted into chloromethylated resins useful for the preparation of polymer supported coupling reagents. Examples of these hydroxylated resins include the 4-hydroxymethylphenylacetamidomethyl resin (Pam Resin) and 4-benzyloxybenzyl alcohol resin (Wang Resin) available from Advanced Chemtech of Louisville, Kentucky, USA (see Advanced Chemtech 1993-1994 catalog, page 115). The hydroxymethyl groups of these resins may be converted into the desired chloromethyl groups by any of a number of methods well known to the skilled artisan.

Preferred resins are the chloromethylated styrene/divinylbenzene resins because of their ready commercial availability. As the name suggests, these resins are already chloromethylated and require no chemical modification prior to use. These resins are commercially known as Merrifield's resins and are available from Aldrich Chemical Company of Milwaukee, Wisconsin, USA (see Aldrich 1994-1995 catalog, page 899). Methods for the preparation of PEPC and its polymer supported forms are outlined in the following scheme.

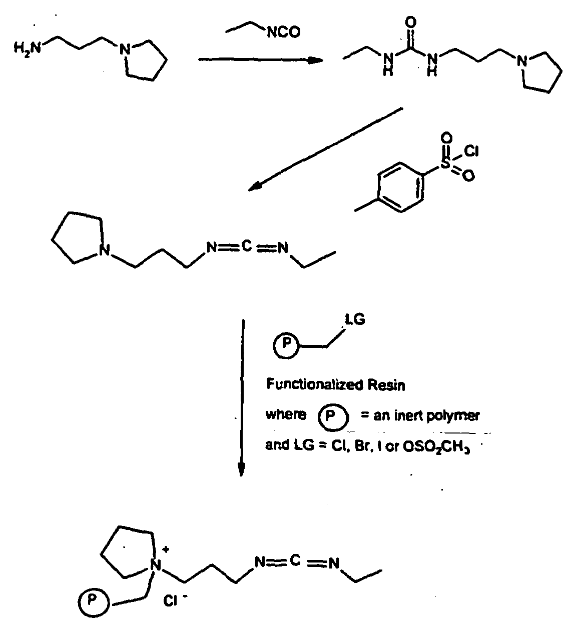

Such methods are described more fully in U.S. Patent Application Serial No. 60/019,790 filed June 14, 1996 which application is incorporated herein by reference in its entirety. Briefly, PEPC is prepared by first reacting ethyl isocyanate with 1-(3-aminopropyl)pyrrolidine. The resulting urea is treated with 4-toluenesulfonyl chloride to provide PEPC. The polymer supported form is prepared by reaction of PEPC with an appropriate resin under standard conditions to give the desired reagent.

The carboxylic acid coupling reactions employing these reagents are performed at about ambient to about 45°C, for from about 3 to 120 hours. Typically, the product may be isolated by washing the reaction with CHCl, and concentrating the remaining organics under reduced pressure. As discussed *supra*, isolation of products from reactions where a polymer bound reagent has been used is greatly simplified, requiring only filtration of the reaction mixture and then concentration of the filtrate under reduced pressure.

### Preparation of Cyclic Amino Compounds

Cyclic amino compounds **2** employed in reactions (1) can be prepared by use or adaptation of known chemical syntheses which syntheses are well described in the literature, See, e.g., Ogliaruso and Wolfe, Synthesis of Lactones and Lactams, Patai, et al. Editor, J. Wiley & Sons, New York, New York, USA, pp. 1085 et seq. (1993)¹⁵.

Specifically, 3-amino substituted lactams **13** with 7 ring atoms may be prepared by the direct cyclization of a suitable alpha, omega-diamino acid ester **12** as shown in reaction (5) below: 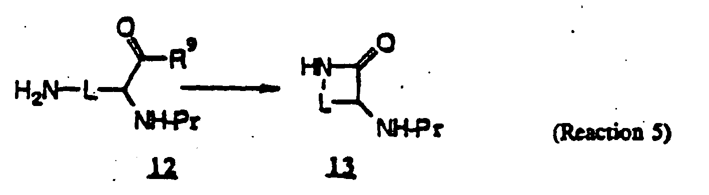 wherein L is a linking group (typically an alkylene group) of -4 atoms, Pr is a suitable protecting group such as *t*-butoxycarbonyl, carbobenzyloxy, or the like and R⁹ is an alkoxy or aryloxy group such as methoxy, ethoxy, *p*-nitrophenoxy, *N*-succinimidoxy, and the like. The reaction may be carried out in a solvent such as water, methanol, ethanol, pyridine, and the like. Such reactions are exemplified by cyclization of a lysine ester to a caprolactam as described by Ugi, et al., Tetrahedron, 52(33):11657-11664 (1996)¹⁶. Alternatively, such a cyclization can also be conducted in the presence of dehydrating agents such as ahrmina or silica to form lactams as described by Blade-Font, Tetrahedron Lett., 21:2443 (1980)¹⁷.

The preparation of aminolactams alkylated on the amino group of the cyclic lactam is described by Freidinger, et al., J. Org. Chem., 47:104-109 (1982)¹⁸ and illustrated in reaction (6) below: 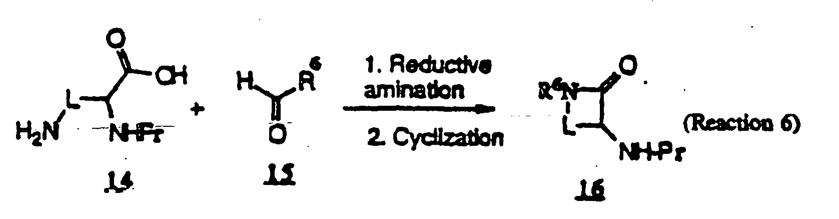 wherein L and R⁶ are as defined above.

In reaction (6), reductive amination of **14** with aldehyde **15** and subsequent ring closure by methods using, for example, EDC provides for aminolactam **16**. The preparation of 6 membered lactams using this general procedure is described by Sample, et al., J. Med. Chem., 39:4531-4536 (1996)¹⁹.

In another methods, lactams **20** can be prepared from cyclic ketones **19** using either the well known Beckmann rearrangement (e.g., Donaruma, et al., Organic Reactions, 11:1-156 (1960))²¹ or the well known Schmidt reaction (Wolff, Organic Reactions, 3:307-336 (1946))²² as shown in reaction (8) below: 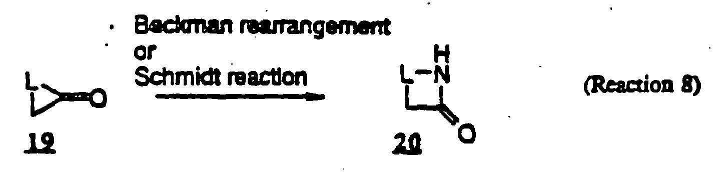 wherein L is as defined above.

Application of these two reactions leads to a wide variety of lactams especially lactams having two hydrogen atoms on the carbon alpha to the lactam carbonyl which lactams form a preferred group of lactams in the synthesis of the compounds of formula I above. In these reactions, the L group can be highly variable including, for example, alkylene, substituted alkylene and hetero containing alkylene with the proviso that a heteroatom is not adjacent to the carbonyl group of compound **19**. Additionally, the Beckmann rearrangement can be applied to bicyclic ketones as described in Krow, et al., J. Org. Chem., 61:5574-5580 (1996)²³.

The preparation of lactones can be similarly conducted using peracids in a Baeyer-Villiger reaction on ketones. Alternatively, thiolactones can be prepared by cyclization of an omega -SH group to a carboxylic acid and thiolactams can be prepared by conversion of the oxo group to the thiooxo group by P₂S₅ or by use of the commercially available Lawesson's Reagent, Tetrahedron, 35:2433 (1979)²⁴.

One recently reported route for lactam synthesis is a variation of the Schmidt reaction through the use of an alkyl azide, either intermolecularly or intramolecularly, through a tethered alkylazide function that attacks a ketone under acidic conditions. Gracias, et al., J. Am. Chem. Soc., 117:8047-8048 (1995)²⁵ describes the intermolecular version whereas Milligan, et al., J. Am. Chem. Soc., 117:10449-10459 (1995)²⁶ describes the intramolecular version. One example of the intramolecular version is illustrated in reaction (9) below: 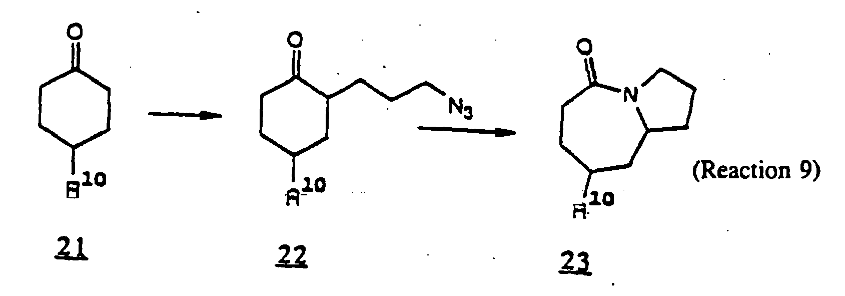 where R¹⁰ is exemplified by alkyl, substituted alkyl, alkoxy, substituted alkoxy, aryl, heteroaryl, cycloalkyl and heterocyclic.

In this reaction, ketone **21** is converted to an α-(ω-alkyl)ketone **22** which is cyclized to form bicyclic lactam **23**. Such intramolecular reactions are useful in forming bicyclic lactams having 5-7 members and the lactam ring of 6-13 members. The use of hetero atoms at non-reactive sites in these rings is feasible in preparing heterobicyclic lactams.

Still another recent approach to the synthesis of lactams is described by Miller, et al., J. Am. Chem. Soc., 118:9606-9614 (1996)²⁷ and references cited and is illustrated in reaction (10) below: 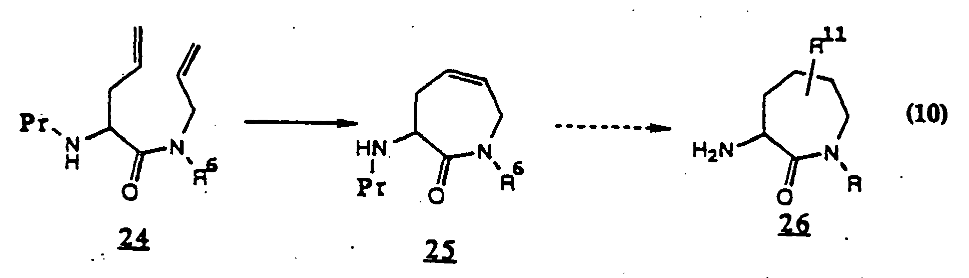 where R⁶ and Pr are as defined above and R¹¹ is exemplified by halo, alkyl, substituted alkyl, alkoxy, substituted alkoxy, aryl, heteroaryl, cycloalkyl and heterocyclic wherein the aryl, heteroaryl, cycloalkyl and heterocyclic group is optionally fused to the lactam ring structure.

Specifically, in reaction (10), lactam **26** is formed from an appropriate unsaturated amide (e.g., **24**) through a ruthenium or molybdenum complexes catalyzed olefin metathesis reaction to form unsaturated lactam **25** which can be used herein without further modification. However, the unsaturation in **25** permits a myriad of techniques such as hydroboration, Sharpless or Jacobsen epoxidations, Sharpless dihydroxylations, Diels-Alder additions, dipolar cycloaddition reactions and many more chemistries to provide for a wide range of substituents on the lactam ring. Moreover, subsequent transformations of the formed substitution leads to other additional substituents (e.g., mesylation of an alcohol followed by nucleophilic substitution reactions). See, for example, March, et al. for a recitation of numerous such possible reactions.²⁸ Saturated amides used in this reaction are conventional with amide **24** being commercially available.

Related chemistry to cyclize amides to form lactams is disclosed by Colombo, et al., Tetrahedron Lett., 35(23):4031-4034 (1994)²⁹ and is illustrated in reaction (11) below: 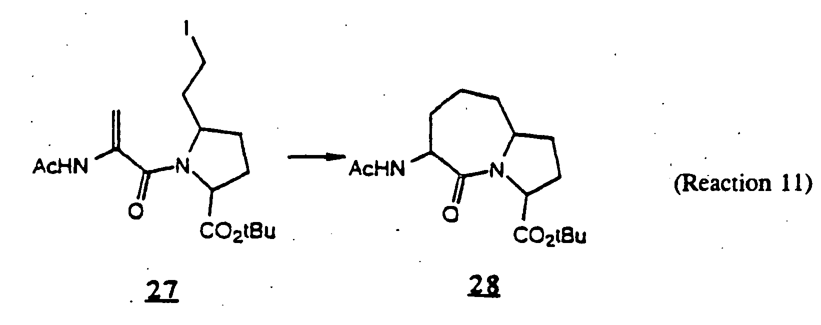 In this reaction, proline derivative **27** is cyclized via a tributyltin-radical cyclization to provide for lactam **28**.

Some of the lactams described above contain the requisite amino group alpha to the lactam carbonyl whereas others did not. However, the introduction of the required amino group can be achieved by any of several routes delineated below which merely catalogue several recent literature references for this synthesis.

For example, in a first general synthetic procedure, azide or amine displacement of a leaving group alpha to the carbonyl group of the lactam leads to the alpha-aminolactams. Such general synthetic procedures are exemplified by the introduction of a halogen atom followed by displacement with phthalimide anion or azide and subsequent conversion to the amine typically by hydrogenation for the azide as described in Rogriguez, et al., Tetrahedron, 52:7727-7736 (1996)³⁰, Parsons, et al., Biochem. Biophys. Res. Comm., 117:108-113 (1983)³¹ and Watthey, et al., J. Med. Chem., 28:1511-1516 (1985)³². One particular method involves iodination and azide displacement on, for example, benzyllactams as described by Armstrong, et al., Tetrahedron Lett., 35:3239 (1994)³³ and by King, et al., J. Org. Chem., 58:3384 (1993)³⁴.

Another example of this first general procedure for the synthesis of alpha-aminolactams from the corresponding lactam involves displacement of a triflate group by an azido group as described by Hu, et al., Tetrahedron Lett., 36(21):3659-3662 (1995)³⁵.

Still another example of this first general procedure uses a Mitsunobu reaction of an alcohol and a nitrogen equivalent (either -NH₂ or a phthalimido group) in the presence of an azodicarboxylate and a triarylphosphine as described in Wada, et al., Bull. Chem. Soc. Japan, 46:2833-2835 (1973)³⁶ using an open chain reagent.

Yet another example of this first general procedure involves reaction of alpha-chlorolactams with anilines or alkyl amines in a neat mixture at 120°C to provide for 2-(N-aryl or N-alkyl)lactams as described by Gaetzi, Chem. Abs., 66:28690m.³⁷

In a second general synthetic procedure, reaction of an enolate with an alkyl nitrite ester to prepare the alpha oxime followed by reduction yields the alpha-aminolactam compound. This general synthetic procedure is exemplified by Wheeler, et al., Organic Syntheses, Coll. Vol. VI, p. 840³⁸ which describes the reaction of isoamyl nitrite with a ketone to prepare the desired oxime. The reduction of the oxime methyl ester (prepared from the oxime by reaction with methyl iodide) is described in the J. Med. Chem., 28(12):1886 (1985)³⁹ and the reduction of alpha-oximino caprolactams by Raney-nickel and palladium catalysts is described by Brenner, et al., U.S. Patent No. 2,938,029.⁴⁰

In a third general synthetic procedure, direct reaction of an enolate with an electrophilic nitrogen transfer agent can be used. The original reaction employed toluenesulfonyl azide but was improved as described by Evans, et al., J. Am. Chem. Soc., 112:4011-4030 (1990)⁴¹. Specifically, direct introduction of an azido group which can be reduced to the amine by hydrogenation is described by Micouin, et al., Tetrahedron, 52:7719-7726 (1996)⁴². Likewise, the use of triisopropylbenzenesulfonyl azide as the azide transferring agent for reaction with an enolate is described by Evans, et al., *supra*. The use of triphenylphosphine to reduce the alpha-azidolactams to the corresponding aminolactams in the benzodiazepine series is disclosed by Butcher, et al., Tetrahedron Lett., 37(37):6685-6688 (1996).⁴³ Lastly, diazo transfer of beta-diketones and subsequent reduction of the diazo group to the amino group is exemplified by Hu, et al., Tetrahedron Lett., 36(21):3659-3662 (1995)³⁵ who used Raney-nickel and hydrogen in acetic acid and acetic anhydride as the solvent.

In a fourth general procedure, N-substituted lactams are first converted to the 3-alkoxycarbonyl derivatives by reaction with a dialkyl carbonate and a base such as sodium hydride. See, for example, M.L. Reupple, et al., J. Am. Chem. Soc., 93:7021 et seq. (1971)⁴⁴ The resulting esters serve as starting materials for conversion to the 3-amino derivatives. This conversion is achieved via the Curtius reaction as shown in reaction (12) below: 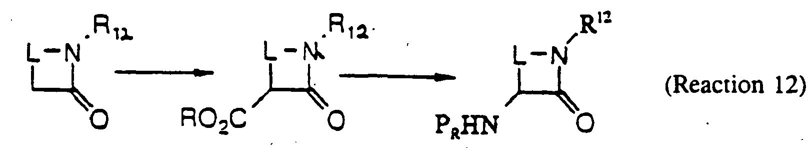 where Pr is as defined above and R¹² is typically hydrogen, an alkyl or an aryl group.

The Curtius reaction is described by P.A.S. Smith, Organic Reactions, 3:337-449 (1946).⁴⁵ Depending on the reaction conditions chosen, Pr = H or a protecting group such as Boc. For example, when R = H, treatment of the acid with diphenylphosphoryl azide in the presence of t-butanol provides the product wherein Pr = Boc.

The alpha-aminolactams employed as the cyclic amino compounds **2** in reaction (1) above include ring N-substituted lactams in addition to ring N-H lactams. Some methods for preparing ring N-substituted lactams have been described above. More generally, however, the preparation of these compounds range from the direct introduction of the substituent after lactam formation to essentially introduction before lactam formation. The former methods typically employ a base and an primary alkyl halide although it is contemplated that a secondary alkyl halide can also be employed although yields may suffer.

Accordingly, a first general method for preparing N-substituted lactams is achieved via reaction of the lactam with base and alkyl halide (or acrylates in some cases). This reaction is quite well known and bases such as sodamide, sodium hydride, LDA, LiHMDS in appropriate solvents such as THF, DMF, etc. are employed provided that the selected base is compatible with the solvent. See for example: K. Orito, et al., Tetrahedron. 36:1017-1021 (1980)⁴⁶ and J.E. Semple, et al., J. Med. Chem., 39:4531-4536 (1996)¹⁹ (use of LiHMDS with either R-X or acrylates as electrophiles).

A second general method employs reductive amination on an amino function which is then cyclized to an appropriate ester or other carbonyl function.

A third general method achieves production of the N-substitution during lactam formation. Literature citations report such production from either photolytic or thermal rearrangement of oxaziridines, particularly of N-aryl compounds. See, for example, Krimm. Chem. Ber., 91:1057 (1958)⁴⁷ and Suda, et al.. J. Chem. Soc. Chem Comm. 949-950, (1994).⁴⁹ Also, the use of methyl hydroxylamine for the formation of nitrones and their rearrangement to the N-methyl derivatives is reported by Barton, et al.. J. Chem. Soc., 1764-1767 (1975).⁴⁹ Additionally, the use of the oxaziridine process in chiral synthesis has been reported by Kitagawa, et al.. J. Am. Chem. Soc., 117:5169-5178 (1975).⁵⁰

Additional references for the synthesis of alpha aminolactams are as follows:
5. The synthesis of benzolactams (benzazepinones) has been reported by Busacca, et al., Tet. Lett., 33:165-168 (1992)⁶⁴: 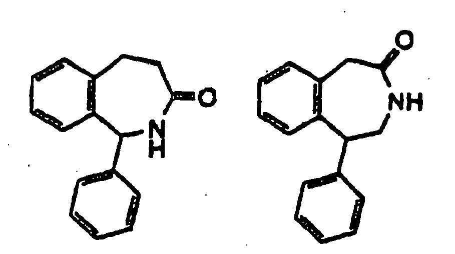 by Croisier, et al., U.S. Patent No. 4,080,449⁴⁶: 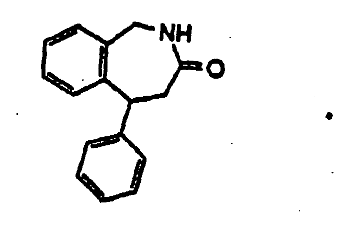
6. Orito, et al., Tetrahedron, 36:1017-1021 (1980)⁶⁸ discloses phenyl substituted benzazepinones represented by the formula: 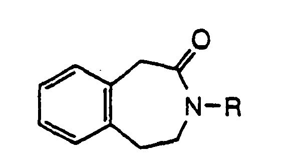 wherein R = H or CH₃-;
   Kawase, et al.; J. Org. Chem.; 54:3394-3403 (1989)⁶⁹ discloses a N-methoxy benzazepinone represented by the formula: 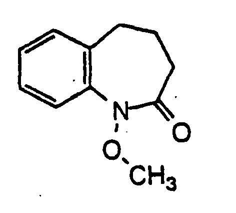
7. Lowe, et al. , J. Med. Chem., 37:3789-3811 (1994)⁷⁰ describes several synthetic pathways to substituted benzazepinones of the formula: 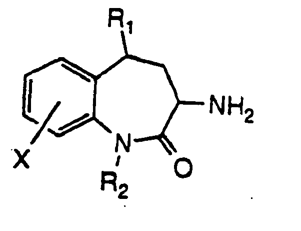 where R₁ is substituted aryl or cyclohexyl, X is a suitable substituent and R₂ can be H or alkyl. The syntheses described in Lowe are, however, adaptable to form numerous R¹ substituents.
8. Robl, et al., Bioorg. Med. Chem. Lett., 4:1789-1794 (1994)⁷¹ and references cited therein as well as Skiles, et al., Bioorg. Med. Chem. Lett., 3:773-778 (1993)⁷² disclose benzofused lactams which contain additional heteroatoms in the lactam ring. These compounds are represented by the formula: 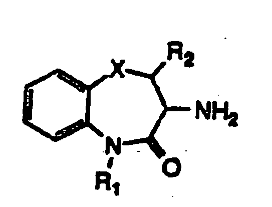 where X is O and R₂ = H or CH, or X = S and R₂ = H. In either case, R₁ = H or alkyl. Also, in Skiles, the thio group of the thiolactam can be oxidized to the SO₂ group. These structures are also presented from Beckmann rearrangement in Grunewald, et al., J. Med. Chem., 39(18):3539 (1996),⁷³

As noted above, the primary approaches to the preparation of lactams is the Beckmann/Schmidt ring expansion reaction using either inter - or intramolecular approaches serves to prepare lactams of various ring sizes. The intramolecular approach generates bicyclic materials with the lactam nitrogen incorporated into the ring fusion. Additional approaches set forth above are at the base of the methodology are internal cyclization of omega-amino acida/esters where the construction of the substituent pattern takes place prior to cyclization, and internal cyclization of an electrophilic center onto a nucleophilic functional group as in the Friedel Crafts type cyclization at the center of the Ben-Ishal procedure for making benzazepinones. This latter procedure is applicable to a wide variety of heteroaromatics as well as benzenoid rings, and may also be applied to non-aromatic double or triple bonds to generate a wide array of substituents or ring fusions.

Deoxygenation of the lactam by reagents such as diborane, LiAlH₄, and the like leads to azaheterocycles (=X is dihydro).

Additionally, the 5,7-dihydro-6H-diben[b,d]azepin-6-one derivatives employed in this invention can be prepared using conventional procedures and reagents. For example, an appropriately substituted N-*tert*-Boc-2-amino-2-amino-2'-methylbiphenyl compound can be cyclized to form the corresponding 5,7-dihydro-6H-diben[b,d]azepin-6-one derivative by first treating the biphenyl compound with about 2.1 to about 2.5 equivalents of a strong base, such as sec-butyl lithium. This reaction is typically conducted at a temperature ranging from about -80°C to about -60°C in an inert diluent such as THF. The resulting dianion is then treated with dry carbon dioxide at a temperature of about -78°C to afford the 5,7-dihydro-6H-diben[b,d]azepin-6-one. This procedure is described further in R.D. Clark et al., Tetrahedron, 49(7). 1351-1356(1993) and references cited therein.

After forming the 5,7-dihydro-6H-diben[b,d]azepio-6-one, the amide nitrogen can be readily alkylated by first treating the dibenazepinone with about 1.1 to about 1.5 equivalents of a strong base, such as sodium hydride, in an inert diluent, such as DMF. This reaction is typically conducted at a temperature ranging from about -10°C to about 80°C for about 0.5 to about 6 hours. The resulting anion is then contacted with an excess, preferably about 1.1 to about 3.0 equivalents, of an alkyl halide, typically an alkyl chloride, bromide or iodide. Generally, this reaction is conducted at a temperature of about 0°C to about 100°C for about 1 to about 48 hours.

An amino group can then be introduced at the 5-position of the 7-alkyl-5,7-dihydro-6H-diben[b,d]azepin-6-one using conventional procedures and reagents. For example, treatment of 7-methyl-5,7-dihydro-6H-diben[b,d]azepin-6-one with an excess of butyl nitrite in the presence of a strong base, such as potassium 1,1,1,3,3,3-hexamethyldisilazane (KHMDS), affords 5-oximo-7-methyl-5,7-dihydro-6H-diben[b,d]azepin-6-one. Subsequent reduction of the oximo group by hydrogenation in the presence of a catalyst, such as palladium on carbon, then provides 5-amino-7-methyl-5,7-dihydro-6H-diben[b,d]azepin-6-one. Other conventional amination procedures, such as azide transfer followed by reduction of the azido group, may also be employed.

Similarly, various benzodiazepine derivatives suitable for use in this invention can be prepared using conventional procedures and reagents. For example, a 2-aminobenzophenone can be readily coupled to α-(isopropylthio)-N-(benzyloxycarbonyl)glycine by first forming the acid chloride of the glycine derivative with oxayl chloride, and then coupling the acid chloride with the 2-aminobenzophenone in the presence of a base, such as 4-methylmorpholine, to afford the 2-[α-(isopropylthio)-N-(benzyloxycarbonyl)glycinyl]-aminobenzophenone. Treatment of this compound with ammonia gas in the presence of an excess, preferably about 1.1 to about 1.5 equivalents, of mercury (II) chloride then affords the 2-[N-(α-amino)-N'-(benzyloxycarbonyl)-glycinyl]aminobenzophenone. This intermediate can then be readily cyclized by treatment with glacial acetic acid and ammonium acetate to provide the 3-(benzyloxycarbonyl)amino-2,3-dihydro-5-phenyl-1H-1,4-benzodiazepin-2-one1. Subsequent removal of the Cbz group affords the 3-amino-2,3-dihydro-5-phenyl-1H-1,4-benzodiazepin-2-one.

Alternatively, 2,3-dihydro-5-phenyl-1H-1,4-benzodiazepin-2-ones can be readily aminated at the 3-position using conventional azide transfer reactions followed by reduction of the resulting azido group to form the corresponding amino group. The conditions for these and related reactions are described in the examples set forth below. Additionally, 2,3-dihydro-5-phenyl-1H-1,4-benzodiazepin-2-ones are readily alkylated at the 1-position using conventional procedures and reagents. For example, this reaction is typically conducted by first treating the benzodiazepinone with about 1.1 to about 1.5 equivalents of a base, such as sodium hydride, potassium *tert*-butoxide, potassium 1,1,1,3,3,3-hexamethyldisilazane, cesium carbonate, in an inert diluent, such as DMF. This reaction is typically conducted at a temperature ranging from about -78°C to about 80°C for about 0.5 to about 6 hours. The resulting anion is then contacted with an excess, preferably about 1.1 to about 3.0 equivalents, of an alkyl halide, typically an alkyl chloride, bromide or iodide. Generally, this reaction is conducted at a temperature of about 0°C to about 100°C for about 1 to about 48 hours.

Additionally, the 3-amino-2,4-dioxo-2,3,4,5-tetrahydro-1H-1,5-benzodiazepines employed in this invention are typically prepared by first coupling malonic acid with a 1,2-phenylenediamine. Conditions for this reaction are well known in the art and are described, for example, in PCT Application WO 96-US8400 960603. Subsequent alkylation and amination using conventional procedures and reagents affords various 3-amino-1,5-bis(alkyl)-2,4-dioxo-2,3,4,5-tetrahydro-1H-1,5-benzodiazepines. Such procedures are described in further detail in the example set forth below.

In the synthesis of compounds of formula 1 using the synthetic methods described above, the starting materials can contain a chiral center (e.g., alanine) and, when a racemic starting material is employed, the resulting product is a mixture of R,S enantiomers. Alternatively, a chiral isomer of the starting material can be employed and, if the reaction protocol employed does not racemize this starting material, a chiral product is obtained. Such reaction protocols can involve inversion of the chiral center during synthesis.

Accordingly, unless otherwise indicated, the products of this invention are a mixture of R,S enantiomers. Preferably, however, when a chiral product is desired, the chiral product corresponds to the L-amino acid derivative. Alternatively, chiral products can be obtained via purification techniques which separates enantiomers from a R,S mixture to provide for one or the other stereoisomer. Such techniques are well known in the art.

### Pharmaceutical Formulations

When employed as pharmaceuticals, the compounds according to any one of claims 1 to 4 are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

This invention also includes pharmaceutical compositions which contain, as the active ingredient, one or more of the compounds of formula I above associated with pharmaceutically acceptable carriers. In making the compositions of this invention, the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 5 to about 100 mg, more usually about 10 to about 30 mg, of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Preferably, the compound of formula I above is employed at no more than about 20 weight percent of the pharmaceutical composition, more preferably no more than about 15 weight percent, with the balance being pharmaceutically inert carrier(s).

The active compound is effective over a wide dosage range and is generally administered in a pharmaceutically effective amount. It, will be understood, however, that the amount of the compound actually administered will be determined by a physician, in the light of the relevant circumstances, including the condition to be treated, the chosen route of administration, the actual compound administered, the age, weight, and response of the individual patient and the severity of the patient's symptoms.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can separated by enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra*. Preferably the compositions are administered by the oral or nasal respiratory route for local or systemic effect. Compositions in preferably pharmaceutically acceptable solvents may be nebulized by use of inert gases. Nebulized solutions may be breathed directly from the nebulizing device or the nebulizing device may be attached to a face masks tent, or intermittent positive pressure breathing machine. Solution, suspension, or powder compositions may be administered, preferably orally or nasally, from devices which deliver the formulation in an appropriate manner.

The following formulation examples illustrate the pharmaceutical compositions of the present invention.

### Formulation Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

### Formulation Example 2

A tablet formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

### Formulation Example 3

A dry powder inhaler formulation is prepared containing the following components:

| Ingredient | Weight % |
|---|---|
| Active Ingredient | 5 |
| Lactose | 95 |

The active, ingredient is mixed with the lactose and the mixture is added to a dry powder inhaling appliance.

### Formulation Example 4

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose Polyvinylpyrrolidone (as 10% solution in sterile water) | 35.0 mg 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinyl-pyrrolidone is mixed with the resultant powders, which are then passed through a 16 mesh U.S. sieve. The granules so produced are dried at 50° to 60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 150 mg.

### Formulation Example 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, starch, and magnesium stearate are blended, passed through a No. 20 mesh U.S. sieve, and filled into hard gelatin capsules in 150 mg quantities.

### Formulation Example 6

Suppositories, each containing 25 mg of active ingredient are made as follows:

| Ingredient | Amount |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

### Formulation Example 7

Suspensions, each containing 50 mg of medicament per 5.0 ml dose are made as follows:

| Ingredient | Amount |
|---|---|
| Active Ingredient | 50.0 mg |
| Xanthan gum | 4.0 mg |
| Sodium carboxymethyl cellulose (11 %) Microcrystalline cellulose (89%) | 50.0 mg |
| Sucrose | 1.75 g |
| Sodium benzoate | 10.0 mg |
| Flavor and Color | q.v. |
| Purified water to | 5.0 ml |

The active ingredient, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

### Formulation Example 8

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, starch, and magnesium stearate are blended, passed through a No. 20 mesh U.S. sieve, and filled into hard gelatin capsules in 560 mg quantities.

### Formulation Example 9

A subcutaneous formulation may be prepared as follows:

| Ingredient | Quantity |
|---|---|
| Active Ingredient | 1.0 mg |
| corn oil | 1 ml |

(Depending on the solubility of the active ingredient in corn oil, up to about 5.0 mg or more of the active ingredient may be employed in this formulation, if desired).

### Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Patent 5,023,252, issued June 11, 1991, herein incorporated by reference. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system used for the transport of biological factors to specific anatomical regions of the body is described in U.S. Patent 5,011,472 which is herein incorporated by reference.

Indirect techniques, which are generally preferred, usually involve formulating the compositions to provide for drug latentiation by the conversion of hydrophilic drugs into lipid-soluble drugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

Other suitable formulations for use in the present invention can be found in Remington's Pharmaceutical Sciences, Mace Publishing Company, Philadelphia, PA, 17th ed. (1985).

### Utility

The compounds and pharmaceutical compositions of the invention are useful in inhibiting β-amyloid peptide release and/or its synthesis, and, accordingly, have utility in diagnosing and treating Alzheimer's disease in mammals including humans.

As noted above, the compounds described herein are suitable for use in a variety of drug delivery systems described above. Additionally, in order to enhance the *in vivo* serum half-life of the administered compound, the compounds may be encapsulated, introduced into the lumen of liposomes, prepared as a colloid, or other conventional techniques may be employed which provide an extended serum half-life of the compounds. A variety of methods are available for preparing liposomes, as described in, e.g., Szoka, et al., U.S. Patent Nos. 4,235,871, 4,501,728 and 4,837,028 each of which is incorporated herein by reference.

The amount of compound administered to the patient will vary depending upon what is being administered, the purpose of the administration, such as prophylaxis or therapy, the state of the patient, the manner of administration, and the like. In therapeutic applications, compositions are administered to a patient already suffering from AD in an amount sufficient to at least partially arrest further onset of the symptoms of the disease and its complications. An amount adequate to accomplish this is defined as "therapeutically effective dose." Amounts effective for this use will depend on the judgment of the attending clinician depending upon factors such as the degree or severity of AD in the patient, the age, weight and general condition of the patient, and the like. Preferably, for use as therapeutics, the compounds described herein are administered at dosages ranging from about 1 to about 500 mg/kg/day.

In prophylactic applications, compositions are administered to a patient at risk of developing AD (determined for example by genetic screening or familial trait) in an amount sufficient to inhibit the onset of symptoms of the disease. An amount adequate to accomplish this is defined as "prophylactically effective dose. " Amounts effective for this use will depend on the judgment of the attending clinician depending upon factors such as the age, weight and general condition of the patient, and the like. Preferably, for use as prophylactics, the compounds described herein are administered at dosages ranging from about 1 to about 500 mg/kg/day.

As noted above, the compounds administered to a patient are in the form of pharmaceutical compositions described above. These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the compound preparations typically will be between 3 and 11, more preferably from 5 to 9 and most preferably from 7 and 8. It will be understood that use of certain of the foregoing excipients, carriers, or stabilizers will result in the formation of pharmaceutical salts.

The compounds described herein are also suitable for use in the administration of the compounds to a cell for diagnostic and drug discovery purposes. Specifically, the compounds may be used in the diagnosis of cells releasing and/or synthesizing β-amyloid peptide. In addition the compounds described herein are useful for the measurement and evaluation of the activity of other candidate drugs on the inhibition of the cellular release and/or synthesis of β-amyloid peptide.

The following synthetic and biological examples are offered to illustrate this invention and are not to be construed in any way as limiting the scope of this invention.

### EXAMPLES

In the examples below, the following abbreviations have the following meanings. If an abbreviation is not defined, it has its generally accepted meaning.
- BEMP =: 2-tert-butylimino-2-diethylamino-1,3-dimethylperhydro-1,3,2-diazaphosphorine
- Boc =: *t*-butoxycarbonyl
- BOP =: benzotriazol-1-yloxy-tris(dimethylamino)phosphonium hexafluorophosphate
- bd =: broad doublet
- bs =: broad singlet
- d =: doublet
- dd =: doublet of doublets
- DIC =: diisopropylcarbodiimide
- DMF =: dimethylformamide
- DMAP =: dimethylaminopyridine
- DMSO =: dimethylsulfoxide
- EDC =: ethyl-1-(3-dimethyaminopropyl)carbodiimide
- eq. =: equivalents
- EtOAc =: ethyl acetate
- g =: grams
- HOBT =: 1-hydroxybenzotriazole hydrate
- Hunig's base =: diisopropylethylamine
- L =: liter
- m =: multiplet
- M **=**: molar
- max =: maximum
- meq =: milliequivalent
- mg =: milligram
- mL =: milliliter
- mm =: millimeter
- mmol =: millimole
- MOC =: methoxyoxycarbonyl
- N =: normal
- N/A =: not available
- ng =: nanogram
- nm =: nanometers
- OD =: optical density
- PEPC =: 1-(3-(1-pyrrolidinyl)propyl)-3-ethylcarbodiimide
- PP-HOBT =: piperidine-piperidine-1-hydroxybenzotrizole
- psi =: pounds per square inch
- φ =: phenyl
- q =: quartet
- quint. =: quintet
- rpm =: rotations per minute
- s =: singlet
- t =: triplet
- TFA =: trifluoroacetic acid
- THF =: tetrahydrofuran
- tlc =: thin layer chromatography
- µL =: microliter
- UV =: ultra-violet

In the examples below, all temperatures are in degrees Celcius (unless otherwise indicated). The compounds set forth in the examples below were prepared using the following general procedures as indicated.

In the following examples and procedures, the term "Aldrich" indicates that the compound or reagent used in the procedure is commercially available from Aldrich Chemical Company, Inc., 1001 West Saint Paul Avenue, Milwaukee, WI 53233 USA; the term "Fluka" indicates that the compound or reagent is commercially available from Fluka Chemical Corp., 980 South 2nd Street, Ronkonkoma NY 11779 USA; the term "Lancaster" indicates that the compound or reagent is commercially available from Lancaster Synthesis, Inc., P.O. Box 100 Windham, NH 03087 USA; the term "Sigma" indicates that the compound or reagent is commercially available from Sigma, P.O. Box 14508, St. Louis MO 63178 USA; the term "Chemservice" indicates that the compound or reagent is commercially available from Chemservice Inc., Westchester, PA; the term "Bachem" indicates that the compound or reagent is commercially available from Bachem Biosciences Inc., 3700 Horizon Drive, Renaissance at Gulph Mills, King of Prussia, PA 19406 USA; the term "Maybridge" indicates that the compound or reagent is commercially available from Maybridge Chemical Co. Trevillett, Tintagel, Cornwall PL34 OHW United Kingdom; and the term "TCI" indicates that the compound or reagent is commercially available from TCI America, 9211 North Harborgate Street, Portland OR 97203; the term "Alfa" indicates that the compound or reagent is commercially available from Johnson Matthey Catalog Company, Inc. 30 Bond Street, Ward Hill, MA 01835-0747; the term "Novabiochem" indicates that the compound or reagent is commercially available from Calbiochem-Novabiochem Corp. 10933 North Torrey Pines Road, P.O. Box 12087, La Jolla CA 92039-2087; the term "Oakwood" indicates that the compound or reagent is commercially available from Oakwood, Columbia, South Carolina; the term "Advanced Chemtech" indicates that the compound or reagent is commercially available from Advanced Chemtech, Louisville, KY; and the term "Pfaltz & Bauer" indicates that the compound or reagent is commercially available from Pfaltz & Bauer, Waterbury, CT, USA.

### I. Coupling Procedures

### GENERAL PROCEDURE A

### First EDC Coupling Procedure

To a 1:1 mixture of the corresponding carboxylic acid and the corresponding amino acid ester or amide in CH₂Cl₂ at O°C was added 1.5 equivalents triethylamine, followed by 2.0 equivalents hydroxybenzotriazole monohydrate and then 1.25 equivalents of ethyl-3-(3-dimethylamino)propyl carbodiimide HCl. The reaction mixture was stirred overnight at room temperature and then transferred to a separatory funnel. The mixture was washed with water, saturated aqueous NaHCO₃, 1N HCl and saturated aqueous NaCl, and then dried over MgSO₄. The resulting solution was stripped free of solvent on a rotary evaporator to yield the crude product.

### GENERAL PROCEDURE B

### Second EDC Coupling Procedure

A mixture of the corresponding acid (1 eqv), N-1-hydroxybenzotriazole (1.6 eqv), the corresponding amine (1 eqv), N-methylmorpholine ( 3 eqv) and dichloromethane (or DMF for insoluble substrates) was cooled in an ice-water bath and stirred until a clear solution was obtained. EDC (1.3 eqv) was then added to the reaction mixture. The cooling bath was then allowed to warm to ambient temperature over 1-2 h and the reaction mixture was stirred overnight. The reaction mixture was then evaporated to dryness under vacuum. To the residue was added 20% aqueous potassium carbonate and the mixture was shaken throughly and then allowed to stand until the oily product solidified (overnight if necessary). The solid product was then collected by filteration, washed thoroughly with 20% aqueous potassium carbonate, water, 10% HCl, and water to give the product, usually in pure state. No racemization was observed.

### GENERAL PROCEDURE C

### Third EDC Coupling Procedure

The carboxylic acid was dissolved in methylene chloride. The corresponding amino acid ester or amide (1 eq.), N-methylmorpholine (5 eq.) and hydroxybenzotriazole monohydrate (1.2 eq.) were added in sequence. A cooling bath was applied to the round bottomed flask until the solution reached 0°C. At that time, 1.2 eq. of 1-(3-dimethylaminopropyl)-3-ethylcarbodiimide hydrochloride was added. The solution was allowed to stir overnight and come to room temperature under nitrogen pressure. The reaction mixture was worked up by washing the organic phase with saturated aqueous sodium carbonate, 0.1M citric acid, and brine before drying with sodium sulfate. The solvents were then removed to yield crude product.

### GENERAL PROCEDURE D

### Fourth EDC Coupling Procedure

A round bottom flask was charged with the corresponding carboxylic acid (1.0 eq.), hydroxybenzotriazole hydrate (1.1 eq.) and the corresponding amine (1.0 eq.) in THF under nitrogen atmosphere. An appropriate amount (1.1 eq for free amines and 2.2 eq. for hydrochloride amine salts) of base, such as Hunig's base was added to the well stirred mixture followed by EDC (1.1 eq.). After stirring from 4 to 17 hours at room temperature the solvent was removed at reduced pressure, the residue taken up in ethyl acetate (or similar solvent) and water, washed with saturated aqueous sodium bicarbonate solution, 1 N HCl, brine, dried over anhydrous sodium sulfate and the solvent removed at reduced pressure to provide the product.

### GENERAL PROCEDURE E

### BOP Coupling Procedure

To a stirred solution of *N*-(3,5-difluorophenylacetyl)alanine (2 mmol) in . DMF, cooled in an ice-water bath, was added BOP (2.4 mmol) and N-methylmorpholine (6 mmol). The reaction mixture was stirred for 50 min. and then a solution of α-amino-γ-lactam (2 mmol) in DMF cooled at 0 °C was added. The cooling bath was allowed to warm to ambient temperature over 1-2 h and the reaction mixture was then stirred overnight. A 20% aqueous potassium carbonate solution (60 mL) was added and this mixture shaken throughly. No solid formed. The mixture was then washed with ethyl acetate (150 mL) and evaporated to dryness under vacuum to give a white solid. Water (50 mL) was then added and this mixture shaken throughly. The precipitate that formed was collected by filtration, then washed thoroughly with water, followed by 1 mL of diethyl ether to give the product (51 mg, 0.16 mmol, 7.8%).

### GENERAL PROCEDURE F

### Coupling of an Acid Chloride with an Amino Acid Ester

To a stirred solution of (D,L)-alanine isobutyl ester hydrochloride (4.6 mmol) in 5 ml of pyridine was added 4.6 mmol of the acid chloride. Precipitation occurred immediately. The mixture was stirred for 3.5 h, dissolved in 100 mL of diethyl ether, washed with 10% HCl three times, brine once, 20% potassium carbonate once and brine once. The solution was dried over magnesium sulfate, filtered, and evaporated to yield the product. Other amino acid esters may also be employed in this procedure.

### GENERAL PROCEDURE G

### Coupling of a Carboxylic Acid with an Amino Acid Ester

A solution of the carboxylic acid (3.3 mmol) and 1,1'-carbodiimidazole (CDI) in 20 mL THF was stirred for 2 h. (D,L)-alanine isobutyl ester hydrochloride (3.6 mmol) was added, followed by 1.5 mL (10.8 mmol) of triethylamine. The reaction mixture was stirred overnight. The reaction mixture was dissolved in 100 mL of diethyl ether, washed with 10% HCl three times, brine once, 20% potassium carbonate once and brine once. The solution was dried over magnesium sulfate, filtered, and evaporated to yield the product. Other amino acid esters may also be employed in this procedure.

### GENERAL PROCEDURE H

### Fifth EDC Coupling Procedure

In a round bottom flask was added a carboxylic acid (1.1 eq.) in THF, an amine hydrochloride (1.0 eq.), 1-hydroxybenzotriazole hydrate (1.1 eq.), N,N-diisopropylethylamine (2.1 eq.), followed by 1-(3-dimethylaminopropyl)-3-ethylcarbodiimide hydrochloride (EDC) (1.1 eq.). The reaction mixture stirred at room temperature for 10-20 hours under an atmosphere of nitrogen. The mixture was diluted with EtOAc and washed with 0.1 M HCl (1 x 10 mL), saturated NaHCO₃ (1 x 10 mL), H₂O (1 x 10 mL), and brine and dried over MgSO₄. The drying agent was removed by filtration and the filtrate was concentrated *in vacuo.* The residue was purified by flash column chromatography on silica gel followed by trituration from EtOAc and hexanes.

### GENERAL PROCEDURE I

### Sixth EDC Coupling Procedure

To a solution or suspension of the amine or amine hydrochloride (1.0 eq.) in THF (0.05-0.1 M) under N₂ at 0°C was added the carboxylic acid (1.0-1.1 eq.), hydroxybenzotriazole monohydrate (1.1-1.15 eq.), Hunig's base (1.1 eq. for free amines and 1.1-2.3 eq. for hydrochloride amine salts), followed by 1-(3-dimethylaminopropyl)-3-ethylcarbodiimide hydrochloride (1.1-1.15 eq.). The cooling bath was removed and the mixture allowed to warm to room temperature for 10-24 hours. The solution or mixture was diluted with EtOAc, in a 3-5 volume multiple of the initial THF volume, and washed with 0.1-1.0 M aq. HCl (1 or 2x), dilute NaHCO₃ (1 or 2x), and brine (1x). Then, the organic phase was dried over either MgSO₄ or Na₂SO₄, filtered, concentrated to provide the crude product, which was either further purified or utilized without further purification.

### GENERAL PROCEDURE J

### EEDO Coupling Procedure

To a solution of the amine in THF (1.0 eq., 0.05-0.08 M, final molarity) under N₂ at room temperature was added the N-t-Boc protected amino acid (1.1 eq., either as a solid or in THF via cannula), followed by EEDQ (Aldrich, 1.1 eq.). The pale yellow solution was stirred at room temperature for 16-16.5 hours, then diluted with EtOAc (in a 3-5 volume multiple of the initial THF volume), and washed with 1M aq. HCl (2x), dilute aq. NaHCO₃ (2x), and brine (1x). The organic phase was dried over either Na₂SO₄ or MgSO₄, filtered, and concentrated.

### II. Carboxylic Acids

### GENERAL PROCEDURE II-A

### Ester Hydrolysis to Free Acid

Ester hydrolysis to the free acid was conducted by conventional methods. Below are two examples of such conventional de-esterification methods.

Method A: To a carboxylic ester compound in a 1:1 mixture of CH₃OH/H₂O was added 2-5 equivalents of K₂CO₃. The mixture was heated to 50°C for 0.5 to 1.5 hours until tlc showed complete reaction. The reaction was cooled to room temperature and the methanol was removed on a rotary evaporator. The pH of the remaining aqueous solution was adjusted to ∼2, and ethyl acetate was added to extract the product. The organic phase was then washed with saturated aqueous NaCl and dried over MgSO₄. The solution was stripped free of solvent on a rotary evaporator to yield the product.

Method B: The amino acid ester was dissolved in dioxane/water (4:1) to which was added LiOH (∼2 eq.) that was dissolved in water such that the total solvent after addition was about 2:1 dioxane:water. The reaction mixture was stirred until reaction completion and the dioxane was removed under reduced pressure. The residue was dissolved in water and washed with ether. The layers were separated and the aqueous layer was.acidified to pH 2. The aqueous layer was extracted with ethyl acetate. The ethyl acetate extracts were dried over Na₂SO₄ and the solvent was removed under reduced pressure after filtration. The residue was purified by conventional methods (e.g., recrystallization).

### GENERAL PROCEDURE II-B

### Acid Chloride Preparation

3,5-Difluorophenylacetic acid (30 g, 0.174 mol) (Aldrich) was dissolved in dichloromethane and this solution was cooled to 0°C. DMF (0.5 mL, catalytic) was added followed by the dropwise addition of oxalyl chloride (18 mL, 0.20 mol) over a 5 minute period. The reaction was stirred for 3 h and then rotoevaporated at reduced pressure to give an oil which was placed on a high vacuum pump for 1 h to afford 3,5-difluorophenylacetyl chloride as a thin yellow oil. Other acid chlorides can be prepared in a similar manner.

### GENERAL PROCEDURE II-C

### Schotten-Baumann Procedure

3,5-Difluorophenylacetyl chloride (from General Procedure II-B) was added dropwise to a 0°C solution of L-alanine (Aldrich) (16.7 g, 0.187 mol) in 2 N sodium hydroxide (215 mL, 0.43 mol). The reaction was stirred for 1 h at 0°C and then overnight at room temperature. The reaction was diluted with water (100 mL), then extracted with ethyl acetate (3 x 150 mL). The organic layer was then washed with brine (200 mL), dried over MgSO₄, and rotoevaporated at reduced pressure to a residue. Recrystallization of the residue from ethyl acetate/hexanes afforded the desired product (34.5 g, 82% yield). Other acid chlorides may be used in this procedure to provide for intermediates useful in this invention.

### GENERAL PROCEDURE II-D

### Reductive Amination

To a solution of the arylamine in ethanol in a hydrogenation flask was added 1 equivalent of the 2-oxocarboxylic acid ester (e.g., pyruvate ester), followed by 10% palladium on carbon (25 weight percent based on the arylamine). The reaction was hydrogenated at 20 psi H₂ on a Parr shaker until complete reaction was indicated by tlc (30 minutes to 16 hours). The reaction mixture was then filtered through a pad of Celite 545 (available from Aldrich Chemical Company, Inc.) and stripped free of solvent on a rotary evaporator. The crude product residue was then further purified via chromatography.

### Example A

### Synthesis of N-(Phenylacetyl)-L-alanine

Using General Procedure II-C, the title compound was prepared from phenylacetyl chloride (Aldrich) and L-alanine (Aldrich) as a solid having a melting point of 102-104°C.
NMR data was as follows:
¹H-nmr (CDCl₃): δ = 9.14 (br s, 1H), 7.21-7.40 (m, 5H), 6.20 (d, J = 7.0 Hz, 1H), 4.55 (m, 1H), 3.61 (s, 2H), 1.37 (d, J = 7.1 Hz, 3H).
¹³C-nmr (CDCl₃): δ = 176.0, 171.8, 134.0, 129.4, 127.5, 48.3, 43.2, 17.9.

### Example B

### Synthesis of N-(3,5-Difluorophenylacetyl)-L-alanine

Using General Procedure II-C, the title compound was prepared from 3,5-difluorophenylacetyl chloride (General Procedure II-B) and L-alanine (Aldrich).
NMR data was as follows:
¹H-nmr (CD₃OD): δ = 8.32 (br s, 0.3H), 6.71 (m, 2H), 6.60 (m, 1H), 4.74 (br s, 1.7H), 4.16 (m, 1H), 3.36 (s, 2H), 1.19 (d, J = 7.3 Hz, 3H).
¹³C-nmr (CD₃OD): δ = 175.9, 172.4, 164.4 (dd, J = 13.0, 245.3 Hz), 141.1, 113.1 (dd, J = 7.8, 17.1 Hz), 102.9 (t, J = 25.7 Hz), 49.5, 42.7, 17.5.

### Example C

### Synthesis of N-(Cyclopentylacetyl)-L-phenylglycine

### Step A - Preparation of N-(Cyclopentylacetyl)-L-phenylglycine Methyl Ester

Following General Procedure A above using cyclopentylacetic acid (Aldrich) and phenylglycine methyl ester hydrochloride (Novabiochem), the title compound was prepared as a solid having a melting point of 83-86°C. The reaction was monitored by tlc on silica gel (Rf = 0.28 in 25% ethyl acetate/hexanes) and purification was by recrystallization from ethyl acetate/hexanes.
NMR data was as follows:
¹H-nmr (CDCl₃): δ = 7.35 (s, 5H), 6.44 (bd, 1H), 5.6 (d, 1H), 3.72 (s, 3H), 2.24 (bs, 3H), 1.9-1.4 (m, 6H), 1.2-1.05 (m, 2H).
¹³C-nmr (CDCl₃): δ = 172.3, 171.7, 136.7, 129.0, 128.6, 127.3, 56.2, 52.7, 42.5, 36.9, 32.40, 32.38, 24.8.
C₁₆H₂₁NO₃ (MW = 275.35); mass spectroscopy (M+Na) 298.

### Step B - Preparation of N-(Cyclopentylacetyl)-L-phenylglycine

Following General Procedure II-A above using N-(cyclopentylacetyl)-L-phenylglycine methyl ester (from Step A), the title compound was prepared as a solid having a melting point of 155-158°C. The reaction was monitored by tlc on silica gel (Rf = 0.18 in 10% methanol/dichloromethane).
NMR data was as follows:
¹H-nmr (CDCl₃): δ = 8.60 (d, J = 7.8 Hz, 1H), 7.45 (m, 5H0, 5.41 (d, J = 7.2 Hz, 1H), 2.20 (m, 3H), 1.8-1.1 (m, 8H)..
¹³C-nmr (CDCl₃): δ = 172.3, 172.0, 137.5, 128.7, 128.1, 127.8, 56.2, 40.9, 36.8, 31.8, 24.5.
C₁₅H₁₉NO₃ (MW = 261.32); mass spectroscopy (M+Na) 284.

### Example D

### Synthesis of N-(Cyclopentylacetyl)-L-alanine

### Step A - Preparation of N-(Cyclopentylacetyl)-L-alanine Methyl Ester

Following General Procedure A above using cyclopentylacetic acid (Aldrich) and L-alanine methyl ester hydrochloride (Sigma), the title compound was prepared as a solid having a melting point of 43-46°C. Purification was by recrystallization from ethyl acetate/hexanes.
NMR data was as follows:
¹H-nmr (CDCl₃): δ = 6.38 (d, 1H), 4.50 (m, 1H), 3.65 (s, 3H), 2.13 (bs,
3H), 1.80-1.00 (m (includes d at 1.30, 3H), 11H).
¹³C-nmr (CDCl₃): δ = 173.7, 172.5, 52.1, 47.6, 42.3, 36.8, 32.15, 32.14, 18.0.
C₁₁H₁₉NO₃ (MW = 213.28); mass spectroscopy (MH⁺) 214.

### Step B - Preparation of N-(Cyclopentylacetyl)-L-alanine

Following General Procedure II-A above using N-(cyclopentylacetyl)-L-alanine methyl ester (from Step A), the title compound was prepared. The reaction was monitored by tlc on silica gel (Rf = 0.18 in 10% methanol/dichloromethane).
NMR data was as follows:
¹H-nmr (DMSO-d₆): δ = 12.45 (bs, 1H), 8.12 (d, J=7.2 Hz, 1H), 4.24 (quint, J = 7.2 Hz, 1H), 2.14 (m, 3H), 1.8-1.4 (m, 6H), 1.29 (d, J = 7.2 Hz, 3H), 1.2-1.0 (m, 3H).
¹³C-nmr (DMSO-d₆): δ = 174.6, 171.9, 47.3, 41.1, 36.7, 31.8, 24.5, 17.2.
C₁₀H₁₇NO₃ (MW = 199.25); mass spectroscopy (MH⁺) N/A.

### Example E

### Synthesis of N-(Cyclopropylacetyl)-L-alanine

### Step A - Preparation of N-(Cyclopropylacetyl)-L-alanine Methyl Ester

Following General Procedure A above using cyclopropylacetic acid (Aldrich) and L-alanine methyl ester hydrochloride (Sigma), the title compound was prepared as an oil. The reaction was monitored by tlc on silica gel (Rf = 0.15 in 25% ethyl acetate/hexanes) and purification was by flash column chromatography using 25% ethyl acetate/hexanes as the eluant.
NMR data was as follows:
¹H-nmr (CDCl₃): δ = 6.60 (d, 1H), 4.55 (m, 1H), 3.69 (s, 3H), 2.10 (m, 2H), 1.34 (d, 3H), 0.95 (m, 1H), 0.58 (m, 2H), 0.15 (m, 2H).
¹³C-nmr (CDCl₃): δ = 173.7, 172.3, 52.3, 47.7, 41.0, 18.2, 6.7, 4.27, 4.22.
C₉H₁₅NO₃ (MW = 185.22); mass spectroscopy (MH⁺) N/A.

### Step B - Preparation of N-(Cyclopentylacetyl)-L-alanine

Following General Procedure II-A above using N-(cyclopropylacetyl)-L-alanine methyl ester (from Step A), the title compound was prepared as an oil. The reaction was monitored by tlc on silica gel (Rf = 0.27 in 10% methanol/dichloromethane).
NMR data was as follows:
¹H-nmr (DMSO-d₆): δ = 8.18 (d, 1H), 4.25 (m, 1H), 2.08 (m, 2H), 1.30 (d, 3H), 1.00 (m, 1H), 0.50 (m, 2H), 0.19 (m, 2H).
¹³C-nmr (DMSO-d₆): δ = 174.6, 171.7, 47.4, 17.3, 7.6, 4.12, 4.06.
C₈H₁₃NO₃ (MW = 199.25); mass spectroscopy (MH⁺) N/A.

### Example F

### Synthesis of N-(Cyclopropylacetyl)-L-phenylglycine

### Step A - Preparation of N-(Cyclopropylacetyl)-L-glvcine Methyl Ester

Following General Procedure A above using cyclopropylacetic acid (Aldrich) and L-phenylglycine methyl ester, the title compound was prepared as a solid having a melting point of 74-76°C. The reaction was monitored by tic on silica gel (Rf = 0.61 in 50% ethyl acetate/hexanes) and purification was by recrystallization from ethyl acetate/hexanes.
NMR data was as follows:
¹H-nmr (CDCl₃): δ = 7.35 (m, 5H), 6.97 (bd, J=7.2 Hz, 1H), 5.59 (d, J=7.8 Hz, 1H), 3.71 (s, 3H), 2.17 (m, 2H), 1.05-0.95 (m, 1H), 0.62 (m, 2H), 0.20 (m, 2H).
¹³C-nmr (CDCl₃): δ = 171.9, 174.6, 136.6, 129.0, 128.5, 127.2, 56.1, 52.7, 41.0, 6.9, 4.37, 4.33.
C₁₄H₁₇NO₃ (MW = 247.30); mass spectroscopy (MH⁺) N/A.

### Step B - Preparation of N-(Cyclopentylaceyl)-L-phenylglycine

Following General Procedure II-A above using N-(cyclopropylacetyl)-Lphenylglycine methyl ester (from Step A), the title compound was prepared as a solid having melting point of 152-157°C. The reaction was monitored by tlc on silica gel (Rf = 0.23 in 10% methanol/dichloromethane) and purification was by recrystallization from ethyl acetate/hexanes.
NMR data was as follows:
¹H-nmr (CDCl₃): δ = 8.47 (d, J = 7.69 Hz, 1H), 7.35 (m, 5H), 5.34 (d, J = 7.69 Hz, 1H), 2.10 (m, 2H), 0.90 (m, 1H), 0.40 (m, 2H), 0.10 (m, 2H).
¹³C-nmr (CDCl₃): δ = 172.3, 171.8, 137.6, 128.7, 56.2, 7.7, 4.0.
C₁₃H₁₅NO₃ (MW = 233.27); mass spectroscopy (MH⁺) N/A.

### Example H

### Synthesis of N-(2-Biphenyl)-D,L-alanine

2-Aminobiphenyl (2 g, 11.8 mmol, Aldrich), triethylamine (1.2 eq.) and ethyl 2-bromopropionate (1.1 eq., Aldrich) were combined and heated to 85°C with stirring. After 7 days, the mixture was diluted with chloroform and washed with water. The organic portion was dried and concentrated to yield an oil which was purified by silica gel chromatography (1:1 CH₂Cl₂/hexanes). The resulting oil was dissolved in a 1:2 mixture of water/dioxane (200 mL) and LiOH (2 eq.) was added. After 2 hours, the mixture was concentrated to yield an oil which was dissolved in water. The aqueous solution was washed with ether then was adjusted to pH 3 with 5N HCl and extracted with ethyl acetate. The organic portion was dried and concentrated to yield an oil which was purified by silica gel chromatography (EtOAc) to yield the title compound.

### Example I

### Synthesis of N-(Phenyl-furazan-3-yl)-D,L-alanine

Following General Procedure II-D and using 4-phenyl-furazan-3-ylamine (Maybridge) and ethyl pyruvate (Aldrich), the ethyl ester was prepared. Following General Procedure II-A, Method B (LiOH/H₂O/dioxane) and using the ethyl ester, the title compound was prepared.

### Example L

### Synthesis of S-(+)-3,5-Difluoromandelic Acid

### Step A - Preparation of Methyl S-(±)-3.5-difluoromandelate

To a solution of 3,5-difluorobenzaldehyde (Aldrich) in CH₂Cl₂ (100 mL) was added ZnCl₂ (6.7 g, 21.1 mmol) to form a slurry. Trimethysilyl cyanide (21.0 g, 211.2 mmol) dissolved in CH₂Cl₂ (100 mL) was slowly added to the slurry at 0°C. The resulting solution was stirred at room temperature for 4 h. The reaction mixture was then diluted with water and the organic layer separated. The combined organic layers were concentrated to a residue. The residue was dissolved with MeOH (200 mL) at 0°C and anhydrous HCl gas bubbled into the solution for 10 min. After stirring at room temperature for 18 h, the solution was concentrated to a solid. The solid was dissolved in CH₂Cl₂ /H₂O and the aqueous portion extracted with CH₂Cl₂. The combined organics were washed with brine, dried over anhydrous MgSO₄ and concentrated to a solid (37.4 g, 87.6%), mp = 77-78°C.
¹H NMR (300 MHz, CDCl₃): δ = 6.97 (dd, J = 9.6 Hz, J = 1.79 Hz, 2H), 6.74 (dt, J = 8.82, J = 2.28 Hz, 1H), 5.14 (d, J = 4.64 Hz, 1H), 3.78 (s, 3H), 3.54 (d, J = 5.1 Hz, 1H).

### Step B - Preparation of Methyl S-(+)-3,5-difluoromandelate

Methyl (±)-3,5-difluoromandelate was separated via preparative chiral HPLC to give a white solid having a melting point of 70-71°C.
C₉H₈F₂O₃ (MW = 202.17); mass spectroscopy found (M+NH₄⁺) 220.0
Anal. calcd for C₉H₈F₂O₃: C, 53.47; H, 3.99. Found: C, 53.40; H, 3.89.

### Step C - Preparation of S-(+)-3,5-Difluoromandelic acid

A solution of methyl S-(+)-3,5-difluoromandelate (1 eq.) in 74% aqueous THF was cooled to 0 °C and treated with lithium hydroxide. After 40 minutes at 0 °C the reaction was complete by TLC. The contents were transferred to a separatory funnel and partitioned between CH₂Cl₂ and saturated aqueous NaHCO₃. The aqueous layer was acidified with 0.5 N NaHSO₄ and extracted thrice with ethyl acetate. The combined extracts were washed with brine, dried over Na₂SO₄, filtered, and concentrated to a white solid having a melting point of 119-122 °C. The ¹H NMR was consistent with known 3,5-difluoromandelic acid.

### Example M

### Synthesis of 2-Azido-(3,5-difluorophenyl)acetic Acid

Step A: To a three-necked flask equipped with a mechanical stirrer and a nitrogen inlet tube was added 3,5-difluorophenylacetic acid and THF. The reaction mixture was cooled to -78°C and 1.2 eq. of triethylamine was added, followed by dropwise addition of trimethylacetyl chloride (1.05 eq.). During the addition, the temperature was maintained at -78°C. The cold bath was then removed and replaced with an ice bath. The temperature was allowed to warm to 0°C and stirring was continued for 1 hour. The reaction mixture was then recooled to -78°C. To a second flask charged with THF, triphenylmethane (cat, 0.1 mole %) and (S)-(-)-4-benzyl-2-oxazolidione (1.1 eq.) (Aldrich) at -78°C was added an n-butyl lithium solution dropwise until an orange color persisted. This reaction mixture was stirred at -78°C for 30 min. and then cannulated into the first reaction mixture. The resulting mixture was allowed to stir at -78°C for 1 hour and then quenched with 2.2 eq. of acetic acid. The solvent was removed under reduced pressure and the residue was redissolved in dichloromethane and this solution washed with water, followed by 1M potassium carbonate. The organic layer was then dried over sodium sulfate, filtered and concentrated. The residue was purified by LC 2000 chromatograph, eluting with EtOAC/Hexane (15:85). The resulting oil was slurried in hexane to afford a white solid which was collected by filtration to give (S)-(-)-3-(3,5-difluorophenyacetyl)-4-benzyl-2-oxazolidione.
Step B: To (S)-(-)-3-(3,5-difluorophenyacetyl)-4-benzyl-2-oxazolidione (3.0 mM) in 20 mL of dry THF cooled to -78°C was added LiHMDS (1.05 eq.) dropwise while maintaining the temperature at -78°C. The reaction mixture was allowed to stir at -78°C for 15 min. and then a pre-cooled (-60°C) solution of trisyl azide (1.12 eq.) in 10 mL of THF was added. The reaction mixture was allowed to stir an additional 10 min. and then was quenched with 4.4 eq. of acetic acid. Using a warm water bath, the temperature was raised to 30-40°C for 6 hrs. The reaction mixture was then poured into a separatory funnel and extracted into dichloromethane. The organic layer was washed with bicarbonate solution, followed by brine, and then dried over sodium sulfate, filtered and solvent removed. The residue was purified by LC 2000 chromatography to afford methyl 2-azido-2-(3,5-difluorophenyl)acetate.
Step C: To a solution of methyl 2-azido-2-(3,5-difluorophenyl)acetate in THF/H₂O (2.6:1) cooled to 0°C was added 1.7 eq. of lithium hydroxide. The reaction mixture was stirred at room temperature for 3 hours and then poured into a separatory funnel. The mixture was extracted into water and washed with ether. The aqueous layer was acidified with 1N HCl and extracted with ethyl acetate. The organic layer was then washed with water and brine. The organic layer was dried over sodium sulfate, filtered and concentrated under reduced pressure to give 2-azido-2-(3,5-difluorophenyl)acetic acid.

### Example O

### Synthesis of 3,5-Difluorophenyl-α-oxoscetic Acid

Step A: Ethyl 3,5-difluorophenyl-α-oxoacetate was prepared from 1-bromo-3,55-difluorobenzene (Aldrich) according to the procedure described in J. Org. Chem, 45 (14), 2883-2887 (1980).
Step B: Ethyl 3,5-difluorophenyl-α-oxoacetate was hydrolyzed using General Procedure II-A (Method B) to afford 3,5-difluorophenyl-α-oxoacetic acid.

### Example P

### Synthesis of Cyclopentyl-α-hydroxyacetic Acid

The title compound (CAS No. 6053-71-0) was prepared in two steps from cyclopentylmethanal (CAS No. 872-53-7, Wiley) using the procedure described by Gibby, W. A.; Gubler, C. J. Biochemical Medicine 1982, 27, 15-25.

### Example Q

### Synthesis of N-(3,4-dichlorophenyl)alanine

Using the procedure set forth in U.S. Patent No. 3,598,859, the disclosure of which is incorporated herein by reference in its entirety, N-(3,4-dichlorophenyl)alanine was prepared. Specifically, to a solution of 3,4-dichloroaniline (1 equivalent) (Aldrich) in isopropanol (about 500 mL per mole of 3,4-dichloroaniline) is added water (about 0.06 mL per mL of isopropanol) and 2-chloropropionic acid (2 equivalents) (Aldrich). This mixture is warmed to 40°C and sodium bicarbonate (0.25 equivalents) is added in successive portions before heating under reflux for 4-5 days. After cooling, the reaction mixture is poured into water and the unreacted 3,4-dichloroaniline is removed by filtration. The filtrate is acidified to pH 3-4 with concentrated hydrochloric acid and the resultant precipitate is filtered, washed and dried to yield the title compound, m.p. = 148-149°C.

### Example R

### Synthesis of N-(3,5-difluorophenyl)alanine

Using the procedure set forth in U.S. Patent No. 3,598,859 and Example Q above, N-(3,5-difluorophenyl)alanine was prepared using 3,5-difluoroaniline (Aldrich) and 2-chloropropionic acid (Aldrich).

### Example S

### Synthesis of α-Fluoro-3,5-difluorophenylacetic Acid

### Step A - Synthesis of Methyl 3,5-Difluoromandelate

To a solution of 3,5-difluoromandelic acid (Fluorochem) in methanol was bubbled HCl gas for 10 minutes. The reaction was refluxed overnight. The mixture was then concentrated *in vacuo* and the residue was taken up in ethyl acetate and washed with saturated NaHCO₃ and brine. The organic layer was dried over Na₂SO₄, filtered, and concentrated to give the title intermediate as a white solid.
C₉H₈F₂O₃ (MW=202.17); mass spectroscopy 202.
¹H NMR (300 MHz, CDCl₃): δ = 7.00 (2H, d, J=6.58 Hz), 6.76 (1H, t, J=8.86 Hz), 5.16 (1H, d, J=5.29 Hz), 3.81 (3H, s), 3.54 (1H, d, J=5.39 Hz).

### Step B - Synthesis of Methyl α-Fluoro-3,5-difluorophenylacetate

A solution of diethylaminosulfur trifluoride (DAST) (1.1 eq) in methylene chloride was cooled to 0°C and a pre-cooled solution of methyl 3,5-difluoromandelate (1 eq) in methylene chloride was added. The transfer flask was rinsed with a small portion of methylene chloride. After 15 minutes, the cooling bath was removed and the reaction mixture was stirred an additional 40 minutes at ambient temperature. The mixture was poured over ice and the layers separated. The organic phase was washed with saturated NaHCO₃ and brine. The organic layer was dried over Na₂SO₄, and concentrated. The residue was purified via HPLC eluting with 7% ethyl acetate/hexanes providing the title intermediate as a yellow oil.
C₉H₇F₃O₂ (MW-204.16); mass spectroscopy 204.
Anal). calcd for C₉H₇F₃O₂: C, 52.95; H, 3.46. Found: C, 52.80; H, 3.73.

### Step C- Synthesis of α-Fluoro-3,5-difluorophenylacetic Acid

Following General Procedure II-A, Method B and using methyl α-fluoro-3,5-difluorophenylacetate, the title intermediate was prepared as a white solid having a melting point of 100-102°C.
C₈H₅F₃O₂ (MW-190.13); mass spectroscopy 190.
Anal. calcd for C₈H₅F₃O₂: C, 50.54; H, 2.65. Found: C, 50.47; H, 2.79.

### III Lactams

### GENERAL PROCEDURE 5-A

### N-Alkylation of Lactams

To a stirred solution of a BOC-protected α-aminocaprolactam (6.87 g, 30 mmol) in DMF (150 mL) was added in portions 97% NaH (1.08g, 45 mmol). Bubbling occured immediately and followed by heavy precipitation. After 10 min., benzyl bromide (3.93 mL, 33 mmol) was added. The precipitate dissolved quickly and in about 10 min. a clear solution was obtained. The reaction mixture was stirred overnight and then evaporated as completely as possible on a rotovap at 30°C. Ethyl acetate (100 mL) was added to the residue and this mixture was washed with water, brine, and dried over magnesium sulfate. After filtration and concentration, a thick liquid (10 g) was obtained which was then chromatographed over silica gel with 1:3 ethyl acetate/hexane as the eluant to provide 5.51 g (58%) of the N-benzylated product as an oil. Other lactams and alkylating agents may be used in this procedure to obtain a wide variety of N-alkylated lactams. Various bases, such as LiN(SiMe₃), may also be employed.

### GENERAL PROCEDURE 5-B

### BOC Removal Procedure

The BOC-protected compound in a 1:1-2:1 mixture of CH₂Cl₂ and trifluoroacetic acid was stirred until tlc indicated complete conversion, typically 2 hours. The solution was then stripped to dryness and the residue was taken up in ethyl acetate or CH₂Cl₂. The solution was washed with saturated aqueous NaHCO₃ and the aqueous phase was adjusted to a basic pH, then extracted with ethyl acetate or CH₂Cl₂. The organic phase was washed with saturated aqueous NaCl and dried over MgSO₄. The solution was stripped free of solvent on a rotary evaporator to yield the product.

### GENERAL PROCEDURE 5-C

### Synthesis of α-Aminolactams

The Schmidt reaction was conducted on 4-ethylcyclohexanone using hydroxyamine sulfonic acid as described in Olah, Org. Synth. Collective, Vol. VII, page 254, to provide 5-ethylcaprolactam in 76% yield. Using the procedure described in Watthey, et al., J. Med. Chem., 1985, 28, 1511-1516, this lactam was then dichlorinated with PCl₅ at the alpha position and reduced by hydrogenation to provide four isomeric monochlorides (two racemic mixtures). The two racemic mixtures were separated from each other by column chromatography using silica gel and each racemic mixture was reacted with sodium azide to yield the corresponding azide which was hydrogenated to provide the corresponding α-aminolactams. Other cycloalkanones may be employed in this procedure to provide a wide variety of α-aminolactams. In some cases, such as when preparing the 9-membered ring α-aminolactam, longer reaction times, higher reaction temperatures and an excess of sodium azide may be required. For example, the 9-membered ring α-aminolactam required 5 equivalents of sodium azide, a reaction temperature of 120°C and a reaction time of 4 days. Such conditions can be readily determined by those of ordinary skill in the art.

### 6. Benzazepinone Derivatives and Related Compounds

### GENERAL PROCEDURE 6-A

### Alkylation of 1-Amino-1,3,4,5-tetrahydro-2H-3-benzazepin-2-one

Step A: 1-Ethoxycarbonylamino-1,3,4,5-tetrahydro-2H-3-benzazepin-2-one was prepared according to the procedure of Ben-Ishai et al., Tetrahedron, 1987, 43, 430.
Step B: 1-Ethoxycarbonylamino-1,3,4,5-tetrahydro-2H-3-benzazepin-2-one (2.0 g, 100 M%) was dissolved in DMF (30 mL) and NaH (95%, 0.17 g, 100M%) was added in one portion. The reaction mixture was stirred for 1 hour and then the appropriate alkyl iodide (300M%) was added and the mixture was stirred for 12 hours. The reaction was poured into water and extracted with ethyl acetate (3x). The ethyl acetate extracts were then washed with water (3x) and brine (1x). Treatment with MgSO₄, rotoevaporation, and chromotography (30% EtOAc/hexanes) yielded 1-ethoxycarbonylamino-3-alkyl-1,3,4,5-tetrahydro-2H-3-benzazepin-2-one in 87% yield.
Step C: 1-Ethoxycarbonylamino-3-alkyl-1,3,4,5-tetrahydro-2H-3-benzazepin-2-one (1.0g, 100M%) was suspended in 30 mL of 30% HBr/HOAc and heated to 100°C. The reaction mixture was stirred for 5 hours at this temperature and then the reaction was cooled and rotoevaporated to yield 1-amino-3-alkyl-1,3,4,5-tetrahydro-2H-3-benzazepin-2-one as the hydrobromide salt (100% yield).

### Example 6-1

### Synthesis of 1-(N'-(3,5-Difluorophenylacetyl)-L-alaninyl)amino-3-methyl-1,3,4,5-tetrahydro-2H-3-benzazepin-2-one

Following General Procedure A above using *N-*(3,5-difluorophenylacetyl)-L-alanine (Example B) and 1-amino-3-methyl-1,3,4,5-tetrahydro-2H-3-benzazepin-2-one, the title compound was prepared. The reaction was monitored by tlc on silica gel (Rf = 0.15 in ethyl acetate) and purification was by flash chromatography using ethyl acetate as the eluant.
NMR data was as follows:
¹H-nmr (CDCl₃; 2 diasteromers): δ = 8.10 (m, 1H), 7.58 (d, 0.5H), 7.42 (d, 0.5H), 7.05 (m, 4H0, 6.65 (m, 3H), 6.29 (m, 1H), 4.80 (t, 1H), 4.20 (m, 1H), 3.36 (s, 0.5H), 3.34 (s, 0.5H), 3.26 (bd, 2H), 3.10 (m, 2H), 3.01 (s, 3H), 2.98 (s, 3H), 1.36 (d, 3H), 1.29 (s, 3H).
¹³C-nmr (CDCl₃; 2 diasteromers): δ = 168.2, 167.9, 165.3, 165.2, 165.1, 164.9, 160.3, 160.1, 157.0, 156.8, 134.4, 134.3, 130.1, 129.9, 129.0, 128.8, 126.0, 123.3, 122.5, 119.5, 119.1, 107.9, 107.8, 107.6, 98.3, 98.0, 97.6, 47.6, 47.4, 44.6, 44.5, 43.7, 43.6, 38.0, 37.8, 30.6, 30.5, 26.6; 14.6, 14.1.
C₂₂H₂₃N₃O₃F₂ (MW = 415.44); mass spectroscopy (M⁺) 415.

### Example 6-2

### Synthesis of 1-(S)-(N'-(3,5-Difluorophenylacetyl)-L-alaninyl)amino-3-ethyl-7-fluoro-1,3,4,5-tetrahydro-2H-3-benzazepin-2-one

Following General Procedure C above using *N*-(3,5-difluorophenylacetyl)-L-alanine (Example B) and 1-(S)-amino-3-ethyl-7-fluoro-1,3,4,5-tetrahydro-2H-3-benzazepin-2-one (General Procedure 6-A), the title compound was prepared. Purification was by flash chromatography using 5% methanol/dichloromethane as the eluant.
NMR data was as follows:
¹H-nmr (CDCl₃): δ = 7.8-7.7 (2xd, J = 7 Hz, 1H0 7.1-7.0 (m, 2H), 6.8 (m, %H), 6.2 (t, 1H), 4.7 (t, 1H0, 4.2 (m, 1H), 3.6-3.4 (m, 6H), 3.2 (m, 2H), 1.5-1.3 (2xd, J = 7 Hz, 3H), 1.1 (2xt, J = 7 Hz, 3H).
¹³C-nmr (CDCl₃): δ = 177.3, 172.5, 172.1, 169.6, 169.4, 163.8, 160.5, 126.3, 126.2, 125-9, 125.8, 117.4, 117.2, 117.1, 116.9, 113.7, 113.4, 112.4, 112.3, 112.1, 112.0, 103.0, 102.9, 102.7, 102.6, 102.2., 53.3, 51.7, 51.4, 49.2, 49.0, 44.8, 44.5, 42.6, 42.5, 42.4. 42.3, 32.2, 19.0, 13.0, 12.9.
C₂₃H₂₄N₃O₃F₃ (MW - 447.19); mass spectroscopy (MH⁺) N/A.

### Example 6-16

### Synthesis of 1-(N'-(3,5-Difluorophenylacetyl)-L-alaninyl)amino-5-phenyl-1,2,4,5-tetrahydro-2H-3-benzazepin-2-one

Following General Procedure D above using 3,5-difluorophenylacetic acid (Oakwood) and 1-(N'-(3,5-difluorophenylacetyl)-L-alaninyl)amino-5-phenyl-1,3,4,5-tetrahydro-2H-3-benzazepin-2-one, the title compound was prepared. Purification was by LC 2000 chromatography using ethyl acetate as the eluant
C₂₇H₂₅N₃O₃F₂ (MW = 477); mass spectroscopy (MH⁺) 478.1.
Anal. Calc. for C₂₇H₂₅N₃O₃F₂: C, 67.91; H, 5.28; N, 8.8. Found: C, 68.2; H, 5.35; N, 8.58.

### 7. Dibenzazepinone Derivatives and Related Compounds

### GENERAL PROCEDURE 7-A

### Preparation of 5-Amino-7-alkyl-5,7-dihydro-6H-dibenz[b,d]azepin-6-one Derivatives

Step A: Following General Procedure 5-A and using 5,7-dihydro-6H-dibenz[b,d]azepin-6-one and an alkyl halide, the 7-alkyl-5,7-dihydro-6H-dibenz[b,d]azepin-6-one was prepared.

Step B: The 7-alkyl-5,7-dihydro-6H-dibenz[b,d]azepin-6-one (1 eq.) was dissolved in THF and isoamylnitrite (1.2 eq.) was added. The mixture was cooled to 0°C in an ice bath. NaHMDS (1.1 eq., 1M in THF) was added dropwise. After stirring for 1 hour or until the reaction was complete, the mixture was concentrated then acidified with 1N HCl and extracted with EtOAc. The organic portion was dried and concentrated to yield a crude product which was purified by silica gel chromatography.

Step C: The resulting oxime was dissolved in EtOH/NH₃ (20: 1) and hydrogenated in a bomb using Raney nickel and hydrogen (500 psi) at 100°C for 10 hours. The resulting mixture was filtered and concentrated to provide an oil which was purified by silica gel chromatography to yield the title compound.

### GENERAL PROCEDURE 7-B

### Preparation of Fluoro-substitued 5,7-dihydro-6H-dibenz[b,d]azepin-6-one Derivatives

A modification of the procedure of Robin D. Clark and Jahangir, Tetrahedron, Vol. 49, No. 7, pp. 1351-1356, 1993 was used. Specifically, an appropriately substituted N-t-Boc-2-amino-2'-methylbiphenyl was dissolved in THF and cooled to -78°C: s-Butyl lithium (1.3M in cyclohexane, 2.2 eq.) was added slowly so that the temperature remained below -65°C. The resulting mixture was allowed to warm to -25 °C and was stirred at that temperature for 1 hour. The mixture was cooled to -78°C. Dry CO₂ was bubbled through the mixture for 30 seconds. The mixture was allowed to warm to ambient temperature then was carefully quenched with water. The mixture was concentrated under reduced pressure then was adjusted to pH 3 with 1N HCl. The mixture was extracted with EtOAc and the organic portion was dried and concentrated to yield a crude material. The crude material was dissolved in methanol and the solution was saturated with HCl. The mixture was heated at reflux for 12 hours then was allowed to cool. The mixture was concentrated to provide crude lactam which was purified by chromatography or crystallization.

### GENERAL PROCEDURE 7-C

### Resolution of 5-amino-7-methyl-5,7-dihydro-6H-dibenz[b,d]azepin-6-one

In a round bottom flask was added the racemic freebase amine (1.0 eq.) in methanol followed by di-*p*-toluoyl-D-tartaric acid monohydrate (1.0 eq.). The mixture was concentrated *in vacuo* to a residue and redissolved in a moderate volume of methanol and allowed to stir at room temperature open to the atmosphere (8-72 hours). The solid was removed by filtration. The enantiomeric excess was determined by chiral HPLC (Chiracel ODR) using 15% acetonitrile and 85% H₂O with 0.1% trifluoroacetic acid and a flow rate of 1.0 mL/min at 35°C. The resolved di-*p*-toluoyl-D-tartaric salt was then dissolved in EtOAc and saturated NaHCO₃ until pH 9-10 was reached. The layers were separated and the organic layer was washed again with saturated NaHCO₃, H₂O, and brine. The organic layer was dried over MgSO₄ and the drying agent was removed by filtration. The filtrate was concentrated *in vacuo.* The free amine was dissolved in MeOH and HCl (12M, 1.0 eq.) was added. The salt was concentrated *in vacuo* and the resulting film was triturated with EtOAc. The HCl salt was filtered and rinsed with EtOAc. The ee was determined by chiral HPLC.

### Example 7-A

### Synthesis of 5-Amino-7-methyl-5,7-dihydro-6H-dibenz[b,d]azepin-6-one Hydrochloride

### Step A - Synthesis of 7-Methyl-5,7-dihydro-6H-dibenz[b,d]azepin-6-one

A round bottom flask was charged with sodium hydride (0.295 g, 7.46 mmol) in 9.0 ml of DMF and treated with 5,7-dihydro-6H-dibenz[b,d]azepin-6-one (1.3 g, 6.22 mmol) (CAS # 20011-90-9, prepared as described in Brown, et. al., Tetrahedron Letters, No. 8, 667-670, (1971) and references cited therein). After stirring at 60°C for 1 h, the solution was treated with methyl iodide (1.16 ml, 18.6 mmol) and stirring continued for 17 h with the exclusion of light. After cooling, the reaction was diluted with CH₂Cl₂/H₂O, washed with NaHSO₄ solution, H₂O, and dried over Na₂SO₄. Evaporation and flash chromatography (SiO₂, CHCl₃) gave 0.885 g (63%) of the title compound as a colorless solid.
NMR data was as follows:
¹H-nmr (CDCl₃): *δ* = 7.62 (d, 2H), 7.26-7.47 (m, 6H), 3.51 (m, 2H), 3.32 (s, 3H).
C₁₅H₁₃NO(MW = 223.27); mass spectroscopy (MH+) 223.
Anal. Calcd for C₁₅H₁₃NO; C, 80.69 H, 5.87 N, 6.27. Found: C, 80.11 H, 5.95 N, 6.23.

### Step B - Synthesis of 7-Methyl-5-oximo-5,7-dihydro-6H-dibenz[b,d]azepin-6-one

The compound isolated above (0.700 g, 3.14 mmol) was dissolved in 20 ml of toluene and treated with butyl nitrite (0.733 ml, 6.28 mmol). The reaction temperature was lowered to 0°C and the solution was treated with KHMDS (9.42 ml, 0.5 M) under N₂ atmosphere. After stirring for 1 h the reaction was quenched with a saturated solution of NaHSO₄, diluted with CH₂Cl₂ and separated. The organic layer was dried over Na₂SO₄ and the title compound purified by chromatography (SiO₂, 98:2 CHCl₃/MeOH) giving 0.59 g (80 %) as a colorless solid.
C₁₅H₁₂N₂O₂ (MW = 252.275); mass spectroscopy (MH+) 252.
Anal. Calcd for C₁₅H₁₂N₂O₂; C, 71.42 H, 4.79 N, 11.10. Found: C, 71.24 H, 4.69 N, 10.87.

### Step C - Synthesis of 5-Amino-7-Methyl-5,7-dihydro-6H-dibenz[b,d]azepin-6-one Hydrochloride

The oxime isolated above (0.99 g, 3.92 mmol) was hydrogenated in a Parr apparatus at 35 psi over 10 % Pd/C (0.46 g) in 3A ethanol. After 32 h the reaction mixture was filtered through a plug of celite, the filtrate evaporated to a foam and treated with a saturated solution of HCl (g) in Et₂O. The resulting colorless solid was filtered, rinsed with cold Et₂O and vacuum dried to give 0.66 g (61 %) of the title compound.
NMR data was as follows:
¹H-nmr (DMSOd6): δ = 9.11 (bs, 3H), 7.78-7.41(m, 8H), 4.83 (s, 1H), 3.25 (s, 3H).
C₁₅H₁₄N₂O HCl (MW = 274.753); mass spectroscopy (MH+ free base) 238.
Anal. Calcd for C₁₅H₁₄N₂O HCl; C, 65.57 H, 5.50 N, 10.19 Found: C, 65.27 H, 5.67 N, 10.13.

### Example 7-B

### Synthesis of (S)- and (R)-5-(L-Alaninyl)-amino-7-methyl-5,7-dihydro-6H-dibenz[b,d]azepin-6-one

### Step A - Synthesis of (S)- and (R)-5-(N-Boc-L-Alaninyl)-amino-7-methyl-5,7-dihydro-6H-dibenz[b,d]azepin-6-one

Boc-L-Alanine (0.429 g, 2.26 mmol) (Aldrich) was dissolved in THF and treated with HOBt hydrate (0.305 g, 2.26 mmol), and 5-amino-7-methyl-5,7-dihydro-6H-dibenz[b,d]azepin-6-one (0.45 g, 1.89 mmol) (Example 7-A). The temperature was lowered to 0°C and the reaction mixture treated with EDC (0.449 g, 2.26 mmol) (Alrich) and stirred 17 hours under N₂. The reaction mixture was evaporated, the residue diluted with EtOAc/H₂O, washed 1.0 N HCl, sat. NaHCO₃, brine and dried over Na₂SO4. The diastereomers were separated on a Chiralcel OD column using 10% IPA/heptane at 1.5 ml/minute.
Isomer 1: Retention time 3.37 minutes.
   NMR data was as follows:
   ¹H-nmr (CDCl₃): δ = 7.62-7.33 (m, 9H), 5.26 (d, 1H), 5.08 (m, 1H), 4.34 (m, 1H), 3.35 (s, 3H), 1.49 (s, 9H), 1.40 (d, 3H).
   Optical Rotation: [α]₂₀ = - 96 @ 589 nm (c = 1, MeOH).
   C₂₃H₂₇N₃O₄ (MW = 409.489); mass spectroscopy (MH+) 409.
   Anal. Calcd for C₂₃H₂₇N₃O₄; C, 67.46 H, 6.64 N, 10.26. Found: C, 68.42 H, 7.02 N, 9.81.
Isomer 2: Retention time 6.08 minutes.
   NMR data was as follows:
   ¹H-nmr (CDCl₃): δ = 7.74 (bd,1H), 7.62-7.32 (m, 8H), 5.28 (d, 1H), 4.99 (m, 1H), 4.36 (m, 1H), 3.35 (s, 3H), 1.49 (s, 9H), 1.46 (d, 3H).
   Optical Rotation: [α]₂₀ = 69 @ 589 nm (c = 1, MeOH).
   C₂₃H₂₇N₃O₄ (MW = 409.489); mass spectroscopy (MH+) 409.
   Anal. Calcd for C₂₃H₂₇N₃O₄; C, 6.7.46 H, 6.64 N, 10.26. Found: C, 67.40 H, 6.62 N, 10.02

### Step B - Synthesis of (S)- and (R)-5-(L-Alaninyl)-amino-7-methyl-5,7-dihydro-6H-dibenz[b,d]azepin-6-one Hydrochloride

The compounds isolated in Part A (each isomer separately) were dissolved in dioxane and treated with excess HCl (g). After stirring for 17 hours, the title compounds were isolated as colorless solids after evaporation and vacuum drying.
Isomer 1:
   C₁₈H₁₉N₃O₂.HCl (MW = 345.832); mass spectroscopy (MH+ free base) 309.
   Optical Rotation: [α]₂₀ = - 55 @ 589 nm (c = 1, MeOH).
Isomer 2:
   C₁₈H₁₉N₃O₂.HCl (MW = 345.832); mass spectroscopy (MH+ free base) 309.
   Optical Rotation: [α]₂₀ = 80 @ 589 nm (c = 1, MeOH).

### Example 7-C

### Synthesis of (S)- and (R)-5-(L-Valinyl)-amino-7-methyl-5,7-dihydro-6H-dibenz[b,d]azepin-6-one

### Step A - Synthesis of (S)- and (R)-5-(N-Boc-L-Valinyl)-amino-7-methyl-5,7-dihydro-6H-dibenz[b,d]azepin-6-one

Boc-L-Valine (0.656 g, 3.02 mmol) (Aldrich) was dissolved in THF and treated with HOBt hydrate (0.408, 3.02 mmol), Dipea (1.05 ml, 6.05 mmol) and 5-amino-7-methyl-5,7-dihydro-6H-dibenz[b,d]azepin-6-one hydrochloride (0.75 g, 2.75 mmol)(Example 7-A). The temperature was lowered to 0°C and the reaction mixture treated with EDC (0.601 g, 3.02 mmol)(Alrich) and stirred 17 hours under N₂. The reaction mixture was evaporated, the residue diluted with EtOAc/H₂O, washed 1.0 N HCl, sat. NaHCO₃, brine and dried over Na₂SO₄. The diastereomers were separated on a Chiralcel OD column using 10% IPA/heptane at 1.5 ml/minute.
Isomer 1: Retention time 3.23 minutes.
   Optical Rotation: [α]₂₀ = - 120 @ 589 nm (c = 1, MeOH).
   C₂₅H₃₁N₃O₄ (MW = 437.544); mass spectroscopy (MH+) 438
Isomer 2: Retention time 6.64 minutes.
   Optical Rotation: [α]₂₀ = 50 @ 589 nm (c = 1, MeOH).
   C₂₅H₃₁N₃O₄ (MW = 437.544); mass spectroscopy (MH+) 438

### Step B - Synthesis of (S)- and (R)-5-(L-Valinyl)-amino-7-methyl-5,7-dihydro-6H-dibenz[b,d]azepin-6-one Hydrochloride

The compounds isolated in Part A (each isomer separately) were dissolved in dioxane and treated with excess HCl (g). After stirring for 17 hours, the title compounds were isolated as colorless solids after evaporation and vacuum drying.
Isomer 1:
   C₂₀H₂₃N₃O₂.HCl (MW = 373.88); mass spectroscopy (MH+ free base) 338.
   Optical Rotation: [α]₂₀ = - 38 @ 589 nm (c = 1, MeOH).
Isomer 2:
   C₂₀H₂₃N₃O₂.HCl (MW = 373.88); mass spectroscopy (MH+ free base) 338.
   Optical Rotation: [α]₂₀ = 97 @ 589 nm (c = 1, MeOH).

### Example 7-D

### Synthesis of (S)- and (R)-5-(L-tert-Leucine)-amino-7-methyl-5,7-dihydro-6H-dibenz[b,d]azepin-6-one

### Step A - Synthesis of (S)- and (R)-5-(N-Boc-L-tert-Leucinyl)-amino-7-methyl-5,7-dihydro-6H-dibenz[b,d]azepin-6-one

Boc-L-tert-Leucine (0.698 g, 3.02 mmol) (Fluka) was dissolved in THF and treated with HOBt hydrate (0.408, 3.02 mmol), Dipea (1.05 ml, 6.05 mmol) and 5-amino-7-methyl-5,7-dihydro-6H-dibenz[b,d]azepin-6-one hydrochloride (0.75 g, 2.75 mmol)(Example 7-A). The temperature was lowered to 0°C and the reaction mixture treated with EDC (0.601 g, 3.02 mmol) (Alrich) and stirred 17 hours under N₂. The reaction mixture was evaporated, the residue diluted with EtOAc/H₂O, washed 1.0 N HCl, sat. NaHCO₃, brine and dried over Na₂SO₄. The diastereomers were separated on a Chiralcel OD column using 10% IPA/heptane at 1.5 ml/minute.
Isomer 1: Retention time 3.28minutes.
   Optical Rotation: [α]₂₀ = - 128 @ 589 nm (c = 1, MeOH).
   C₂₆H₃₃N₃O₄ (MW = 451.571); mass spectroscopy (MH+) 452
Isomer 2: Retention time 5.52 minutes.
   Optical Rotation: [α]₂₀ = 26 @ 589 nm (c = 1, MeOH).
   C₂₆H₃₃N₃O₄ (MW = 451.571); mass spectroscopy (MH+) 452

### Step B. Synthesis of (S)- and (R)-5-(L-tert-Leucinyl)-amino-7-methyl-5,7-dihydro-6H-dibenz[b,d]azepin-6-one Hydrochloride

The compounds isolated in Part A (each isomer separately) were dissolved in dioxane and treated with excess HCl (g). After stirring for 17 hours, the title compounds were isolated as colorless solids after evaporation and vacuum drying.
Isomer 1:
   C₂₁H₂₅N₃O₂.HCl (MW = 387.91); mass spectroscopy (MH+ free base) 352.
   Optical Rotation: [α]₂₀= - 34 @ 589 nm (c = 1, MeOH).
Isomer 2:
   C₂₁H₂₅N₃O₂.HCl (MW = 387.91); mass spectroscopy (MH+ free base) 352.
   Optical Rotation: [α]₂₀ = 108 @ 589 nm (c = 1, MeOH).

### Example 7-E

### Synthesis of 5-(N-Boc-Amino)-5,7-dihydro-6H,7H-dibenz[b,d]azepin-6-one

### Step A - Synthesis of 5-Iodo-5,7-dihydro-6H-dibenz[b,d]azepin-6-one

A solution of 5,7-dihydro-6H-dibenz[b,d]azepin-6-one (1.0 g, 4.77 mmol) (Example 7-A) and Et₃N (2.66 ml, 19.12 mmol) were stirred for 5.0 minutes at -15°C in CH₂Cl₂ and treated with TMSI (1.36 ml, 9.54 mmol). After stirring for 15 minutes I₂ (1.81 g, 7.16 mmol) was added in a single portion and the reaction allowed to warm to 5-10°C over 3 h. The reaction was quenched with sat. Na₂SO₃, diluted with CH₂Cl₂ and separated. The organics were washed with Na₂SO₃ and NaHSO₃ and dried over MgSO₄. After filtration, the organics were concentrated to approximately 20 ml and diluted with an additional 20 ml of hexanes. The title compound was isolated as a tan precipitate by filtration.

### Step B - Synthesis of 5-Azido-5,7-dihydro-6H-dibenz[b,d]azepin-6-one

The iodide isolate above was dissolved in DMF and treated with 1.2 equivalents of NaN₃. After stirring 17 h at 23°C the mixture was diluted with EtOAc/H₂O, separated, washed with brine and dried over MgSO₄. The title compound was triturated from hot EtOAc as a tan powder.

### Step C - Synthesis of 5-(N-Boc-Amino)-5,7-dihydro-6H,7H-dibenz[b,d]azepin-6-one

The azide was dissolved in THF/H₂O and stirred at 23°C for 17 h in the presence of 3.0 equivalents of Ph₃P. The reaction was diluted with 50 % HOAc/toluene, separated, the aqueous layer extracted with toluene and evaporated to an oily residue. This was taken to pH 7.0 by the addition of 1 N NaOH, the resulting HOAc salt was collected and vacuum, dried. Finally, the compound was treated with Boc anhydride (1.05 equivalents) and Et₃N (2.1 equivalents) in THF. After stirring for 5 h at 23°C the reaction was filtered and the title compound isolated as a colorless powder.

### Example 7-F

### Synthesis of 5-Amino-7-(2-methylpropyl)-5,7-dihydro-6H-dibenz[b,d]azepin-6-one Hydrochloride

### Step A - Synthesis of 5-(N-Boc-Amino)-7-(2-methylpropyl)-5,7-dihydro-6H-dibenz[b,d]azepin-6-one

A solution of 5-(N-Boc-amino)-5,7-dihydro-6H-dibenz[b,d]azepin-6-one (0.2g, 0.617 mmol) (Example 7-E) in DMF was treated with Cs₂CO₃ (0.22 g, 0.678 mmol) and warmed to 60°C. To the reaction mixture was added 1-iodo-2-methylpropane (0.078 ml, 0.678 mmol) and stirring continued for 17 h. After cooling to 23 °C the mixture was diluted with CH₂Cl₂, washed with several portions of brine and dried over Na₂SO₄. The title compound was purified by chromatography (SiO₂, CHCl₃/MeOH 9:1).
C₂₃H₂₈N₂O₃ (MW = 380.41); mass spectroscopy (MH+) 381
Anal. Calcd for C₂₃H₂₈N₂O₃; C, 72.61 H, 7.42 N, 7.36. Found: C, 72.31 H, 7.64 N,7.17.

### Step B - Synthesis of 5-Amino-7-(2-methylpropyl)-5,7-dihydro-6H-dibenz[b,d]azepin-6-one Hydrochloride

The compound isolated in Part A was deprotected in dioxane saturated with gaseous HCl. The title compound was isolated as a slightly colored solid after evaporation and vacuum drying.

### Example 7-G

### Synthesis of 5-Amino-7-(methoxyacetyl)-5,7-dihydro-6H-dibenz[b,d]azepin-6-one Hydrochloride

### Step A- Synthesis of 5-(N-Boc-Amino)-7-(methoxyacetyl)-5,7-dihydro-6H-dibenz[b,d]azepin-6-one

A solution of 5-(N-Boc-amino)-5,7-dihydro-6H-dibenz[b,d]azepin-6-one (1.03, 3.08 mmol) (Example 7-E) in DMF was treated with Cs₂CO₃ (1.10 g, 3.39 mmol) and warmed to 60°C. To the reaction mixture was added bromomethyl acetate (0.321 ml, 3.39 mmol) (Aldrich) and stirring continued for 17 h. After cooling to 23 °C the mixture was diluted with CH₂Cl₂, washed with several portions of brine and dried over Na₂SO₄. The title compound was purified by chromatography (SiO₂, CHCl₃).
C₂₂H₂₄N₂O₅ (MW = 396.44); mass spectroscopy (MH+) 397
Anal. Calcd for C₂₂H₂₄N₂O₅; C, 66.65 H, 6.10 N, 7.07. Found: C, 66.28 H, 5.72 N, 6.50.

### Step B - Synthesis of 5-Amino-7-(methoxyacetyl)-5,7-dihydro-6H-dibenz[b,d]azepin-6-one Hydrochloride

The compound isolated in Part A was deprotected in dioxane saturated with gaseous HCl. The title compound was isolated as a colorless solid after evaporation and vacuum drying.
C₁₇H₁₆N₂O₃ HCl (MW = 332.78); mass spectroscopy (MH+ free base) 297.

### Example 7-H

### Synthesis of 5-Amino-7-(3,3-dimethyl-2-butanonyl)-5,7-dihydro-6H-dibenz[b,d]azepin-6-one Hydrochloride

### Step A- Synthesis of 5-(N-Boc-Amino)-7-(3,3-dimethyl-butanonyl)-5,7-dihydro-6H-dibenz[b,d]azepin-6-one

A solution of 5-(N-Boc-amino)-5,7-dihydro-6H-dibenz[b,d]azepin-6-one (0.2 g, 0.617 mmol) (Example 7-E) in DMF was treated with Cs₂CO₃ (0.3 g, 0.925 mmol) and warmed to 60°C. To the reaction mixture was added 1-chloro-3,3-dimethyl-2-butanone (0.096 ml, 0.74 mmol) (Aldrich) and stirring continued for 17 h. After cooling to 23 °C, the mixture was diluted with CH₂Cl₂, washed with several portions of brine and dried over Na₂SO₄. The title compound was isolated as a colorless solid.
C₂₅H₃₀N₂O₄ (MW = 422.522); mass spectroscopy (MH+) 423

### Step B - Synthesis of 5-Amino-7-(3,3-dimethyl-2-butanonyl)-5,7-dihydro-6H-dibenz[b,d]azepin-6-one Hydrochloride

The compound isolated in Part A was deprotected in dioxane saturated with gaseous HCl. The title compound was isolated as a colorless solid after evaporation and vacuum drying.

### Example 7-I

### Synthesis of L-Alaninyl-5-amino-7-methyl-5,7-dihydro-6H-dibenz[b,d]azepin-6-one Hydrochloride

Step A: Following General Procedure D and using N-t-Boc-L-alanineand 5-amino-7-methyl-5,7-dihydro-6H-dibenz[b,d]azepin-6-one, N-t-Boc-L-alaninyl-5-amino-7-methyl-5,7-dihydro-6H-dibenz[b,d]azepin-6-one was prepared.
Step B: Following General Procedure 8-N and using the N-t-Boc-L-alaninyl-5-amino-7-methyl-5,7-dihydro-6H-dibenz[b,d]azepin-6-one, the title compound was prepared. Other substituted N-t-Boc-L-alaninyl-5-amino-7-methyl-5,7-dihydrb-6H-dibenz[b,d]azepin-6-ones can also be prepared by this procedure.

### Example 7-J

### Synthesis of L-Valinyl-5-amino-7-methyl-5,7-dihydro-6H-dibenz[b,d]azepin-6-one Hydrochloride

Step A: Following General Procedure D and using N-t-Boc-L-valine and 5-amino-7-methyl-5,7-dihydro-6H-dibenz[b,d]azepin-6-one, N-t-Boc-L-valinyl-5-amino-7-methyl-5,7-dihydro-6H-dibenz[b,d]azepin-6-one was prepared.
Step B: Following General Procedure 8-N and using the N-t-Boc-L-valinyl-5-amino-7-methyl-5,7-dihydro-6H-dibenz[b,d]azepin-6-one, the title compound was prepared. Other substituted N-t-Boc-L-valinyl-5-amino-7-methyl-5,7-dihydro-6H-dibenz[b,d]azepin-6-ones can also be prepared by this procedure.

### Example 7-K

### Synthesis of 5-Amino-7-phenbutyl-5,7-dihydro-6H-dibenz[b,d]azepin-6-one

Following General Procedure 7-A and using 5,7-dihydro-6H-dibenz[b,d]azepin-6-one (prepared as described in Brown, et. al., Tetrahedron Letters, No. 8, 667-670, (1971) and references cited therein) and 1-chloro-4-phenylbutane (Aldrich), the title compound was prepared.

### Example 7-L

### Synthesis of 5-Amino-7-cyclopropymethyl-5,7-dihydro-6H-dibenz[b,d]azepin-6-one

Following General Procedure 7-A and using 5,7-dihydro-6H-dibenz[b,d]azepin-6-one (prepared as described in Brown, et. al., Tetrahedron Letters, No. 8, 667-670, (1971) and references cited therein) and (bromomethyl)cyclopropane (Aldrich), the title compound was prepared.

### Example 7-M

### Synthesis of 5-Amino-7-(2',2,2'-trifluoroethyl)-5,7-dihydro-6H-dibenz[b,d]azepin-6-one

Following General Procedure 7-A and using 5,7-dihydro-6H-dibenz[b,d]azepin-6-one (prepared as described in Brown, et. al., Tetrahedron Letters, No. 8, 667-670, (1971) and references cited therein) and 1-bromo-2,2,2-trifluoroethane (Aldrich), the title compound was prepared.

### Example 7-N

### Synthesis of 5-Amino-7-cyclohexyl-5,7-dihydro-6H-dibenz[b,d]azepin-6-one

Following General Procedure 7-A and using 5,7-dihydro-6H-dibenz[b,d]azepin-6-one (prepared as described in Brown, et. al., Tetrahedron Letters, No. 8, 667-670, (1971) and references cited therein) and bromocyclohexane (Aldrich), the title compound was prepared.

### Example 7-O

### Synthesis of 5-(L-Alaninyl)amino-9-fluoro-7-methyl-5,7-dihydro-6H-dibenz[b,d]azepin-6-one Hydrochloride

Step 1: 2-Bromo-5-fluorotoluene was stirred in THF at -78C. s-BuLi (1.05 eq., 1.3 M in cyclohexane) was slowly added and the mixture was stirred for 45 minutes. Trimethylborate (1.5 eq) was added and the mixture was allowed to warm to ambient temperature. After stirring for 1 hour, pinacol (2 eq.) was added. The mixture was stirred for 16 hours then was concentrated under reduced pressure. The resulting residue was slurried in CH₂Cl₂ and filtered through Celite. The filtrate was concentrated to yield an oil which was purified by chromatography on deactivated silica gel (Et₃N) to yield the arylboronate ester.
Step 2: 2-Bromoaniline (1 eq.) and di-t-butyl-dicarbonate (1.1 eq.) were stirred at 80°C for 20 hours. The resulting mixture was allowed to cool and was directly distilled using house vacuum to provide N-t-Boc-2-bromoaniline.
Step 3: N-t-Boc-2-bromoaniline (Step 2, 1 eq.), the arylboronate ester (Step 1, 1.1 eq.), K₂CO₃ (1.1 eq.) and tetrakis(triphenylphosphine)palladium(0) (0.02 eq) were stirred in 20% water/dioxane under nitrogen. The solution was heated at reflux for 10 hours. The mixture was allowed to cool then was concentrated. The resulting residue was partitioned between water and chloroform. The organic portion was dried and concentrated to yield an oil which was purified by silica gel chromatography using 1:1 CH₂Cl₂/hexanes.
Step 4: Following General Procedure 7-B and using the substituted biphenyl from step 3, the 9-fluoro-5,7-dihydro-6H-dibenz[b,d]azepin-6-one was prepared.
Step 5: 9-Fluoro-5,7-dihydro-6H-dibenz[b,d]azepin-6-one (1 eq., Step 4), cesium carbonate (1.1 eq., Aldrich) and methyl iodide (1.1 eq., Aldrich) were stirred in dry DMF at ambient temperature for 16 hours. The mixture was concentrated under reduced pressure to provide a residue which was partitioned between EtOAc and water. The organic portion was dried and concentrated to yield an oil which was purified by silica gel chromatography to 9-fluoro-7-methyl-5,7-dihydro=6H-dibenz[b,d]azepin-6-one.
Step 6: Following General Procedure 7-A, Step B and 9-fluoro-7-methyl-5,7-dihydro-6H-dibenz[b,d]azepin-6-one from Step 5, 5-amino-9-fluoro-7-methyl-5,7-dihydro-6H-dibenz[b,d]azepin-6-one was prepared.
Step 7: Following the procedure of Example 7-1 and using 5-amino-9-fluoro-7-methyl-5,7-dihydro-6H-dibenz[b,d]azepin-6-one from Step 6, the title compound was prepared.

### Example 7-P

### Synthesis of 5-(L-Alaninyl)amino-13-fluoro-7-methyl-5,7-dihydro-6H-dibenz[b,d]azepin-6-one Hydrochloride

Following the procedure of Example 7-O and using 2-bromo-4-fluoroaniline (Step 2, Lancaster) and o-tolylboronic acid (Step 3, Aldrich), the title compound was prepared.

### Example 7-Q

### Synthesis of 5-(L-Alaninyl)amino-10-fluoro-7-methyl-5,7-dihydro-6H-dibenz[b,d]azepin-6-one Hydrochloride

Following the procedure of Example 7-O and using 2-bromo-4-fluorotoluene (Step 1), the title compound was prepared.

### Example 7-R

### Synthesis of 5-(L-Alanyl)-amino-7-cyclopropylmethyl-5,7-dihydro-6H-dibenz[b,d]azepin-6-one Hydrochloride

Following the procedure of Example 7-1 and using 5-amino-7-cyclopropylmethyl-5,7-dihydro-6H-dibenz[b,d]azepin-6-one (Example 7-L), the title compound was prepared.

### Example 7-S

### Synthesis of 5-(L-Alaninyl)amino-7-phenbutyl-5,7-dihydro-6H-dibenzib,diazepin-6-one Hydrochloride

Following the procedure of Example 7-1 and using 5-amino-7-phenbutyl-5,7-dihydro-6H-dibenz[b,d]azepin-6-one (Example 7-K), the title compound was prepared.

### Example 7-T

### Synthesis of 5-(L-Valinyl)amino-7-cyclopropylmethyl-5,7-dibydro-6H-dibenzib,djazepin-6-one Hydrochloride

Following the procedure of Example 7-J and using 5-amino-7-cyclopropylmethyl-5,7-dihydro-6H-dibenz[b,d]azepin-6-one (Example 7-L), the title compound was prepared.

### Example 7-U

### Synthesis of 5-(L-Valinyl)amino-7-phenbutyl-5,7-dihydro-6H-dibenz[b,d]azepin-6-one Hydrochloride

Following the procedure of Example 7-J and using 5-amino-7-phenbutyl-5,7-dihydro-6H-dibenz[b,d]azepin-6-one (Example 7-U), the title compound was prepared.

### Example 7-V

### Synthesis of 5-(L-Valinyl)amino-7-hexyl-5,7-dihydro-6H-dibenz[b,d]azepin-6-one Hydrochloride

Step A: Following General Procedure 7-A and using 5,7-dihydro-6H-dibenz[b,d]azepin-6-one (prepared as described in Brown, et. al., Tetrahedron Letters, No. 8, 667-670, (1971) and references cited therein) and 1-bromohexane (Aldrich), 5-amino-7-hexyl-5,7-dihydro-6H-dibenz[b,d]azepin-6-one was prepared.
Step B: Following the procedure of Example 7-J and using 5-amino-7-hexyl-5,7-dihydro-6H-dibenz[b,d]azepin-6-one, the title compound was prepared.

### Example 7-W

### Synthesis of 5-(L-Valinyl)amino-10-fluoro-7-methyl-5,7-dihydro-6H-dibenz[b,d]azepin-6-one Hydrochloride

Following the procedure of Example 7-J and using 5-amino-10-fluoro-7-methyl-5,7-dihydro-6H-dibenz[b,d]azepin-6-one (as prepared in Example 7-Q, the title compound was prepared.

### Example 7-X

### Synthesis of 5-(L-Valinyl)amino-13-fluoro-7-methyl-5,7-dihydro-6H-dibenz[b,d]azepin-6-one Hydrochloride

Following the procedure of Example 7-J and using the 5-amino-13-fluoro-7-methyl-5,7-dihydro-6H-dibenz[b,d]azepin-6-one (as prepared in Example 7-P), the title compound was prepared.

### Example 7-Y

### Synthesis of 5-(L-Valinyl)amino-13-fluoro-7-methyl-5,7-dihydro-6H-dibenz[b,d]azepin-6-one Hydrochloride

Following the procedure of Example 7-J and using the 5-amino-9-fluoro-7-methyl-5,7-dihydro-6H-dibenz[b,d]azepin-6-one (as prepared in Example 7-O), the title compound was prepared.

### Example 7-Z

### Synthesis of (5-Amino-7-methyl-1,2,3,4,5,7-hexahydro-6H-dicyclohexyl[b,d]azepin-6-one

The 5-amino-7-methyl-5,7-dihydro-6H-dibenz[b,d]azepin-6-one hydrochloride (Example 7-A) was dissolved in a 1:1 mixture of EtOAc/HOAc. 5% Rh/C was added and the mixture was stirred at 60°C under 60 psi of hydrogen. After 3 days, the mixture was filtered and the filtrate was concentrated to provide an oil which was purified by SCX-cation exchange chromatography to yield the title compound.

### Example 7-AA

### Synthesis of 5-(S)-Amino-7-methyl-5,7-dihydro-6H-dibenz[b,d]azepin-6-one Hydrochloride

Following General Procedure 7-C using racemic 5-amino-7-methyl-5,7-dihydro-6H-dibenz[b,d]azepin-6-one (1.0 eq.) and di-*p*-toluoyl-D-tartaric acid monohydrate (1.0 eq.) in methanol, the title compound was prepared as a solid. The product was collected by filtration. Enantiomeric excess was determined by chiral HPLC.
Desired enantiomer 1: retention time of 9.97 minutes.
Undesired enantiomer 2: retention time of 8.62 minutes.
NMR data was as follows:
¹H-nmr (CDCl₃): δ = 9.39 (s, 2H), 7.75-7.42 (m, 8H), 4.80 (s, 1H), 3.30 (s. 3H).
C₁₅H₁₅ClN₂O (MW = 274.75); mass spectroscopy (MH⁻) 239.1.
Anal Calcd for C₁₅H₁₅ClN₂O₃; C, 65.57; H, 5.50; N, 10.20; Found: C, 65.51, H, 5.61; N, 10.01.

### Example 7-1

### Synthesis of 5-(S)-[N'(3,5-Difluorophenylacetyl)-L-alaninyl]amino-7-methyl-5,7-dihydro-6H-dibenz[b,d]azepin-6-one

Following General Procedure D above using N-(3,5-difluorophenylacetyl)-L-alanine (Example B) and 5-amino-7-methyl-5,7-dihydro-6H-dibenz[b,d]azepin-6-one hydrochloride (Example 7-A), the title compound was prepared as a colorless solid. The diastereomers were purified by HPLC (Bulk OD-25) using 15% EtOH in heptane as eluent and a flow rate of 1.5 ml/min.
Isomer 1: retention time of 11.4 minutes.
   NMR data was as follows:
   ¹H-nmr (CDCl₃): δ = 7.62-7.33. (m, 8H), 6.79 (m, 2H), 6.71 (m,1H), 6.47 (m,1H), 5.24 (d 1H), 4.70 (m,1H), 3.48 (s, 2H), 3.34 (s, 3H), 1.42 (d, 3H).
   Optical Rotation: [α]₂₀ = - 125 @ 589 nm (c = 1, MeOH).
   C₂₆H₂₃F₂N₃O₃ (MW = 463.49); mass spectroscopy (MH+) 463.
   Anal. Calcd for C₂₆H₂₃F₂N₃O₃; C, 67.38 H, 5.00 N, 9.06. Found: C, 67.49 H, 5.06 N, 8.93.

### Example 7-2

### Synthesis of 5-(S)-[N'-((S)-3,5-Difluorophenyl-α-hydroxyacetyl)-L-alaninyl]amino-7-methyl-5,7-dihydro-6H-dibenz[b,d]azepin-6-one and 5-(S)-[N'-((R)-3,5-Difluorophenyl-α-hydroxyacetyl)-L-alaninyl]amino-7-methyl-5,7-dihydro-6H-dibenz[b,d]azepin-6-one

Following General Procedure D above using 3,5-difluoromandelic acid and 5-(S)-[L-alaninyl]-amino-7-methyl-5,7-dihydro-6H-dibenz[b,d]azepin-6-one hydrochloride (Example 7-B), the title compound was prepared as a colorless solid. The diastereomers were purified by flash chromatography using 98:2 CHCl₃/MeOH.
Isomer 1:
   NMR data was as follows:
   ¹H-nmr (CDCl₃): δ = 7.67 (d, 1H), 7.60-7.28 (m, 8H), 7.15 (d, 1H), 6.98 (m, 2H), 6.74 (m,1H), 5.21 (d,1H), 4.94 (d,1H), 4.61 (m,1H), 4.56 (m, 1H), 3.34 (s, 3H), 1.42 (d, 3H).
   Optical Rotation: [α]₂₀ = - 121 @ 589 nm (c = 1, MeOH).
   C₂₆H₂₃F₂N₃O₄ (MW = 479.488); mass spectroscopy (MH+) 479.
   Anal. Calcd for C₂₆H₂₃F₂N₃O₄; C, 65.13 H, 4.83 N, 8.76. Found: C, 65.42 H, 4.73 N, 8.65.
Isomer 2:
   NMR data was as follows:
   ¹H-nmr (CDCl₃): δ = 7.78 (d, 1H), 7.66 (d, 1H), 7.54-7.28 (m, 8H), 6.89 (m, 2H), 6.71 (m, 2H), 5.22 (d 1H), 4.92 (m,1H), 4.65 (m,1H), 4.01 (m, 1H), 3.37 (s, 3H), 1.39 (d, 3H).
   Optical Rotation: [α]₂₀ = - 146 589 nm (c = 1, MeOH).
   C₂₆H₂₃F₂N₃O₄ (MW = 479.488); mass spectroscopy (MH+) 479.
   Anal. Calcd for C₂₆H₂₃F₂N₃O₄; C, 65.13 H, 4.83 N, 8.76. Found: C, 65.18, 4.82, 8.65.

### Example 7-3

### Synthesis of 5-(S)-[N'-(3,5-Difluorophenyl-α-ketoacetyl)-L-alaninyl]amino-7-methyl-5,7-dihydro-6H-dibenz[b,d]azepin-6-one

Following the Jones oxidation procedure (Fieser and Fieser, Reagents for Organic Synthesis, Vol. 1, p. 142) using 5-(S)-[((S/R)-3,5-difluorophenyl-α-hydroxyacetyl)-L-alaninyl]-amino-7-methyl-5,7-dihydro-6H-dibenz[b,d]azepin-6-one (Example 7-2), the title compound was prepared as a colorless solid.
NMR data was as follows:
¹H-nmr (CDCl₃): δ = 7.92 (m, 2H), 7.61-7.35 (m, 8H), 7.08 (m, 1H), 5.31 (d,1H), 4.74 (m,1H), 3.38 (s, 3H), 1.56 (d, 3H).
C₂₆H₂₁F₂N₃O₄ (MW = 477.472); mass spectroscopy (MH+) 477.
Anal. Calcd for C₂₆H₂₁F₂N₃O₄; C, 65.40 H, 4.43 N, 8.80. Found: C, 65.66 H, 4.71 N, 8.54.

### Example 7-4

### Synthesis of 5-(S)-[N'-(3,5-difluorophenylacetyl)-L-valinyl]amino-7-methyl-5,7-dihydro-6H-dibenz[b,d]azepin-6-one

Following General Procedure D above using 3,5-difluorophenylacetic acid and 5-(S)-[L-valinyl]-amino-7-methyl-5,7-dihydro-6H-dibenz[b,d]azepin-6-one hydrochloride (Example 7-C), the title compound was prepared as a colorless solid. The product was purified by flash chromatography using 98:2 CHCl₃/MeOH.
NMR data was as follows:
¹H-nmr (CDCl₃): δ = 7.54-7.25 (m, 8H), 6.74 (m, 2H), 6.74 (m, 2H), 6.70 (m, 1H), 6.49 (d, 1H), 5.26 (d, 1H), 4.49 (m, 1H), 3.43 (s, 2H), 3.35 (s, 3H), 2.06 (m, 1H), 0.91 (m, 6H).
Optical Rotation: [α]₂₀ = - 144 @ 589 nm (c = 1, MeOH).
C₂₈H₂₇F₂N₃O₃ (MW = 491.543); mass spectroscopy (MH+) 490.9
Anal. Calcd for C₂₈H₂₇F₂N₃O₃; C, 68.42 H, 5.54 N, 8.55. Found: C, 68.51 H, 5.82, N, 8.61.

### Example 7-5

### Synthesis of 5-(S)-[N'-(3,5-difluorophenylacetyl)-L-tert-leucinyl] amino-7-methyl-5,7-dihydro-6H-dibenz[b,d]-azepin-6-one

Following General Procedure D above using 3,5-difluorophenylacetic acid (Oakwood) and 5-(S)-[L-*tert*-leucinyl]-amino-7-methyl-5,7-dihydro-6H-dibenz[b,d]azepin-6-one hydrochloride (Example 7-D), the title compound was prepared as a colorless solid. The product was purified by flash chromatography using 98:2 CHCl₃/MeOH.
NMR data was as follows:
¹H-nmr (CDCl₃): δ = 7.58-7.36 (m, 9H), 6.80 (m, 2H), 6.72 (m, 1H), 6.25 (d, 1H), 5.27 (d, 1H), 4.52 (d, 1H), 3.53 (s, 2H), 3.35 (s, 3H), 0.97 (m, 9H).
Optical Rotation: [α]₂₀ = - 137@ 589 nm (c = 1, MeOH)
C₂₉H₂₉F₂N₃O₄, (MW = 505.57); mass spectroscopy (MH+) 504.9
Anal. Calcd for C₂₈H₂₇F₂N₃O₄•H₂O; C, 66.52 H, 5.92 N, 8.02. Found: C, 66.39 H, 5.76, N, 7.79.

### Example 7-6

### Synthesis of 5-(S)-[N'-((S)-3,5-Difluorophenyl-α-hydroxyacetyl)-L-valinyl]amino-7-methyl-5,7-dihydro-6H-dibenz[b,d]azepin-6-one

Following General Procedure D above using (S)-3,5-difluoromandelic acid and 5-(S)-[L-valinyl]-amino-7-methyl-5,7-dihydro-6H-dibenz[b,d]azepin-6-one hydrochloride (Example 7-C), the title compound was prepared as a colorless solid: The product was purified by flash chromatography using 98:2 CHCl₃/MeOH.
NMR data was as follows:
¹H-nmr (CDCl₃): δ = 7.78 (d, 1H), 7.53-7.25 (m, 8H), 6.86 (m, 2H), 6.71 (m, 2H), 5.22 (d, 1H), 4.76 (s, 1H) 4.43 (m, 1H), 3.34 (s, 3H), 2.08 (m, 1H), 0.91 (m,6H).
C₂₈H₂₇F₂N₃O₄ (MW = 507.542); mass spectroscopy (MH+) 506.9
Anal. Calcd for C₂₈H₂₇F₂N₃O₄; C, 66.26 H, 5.32 N, 8.27. Found: C, 66.08 H, 5.62, N, 7.97.

### Example 7-7

### Synthesis of 5-(S)-[N'((S)-3,5-difluorophenyl-α-hydroxyacetyl)-L-tert-leucinyl]amino-7-methyl-5,7-dihydro-6H-dibenz[b,d]azepin-6-one

Following General Procedure D above using (S)-3,5-difluoromandelic acid and 5-(S)-[L-*tert*-leucinyl]-amino-7-methyl-5,7-dihydro-6H-dibenz[b,d]azepin-6-one hydrochloride (Example 7-D), the title compound was prepared as a colorless solid. The product was purified by flash chromatography using 98:2 CHCl₃/MeOH.
NMR data was as follows:
¹H-nmr (CDCl₃): δ = 7.67 (d, 1H), 7.54-7.25 (m, 8H), 6.83 (m, 2H), 6.69 (m, 2H), 5.22 (d, 1H), 4.74 (s, 1H) 4.44 (d, 1H), 3.35 (s, 3H), 0.97 (m, 9H).
C₂₉H₂₉F₂N₃O₄ (MW = 521.569); mass spectroscopy (MH+) 520.9
Anal. Calcd for C₂₉H₂₉F₂N₃O₄; C, 66.78 H, 5.60 N, 8.06. Found: C, 66.56 H, 5.85, N, 7.83.

### Example 7-8

### Synthesis of 5-[N'-(3,5-difluorophenylacetyl)-L-alaninyl]amino-7-(methoxyacetyl)-5,7-dihydro-6H-dibenz[b,d]azepin-6-one

Following General Procedure D and using N-(3,5-difluorophenylacetyl)-L-alanine (Example B) and 5-amino-7-(methoxyacetyl)-5,7-dihydro-6H-dibenz[b,d]azepin-6-one hydrochloride (Example 7-G), the title compound was prepared as a colorless solid. The product was purified by flash chromatography. -
NMR data was as follows:
¹H-nmr (CDCl₃): δ = 7.61-7.215 (m, 8H), 6.76 (m, 2H), 6.68 (m, 1H), 6.53 and 6.40 (two d, 1 H), 5.32 (d, 1 H), 4.71 (m, 1 H) 4.37-(m. 2H), 3.69 (s, 3H), 1.49 and 1.39 (two d, 3H).
C₂₈H₂₅F₂N₃O₅ (MW = 521.518); mass spectroscopy (MH+) 522
Anal. Calcd for C₂₈H₂₅F₂N₃O₅.1.5 mol H₂O; C, 61.30 H, 4.55 N, 7.65. Found: C, 61.30 H, 4.53, N, 7.68.

### Example 7-9

### Synthesis of 5-[N'-(3,5-Difluorophenylacetyl)-L-alaninyl]amino-7-(methylcarboxylate)-5,7-dihydro-6H-dibenz[b,d]azepin-6-one

Following General Procedure II-A, Method B and using 5-[(3,5-difluorophenylacetyl)-L-alaninyl]-amino-7-(methoxyacetyl)-5,7-dihydro-6H-dibenz[b,d]azepin-6-one (Example 7-8), the title compound was prepared as a colorless solid. The product was purified by flash chromatography.
C₂₇H₂₃F₂N₃O₅ (MW = 507.49); mass spectroscopy (MH+) 508
Anal. Calcd for C₂₇H₂₃F₂N₃O₅ .2 mol H₂O; C, 59.66 H, 4.23 N, 7.72. Found: C, 59.88 H, 4.29, N, 7.66.

### Example 7-10

### Synthesis of 5-[N'-(3,5-difluorophenylacetyl)-L-alaninyl]amino-7-(3,3-dimethyl-2-butanoyl)-5,7-dihydro-6H-dibenz[b,d]azepin-6-one

Following General Procedure D and using N-(3,5-difluorophenylacetyl)-L-alanine (Example B) and 5-amino-7-(3,3-dimethyl-2-butanoyl)-5,7-dihydro-6H-dibenz[b,d]azepin-6-one hydrochloride (Example 7-H), the title compound was prepared as a colorless solid. The product was purified by flash chromatography.
NMR data was as follows:
¹H-nmr (CDCl₃): δ = 7.57 (m, 3H), 7.41 (m, 5H), 7.14 (m, 1H), 6.78 (m, 2H), 6.68 (m, 1 H), 6.44 and 6.26 (two d, 1 H), 5.34(d, 1 H), 4.68 (m, 1H) 4.59 (m, 2H), 3.52 and 3.47 (two s, 2H), 1.52 and 1.42 (two d, 3H), 1.23 (s, 9H).
C₃₁H₃₁F₂N₃O₄ (MW = 547.599); mass spectroscopy (MH+) 548
Anal. Calcd for C₃₁H₃₁F₂N₃O₄ .0.5 mol H₂O; C, 66.89 H, 5.59 N, 7.54. Found: C, 66.52 H, 5.73, N, 7.18.

### Example 7-11

### Synthesis of 5-[N'-(3,5-difluorophenylacetyl)-L-alaninyl]amino-7-(morpholinylacetyl)-5,7-dihydro-6H-dibenz[b,d]azepin-6-one

Following General Procedure D using 5-[N'-(3,5-difluorophenylacetyl)-L-alaninyl]-amino-7-(methylcarboxylate)-5,7-dihydro-6H-dibenz[b,d]azepin-6-one (Example 7-9) and morpholine (Aldrich), the title compound was prepared as a colorless foam. The product was purified by flash chromatography.
NMR data was as follows:
¹H-nmr (CDCl₃): δ = 7.57-7.37 (m, 8H), 6.81-6.69 (m, 3H), 5.35 (m, 1H), 4.73- 4.67 (m, 2H), 4.17 (m, 1H). 3.66-3.26 (m, 10 H), 1.46 and 1.40 (two d, 3H).
C₃₁H₃₀F₂N₄O₅ (MW = 576.592); mass spectroscopy (MH+) 577
Anal. Calcd for C₃₁H₃₀F₂N₄O₅ .0.5 mol H₂O; C, 63.57 H, 5.12 N, 9.56. Found: C, 63.41 H, 5.51, N, 8.92.

### Example 7-12

### Synthesis of 5-(S)-(N'-((S)-(+)-2-Hydroxy-3-methylbutyryl)-L-alaninyl)amino-7-methyl-5,7-dihydro-6H-dibenz[b,d]azepin-6-one

Following General Procedure H using (S)-(+)-2-hydroxy-3-methylbutyric acid (Aldrich) and 5-S-(L-alaninyl)-amino-7-methyl-5,7-dihydro-6H-dibenz[b,d]azepin-6-one (Example 7-B), the title compound was prepared as a white solid. The product was purified by silica gel chromatography using gradient elution of MeOH/CH₂Cl₂ (1:99 - 3:97).
NMR data was as follows:
¹H-nmr (CDCl₃): δ = 7.94 (d, J = 7.0 Hz, 1H), 7.55-7.22 (m, 9H), 5.25 (d, J = 7.5 Hz, 1 H), 4.79-4.75 (m, 1 H), 3.83 (d, J = 3.1 Hz, 1H), 3.78 (br s, 1H), 3.32 (s, 3H), 2.08-2.01 (m, 1H), 1.36 (d, J = 7.0. Hz, 3H), 0.83 (d, J = 7.0 Hz, 3H), 0.76 (d, J = 6.5 Hz, 3H).
C₂₃H₂₇N₃O₄ (MW = 409.48); mass spectroscopy (MH⁻) 410.4.
Anal Calcd for C₂₃H₂₇NO₄, C, 67.46; H, 6.65; N, 10.26; Found: C, 67.59; H, 6.66; N, 10.34.

### Example 7-13

### Synthesis of 5-[N'-Cyclopentyl-α-hydroxyacetyl)-L-valinyl]amino-7-methyl-5,7-dihydro-6H-dibenz[b,d]azepin-6-one

Following General Procedure D above using cyclopentyl-α-hydroxyacetic acid (Example P) and 5-(S)-[L-valinyl]-amino-7-methyl-5,7-dihydro-6H-dibenz[b,d]azepin-6-one hydrochloride (Example 7-C), the title compound was prepared as a colorless solid. The product was purified by flash chromatography using 98:2 CHCl₃/MeOH.
C₂₇H₃₃N₃O₄ (MW = 463.5); mass spectroscopy (MH+) 464.
Anal. Calcd for C₂₇H₃₃N₃O₄; C, 69.96 H, 7.18 N, 9.06. Found: C, 69.72 H, 6.99, N, 8.91.

### Example 7-14

### Synthesis of 5-(S)-(N'-((S)-3,3-dimethyl-2-hydroxybutyryl)-L-alaninyl)amino-7-methyl-5,7-dihydro-6H-dibenz[b,d]azepin-6-one and 5-(S)-(N'-((R)-3,3-dimethyl-2-hydroxybutyl-yl)-L-alaminyl)amino-7-methyl-5,7-dihydro-6H-dibenz[b,d]azepin-6-one

Following General Procedure H using 2-hydroxy-3,3-dimethylbutyric acid (Aldrich) and 5-(S)-(L-alaninyl)-amino-7-methyl-5,7-dihydro-6H-dibenz[b,d]azepin-6-one (Example 7-B), the title compound was prepared as a white solid. The product was purified by silica gel chromatography using gradient elution of MeOH/CH₂Cl₂ (1:99 -3:97).
NMR data for isomer 1 was as follows:
¹H-nmr (CDCl₃): δ = 7.90 (d, J = 6.6 Hz, 1H), 7.57-7.24 (m, 8H), 6.99 (d, J = 7.5 Hz, 1H), 5.24 (d, J = 6.5 Hz, 1H), 4.83-4.76 (m, 1H), 3.69 (s, 1H), 3.32 (s, 3H), 3.19 (br s, 1H), 1.39 (d, J = 7.0 Hz, 3H), 0.96 (s, 9H).
C₂₄H₂₉N₃O₄ (MW = 423.51); mass spectroscopy (MH⁺) 424.1.
Anal Calcd for C₂₄H₂₉N₃O₄₍isomer 1), C, 68.07; H, 6.90; N, 9.92; Found: C, 68.22, H, 7.04; N, 9.91.
NMR data for isomer 2 was as follows:
¹H-nmr (CDCl₃): δ = 8.00-7.99 (m, 1H), 7.97-7.30 (m, 8H), 7.03-7.00 (m, 1H), 5.25 (d, J = 7.0 Hz, 1H), 4.82-4.75 (m, 1H), 3.69 (s, 1H), 3.33 (s, 3H), 2.66 (br s, 1H), 1.48 (d, J = 7.0 Hz, 3H), 0.98 (s, 9H).
C₂₄H₂₉N₃O₄ (MW = 423.51); mass spectroscopy (MH⁺) 424.1.
Anal Calcd for C₂₄H₂₉N₃O₄(isomer 2), C, 68.07; H, 6.90; N, 9.92; Found: C, 67.77, H, 7.08; N, 9.66.

### Example 7-15

### Synthesis of 5-[N'-Cyclopentyl-α-hydroxyacetyl)-L-tert-leucinyl]amino-7-methyl-5,7-dibydro-6H-dibenz[b,d]azepin-6-one

Following General Procedure D above using cyclopentyl-α-hydroxyacetic acid (Example P) and 5-(S)-[L-*tert*-leucinyl]-amino-7-methyl-5,7-dihydro-6H-dibenz[b,d]azepin-6-one hydrochloride (Example 7-D), the title compound was prepared as a colorless solid. The product was purified by flash chromatography using 98:2 CHCl₃/MeOH.
C₂₈H₃₅N₃O₄.(477.6); mass spectroscopy (MH+) 478.
Anal. Calcd for C₂₈H₃₅N₃O₄; C, 66.39 H, 5.57 N, 11.06. Found: C, 66.33 H, 5.67, N, 10.89.

### Example 7-16

### Synthesis of 5-[N'-Cyclopentyl-α-hydroxyacetyl)-L-alaninyl]amino-7-methyl-5,7-dihydro-6H-dibenz[b,d]azepin-6-one

Following General Procedure D above using cyclopentyl-α-hydroxyacetic acid (Example P) and 5-(S)-[L-alaninyl]-amino-7-methyl-5,7-dihydro-6H-dibenz[b,d]azepin-6-one hydrochloride (Example 7-B), the title compound was prepared as a colorless solid. The product was purified by flash chromatography using 99:1 CHCl₃/MeOH.
NMR data was as follows:
¹H-nmr (CDCl₃): δ = 7.78 (m, 2H), 7.62-7.28 (m, 8H), 7.08 and 6.99 (two d, 1H), 5.27 (d, 1H), 4.78 (m, 1H), 4.06 (m, 1H), 3.34 (s, 3H), 2.54 (m, 2H), 2.29 (m, 1H), 1.76-1.48 (m, 6H)1.43 (d, 3H).
C₂₅H₂₉N₃O₄.(435.52); mass spectroscopy (MH+) 436
Anal. Calcd for C₂₅H₂₉N₃O₄; C, 68.95 H, 6.71 N, 9.65. Found: C, 69.06 H; 6.89, N, 9.51.

### Example 7-17

### Synthesis of 5-[N'-(3,5-Difluorophenylacetyl)-L-alaninyl]-L-amino-5,7-dihydro-6H,7H-dibenz[b,d]azepin-6-one

Following General Procedure D above using N-(3,5-difluorophenylacetyl)-L-alanine (Example B) and 5-amino-5,7-dihydro-6H,7H-dibenz[b,d]azepin-6-one hydrochloride (prepared using the compound of Example 7-E, followed by Boc removal as in Example 7-B, Step B), the title compound was prepared as a. colorless solid. The product was purified by flash chromatography using 95:5 CHCl₃/MeOH.
NMR data was as follows:
¹H-nmr (DMSO_{d6}): δ = 8.86 (m, 1H), 8.75 (m, 1H), 8.49 (m, 1H), 7.78-7.23 (m, 8H), 7.09 (m, 1H), 7.03 (m, 2H), 5.07 (m, 1H), 4.60 (m, 1H), 3.55 (s, 2H), 1.32 (d, 3H).
C₂₅H₂₁F₂N₃O₃.(449.45); mass spectroscopy (MH+) 450.
Anal. Calcd for C₂₅H₂₁F₂N₃O_{3;} C, 66.81 H, 4.71 N, 9.35. Found: C, 67.11 H, 4.84, N, 9.09.

### Example 7-18

### Synthesis of 5-[N'-(3,5.Difluorophenylacetyl)-L-alaninyl]amino-7-(2-methylpropyl)-5,7-dihydro-6H-dibenz[b,d]azepin-6-one

Following General Procedure D above using N-(3,5-difluorophenylacetyl)-L-alanine (Example B) and 5-amino-7-(2-methylpropyl)-5,7-dihydro-6H-dibenz[b,d]azepin-6-one hydrochloride (Example 7-F), the title compound was prepared as a colorless solid. The product was purified by flash chromatography using 99:1 CHCl₃/MeOH.
NMR data was as follows:
¹H-nmr (CDCl₃): δ = 7.58-7.33 (m, 4H), 7.40 (m, 4H), 6.81 (m, 2H), 6.71 (m,1H), 6.34 and 6.27 (two d, 1H), 5.22 (d 1H), 4.69 (m,1H), 4.27 (m,1H), 3.52 (s, 2H), 3.33 (m, 1H), 1.52 and 1.42 (two d, 3H), 0.57 and 0.29 (two d, 3H).
C₂₉H₂₉F₂N₃O₃ (MW = 505.562); mass spectroscopy (MH+) 505.
Anal. Calcd for C₂₉H₂₉F₂N₃O₃; C, 68.89 H, 5.78 N, 8.31. Found: C, 69.01 H, 6.02 N, 8.33.

### Example 7-19

### Synthesis of 5-[N'-(2-Hydroxy-3-methylbutyryl)-L-valinyl]amino-7-methyl-5,7-dihydro-6H-dibenz[b,d]azepin-6-one

Following General Procedure D above using 2-hydroxy-3-methylbutyric acid (Aldrich) and 5-(S)-[L-valinyl]-amino-7-methyl-5,7-dihydro-6H-dibenz[b,d]azepin-6-one hydrochloride (Example 7-C), the title compound was prepared as a colorless solid. The product was purified by flash chromatography using 98:2 CHCl₃/MeOH.
NMR data was as follows:
¹H-nmr (CDCl₃): δ = 7.69-7.25 (m, 8H), 7.08. and 6.92 (two d, 1H), 5.29 (d, 1H), 4.54 (m, 1H), 4.01 (m, 1H), 3.36 (s, 3H), 2.12 (m, 2H), 0.99 (m, 6H), 0.83 (m, 6H).
C₂₅H₃₁N₃O₄.(437.537); mass spectroscopy (MH+) 438.
Anal. Calcd for C₂₅H₃₁N₃O₄; C, 68.63 H, 7.14 N, 9.60. Found: C, 68.71 H, 6.99, N, 9.42.

### Example 7-20

### Synthesis of 5-(S)-[N'-((S or R)-2-Hydroxy-3,3-dimethylbutyryl)-L-valinyl]amino-7-methyl-5,7-dihydro-6H-dibenz[b,d]azepin-6-one and 5-(S)-[N'-((S or R)-2-Hydroxy-3,3-dimethylbutyryl)-L-valinyl]amino-7-methyl-5,7-dihydro-6H-dibenz[b,d]azepin-6-one

Following General Procedure D above using 2-hydroxy-3,3-dimethylbutyric acid (Aldrich) and 5-(S)-[L-valinyl]-amino-7-methyl-5,7-dihydro-6H-dibenz[b,d]azepin-6-one hydrochloride (Example 7-C), the title compound was prepared as a colorless solid. The diastereomers were purified by flash chromatography using 99:1 CHCl3/MeOH.
Isomer 1:
   NMR data was as follows:
   ¹H-nmr (CDCl₃): δ = 7.60-7.28 (m, 8H), 6.63 (d, 1H), 5.26 (d, 1H), 4.53 (m,1H), 3.74 (s, 1H), 3.35 (s, 3H), 2.12 (m, 1H), 0.998 (m, 15H).
   C₂₈H₃₃N₃O₄ (MW = 451); mass spectroscopy (MH+) 452.
   Anal. Calcd for C₂₈H₃₃NO₄. 0.5 mol H_{2O}; C, 67.80 H, 7.16 N, 9.11. Found: C, 68.32 H, 7.06 N, 8.91.
Isomer 2:
   NMR data was as follows:
   ¹H-nmr (CDCl₃): δ = 7.59-7.28 (m, 8H), 6.82 (d, 1H), 5.25 (d, 1H). 4.52(m,1H), 3.74 (s, 1H), 3.33 (s, 3H), 2.16 (m, 1H), 0.997 (m, 15H).
   C₂₈H₃₃N₃O₄ (MW = 451); mass spectroscopy (MH+) 452
   And. Calcd for C₂₈H₃₃N₃O₄; C, 69.16 H, 7.37 N. 9.31. Found: C, 69.33 H, 7.49 N, 9.22.

### Example 7-22

### Synthesis of 5-[N'-(4-phenyl-furazan-3-yl)alaninyl]-amino-7-methyl-5,7-dihydro-6H-dibenz[b,d]azepin-6-one

Hollowing General Procedure D and using N-(4-phenyl-furazan-3-yl)alanine (Example 1) and 5-amino-7-methyl-5,7-dihydro-6H-dibenz[b,d]azepin-6-one (Example 7-A), the title compound was prepared. The reaction was monitored by tlc (Rf = 0.75, 5% MeOH/CHCl₃ and product was purified by chromatography (silica, CHCl₃).
NMR data was as follows:
¹H-nmr (CDCl₃₎: δ = 4.52 (m, 1H); 4.87 (t, 1H).
MW = 453-50; mass spectroscopy (M+) 454.

### Example 7-24

### Synthesis of 5-{N'-(3,5-Difluorophenylacetyl)-L-alaninyl}amino-7-phenbutyl-5,7-dihydro-6H-dibenz[b,d]azepin-6-one

Following General Procedure D and using N-(3,5-difluorophenylacetyl)-L-alanine (Ex. B) and 5-amino-7-phenbutyl-5,7-dihydro-6H-dibenz[b,d]azepin-6-one (Example 7-K), the tide compound was prepared. The reaction was monitored by tlc (Rf = 0.35, 3% MeOH/CHCl₃ and product was purified by chromatography (silica, 3% MeOH/CHCl₃).
NMR data was as follows:
¹H-nmr (CDCl₃): δ = 4.68 (m, 1H); 6.32 (dd, 1H).
MW = 581.66; mass spectroscopy (M+) 582.

### Example 7-25

### Synthesis of 5-{N'-(3,3-Difluorophenylacetyl)-L-alaninyl}amino-7-cyclopropymethyl-5,7-dihydro-6H-dibenz[b,d]azepin-6-one

Following General Procedure D and using N-(3,5-difluorophenylacetyl)-L-alanine (Ex. B) and 5-amino-7-cyclopropymethyl-5,7-dihydro-6H-dibenz[b,d]azepin-6-one (Example 7-L), the title compound was prepared. The reaction was monitored by tlc (Rf = 0.30, 5% MeOM/CHCl₃) and product was purified by chromatography (silica. 3% MeOH/CHCl₃).
NMR data was as follows:
¹H-nmr (CDCl₃): δ = 4.07 (m, 1H); 4.70 (m, 1H); 5.24 (d, 1H).
MW = 503.55; mass spectroscopy (M+) 504.

### Example 7-26

### Synthesis of 5-{N'-(3,5-Difluorophenylacetyl)-L-alaninyl}amino-7-(2',2',2'-trifluoroethyl)-5,7-dihydro-6H-dibenz[b,d]azepin-6-one

Following General Procedure D and using N-(3,5-difluorophenylacetyl)-L-alanine (Ex. B) and 5-amino-7-(2',2',2'-trifluoroethyl)-5,7-dihydro-6H-dibenz[b,d]azepin-6-one (Example 7-M), the title compound was prepared. The reaction was monitored by tlc (Rf = 0.15, 5% MeOH/CHCl₃) and product was purified by chromatography (silica, 5% MeOH/CHCl₃).
NMR data was as follows:
¹H-nmr (CDCl₃): δ = 4.07 (m, 1H); 4.69 (m, 1H); 5.02 (m, 1H); 5.37 (d, 1H).
MW = 531.48; mass spectroscopy (MH+) 530.

### Example 7-27

### Synthesis of 5-{N'-(3,5-Difluorophenylacetyl)-L-alaninyl}amino-7-cyclohexyl-5,7-dihydro-6H-dibenz[b,d]azepin-6-one

Following General Procedure D and using N-(3,5-difluorophenylacetyl)-L-alanine (Ex. B) and 5-amino-7-cyclohexyl-5,7-dihydro-6H-dibenz[b,d]azepin-6-one (Example 7-N), the title compound was prepared. The reaction was monitored by tlc (Rf = 0.35, 5% MeOH/CHCl₃) and product was purified by chromatography (silica, 5% MeOH/CHCl₃).
NMR data was as follows:
¹H-nmr (CDCl₃): δ = 1.43 (dd, 3H); 3.94 (m, 1H); 4.68 (m, 1H); 5.18 (d, 1H).
MW = 531.60); mass spectroscopy (M+) 533.

### Example 7-28

### Synthesis of 5-{N'-[(S)-3,5-Difluoromandelyl]-L-alaninyl}amino-9-fluoro-7-methyl-5,7-dihydro-6H-dibenz[b,d]azepin-6-one

Following General Procedure D and using (S)-3,5-difluoromandelic acid (Example L) and 5-(L-alaninyl)-amino-9-fluoro-7-methyl-5,7-dihydro-6H-dibenz[b,d]azepin-6-one hydrochloride (Example 7-O), the title compound was prepared. The reaction was monitored by tic (Rf = 0.4, 10% MeOH/CHCl₃) and product was purified by chromatography (silica, 2.5% MeOH/CHCl₃).
NMR data was as follows:
¹H-nmr (CDCl₃): δ = 3.36 (s, 3H); 4.67 (m, 1H); 5.05 (s, 1H); 5.21 (m, 1H).
MW = 497.47; mass spectroscopy (M+) 498.

### Example 7-29

### Synthesis of 5-{N'-[(S)-3,5-Difluoromandelyl]-L-alaninyl}-amino-13-fluoro-7-methyl-5,7-dihydro-6H-dibenz[b,d]azepin-6-one

Following General Procedure D and using (S)-3,5-difluoromandelic acid (Example L) and 5-(L-alaninyl)-amino-13-fluoro-7-methyl-5,7-dihy4ro-6H-dibenz[b,d]azepin-6-one hydrochloride (Example 7-P), the title compound was prepared. The reaction was monitored by tlc (Rf = 0.4, 10% MeOH/CHCl₃) and product was purified by 2.5% chromatography (silica, MeOH/CHCl₃).
NMR data was as follows:
¹H-nmr (CDCl₃): δ = 1.45 (dd, 3H); 3.31 (d, 3H).
MW = 497.47; mass spectroscopy (MH+) 498.

### Example 7-30

### Synthesis of 5-{N'-[(S)-3,5-Difluoromandelyl]-L-alaninyl}amino-10-fluoro-7-methyl-5,7-dihydro-6H-dibenz[b,d]azepin-6-one

Following General Procedure D and using (S)-3,5-difluoromandelic acid (Example L) and 5-(L-alaninyl)-amino-10-fluoro-7-methyl-5,7-dihydro-6H-dibenz[b,d]azepin-6-one hydrochloride (Example 7-Q), the title compound was prepared. The reaction was monitored by tlc (Rf = 0.4, 10% MeOH/CHCl₃) and product was purified by chromatography (silica, 2.5% MeOH/CHCl₃).
NMR data was as follows:
¹H-nmr (CDCl₃): δ = 1.44 (dd, 3H); 3.35 (d, 3H).
MW = 497.47; mass spectroscopy (M+) 498.

### Example 7-31

### Synthesis of 5-{N'-[(S)-3,5-Difluoromandelyl]-L-alaninyl}amino-7-cyclopropylmethyl-5,7-dihydro-6H-dibenz[b,d]azepin-6-one

Following General Procedure D and using (S)-3,5-difluoromandelic acid (Example L) and 5-(L-alaninyl)-amino-7-cyclopropylmethyl-5,7-dihydro-6H-dibenz[b,d]azepin-6-one hydrochloride (Example 7-R), the title compound was prepared. The product was purified by chromatography (silica, 2.5% MeOH/CHCl₃).
NMR data was as follows:
¹H-nmr (CDCl₃): δ = 1.48 (dd, 3H); 3.45 (m, 1H).
MW = 519.55; mass spectroscopy (M+) 520.

### Example 7-32

### Synthesis of 5-{N'-[(S)-3,5-Difluoromandelyl]-L-alaninyl}amino-7-phenbutyl-5,7-dihydro-6H-dibenz[b,d]azepin-6-one

Following General Procedure D and using (S)-3,5-difluoromandelic acid (Example L) and 5-(L-alaninyl)-amino-7-phenbutyl-5,7-dihydro-6H-dibenz[b,d]azepin-6-one hydrochloride (Example 7-S), the title compound was prepared. The product was purified by chromatography (silica, 1-2% MeOH/CHCl₃).
NMR data was as follows:
¹H-nmr (CDCl₃): δ = 1.48 (dd, 3H); 5.04 (d, 1H).
MW = 597.66; mass spectroscopy (M+) 599.

### Example 7-33

### Synthesis of 5-{N'-[(S)-3,5-Difluoromandelyl]-L-valinyl}amino-7-cyclopropylmethyl-5,7-dihydro-6H-dibenz[b,d]azepin-6-one

Following General Procedure D and using (S)-3,5-difluoromandelic acid (Example L) and 5-(L-valinyl)-amino-7-cyclopropylmethyl-5,7-dihydro-6H-dibenz[b,d]azepin-6-one hydrochloride (Example 7-T), the title compound was prepared. The reaction was monitored by tlc (Rf = 0.3, 2.5% MeOH/CHCl₃) and product was purified by chromatography (silica, 2.5% MeOH/CHCl₃).
NMR data was as follows:
¹H-nmr (CDCl₃): δ = 3.42 (m, 1H); 4.07 (m, 1H); 5.03 (d, 1H).

### Example 7-34

### Synthesis of 5-{N'-[(S)-3,5-Difluoromandelyl]-L-valinyl}amino-7-phenbutyl-5,7-dihydro-6H-dibenz[b,d]azepin-6-one

Following General Procedure D and using (S)-3,5-difluoromandelic acid (Example L) and 5-(L-valinyl)-amino-7-phenbutyl-5,7-dihydro-6H-dibenz[b,d]azepin-6-one hydrochloride (Example 7-U), the title compound was prepared. The product was purified by chromatography (silica, 1-2% MeOH/CHCl₃).
NMR data was as follows:
¹H-nmr (CDCl₃): δ = 3.54 (m, 1H); 4.35 (m, 1H); 5.03 (d, 1H).
MW = 625.71; mass spectroscopy (M+) 625.

### Example 7-35

### Synthesis of 5-{N'-[(S)-3,5-Difluoromandelyl]-L-valinyl}amino-7-hexyl-5,7-dihydro-6H-dibenz[b,d]azepin-6-one

Following General Procedure D and using (S)-3,5-difluoromandelic acid (Example L) and 5-(L-valinyl)-amino-7-hexyl-5,7-dihydro-6H-dibenz[b,d]azepin-6-one hydrochloride (Example 7-V), the title compound was prepared. The product was purified by chromatography (silica, 5% MeOH/CHCl₃).
NMR data was as follows:
¹H-nmr (DMSO-d₆): δ = 4.25 (m, 1H); 4.52 (m, 1H); 5.05 (t, 1H); 5.24 (2 doublets, 1H).
MW = 577.67; mass spectroscopy (M+) 578.

### Example 7-36

### Synthesis of 5-{N'-[(S)-3,5-Difluoromandelyl]-L-valinyl}amino-10-fluoro-7-methyl-5,7-dihydro-6H-dibenz[b,d]azepin-6-one

Following General Procedure D and using (S)-3,5-difluoromandelic acid (Example L) and 5-(L-valinyl)-amino-10-fluoro-7-methyl-5,7-dihydro-6H-dibenz[b,d]azepin-6-one hydrochloride (Example 7-W), the title compound was prepared. The product was purified by chromatography (silica, 2.5% MeOH/CHCl₃).
Anal. Calc.: C, 71.02; H, 5.96; N, 6.72. Found: C, 71.10, H, 6.12, N, 6.63.

### Example 7-37

### Synthesis of 5-{N'-[(S)-3,5-Difluoromandelyl]-L-valinyl}amino-13-fluoro-7-methyl-5,7-dihydro-6H-dibenz[b,d]azepin-6-one

Following General Procedure D and using (S)-3,5-difluoromandelic acid (Example L) and 5-(L-valinyl)-amino-13-fluoro-7-methyl-5,7-dihydro-6H-dibenz[b,d]azepin-6-one hydrochloride (Example 7-X), the title compound was prepared. The product was purified by chromatography (silica, 2.5% MeOH/CHCl₃).
Anal. Calc.: C, 71.02; H, 5.96; N, 6.72. Found: C, 71:10, H, 6.12, N, 6.63.

### Example 7-38

### Synthesis of 5-{N'-[(S)-3,5-Difluoromandelyl]-L-valinyl}amino-9-fluoro-7-methyl-5,7-dihydro-6H-dibenz[b,d]azepin-6-one

Following General Procedure D and using (S)-3,5-difluoromandelic acid (Example L) and 5-(L-valinyl)-amino-9-fluoro-7-methyl-5,7-dihydro-6H-dibenz[b,d]azepin-6-one hydrochloride (Example 7-Y), the title compound was prepared. The product was purified by chromatography (silica, 2.5% MeOH/CHCl₃).
Anal. Calc.: C, 71.02; H, 5.96; N, 6.72. Found: C, 71.10, H, 6.12, N, 6.63.

### 8. Benzodiazepine Derivatives and Related Compounds

### GENERAL PROCEDURE 8-A

### N-1-Methylation of Benzodiazepines

A solution of benzodiazepine (1 eq.) in DMF (0.1 M concentration) at 0°C was treated with potassium tert-butoxide (1.0 eq., 1.0 M solution in THF). After stirring for 30 minutes at 0°C, iodomethane (1.3 eq.) was added and stirring continued for 25 minutes. The mixture was diluted with methylene chloride and washed with water and brine. The organic phase was dried over Na₂SO₄, filtered, and concentrated. The crude product was then either purified by trituration with 1:1 ether/hexanes or chromatographed via HPLC using ethyl acetate/hexanes as the eluent.

### GENERAL PROCEDURE 8-B

### Cbz Removal Procedure

A flask was charged with the Cbz-protected 3-aminobenzodiazepine (1 eq.). To this was added HBr (34 eq.; 30% solution in acetic acid). Within 20 minutes all of the starting material dissolves. The reaction was stirred for 5 hours at ambient temperature. Ether was added to the orange solution causing the HBr•amine salt to precipitate. The mixture was decanted. This process of adding ether and decanting was repeated thrice in an effort to remove acetic acid and benzyl bromide. Toluene was added and the mixture concentrated *in vacuo.* This step was also repeated. The HBr salt was partitioned between ethyl acetate and 1 M K₂CO₃. The aqueous layer was back-extracted with ethyl acetate. The combined organics were washed with brine, dried over Na₂SO₄, filtered, and concentrated.

### GENERAL PROCEDURE 8-C

### Boc Removal Procedure

A solution of Boc-protected amine (1 eq.) in methylene chloride (0.15 M concentration) was cooled to 0°C and treated with trifluoroacetic acid (30 eq.). After 10 minutes at 0°C, the cooling bath was removed and stirring continued at ambient for 20 minutes to 1 hour. The mixture was concentrated *in vacuo* to remove excess trifluoroacetic acid. The residue was dissolved in methylene chloride and washed with saturated aqueous NaHCO₃ or 1 M K₂CO₃ and brine. The organic layer was dried over Na₂SO₄, filtered, and concentrated.

### GENERAL PROCEDURE 8-D

### Azide Transfer Reaction Usine KHMDS

The azido derivative was prepared using the procedure described in John W. Butcher et al., Tet. Lett., 37, 6685-6688 (1996).

### GENERAL PROCEDURE 8-E

### Azide Transfer Reaction Using LDA

To a solution of diisopropylamine (1.1 eq.) in 1 mL of dry THF cooled to - 78°C was added n-butyl lithium (1.6M in hexane) (1.1 eq.) dropwise maintaing the reaction temperature at -78°C. The reaction mixture was stirred for 30 min. at -78°C and then the lactam (0.471 mM) was added dropwise as a solution in I mL of dry THF. The reaction mixture was stirred at -78°C for 30 min. and then a pre-cooled solution of trisyl azide (1.2 eq.) was added as a solution in 1 mL of dry THF. The reaction mixture was stirred at -78°C for 20 min. and then quenched with acetic acid (4.0 eq.). The reaction mixture was then stirred at 40°C for 2 hrs. The reaction was then poured into EtOAc and washed with water, sodium bicarbonate and brine, and then dried over sodium sulfate, filtered and concentrated. The residue was purified by LC 2000 chromatography.

### GENERAL PROCEDURE 8-F

### Azido Group Reduction

The azido group was reduced to the corresponding primary amine using the procedure described in John W. Butcher et al., Tet. Lett., 37, 6685-6688 (1996).

### GENERAL PROCEDURE 8-G

### N-Alkylation of Amides or Lactams Using Sodium Hydride or Potassium tert-Butoxide

To a slurry of sodium hydride or potassium tert-butoxide (1.1 eq) in 15 mL of dry DMF was added the appropriate amide (0.0042 moles) as a solution in 10 mL of DMF. The alkyl iodide was then added and a thick slurry resulted. The reaction became less thick as time elapsed and when complete by TLC the reaction had become homogeneous. The reaction mixture was poured over ice and extracted into ethyl acetate. The organic layer was washed with water, followed by brine. The organic layer was then dried over sodium sulfate, filtered and concentrated under reduced pressure. The residue was purified by HPLC (LC 2000), eluting with an ethyl acetate/hexane system.

### GENERAL PROCEDURE 8-H

### N-Alkylation of Amides or Lactams Using KHMDS

To the appropriate amide or lactam in THF cooled to -78°C was added KHMDS dropwise and the reaction mixture was stirred for 30 min. at -78°C. The alkyl iodide was then added dropwise while maintaining the temperature at -70°C. The cooling bath was then removed and reaction was allowed to warm to room temperature and stirring was continued for 2 hours. The reaction mixture was then poured over ice and extracted into ethyl acetate. The organic extracts were washed with water, followed by brine. The organic layer was then dried over sodium sulfate, filtered and concentrated under reduced pressure. The residue was purified by HPLC (LC 2000), eluting with an ethyl acetate/hexane system.

### GENERAL PROCEDURE 8-I

### N-Alkylation of Amides or Lactams Using Cesium Carbonate

To a solution of the amide or lactam in DMF was added cesium carbonate (1.05 eq) and an alkyl iodide (1.1 eq). The mixture was allowed to stir overnight at room temperature and then the reaction mixture was dilluted with ethyl acetate and washed with water, followed by brine. The organic layer was dried over sodium sulfate, filtered and concentrated under reduced pressure. The residue was purified by HPLC (LC 2000), eluting with an ethyl acetate/hexane system.

### GENERAL PROCEDURE 8-J

### BOC Removal Procedure

To an N-Boc protected compound was added CH₂Cl₂/TFA (4:1) at room temperature. The reaction mixture was stirred at room temperature for 3 hours and then concentrated. The residue was extracted into dichloromethane and washed with water, saturated sodium bicarbonate, dried over Na₂SO₄, filtered and concentrated to give the free amine.

### GENERAL PROCEDURE 8-K

### Azide Transfer Procedure

This azide transfer procedure is a modification of the procedure described in Evans, D. A.; Britton, T. C.; Ellman, J. A.; Dorow, R. L. J. Am. Chem. Soc. 1990, 112, 4011-4030. To a solution of the lactam substrate (1.0 eq.) in THF (∼0.1 M) under N₂ at -78 °C was added a solution of KN(TMS)₂ (1.1 eq. of 0.5 M in Toluene, Aldrich) dropwise over a period of 2-10 minutes. A slight exotherm was often observed by an internal thermometer, and the resulting solution was stirred for 5-15 minutes, while re-cooling to -78°C. Then, trisyl azide (1.1-1.5 eq., CAS No. 36982-84-0, prepared as described by references in the Evans reference above) in THF (∼0.5 M), either precooled to -78°C or at room temperature, was added via cannula over a period of 0.5-5 minutes. Again, a slight exotherm was generally noted. The resulting solution was stirred for from 5-10 minutes, while re-cooling to -78°C. Then, AcOH (4.5-4.6 eq., glacial) was added, the cooling bath removed and the mixture allowed to warm to room temperature with stirring for 12-16 hours. The mixture was diluted with EtOAc, in a 2-5 volume multiple of the initial THF volume, and washed with dilute aq. NaHCO₃ (1-2x), 0.1-1.0 M aq. HCl (0-2x), and brine (1x). The organic phase was then dried over MgSO₄, filtered, concentrated to provide the crude product.

### GENERAL PROCEDURE 8-L

### Azide Reduction to an Amine

A mixture of the azide in absolute EtOH (0.03-0.07 M) and 10% Pd/C (∼1/3 by weight of the azide) was shaken in a Parr apparatus under H₂ (35-45 psi) at room temperature for 3-6 hours. The catalyst was removed by filtration through a plug of Celite, rinsing with absolute EtOH, and the filtrate concentrated to provide the crude amine product.

### GENERAL PROCEDURE 8-M

### Amide Alkylation Using Cesium Carbonate

This procedure is a modification of the procedure described in Claremon, D. A.; et al, PCT Application: WO 96-US8400 960603. To a mixture of 2,4-dioxo-2,3,4,5-tetrahydro-1H-1,5-benzodiazepine (CAS No. 49799-48-6) in DMF (1.0 eq., 0.7 M) under N₂ at room temperature was added Cs₂CO₃ (2.2 eq.) and the appropriate alkyl halide (2.2 eq.). The mixture was stirred at room temperature for 5.5-16 hours. The mixture was partitioned between EtOAc and sat. NaHCO₃. The aqueous layer was extracted with EtOAc (1-2x) and the combined EtOAc extracts were dried over Na₂SO₄, filtered, and concentrated to provide the crude product.

### GENERAL PROCEDURE 8-N

### BOC Removal Procedure

A stream of anhydrous HCl gas was passed through a stirred solution of the N-t-Boc protected amino acid in 1,4-dioxane (0.03-0.09 M), chilled in a ice bath to ∼10°C under N₂, for 10-15 minutes. The solution was capped, the cooling bath removed, and the solution was allowed to warm to room temperature with stirring for 2-8 hours, monitoring by TLC for the consumption of starting material. The solution was concentrated (and in some instances dissolved in CH₂Cl₂ then re-concentrated and placed in vacuum oven at 60-70°C to remove most of the residual dioxane) and used without further purification.

### Example 8-A

### Synthesis of 3-Amino-1,3-dihydro-5-(1-piperidinyl)-2H-1,4-benzodiazepin-2-one

### Step A - Preparation of 1,2-Dihydro-3H-1-methyl-5-(1-piperidiny)-1,4-benzodiazepin-2-one

A solution of phosphorous pentachloride (1.2 eq) in methylene chloride was added dropwise to a solution of 1-methyl-1,2,3,4-tetrahydro-3H-1,4-benzodiazepin-2,5-ione (Showell, G. A.; Bourrain, S.; Neduvelil, J. G.; Fletcher, S. R.; Baker, R.; Watt, A. P.; Fletcher,A.E.; Freedman, S. B.; Kemp, J. A.; Marshall, G. .; Patel, S.; Smith, A. J.; Matassa, V. G. J. Med.Chem. 1994, 37, 719.) in methylene chloride. The resultant yellowish-orange solution was stirred at ambient temperature for 2.5 hours; the solvent was removed *in vacuo.* The orange residue was redissolved in methylene chloride, cooled to 0 °C, and treated with a solution of piperidine (2 eq) and triethylamine (2 eq) in methylene chloride. The cooling bath was removed and the reaction stirred for 18 hours. The reaction mixture was washed with saturated aqueous NaHCO₃ (back-extracted with methylene chloride) and brine. The organic phase was dried over Na₂O₄, filtered, and concentrated. The residue was purified via HPLC eluting with a gradient of 4 to 10% methanol/methylene chloride affording the title intermediate as a yellow solid having a melting point of 103-105°C.
C₁₅H₁₉N₃O (MW 257.37); mass spectroscopy 257.
Anal. Calcd for C₁₅H₁₉N₃O: C, 70.01; H, 7.44; N, 16.33. Found: C, 69.94; H, 7.58; N, 16.23.

### Step B - Preparation of 1,2-Dihydro-3H-1-methyl-3-oximido-5-(1-piperidinyl)-1,4-benzodiazepin-2-one

Potassium tert-butoxide (2.5 eq) was added in two portions to a -20°C solution of 1,2-dihydro-3H-1-methyl-5-(1-piperidinyl)-1,4-benzodiazepin-2-one (1 eq) in toluene). After stirring at - 20°C for 20 min, isoamyl nitrite (1.2 eq.; Aldrich) was added to the red reaction mixture. The reaction was stirred at -20 °C for 5 hours at which time the reaction was done by TLC. The cooling bath was removed and the reaction quenched with 0.5 M citric acid. After stirring for 10 minutes, diethyl ether was added. The suspension was stirred at ambient temperature overnight then filtered washing with ether. The resultant cream colored solid had a melting point of 197-200°C.
¹H NMR data of the E/Z isomers was as follows:
¹H NMR (300 MHz, CDCl₃): δ = 7.64 (1H, bs), 7.48 (2H, d, J=7.4 Hz), 7.35-7.20 (6H, m), 6.75 (1H, bs), 3.8-3.2 (8H, m), 3.46 (3H, s), 3.42 (3H, s), 1.90-1.40 (12H, m).
C₁₅H₁₈N₄O₂ (MW = 286.37); mass spectroscopy 286.

### Step C - Preparation of 1,2-dihydro-3H-1-methyl-3-[O-(ethylaminocarbonyl)oximido]-5-(1-piperidinyl)-1,4-benzodiazepin-2-one

A mixture of 1,2-dihydro-3H-1-methyl-3-oximido-5-(1-piperidinyl)-1,4-benzodiazepin-2-one (1 eq) in THF was treated with ethyl isocyanate (1.7 eq) and triethylamine (0.6 eq). The mixture was heated to 64°C for 4 hours. The mixture was concentrated and the residue purified by HPLC eluting with 5% methanol/methylene chloride.
¹H NMR data of the E/Z isomers was as follows:
¹H NMR (300 MHz, CDCl₃): δ = 7.50 (2H, dd, J=8.4, 1.5 Hz), 7.35-7.22 (6H, m), 6.42 (1H, bt), 6.20 (1H, bt), 3.7-3.4 (8H, m), 3.46 (3H, s), 3.44 (3H, s), 3.25 (4H, m), 1.9-1.4 (12H, m), 1.12 (3H, t, J=6.3 Hz), 1.10 (3H, t, J=6.3 Hz).
C₁₈H₂₃N₅O₃ (MW = 357.46); mass spectroscopy 357.

### Step D - Preparation of 3-Amino-1,3-dihydro-2H-1-methyl-5-(1-piperidinyl)-1,4-benzodiazepin-2-one

The 1,2-dihydro-3H-1-methyl-3-[O-(ethylaminocarbonyl)oximido]-5-(1-piperidinyl)-1,4-benzodiazepin-2-one (1 eq) was hydrogenated in methanol over 5% palladium on carbon (0.15 eq) at 43 psi for 3.25 hours. The reaction was filtered through celite and concentrated *in vacuo.* The residue was taken up in methylene chloride and filtered a second time through celite. The filtrate was concentrated and the resultant foam was used immediately.

### Example 8-B

### Synthesis of 3-(L-Alaninyl)-amino-2,3-dihydro-1-methyl-5-phenyl-1H-1,4-benzepin-2-one

### Step A - Preparation of (S)-3-amino-1,3-dihydro-1-methyl-5-phenyl-2H-1,4-benzodiazepin-2-one, (1S)-7,7-dimethyl-2-oxobicyclo[2.2.1]heptane-1-methanesulfonate

The title intermediate was prepared according to Reider, P. J.; Davis, P.; Hughes, D. L.; Grabowski, E. J. J. J. Org. Chem. 1987, 52, 955 using 3-amino-1,3-dihydro-1-methyl-5-phenyl-2H-1,4-benzodiazepin-2-one (Bock M. G.; DiPardo, R. M.; Evans, B. E.; Rittle, K. E.; Veber, D. F.; Freidinger, R. M.; Hirshfield, J.; Springer, J. P. J. Org. Chem. 1987, 52, 3232.) as the starting material.

### Step B - Preparation of 3-[N'-(tert-Butylcarbamate)-L-alaninyl]-amino-2,3-dihydro-1-methyl-5-phenyl-1H-1,4-benzodiazepin-2-one

(S)-3-Amino-1,3-dihydro-1-methyl-5-phenyl-2H-1,4-benzodiazepin-2-one, (1S)-7,7-dimethyl-2-oxobicyclo[2.2.1]heptane-1-methanesulfonate was free based by partitioning between methylene chloride and 1M potassium carbonate. The free amine was then coupled with N-Boc-alanine following General Procedure D.
C₂₄H₂₈N₄O₄ (MW = 436.56); mass spectroscopy 436.
Anal. Calc. for C₂₄H₂₈N₄O₄: C. 66.03; H, 6.47; N, 12.84. Found: C, 65.79; H, 6.68; N, 12.80.

### Step C - Preparation of 3-(L-Alaninyl)-amino-2.3-dihydro-1-methyl-5-phenyl-1H-1,4-benzodiazepin-2-one

Following General Procedure 8-C using 3-[N'-(tert-butylcarbamate)-L-alaninyl]-amino-2,3-dihydro-1-methyl-5-phenyl-1H-1,4-benzodiazepin-2-one, the title compound was prepared as a white foam.
Anal. Calc. for C₁₉H₁₉N₄O₂: C, 69.21; H, 6.64; N, 15.37. Found: C, 70.11; H, 6.85; N, 15.01.

### Example 8-C

### Synthesis of 3-(L-Alaninyl)-amino-7-chloro-2,3-dihydro-1-methyl-5-phenyl-1H-1,4-benzodiazepin-2-one

### Step A - Preparation of 3-(Benzyloxvcarbonyl)-amino-7-chloro-2,3-dihydro-1-methyl-5-phenyl-1H-1,4-benzodiazepin-2-one

A solution of 3-(benzyloxycarbonyl)-amino-7-chloro-2,3-dihydro-5-phenyl-1H-1,4-Benzodiazepin-2-one (1 eq; Neosystem) in DMF was cooled to 0°C and treated with potassium *tert*-butoxide (1 eq; 1.0M solution in THF). The resultant yellow solution was stirred at 0°C for 30 minutes then quenched with methyl iodide (1.3 eq). After stirring an addition 25 minutes the reaction was diluted with methylene chloride and washed with water and brine. The organic phase was dried over Na₂SO₄, filtered, and concentrated. The residue was purified via HPLC chromatography eluting with a gradient of 20→30% ethyl acetate/hexanes.
C₁₄H₂₀ClN₃O₃ (MW =.433.92); mass spectroscopy 433.
Anal. calcd for C₂₄H₂₀ClN₃O₃: C, 66.44; H, 4.65; N, 9.68. Found: C, 66.16; H, 4.50; N, 9.46.

### Step B - Preparation of 3-Amino-7-chloro-1,3-dihydro-1-methyl-5-phenyl-2H-1,4-benzodiazepin-2-one

Following General Procedure 8-B using 3-(benzyloxycarbonyl)-amino-7-chloro-2,3-dihydro-1-methyl-5-phenyl-1H-1,4-benzodiazepin-2-one, the title intermediate was prepared as a white foam which was used immediately in Step C.

### Step C - Preparation of 3-[N'-tert-Butylcarbamate)-L-alaninyl]-amino-7-chloro-1.3-dihydro-1-methyl-5-phenyl-2H-1,4-benzodiazepin-2-one

Following General Procedure D using N-Boc-L-alanine and 3-amino-7-chloro-1,3-dihydro-1-methyl-5-phenyl-2H-1,4-benzodiazepin-2-one, the title intermediate was prepared as a white foam.
C₂₄H₂₈ClN₄O₄ (MW = 471.18); mass spectroscopy 471
Anal. calcd for C₂₄H₂₈ClN₄O₄: C, 61.21; H, 5.78; N, 11.90. Found: C, 61.24; H, 5.59; N, 11.67.

### Step D - Preparation of 3-(L-Alaninyl)amino-7-chloro-1.3-dihydro-1-methyl-5-phenyl-2H-1,4-benzodiazepin-2-one

Following General Procedure 8-C using 3-[N'-*tert*-butylcarbamate)-L-alaninyl]-amino-7-chloro-1,3-dihydro-1-methyl-5-phenyl-2H-1,4-benzodiazepin-2-one, the title intermediate was prepared as a white foam. The crude material was used immediately.

### Example 8-D

### Synthesis of 3-(L-Alaninyl)amino-7-bromo-2,3-dihydro-1-methyl-5-(2-fluorophenyl)-1H-1,4-benzodiazepin-2-one

### Step A - Preparation of 3-(Benzyloxycarbonyl)-amino-7-bromo-2,3-dihydro-1-methyl-5-(2-fluorophenyl)-1H-1,4-benzodiazepin-2-one

Following General Procedure 8-A using 3-(benzyloxycarbonyl)-amino-7-bromo-2,3-dihydro-5-(2-fluorophenyl)-1H-1,4-benzodiazepin-2-one (Neosystem), the title intermediate was prepared as a white foam.
C₂₄H₁₉BrFN₃O₃ (MW = 496.36); mass spectroscopy 497.
Anal. calcd for C₂₄H₁₉BrFN₃O₃: C, 58.08; H, 3.86; N, 8.47. Found: C, 57.90; H, 4.15; N, 8.20.

### Step B - Preparation of 3-Amino-7-bromo-1.3-dihydro-1-methyl-5-(2-fluorophenyl)-2H-1,4-benzodiazepin-2-one

Following General Procedure 8-B using 3-(benzyloxycarbonyl)-amino-7-bromo-2,3-dihydro-1-methyl-5-(2-fluorophenyl)-1H-1,4-benzodiazepin-2-one, the title intermediate was prepared as a white foam which was used immediately in Step C.

### Step C - Preparation of 3-[N'-(tert-Butylcarbamate)-L-alaninyl]-amino-7-bromo-1,3-dihydro-1-methyl-5-(2-fluorophenyl)-2H-1,4-benzodiazepin-2-one

Following General Procedure D using N-Boc-L-alanine (Novo) and 3-amino-7-bromo-1,3-dihydro-1-methyl-5-(2-fluorophenyl)-2H-1,4-benzodiazepin-2-one, the title intermediate was prepared as a white foam.
C₂₄H₂₆BrFN₄O₄ (MW = 533.12); mass spectroscopy 533.2.
Anal. calcd for C₂₄H₂₆BrFN₄O₄: C, 54.04; H, 4.91; N, 10.50. Found: C, 53.75; H, 4.92; N, 10.41.

### Step D - Preparation of 3-(L-Alaninyl)-amino-7-bromo-1,3-dihydro-1-methyl-5-(2-fluorophenyl)-2H-1,4-benzodiazepin-2-one

Following General Procedure 8-C using 3-[N'-(*tert*-butylcarbamate)-L-alaninyl]-amino-7-bromo-1,3-dihydro-1-methyl-5-(2-fluorophenyl)-2H-1,4-benzodiazepin-2-one, the title intermediate was prepared as a white foam. The crude material was used immediately.

### Example 8-E

### Synthesis of 3-(N'-Methyl-L-alaninyl)-amino-2,3-dihydro-1-methyl-5-phenyl-1H-1,4-benzodiazepin-2-ooe

### Step A - Preparation of 3-[N'-(tert-Butylcarbamate)-N'-methyl-L-alaninyl]-amino-2,3-dihydro-1-methyl-5-phenyl-1H-1,4-benzodiazepin-2-one

Following General Procedure D and using (S)-3-amino-1,3-dihydro-1-methyl-5-phenyl-2H-1,4-benzodiazepin-2-one (Example 8-B) and N-*tert*-Boc-N-methyl-alanine (Sigma), the title intermediate was obtained as a white solid.
C₂₅H₃₀N₄O₄ (MW = 450.2); mass spectroscopy (M+1) 451.2.
Anal. calcd for C₂₅H₃₀N₄O₄: C, 66.65; H, 6.71; N, 12.44. Found: C, 66.66; H, 6.89; N, 12.21.

### Step A - Preparation of 3-(N'-Methyl-L-alaninyl)-amino-2,3-dihydro-1-methyl-5-phenyl-1H-1,4-benzodiazepin-2-one

Following General Procedure 8-C and using 3-[N'-(*tert*-butylcarbamate)-N'-methyl-L-alaninyl]-amino-2,3-dihydro-1-methyl-5-phenyl-1H-1,4-benzodiazepin-2-one, the title intermediate was prepared as a white foam.
C₂₀H₂₂N₄O₂ (MW =350.46); mass spectroscopy (M+1) 351.4.
Anal. calcd for C₂₀H₂₂N₄O₂: C, 68.55; H, 6.33; N, 15.99. Found, C, 68.36; H, 6.20; N, 15.79.

### Example 8-F

### Synthesis of 3-(L-Alaninyl)amino-7-chloro-2,3-dihydro-1-methyl-5-(2-chlorophenyl)-1H-1,4-benzodiazepin-2-one

### Step A - Preparation of 3-(Benzyloxycarbonyl)-amino-7-chloro-2.3-dihydro-1-methyl-5-(2-chlorophenyl)-1H-1,4-benzodiazepin-2-one

Following General Procedure 8-A using 3-(benzyloxycarbonyl)-amino-7-chloro-2,3-dihydro-5-(2-chlorophenyl)-1H-1,4-benzodiazepin-2-one (Neosystem), the title intermediate was prepared as a white solid having a melting point of 232-233°C.
C₂₄H₁₉Cl₂N₃O₃ (MW = 468.36); mass spectroscopy 468.
¹H NMR (300 MHz, CDCl₃): δ = 7.67 (1H, m), 7.52 (1H, dd, J=2.4, 8.7 Hz), 7.42-7.26 (9H, m), 7.07 (1H, d, J=2.4 Hz), 6.70 (1H, d, J=8.3 Hz), 5.35 (1H, d, J=8.4 Hz), 5.14 (2H, ABq, J=19.6 Hz), 3.47 (3H, s).
¹³C NMR (75 MHz, CDCl₃): δ = 166.66, 165.65, 155.72, 140.52, 136.99, 136.0, 132.87, 131.99, 131.47, 131.40, 131.38, 131.16, 130.54, 130.06, 128.45, 128.08, 128.03, 127.72, 127.22, 123.28, 122.01, 68.95, 67.02, 35.32.

### Step B - Preparation of 3-Amino-7-chloro-1,3-dihydro-1-methyl-5-(2-chlorophenyl)-2H-1,4-benzodiazepin-2-one

Following General Procedure 8-B using 3-(benzyloxycarbonyl)-amino-7-chloro-2,3-dihydro-1-methyl-5-(2-chlorophenyl)-1H-1,4-benzodiazepin-2-one, the title intermediate was prepared as a white foam which was used immediately in Step C.

### Step C - Preparation of 3-[N'-(tert-Butylcarbamate)-L-alaninyl]-amino-7-chloro-1,3-dihydro-1-methyl-5-(2-chlorophenyl)-2H-1,4-benzodiazepin-2-one

Following General Procedure D using N-Boc-L-alanine and 3-amino-7-chloro-1,3-dihydro-1-methyl-5-(2-chlorophenyl)-2H-1,4-benzodiazepin-2-one, the title intermediate was prepared as a white foam.
C₂₄H₂₆Cl₂N₄O₄ (MW = 505.44); mass spectroscopy 505.2.

### Step D - Preparation of 3-(L-Alaninyl)-amino-7-chloro-1,3-dihydro-1-methyl-5-(2-chlorophenyl)-2H-1,4-benzodiazepin-2-one

Following General Procedure 8-C using 3-[N'-(*tert*-butylcarbamate)-L-alaninyl]-amino-7-chloro-1,3-dihydro-1-methyl-5-(2-chlorophenyl)-2H-1,4-benzodiazepin-2-one, the title intermediate was prepared as a white foam. The crude material was used immediately.

### Example 8-G

### Synthesis of 3-(L-Alaninyl)amino-5-cyclohexyl-2,3-dihydro-1-methyl-1H-1,4-Benzodiazepin-2-one

### Step A - Preparation of 3-(Benzyloxycarbonyl)-amino-5-cylclohexyl-2,3-dihydro-1-methyl-1H-1,4-benzodiazepin-2-one

Following General Procedure 8-A using 3-(benzyloxycarbonyl)-amino-5-cyclohexyl-2,3-dihydro-1H-1,4-benzodiazepin-2-one (Neosystem), the title intermediate was prepared as a white solid having a melting point of 205-206°C.
C₂₄H₂₇N₃O₃ (MW = 405.54); mass spectroscopy 405.
¹H NMR (300 MHz, CDCl₃): δ = 7.54 (1H, d, J=7.9 Hz), 7.48 (1H, d, J=7.7 Hz), 7.36-7.26 (7H, m), 6.54 (1H, d, J= 8.3 Hz), 5.15 (1H, d, J=8.0 Hz), 5.09 (2H, ABq, J=17.1 Hz), 3.39 (3H, s), 2.77 (1H, m), 2.01 (1H, bd, J=13.6 Hz), 1.85 (1H, bd, J=12.4 Hz), 1.68-1.49 (4H, m), 1.34-.1.02 (4H, m).

### Step B - Preparation of 3-Amino-5-cyclohexyl-1,3-dihydro-1-methyl-2H-1,4-benzodiazepin-2-one

Following General Procedure 8-B using 3-(benzyloxycarbonyl)-amino-5-cyclohexyl-2,3-dihydro-1-methyl-1H-1,4-benzodiazepin-2-one, the title intermediate was prepared as a white foam which was used immediately in Step C.
C₁₆H₂₁N₃O (MW+H= 272.1763); mass spectroscopy 272.1766

### Step C - Preparation of 3-[N'-(tert-Butylcarbamate)-L-alaninyl]-amino-5-cyclohexyl-1,3-dihydro-1-methyl-2H-1,4-benzodiazepin-2-one

Following General Procedure D using N-Boc-L-alanine and 3-amino-5-cyclohexyl-1,3-dihydro-1-methyl-2H-1,4-benzodiazepin-2-one, the title intermediate was prepared as a white foam.
C₂₄H₃₄N₄O₄ (MW = 442.62); mass spectroscopy (M+H) 443.2.

### Step D - Preparation of 3-(L-Alaninyl)amino-5-cyclohexyl-1,3-dihydro-1-methyl-2H-1.4-benzodiazepin-2-one

Following General Procedure 8-C using 3-[N'-(*tert*-butylcarbamate)-L-alaninyl]-amino-5-cyclohexyl-1,3-dihydro-1-methyl-2H-1,4-benzodiazepin-2-one, the title intermediate was prepared as a white foam. The crude material was used immediately.
C₁₉H₂₆N₄O₂ (M+H = 343.2136); mass spectroscopy found 343.2139.

### Example 8-H

### Synthesis of 3-(L-Alaninyl)amino-2,3-dihydro-1-methyl-7-nitro-5-phenyl-1H-1,4-benzodiazepin-2-one

### Step A - Preparation of 2-[N-(α-Isopropylthio)-N'-(benzyloxycarbonnyl)-glycinyl]-amino-5-nitrobenzophenone

A solution of α-(isopropylthio)-N-(benzyloxycarbonyl)glycine (1 eq; prepared according to Zoller, V.; Ben-Ishai, D. Tetrahedron 1975, 31, 863.) in dry THF was cooled to 0 °C and treated with oxalyl chloride (1 eq.) and 3 drops of DMF. After stirring for 15 minutes at 0°C, the cooling bath was removed and stirring continued at ambient temperature for 40 minutes. The solution was recooled to 0°C. A solution of 2-amino-5-nitrobenzophenone (0.9 eq.; Acros) and 4-methylmorpholine (2.0 eq.) in dry THF was added via cannulation to the acid chloride. The cooling bath was removed and the reaction stirred at ambient for 5 hours. The reaction was diluted with methylene chloride and washed with 0.5 M citric acid, saturated aqueous NaHCO₃, and brine. The organic phase was dried over Na₂SO₄, filtered, and concentrated. The residue was purified via preparative LC2000 eluting with a gradient of 15→20% ethyl acetate/hexanes giving an off-white foam.
C₂₆H₂₅N₃O₆S (MW = 507.61); mass spectroscopy found 507.9.
Anal. calcd for C₂₆H₂₅N₃O₆S: C, 61.53; H, 4.96; N, 8.28. Found: C, 61.70; H, 4.99; N, 8.22.

### Step B - Preparation of 2-[N-(α-Amino)-N'-(benzyloxycarbonyl)-glycinyl]-amino-5-nitrobenzophenone

Ammonia gas was bubbled into a solution 2-[N-(α-isopropylthio)-N'-(benzyloxycarbonyl)-glycinyl]-amino-5-nitrobenzophenone (1 eq) in THF at 0°C. After 35 minutes mercury(II) chloride (1.1 eq) was added. The ice bath was removed and ammonia gas was continued to bubble through the suspension for 4 hours. The bubbler was removed and the reaction continued to stir for 16 hours. The mixture was filtered through celite washing with THF. The filtrate was concentrated *in vacuo.* The crude solid was used in step C without further purification.

### Step C - Preparation of 3-(Benzyloxycarbonyl)-amino-2,3-dihydro-7-nitro-5-phenyl-1H-1,4-benzodiazepin-2-one

2-[N-(α-Amino)-N'-(benzyloxycarbonyl)-glycinyl]-amino-5-nitrobenzophenone (1 eq) was treated with glacial acetic acid and ammonium acetate (4.7 eq). The suspension was stirred at ambient temperature for 21 hours. After concentrating the reaction in vacuo, the residue was partitioned between ethyl acetate and 1 N NaOH. The aqueous layer was back-extracted with ethyl acetate. The combined organics were washed with brine, dried over Na₂SO₄, filtered, and concentrated. The residue was purified via flash chromatography eluting with a gradient of 2→3% isopropyl alcohol/methylene chloride.
C₂₃H₁₈N₄O₅ (MW = 430.45); mass spectroscopy found (M+H) 431.2.
Anal. calcd for C₂₃H₁₈N₄O₅: C, 64.18; H, 4.22; N, 13.02. Found: C, 64.39; H, 4.30; N, 13.07.

### Step D - Preparation of 3-(Benzyloxycarbonyl)-amino-2,3-dihydro-1-methyl-7-nitro-5-phenyl-1H-1,4-benzodiazepin-2-one

Following General Procedure 8-A and using 3-(benzyloxycarbonyl)-amino-2,3-dihydro-7-nitro-5-phenyl-1H-1,4-benzodiazepin-2-one, the title intermediate was prepared as a yellow foam.
C₂₄H₂₀N₄O₅ (MW = 444.48); mass spectroscopy found (M+H) 445.2.
Anal. calcd for C₂₄H₂₀N₄O₅: C, 64.86; H, 4.54; N, 12.60. Found: C, 65.07; H, 4.55; N, 12.46.

### Step E - Preparation of 3-Amino-1,3-dihydro-1-methyl-7-nitro-5-phenyl-2H-1,4-benzodiazepin-2-one

Following General Procedure 8-B and using 3-(benzyloxycarbonyl)-amino-2,3-dihydro-1-methyl-7-nitro-5-phenyl-1H-1,4-benzodiazepin-2-one, the title intermediate was prepared as a yellow foam which was used immediately in Step F.

### Step F - Preparation of 3-[N'-(tert-Butylcarbamate)-L-alaninyl]-amino-2,3-dihydro-1-methyl-7-nitro-5-phenyl-1H-1,4-benzodiazepin-2-one

Following General Procedure D using N-Boc-L-alanine and 3-amino-1,3-dihydro-1-methyl-7-nitro-5-phenyl-2H-1,4-benzodiazepin-2-one, the title intermediate was prepared as a yellow solid.
C₁₄H₂₇N₅O₆ (MW = 481.56); mass spectroscopy found (M+H) 482.3.
Anal. calcd for C₂₄H₂₇N₅O₆: C, 59.88; H, 5.61; N, 14.55. Found: C, 60.22; H, 5.75; N, 13.91.

### Step G - Preparation of 3-(L-Alaninyl)-amino-2,3-dihydro-1-methyl-7-nitro-5-phenyl-1H-1,4-benzodiazepin-2-one

Following General Procedure 8-C using 3-[N'-(*tert*-butylcarbamate)-L-alaninyl]-amino-2,3-dihydro-1-methyl-7-nitro-5-phenyl-1H-1,4-benzodiazepin-2-one, the title intermediate was prepared as a yellow foam. The crude material was used immediately.

### Example 8-1

### Synthesis of 3-(L-Alaninyl)amino-2,3-dihydro-1-methyl-5-(2-fluorophenyl)-1H-1,4-benzodiazepin-2-one

### Step A - Preparation of 3-Amino-1,3-dihydro-1-methyl-5-(2-fluoronhenyl)-2H-1,4-benzodiazepin-2-one

A flask was charged with 3-(benzyloxycarbonyl)-amino-7-bromo-2,3-dihydro-1-methyl-5-(2-fluorophenyl)-1H-1,4-benzodiazepin-2-one (1 eq.; Example 8-D, Step A) and 10% palladium on carbon. Methanol was added, and the flask was placed under a balloon of H₂. The reaction was stirred for 21 hours. The mixture was filtered through celite washing with methanol. The filtrate was concentrated to a white solid.

C₁₆H₁₄FN₃O(MW = 283.33); mass spectroscopy found (M+H) 284.1.

### Step B - Preparation of 3-[N'-(tert-Butylcarbamate)-L-alaninyl]-amino-1,3-dihydro-1-methyl-5-(2-fluorophenyl)-2H-1,4-benzodiazepin-2-one

Following General Procedure D using N-Boc-L-alanine and 3-amino-1,3-dihydro-1-methyl-5-(2-fluorophenyl)-2H-1,4-benzodiazepin-2-one, the title intermediate was prepared as a white solid.
C₂₄H₂₇FN₄O₄ (MW = 454.50); mass spectroscopy found (M+H) 455.4.
Anal. calcd for C₂₄H₂₇FN₄O₄: C, 63.44; H, 5.95; N, 12.33. Found: C, 63.64; H, 6.08; N, 12.16.

### Step C - Preparation of 3-(L-Alaninyl)-amino-7-bromo-1,3-dihydro-1-methyl-5-(2-fluorophenyl)-2H-1,4-benzodiazepin-2-one

Following General Procedure 8-C using 3-[N'-(*tert*-butylcarbamate)-L-alaninyl]-amino-1,3-dihydro-1-methyl-5-(2-fluorophenyl)-2H-1,4-benzodiazepin-2-one, the title intermediate was prepared as a white foam. The crude material was used immediately.

### Example 8-J

### Synthesis of 3-(L-Alaninyl)-amino-2,3-dihydro-1-methyl-5-(3-nuorophenyl)-1H-1,4-benzodiazepin-2-one

### Step A - Preparation of 2-Amino-3'-fluorobenzophenone

A solution of 3-bromofluorobenzene (1 eq.) in THF was cooled to -78°C under nitrogen and treated with *tert*-butyllithium (2.05 eq., 1.6 M solution in pentane) at a rate of 40 ml/h. The internal temperature did not rise above-74°C. The orange solution was stirred at -78°C for 30 minutes prior to the addition of anthranilonitrile (0.6 eq.) as a solution in THF. The reaction was warmed to 0°C and stirred for 2 hours. 3N HCl was added to the mixture and stirring continued for 30 minutes. The reaction was diluted with ethyl acetate and the layers were separated. The aqueous layer was back-extracted thrice with ethyl acetate. The combined extracts were washed with brine, dried over Na₂SO₄, filtered, and concentrated. The residue was purified via HPLC eluting with 93:7 hexanes/ethyl acetate.
C₁₃H₁₀NO (MW = 215.24); mass spectroscopy found (M+H) 216.3.
¹H NMR (300 MHz, CDCl₃) d 7.44-7.19 (6H, m), 6.74 (1H, d, J=8.0 Hz), 6.61 (1H, dd, J=0.94, 7.9 Hz), 6.10 (2H, bs).

### Step B - Preparation of 2-[N-(α-Isopropylthio)-N'-(benzyloxycarbonyl)-glycinyl]-amino-3'-fluorobenzophenone

A solution of α-(isopropylthio)-N-(benzyloxycarbonyl)glycine (1 eq; prepared according to Zoller, V.; Ben-Ishai, D. Tetrahedron 1975, 31, 863.) in dry THF was cooled to 0°C and treated with oxalyl chloride (1 eq.) and 3 drops of DMF. After stirring for 15 minutes at 0°C, the cooling bath was removed and stirring continued at ambient temperature for 40 minutes. The solution was recooled to 0°C. A solution of 2-amino-3'-fluorobenzophenone (0.9 eq.) and 4-methylmorpholine (2.0 eq.) in dry THF was added via cannulation to the acid chloride. The cooling bath was removed and the reaction stirred at ambient for 5 hours. The reaction was diluted with methylene chloride and washed with 0.5 M citric acid, saturated aqueous NaHCO₃, and brine. The organic phase was dried over Na₂SO₄, filtered, and concentrated. The residue was purified via preparative LC2000 eluting with a gradient of 15→20% ethyl acetate/hexanes giving an off-white foam.
C₂₆H₂₅N₂O₄S (MW = 480.60); mass spectroscopy found (M+NH₄⁺) 498.3.
¹H NMR (300 MHz, CDCl₃) d 11.39 (1H, s), 8.59 (1H, d, J=6.0 Hz), 7.63-7.55 (2H, m), 7.48-7.27 (9H, m), 7.14 (1H, dt, J=1.2, 8.4 Hz), 5.94 (1H, d, J=7.2 Hz), 5.58 (1H, d, J=8.7 Hz), 5.17 (2H, ABq, J=14.7 Hz), 3.25 (1H, sep, J=6.6 Hz), 1.44 (3H, d, J=6.0 Hz), 1.28 (3H, d, J=6.6 Hz).

### Step C - Preparation of 2-[N-(α-Amino)-N'-(benzyloxycarbonyl)-glycinyl]-amino-3'-fluorobenzophenone

Ammonia gas was bubbled into a solution 2-[N-(α-isopropylthio)-N'-(benzyloxycarbonyl)-glycinyl]-amino-3'-fluorobenzophenone (1 eq) in THF at 0°C. After 35 minutes mercury(II) chloride (1.1 eq) was added. The ice bath was removed and ammonia gas was continued to bubble through the suspension for 4 hours. The bubbler was removed and the reaction continued to stir for 16 hours. The mixture was filtered through celite washing with THF. The filtrate was concentrated *in vacuo*. The crude solid was used in step D without further purification.

### Step D - Preparation of 3-(Benzyloxycarbonyl)-amino-2.3-dihydro-5-(3-fluorophenyl)-1H-1,4-benzodiazepin-2-one

2-[N-(α-Amino)-N'-(benzyloxycarbonyl)-glycinyl]-amino-3'-fluorobenzophenone (1 eq) was treated with glacial acetic acid and ammonium acetate (4.7 eq). The suspension was stirred at ambient temperature for 21 hours. After concentrating the reaction in vacuo, the residue was partitioned between ethyl acetate and 1 N NaOH. The aqueous layer was back-extracted with ethyl acetate. The combined organics were washed with brine, dried over Na₂SO₄, filtered, and concentrated. The residue was purified via flash chromatography eluting with a gradient of 2→3% isopropyl alcohol/methylene chloride.
C₂₃H₁₈FN₃O₃ (MW = 403.44); mass spectroscopy found (M+H) 404.4.
Anal. calcd for C₂₃H₁₈FN₃O₃•0.5H₂O: C, 66.98; H, 4.64; N, 10.18. Found: C, 67.20; H, 4.64; N, 9.77.

### Step E - Preparation of 3-(Benzyloxycarbonyl)-amino-2,3-dihydro-1-methyl-5-(3-fluorophenyl)-1H-1,4-benzodiazepin-2-one

Following General Procedure 8-A and using 3-(benzyloxycarbonyl)-amino-2,3-dihydro-5-(3-fluorophenyl)-1H-1,4-benzodiazepin-2-one, the title intermediate was prepared as a yellow foam.
C₂₄H₂₀FN₃O₃ (MW = 417.47); mass spectroscopy found (M+H) 418.3.
Anal. calcd for C₂₄H₂₀FN₃O₃: C, 69.06; H, 4.83; N, 10.07. Found: C, 69.33; H, 4.95; N, 9.82.

### Step F - Preparation of 3-Amino-1,3-dihydro-1-methyl-5-(3-fluorophenyl)-2H-1,4-benzodiazepin-2-one

Following General Procedure 8-B and using 3-(benzyloxycarbonyl)-amino-2,3-dihydro-1-methyl-5-(3-fluorophenyl)-1H-1,4-benzodiazepin-2-one, the title intermediate was prepared as a yellow foam which was used immediately in Step G.

### Step G - Preparation of 3-[N'-(tert-Butylcarbamate)-L-alaninyl]-amino-2,3-dihydro-1-methyl-5-(3-fluorophenyl)-1H-1,4-benzodiazepin-2-one

Following General Procedure D using N-Boc-L-alanine and 3-amino-1,3-dihydro-1-methyl-5-(3-fluorophenyl)-2H-1,4-benzodiazepin-2-one, the title intermediate was prepared as a yellow solid.
C₂₄H₂₇FN₄O₄ (MW = 454.50); mass spectroscopy found (M+H) 455.3.
Anal. calcd for C₂₄H₂₇FN₄O₄: C, 63.42; H, 5.99; N, 12.33. Found: C, 63.34; H, 6.01; N, 12.08.

### Step H - Preparation of 3-(L-Alaninyl)-amino-2,3-dihydro-1-methyl-5-(3-fluorophenyl)-1H-1,4-benzodiazepin-2-one

Following General Procedure 8-C using 3-[N'-(*tert*-butylcarbamate)-L-alaninyl]-amino-2,3-dihydro-1-methyl-5-(3-fluorophenyl)-1H-1,4-benzodiazepin-2-one, the title intermediate was prepared as a yellow foam. The crude material was used immediately.

### Example 8-K

### Synthesis of 3-(L-Alaninyl)amino-2,3-dihydro-1-methyl-5-(4-fluorophenyl)-1H-1,4-benzodiazepin-2-one

### Step A - Preparation of 2-Amino-4'-fluorobenzophenone

A solution of 4-bromofluorobenzene (1 eq.) in THF was cooled to -78°C under nitrogen and treated with *tert*-butyllithium (2.05 eq., 1.6 M solution in pentane) at a rate of 40 ml/h. The internal temperature did not rise above - 74°C. The orange solution was stirred at -78°C for 30 minutes prior to the addition of anthranilonitrile (0.6 eq.) as a solution in THF. The reaction was warmed to 0°C and stirred for 2 hours. 3N HCl was added to the mixture and stirring continued for 30 minutes. The reaction was diluted with ethyl acetate and the layers were separated. The aqueous layer was back-extracted thrice with ethyl acetate. The combined extracts were washed with brine, dried over Na₂SO₄, filtered, and concentrated. The residue was purified via HPLC eluting with 93:7 hexanes/ethyl acetate.
C₁₃H₁₀FNO (MW = 215.24); mass spectroscopy found (M+H) 216.3.
Anal. calcd for C₁₃H₁₀FNO: C, 72.55; H, 4.68; N, 6.51. Found: C, 72.80; H, 4.51; N, 6.74.

### Step B - Preparation of 2-[N-(α-Isopropylthio)-N'-(benzyloxycarbonyl)-glycinyl]-amino-4'-fluorobenzophenone

A solution of α-(isopropylthio)-N-(benzyloxycarbonyl)glycine (1 eq; prepared according to Zoller, V.; Ben-Ishai, D. Tetrahedron 1975, 31, 863.) in dry THF was cooled to 0°C and treated with oxalyl chloride (1 eq.) and 3 drops of DMF. After stirring for 15 minutes at 0°C, the cooling bath was removed and stirring continued at ambient temperature for 40 minutes. The solution was recooled to 0°C. A solution of 2-amino-4'-fluorobenzophenone (0.9 eq.) and 4-methylmorpholine (2.0 eq.) in dry THF was added via cannulation to the acid chloride. The cooling bath was removed and the reaction stirred at ambient for 5 hours. The reaction was diluted with methylene chloride and washed with 0.5 M citric acid, saturated aqueous NaHCO₃, and brine. The organic phase was dried over Na2SO4, filtered, and concentrated. The residue was purified via preparative LC2000 eluting with a gradient of 15→20% ethyl acetate/hexanes giving an off-white foam.
C₂₆H₂₅N₂O₄S (MW = 480.60); mass spectroscopy found (M+NH₄⁺) 498.2.
¹H NMR (300 MHz, CDCl₃) d 11.28 (1H, s), 8.56 (1H, d, J=8.4 Hz), 7.78-7.73 (2H, m), 7.61-7.53 (2H, m), 7.36-7.32 (5H, m), 7.20-7.14 (3H, m), 5.98 (1H, d, J=7.5 Hz), 5.57 (1H, d, J=7.8 Hz), 5.16 (2H, ABq, J=14.7 Hz), 3.25 (1H, sep, J=6.0 Hz), 1.43 (3H, d, J=6.3 Hz), 1.27 (3H, d, J=6.6 Hz).

### Step C - Preparation of 2-[N-(α-Amino)-N'-(benzyloxycarbonyl)-glycinyl]-amino-4'-fluorobenzophenone

Ammonia gas was bubbled into a solution 2-[N-(α-isopropylthio)-N'-(benzyloxycarbonyl)-glycinyl]-amino-3'-fluorobenzophenone (1 eq) in THF at 0°C. After 35 minutes mercury(II) chloride (1.1 eq) was added. The ice bath was removed and ammonia gas was continued to bubble through the suspension for 4 hours. The bubbler was removed and the reaction continued to stir for 16 hours. The mixture was filtered through celite washing with THF. The filtrate was concentrated *in vacuo.* The crude solid was used in step D without further purification.

### Step D - Preparation of 3-(Benzyloxvcarbonyl)amino-2.3-dihvdro-5-(4-fluorophenyl)-1H-1,4-benzodiazepin-2-one

2-[N-(α-Amino)-N'-(benzyloxycarbonyl)-glycinyl]-amino-4'-fluorobenzophenone (1 eq) was treated with glacial acetic acid and ammonium acetate (4.7. eq). The suspension was stirred at ambient temperature for 21 hours. After concentrating the reaction in vacuo, the residue was partitioned between ethyl acetate and 1 N NaOH. The aqueous layer was back-extracted with ethyl acetate. The combined organics were washed with brine, dried over Na₂SO₄, filtered, and concentrated. The residue was purified via flash chromatography eluting with a gradient of 2→3% isopropyl alcohol/methylene chloride.
C₂₃H₁₈FN₃O₃ (MW = 403.44); mass spectroscopy found (M+H) 404.4.
Anal. calcd for C₂₃H₁₈FN₃O₃•1.25H₂O: C, 64.85; H, 4.85. Found: C, 64.80; H, 4.55.

### Step E - Preparation of 3-(Benzyloxycarbonyl)-amino-2,3-dihydro-1-methyl-5-(4-fluorophenyl)-1H-1,4-benzodiazepin-2-one

Following General Procedure 8-A and using 3-(benzyloxycarbonyl)-amino-2,3-dihydro-5-(4-fluorophenyl)-1H-1,4-benzodiazepin-2-one, the title intermediate was prepared as a yellow foam.
C₂₄H₂₀FN₃O₃ (MW = 417.47); mass spectroscopy found (M+H) 418.2.
Anal. calcd for C₂₄H₂₀FN₃O₃: C, 69.06; H, 4.83; N, 10.07. Found: C, 69.35; H, 4.93; N, 9.97.

### Step F - Preparation of 3-Amino-1,3-dihydro-1-methyl-5-(4-fluorophenyl)-2H-1,4-benzodiazepin-2-one

Following General Procedure 8-B and using 3-(benzyloxycarbonyl)-amino-2,3-dihydro-1-methyl-5-(4-fluorophenyl)-1H-1,4-benzodiazepin-2-one, the title intermediate was prepared as a yellow foam which was used immediately in Step G.

### Step G - Preparation of 3-[N'-(tert-Butylcarbamate)-L-alaninyl]-amino-2,3-dihydro-1-methyl-5-(3-fluorophenyl)-1H-1,4-benzodiazepin-2-one

Following General Procedure D using N-Boc-L-alanine and 3-amino-1,3-dihydro-1-methyl-5-(3-fluorophenyl)-2H-1,4-benzodiazepin-2-one, the title intermediate was prepared as a yellow solid.
C₂₄H₂₇FN₄O₄ (MW = 454.50); mass spectroscopy found (M+H) 455.4.
Anal. calcd for C₂₄H₂₇FN₄O₄•1.5H₂O: C, 59.86; H, 6.28; N, 11.64. Found: C, 60.04; H, 5.62; N, 11.27.

### Step H - Preparation of 3-(L-Alaninyl)-amino-2,3-dihydro-1-methyl-5-(4-fluorophenyl)-1H-1,4-benzodiazepin-2-one

Following General Procedure 8-C using 3-[N'-(*tert*-butylcarbamate)-L-alaninyl]-amino-2,3-dihydro-1-methyl-5-(4-fluorophenyl)-1H-1,4-benzodiazepin-2-one, the title intermediate was prepared as a yellow foam. The crude material was used immediately.

### Example 8-L

### Synthesis of 3-(N'-L-Alaninyl)amino-2,3-dihydro-1-isobutyl-5-phenyl-1H-1,4-benzodiazepin-2-one

Step A: 1,3-Dihydro-5-phenyl-2H-1,4-benzodiazepin-2-one (prepared according to the procedure of M. G. Bock et al., J. Org. Chem. 1987, 52, 3232-3239) was alkylated with isobutyl iodide using General Procedure 8-G to afford 1,3-dihydro-1-isobutyl-5-phenyl-2H-1,4-benzodiazepin-2-one.
Step B: Following General Procedures 8-D and 8-F and using the product from Step A, 3-amino-1,3-dihydro-1-isobutyl-5-phenyl-2H-1,4-benzodiazepin-2-one was prepared.
Step C: The product from Step B and N-Boc-L-alanine (Sigma) were coupled using General Procedure D, followed by removal of the Boc group using General Procedure 8-J, to afford 3-(N'-L-alaninyl)amino-1,3-dihydro-1-isobutyl-5-phenyl-2H-1,4-benzodiazepin-2-one.

By substituting isopropyl iodide, n-propyl iodide, cyclopropylmethyl iodide and ethyl iodide for isobutyl iodide in Step A above, the following additional intermediates were prepared:
3-(N'-L-alaninyl)amino-1,3-dihydro-1-isopropyl-5-phenyl-2H-1,4-benzodiazepin-2-one
3-(N'-L-alaninyl)amino-1,3-dihydro-1-propyl-5-phenyl-2H-1,4-benzodiazepin-2-one
3-(N'-L-alaninyl)amino-1,3-dihydro-1-cyclopropylmethyl-5-phenyl-2H-1,4-benzodiazepin-2-one
3-(N'-L-alaninyl)amino-1,3-dihydro-1-ethyl-5-phenyl-2H-1,4-benzodiazepin-2-one.

### Example 8-M

### Synthesis of 3-(N'-L-Alaninyl)amino-1-methyl-5-phenyl-1,3,4,5-tetrahydro-2H-1,5-benzodiazepin-2-one

Step A: 1,3,4,5-Tetrahydro-5-phenyl-2H-1,5-benzodiazepin-2-one (CAS No. 32900-17-7) was methylated using General Procedure 8-1 to afford 1-methyl-5-phenyl-1,3,4,5-tetrahydro-2H-1,5-benzodiazepin-2-one.
Step B: Following General Procedures 8-E and 8-F and using the product from Step A, 3-amino-1-methyl-5-phenyl-1,3,4,5-tetrahydro-2H-1,5-benzodiazepin-2-one was prepared.
Step C: The product from Step B and N-Boc-L-alanine (Sigma) were coupled using General Procedure D, followed by removal of the Boc group using General Procedure 8-N, to afford 3-(N'-L-alaninyl)amino-1-methyl-5-phenyl-1,3,4,5-tetrahydro-2H-1,5-benzodiazepin-2-one.

### Example 8-N

### Synthesis of 3-(N'-L-Alaninyl)amino-2,4-dioxo-1-methyl-5-phenyl-2,3,4,5-tetrahydro-1H-1,5-benzodiazepine

3-Amino-2,4-dioxo-1-methyl-5-phenyl-2,3,4,5-tetrahydro-1 H-1,5-benzodiazepine (CAS No. 131604-75-6) was coupled with N-Boc-L-alanine (Sigma) using General Procedure D, followed by removal of the Boc group using General Procedure 8-N, to afford the title compound.

### Example 8-O

### Synthesis of 3-((R)-Hydrazinopropionyl)amino-2,3-dihydro-1-methyl-5-phenyl)-1H-1,4-benzodiazepin-2-one

3-Amino-2,3-dihydro-1-methyl-5-phenyl-1H-1,4-benzodiazepin-2-one was coupled to (R)-N,N'-di-BOC-2-hydrazinopropionic acid (Example N) using General Procedure D. Removal of the Boc group using General Procedure 5-B afforded the title compound.

### Example 8-P

### Synthesis of 3-Amino-2,4-dioxo-1,5-bis-(1-methylethyl)-2,3,4,5-tetrahydro-1H-1,5-benzodiazepine

### Step A: - Synthesis of 2,4-Dioxo-2,3,4,5-tetrahydro-1H-1,5-benzodiazepine

2,4-Dioxo-2,3,4,5-tetrahydro-1H-1,5-benzodiazepine (CAS No. 49799-48-6) was prepared from 1,2-phenylenediamine (Aldrich) and malonic acid (Aldrich) using the procedure of Claremon, D. A.; et al, PCT Application: WO 96-US8400 960603.

### Step B: - Synthesis of 2,4-Dioxo-1,5-bis-(1-methylethyl)-2,3,4,5-tetrahydro-1H-1,5-benzodiazepine

2,4-Dioxo-1,5-bis-(1-methylethyl)-2,3,4,5-tetrahydro-1H-1,5-benzodiazepine (CAS No. 113021-84-4) was prepared following General Procedure 8-M using the product from Step A and 2-iodopropane (Aldrich). Purification was by flash chromatography eluting with EtOAc/hexanes (3:7 gradient to 1:1), then recrystalization from EtOAc/hexanes.

### Step C: - Synthesis of 3-Azido-2,4-dioxo-1,5-bis-(1-methylethyl)-2,3,4,5-tetrahydro-1H-1,5-benzodiazepine

Following General Procedure 8-K using the product from Step B, 3-azido-2,4-dioxo-1,5-bis-(1-methylethyl)-2,3,4,5-tetrahydro-1H-1,5-benzodiazepine (CAS No. 186490-50-6) was prepared as a white solid. The product was purified by flash chromatography eluting with hexanes/EtOAc (4: 1) to provide a separable 23:1 mixture of pseudo-axial/pseudo-equatorial azides. The pure pseudo-axial azide was used in the next step.

### Step D: - Synthesis of 3-Amino-2,4-dioxo-1,5-bis-(1-methylethyl)-2,3,4,5-tetrahydro-1H-1,5-benzodiazepine

Following General Procedure 8-L using the product from Step C, 3-amino-2,4-dioxo-1,5-bis-(1-methylethyl)-2,3,4,5-tetrahydro-1H-1,5-benzodiazepine (CAS No. 186490-51-7) was prepared as a white solid. Purification was by flash chromatography eluting with CH₂Cl₂/MeOH (98:2 gradient to 95:5). The isolated pseudo-axial amine atropisomer was completely converted to the pseudo-equatorial amine atropisomer by heating in toluene to 100-105 °C for 15 minutes, and the pseudo-equatorial amine atropisomer was used in the next step. The isomers were distinguished by ¹H-NMR in CDCl₃. Selected ¹H-NMR (CDCl₃): Pseudo-axial amine 4.40 (s, 1H); Pseudo-equatorial amine 3.96 (s, 1H).

### Example 8-Q

### Synthesis of 3-(R-2-Thienylglycinyl)amino-2,4-dioxo-1,5-bis-(1-methylethyl)-2,3,4,5-tetrahydro-1H-1,5-benzodiazepine Hydrochloride

### Step A: - Synthesis of N-(t-Butoxycarbonyl)-R-2-thienylglycine

N-(*t*-Butoxycarbonyl)-R-2-thienylglycine (CAS No. 74462-03-1) was prepared from L-α-(2-thienyl)glycine (Sigma) by the procedure described in Bodansky, M. et al; The Practice of Peptide Synthesis; Springer Verlag; 1994, p. 17.

### Step B: - Synthesis of 3-[N'-(t-Butoxycarbonyl)-R-2-thienylglycinyl]-amino-1,4-dioxo-1,5-bis-(1-methylethyl)-1,3,4,5-tetrahydro-1H-1,5-benzodiazepine

Following General Procedure J above using the product from Example 8-P and the product from Step A above, 3-[N'-(t-butoxycarbonyl)-R-2-thienylglycinyl]-amino-2,4-dioxo-1,5-bis-(1-methylethyl)-2,3,4,5-tetrahydro- 1H-1,5-benzodiazepine was prepared as a white foam. Purification was by flash chromatography eluting with CH₂Cl₂/EtOAc (9:1 gradient to 5:1).

### Step C: - Synthesis of 3-(R-2-Thienylglycinyl)amino-2,4-dioxo-1,5-bis-(1-methylethyl)-2,3,4,5-tetrahydro-1H-1,5-benzodiazepine Hydrochloride

Following General Procedure 8-N above using the product from Step B, the title compound was prepared as a white solid.

### Example 8-R

### Synthesis of 3-(L-Alaninyl)-amino-2,4-dioxo-1,5-bis-methyl-2,3,4,5-tetrahydro-1H-1,5-benzodiazepine Hydrochloride

### Step A: - Synthesis of 2,4-Dioxo-1,5-bis-methyl-2,3,4,5-tetrahydro-1H-1,5-benzodiazepine

2,4-Dioxo-1,5-bis-methyl-2,3,4,5-tetrahydro-1H-1,5-benzodiazepine (CAS No. 23954-54-3) was prepared following General Procedure 8-M using the product from Example 8-P, Step A and iodomethane (Aldrich). The white solid product precipitated during partial concentration of the reaction after work-up, and was isolated by filtration.

### Step B: - Synthesis of 3-Azido-2,4-dioxo-1,5-bis-methyl-2,3,4,5-tetrahydro-1H-1,5-benzodiazepine

For this substrate, General Procedure 8-K was modified in the following manner. Initially the product from Step A was suspended. (not a solution) in THF at -78°C, and following addition of the KN(TMS)₂ solution, this suspension was allowed to warm to -35°C over a period of 12 minutes, during which the suspension became a solution, and was re-cooled to -78°C; then treated as described in the General Procedure. 3-Azido-2,4-dioxo-1,5-bis-methyl-2,3,4,5-tetrahydro-1H-1,5-benzodiazepine was purified by flash chromatography eluting with CHCl₃/EtOAc (7:1), then trituration from hot CHCl₃ with hexanes and cooled to -23°C. The product was isolated as a white solid.

### Step C: - Synthesis of 3-Amino-2,4-dioxo-1,5-bis-methyl-2,3,4,5-tetrahydro-1H-1,5-benzodiazepine

Following General Procedure 8-L using the product from Step B, 3-amino-2,4-dioxo-1,5-bis-methyl-2,3,4,5-tetrahydro-1H-1,5-benzodiazepine was prepared as a white solid. The crude product was used without further purification.

### Step D: - Synthesis of 3-[N'-(t-Butoxycarbonyl)-L-alaninyl]-amino-2,4-dioxo-1,5-bis-methyl-2,3,4,5-tetrahydro-1H-1,5-benzodiazepine

Following General Procedure I above using N-Boc-L-alanine (Novabiochem) and the product from Step C, 3-[N'-(*t*-butoxycarbonyl)-L-alaninyl]-amino-2,4-dioxo-1,5-bis-methyl-2,3,4,5-tetrahydro-1H-1,5-benzodiazepine was prepared as a white foam. Purification was by flash chromatography eluting with CH₂Cl₂/EtOAc (2:1 gradient to 1:1).

### Step E: - Synthesis of 3-(L-alaninyl)-amino-2,4-dioxo-1,5-bis-methyl-2,3,4,5-tetrahydro-1H-1,5-benzodiazepine Hydrochloride

Following General Procedure 8-N above using the product from Step D, the title compound was prepared as an off-white amorphous solid.

### Example 8-S

### Synthesis of 3-(L-Alaninyl)amino-2,4-dioxo-1,5-bis-(2-methylpropyl)-2,3,4,5-tetrahydro-1H-1,5-benzodiazepine Hydrochloride

### Step A: - Synthesis of 2,4-Dioxo-1,5-bis-(2-methylpropyl)-2,3,4,5-tetrahydro-1H-1,5-benzodiazepine

2,4-Dioxo-1,5-bis-(2-methylpropyl)-2,3,4,5-tetrahydro-1H-1,5-benzodiazepine was prepared following General Procedure 8-M using the product from Example 8-P, Step A and 1-iodo-2-methylpropane (Aldrich). Purification was by flash chromatography eluting with EtOAc/hexanes (3:7 gradient to 1:1), then recrystalization from EtOAc/hexanes.

### Step B: - Synthesis of 3-Azido-2,4-dioxo-1,5-bis-(2-methylpropyl)-2,3,4,5-tetrahydro-1H-1,5-benzodiazepine

Following General Procedure 8-K (a precipitate formed during the addition of the KN(TMS)₂, but dissolved upon addition of the trisyl azide) using the product from Step A, 3-azido-2,4-dioxo-1,5-bis-(2-methylpropyl)-2,3,4,5-tetrahydro-1H-1,5-benzodiazepine was prepared as a white solid. The product was purified by flash chromatography eluting with hexanes/EtOAc (4:1) and a second flash chromatography eluting with CH₂Cl₂/hexanes/EtOAc (10:10:1 gradient to 8:6:1).

### Step C: - Synthesis of 3-Amino-2,4-dioxo-1,5-bis-(2-methylpropyl) 2,3,4,5-tetrahydro-1H-1,5-benzodiazepine.

Following General Procedure 8-L using the product from Step B, 3-amino-2,4-dioxo-1,5-bis-(2-methylpropyl)-2,3,4,5-tetrahydro-1H-1,5-benzodiazepine was prepared as a white solid. Purification was by flash chromatography eluting with CH₂Cl₂/MeOH (98:2 gradient to 95:5, with 5% NH₃ in the MeOH).

### Step D: - Synthesis of 3-(N'-(t-Butoxycarbonyl)-L-alaninyl)-amino-2,4-dioxo-1,5-bis-(2-methylpropyl)-2,3,4,5-tetrahydro-1H-1,5-benzodiazepine

Following General-Procedure I above using N-Boc-L-alanine (Novabiochem) and the product from Step C, 3-[N'-(*t*-butoxycarbonyl)-L-alaninyl]-amino-2,4-dioxo-1,5-bis-(2-methylpropyl)-2,3,4,5-tetrahydro-1H-1,5-benzodiazepine was prepared as a white foam. Purification was by flash chromatography eluting with CH₂Cl₂ÆtOAc (3:1 gradient to 3:2).

### Step E: Synthesis of 3-(L-Alaninyl)-amino-2,4-dioxo-1,5-bis-(2-methylpropyl)-2,3,4,5-tetrahydro-1H-1,5-benzodiazepine Hydrochloride

Following General Procedure 8-N above using the product from Step D, the title compound was prepared as an amorphous white solid.

### Example 8-T

### Synthesis of 3-(S-Phenylglycinyl)amino-2,4-dioxo-1,5-bis-(2-methylpropyl)-2,3,4,5-tetrahydro-1H-1,5-benzodiazepine Hydrochloride

### Step A: - Synthesis of 3-[N'-(t-Butoxycarbonyl)-S-phenylglycinyl]-amino-2,4-dioxo-1,5-bis-(2-methylpropyl)-2,3,4,5-tetrahydro-1H-1,5-benzodiazepine

Following General Procedure J above using the product from Example 8-S, Step C and the Boc-L-phenylglycine (Novabiochem, CAS No. 2900-27-8), 3-[N'-(*t*-butoxycarbonyl)-S-phenylglycinyl]-amino-2,4-dioxo-1,5-bis-(2-methylpropyl)-2,3,4,5-tetrahydro-1H-1,5-benzodiazepine was prepared as a white foam. Purification was by flash chromatography eluting with CH₂Cl₂/EtOAc (9:1 gradient to 5:1).

### Step B: - Synthesis of 3-(S-Phenylglycinyl)-amino-2,4-dioxo-1,5-bis-(2-methylpropyl)-2,3,4,5-tetrahydro-1H-1,5-benzodiazepine Hydrochloride

Following General Procedure 8-N above using the product from Step A, 3-(S-phenylglycinyl)-amino-2,4-dioxo-1,5-bis-(2-methylpropyl)-2,3,4,5-tetrahydro-1H-1,5-benzodiazepine hydrochloride was prepared as an off-white solid.

### Example 8-U

### Synthesis of 3-(L-Alaninyl)amino-2,4-dioxo-1,5-bis-(cyclopropylmethyl)-2,3,4,5-tetrahydro-1H-1,5-benzodiazepine Hydrochloride

### Step A: - Synthesis of 2,4-Dioxo-1,5-bis-(cyclopropylmethyl)-2,3,4,5-tetrahydro-1H-1,5-benzodiazepine

2,4-Dioxo-1,5-bis-(cyclopropylmethyl)-2,3,4,5-tetrahydro-1H-1,5- , benzodiazepine was prepared following General Procedure 8-M using the product from Example 8-P, Step A, and (bromomethyl)cyclopropane (Lancaster). Purification was by flash chromatography eluting with EtOAc/hexanes (3:7 gradient to straight EtOAc), then recrystalization from EtOAc/hexanes.

### Step B: - Synthesis of 3-Azido-2,4-dioxo-1,5-bis-(cyclopropylmethyl)-2,3,4,5-tetrahydro-1H-1,5-benzodiazepine

For this substrate General Procedure 8-K was modified in the following manner. Initially the product from Step A was suspended (not a solution) in THF at -78°C, and following addition of the KN(TMS)₂ solution, this suspension was allowed to warm to -30°C, during which the suspension became a solution, and was re-cooled to -78°C. Upon re-cooling to -78°C a precipitate began to form, therefore the reaction flask containing the mixture was partially raised above the cooling bath until the internal temperature rose to -50°C; then the trisyl azide solution was added. The cooling bath was removed and the mixture allowed to warm to -20°C whereupon the mixture had become a nearly homogenous solution, and the AcOH was added. Then, treated as described in the general procedure. 3-Azido-2,4-dioxo-1,5-bis-(cyclopropylmethyl)-2,3,4,5-tetrahydro-1H-1,5-benzodiazepine was purified by trituration with hot to room temperature EtOAc, followed by recrystalization from hot to -23°C CHCl₃/EtOAc/EtOH (5:5:1) and isolated as a white solid.

### Step C: - Synthesis of 3-Amino-2,4-dioxo-1,5-bis-(cyclopropylmethyl)-2,3,4,5-tetrahydro-1H-1,5-benzodiazepine

Following General Procedure 8-L using the product from Step B, 3-amino-2,4-dioxo-1,5-bis-(cyclopropylmethyl)-2,3,4,5-tetrahydro-1H-1,5-benzodiazepine was prepared as a white solid. Purification was by flash chromatography eluting with CH₂Cl₂MeOH (98:2 gradient to 95:5, with 5% NH₃ in the MeOH) followed by recrystalization from warm CH₂Cl₂/hexanes (1:1) to -23°C.

### Step D: - Synthesis of 3-[N'-(t-Butoxycarbonyl)-L-alaninyl]-amino-2,4-dioxo-1,5-bis-(cyclopropylmethyl)-2,3,4,5-tetrahydro-1H-1,5-benzodiazepine

Following General Procedure I above using N-Boc-L-alanine (Novabiochem) and the product from Step C, 3-[N'-(*t*-butoxycarbonyl)-L-alaninyl]-amino-2,4-dioxo-1,5-bis-(cyclopropylmethyl)-2,3,4,5-tetrahydro-1H-1,5-benzodiazepine was prepared as a white foam. Purification was by flash chromatography eluting with CH₂Cl₂/EtOAc (3:1 gradient to 2:1).

### Step E: - Synthesis of 3-(L-Alaninyl)-amino-2,4-dioxo-1,5-bis-(cyclopropylmethyl)-2,3,4,5-tetrahydro-1H-1,5-benzodiazepine Hydrochloride

Following General Procedure 8-N above using the product from Step D, the title compound was prepared as an off-white solid.

### Example 8-V

### Synthesis of 3-(L-Alaninyl)-amino-2,4-dioxo-1,5-bis-(2,2-dimethylpropyl)-2,3,4,5-tetrahydro-1H-1,5-benzodiazepine Hydrochloride

### Step A: - Synthesis of 2,4-Dioxo-1,5-bis-(2,2-dimethylpropyl)-2,3,4,5-tetrahydro-1H-1,5-benzodiazepine

To a stirred suspension of the product from Example 8-P, Step A (1.0 eq 17.08 g) in DMSO (500 mL) at room temperature was added neopentyl iodide (43.01 g, 2.24 eq., Aldrich) and CS₂CO₃ (72.65 g, 2.3 eq., Aldrich). The resulting mixture was heated to 75°C for 30 minutes, then additional Cs₂CO₃ (31.59 g, 1.0 eq.) was added and the mixture rapidly stirred at 75°C for 6 hours. The mixture was allowed to cool and H₂O (500 mL) and EtOAc (1000 mL) were added. The phases were partitioned and the organic phase washed with H₂O (1x500 mL), 1M aq. HCl (2x500 mL), and brine (1x500 mL). Then, the organic phase was dried over MgSO₄, filtered, concentrated, and purified by flash chromatography eluting with hexanes/EtOAc (3:2 gradient to 2:3) to provide 2,4-dioxo-1,5-bis-(2,2-dimethylpropyl)-2,3,4,5-tetrahydio-1H-1,5-benzodiazepine as a white solid.

### Step B: - Synthesis of 3-Azido-2,4-dioxo-1,5-bis-(2,2-dimethylpropyl)-2,3,4,5-tetrahydro-1H-1,5-benzodiazepine

Following General Procedure 8-K using the product from Step A, 3-azido-2,4-dioxo-1,5-bis-(2,2-dimethylpropyl)-2,3,4,5-tetrahydro-1H-1,5-benzodiazepine was prepared as a white solid. The product was purified by flash chromatography eluting with hexanes/CH₂Cl₂/EtOAc (10:5:1 gradient to 5:5:1) to provide a separable 13:1 mixture of pseudo-axial/pseudo-equatorial azides. The pure pseudo-axial azide was used in the next step. Selected ¹H-NMR (CDCl₃): Pseudo-axial azide 5.12 (s, 1H); Pseudo-equatorial azide 4.03 (s, 1H).

### Step C: - Synthesis of 3-Amino-2,4-dioxo-1,5-bis-(2,2-dimethylpropyl)-2,3,4,5-tetrahydro-1H-1,5-benzodiazepine

Following General Procedure 8-L using the product from Step B, 3-amino-2,4-dioxo-1,5-bis-(2,2-dimethylpropyl)-2,3,4,5-tetrahydro-1H-1,5-benzodiazepine was prepared as a white solid. Purification was by flash chromatography eluting with CH₂Cl₂/MeOH (98:2 gradient to 95:5, with 5% NH₃ in the MeOH). The isolated white solid product was identified as a ~4:1 mixture of pseudo-axial and pseudo-equatorial amines atropisomers by ¹H-NMR. The mixture was heated in toluene to 100°C for 20 minutes, then re-concentrated to provide the pure pseudo-equatorial amine atropisomer, as a white solid, and this was for the next step. Selected ¹H-NMR (CDCl₃): Pseudo-axial amine 4.59 (s, 1H); Pseudo-equatorial amine 4.03 (s, 1H).

### Step D: - Synthesis of 3-[N'(t-Butoxycarbonyl)-L-alaninyl]-amino. 2,4-dioxo-1,5-bis-(2,2-dimethylpropyl)-2,3,4,5-tetrahydro-1H-1,5-benzodiazepine

Following General Procedure I above using N-Boc-L-alanine (Novabiochem) and the product from Step C, 3-[N'-(*t*-butoxycarbonyl)-L-alaninyl]-amino-2,4-dioxo-1,5-bis-(2,2-dimethylpropyl)-2,3,4,5-tetrahydro-1H-1,5-benzodiazepine was prepared as a white foam. Purification was by flash chromatography eluting with CH₂Cl₂/EtOAc (4:1 gradient to 5:2).

### Step E: - Synthesis of 3-(L-Alaninyl)-amino-2,4-dioxo-1,5-bis-(2,2-dimethylpropyl)-2,3,4,5-tetrahydro-1H-1,5-benzodiazepine Hydrochloride

Following General Procedure 8-N above using the product from Step D, the title compound was prepared as an off-white solid.

### Example 8-W

### Synthesis of 3-(L-Alaninyl)Amino-2,4-dioxo-1,5-bis-phenyl-2,3,4,5-tetrahydro-1H-1,5-benzodiazepine hydrochloride

### Step A: - Synthesis of 2,4-Dioxo-1,5-bis-phenyl-2,3,4,5-tetrahydro₋ 1H-1,5-benzodiazepine

This procedure is a modification of the procedure described in Chan, D. M. T. Tetrahedron Lett. 1996, 37, 9013-9016. A mixture of the product from Example 8-P, Step A (1.0 eq., 7.50 g), Ph₃Bi. (2.2 eq., 41.26 g, Aldrich), Cu(OAc)₂ (2.0 eq., 15.48 g, Aldrich), Et₃N (2.0 eq., 8.62 g) in CH₂Cl₂ (100 mL) was stirred under N₂ at room temperature for 6 days (monitoring by TLC). The solids were removed by filtration through a plug of Celite rinsing with CH₂Cl₂/MeOH (3x75 mL). The filtrate was concentrated, dissolved in hot CH₂Cl₂/MeOH (9:1) and filtered through a large plug of silica gel eluting with CH₂Cl₂/MeOH (9:1, 2L). The filtrate was concentrated and the residue purified by flash chromatography eluting with straight CH₂Cl₂ gradient to CH₂Cl₂/MeOH (9:1). 2,4-Dioxo-1,5-bis-phenyl-2,3,4,5-tetrahydro-1H-1,5-benzodiazepine crystallize during concentration of the fractions containing the product, and was isolated by filtration as a white solid.

### Step B: - Synthesis of 3-Azido-2,4-dioxo-1,5-bis-phenyl-2,3,4,5-tetrahydro-1H-1,5-benzodiazepine

For this substrate, General Procedure 8-K was modified in the following manner. Initially the product from Step A was suspended (not a solution) in THF at -70°C, and following addition of the KN(TMS)₂ solution, this suspension was allowed to warm to -20°C over a period of 10 minutes, during which the suspension became a solution, and was re-cooled to -70°C; then treated as described in the general procedure. The title compound was purified by trituration with hot CHCl₃/hexanes (1:1) to yield 3-azido-2,4-dioxo-1,5-bisphenyl-2,3,4,5-tetrahydro-1H-1,5-benzodiazepine as a white solid.

### Step C: - Synthesis of 3-Amino-2,4-dioxo-1,5-bis-phenyl-2,3,4,5-tetrahydro-1H-1,5-benzodiazepine

Following General Procedure 8-L using the product from Step B, 3-amino-2,4-dioxo-1,5-bis-phenyl-2,3,4,5-tetrahydro-1H-1,5-benzodiazepine was prepared as a white solid. Purification was by flash chromatography eluting with CH₂Cl₂/MeOH (98:2 gradient to 95:5, with 5% NH₃ in the MeOH).

### Step D: - Synthesis of 3-[N'-(t-Butoxycarbonyl)-L-alaninyl]-amino-2,4-dioxo-1,5-bis-phenyl-2,3,4,5-tetrahydro-1H-1,5-benzodiazepine

Following General Procedure I above using N-Boc-L-alanine (Novabiochem) and the product from Step C, 3-[N'-(*t*-butoxycarbonyl)-L-alaninyl]-amino-2,4-dioxo-1,5-bis-phenyl-2,3,4,5-tetrahydro-1H-1,5-benzodiazepine was prepared as a white foam. Purification was by flash chromatography eluting with CH₂Cl₂/EtOAc (4:1 gradient to 3:1).

### Step E: - Synthesis of 3-(L-Alaninyl)-amino-2,4-dioxo-1,5-bis-phenyl-2,3,4,5-tetrahydro-1H-1,5-benzodiazepine Hydrochloride

Following General Procedure 8-N above using the product from Step D, the title compound was prepared as a white amorphous solid.

### Example 8-X

### Synthesis of 3-Amino-2,3-dibydro-1-methyl-5-phehyl-1H-1,4-benzodiazepin-2-one

Following the method of R. G. Sherrill et al., J. Org. Chem., 1995, 60, 730-734 and using glacial acetic acid and HBr gas, the title compound was prepared.

### Example 8-Y

### Synthesis of 3-(L-Valinyl)-amino-2,3-dihydro-1-methyl. 5-phenyl-1H-1,4-benzodiazepin-2-one

### Step A - Synthesis of 3-[N'-(tert-Butylcarbamate)-L-valinyl]-amino-2,3-dihydro-1-methyl-5-phenyl-1H-1,4-benzodiazepin-2-one

(S)-3-Amino-1,3-dihydro-1-methyl-5-phenyl-2H-1,4-benzodiazepin-2-one, (1 S)-7,7-dimethyl-2-oxobicyclo[2.2.]heptane-1-methanesulfonate (Example 8-B, Step A) was free based by partitioning between methylene chloride and 1M potassium carbonate. The free amine was then coupled with N-Boc-valine following General Procedure D to give the title compound.
C₂₆H₃₂N₄O₄ (MW 464.62); mass spectroscopy 464.3.
Anal. Calcd for C₂₆H₃₂N₄O₄: C, 67.22; H, 6.94; N, 12.06. Found: C, 67.29; H, 6.79; N, 11.20.

### Step B - Synthesis of-3-(L-valinyl)-amino-2,3-dihydro-1-methyl-5-phenyl-1H-1,4-benzodiazepin-2-one

Following General Procedure 8-C and using 3-[N'-(*tert*-butylcarbamate)-L-alaninyl]-amino-2,3-dihydro-1-methyl-5-phenyl-1 H-1,4-benzodiazepine-2-one, the title compound was prepared as a white foam.
C₂₁H₂₃N₄O₂ (MW 363.48); mass spectroscopy (M+H) 364.2.

### Example 8-Z

### Synthesis of 3-(L-tert-Leucinyl)-amino-2,3-dihydro-1-methyl-5-phenyl-1H-1,4-benzodiazepin-2-one

### Step A - Synthesis of 3-[N'-(tert-Butylcarbamate)-L-tert-leucinyl]-amino-2,3-dihydro-1-methyl-5-phenyl-1H-1,4-benzodiazepin-2-one

(S)-3-amino-1,3-dihydro-1-methyl-5-phenyl-2H-1,4-benzodiazepin-2-one, (1S)-7,7-dimethyl-2-oxobicyclo[2.2.1]heptane-1-methanesulfonate (Example 8-B, Step A) was free based by partitioning between methylene chloride and 1M potassium carbonate. The free amine was then coupled with N-Boc-*tert*-leucine following General Procedure D to give the title compound.
C₂₇H₃₅N₄O₄ (MW 479.66); mass spectroscopy 479.

### Step B - Synthesis of 3-(L-tert-Leucinyl)-amino-2,3-dihydro-1-methyl-5-phenyl-1H-1,4-benzodiazepin-2-one

Following General Procedure 8-C and using 3-[N'-(*tert*-butylcarbamate)-L-*tert*-leucinyl]-amino-2,3-dihydro-1-methyl-5-phenyl-1H-1,4-benzodiazepine-2-one, the title compound was prepared as a white foam.
Anal. Calcd for C₂₂H₂₅N₄O₂•0.5H₂O: C, 68.19; H, 7.02; N, 14.40. Found: C, 68.24; H, 7.00; N, 14.00.

### Example 8-AA

### Synthesis of 3-(L-Alaninyl)-amino-2,3-dihydro-1,5-dimethyl-1H-1,4-benzodiazepine

2,3-Dihydro-1,5-dimethyl-1H-1,4-benzodiazepine was prepared following general Procedures 8-I (using methyl iodide), 8-D and 8-F. Coupling of this intermediate with Boc-L-alanine (Novo) using General Procedure D, followed by deprotection using General Procedure 5-B afforded the title compound which was used without further purification.

### Example 8-AB

### Synthesis of 3-(L-3-Thienylglycinyl)amino-2,4-dioxo-1,5-bis-(2,2-dimethylpropyl)-2,3,4,5-tetrahydro-1H-1,5-benzodiazepine

### Step A: - Synthesis of N-(t-Butoxycarbonyl)-L-3-thienylglycine

N-(*t*-Butoxycarbonyl)-L-3-thienylglycine was prepared from L-α-(3-thienyl)glycine (Sigma) by the procedure described in Bodansky, M. et al; The Practice of Peptide Synthesis; Springer Verlag; 1994, p. 17.

### Step B: - Synthesis of 3-[N'-(t-Butoxycarbonyl)-L-3-thienylglycinyl]-amino-2,4-dioxo-1,5-bis-(2,2-dimethylpropyl)-2,3,4,5-tetrahydro-1H-1,5-benzodiazepine

Following General Procedure D above using the product from Example 8-V, Step C and the product from Step A above, 3-[N'-(*t*-butoxycarbonyl)-L-3-thienylglycinyl]-amino-2,4-dioxo-1,5-bis-(2,2-dimethylpropyl)-2,3,4,5-tetrahydro-1H-1,5-benzodiazepine was prepared.

### Step C: - Synthesis of 3-(L-3-Thienylglycinyl)amino-2,4-dioxo-1,5-bis-(2,2-dimethylpropyl)-2,3,4,5-tetrahydro-1H-1,5-benzodiazepine

Following General Procedure 8-N above using the product from Step B, the title compound was prepared.

### Example 8-1

### Synthesis of 3-(3,5-Difluorophenylacetyl)amino-2,3-dihydro-1-methyl-5-phenyl-1H-1,4-benzodiazepin-2-one

Following General Procedure A above using 3,5-difluorophenylacetic acid (Oakwood) and 3-amino-2,3-dihydro-1-methyl-5-phenyl-1*H*-1,4-benzodinepin-2-one (Example 8-X), the title compound was prepared as a solid having a melting point of 236-239°C. The reaction was monitored by tic on silica gel (Rf = 0.7 in 10% methanol/dichloromethane) and purification was by silica gel chromatography using 10% methanol/dichloromethane as the eluant.
NMR data was as follows:
¹H-nmr (CDCl₃): δ = 7.4 (m, 9H), 6.90 (dd, J = 6.0, 2.2, 2H), 6.73 (dt, J = 6.6, 2.2, 2.2, 6.6, 1H), 5.50 (d, J = 7.7, 1H), 3.68 (s, 2H), 3.46 (s, 3H).
¹³C-nmr (CDCl₃): δ = 172.9, 165.2, 163.5, 138.3, 133.6, 127.7, 126.4, 125.4, 124.6, 123.9, 120.3, 117.2, 108.2, 107.9. 98.4, 62.8, 38.6, 30.9.
C₂₄H₁₉N₃O₂F (MW = 419); mass spectroscopy (MH⁺) 420.

### Example 8-2

### Synthesis of 3-(N'-(3,5-Difluorophenylacetyl)-L-alaninyl)amino-2,3-dihydro-1-ethyl-5-phenyl-1H-1,4-benzodiazepin-2-one

Following General Procedure A above using *N*-(3,5-difluorophenylacetyl)-L-alanine (Example B) and 3-amino-2,3-dihydro-1-ethyl-5-phenyl-1*H*-1,4-benzodiazepin-2-one (prepared as described in Example 8-X using ethyl iodide), the title compound was prepared as a solid having a melting point of 155-158°C. The reaction was monitored by tic on silica gel (Rf = 0.48 in 10% methanol/dichloromethane) and purification was by silica gel chromatography using 10% methanol/dichloromethane as the eluant, followed by recrystallization from diethyl ether.
NMR data was as follows:
¹H-nmr (CDCl₃): δ = 7/73 (d, J = 7.7, 1H), 7.4 (m. 9H), 6.86 (m, 2H), 6.68 (m, 1H), 6.58 (d, J = 7.2, 1H), 5.43 (dd, J = 2.7, 4.9, 2.7, 1H), 4.67 (m, 1H), 4.3 (m, 1H), 3.7 (m, 1H), 3,52 (s, 2H), 1.46 (dd, J = 6.6, 6.6, 3H), 1.10 (dt, J = 7.1, 1.1, 6.0, 3H).
¹³C-nmr (CDCl₃): δ = 167.8, 164.8, 163.4, 161.3, 136.7, 133.6, 127.6, 126.4, 125.2, 124.0, 118.1, 107.8, 98.3, 95.5, 62.9, 44.7, 38.6, 14.5, 8.7.
C₂₈H₂₆N₄O₃F₂ (MW = 504); mass spectroscopy (MH⁺) 505.

### Example 8-3

### Synthesis of 3-[N'-(3,5-Difluorophenylacetyl)-L-alaninyl]-amino-2,3-dihydro-5-phenyl-1H-1,4-benzodiazepin-2-one

Following General Procedure D above using N-(3,5-difluorophenylacetyl)-L-alanine (Example B) and 3-amino-1,3-dihydro-5-phenyl-2H-1,4-benzodiazepin-2-one (CAS: 103343-47-1. Sherrill, R. G.; Sugg, E. E. J. Org. Chem. 1995, 60, 730.), the title compound was prepared as a white solid. Purification was by trituration with 1:1 ether/hexanes.
C₂₆-H₂₁F₂N₄O₃ (MW = 475.51); mass spectroscopy (MH⁺) 476.
Anal. Calcd for C₂₆H₂₁F₂N₄O₃: C, 65.54; H, 4.65; N; 11.76. Found: C, 65.37; H, 4.67; N, 11.63.

### Example 8-4

### Synthesis of 3-[N'-(3,5-Difluorophenylacetyl)-L-alaninyl]-amino-2,3-dihydro-1-methyl-5-(1-piperidinyl)-1H-1,4-benzodiazepin-2-one

Following General Procedure D above using N-(3,5-difluorophenylacetyl)-L-alanine (Example B) and 3-amino-1,3-dihydro-1-methyl-5-(1-piperidinyl)-2H-1,4-benzodiazepin-2-one (Example 8-A), the title compound was prepared as a white solid having a melting point of 154-160°C.
C₂₆H₂₉F₂N₅O₃ (MW =497.60); mass spectroscopy 497.
Anal. Calcd for C₂₆H₂₉F₂N₅O₃: C, 62.75; H, 5.89; N, 14.08. Found: C, 62.52; H, 5.81; N, 13.62.

### Example 8-5

### Synthesis of 3-[N'-(3,5-Difluorophenylacetyl)-L-alaninyl]-amino-7-chloro-2,3-dihydro-1-methyl-5-phenyl-1H-1,4-benzodiazepin-2-one

Following General Procedure D above using 3,5-difluorophenylacetic acid (Oakwood Products, Inc.) and 3-(L-alaninyl)-amino-7-chloro-2,3-dihydro-1-methyl-5-phenyl-1H-1,4-benzodiazepin-2-one (Example 8-C), the title compound was prepared as a white solid having a melting point of 126.5-130°C.
C₂₇H₂₃ClF₂N₄O₃ (MW = 524.1); mass spectroscopy 523.7.
Anal. calcd for C₂₇H₂₃ClF₂N₄O₃: C, 61.78; H, 4.42; N, 10.67. Found: C, 61.92; H, 4.52; N, 10.46.

### Example 8-6

### Synthesis of 3-[N'-(3,5-Difluorophenylacetyl)-L-alaninyl]-amino-7-bromo-2,3-dihydro-1-methyl-5-(2-fluorophenyl)-1H-1,4-benzodiazepin-2-one

Following General Procedure D above using 3,5-difluorophenylacetic acid (Oakwood Products, Inc.) and 3-(L-alaninyl)-amino-7-bromo-2,3-dihydro-1-methyl-5-(2-fluorophenyl)-1H-1,4-benzodiazepin-2-one (Example 8-D), the title compound was prepared as a white solid.
C₂₇H₂₂BrF₃N₄O₃ (MW = 587-43); mass spectroscopy 587.
Anal calcd for C₂₇H₂₂BrF₃N₄O₃: C, 55.21; H, 3.78; N, 9.54. Found: C, 55.25; H, 4.00; N, 9.72.

### Example 8-7

### Synthesis of 3-[N'-(3,5-Difluorophenylacetyl)-N'-methyl-L-alaninyl]-amino-2,3-dihydro-1-methyl-5-phenyl-1H-1,4-benzodiazepin-2-one

Following General Procedure D above using 3,5-difluorophenylacetic acid (Oakwood Products, Inc.) and 3-(N'-methyl-L-alaninyl)-amino-2,3-dihydro-1-methyl-5-phenyl-1H-1,4-benzodiazepin-2-one (Example 8-E), the title compound was prepared as a white solid.
¹H NMR (300 MHz, CDCl₃): δ = 7.65 (1H, d, J=7.9 Hz), 7.59-7.34 (8H, m), 7.23 (1H, t, J=7.2 Hz), 6.84 (2H, d, J=6.0 Hz), 6.65 (1H, t, J=7.2 Hz), 5.46 (1H; d, J=7.9 Hz), 5.42 (1H, d, J=7.2 Hz), 3.78 (2H, s), 3.47 (3H, s), 3.02 (3H, s), 1.42 (3H, d, J=7.1 Hz).
C₂₈H₂₆F₂N₄O₃ (MW =505.2051); mass spectroscopy 505.2046.

### Example 8-8

### Synthesis of 3-[N'-(3,5-Difluorophenylacetyl)-L-alaninyl]-amino-7-chloro-2,3-dihydro-1-methyl-5-(2-chlorophenyl)-1H-1,4-benzodiazepin-2-one

Following General Procedure D above using 3,5-difluorophenylacetic acid (Oakwood Products, Inc.) and 3-(L-alaninyl)-amino-7-chloro-2,3-dihydro-1-methyl-5-(2-chlorophenyl)-1H-1,4-benzodiazepin-2-one (Example 8-F), the title compound was prepared as a white solid.
C₂₇H₂₂Cl₂F₂N₄O₃ (MW = 559.43); mass spectroscopy 559.2.
Anal. calcd for C₂₇H₂₂Cl₂F₂N₄O₃: C, 57.97; H, 3.96; N, 10.02. Found: C, 57.99; H, 3.98; N, 9.92.

### Example 8-9

### Synthesis of 3-[N'-(3,5-Difluorophenylacetyl)-L-alaninyl]-amino-5-cyclohexyl-2,3-dihydro-1-methyl-1H-1,4-benzodiazepin-2-one

Following General Procedure D above using 3,5-difluorophenylacetic acid (Oakwood Products, Inc.) and 3-(L-alaninyl)-amino-5-cylcohexyl-2,3-dihydro-1-methyl-1H-1,4-benzodiazepin-2-one (Example 8-G), the title compound was prepared as a white solid.
C₂₇H₃₀F₂N₄O₃ (MW = 497.2364); mass spectroscopy 497.2370.

### Example 8-10

### Synthesis of 3-[N'-(3,5-Difluorophenylacetyl)-L-alaninyl]-amino-2,3-dihydro-1-methyl-7-nitro-5-phenyl-1H-1,4-benzodiazepin-2-one

Following General Procedure D above using 3,5-difluorophenylacetic acid (Oakwood Products, Inc.) and 3-(L-alaninyl)-amino-2,3-dihydro-1-methyl-7-nitro-5-phenyl-1H-1,4-benzodiazepin-2-one (Example 8-H), the title compound was prepared as a yellow solid.
¹H NMR (300 MHz, CDCl₃): δ = 8.44 1H, dd, J=2.2, 9.0 Hz), 8.42 (1H, dd, J=2.3, 9.0 Hz), 8.23 (2H, d, J=2.6 Hz), 7.73 (2H, m), 7.56-7.40 (12H, m), 6.83 (4H, m), 6.69 (2H, m), 6.37 (2H, apt, J=7.8 Hz), 5.45 (1H, d, J=7.7 Hz), 5.44 (1H, d, J=7.7 Hz), 4.71 (2H, m), 3.56 (2H, s), 3.55 (2H, s), 3.52 (3H, s), 3.51 (3H, s), 1.47 (3H, d, J=7.0 Hz), 1.46 (3H, d, J=7.0 Hz).
C₂₇H₂₃F₂N₅O₅ (M+H = 536.1747); mass spectroscopy found 536.1749.

### Example 8-11

### Synthesis of 3-[N'-(3,5-Difluorophenylacetyl)-L-alaninyl]-amino-2,3-dihydro-1-methyl-5-(2-fluorophenyl)-1H-1,4-benzodiazepin-2-one

Following General Procedure D above using 3,5-difluorophenylacetic acid (Oakwood Products, Inc.) and 3-(L-alaninyl)-amino-2,3-dihydro-1-methyl-5-(2-fluorophenyl)-1H-1,4-benzodiazepin-2-one (Example 8-1), the title compound was prepared as a white solid having a melting point of 185-188°C.
C₂₇H₂₃F₃N₄O₃ (MW = 508.54); mass spectroscopy found (M+H) 509.3.
Anal. calcd for C₂₇H₂₃F₃N₄O₃: C, 63.78; H, 4.53; N, 11.02. Found: C, 63.99; H, 4.49; N, 10.84.

### Example 8-12

### Synthesis of 3-[N'-(3,5-Difluorophenyl-α-hydroxyacetyl)-L-valinyl]-amino-2,3-dihydro-1-methyl-5-phenyl-1H-1,4-benzodiazepin-2-one

Following General Procedure D above using S-(+)-3,5-difluoromandelic acid (Example L) and 3-(L-valinyl)-amino-2,3-dihydro-1-methyl-5-1H-1,4-benzodiazepin-2-one (Example 8-Y), the title compound was prepared as a white solid.
C₂₉H₂₈F₂N₄O₄ (MW = 534.61); mass spectroscopy found (M+H) 535.3.
Anal. calcd for C₂₉H₂₈F₂N₄O₄: C, 65.16; H, 5.28; N, 10.48. Found: C,
65.34; H, 5.43; N, 10.35.

### Example 8-13

### Synthesis of 3-[N'-(3,5-Difluorophenyl-α-hydroxyacetyl)-L-tert-leucinyl]-amino-2,3-dihydro-1-methyl-5-phenyl-1H-1,4-benzodiazepin-2-one

Following General Procedure D above using S-(+)-3,5-difluoromandelic acid (Example L) and 3-(tert-leucinyl)-amino-2,3-dihydro-1-methyl-5-1H-1,4-benzodiazepin-2-one (Example-8-Z), the title compound was prepared as a white solid.
C₃₀H₃₀F₂N₄O₄ (MW = 548. 64); mass spectroscopy found (M+H) 549.3.
Anal. calcd for C₃₀H₃₀F₂N₄O₄: C, 65.68; H, 5.51; N, 10.21. Found: C, 65.38; H, 5.44; N, 10.14.

### Example 8-14

### Synthesis of 3-[N'-(3,5-Difluorophenylacetyl)-L-alaninyl]-amino-2,3-dihydro-1-methyl-5-(3-fluorophenyl)-1H-1,4-benzodiazepin-2-one

Following General Procedure D above using 3,5-difluorophenylacetic acid (Oakwood Products, Inc.) and 3-(L-alaninyl)-amino-2,3-dihydro-1-methyl-5-(3-fluorophenyl)-1H-1,4-benzodiazepin-2-one (Example 8-J), the title compound was prepared as an off white solid.
C₂₇H₂₃F₃N₄O₃ (MW = 508.50); mass spectroscopy found (M+H) 509.3.
¹H NMR (300 MHz, CDCl₃): δ = 7.65-7.53 (4H, m), 7.42-7.24 (12H, m), 7.22-7.14 (2H, m), 6.87-6.81 (4H, m), 6.75-6.65 (2H, m), 6.29 (1H, d, J=6.6 Hz), 6.21 (1H, d, J=7.2 Hz), 5.45 (1H, d, J=7.8 Hz), 5.44 (1H, d, J=7.5 Hz), 4.67 (2H, m), 3.57 (2H, s), 3.55 (2H, s), 3.474 (3H, s), 3.468 (3H, s), 1.48 (3H, d, J=7.2 Hz), 1.47 (3H, d, J=6.8 Hz).

### Example 8-15

### Synthesis of 3-[N'-(3,5-Difluorophenylacetyl)-L-alaninyl]amino-2,3-dihydro-1-methyl-5-(4-fluorophenyl)-1H-1,4-benzodiazepin-2-one

Following General Procedure D above using 3,5-difluorophenylacetic acid (Oakwood Products, Inc.) and 3-(L-alaninyl)-amino-2,3-dihydro-1-methyl-5-(4-fluorophenyl)-1H-1,4-benzodiazepin-2-one (Example 8-K), the title compound was prepared as an off-white solid.
C₂₇H₂₃F₃N₄O₃ (MW = 508.50); mass spectroscopy found (M+H) 509.7.
Anal. calcd for C₂₇H₂₃F₃N₄O₃:C, 63.78; H, 4.56; N, 11.01. Found: C, 64.09; H, 4.81; N, 10.40.

### Example 8-16

### Synthesis of 3-[N'-(Cyclopentyl-α-hydroxyacetyl)-L-alaninyl]-amino-2,3-dihydro-1-methyl-5-phenyl-1H-1,4-benzodiazepin-2-one

Following General Procedure D above using (±)-α-hydroxy-cyclopentylacetic acid (Example P) and 3-(L-alaninyl)-amino-2,3-dihydro-1-methyl-5-1H-1,4-benzodiazepin-2-one (Example 8-B), the title compound was prepared as a white solid.
Isomer 1:
   C₂₆H₃₀N₄O₄ (MW = 462.60); mass spectroscopy found (M+H) 463.6.
   Anal. calcd for C₂₆H₃₀N₄O₄: C, 67.51; H, 6.54; N, 12.11. Found: C, 67.78; H, 6.65; N, 12.29.
Isomer 2:
   C₂₆H₃₀N₄O₄ (MW = 462.60); mass spectroscopy found (M+H) 463.4.
   Anal. calcd for C₂₆H₃₀N₄O₄: C, 67.51; H, 6.54; N, 12.11. Found: C, 67.74; H, 6.56; N,11.81.

### Example 8-17

### Synthesis of 3-[N'-(Cyclopentyl-α-hydroxyacetyl)-L-valinyl]-amino-2,3-dihydro-1-methyl-5-phenyl-1H-1,4-benzodiazepin-2-one

Following General Procedure D above using (±)-α-hydroxy-cyclopentylacetic acid (Example P) and 3-(L-valinyl)-amino-2,3-dihydro-1-methyl-5-1H-1,4-benzodiazepin-2-one (Example 8-B), the title compound was prepared as a white solid.
Isomer 1:
   C₂₈H₃₄N₄O₄ (MW = 490.66); mass spectroscopy found (M+H) 491.4.
Isomer 2:
   C₂₈H₃₄N₄O₄ -(MW = 490.66); mass -spectroscopy found (M+H) 491.4.

### Example 8-18

### Synthesis of 3-[N'-(3,5-Difluorophenylacetyl)-L-alaninyl]amino-2,3-dihydro-1,5-dimethyl-1H-1,4-benzodiazepin-2-one

Following General Procedure D above using 3,5-difluorophenylacetic acid (Oakwood Products, Inc.) and 3-(L-alaninyl)-amino-2,3-dihydro-1,5-dimethyl-1H-1,4-benzodiazepin-2-one (Example 8-AA), the title compound was prepared as a solid (mp. = 222-223°C). The product was purified by slurrying in ether.
MW = 429; mass spectroscopy found (M+H) 429.
Anal. calcd: C, 61.67; H, 5.18; N, 13.08. Found: C, 61.43; H, 5.17; N, 12.79.

### Example 8-19

### Synthesis of 3-[N'-(3,5-Difluorophenylacetyl)-L-alaninyl]amino-2,3-dihydro-1-isobutyl-5-phenyl)-1H-1,4-benzodiazepin-2-one

Following General Procedure D above using 3,5-difluorophenylacetic acid (Oakwood Products, Inc.) and 3-(L-alaninyl)-amino-2,3-dihydro-1-isobutyl-5-phenyl-1H-1,4-benzodiazepin-2-one (Example 8-L), the title compound was prepared as a solid (mp. = 210-211°C). The product was purified by tituration from ether/hexanes.
C₃₀H₃₀F₂N₄O₃ (MW = 532.23); mass spectroscopy found (M+H) 532.
Anal. calcd: C, 67.66; H, 5.68; N, 10.52. Found: C, 67.67; H, 5.55; N, 10.34.
Purification by C 2000 chromatography, eluting with hexanes/ethyl acetate (20:80) afforded the following isomers:
Isomer 1:
   Melting Point: 202-203°C.
   C₃₀H₃₀F₂N₄O₃ (MW = 532.23); mass spectroscopy found (M+H) 532.23.
Isomer 2:
   Melting Point: 211-212°C.
   C₃₀H₃₀F₂N₄O₃ (MW = 532.23); mass spectroscopy found (M+H) 532.

### Example 8-20

### Synthesis of 3-[N'-(3,5-Difluorophenyl-α-hydroxyacetyl)-L-alaninyl]amino-2,3-dihydro-1-methyl-5-phenyl-1H-1,4-benzodiazepin-2-one

Following General Procedure D above using 3,5-difluoromandelic acid (Fluorochem) and 3-(L-alaninyl)amino-2,3-dihydro-1-methyl-5-phenyl-1H-1,4-benzodiazepin-2-one (Example 8-B), the title compound was prepared as a solid. The product was purified by LC 2000 chromatography, eluting with hexanes/ethyl acetate (20:80).
Isomer 1:
   Melting Point: 240-241°C.
   C₂₇H₂₄F₂N₄O₄ (MW = 506.51); mass spectroscopy found (M+H) 506.
Anal. calcd for C₂₇H₂₄F₃N₄O₄: C, 64.03; H, 4.78; N, 11.06. Found: C, 64.31; H, 4.86; N, 11.04.
Isomer 2:
   Melting Point: 128°C.
   C₂₇H₂₄F₂N₄O₄ (MW = 506.51); mass spectroscopy found (M+H) 506.
   Anal. calcd for C₂₇H₂₄F₃N₄O₄: C, 64.03; H, 4.78; N, 11.06. Found: C, 63.92; H, 5.00; N, 10.88.

### Example 8-21

### Synthesis of 3-[N'-(3,5-Difluorophenyl-α-oxoacetyl)-L-alaninyl]amino-2,3-dihydro-1-methyl-5-phenyl-1H-1,4-benzodiazepin-2-one

Following General Procedure D above using 3,5-difluoro-α-oxoacetic acid (Example O) and 3-(L-alaninyl)amino-2,3-dihydro-1-methyl-5-phenyl-1H-1,4-benzodiazepin-2-one (Example 8-B), the title compound was prepared as a solid (mp. = 128-129°C). The product was purified by LC 2000 chromatography, eluting with hexanes/ethyl acetate (30:70).
C₂₇H₂₂F₂N₄O₄ (MW = 504); mass spectroscopy found (M+H) 503.9.
Optical Rotation: [α] = -113.64 @ 589; -333.33 @ 365 (c 1, MeOH).
Anal. calcd for C₂₇H₂₂F₃N₄O₄: C, 64.28; H, 4.40; N, 11.11. Found: C, 64.51; H, 4.54; N, 11.04.

### Example 8-22

### Synthesis of 3-[N'-(2-Methylthioacetyl)-L-alaninyl]amino-2,3-dihydro-1-methyl-5-phenyl-1H-1,4-benzodiazepin-2-one

Following General Procedure D above using 2-methylthioacetic acid (Aldrich) and 3-(L-alaninyl)-amino-2,3-dihydro-1-methyl-5-phenyl-1H-1,4-benzodiazepin-2-one (Example 8-B), the title compound was prepared as a solid (mp. = 205-206°C). The product was purified by slurrying in hexanes/ether (1:1).
C₂₂H₂₄N₄O₃S (MW = 424); mass spectroscopy found (M+H) 424.
Anal. calcd for C₂₂H₂₄N₄O₃S: C, 62.25; H, 5.70; N, 13.20. Found: C, 62.11; H, 5.89; N, 13.02.

### Example 8-23

### Synthesis of 3-[N'-(3,5-Difluorophenylacettyl)-L-valinyl]amino-2,3-dihydro-1-methyl-5-phenyl-1H-1,4-benzodiazepin-2-one

Following General Procedure D above using 3,5-difluorophenylacetic acid (Oakwood Products, Inc.) and 3-(L-valinyl)amino-2,3-dihydro-1-methyl-5-phenyl-1H-1,4-benzodiazepin-2-one (Example 8-Y), the title compound was prepared as a solid (mp. = 228-229°C). The product was purified by slurrying in ether/hexanes (80:20).
C₂₉-H₂₈F₂N₄O₃ (MW = 518); mass spectroscopy found (M+H) 518.
Optical Rotation: [α] = -117.96 @ 589; -341.55 @ 365 (c 1, MeOH).
Anal. calcd for C₂₉H₂₈F₂N₄O₃: C, 67.17; H, 5.44; N, 10.8. Found: C, 67.45; H, 5.49; N, 10.61.

### Example 8-24

### Synthesis of 3-[N'-(3,5-Difluorophenylacetyl)-L-tert-leucinyl]amino-2,3-dihydro-1-methyl-5-phenyl-1H-1,4-benzodiazepin-2-one

Following General Procedure D above using 3,5-difluorophenylacetic acid (Oakwood Products, Inc.) and 3-(L-tert-leucinyl)amino-2,3-dihydro-1-methyl-5-phenyl-1H-1,4.-benzodiazepin-2-one (Example 8-Z), the title compound was prepared as a solid (mp. = 221-222°C). The product was purified by slurrying in ether.
C₃₀H₃₀F₂N₄O₃ (MW = 532); mass spectroscopy found (M+H) 532.
Anal. calcd for C₃₀H₃₀F₂N₄O_{3:} C, 67.66; H, 5.68; N, 10.52. Found: C, 67.93; H, 5.95; N, 10.25.

### Example 8-25

### Synthesis of 3-[N'-(3,5-Difluorophenylacetyl)-L-alaninyl]amino-2,3-dihydro-1-isopropyl-5-phenyl-1H-1,4-benzodiazepin-2-one

Following General Procedure D above using 3,5-difluorophenylacetic acid (Oakwood Products, Inc.) and 3-(L-alaninyl)amino-2,3-dihydro-1-isopropyl-5-phenyl-1H-1,4-benzodiazepin-2-one (Example 8-L), the title compound was prepared as a solid (mp. = 208-209°C). The product was purified by slurrying in ether/hexanes (1:1).
MW = 518; mass spectroscopy found (M+H) 518.
Anal. calcd: C, 67.17; H, 5.44; N, 10.80. Found: C, 67.39; H, 5.62; N,10.84.

### Example 8-26

### Synthesis of 3-[N'-(3,5-Difluorophenylacetyl)-L-alaninyl]amino-2,3-dihydro-1-cyclopropylmethyl-5-phenyl-1H-1,4-benzodiazepin-2-one

Following General Procedure D above using 3,5-difluorophenylacetic acid (Oakwood Products, Inc.) and 3-(L-alaninyl)amino-2,3-dihydro-1-cyclopropylmethyl-5-phenyl-1H-1,4-benzodiazepin-2-one (Example 8-L), the title-compound was prepared as a solid (mp. = 203-205°C). The product was purified by slurrying in ether/hexanes.
C₃₀H₂₈F₂N₄O₃ (MW = 530.58); mass spectroscopy found (M+H) 530.
Anal. calcd for C₃₀H₂₈F₂N₄O₃: C, 67.91; H, 5.32; N, 10.56. Found: C, 68.14; H, 5.54; N, 10.62.

### Example 8-27

### Synthesis of 3-[N'-(3,5-Difluorophenyl-α-fluoroacetyl)-L-alaninyl]amino-2,3-dihydro-1-methyl-5-phenyl-1H-1,4-benzodiazepin-2-one

Following General Procedure D above using 3,5 difluorophenyl-α-fluoroacetic acid (Example S) and 3-(L-alaninyl)amino-2,3-dihydro-1-methyl-5-phenyl-1H-1,4-benzodiazepin-2-one (Example 8-Y), the title compound was prepared as a solid. The product was purified by LC 2000 chromatography, eluting with hexanes/ethyl acetate (35:65).
Isomer 1:
   Melting Point: 119-120°C.
   C₂₇H₂₃F₄N₄O₃ (MW = 508); mass spectroscopy found (M+H) 508.
   Optical Rotation: [α] = -115.62 @ 589; -292.09 @ 365 (c 1, MeOH).
   Anal. calcd for C₂₇H₂₃F₃N₄O₃: C, 62.66; H, 4.67; N, 10.82. Found: C, 62.55; H, 4.74; N, 10.51.
Isomer 2:
   Melting Point: 198-199°C.
   C₂₇H₂₃F₃N₄O₃ (MW = 508); mass spectroscopy found (M+H) 508.
   Optical Rotation: [α] = -99.65 @ 589; -279.72 @ 365 (c 1, MeOH).
   Anal. calcd for C₂₇H₂₃F₃N₄O₃: C, 62.66; H, 4.67; N, 10.82. Found: C, 62.40; H, 4.62; N, 10.84.

### Examples 8-28 to 8-139

By following the procedures set forth above, the following additional compounds were prepared:
- 8-28: 3-[N'-(3,5-difluorophenylacetyl)-L-alaninyl]amino-2,3-dihydro-1-*n*-propyl-5-phenyl-1H-1,4-benzodiazepin-2-one
- 8-29: 3-[N'-(3-methylbutyryl)-L-phenylglycinyl]amino-2,3-dihydro-1-methyl-5-phenyl-1H-1,4-benzodiazepin-2-one
- 8-30: 3-[N'-(3,5-difluorophenylacetyl)-L-phenylglycinyl]amino-2,3-dihydro-1-methyl-5-phenyl-1H-1,4-benzodiazepin-2-one
- 8-31: 3-[N'-(2-phenylthioacetyl)-L-alaninyl]amino-2,3-dihydro-1-methyl-5-phenyl-1H-1,4-benzodiazepin-2-one
- 8-32: 3-[N'-(3-methylbutyryl)-L-alaninyl]amino-2,3-dihydro-1-methyl-5-phenyl-1H-1,4-benzodiazepin-2-one
- 8-33: 3-[N'-(2-phenylthioacetyl)-L-phenylglycinyl]amino-2,3-dihydro-1-methyl-5-phenyl-1H-1,4-benzodiazepin-2-one
- 8-34: 3-[N'-(3-(4-methoxyphenyl)propionyl)-L-alaninyl]amino-2,3-dihydro-1-methyl-5-phenyl-1H-1,4-benzodiazepin-2-one
- 8-35: 3-[N'-(3-bromophenylacetyl)-L-alaninyl]amino-2,3-dihydro-1-methyl-5-phenyl-1H-1,4-benzodiazepin-2-one
- 8-36: 3-[N'-(4-cyclohexylbutyryl)-L-alaninyl]amino-2,3-dihydro-1-methyl-5-phenyl-1H-1,4-benzodiazepin-2-one
- 8-37: 3-[N'-(4-methoxyphenylacetyl)-L-alaninyl]amino-2,3-dihydro-1-methyl-5-(2-pyridyl)-1H-1,4-benzodiazepin-2-one
- 8-38: 3-[N'-(3-methyl-2-hydroxylbutyryl)-L-alaninyl]amino-2,3-dihydro-1-methyl-5-phenyl-1H-1,4-benzodiazepin-2-one
- 8-39: 3-[N'-(3-methyl-2-hydroxylbutyryl)-L-alaninyl]amino-2,3-dihydro-1-methyl-5-phenyl-1H-1,4-benzodiazepin-2-one
- 8-40: 3-[N'-(3,3-dimethylbutyryl)-L-alaninyl]amino-2,3-dihydro-1-methyl-5-phenyl-1H-1,4-benzodiazepin-2-one
- 8-41: 3-[N'-(thien-2-yl-acetyl)-L-alaninyl]amino-2,3-dihydro-1-methyl-5-(2-pyridyl)-1-H-1,4-benzodiazepin-2-one
- 8-42: 3-[N'-(3,5-difluorophenylacetyl)-L-alaninyl]amino-2,3-dihydro-1-methyl-5-(2-pyridyl)-1H-1,4-benzodiazepin-2-one
- 8-43: 3-[N'-(3-bromophenylacetyl)-L-alaninyl]amino-2,3-dihydro-1-methyl-5-(2-pyridyl)-1H-1,4-benzodiazepin-2-one
- 8-44: 3-[N'-(2-phenylthioacetyl)-L-alaninyl]amino-2,3-dihydro-1-methyl-5-(2-pyridyl)-1H-1,4-benzodiazepin-2-one
- 8-45: 3-[N'-(4-ethoxyphenylacetyl)-L-alaninyl]amino-2,3-dihydro-1-methyl-5-(2-pyridyl)-1H-1,4-benzodiazepin-2-one
- 8-46: 3-[N'-(4-trifluoromethylphenylacetyl)-L-alaninyl]amino-2,3-dihydro-1-methyl-5-(2-pyridyl)-1H-1,4-benzodiazepin-2-one
- 8-47: 3-[N'-(3,5-di(trifluoromethyl)phenylacetyl)-L-alaninyl]amino-2,3-dihydro-1-methyl-5-(2-pyridyl)-1H-1,4-benzodiazepin-2-one
- 8-48: 3-[N'-(2-methylthioacetyl)-L-alaninyl]amino-2,3-dihydro-1-methyl-5-(2-pyridyl)-1H-1,4-benzodiazepin-2-one
- 8-49: 3-[N'-(2-cyclohexylacetyl)-L-alaninyl]amino-2,3-dihydro-1-methyl-5-(2-pyridyl)-1H-1,4-benzodiazepin-2-one
- 8-50: 3-[N'-(2,3,4,5,6-pentafluorophenyloxyacetyl)-L-alaninyl]amino-2,3-dihydro-1-methyl-5-(2-pyridyl)-1H-1,4-benzodiazepin-2-one
- 8-51: 3-[N'-(thionaphth-3-ylacetyl)-L-alaninyl]amino-2,3-dihydro-1-methyl-5-(2-pyridyl)-1H-1,4-benzodiazepin-2-one
- 8-52: 3-[N'-(2,4,6-trimethylphenylacetyl)-L-alaninyl]amino-2,3-dihydro-1-methyl-5-(2-pyridyl)-1H-1,4-benzodiazepin-2-one
- 8-53: 3-[N'-((4-phenyl)phenylacetyl)-L-alaninyl]amino-2,3-dihydro-1-methyl-5-(2-pyridyl)-1H-1,4-benzodiazepin-2-one
- 8-54: 3-[N'-(3,4-difluorophenylacetyl)-L-alaninyl]amino-2,3-dihydro-1-methyl-5-(2-pyridyl)-1H-1,4-benzodiazepin-2-one
- 8-55: 3-[N'-(4-(thien-2-yl)butyryl)-L-alaninyl]amino-2,3-dihydro-1-methyl-5-(2-pyridyl)-1H-1,4-benzodiazepin-2-one
- 8-56: 3-[N'-(5-methylhexanoyl)-L-alaninyl]amino-2,3-dihydro-1-methyl-5-(2-pyridyl)-1H-1,4-benzodiazepin-2-one
- 8-57: 3-[N'-(2-methoxycarbonylacetyl)-L-alaninyl]amino-2,3-dihydro-1-methyl-5-(2-pyridyl)-1H-1,4-benzodiazepin-2-one
- 8-60: 3-[N'-(2,6-difluorophenyl)-α-hydroxyacetyl)-L-alaninyl]amino-2,3-dihydro-1-methyl-5-(2-pyridyl)-1H-1,4-benzodiazepin-2-one
- 8-61: 3-[N'-(4-fluorophenyl)-α-hydroxyacetyl)-L-alaninyl]amino-2,3-dihydro-1-methyl-5-(2-pyridyl)-1H-1,4-benzodiazepin-2-one
- 8-62: 3-[N'-(2,5-difluorophenyl)-α-hydroxyacetyl)-L-alaninyl]amino-2,3-dihydro-1-methyl-5-(2-pyridyl)-1H-1,4-benzodiazepin-2-one
- 8-63: 3-[N'-(2,4,6-trifluorophenyl)acetyl)-L-alaninyl]amino-2,3-dihydro-1-methyl-5-(2-pyridyl)-1H-1,4-benzodiazepin-2-one
- 8-64: 3-[N'-(2-trifluoromethyl-4-fluorophenyl)acetyl)-L-alaninyl]amino-2,3-dihydro-1-methyl-1-5-(2-pyridyl)-1H-1,4-benzodiazepin-2-one
- 8-65: 3-[N'-(4,4,4-trifluorobutyryl)-L-alaninyl]amino-2,3-dihydro-1-methyl-5-(2-pyridyl)-1H-1,4-benzodiazepin-2-one
- 8-66: 3-[N'-(4-iso-propylphenylacetyl)-L-alaninyl]amino-2,3-dihydro-1-methyl-5-(2-pyridyl)-1H-1,4-benzodiazepin-2-one
- 8-67: 3-[N'-(3-phenyl-2-hydroxypropionyl)-L-alaninyl]amino-2,3-dihydro-1-methyl-5-(2-pyridyl)-1H-1,4-benzodiazepin-2-one
- 8-68: 3-[N'-(phenyl-α-hydroxyacetyl)-L-alaninyl]amino-2,3-dihydro-1-methyl-5-(2-pyridyl)-1H-1,4-benzodiazepin-2-one
- 8-69: 3-[N'-(4-chlorophenyl-α-hydroxyacetyl)-L-alaninyl]amino-2,3-dihydro-1-methyl-5-(2-pyridyl)-1H-1,4-benzodiazepin-2-one
- 8-70: 3-[N'-(3-methylbutyryl)-L-alaninyl]amino-2,3-dihydro-1-methyl-5-(2-pyridyl)-1H-1,4-benzodiazepin-2-one
- 8-71: 3-[N'-(2,3,5-trifluorophenylacetyl)-L-alaninyl]amino-2,3-dihydro-1-methyl-5-(2-pyridyl)-1H-1,4-benzodiazepin-2-one
- 8-72: 3-[N'-(3-methylthiopropionyl)-L-alaninyl]amino-2,3-dihydro-1-methyl-5-(2-pyridyl)-1H-1,4-benzodiazepin-2-one
- 8-73: 3-[N'-(3-methyl-2-hydroxybutyryl)-L-alaninyl]amino-2,3-dihydro-1-methyl-5-(2-pyridyl)-1H-1,4-benzodiazepin-2-one
- 8-74: -3-[N'-(3-nitrophenylacetyl)-L-alaninyl]amino-2,3-dihydro-1-methyl-5-(2-pyridyl)-1H-1,4-benzodiazepin-2-one
- 8-75: 3-[N'-(4-methoxyphenylacetyl)-L-alaninyl]amino-2,3-dihydro-1-(*tert*-butylcarbonylmethyl)-5-(2-pyridyl)-1H-1,4-benzodiazepin-2-one
- 8-76: 3-[N'-(2-thienylacetyl)-L-alaninyl]amino-2,3-dihydro-1-(*tert-*butylcarbonylmethyl)-5-(2-pyridyl)-1H-1,4-benzodiazepin-2-one
- 8-77: 3-[N'-(3,5-difluorophenylacetyl)-L-alaninyl]amino-2,3-dihydro-1-(*tert*-butylcarbonylmethyl)-5-(2-pyridyl)-1H-1,4-benzodiazepin-2-one
- 8-78: 3-[N'-(3-bromophenylacetyl)-L-alaninyl]amino-2,3-dihydro-1-(*tert*-butylcarbonylmethyl)-5-(2-pyridyl)-1H-1,4-benzodiazepin-2-one
- 8-79: 3-[N'-(2-phenylthioacetyl)-L-alaninyl]amino-2,3-dihydro-1-(*tert*-butylcarbonylmethyl)-5-(2-pyridyl)-1H-1,4-benzodiazepin-2-one
- 8-80: 3-[N'-(4-ethoxyphenylacetyl)-L-alaninyl]amino-2,3-dihydro-1-(*tert*-butylcarbonylmethyl)-5-(2-pyridyl)-1H-1,4-benzodiazepin-2-one
- 8-81: 3-[N'-(4-trifluoromethylphenylacetyl)-L-alaninyl]amino-2,3-dihydro-1-(*tert*-butylcarbonylmethyl)-5-(2-pyridyl)-1H-1,4-benzodiazepin-2-one
- 8-82: 3-[N'-(3,5-di-(trifluoromethyl)phenylacetyl)-L-alaninyl]amino-2,3-dihydro-1-(*tert*-butylcarbonylmethyl)-5-(2-pyridyl)-1H-1,4-benzodiazepin-2-one
- 8-83: 3-[N'-(2-methylthioacetyl)-L-alaninyl]amino-2,3-dihydro-1-(*tert*-butylcarbonylmethyl)-5-(2-pyridyl)-1H-1,4-benzodiazepin-2-one
- 8-84: 3-[N'-(2-cyclomethylacetyl)-L-alaninyl]amino-2,3-dihydro-1-(*tert*-butylcarbonylmethyl)-5-(2-pyridyl)-1H-1,4-benzodiazepin-2-one
- 8-85: 3-[N'-(2,3,4,5,6-pentafluorophenyloxyacetyl)-L-alaninyl]amino-2,3-dihydro-1-(*tert*-butylcarbonylmethyl)-5-(2-pyridyl)-1H-1,4-benzodiazepin-2-one
- 8-86: 3-[N'-(thionaphth-3-ylacetyl)-L-alaninyl]amino-2,3-dihydro-1-(*tert*-butylcarbonylmethyl)-5-(2-pyridyl)-1H-1,4-benzodiazepin-2-one
- 8-87: 3-[N'-(2,4,6-trimethylphenylacetyl)-L-alaninyl]amino-2,3-dihydro-1-(*tert*-butylcarbonylmethyl)-5-(2-pyridyl)-1H-1,4-benzodiazepin-2-one
- 8-88: 3-[N'-((4-phenyl)phenylacetyl)-L-alaninyl]amino-2,3-dihydro-1-(*tert*-butylcarbonylmethyl)-5-(2-pyridyl)-1H-1,4-benzodiazepin-2-one
- 8-89: 3-[N'-(3,4-difluorophenylacetyl)-L-alaninyl]amino-2,3-dihydro-1-(*tert*-butylcarbonylmethyl)-5-(2-pyridyl)-1H-1,4-benzodiazepin-2-one
- 8-90: 3-[N'-(4-(2-thienyl)butyryl)-L-alaninyl]amino-2,3-dihydro-1-(*tert*-butylcarbonylmethyl)-5-(2-pyridyl)-1H-1,4-benzodiazepin-2-one
- 8-91: 3-[N'-(5-methylhexanoyl)-L-alaninyl]amino-2,3-dihydro-1-(*tert*-butylcarbonylmethyl)-5-(2-pyridyl)-1H-1,4-benzodiazepin-2-one
- 8-95: 3-[N'-(2,6-difluorophenyl-α-hydroxyacetyl)-L-alaninyl]amino-2,3-dihydro-1-(*tert*-butylcarbonylmethyl)-5-(2-pyridyl)-1H-1,4-benzodiazepin-2-one
- 8-96: 3-[N'-(4-fluorophenyl-α-hydroxyacetyl)-L-alaninyl]amino-2,3-dihydro-1-(*tert*-butylcarbonylmethyl)-5-(2-pyridyl)-1H-1,4-benzodiazepin-2-one
- 8-97: 3-[N'-(2,5-difluorophenyl-α-hydroxyacetyl)-L-alaninyl]amino-2,3-dihydro-1-(*tert*-butylcarbonylmethyl)-5-(2-pyridyl)-1H-1,4-benzodiazepin-2-one
- 8-98: 3-[N'-(2,4,6-trifluorophenylacetyl)-L-alaninyl]amino-2,3-dihydro-1-(*tert*-butylcarbonylmethyl)-5-(2-pyridyl)-1H-1,4-benzodiazepin-2-one
- 8-99: 3-[N'-(2-trifluoromethyl-4-fluorophenylacetyl)-L-alaninyl]amino-2,3-dihydro-1-(tert-butylcarbonylmethyl)-5-(2-pyridyl)-1H-1,4-benzodiazepin-2-one
- 8-100: 3-[N'-(4,4,4-trifluorobutyryl)-L-alaninyl]amino-2,3-dihydro-1-(*tert*-butylcarbonylmethyl)-5-(2-pyridyl)-1H-1,4-benzodiazepin-2-one
- 8-101: 3-[N'-(4-*iso*-propylphenylaceryl)-L-alaninyl]amino-2,3-dihydro-1-(*tert*-butylcarbonylmethyl)-5-(2-pyridyl)-1H-1,4-benzodiazepin-2-one
- 8-102: 3-[N'-(3-phenyl-2-hydroxypropionyl)-L-alaninyl]amino-2,3-dihydro-1-(*tert*-butylcarbonylmethyl)-5-(2-pyridyl)-1H-1,4-benzodiazepin-2-one
- 8-103: 3-[N'-(phenyl-α-hydroxyacetyl)-L-alaninyl]amino-2,3-dihydro-1-(*tert*-butylcarbonylmethyl)-5-(2-pyridyl)-1H-1,4-benzodiazepin-2-one
- 8-104: 3-[N'-(4-chlorophenylacetyl)-L-alaninyl]amino-2,3-dihydro-1-(*tert*-butylcarbonylmethyl)-5-(2-pyridyl)-1H-1,4-benzodiazepin-2-one
- 8-105: 3-[N'-(3-methylbutyryl)-L-alaninyl]amino-2,3-dihydro-1-(*tert-*butylcarbonylmethyl)-5-(2-pyridyl)-1H-1,4-benzodiazepin-2-one
- 8-106: 3-[N'-(2,3,5-trifluorophenylacetyl)-L-alaninyl]amino-2,3-dihydro-1-(*tert*-butylcarbonylmethyl)-5-(2-pyridyl)-1H-1,4-benzodiazepin-2-one
- 8-107: 3-[N'-(3-methylthiopropionyl)-L-alaninyl]amino-2,3-dihydro-1-(*tert*-butylcarbonylmethyl)-5-(2-pyridyl)-1H-1,4-benzodiazepin-2-one
- 8-108: 3-[N'-(3-methyl-2-hydroxybutyryl)-L-alaninyl]amino-2,3-dihydro-1-(*tert*-butylcarbonylmethyl)-5-(2-pyridyl)-1H-1,4-benzodiazepin-2-one
- 8-109: 3-[N'-(3-nitrophenylacetyl)-L-alaninyl]amino-2,3-dihydro-1 -(*tert*-butylcarbonylmethyl)-5-(2-pyridyl)-1H-1,4-benzodiazepin-2-one
- 8-110: 3-[N'-(4-methoxyphenylacetyl)-L-alaninyl]amino-2,3-dihydro-1-(2-(N,N-diethylamino)ethyl)-5-(2-pyridyl)-1H-1,4-benzodiazepin-2-one
- 8-111: 3-[N'-(2-thienylacetyl)-L-alaninyl]amino-2,3-dihydro-1-(2-(N,N-diethylamino)ethyl)-5-(2-pyridyl)-1H-1,4-benzodiazepin-2-one
- 8-112: 3-[N'-(3,5-difluorophenylacetyl)-L-alaninyl]amino-2,3-dihydro-1-(2-(N,N-diethylamino)ethyl)-5-(2-pyridyl)-1H-1,4-benzodiazepin-2-one
- 8-113: 3-[N'-(3-bromophenylacetyl)-L-alaninyl]amino-2,3-dihydro-1-(2-(N,N-diethylamino)ethyl)-5-(2-pyridyl)-1H-1,4-benzodiazepin-2-one
- 8-114: 3-[N'-(2-phenylthioacetyl)-L-alaninyl]amino-2,3-dihydro-1-(2-(N,N-diethylamino)ethyl)-5-(2-pyridyl)-1H-1,4-benzodiazepin-2-one
- 8-117: 3-[N'-(2-cyclohexylacetyl)-L-alaninyl]amino-2,3-dihydro-1-(2-(N,N-diethylamino)ethyl)-5-(2-pyridyl)-1H-1,4-benzodiazepin-2-one
- 8-118: 3-[N'-(2,3,4,5,6-pentafluorophenyloxyacetyl)-L-alaninyl]amino-2,3-dihydro-1-(2-(N,N-diethylamino)ethyl)-5-(2-pyridyl)-1H-1,4-benzodiazepin-2-one
- 8-119: 3-[N'-(2-thionaphth-3-ylacetyl)-L-alaninyl]amino-2,3-dihydro-1-(2-(N,N-diethylamino)ethyl)-5-(2-pyridyl)-1H-1,4-benzodiazepin-2-one
- 8-120: 3-[N'-(2-phenyl-2-oxoacetyl)-L-alaninyl]amino-2,3-dihydro-1-(2-(N,N-diethylamino)ethyl)-5-(2-pyridyl)-1H-1,4-benzodiazepin-2-one
- 8-123: 3-[N'-((3,4-difluorophenyl)acetyl)-L-alaninyl]amino-2,3-dihydro-1-(2-(N,N-diethylamino)ethyl)-5-(2-pyridyl)-1H-1,4-benzodiazepin-2-one
- 8-124: 3-[N'-((4-(thien-2-yl)butyryl)-L-alaninyl]amino-2,3-dihydro-1-(2-(N,N-diethylamino)ethyl)-5-(2-pyridyl)-1H-1,4-benzodiazepin-2-one
- 8-125: 3-[N'-(5-methylhexanoyl)-L-alaninyl]amino-2,3-dihydro-1-(2-(N,N-diethylamino)ethyl)-5-(2-pyridyl)-1H-1,4-benzodiazepin-2-one
- 8-130: 3-[N'-(4-fluorophenyl-α-hydroxyacetyl)-L-alaninyl]amino-2,3-dihydro-1-(2-(N,N-diethylamino)ethyl)-5-(2-pyridyl)-1H-1,4-benzodiazepin-2-one
- 8-131: 3-[N'-(2,5-difluorophenyl-α-hydroxyacetyl)-L-alaninyl]amino-2,3-dihydro-1-(2-(N,N-diethylamino)ethyl)-5-(2-pyridyl)-1H-1,4-benzodiazepin-2-one
- 8-135: 3-[N'-(4,4,4-trifluorobutyryl)-L-alaninyl]amino-2,3-dihydro-1-(2-(N,N-diethylamino)ethyl)-5-(2-pyridyl)-1H-1,4-benzodiazepin-2-one
- 8-136: 3-[N'-(4-*iso*-propylphenylacetyl)-L-alaninyl]amino-2,3-dihydro-1-(2-(N,N-diethylamino)ethyl)-5-(2-pyridyl)-1H-1,4-benzodiazepin-2-one
- 8-137: 3-[N'-(3-phenyl-2-hydroxypropionyl)-L-alaninyl]amino-2,3-dihydro-1-(2-(N,N-diethylamino)ethyl)-5-(2-pyridyl)-1H-1,4-benzodiazepin-2-one
- 8-138: 3-[N'-(phenyl-α-hydroxyacetyl)-L-alaninyl]amino-2,3-dihydro-1-(2-(N,N-diethylamino)ethyl)-5-(2-pyridyl)-1H-1,4-benzodiazepin-2-one
- 8-139: 3-[N'-(4-chlorophenyl-α-hydroxyacetyl)-L-alaninyl]amino-2,3-dihydro-1-(2-(N,N-diethylamino)ethyl)-5-(2-pyridyl)-1H-1,4-benzodiazepin-2-one

### Example 8-140

### Synthesis of 3-[N'-(3,5-Difluorophenyl-α-hydroxyacetyl)-L-3-thienylglycinyl]amino-2,4-dioxo-1,5-bis(2,2-dimethylpropyl)-2,3,4,5-tetrahydro-1H-1,5-benzodiazepine

Following General Procedure D above using 3,5-difluoromandelic acid (Fluorochem) and 3-(L-3-thienylglycinyl]amino-2,4-dioxo-1,5-bis(2,2-dimethylpropyl)-2,3,4,5-tetrahydro-1H-1,5-benzodiazepine (Example 8-AB), the title compound was prepared as a solid. The product was purified by LC 2000 chromatography, eluting with hexanes/ethyl acetate (1:1).
Isomer 1:
   Melting Point: 191-192°C.
   Optical Rotation: [α] = +21.47 @ 589; +52.17 @ 365 (c 1, MeOH).
   C₃₃H₃₈F₂N₄O₅S (MW = 640); mass spectroscopy found (M+H) 639.1; 640.1.
   Anal. calcd for. C₃₃H₃₈F₂N₄O₅S_{:} C, 61.68; H, 5.89; N, 8.74. Found: C, 61.87; .H, 6.08; N, 8.84.
Isomer 2:
   Melting Point: 230-231°C.
   Optical Rotation: [α] = +59.26 @ 589; +200.0 @ 365 (c 1, MeOH).
   C₃₃H₃₈F₂N₄O₅S (MW = 640); mass spectroscopy found (M+H) 639.4; 640.4.
   Anal. calcd for C₃₃H₃₈F₂N₄O₅S: C, 61.68; H, 5.89; N, 8.74. Found: C, 62.01; H, 6.07; N, 8.52.

### Example 8-141

### Synthesis of 3-[N'-(3,5-Difluorophenyl-α-hydroxyacetyl)-L-alaninyl]amino-2,4-dioxo-1-phenyl-5-methyl-2,3,4,5-tetrahydro-1H-1,5-benzodiazepine

Following General Procedure D above using 3,5-difluoromandelic acid (Fluorochem) and 3-(L-alaninyl)amino-2,4-dioxo-1-phenyl-5-methyl-2,3,4,5-tetrahydro-1H-1,5-benzodiazepine (Example 8-N), the title compound was prepared was a solid. The product was purified by LC 2000 chromatography, eluting with hexanes/ethyl acetate (30:70).
Isomer 1:
   Melting Point: 212-213°C.
   Optical Rotation: [α] - + 101.34 @ 589; +491.4@ 365 (c 1. MeOH).
   C₂₂H₂₄F₂N₄O₉ (MW = 522.17); mass Spectroscopy found (M+H) 523.3; 521.3.
Isomer 2:
   Melting Point: 282-283°C.
   C₂₇H₂₄F₂N₄O₅ (MW = 522.1793); exact mass spectroscopy found (M+) 323.1800.
Isomer 3:
   Melting Point: 147-148°C.
   C₂₇H₂₄F₂N₄O₅ (MW = 322.1793); exact mass spectroscopy found (M+) 323.1793.
Isomer 4:
   Melting Point: 255-256°C.
   C₂₇H₂₄F₂N₄O₅ (MW = 522.17); mass spectroscopy found (M+) 523.2.
Anal. calcd for C₂₇H₂₄F₂N₄O₅: C, 62.07; H, 4.63; N, 10.72. Found: C, 62.18; H, 4.84; N, 10.74.

### Example 8-146

### Synthesis of 3-[N'-(3,5-Difluorophenylacetyl)-L-alaninyl]-amino-2,4-dioxo-1,5-bis-(1-methylethyl)-2,3,4,5-tetrahydro-1H-1,5-benzodiazepine

Following General Procedure I above using N-(3,5-difluorophenylacetyl)-L-alanine (Example B) and 3-amino-2,4-dioxo-1,5-bis-(1-methylethyl)-2,3,4,5-tetrahydro-1H-1,5-benzodiazepine (Example 8-P), the title compound was prepared as a white solid (melting point = 232-233°C). Purification was by flash chromatography eluting with EtOAc/hexanes (4:1 gradient to 6:1). R_{f} = 0.31 (4:1 EtOAc/hexanes).
C₂₀H₃₀F₂N₄O₄ (MW 500.55); mass spectroscopy (MH+) 500.2
Anal. Calcd. for C₂₆H₃₀F₂N₄O₄: C, 62.39; H, 6.04; N, 11.19. Found: C, 62.62; H, 6.00; N, 11.21.

### Example 8-147

### Synthesis of 3-[N'-(3,5-Difluorophenylacetyl)-(R)-2-thienylglycinyl]amino-2,4-dioxo-1,5-bis-(1-methylethyl)-2,3,4,5-tetrahydro-1H-1,5-benzodiazepine

Following General Procedure I above using 3,5-difluorophenylacetic acid (Lancaster) and 3-(R-2-thienylglycinyl)-amino-2,4-dioxo-1,5-bis-(1-methylethyl)-2,3,4,5-tetrahydro-1H-1,5-benzodiazepine hydrochloride (Example 8-Q), the title compound was prepared as an amorphous white solid. Purification was by flash chromatography eluting with CH₂Cl₂/EtOAc (5:1 gradient to 4:1). R_{f} = 0.34 (4:1 CH₂Cl₂/EtOAc).
C₂₉H₃₀F₂N₄O₄S (MW 568.65); mass spectroscopy (MH+) 568.
Anal. Calcd. for C₂₉H₃₀F₂N₄O₄S: C, 61.25; H, 5.32; N, 9.85. Found: C, 61.00; H, 5.42; N, 9.68.

### Example 8-148

### Synthesis of 3-[N'-(Cyclopropylacetyl)-R-2-thienylglycinyl]amino-2,4-dioxo-1,5-bis-(1-methylethyl)-2,3,4,5-tetrahydro-1H-1,5-benzodiazepine

Following General Procedure I above using cyclopropylacetic acid (Lancaster) and the product from Example 8-Q, the title compound was prepared as an amorphous white solid. Purification was by flash chromatography eluting with CH₂Cl₂/EtOAc (4:1 gradient to 5:2). R_{f} = 0.26 (4:1 CH₂Cl₂/EtOAc).
C₂₆H₃₂N₄O₄S (MW 496.63); mass spectroscopy (MH+) 496.5
Anal. Calcd. for C₂H₃₂N₄O₄S: C, 62.88; H, 6.49; N, 11.28. Found: C, 62.65; H, 6.57; N, 11.55.

### Example 8-149

### Synthesis of 3-[N'-(Cyclopentylacetyl)-R-2-thienylglycinyl]amino-2,4-dioxo-1,5-bis-(1-methylethyl)-2,3,4,5-tetrahydro-1H-1,5-benzodiazepine

Following General Procedure I above using cyclopentylacetic acid (Aldrich) and the product from Example 8-Q, the title compound was prepared as an amorphous white solid. Purification was by flash chromatography eluting with CH₂Cl₂/EtOAc (5:1 gradient to 4:1). R_{f} = 0.26 (4:1 CH₂Cl₂/EtOAc).
C₂₈H₃₆N₄O₄S (MW 524.69); mass spectroscopy (MH+) 524.5
Anal. Calcd. for C₂₉H₃₆N₄O₄S: C, 64.10; H, 6.92; N, 10.68. Found: C, 64.07; H, 6.91; N, 10.67.

### Example 8-150

### Synthesis of 3-[N'-(3,5-Difluorophenylacetyl)-L-alaninyl]amino-2,4-dioxo-1,5-bis-methyl-2,3,4,5-tetrahydro-1H-1,5-benzodiazepine

Following General Procedure I above using 3,5-difluorophenylacetic acid (Lancaster) and 3-(L-alaninyl)-amino-2,4-dioxo-1,5-bis-methyl-2,3,4,5-tetrahydro-1H-1,5-benzodiazepine hydrochloride (Example 8-R), the title compound was prepared as a white solid (melting point = 206-207°C). Purification was by flash chromatography eluting with straight EtOAc gradient to EtOAc/Acetone (95:5). R_{f} = 0.32 (EtOAc).
C₂₂H₂₂F₂N₄O₄ (MW 444.42); mass spectroscopy (MH+) 444.
Anal. Calcd. for C₂₂H₂₂F₂N₄O₄ : C, 59.46; H, 4.99; N, 12.61. Found: C, 59.54; H, 5.09; N, 12.56.

### Example 8-151

### Synthesis of 3-[N'-(3,5-Difluorophenyl-α-hydroxyacetyl)-L-alaninyl]amino-2,4-dioxo-1,5-bis-methyl-2,3,4,5-tetrahydro-1H-1,5-benzodiazepine

Following General Procedure I above using 3,5-difluoromandelic acid (Lancaster) and the product from Example 8-R, the title compound was prepared as an amorphous white solid. Purification was by L.C. 2000 eluting with straight EtOAc then flash chromatography eluting with CH₂Cl_{2/}Acetone (4:1 gradient to 3:1). R_{f} = 0.39 and 0.34 (EtOAc).
C₂₂H₂₂F₂N₄O₅ (MW 460.44); mass spectroscopy (MH+) 461.0.
Anal. Calcd. for C₂₂H₂₂F₂N₄O₅: C, 57.39; H, 4.82; N, 12.17. Found: C, 57.16; H, 4.88; N, 11.97.

### Example 8-152

### Synthesis of 3-[N'-(3,5-Difluorophenylacetyl)-L-alaninyl]amino-2,4-dioxo-1,5-bis-(2-methylpropyl)-2,3,4,5-tetrahydro-1H-1,5-benzodiazepine

Following General Procedure I above using 3,5-difluorophenylacetic acid (Lancaster) and 3-(L-alaninyl)amino-2,4-dioxo-1,5-bis-(2-methylpropyl)-2,3,4,5-tetrahydro-1H-1,5-benzodiazepine hydrochloride (Example 8-S), the title compound was prepared as a white solid (melting point = 197-198°C). Purification was by flash chromatography eluting with CH₂Cl₂/EtOAc (2:1 gradient to 3:4). R_{f} = 0.23 (CH₂Cl₂/EtOAc, 1:1).
C₂₈H₃₄F₂N₄O₄ (MW 528.60); mass spectroscopy (MH +) 528.
Anal. Calcd. for C₂₈H₃₄F₂N₄O₄: C, 63.62; H, 6.48; N, 10.60. Found: C, 63.75; H, 6.63; N, 10.67.

### Example 8-153

### Synthesis of 3-[N'-(Cyclopentylacetyl)-L-alaninyl]amino-2,4-dioxo-1,5-bis-(2-methylpropyl)-2,3,4,5-tetrahydro-1H-1,5-benzodiazepine

Following General Procedure I above using cyclopentylacetic acid (Aldrich) and 3-(L-alaninyl)amino-2,4-dioxo-1,5-bis-(2-methylpropyl)-2,3,4,5-tetrahydro-1H-1,5-benzodiazepine hydrochloride (Example 8-S), the title compound was prepared as an amorphous white solid. Purification was by flash chromatography eluting with CH₂Cl₂/EtOAc (1:1). R_{f} = 0.31 (CH₂Cl₂/EtOAc, 1:1).
C₂₇H₄₀N₄O₄ (MW 484.64); mass spectroscopy (MH+) 484.
Anal. Calcd. for C₂₇H₄₀N₄O₄: C, 66.92; H, 8.32; N, 11.56. Found: C, 66.86; H, 8.64; N, 11.41.

### Example 8-154

### Synthesis of 3-[N'-(Cyclopropylacetyl)-L-alaninyl]-amino-2,4-dioxo-1,5-bis-(2-methylpropyl)-2,3,4,5-tetrahydro-1H-1,5-benzodiazepine

Following General Procedure I above using cyclopropylacetic acid (Lancaster) and 3-(L-alaninyl)amino-2,4-dioxo-1,5-bis-(2-methylpropyl)-2,3,4,5-tetrahydro-1H-1,5-benzodiazepine hydrochloride (Example 8-S), the title compound was prepared as a white solid (melting point = 190-191°C). Purification was by flash chromatography eluting with CH₂Cl₂/EtOAc (1:1 gradient to 3:4) and a second flash chromatography eluting with EtOAc/Toluene (7:3). R_{f} = 0.28 (EtOAc/Toluene, 7:3).
C₂₅H₃₆N₄O₄ (MW 456.59); mass spectroscopy (MH+) 456.1.
Anal. Calcd. for C₂₅H₃₆N₄O₄: C, 65.77; H, 7.95; N, 12.27. Found: C, 66.01; H, 8.03; N, 12.35.

### Example 8-155

### Synthesis of 3-[N'-(3,5-Difluorophenylacetyl)-S-phenylglycinyl]-amino-2,4-dioxo-1,5-bis-(2-methylpropyl)-2,3,4,5-tetrahydro-1H-1,5-benzodiazepine

Following General Procedure I above using 3,5-difluorophenylacetic acid (Lancaster) and 3-(S-phenylglycinyl)-amino-2,4-dioxo-1,5-bis-(2-methylpropyl)-2,3,4,5-tetrahydro-1H-1,5-benzodiazepine hydrochloride (Example 8-T), the title compound was prepared as a white solid (melting point = 186-187°C). Purification was by flash chromatography eluting with CH₂Cl₂/EtOAc (7:1 gradient to 4:1). R_{f} = 0.39 (CH₂Cl₂/EtOAc, 7:1).
C₃₃H₃₆F₂N₄O₄ (MW 590.68); mass spectroscopy (MH+) 590.0.
Anal. Calcd. for C₃₃H₃₆F₂N₄O₄: C, 67.10; H, 6.14; N, 9.49. Found: C, 67.36; H, 6.38; N, 9.56.

### Example 8-156

### Synthesis of 3-[N'-(3,5-Difluorophenylacetyl)-L-alaninyl]-amidno-2,4-dioxo-1,5-bis- (cyclopropytmethyl)-2,3,4,5-tetrahydro-1H-1,5-benzodiazepine

Following General Procedure I above using 3,5-difluorophenylacetic acid (Lancaster) and 3-(L-alaninyl)-amino-2,4-dioxo-1,5-bis-(cyclopropylmethyl)-2,3,4,5-tetrahydro-1H-1,5-benzodiazepine hydrochloride (Example 8-U), the title compound was prepared as a white solid (melting point = 211-212°C). Purification was by flash chromatography eluting with CH₂Cl₂/EtOAc (1:1 gradient to 2:3). R_{f} = 0.44 (CH₂Cl₂/EtOAc; 1:1).
C₂₈H₃₀F₂N₄O₄ (MW 524.57); mass spectroscopy (MH+) 524.1.
Anal. Calcd. for C₂₈H₃₀F₂N₄O₄: C, 64.11; H, 5.76; N, 10.68. Found: C, 64.07; H, 5.79; N, 10.49.

### Example 8-157

### Synthesis of 3-[N'-(Cyclopentylacetyl)-L-alaninyl]-amino-2,4-dioxo-1,5-bis-(cyclopropylmethyl)-2,3,4,5-tetrahydro-1H-1,5-benzodiazepine

Following General Procedure I above using cyclopentylacetic acid (Aldrich) and 3-(L-alaninyl)-amino-2,4-dioxo-1,5-bis-(cyclopropylmethyl)-2,3,4,5-tetrahydro-1H-1,5-benzodiazepine hydrochloride (Example 8-U), the title compound was prepared as a white foam. Purification was by flash chromatography eluting with CH₂Cl₂/EtOAc (1:1 gradient to 2:3). R_{f} = 0.50 (CH₂Cl₂/EtOAc, 1:1).
C₂₇H₃₆N₄O₄ (MW 480.61); mass spectroscopy (MH+) 481.2 and (MH-) 479.2.
Anal. Calcd. for C₂₇H₃₆N₄O: C, 67.48; H, 7.55; N, 11.66. Found: C, 67.33; H, 7.57; N, 11.37.

### Example 8-158

### Synthesis of 3-[N'-(Cyclopentyl-α-hydroxyacetyl)-L-alaninyl]-amino-2,4-dioxo-1,5-bis-(cyclopropylmethyl)-2,3,4,5-tetrahydro-1H-1,5-benzodiazepine

Following General Procedure I above using cyclopentyl-α-hydroxyacetic acid (Example P) and 3-(L-alaninyl)-amino-2,4-dioxo-1,5-bis-(cyclopropylmethyl)-2,3,4,5-tetrahydro-1H-1,5-benzodiazepine hydrochloride (Example 8-U), the title compound was prepared as a white foam. Purification was by L.C. 2000 eluting with CH₂Cl₂/EtOAc (1:1 gradient to 1:2) then flash chromatography eluting with 2:1 EtOAc/CH₂Cl₂. R_{f} = 0.47 and 0.37 (CH₂Cl₂/EtOAc, 1:2).
C₂₇H₃₆N₄O₅ (MW 496.61); mass spectroscopy (MH+) 497.2 and (MH-) 495.2
Anal. Calcd. for Cᵣ₂₇H₃₆N₄O₅ : C, 65.30; H, 7.31; N, 11.28. Found: C, 65.01; H, 7.35; N, 11.28.

### Example 8-159

### Synthesis of 3-[N'-(3,5-Difluorophenylacetyl)-L-alaninyl]-amino-2,4-dioxo-1,5-bis-(2,2-dimethylpropyl)-2,3,4,5-tetrahydro-1H-1,5-benzodiazepine

Following General Procedure I above using 3,5-difluorophenylacetic acid (Lancaster) and 3-(L-alaninyl)-amino-2,4-dioxo-1,5-bis-(2,2-dimethylpropyl)-2,3,4,5-tetrahydro-1H-1,5-benzodiazepine hydrochloride (Example 8-V), the title compound was prepared as a white solid (melting point = 194-195°C). Purification was by flash chromatography eluting with CH₂Cl₂/EtOAc (2:1 gradient to 3:2). R_{f} = 0.46 (CH₂Cl₂/EtOAc, 2: 1).
C₃₀H₃₈F₂N₄O₄ (MW 556.66); mass spectroscopy (MH+) 557.0 (MH-) 555.4.
Anal. Calcd. for C₃₀H₃₈F₂N₄O4 : C, 64.73; H, 6.88; N, 10.06. Found: C, 64.45; H, 6.82; N, 10.08.

### Example 8-160

### Synthesis of 3-[N'-(3,5-Difluorophenyl-α-hydroxyacetyl)-L-alaninyl]-amino-2,4-dioxo-1,5-bis-(2,2-dimethylpropyl)-2,3,4,5-tetrahydro-1H-1,5-benzodiazepine

Following General Procedure I above using 3,5-difluoromandelic acid (Lancaster) and 3-(L-alaninyl)-amino-2,4-dioxo-1,5-bis-(2,2-dimethylpropyl)-2,3,4,5-tetrahydro-1H-1,5-benzodiazepine hydrochloride (Example 8-V), the title compound was prepared as a white solid (melting point = 116-126°C). Purification was by flash chromatography eluting with CH₂Cl₂/EtOAc (1:1 gradient to 2:3). R_{f} = 0.54 and 0.40 (CH₂Cl₂/EtOAc, 1:1).
C₃₀H₃₈F₂N₄O₅ (MW 572.66); mass spectroscopy (MH+) 573.4 (MH-) 571.6.
Anal. Calcd. for C₃₀H₃₈F₂N₄O₅: C, 62.92; H, 6.69; N, 9.78. Found: C, 62.86; H, 6.54; N, 9.65.

### Example 8-161

### Synthesis of 3-[N'-(Cyclopentylacetyl)-L-alaninyl]amino-2,4-dioxo-1,5-bis-(2,2-dimethylpropyl)-2,3,4,5-tetrahydro-1H-1,5-benzodiazepine

Following General Procedure I above using cyclopentylacetic acid (Aldrich) and 3-(L-alaninyl)-amino-2,4-dioxo-1,5-bis-(2,2-dimethylpropyl)-2,3,4,5-tetrahydro-1H-1,5-benzodiazepine hydrochloride (Example 8-V), the title compound was prepared as a white amorphous solid. Purification was by flash chromatography eluting with CH₂Cl₂/EtOAc (2:1 gradient to 3:2). R_{f} = 0.29 (CH₂Cl₂/EtOAc, 2:1).
C₂₉H₄₄N₄O₄ (MW 512.70); mass spectroscopy (MH+) 513.6 (MH-) 511.6.
Anal. Calcd. for C₂₉H₄₄N₄O₄ : C, 67.94; H, 8.65; N, 10.93. Found: C, 68.18; H, 8.60; N, 10.68.

### Example 8-162

### Synthesis of 3-[N'-(Cyclopentyl-α-hydroxyacetyl)-L-alaninyl]amino-2,4-dioxo-1,5-bis-(2,2-dimethylpropyl)-2,3,4,5-tetrahydro-1H-1,5-benzodiazepine

Following General Procedure I above using cyclopentyl-α-hydroxyacetic acid (Example P) and 3-(L-alaninyl)-amino-2,4-dioxo-1,5-bis-(2,2-dimethylpropyl)-2,3,4,5-tetrahydro-1H-1,5-benzodiazepine hydrochloride (Example 8-V), the title compound was prepared as a white solid (melting point = 119-129°C). Purification was by flash chromatography eluting with CH₂Cl₂/EtOAc (1:1 gradient to 2:3). R_{f} = 0.42 and 0.28 (CH₂Cl₂/EtOAc, 1:1).
C₂₉H₄₄N₄O₅ (MW 528.70); mass spectroscopy (MH+) 529.2 (MH-) 527.4.
Anal. Calcd. for C₂₉H₄₄N₄O₅: C, 65.88; H, 8.39; N, 10.60. Found: C, 65.56; H, 8.03; N, 10.35.

### Example 8-163

### Synthesis of 3-[N'-(3,5-Difluorophenylacetyl)-L-alaninyl]-amino-2,4-dioxo-1,5-bis-phenyl-2,3,4,5-tetrahydro-1H-1,5-benzodiazepine

Following General Procedure I above using 3,5-difluorophenylacetic acid (Lancaster) and 3-(L-alaninyl)-amino-2,4-dioxo-1,5-bis-phenyl-2,3,4,5-tetrahydro-1H-1,5-benzodiazepine hydrochloride (Example 8-W), the title compound was prepared as a white solid (melting point = 139-141 °C). Purification was by flash chromatography eluting with CH₂Cl₂/EtOAc (1:1). R_{f} = 0.46 (CH₂Cl₂/EtOAc, 1:1).
C₃₂H₂₆F₂N₄O₄ (MW 568.59); mass spectroscopy (MH+) 569.2 (MH-) 567.4.
Anal. Calcd. for C₃₂H₂₆F₂N₄O₄: C, 67.60; H, 4.61; N, 9.85. Found: C, 67.39; H, 4.66; N, 9.60.

### Example 8-164

### Synthesis of 3-[N'-(Cyclopentylacetyl)-L-alaninyl]amino-2,4-dioxo-1,5-bis-phenyl-2,3,4,5-tetrahydro-1H-1,5-benzodiazepine

Following General Procedure I above using cyclopentylacetic acid (Aldrich) and 3-(L-alaninyl)-amino-2,4-dioxo-1,5-bis-phenyl-2,3,4,5-tetrahydro-1H-1,5-benzodiazepine hydrochloride (Example 8-W), the title compound was prepared as an amorphous white solid. Purification was by flash chromatography eluting with CH₂Cl₂/EtOAc (1:1). R_{f} = 0.44 (CH₂Cl₂/EtOAc, 1:1).
C₃₁H₃₂N₄O₄ (MW 524.63); mass spectroscopy (MH+) 525.2 (MH-) 523.2.
Anal. Calcd. for C₃₁H₃₂N₄O₄ : C; 70.97; H, 6.15; N, 10.68. Found: C, 70.67; H, 5.98; N, 10.43.

### Example 8-165

### Synthesis of 3-[N'-(Cyclopentyl-α-hydroxyacetyl)-L-alaninyl]-amino-2,4-dioxo-1,5-bis-phenyl-2,3,4,5-tetrahydro-1H-1,5-benzodiazepine

Following General Procedure I above using cyclopentyl-α-hydroxyacetic acid (Example P) and 3-(L-alaninyl)-amino-2,4-dioxo-1,5-bis-phenyl-2,3,4,5-tetrahydro-1H-1,5-benzodiazepine hydrochloride (Example 8-W), the title compound was prepared as a white solid (melting point = 139-149°C). Purification was by flash chromatography eluting with CH₂Cl₂/EtOAc (1:2). R_{f} = 0.50 and 0.39 (CH₂Cl₂/EtOAc, 1:2).
C₃₁H₃₂N₄O₅ (MW 540.63); mass spectroscopy (MH+) 541.2 (MH-) 539.6.
Anal. Calcd. for C₃₁H₃₂N₄O₅: C, 68.87; H, 5.97; N, 10.36. Found: C, 68.87; H, 5.88; N, 10.15.

### Example 8-166

### Synthesis of 3-(N'-(3,5-Difluorophenylacetyl)-L-alaninyl)amino-2,3-dihydro-1-methyl-5-phenyl-1H-1,4-benzodiazepin-2-one

Following General Procedure A above using *N*-(3,5-difluorophenylacetyl)-L-alanine (Example B) and 3-amino-2,3-dihydro-1-methyl-5-phenyl-1*H*-1,4-benzodiazepin-2-one (prepared as described in Bock M. G.; DiPardo, R. M.; Evans, B. E.; Rittle, K. E.; Veber, D. F.; Freidinger, R. M.; Hirshfield, J.; Springer, J. P. J. Org. Chem. 1987, 52, 3232), the title compound was prepared as a solid having a melting point of 152-160°C. The reaction was monitored by tlc on silica gel (Rf = 0.15 in 50% ethyl acetate/hexanes) and purification was by silica gel chromatography.
NMR data was as follows:
¹H-nmr (CDCl₃: δ = 7.70 (t. J=7.2. 7.2, 1H): 7.4 (m, 9H); 6.83 (d, J=5.5, 2H); 6.7 (m, 1H): 6.50 (d, 1-7.1, 1H): 5.44 (dd, 2.8, 4.9, 2.8, 1H): 4.7 (m, 1H); 3.53 (s, 2H): 3.45 (s, 3H); 1.46 (dd, J=4.4, 2.2, 4.9, 3H).
¹³C-nmr (CDCl₃): δ = 172.9, 167.8, 138.3, 133.4, 127.7, 126.5, 126.3, 125.4, 123.9, 120.3, 117.2, 108.1, 107.8, 98.4, 63.0, 44.7, 40.1, 38.4, 30.9, 14.5, 14.1.
C₂₇H₂₄F₂N₄O₃ (MW = 490): mass spectroscopy (MH⁺) 491.

The title compound was resolved using a Daicel chiral column (2 x 25 cm, ID x L) (normal phase polysaccharide type: 10 micro particle size). Using a gradient of 40% isopropanol/hexanes (4 mL/min flow rate for 35 minutes), followed by 20% isopropanol/hexanes (3 mL/min), isomer 1 and isomer 2 had retention times of 27.5 and 36.4 minutes, respectively.

Using the following combinatorial procedures, the following additional intermediates and examples were prepared.

### GENERAL PROCEDURE C-A

To a 4 mL vial containing 60-100 mg (0.06-0.1 mmol) of polymer bound 1-(1-pyrrolidinyl propyl)-3-ethyl carbodiimide was added 2 mL of a 0.015 mM stock soludon of starting material 1 in DMF/chloroform and 1 mL of a 0.0148 mM stock solution of starting material 2 in chloroform. The resulting slurry were shaken for 48 h and filtered. The filtered resin was washed with chloroform and the filtrate was concentrated to dryness under vacuum. All product structures and purities were confirmed by HPLC using UV detection and IEX MS. Samples were submitted for testing with out any further purification.

### GENERAL PROCEDURE C-B

To a 4 mL vial was added 840 uL of 0.05 mM stock solution of starting material 1 in DMF/chloroform, 100 uL of a 0.21 mM stock solution of starting material 2 in chloroform and 100 uL of a 0.63 mM stock solution of 1-(3-dimethylaminopropyl)-3-ethyl carbodiimide in chloroform. After allowing to stand undisturbed for 48 h, the reaction mixture was concentrated and the residue redissolved in 2 mL of a 10% methanol/methylene chloride solution. This solution was then filtered through a pre-washed (methanol) 500 mg SCX column (Varian Sample Preparation; Harbor City California) using an additional 8 mL of the same solvent. The filtrate was concentrated under reduced pressure and the residue was dissolved in 20% methanol/methylene chloride and passed through a plug of silica gel (100 mg, Varian Sample Preparation). The collected filtrate was concentrated under reduced pressure and the crude products were submitted for testing without further purification. Product structure and purity were confirmed by HPLC and IEX MS.

### GENERAL PROCEDURE C-C

To a 4 mL vial was added 540 uL of 0.05 mM stock solution of starting material 1 in DMF/chloroform, 100 uL of a 0.44 mM stock solution of starting material 2 in chloroform and 100 uL of a 0.38 mM stock solution of 1-(3-dimethylaminopropyl)-3-ethyl carbodiimide in chloroform. After standing undisturbed for 48 h, the reaction mixture was concentrated and the residue redissolved in 2 mL of a 10% methanol/methylene chloride solution. This solution was then filtered through a pre-washed (methanol) 500 mg SCX column using an additional 8 mL of the same solvent. The filtrate was concentrated under reduced pressure and the residue was dissolved in 20% methanol/methylene chloride and passed through a plug of silica gel (100 mg, Varian Sample Preparation). The collected filtrate was concentrated under reduced pressure and the crude products were submitted for testing without further purification. Product structure and purity were confirmed by HPLC and IEX MS.

### GENERAL PROCEDURE C-D

To a 4 mL vial was added 540 uL of 0.05 mM stock solution of starting material 1 in DMF / chloroform, 100 uL of a 0.44 mM stock solution of starting material 2 in chloroform, 100 uL of a 0.38 mM stock solution of 1-(3-dimethylaminopropyl)-3-ethyl carbodiimide in chloroform and 100 uL of a 0.38 mM stock solution of PP-HOBt in DMF. After standing undisturbed for 48 h, the reaction mixture was concentrated and the residue redissolved in 2 mL of a 10% methanol/methylene chloride solution. This solution was then filtered through a pre-washed (methanol) 500 mg SCX column using an additional 8 mL of the same solvent. The filtrate was concentrated under reduced pressure and the residue was dissolved in 20% methanol/methylene chloride and passed through a plug of silica gel (100 mg, Varian Sample Preparation). The collected filtrate was concentrated under reduced pressure and the crude products were submitted for testing without further purification. Product structure and purity were confirmed by HPLC and IEX MS.

### GENERAL PROCEDURE C-E

To a 4 mL vial was added 870 uL of 0.05 mM stock solution of starting material 1 in DMF/chloroform, 1000 uL of a 0.05 mM stock solution of starting material 2 in chloroform, 1000 uL of a 0.05 mM stock solution of 1-(3-dimethylaminopropyl)-3-ethyl carbodiimide in chloroform and 100 uL of a 0.48 mM stock solution of HOBt in DMF. After standing undisturbed for 48 h, the reaction mixture was concentrated and the residue redissolved in 2 mL of a 10% methanol/methylene chloride solution. This solution was then filtered through a pre-washed (methanol) 500 mg SCX column using an additional 8 mL of the same solvent. The filtrate was concentrated under a stream of nitrogen to approximately 1/3 its original volume and then passed over a plug (200 mg) of AG 1-8x anion exchange resin (BioRad; Hercules, California; Columns were pre-washed with 1N NaOH, water and methanol) using an additional 6 mL of 10% methanol/methylene chloride solution. The resulting filtrate was concentrated under vacuum and the crude products were submitted for testing without further purification. Product structure and purity were confirmed by HPLC and IEX MS.

### GENERAL PROCEDURE C-F

Starting material 1 (9.1 uL, 0.109 mmol) was added neat to a mixture of starting material 2 (22.5 mg, 0.054 mmol) and piperidinylmethyl polystyrene (45 mg, 3.6 mmol/g (Fluka)) in 1 mL of methylene. The mixture was shaken for 80 h at ambient temperatures and then treated with methylisocyanate polystyrene (100 mg, 1.0 mmol/g (Novabiochem)) for 24 h with shaking. The reaction mixture was filtered and the resin washed with methylene chloride. The crude product was loaded onto a 500 mg SCX ion exchange column (Varian Sample Preparation), washed 3X with 3 mL of methanol and then eluted with 4 mL of 2 M ammonia methanol. Further purification of the final product was achieved using semi-preparative HPLC (0-100% acetonitrile (0.08 % TFA)/water (0.1 % TFA); 25 mL/min.; 20X50 ODS-A column) to give 17 mg of the final product as an off white foam.
NMR data was as follows:
¹H NMR (300 MHz, CDCl₃) δ 1.45-1.65 (m, 3 H), 1.70-2.00 (m, 4 H), 2.55-2.80 (m, 4 H), 3.25 (s, 2 H), 3.50 (s, 3H), 4.65-4.80 (m, 1 H), 5.45-5.55 (m, 1H), 7.20-7.80 (m, 11 H).

### GENERAL PROCEDURE C-G

To a 4 mL vial containing 0.03 mmol of starting material 2 was added 100 uL of 0.25 mM stock solution of starting material 1 in chloroform, 100 uL of a 0.3 mM stock solution of 1-(3-dimethylaminopropyl)-3-ethyl carbodiimide in chloroform and 100 uL of a 0.3 mM stock solution of HOBt in DMF. After standing undisturbed for 48 h, the reaction mixture was concentrated and the residue redissolved in 2 mL of a 10% methanol/methylene chloride solution. This solution was then filtered through a pre-washed (methanol) 500 mg SCX column using an additional 8 mL of the same solvent. The filtrate was concentrated under a stream of nitrogen to approximately 1/3 its original volume and then passed over a plug (200 mg) of AG 1-8x anion exchange resin (BioRad; Hercules, California; Columns were pre-washed with 1N NaOH, water and methanol) using an additional 6 mL of 10% methanol/methylene chloride solution. The resulting filtrate was concentrated under vacuum and the crude products were submitted for testing without further purification. Product structure and purity were confirmed by HPLC and IEX MS.

### GENERAL PROCEDURE C-H

The intermediates shown in Table C-1 (i.e., Starting material 2) were synthesized in parallel in using the following procedure:
Step A: To a solution of 3-(tert-butoxycarbonyl)amino-2,3-dihydro-5-phenyl-1H-1,4-benzodiazepin-2-one (CA No. 125:33692: 100 mg, 0.28 mmol) in 1 mL of anhydrous DMF was added 600 uL of a solution of 0.5 M potassium bis(trimethylsilyl)amide (0.30 mmol) in toluene. Neat alkyl halide (0.56 mmol; as indicated in Table C-1) was added immediately in one portion and the reaction mixture was left undisturbed overnight. When an alkyl chloride was used, 1 equivalent of sodium iodide was added to the reaction mixture. After concentration under reduced pressure, the crude reaction residue was partitioned between methylene chloride (2 mL) and aqueous saturated bicarbonate (2 mL) and then passed through a 5 g Extralut QE cartridge (EM Science; Gibbstown, NJ) using 10 mL of methylene chloride. The resulting filtrate was concentrated under reduced pressure and the crude product was further purified using automated semi-preparative HPLC (YMC 20 X 50 mm Silica column; gradient elution; 0-5 % (5.5 min.), 5-20 % (3.5 min.), 20-100 % (2 min.), 100% (4 min.) ethyl acetate/methylene chloride, flow rate of 25 mL/min.). Product provided the expected M+1 peak by IEX MS and were carried on without further purification and characterization.
Step B: The product obtained from Step A was dissolved in 5 mL of a 15 % TFA/methylene chloride solution and allowed to stand undisturbed for 16 h. After concentration under reduced pressure, the TFA salt was dissolved in methanol and loaded directly onto a 1 g SCX column. The column was washed 3 X with 2 mL portions of methanol and the product was eluted from the column using 6 mL of 2.0 M solution of ammonia/methanol. After concentration under reduced pressure, the product were characterized by IEX MS and carried on without further purification.
Step C: To the crude product obtained from Step B (1.05 equiv.) was added sequentially a 0.3 mM stock solution of HOBt·H₂O (1.05 equiv.) in DMF, a 0.3 mM stock solution of N-t-BOC-L-alanine (1.0 equiv.) in THF and 0.3 mM stock solution of 1-(3-dimethylaminopropyl)-3-ethyl carbodiimide (1.05 equiv.) in THF. After standing undisturbed for 24 h, the reaction mixture was concentrated and the residue redissolved in 2 mL of a 10% methanol/methylene chloride solution. This solution was then filtered through a pre-washed (methanol) 1 g SCX (Varian Sample Preparation) column using an additional 8 mL of the same solvent. For Example C-V a 1 g Si column (Varian Sample Preparation) was used). The filtrate was concentrated under a stream of nitrogen to approximately 1/3 its original volume and then passed over a plug (500 mg) of AG 1-8x anion exchange resin (BioRad; Hercules, California; Columns were pre-washed with 1N NaOH, water and methanol) using an additional 10 mL of methanol. The resulting filtrate was concentrated under reduced pressure and the crude product was carried on without further purification after characterization by IEX MS.
Step D: The crude product obtained from Step C was dissolved in 5 mL of a 15 % TFA/methylene chloride solution and allowed to stand undisturbed for 16 h. After concentration under reduced pressure, the TFA salt was dissolved in methanol and loaded directly onto a 1 g SCX column. The column was washed 3 X with 2 mL portions of methanol and the product were eluted from the column using 6 mL of 2.0 M solution of ammonia/methanol. After concentration under reduced pressure, the product were characterized by IEX MS and carried on without further purification. The intermediates prepared by this method are shown in Table C-A.

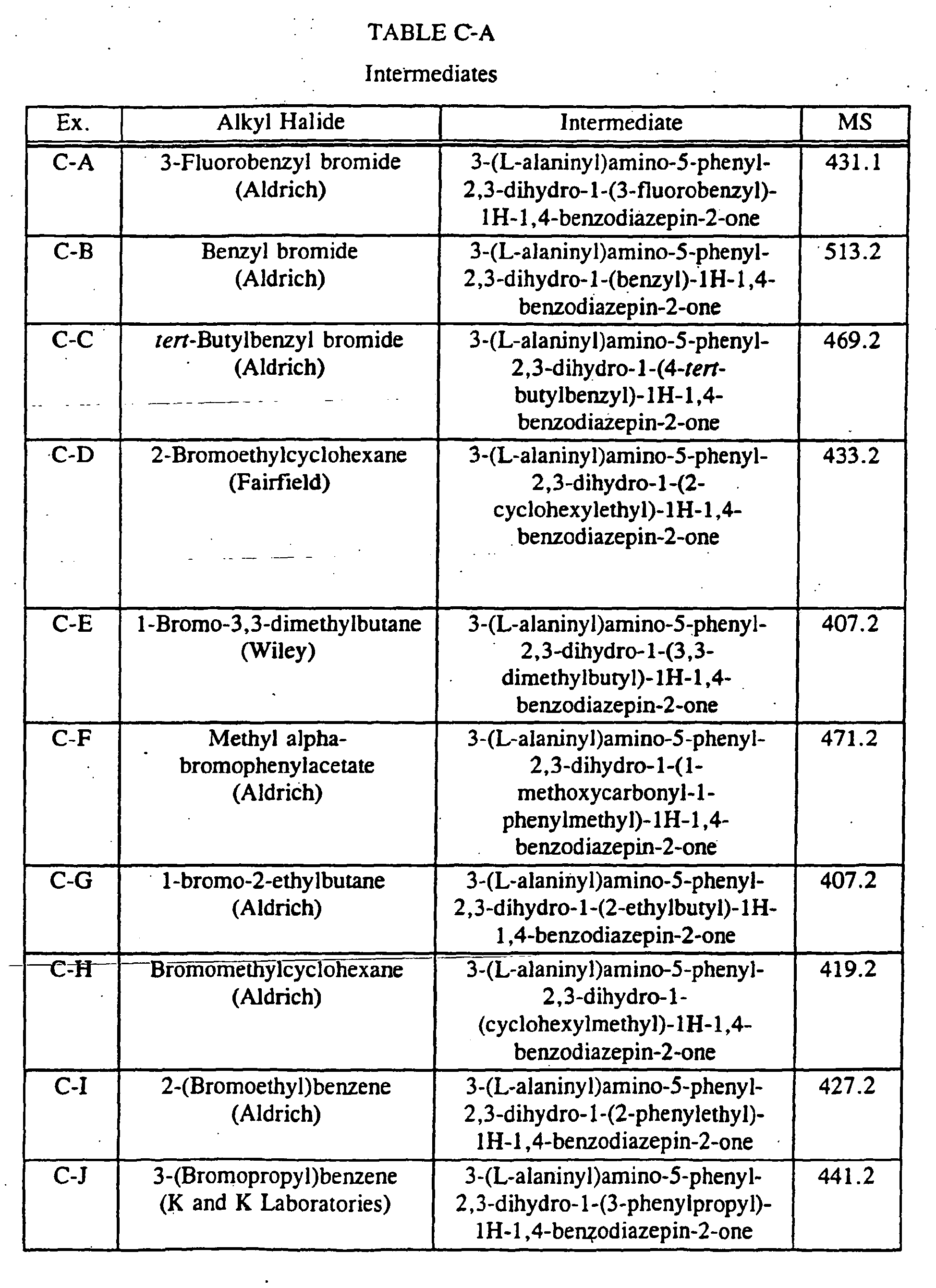

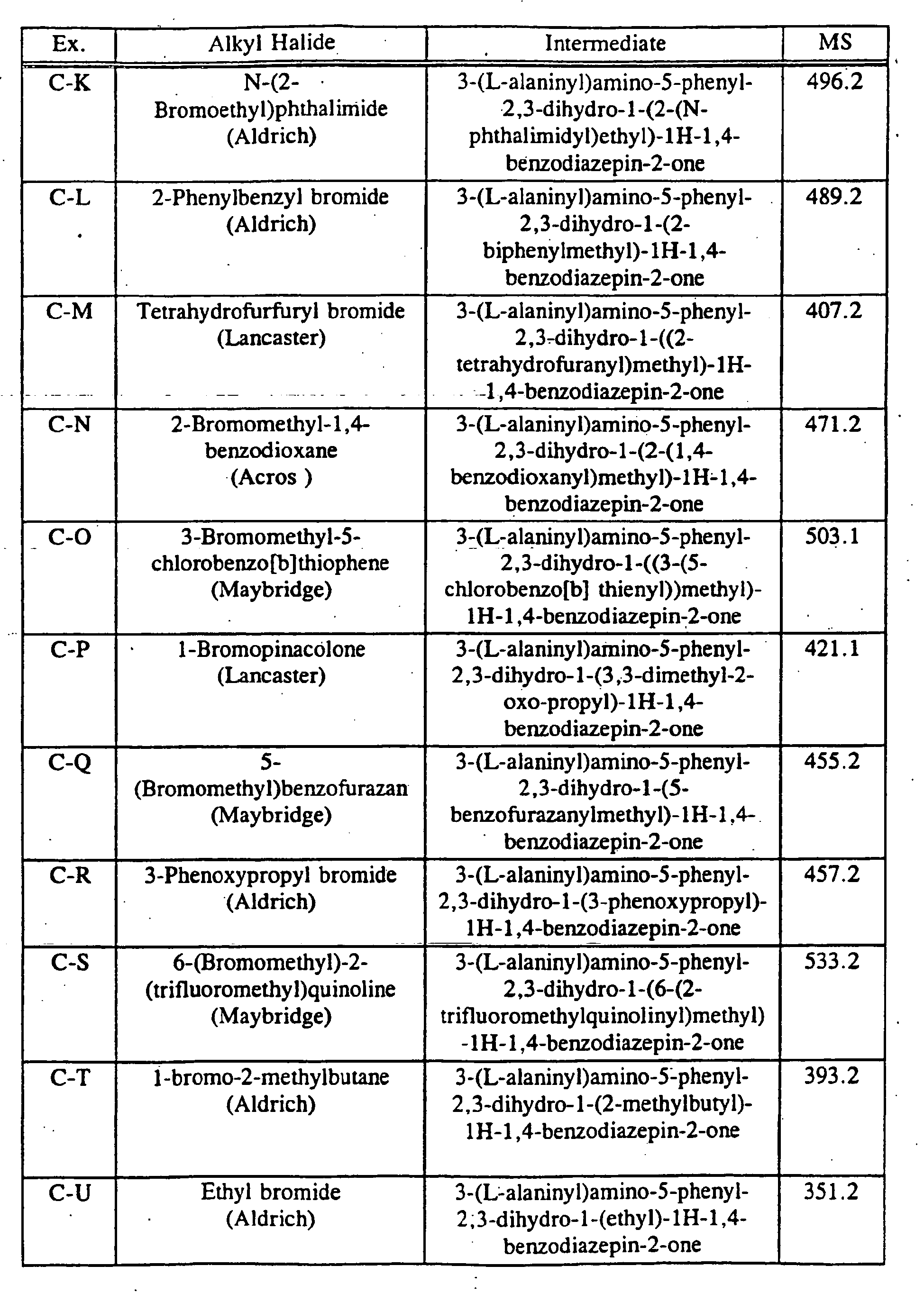

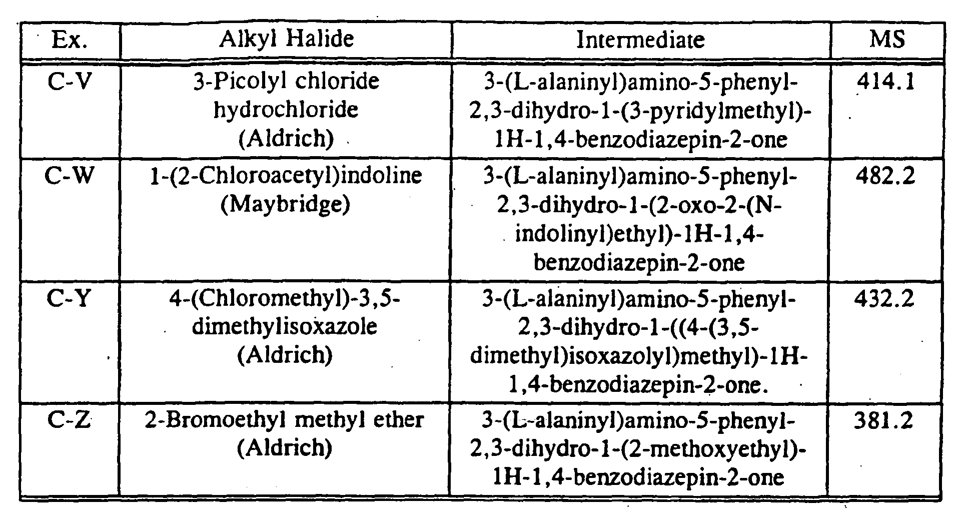

### GENERAL PROCEDURE C-I

To a 4 mL vial containing 0.03 mmol of starting material 2 (from General Procedure C-H) was added 100 uL of 0.25 mM stock solution of starting material 1 in chloroform, 100 uL of a 0.3 mM stock solution of 1-(3-dimethylaminopropyl)-3-ethyl carbodiimide in chloroform and 100 uL of a 0.3 mM stock solution of HOBt in DMF. After standing undisturbed for 48 h, the reaction mixture was concentrated and the residue redissolved in 2 mL of a 10% methanol/methylene chloride solution. This solution was then filtered through a pre-washed (methanol) 500 mg Si column using an additional 8 mL of the same solvent. The filtrate was concentrated under a stream of nitrogen to approximately-1/3-its-original volume and then passed over a plug (200 mg) of AG 1-8x anion exchange resin (Columns were pre-washed with 1N NaOH, water and methanol) using an additional 6 mL of 10% methanol/methylene chloride solution. The resulting filtrate was concentrated under vacuum and the crude products were submitted for testing without further purification. Product structure and purity were confirmed by HPLC and IEX MS.

### Example C-AA

### Synthesis of (S)-3-(L-phenylglycinyl)amino-2,3-dihydro-1-methyl-5-phenyl-1H-1,4-benzodiazepin-2-one

### Step. A: Synthesis of (S)-3-(N'-(tert-Butoxycarbonyl)-L-phenylglycinyl)amino-2,3-dihydro-1-methyl-5-phenyl-1H-1,4-benzodiazepin-2-one

To a solution of triethyl amine (519 uL, 3.8 mmol) and (S)-3-amino-5-phenyl-2-oxo-1,4-benzodiazepine (1.0 g, 3.8 mmol) (prepared according to the procedure of M. G. Bock et al., J. Org. Chem. 1987, 52, 3232-3239) in 100 mL of anhydrous methylene chloride at -20°C was added N-Boc-L-phenylglycine fluoride (Carpino et al, J. Org. Chem. 1991, 56, 2611-2614) in one portion. The reaction mixture was stirred for 15. min. and quenched with saturated aqueous bicarbonate (10 mL). The layers were seperated, the organic layer washed sequentially with saturated aqueous bicarbonate, water and brine and then dried over sodium sulfate. Purification of the crude product using silica gel chromatography (10-50% ethyl acetate / hexane) gave 1.3 g (69%) of a hydroscopic white foam.
NMR data was as follows:
¹H NMR (300 MHz, CDCl₃): δ = 1.35 (br s, 9H), 3.41 (s, 3H), 5.30-5.45 (m, 2H), 5.75-5.95 (m, 1H), 7.15-7.75 (m, 15H).
IR (CDCl₃): 1709.7, 1676.6, 1489, 1166.3 cm⁻¹.
IEX MS (M+1): 498.0.

### Step B: Synthesis of (S) 3 (L-phenylglycinyl)amino-2,3-dihydro-1-methyl-5-phenyl-1H-1,4-benzodiazepin-2-one

(S)-3-(N'-(*tert*-Butoxycarbonyl)-L-phenylglycinyl)amino-2,3-dihydro-1-methyl-5-phenyl-1H-1,4-benzodiazepin-2-one (1.27 g, 2.55 mmol) was added to 50 mL of a stirring solution of 15 % TFA in methylene chloride in one portion. After stirring 1h, the reaction mixture was concentrated under reduced pressure and the residue dissolved in 100 mL of methylene chloride. This solution was washed twice with saturated sodium bicarbonate, once with brine and then dried over sodium sulfate. Purification of the crude product using silica gel column chromatography (5-10% methanol/methylene chloride) gave 743 mg (73 %) of a very light green foam.
NMR data was as follows:
¹H NMR (CDCl₃): δ = 2.05 (br s, 1H), 3.45 (s, 3 H), 5.51 (d, J = 8.39 Hz, 1H), 7.15-7.70 (m, 14 H), 8.60 (d, J = 830 Hz, 1 H).
IR (CDCl₃): 1673.3, 1601.1, 1506.1 cm⁻¹.
IEX MS (M+1): 399.2.

### Example C-AB

### Synthesis of 3-(L-Alaninyl)amino-2,3-dihydro-1-(2-oxo-2-phenylethyl)-5-phenyl-1H-1,4-benzodiazepin-2-one

### Step A: Synthesis of 3-(Benzoxycarbonyl)amino-2,3-dihydro-1-(2-oxo-2-phenylethyl)-5-phenyl-1H-1,4-benzodiazepin-2-one

To a solution of 3-(Benzoxycarbonyl)amino-2,3-dihydro-5-phenyl-1H-1,4-benzodiazepin-2-one (Bock, M. G. et al, Tetrahedron Lett. 1987, 28, 939; 4.0 g, 10.4 mmol) in 40 mL of anhydrous DMF at 0°C was added potassium *tert-*butoxide (1.51 g, 13.5 mmol) in one portion. The reaction mixture was stirred 20 min. and α-bromoacetophenone (Lancaster; Windham, NH; 2.9 g, 14.6 mmol) was added. The reaction mixture was warmed to room temperature over 30 min. and then diluted with 100 mL of water and 200 mL of methylene chloride. The layers were separated. The organic layer was extracted with water and died over sodium sulfate. Purification of the crude product by silica gel column chromatography (0-5% ethyl acetate/methylene chloride) gave 4.2 g (81 %) of an off white foam.
NMR data was as follows:
¹H NMR (300 MHz, CDCl₃): δ = 5.16 (s, 2 H), 5.34 (s, 2 H), 5.50 (d, J = 8.33 Hz, 1 H), 6.70 (d, J = 8.28 Hz, 1 H), 7.20-7.70 (m, 12 H), 7.91 (d, J = 7.54 Hz, 2 H).
IR (CHCl₃): 1706.04, 1685.3, 1505.9, 1489.1, 1450.3, 1244.7 cm⁻¹.
IEX MS (M+1): 504.3.

### Step B: Synthesis of 3-Amino-2,3-dihydro-1-(2-oxo-2-phenylethyl)-5-phenyl-1H-1,4-benzodiazepin-2-one

A solution of 3-(Benzoxycarbonyl)amino-2,3-dihydro-1-(2-oxo-2-phenylethyl)-5-phenyl-1H-1,4-benzodiazepin-2-one (3.7 g, 7.36 mmol) in 100 mL of anhydrous methylene chloride was cooled to 0°C under nitrogen. A stream of anhydrous HBr gas was then bubbled through this solution for 1 h. The bubbler was removed and the reaction was warmed to room temperature under nitrogen. After stirring 1 h the reaction was concentrated under vacuum and the residue was redissolved in 20 mL of methylene chloride. The crude HBr salt of the product was precipitated from solution using 300 mL of anhydrous ether and collected by filtration as a light yellow solid. After washing with ether , the solid was dissolved in methylene chloride and saturated sodium bicarbonate. The layers were separated and the organic layer was extracted with saturated sodium bicarbonate. The combined aqueous layers were then back extracted twice with methylene chloride. The combined organic layers were extracted once with water and dried over sodium sulfate. After concentration under vacuum, 2.27 g of the product was obtained as an orange foam which was carried on without further purification.
NMR data was as follows:
¹H NMR (300 MHz, CDCl₃): δ = 2.60 (br s, 2 H), 4.72 (s, 1 H), 5.34 (s, 2 H), 7.10-7.70 (m, 12 H), 7.91 (d, J = 7.60 Hz, 2 H).
IEX MS (M+1): 370.2

### Step-C: Synthesis of 3-N'-(tert-Butoxycarbonyl)-L-alaninyl)amino-2,3-dihydro-1-(2-oxo-2-phenylethyl)-5-phenyl-1H-1,4-benzodiazepin-2-one

To a solution of HOBt-H₂O (697 mg, 5.16 mmol), N,N-diisopropylethylamine (900 uL, 5.16 mmol) and N-t-BOC-L-alanine (975 mg, 5.16 mmol) in 20 mL of anhydrous THF at 0°C was added 1-(3-dimethylaminopropyl)-3-ethyl carbodiimide hydrochloride (EDCI; 986 mg, 5.16 mmol) in one portion. After stirring 5 min., a solution of 3-amino-2,3-dihydro-1-(2-oxo-2-phenylethyl)-5-phenyl-1H-1,4-benzodiazepin-2-one (2.0 g, 5.43 mmol) in 20 mL of anhydrous THF was added via syringe and the reaction mixture was warmed to room temperature and stirred overnight. The reaction mixture was diluted with 200 mL methylene chloride, extracted sequentially with 10 % citric acid, saturated sodium bicarbonate, water and brine and then dried over sodium sulfate. Purification of the crude product using silica gel chromatography (10%-30% ethyl acetate/methylene chloride) gave 2.59 g (93%) of a white foam.
NMR data was as follows:
¹H NMR (300 MHz, CDCl₃): δ = 1.30-1.60 (m, 12 H), 4.35 (br s, 1H), 5.00-5.50 (m, 3 H), 5.65-5.70 (m, 1H), 7.15-7.65 (m, 12 H), 7.70-7.80 (m, 1 H), 7.85-7.95 (m, 1 H).
IR (CHCl₃): 1705.8, 1678.8, 1488.7, 1450.2, 1230.4, 1164.4 cm⁻¹.
IEX MS (M+1): 541.2.

### Step D: Synthesis of 3-(L-Alaninyl)amino-2,3-dihydro-1-(2-oxo-2-phenylethyl)-5-phenyl-1H-1,4-benzodiazepin-2-one

3-(N'-(*tert*-Butoxycarbonyl)-L-alaninyl)amino-2,3-dihydro-1-(2-oxo-2-phenylethyl)-5-phenyl-1H-1,4-benzodiazepin-2-one (2.5 g, 4.63 mmol) was added to 100 mL of a stirring solution of 15 % TFA/methylene chloride in one portion. After stirring 2 h, the reaction mixture was concentrated under reduced pressure and the residue was dissolved in 150 mL of methylene chloride. This solution was washed twice with saturated sodium bicarbonate, once with brine and then dried over sodium sulfate. Purification of the crude product using silica gel column chromatography (1-10% methanol/methylene chloride) gave 1.91 g (94%) of the title compound as a white foam.
NMR data was as follows:
¹H NMR (300 MHz, CDCl₃): δ = 1.30-1.50 (m, 3 H), 1.80-2.20 (br s, 2 H), 3.55-3.75 (m, 1 H), 5.20-5.45 (m, 2 H), 5.67 (t, J = 7.48 Hz, 1 H), 7.20-7.65 (m, 12 H), 7.90 (d, J = 7.7 Hz, 2 H), 8.80 (dd, J₁ = 25.09 Hz, J₂ = 8.33 Hz, 1 H).
EX MS (M + 1): 441.2.

### Example C-AC

### Synthesis of 3-(L-Alaninyl)amino-2,3-dihydro-1-(4,4,4-trifluorobutyl)-5-phenyl-1H-1,4-benzodiazepin-2-one

### Step A: Synthesis of 3-(Benzoxycarbonyl)amino-2,3-dihydro-1-(4,4,4-trifluorobutyl)-5-phenyl-1H-1,4-benzodiazepin-2-one

To a solution of 3-(benzoxycarbonyl)amino-2,3-dihydro-5-phenyl-1H-1,4-benzodiazepin-2-one (3.7 g, 9.61 mmol) in 40 mL of anhydrous DMF at 0°C was added potassium tert-butoxide (1.6 g, 14.4 mmol) in one portion. The reaction mixture was stirred 20 min. and 4,4,4-trifluoro-1-bromobutane (Lancaster; Windham, NH; 2.6 g, 13.4 mmol) was added. The reaction mixture was warmed to room temperature over 30 min. and then diluted with 100 mL of water and 200 mL of methylene chloride. The layers were separated. The organic layer was extracted with water and dried over sodium sulfate. Purification of the crude product by silica gel column chromatography (0-3. % ethyl acetate) / methylene chloride) gave 1.52 g (32 %) of an off white foam.
NMR data was as follows:
¹H NMR (300 MHz, CDCl₃): δ = 1.50-2.10 (m, 4 H), 3.70-3.90 (m, 1 H), 4.35-4.55 (m, 1 H), 5.15 (s, 2 H), 5.33 (d, J = 8.47 Hz, 1 H), 6.67 (d, J = 8.40 Hz, 1 H), 7.2-7.70 (m, 14 H).
IR (CHCl₃): 1720.4, 1683.0, 1604.8, 1505.5, 1451.1, 1323.9, 1254.5, 1148.4 cm⁻¹.
IEX MS (M+1): 496.3.

### Step B: Synthesis of 3-Amino-2,3-dihydro-1-(4,4,4-trifluorobutyl)-5-phenyl-1H-1,4-benzodiazepin-2-one

A solution of 3-(benzoxycarbonyl)amino-2,3-dihydro-1-(4,4,4-trifluorobutyl)-5-phenyl-1H-1,4-benzodiazepin-2-one (1.42 g, 2.87 mmol) in 50 mL of anhydrous methylene chloride was cooled to 0°C under nitrogen. A stream of anhydrous HBr gas was slowly bubbled through the solution for 1 h. The bubbler was removed and the reaction was warmed to room temperature under nitrogen. After stirring for 1 h, the reaction was concentrated under vacuum and the residue was redissolved in 10 mL of methylene chloride. The crude HBr salt of the product was precipitated from solution using 90 mL of anhydrous ether and collected by filtration. After washing with ether, the HBr salt was dissolved in methylene chloride and saturated sodium bicarbonate. The layers were separated and the organic layer was extracted with saturated sodium bicarbonate. The combined aqueous layers were then back extracted twice with methylene chloride. The combined organic layers were extracted once with water and dried over sodium sulfate. After concentration under vacuum, 1.06 g (100%) of the product was obtained as a white foam which was carried on without further purification.
NMR data was as follows:
¹H NMR (300 MHz, CDCl₃): δ = 1.60-2.10 (m, 4 H), 2.76 (br s, 2 H), 3.75-3.85 (m, 1 H), 4.40-4.60 (m, 2 H), 7.20-7.70 (m, 9 H).
IEX MS (M+1): 362.1.

### Step C: Synthesis of 3-(N'-(tert-Butoxycarbonyl)-L-alaninyl)amino-2,3-dihydro-1-(4,4,4-trifluorobutyl)-5-phenyl-1H-1,4-benzodiazepin-2-one

To a solution of HOBt-H₂O (373 mg, 2.76 mmol), N,N-diisopropylethylamine (481 uL, 2.76 mmol) and N-t-BOC-L-alanine (522 mg, 2.76 mmol) in 10 mL of anhydrous THF at 0°C was added 1-(3-dimethylaminopropyl)-3-ethyl carbodiimide hydrochloride (EDCI; 527 mg, 2.76 mmol) in one portion. After stirring 5 min., a solution of 3-amino-2,3-dihydro-1-(4,4,4-trifluorobutyl)-5-phenyl-1H-1,4-benzodiazepin-2-one (1.05 g, 2.91 mmol) in 10 mL of anhydrous THF was added via syringe and the reaction mixture was warmed to room temperature and stirred overnight. The reaction mixture was diluted with 100 mL methylene chloride, extracted sequentially with 10% citric acid, saturated sodium bicarbonate, water and brine and then dried over sodium sulfate. Purification of the crude product using silica gel chromatography (10%-30% ethyl acetate/methylene chloride) gave 1.28 g (83 %) of a white foam.
NMR data was as follows:
¹H NMR (300 MHz, CDCl₃): δ = 1.40-2.10 (m, 16 H), 3.70-3.85 (m, 1 H), 4.30-4.55 (m, 2 H), 5.10 (br s, 1 H), 5.45-5.55 (m, 1 H), 7.25-7.80 (m, 10 H).
IR (CDCl₃): 1676.6, 1605.2, 1488.6, 1450.9, 1393.2, 1338.7, 1324.9, 1253.8, 1150.4 cm⁻¹.
IEX MS (M+1): 533.1.

### Step D: Synthesis of 3-(L-Alaninyl)amino-2,3-dihydro-1-(4,4,4-trifluorobutyl)-5-phenyl-1H-1,4-benzodiazepin-2-one

3-(N'-(*tert*-Butoxycarbonyl)-L-alaninyl)amino-2,3-dihydro-1-(4,4,4-trifluorobutyl)-5-phenyl-1H-1,4-benzodiazepin-2-one (1.21 g, 2.27 mmol) was added to 50 mL of a stirring solution of 15 % TFA / methylene chloride in one portion. After stirring 2 h, the reaction mixture was concentrated under reduced pressure and the residue was dissolved in 100 mL of methylene chloride. This solution was washed twice with saturated sodium bicarbonate, once with brine and then dried over sodium sulfate. Purification of the crude product using silica gel column chromatography (1-5% methanol / methylene chloride) gave 670 mg (68%) of a light pink foam.
NMR data was as follows:
¹H NMR (300 MHz, CDCl₃): δ = 1.43 (t, J = 7.0 Hz, 3 H), 1.60-2.20 (m, 7 H), 3.60-3.85 (m, 2 H), 4.35-4.55 (m, 1 H), 5.51 (dd, J₁ = 8.36 Hz, J₂ = 2.48 Hz, 1 H), 7.20-7.70 (m, 9 H), 8.80 (dd, J₁ = 27.73 Hz, J₂ = 8.34 Hz, 1 H).
IEX MS (M+1): 433.2.

### Example C-AD

### Synthesis of 3-(N'-(Chloroacetyl)-L-alaninyl)amino-2,3-dihydro-1-methyl-5-phenyl-1H-1,4-benzodiazepin-2-one

A solution of 3-(L-alaninyl)amino-2,3-dihydro-1-methyl-5-phenyl-1H-1,4-benzodiazepin-2-one (20.0 mg, 0.0595 mmol), α-chloroacetyl chloride (5.9 uL, 0.0744 mmol) and piperidinylmethyl polystyrene (59.5 mg, 3.6 mmol/g (Fluka)) in 1 mL of methylene chloride were shaken for 20 min. Aminomethyl polystyrene (58 mg, 3.0 mmol/g (Advanced Chemtech)) was then added and the reaction mixture was shaken for an additional 15 min. and filtered. Removal of the solvent under reduced pressure provided 23.9 mg (98%) of the crude product which was used without further purification.
NMR data was as follows:
¹H NMR (300 MHz, CDCl₃): δ = 1.40-1.60 (m, 3 H), 3.40-3.6 (m, 3 H), 4.1 (s, 2 H), 4.60-4.80 (m, 1 H), 5.45-5.50 (m, 1 H), 7.20-7.90 (m, 11 H).

Using the procedures indicated, the compounds shown in Table C-1 were prepared.

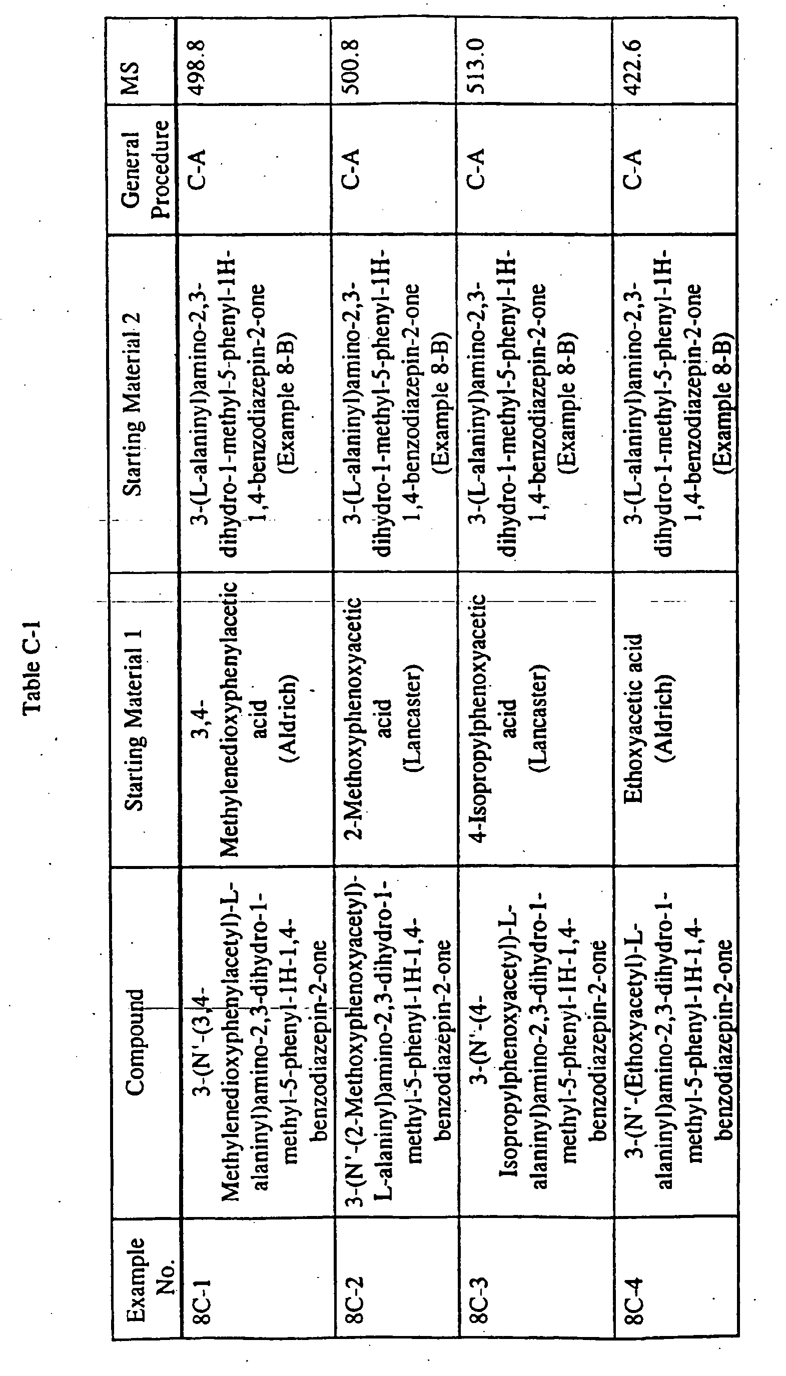

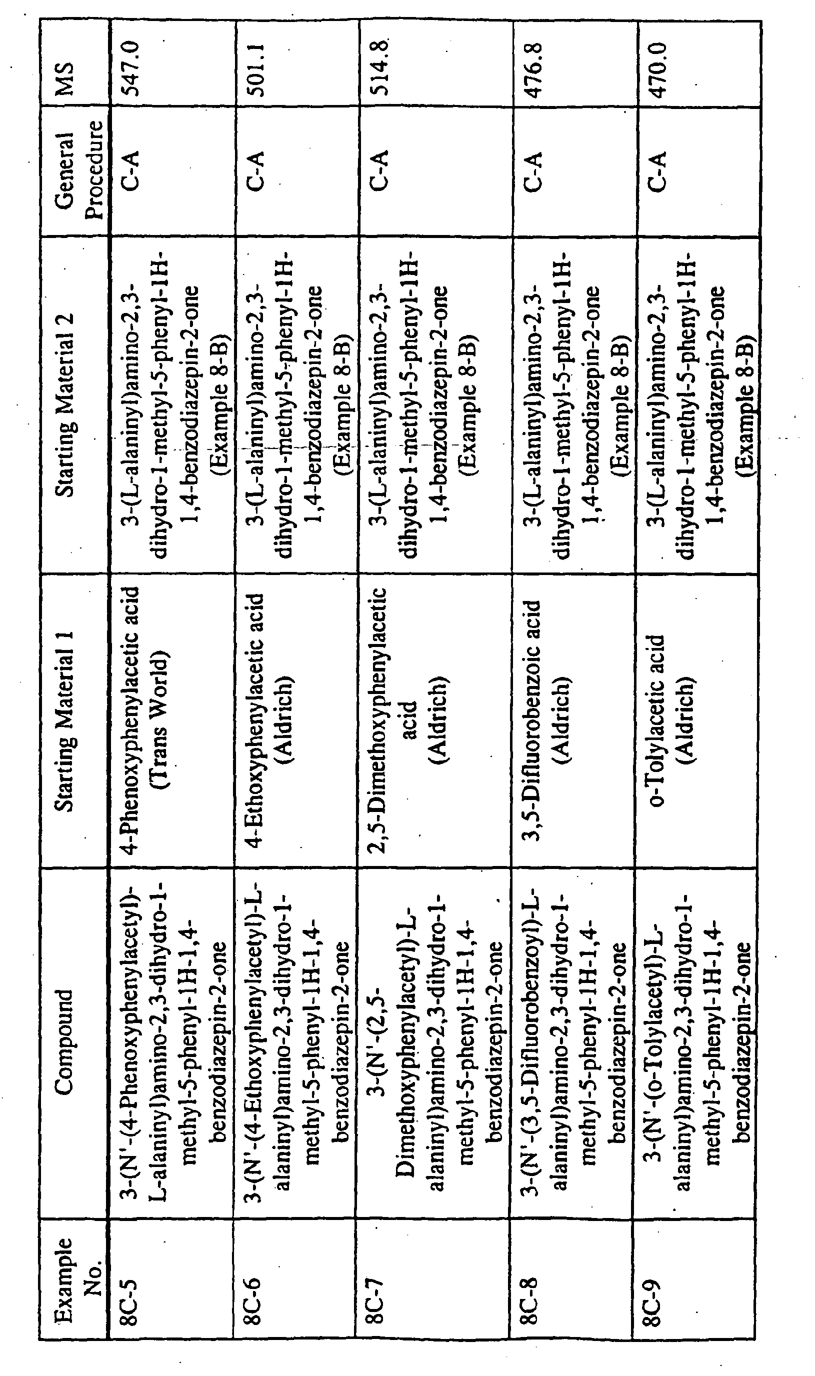

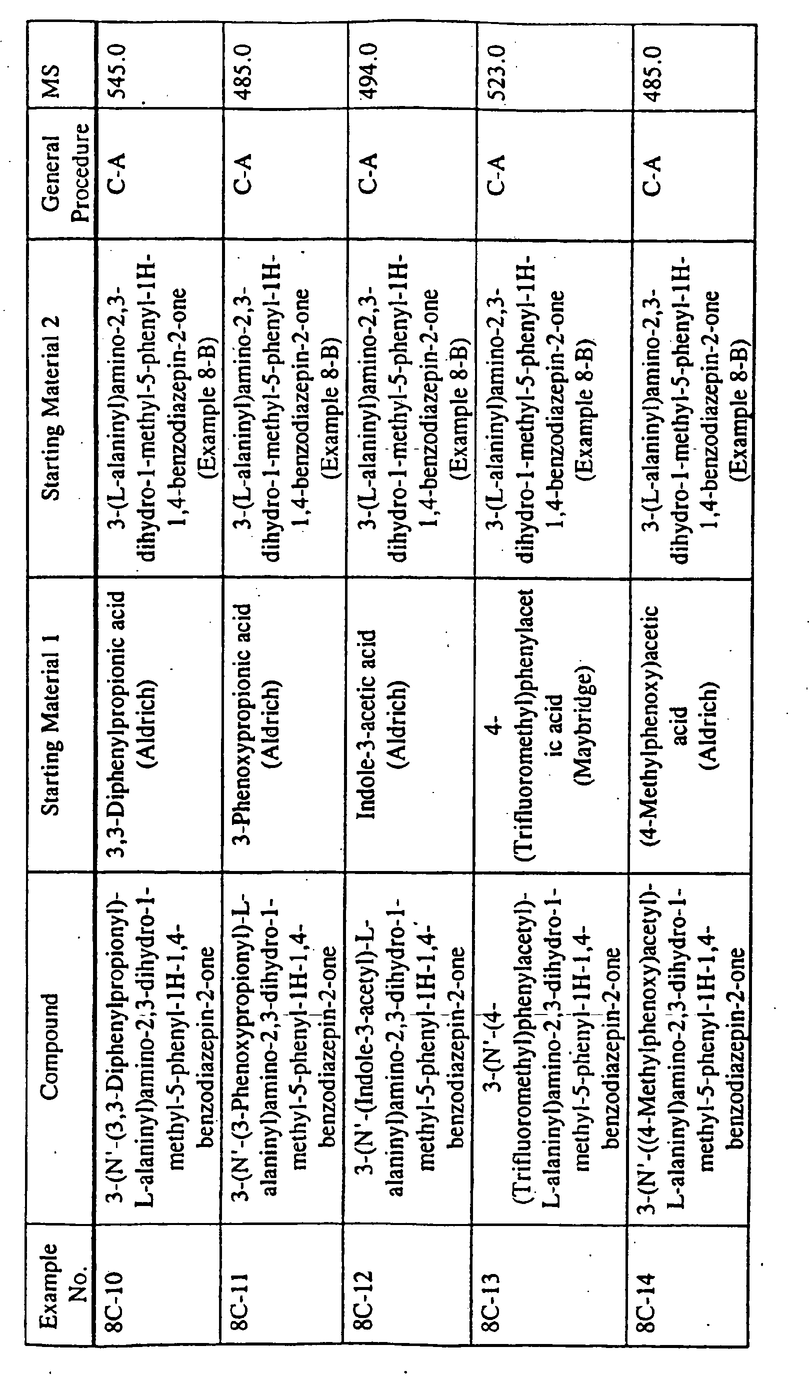

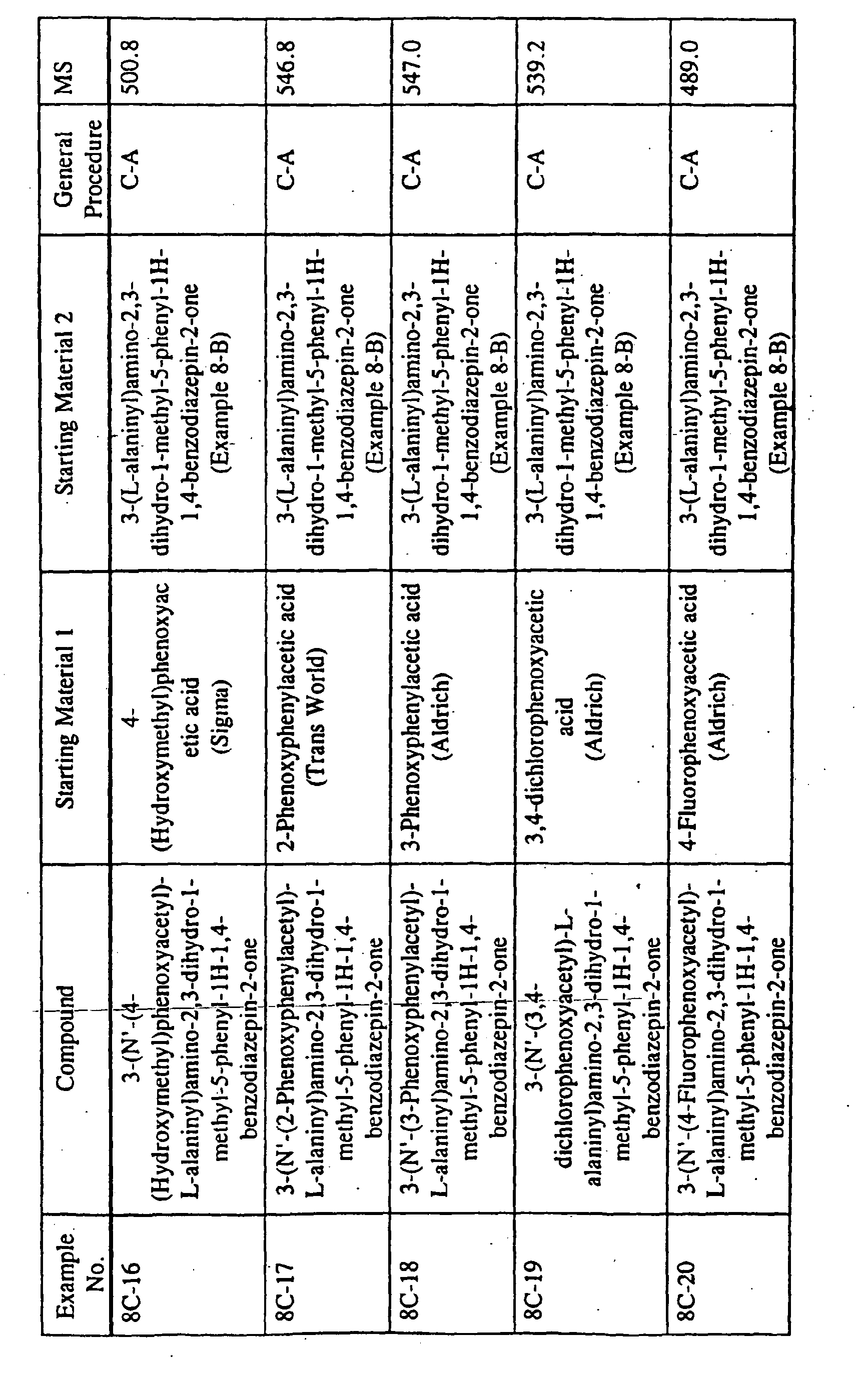

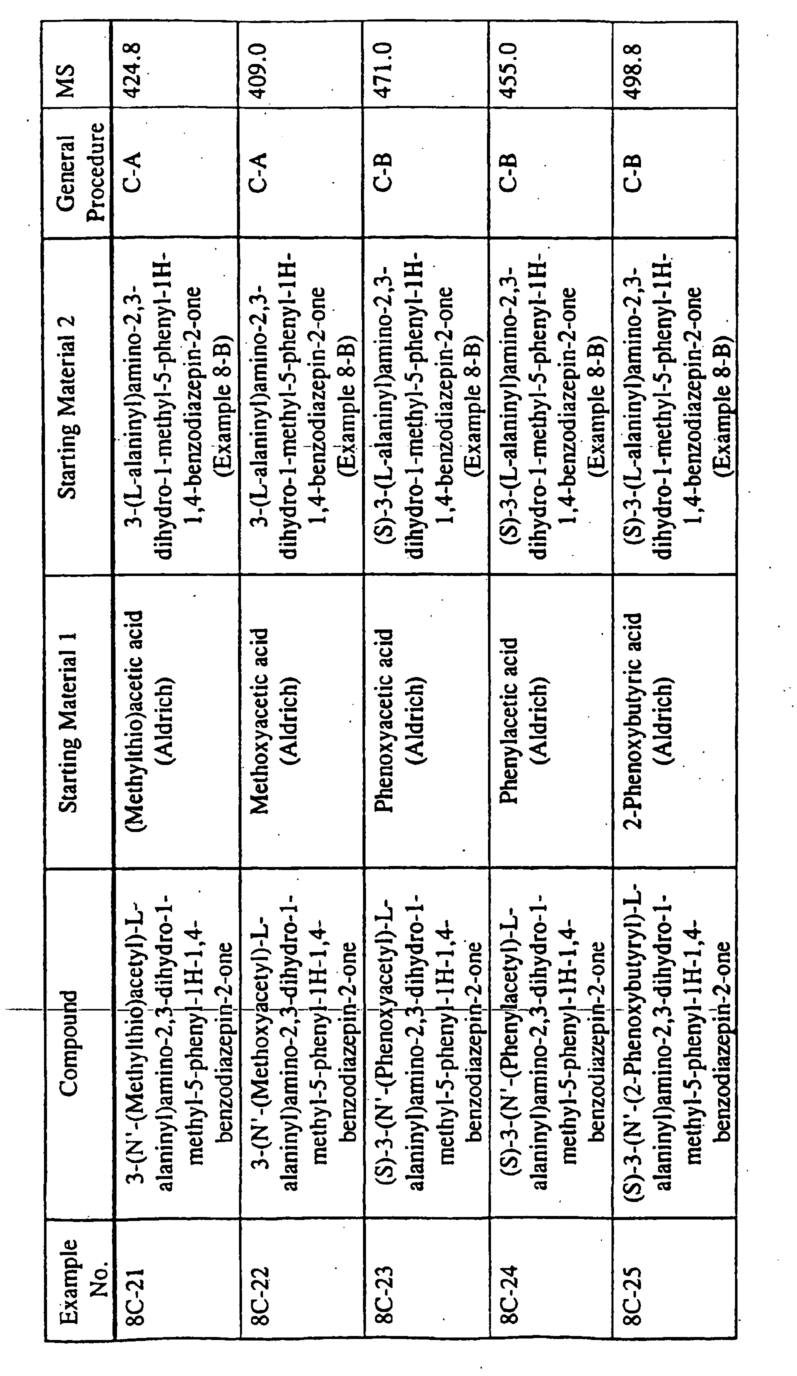

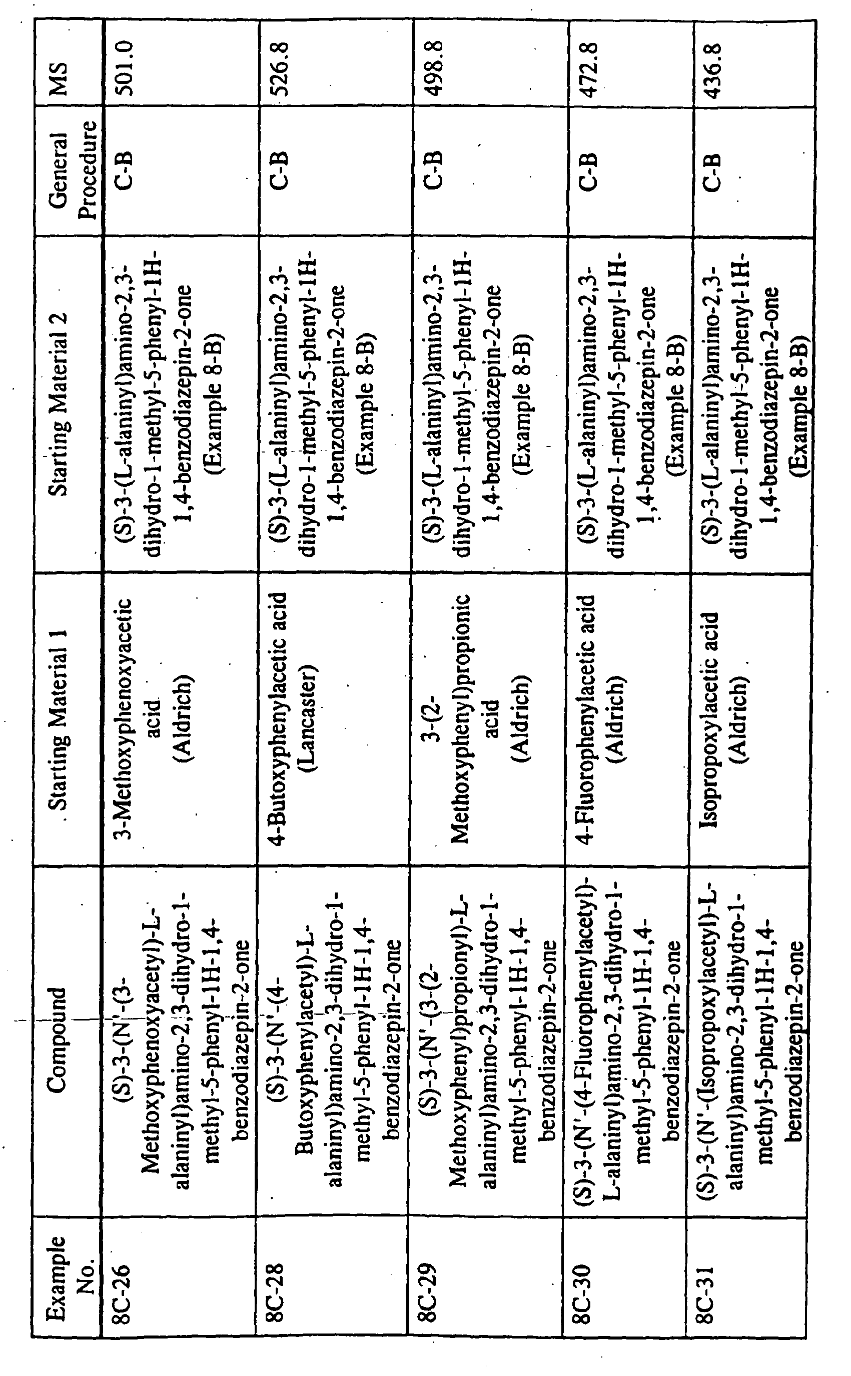

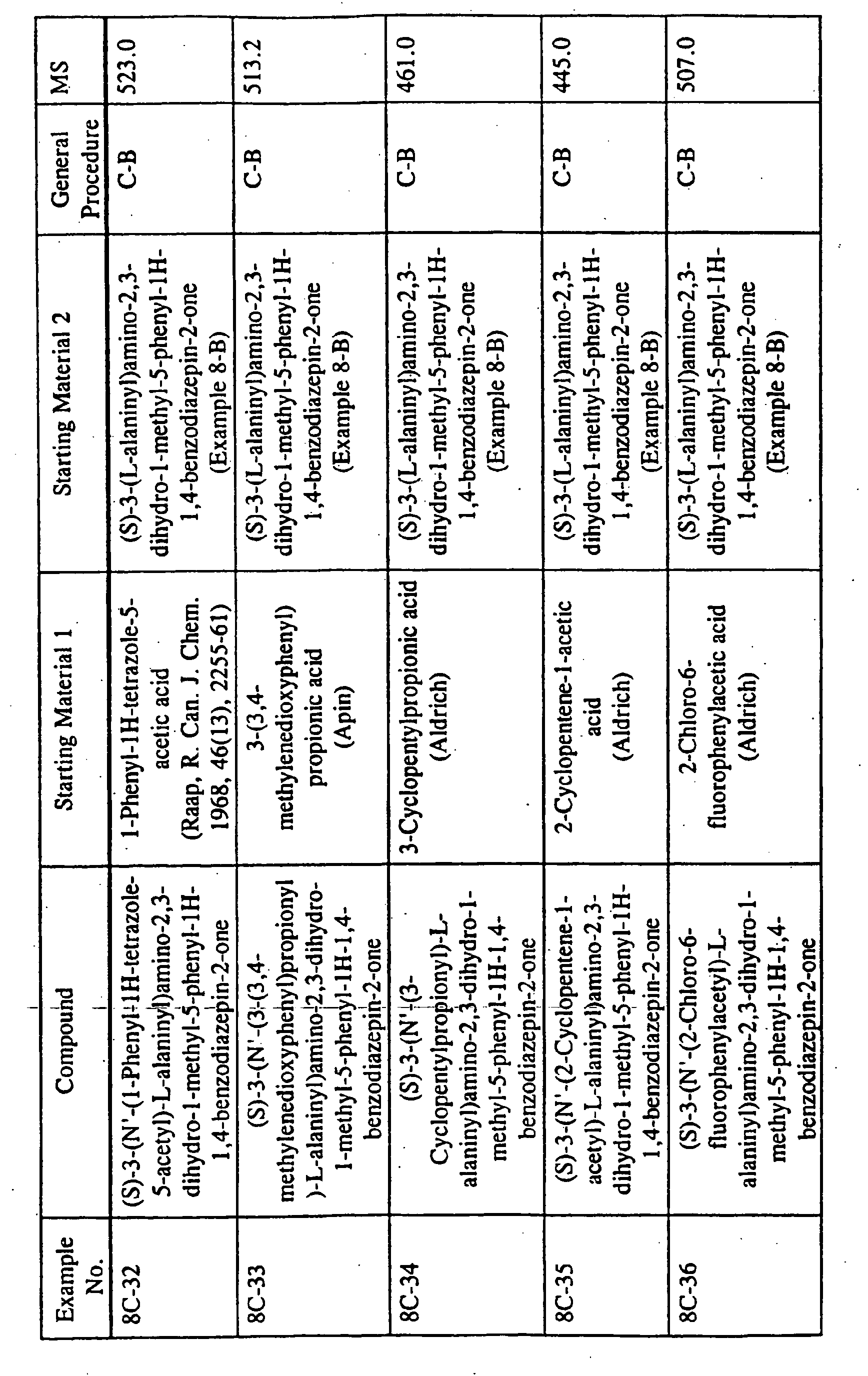

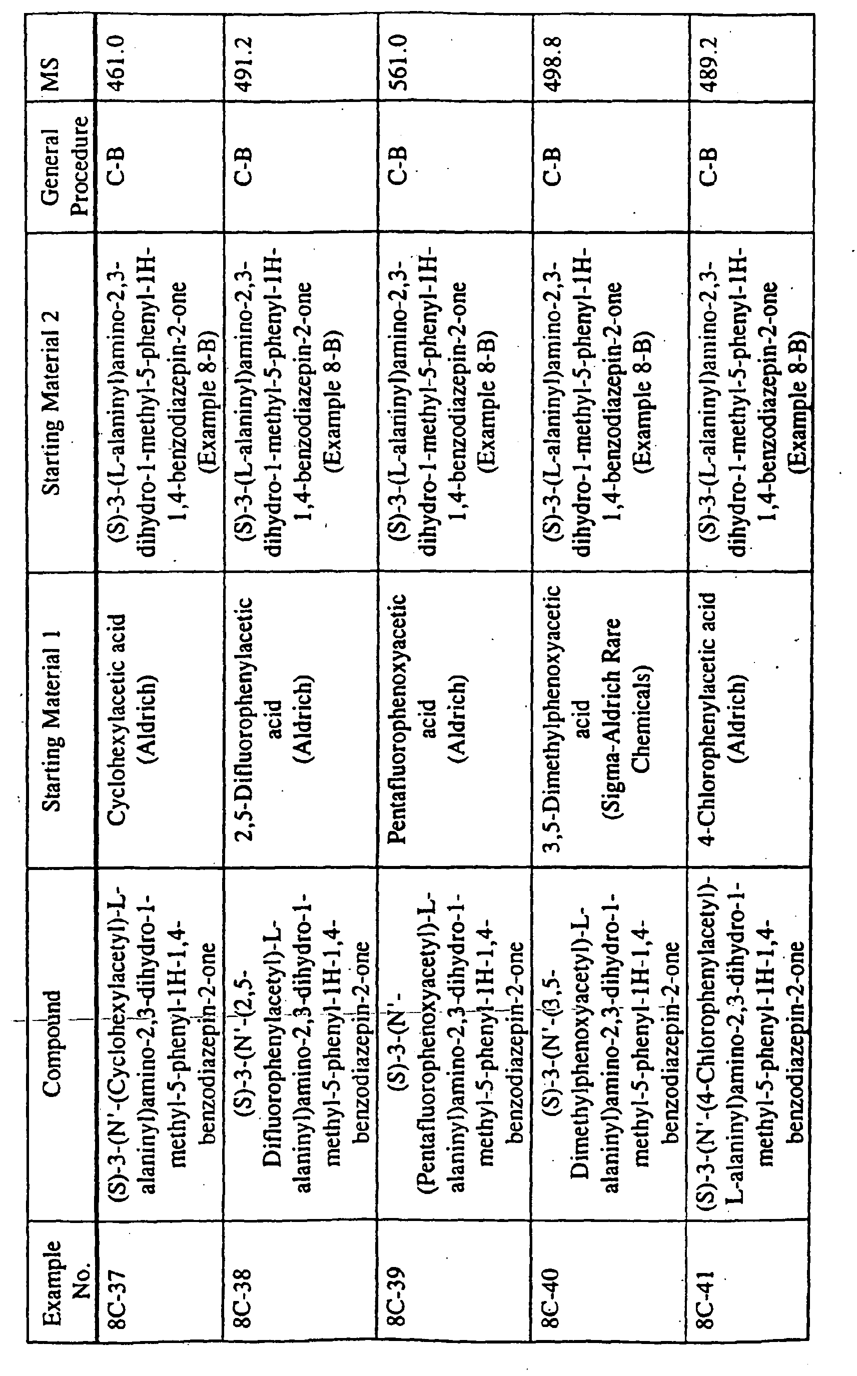

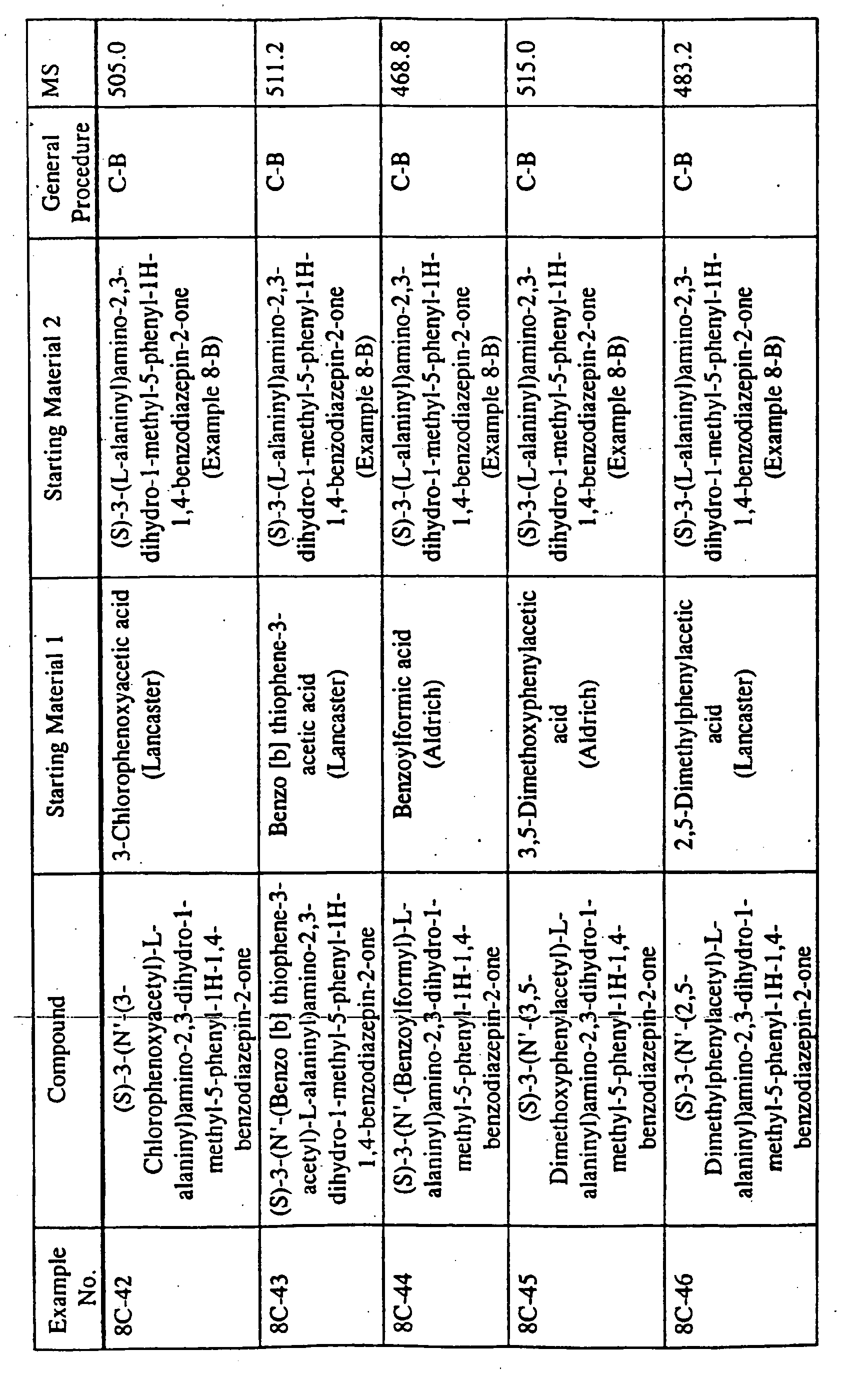

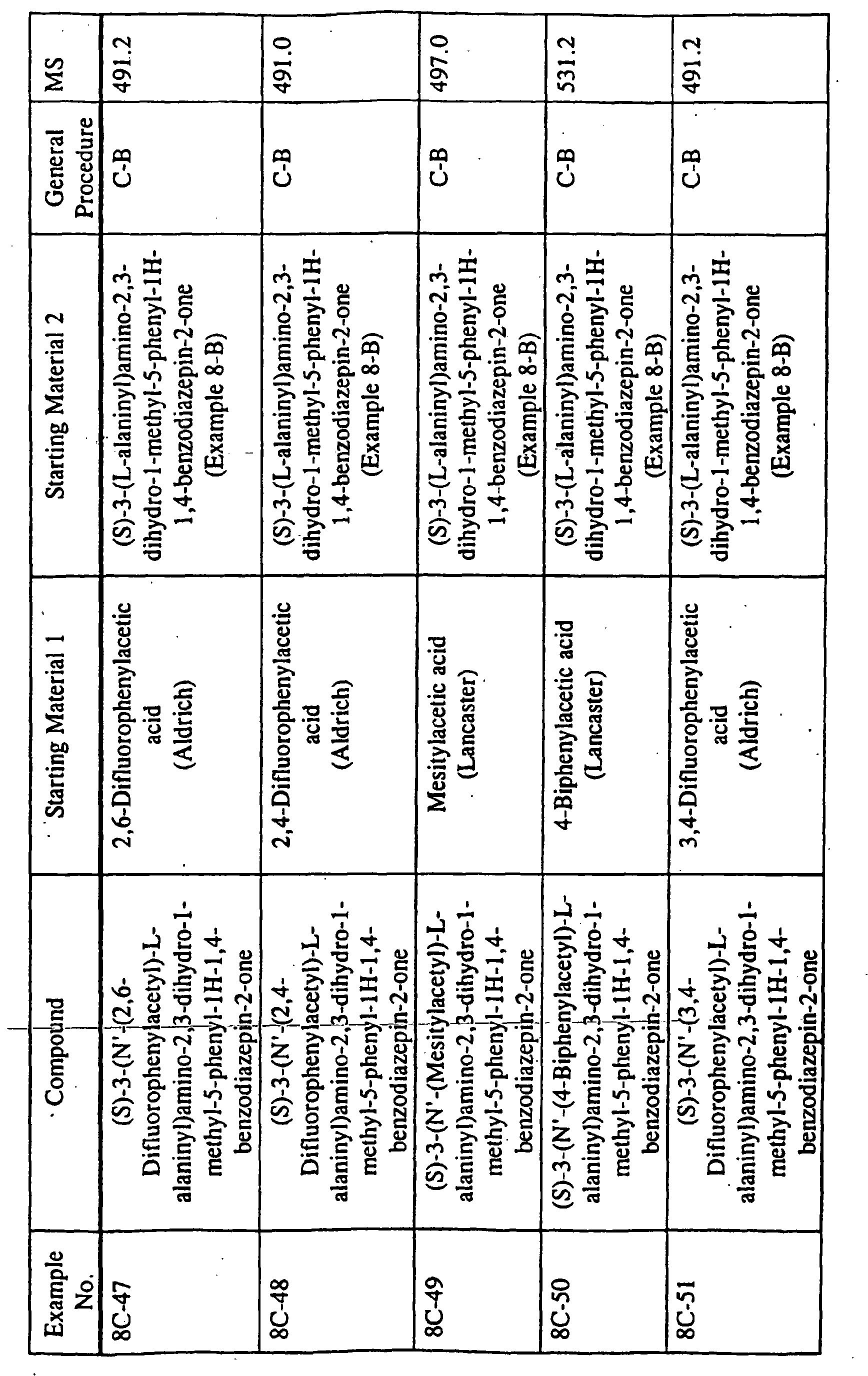

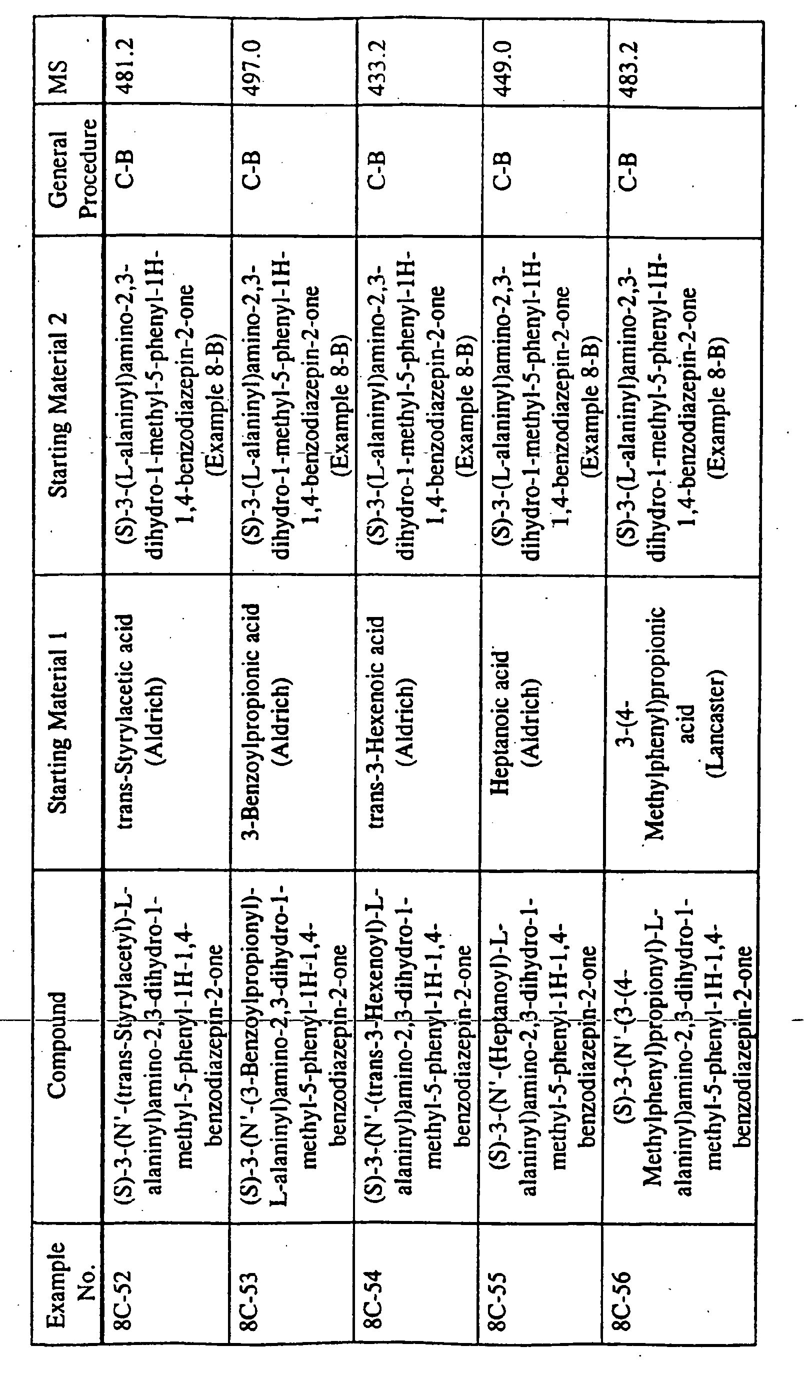

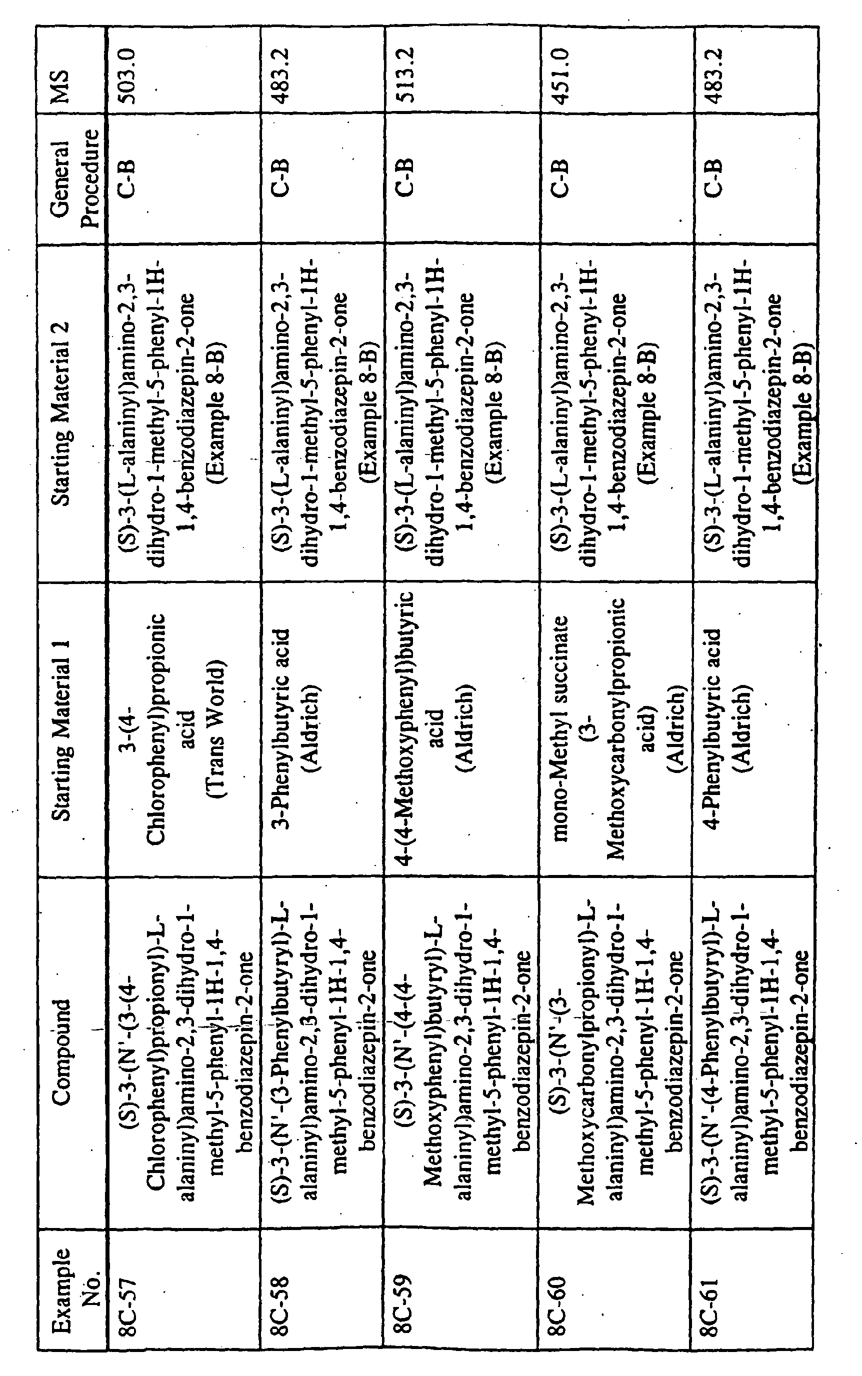

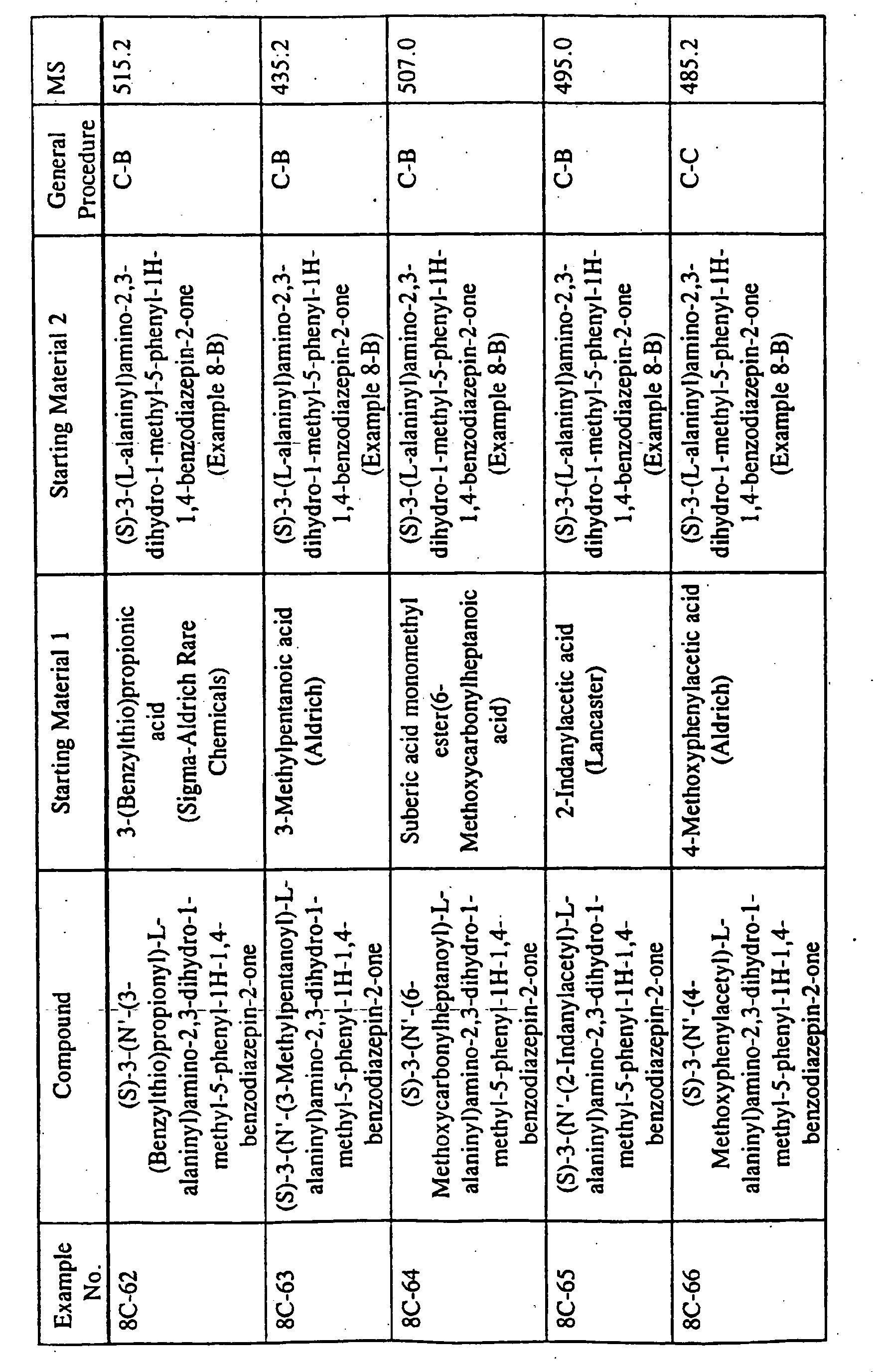

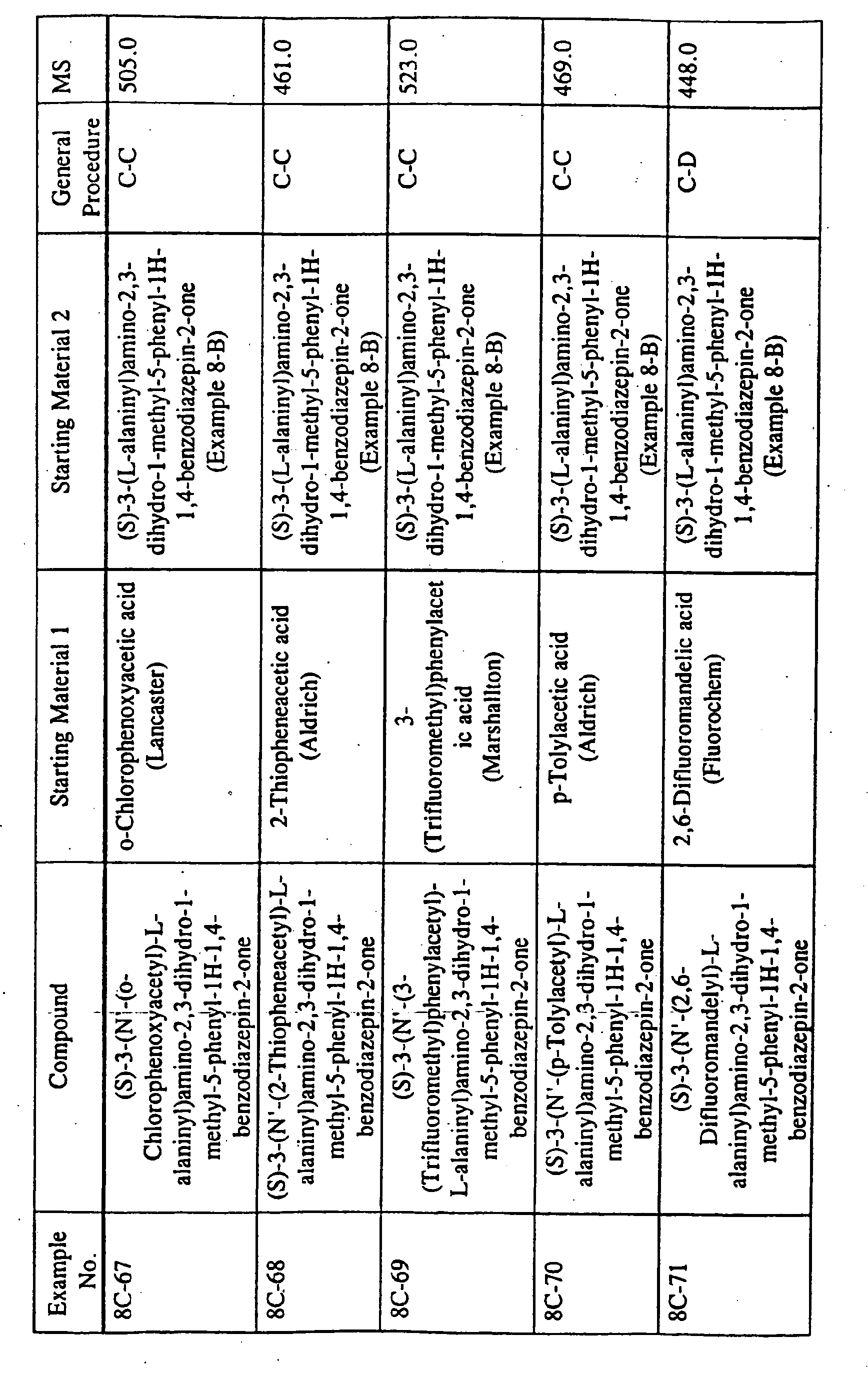

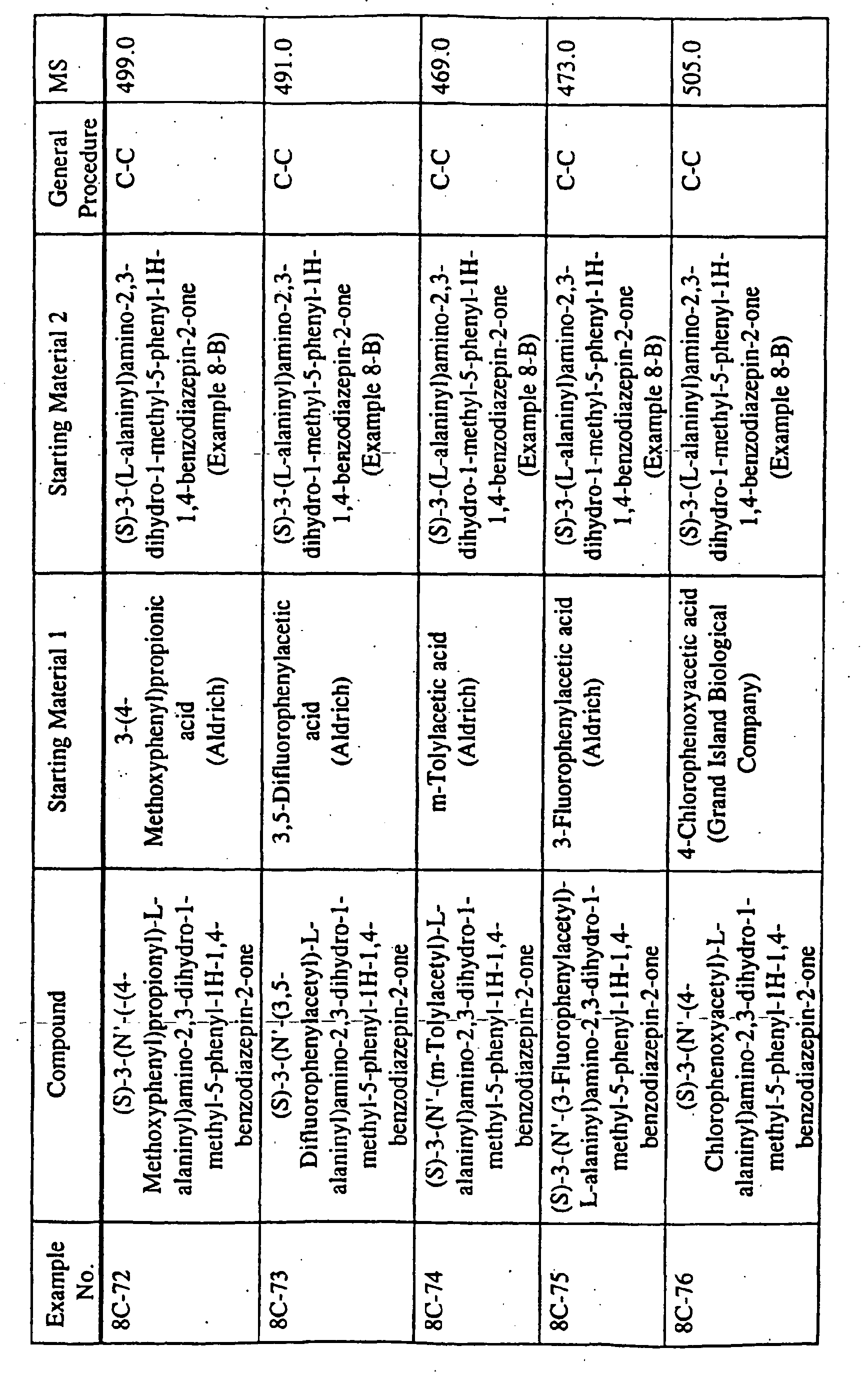

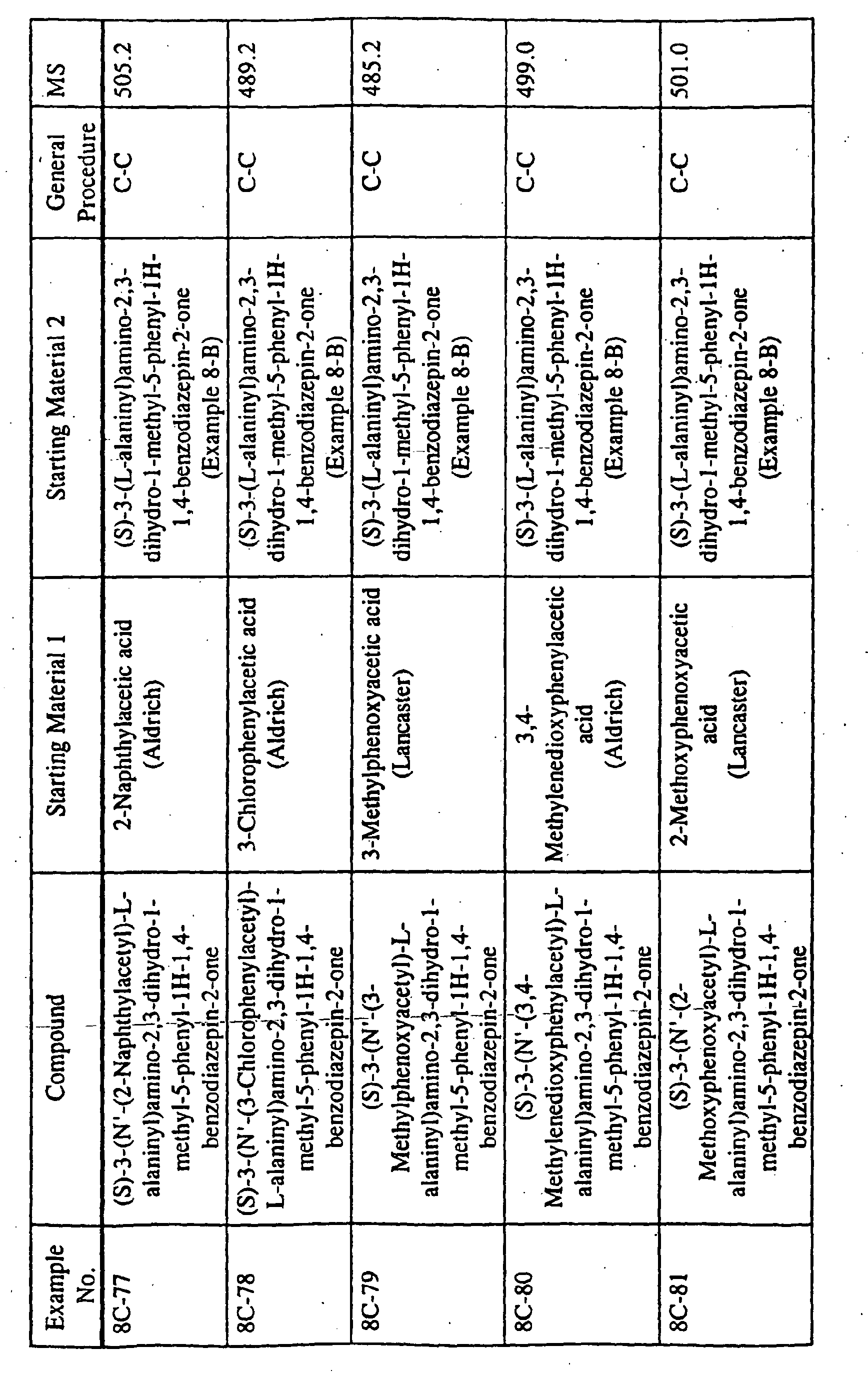

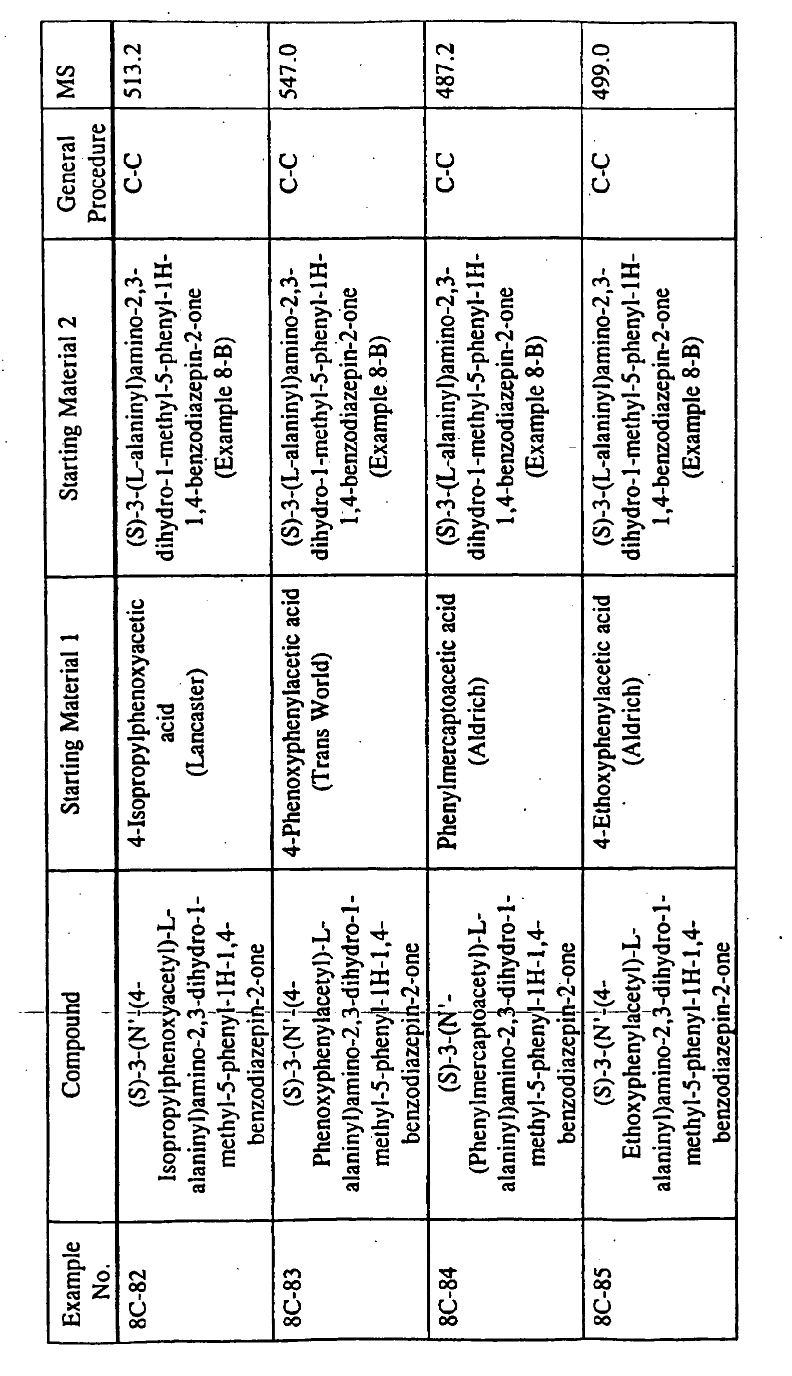

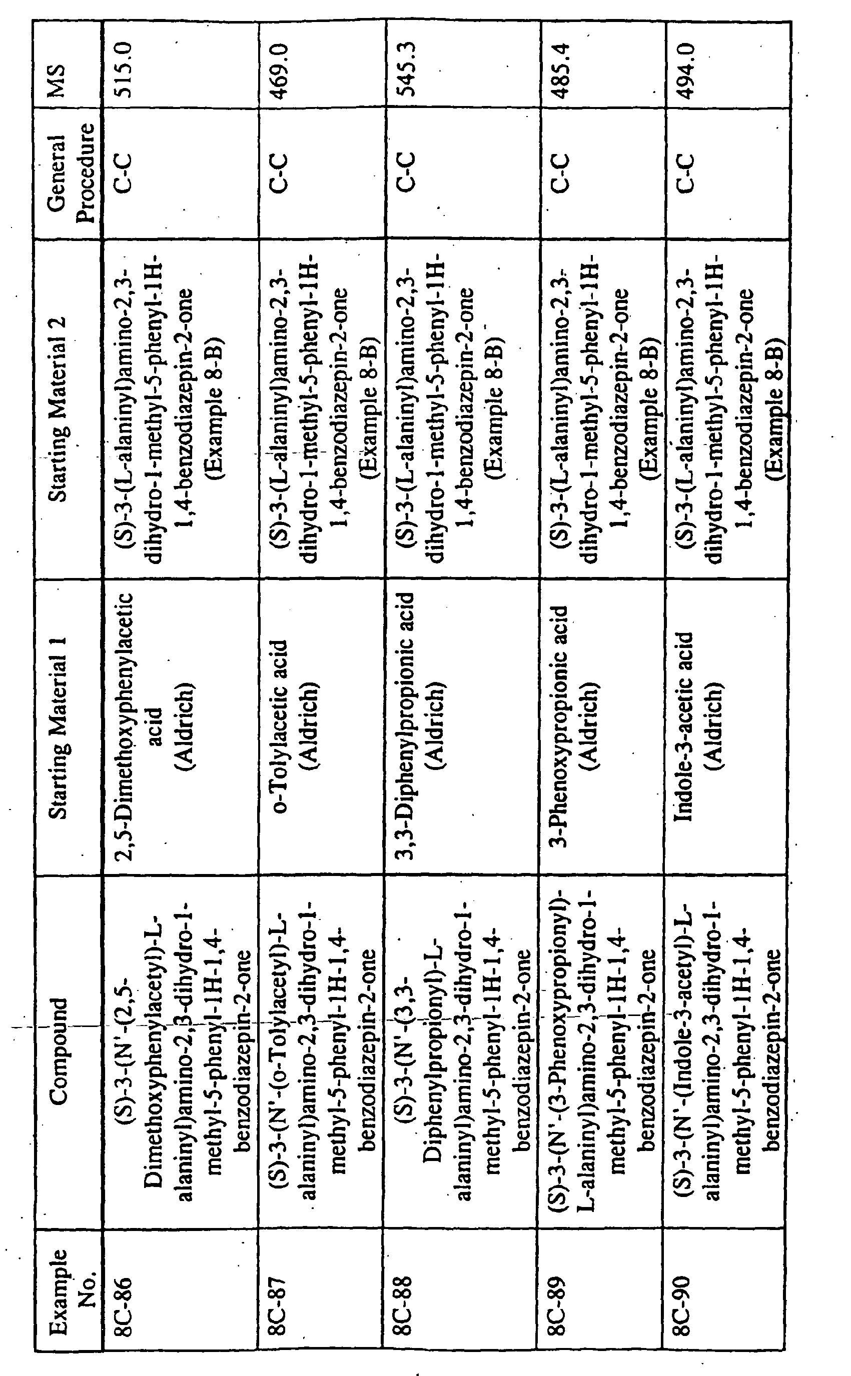

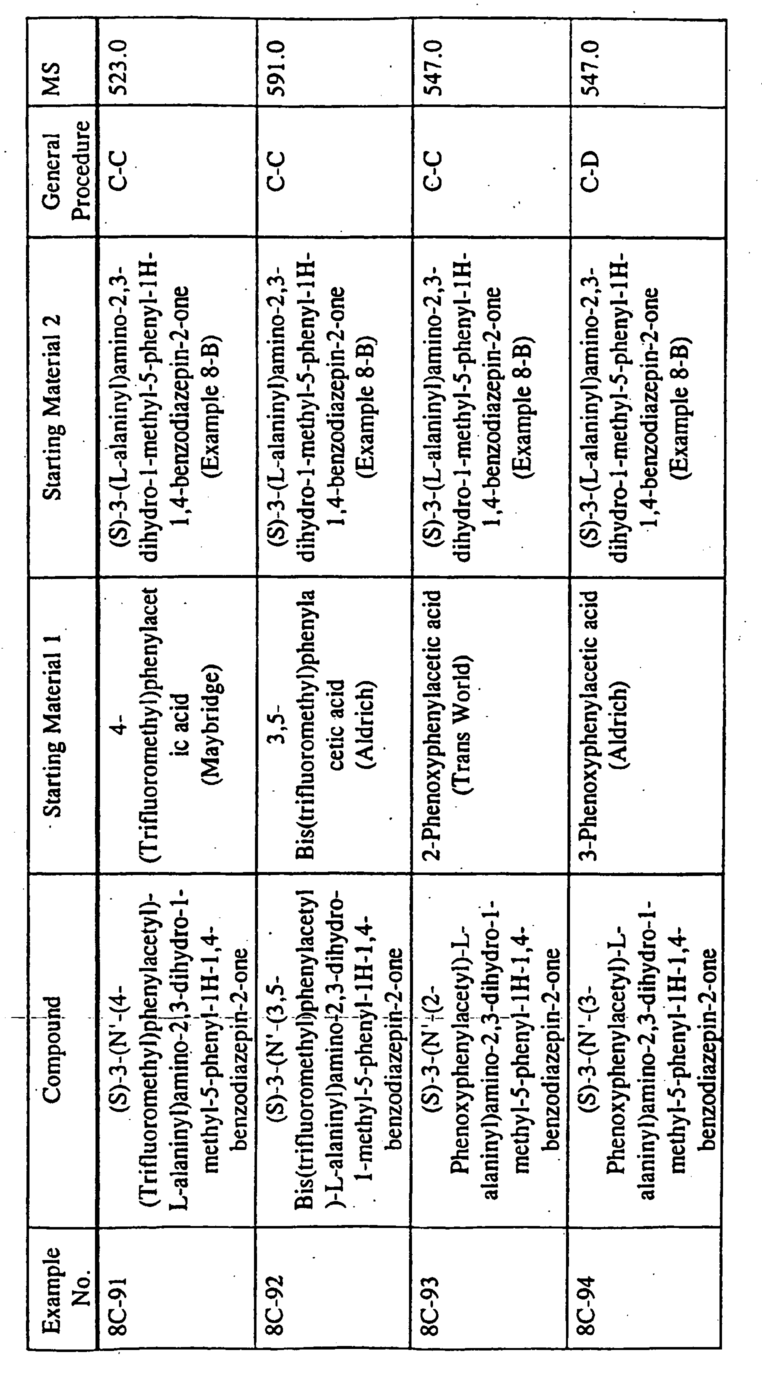

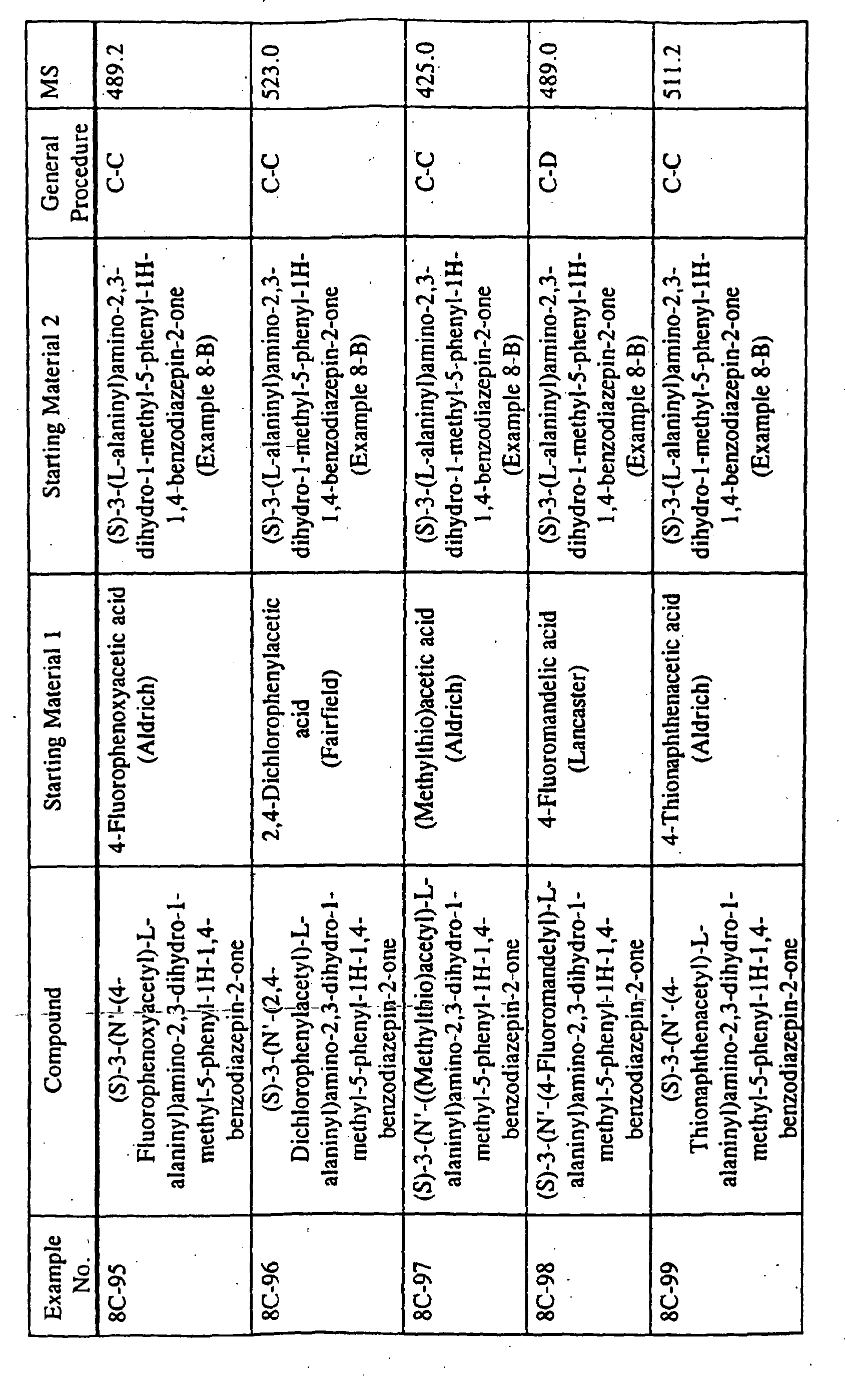

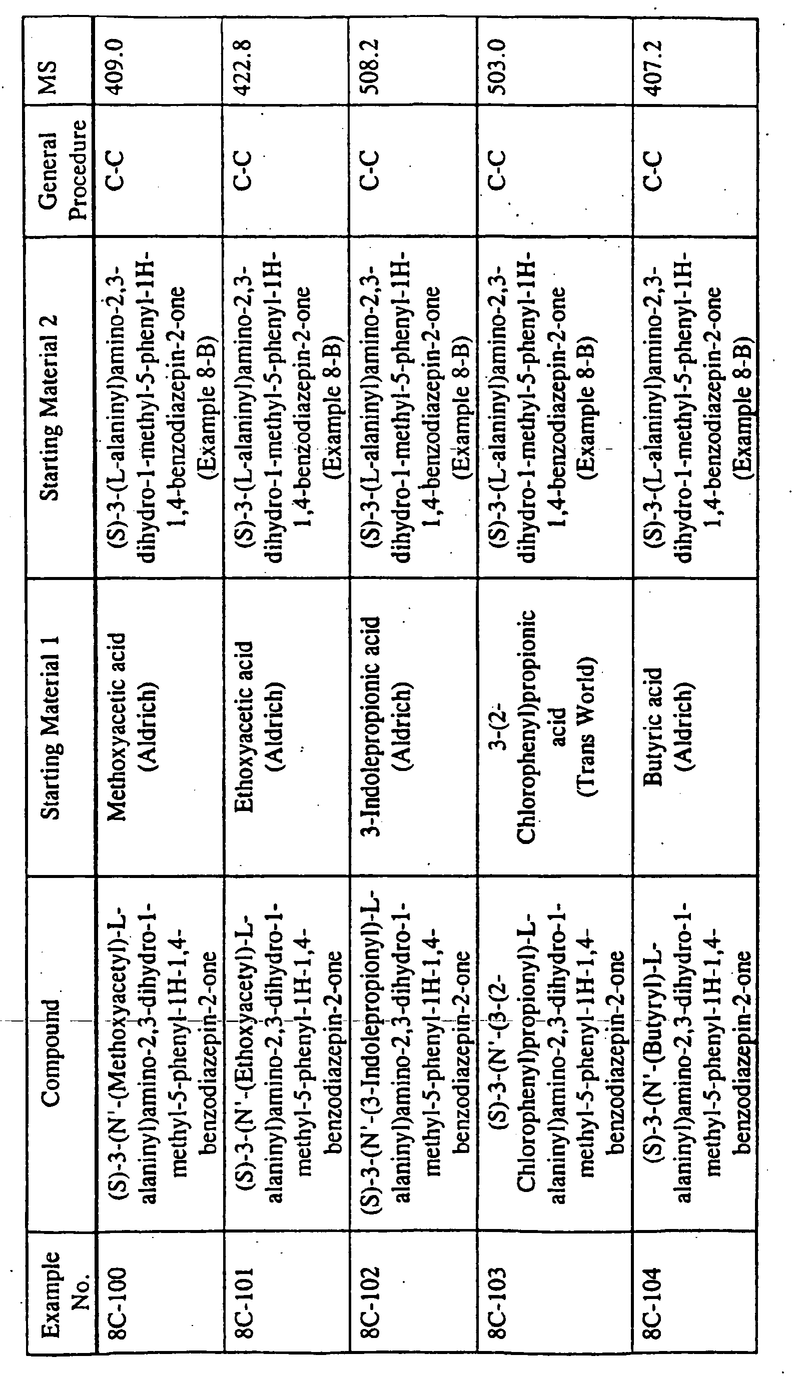

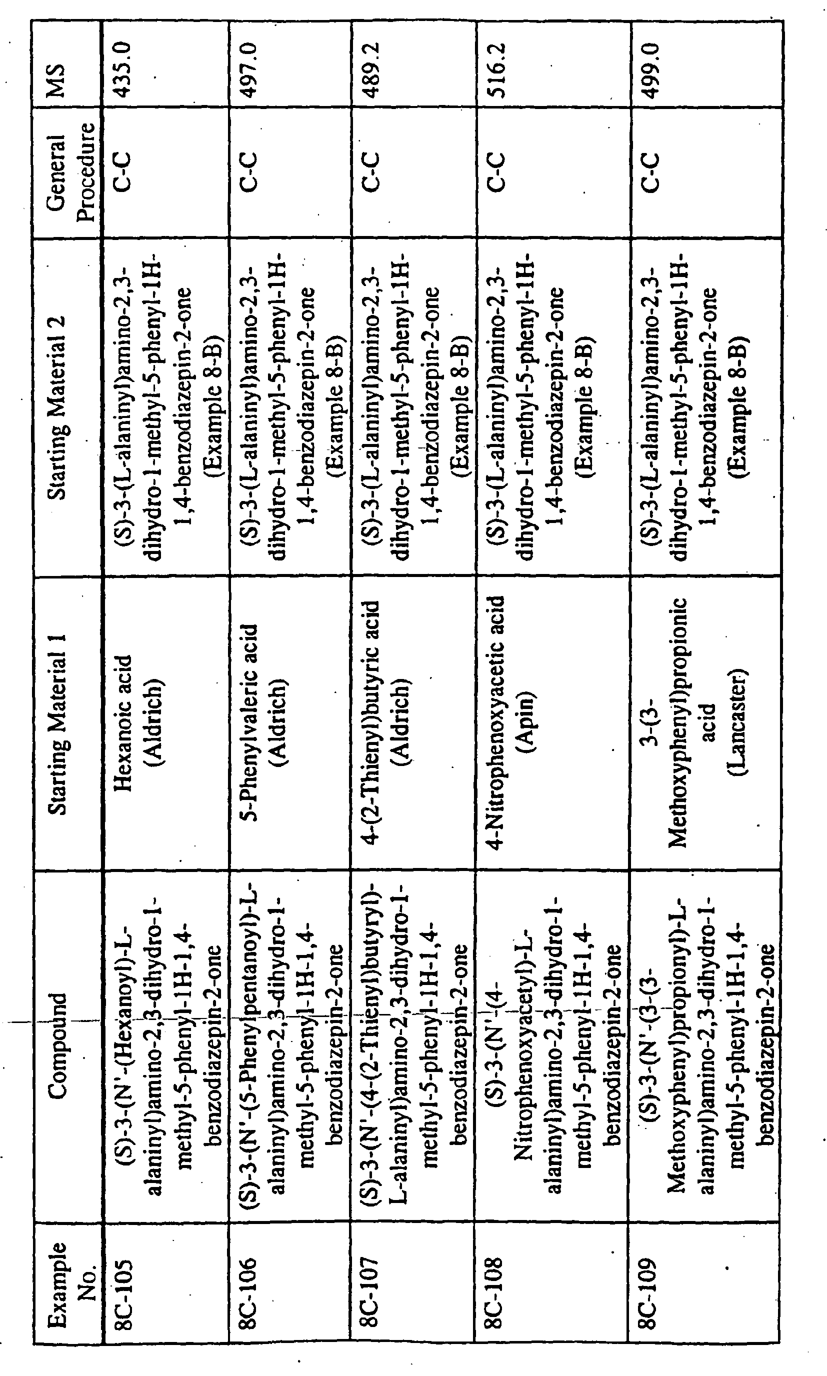

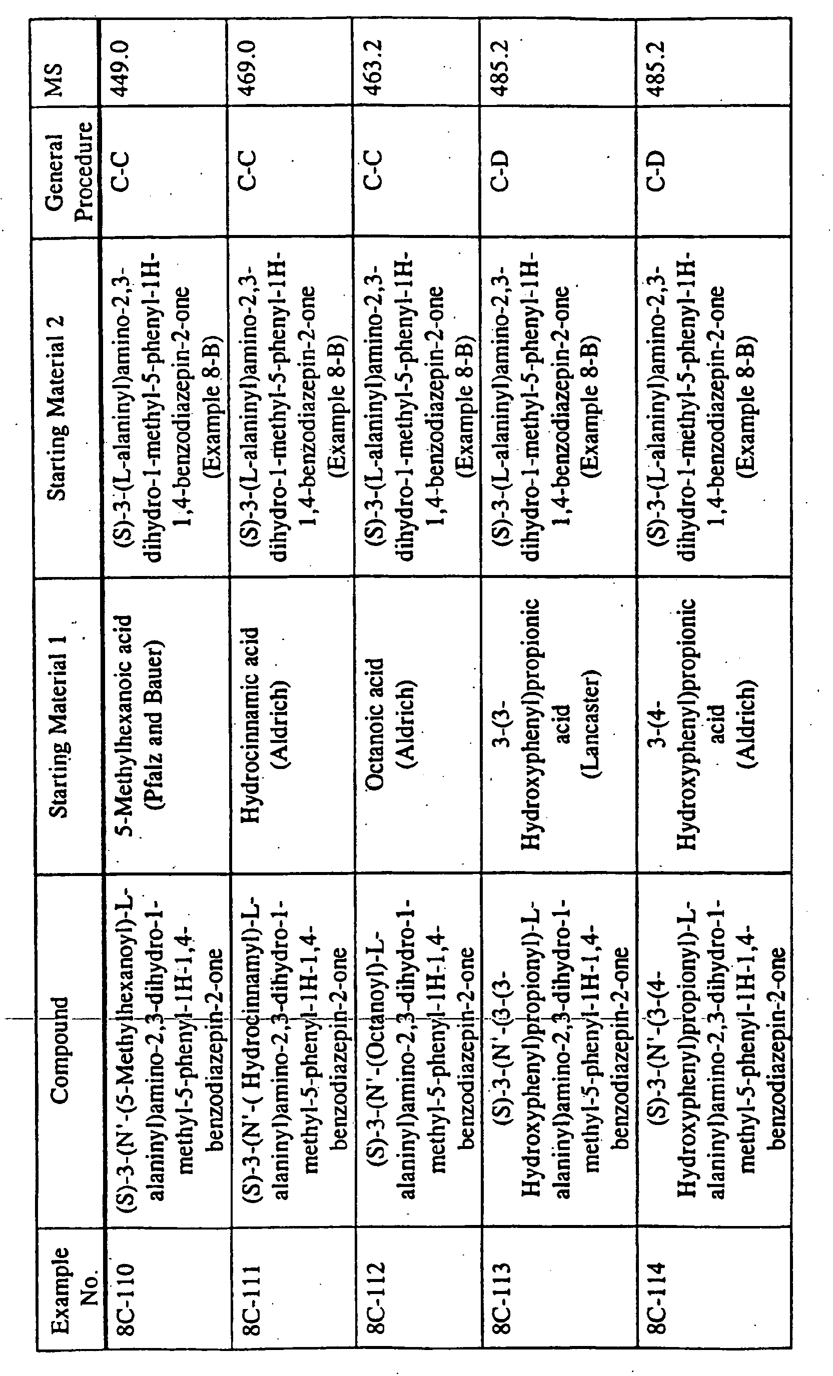

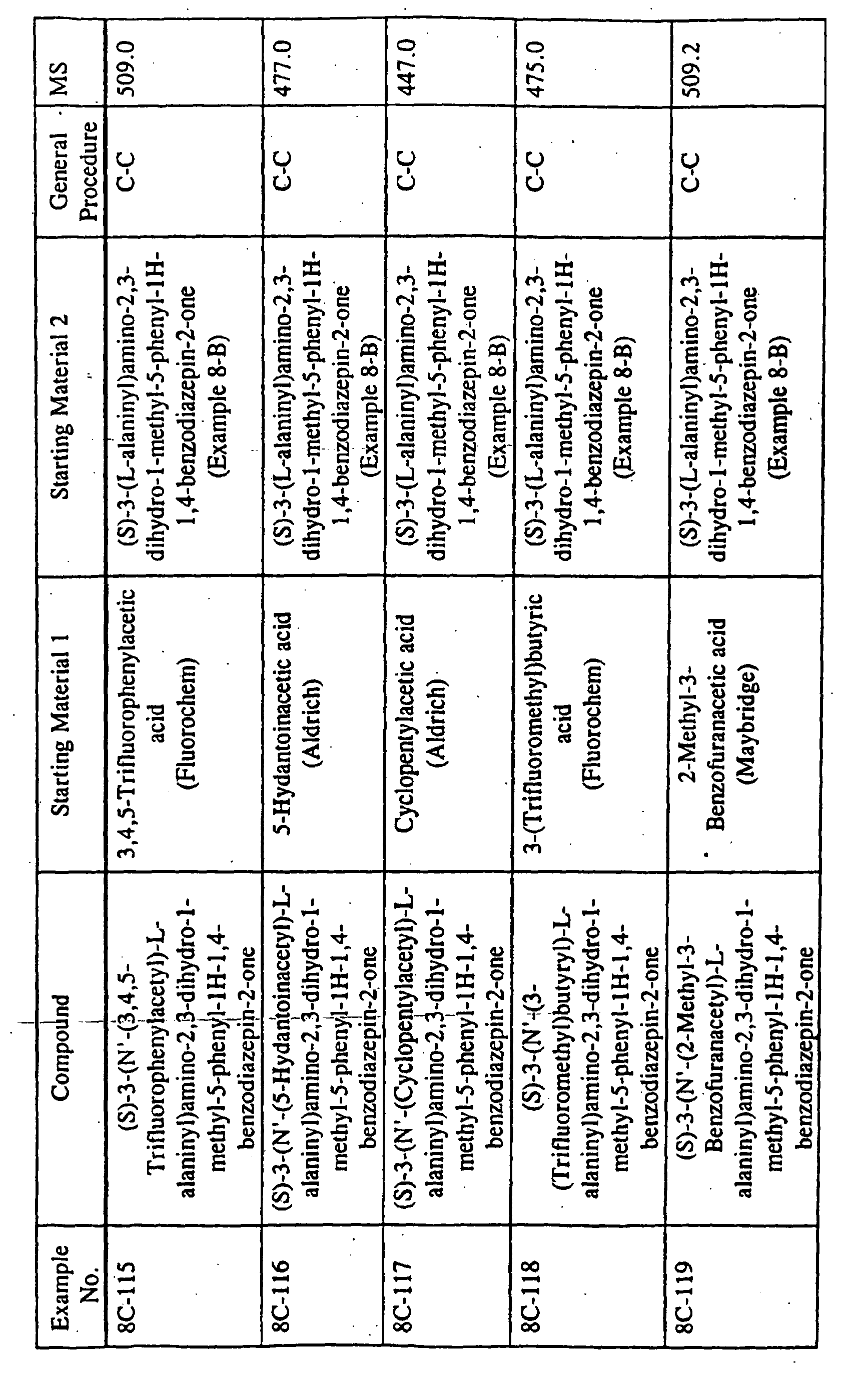

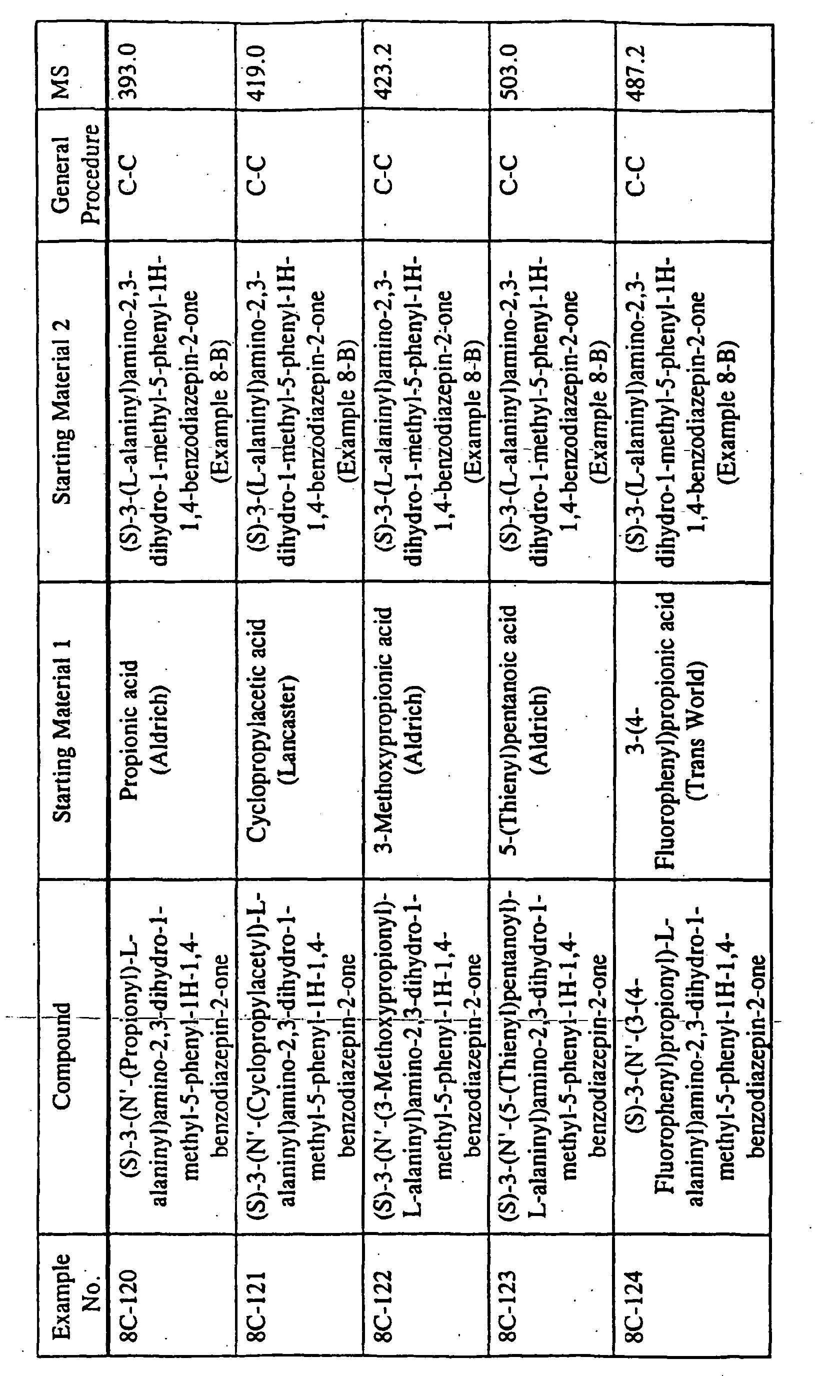

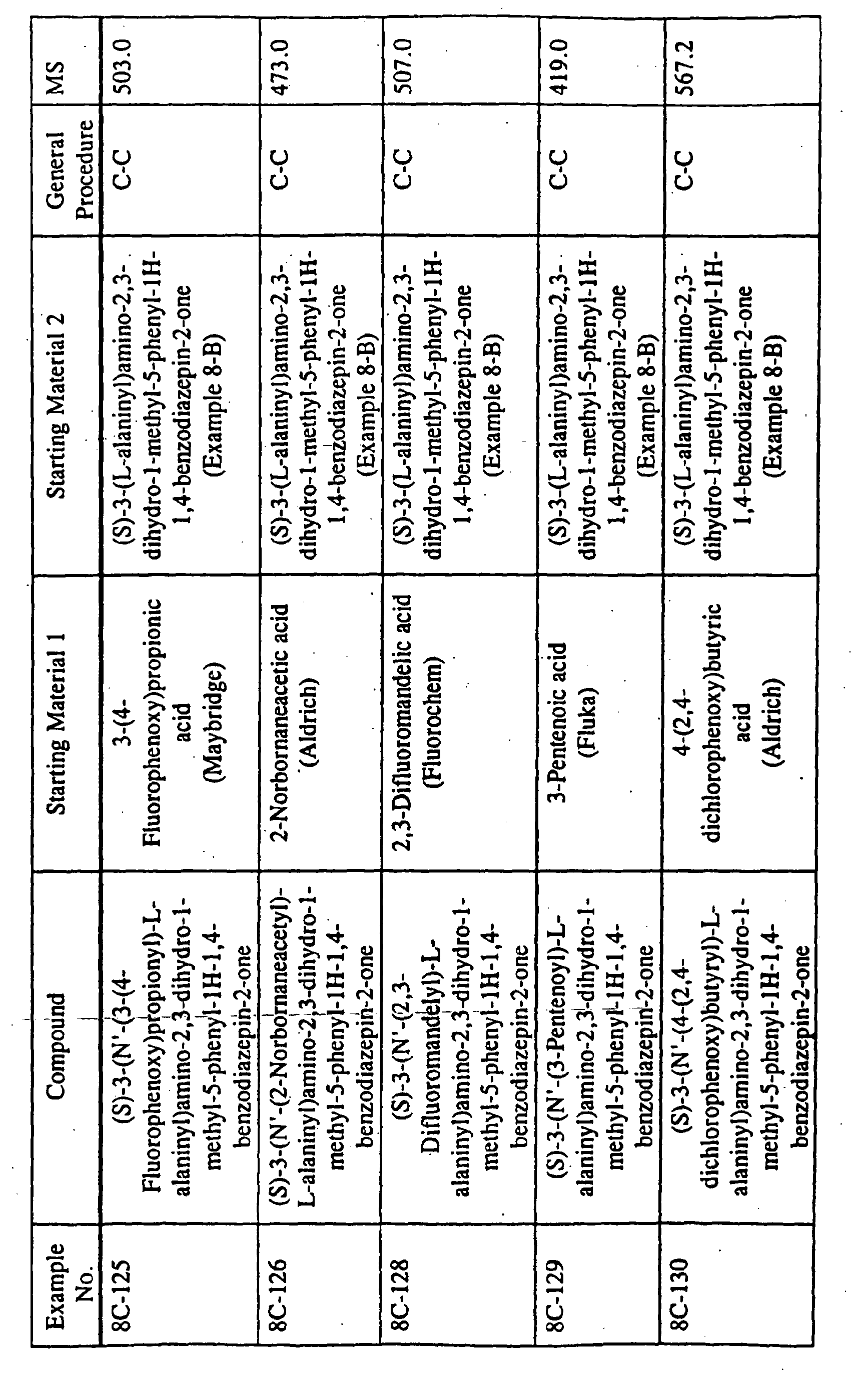

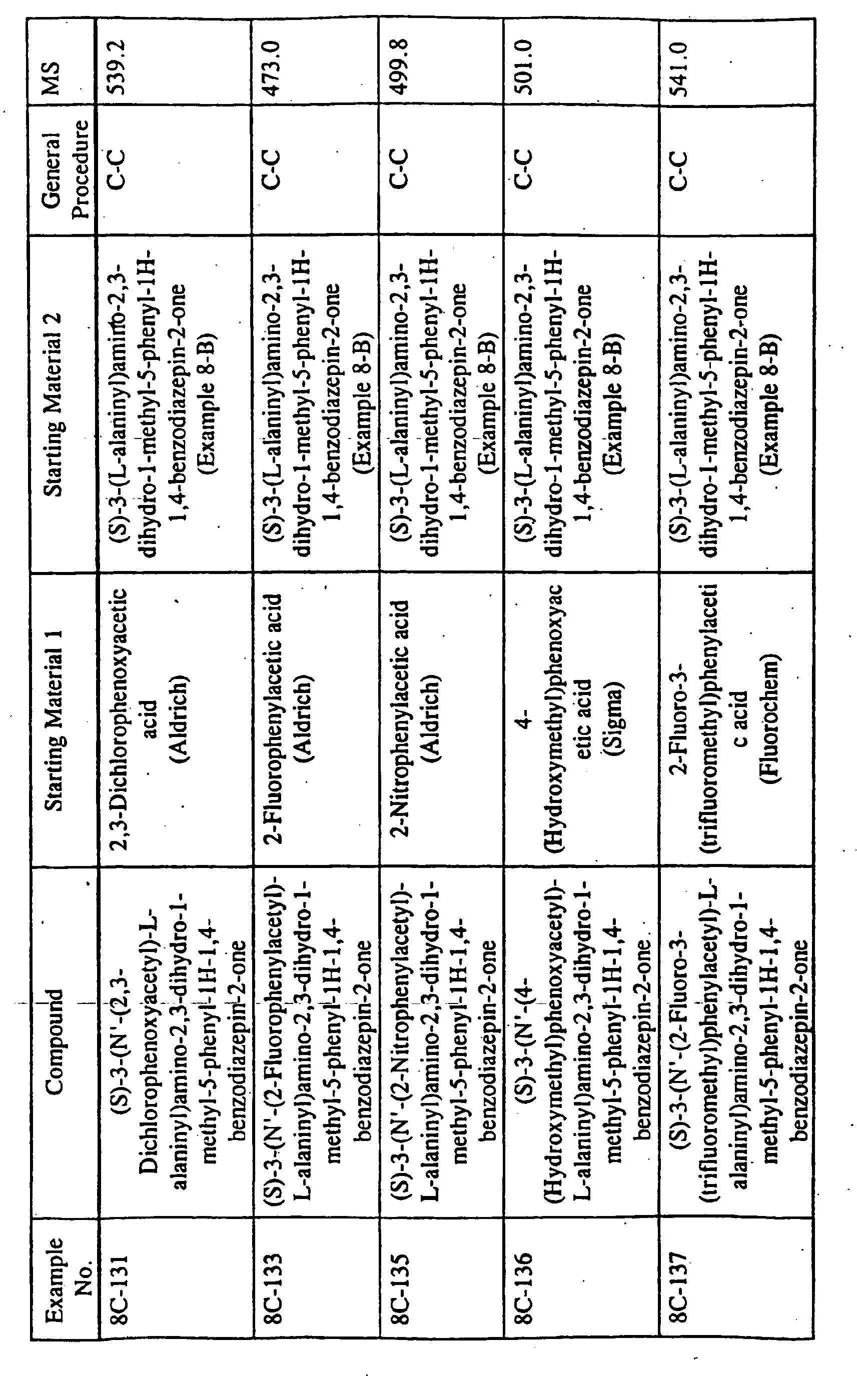

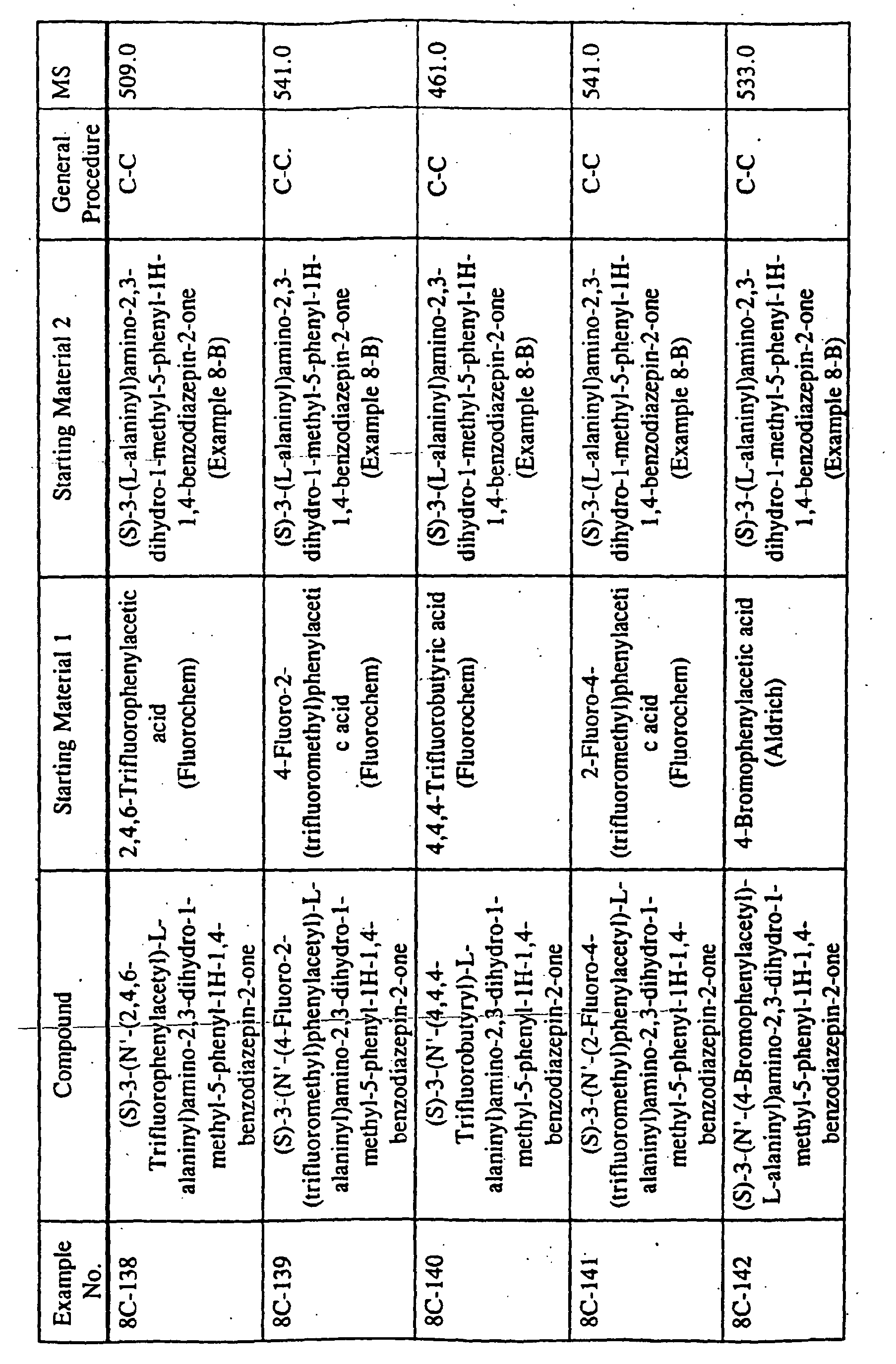

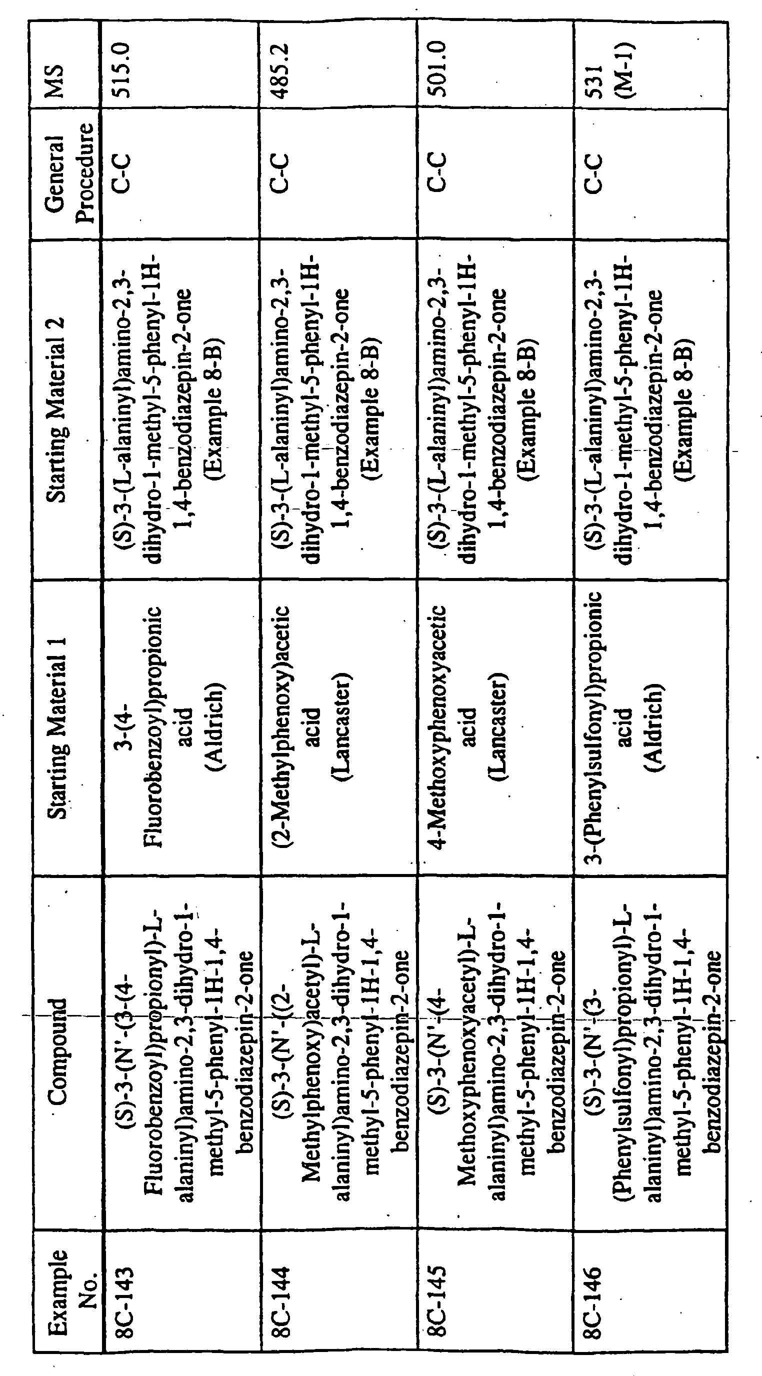

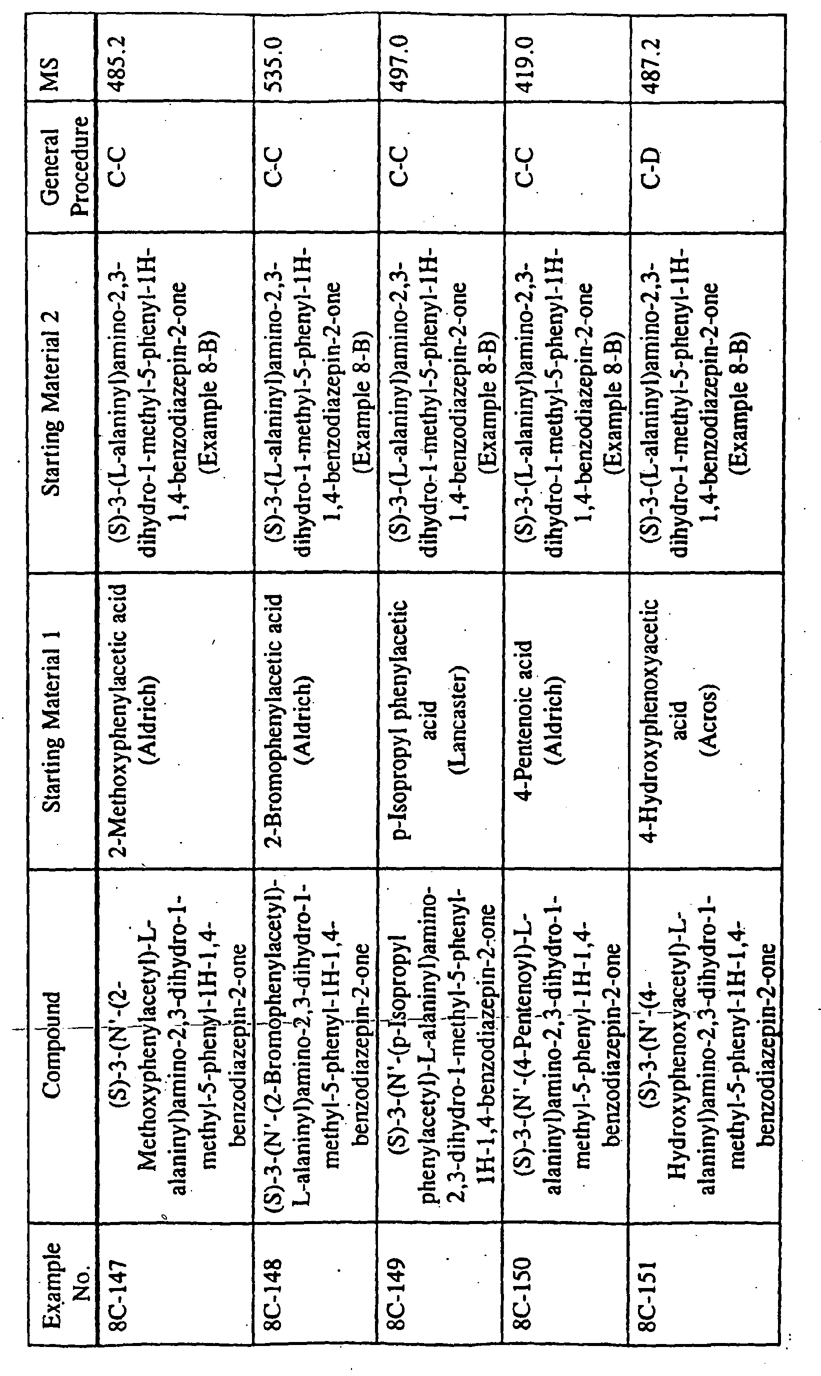

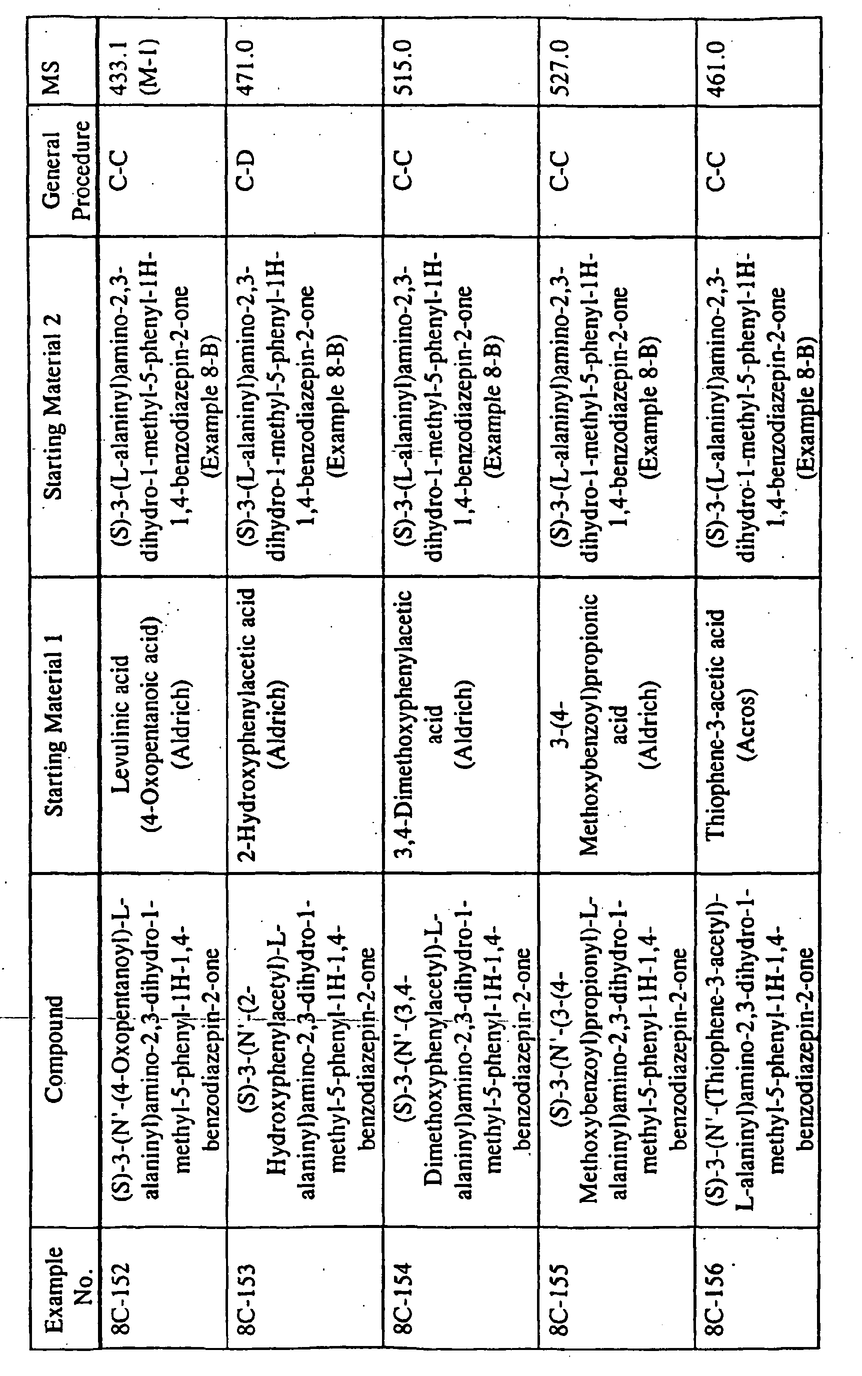

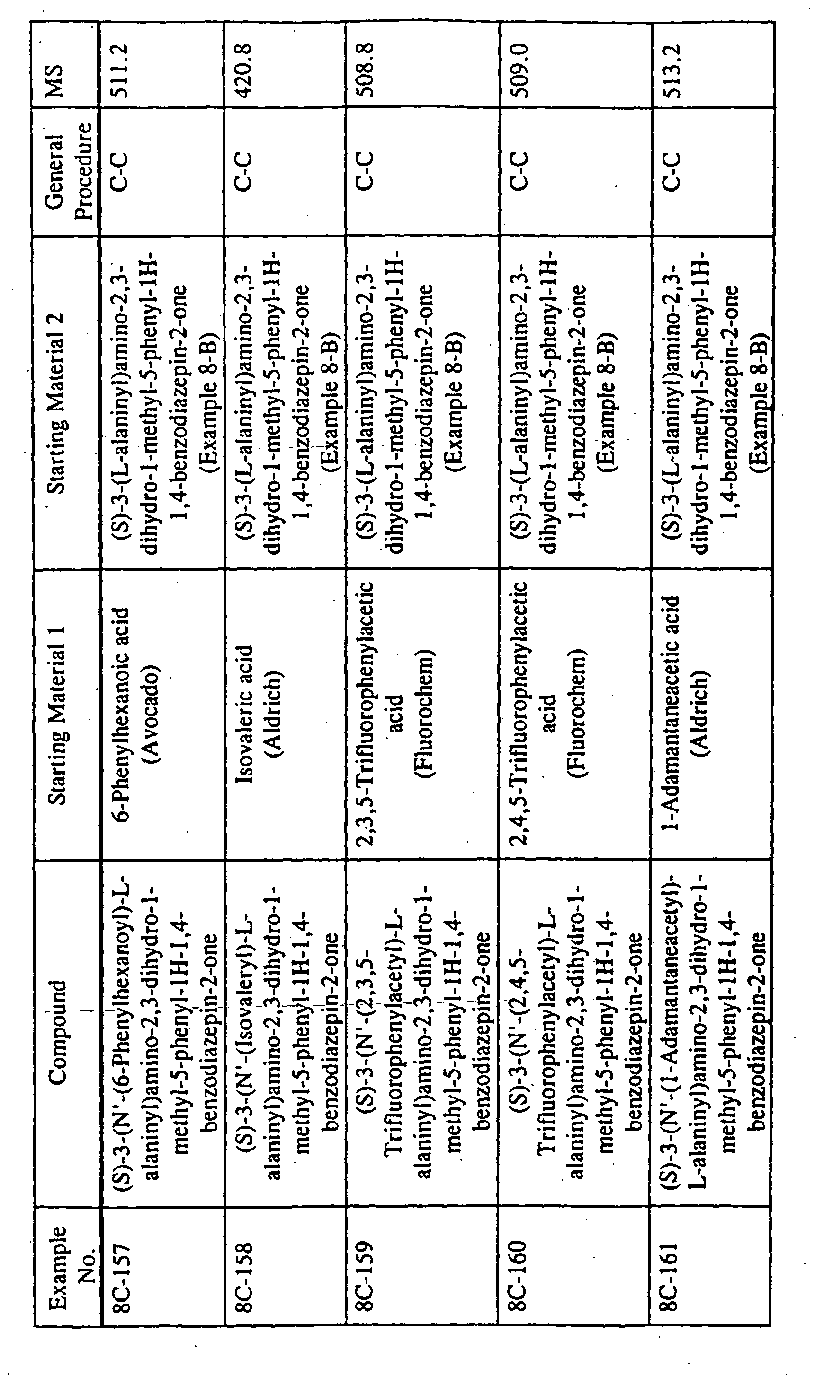

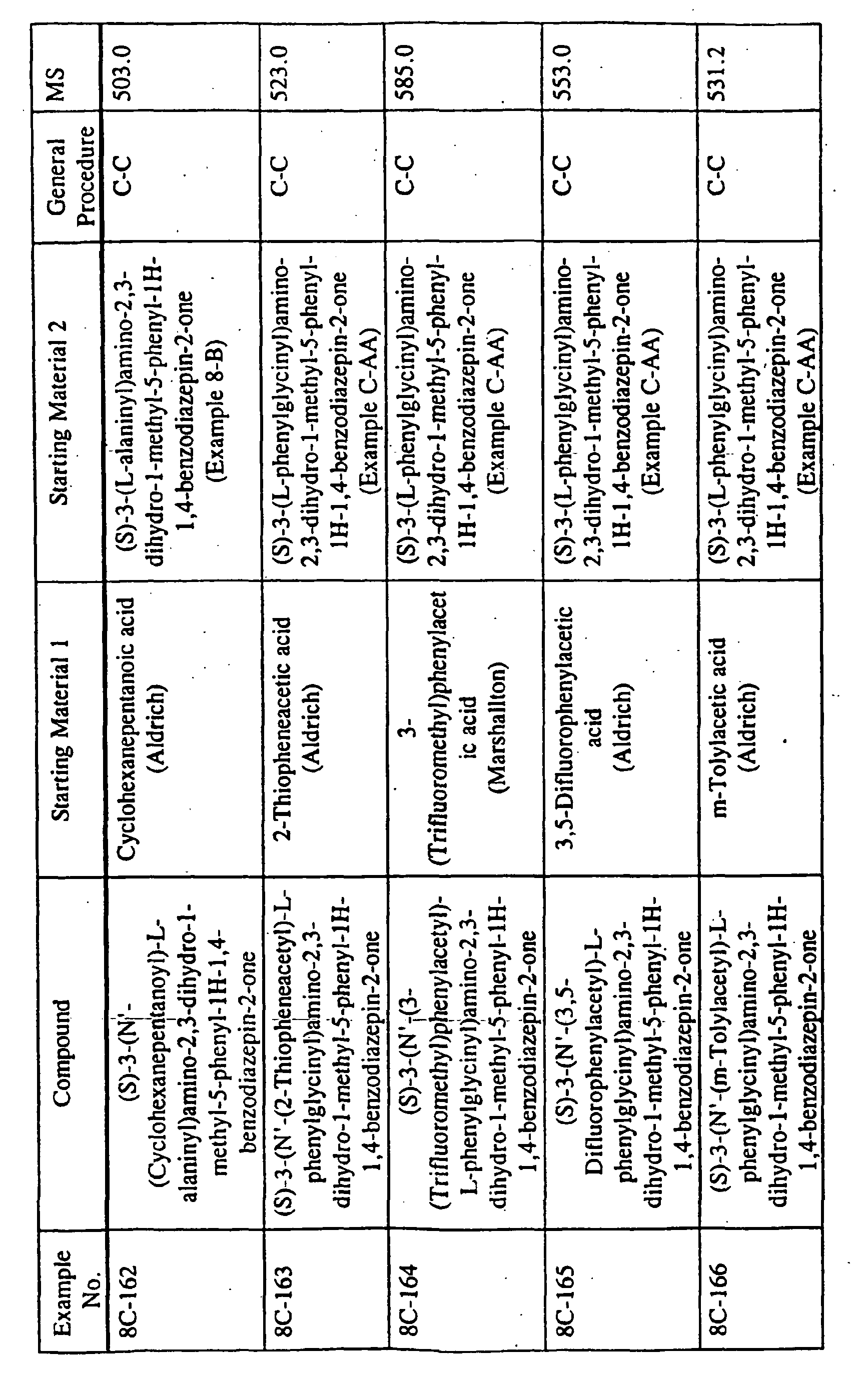

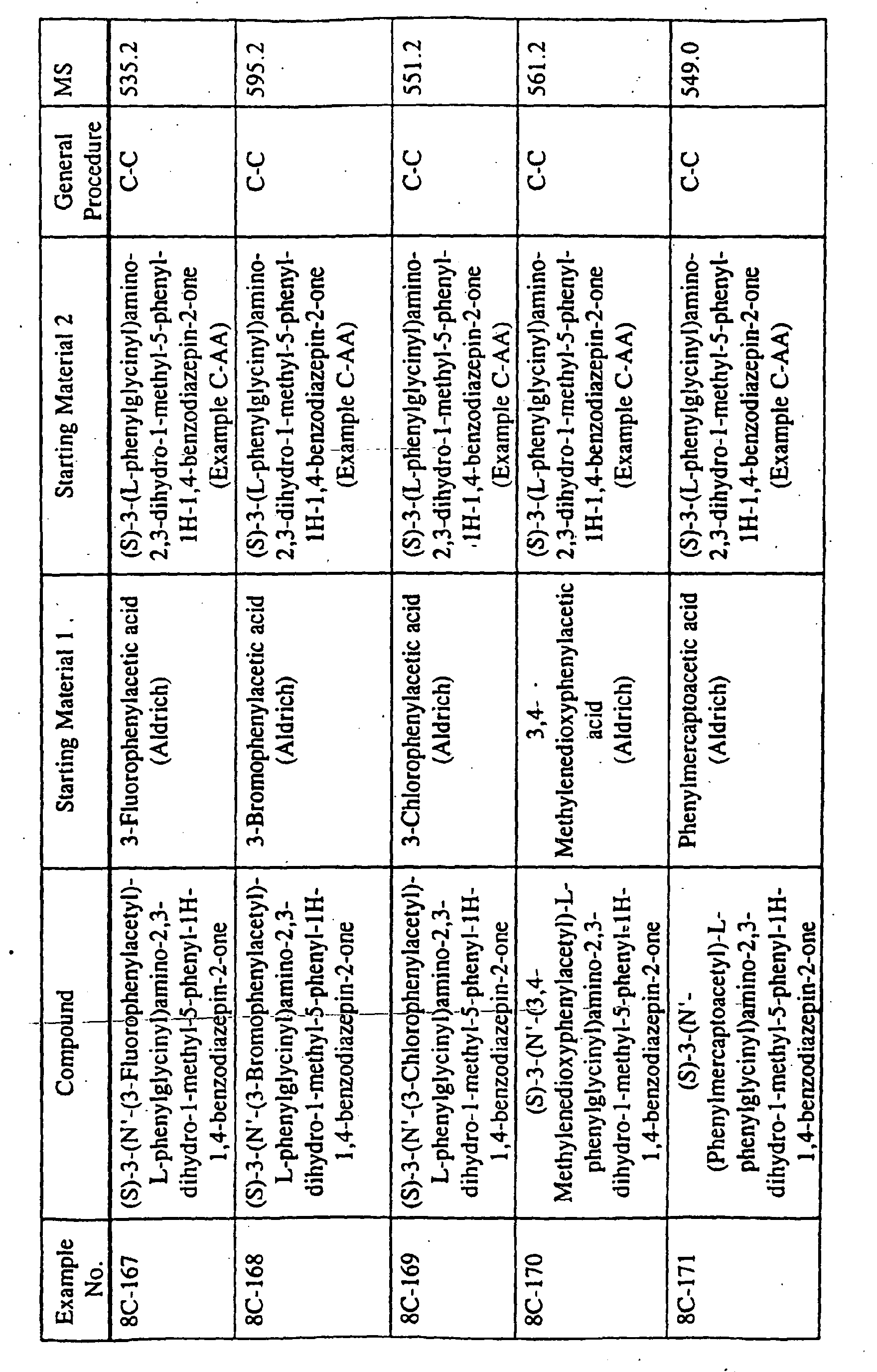

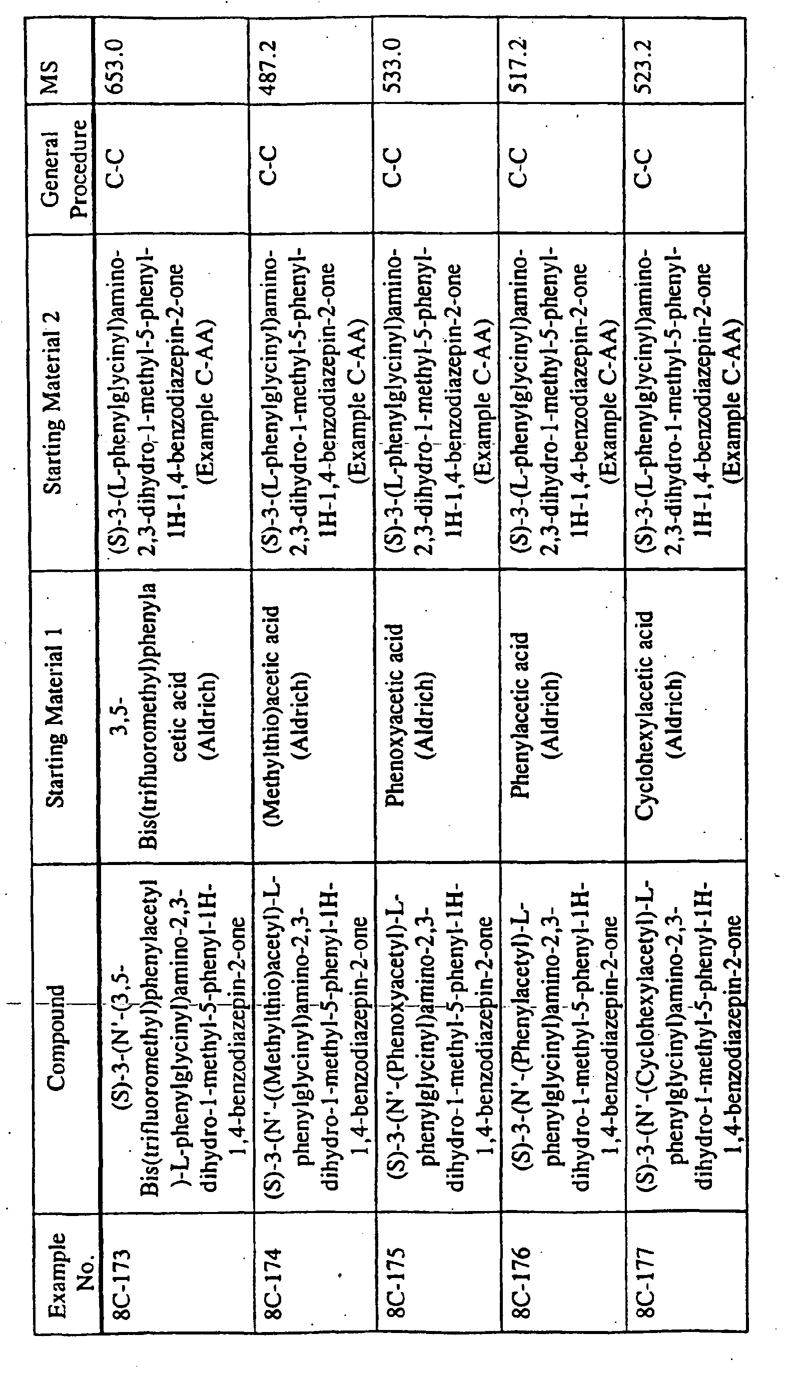

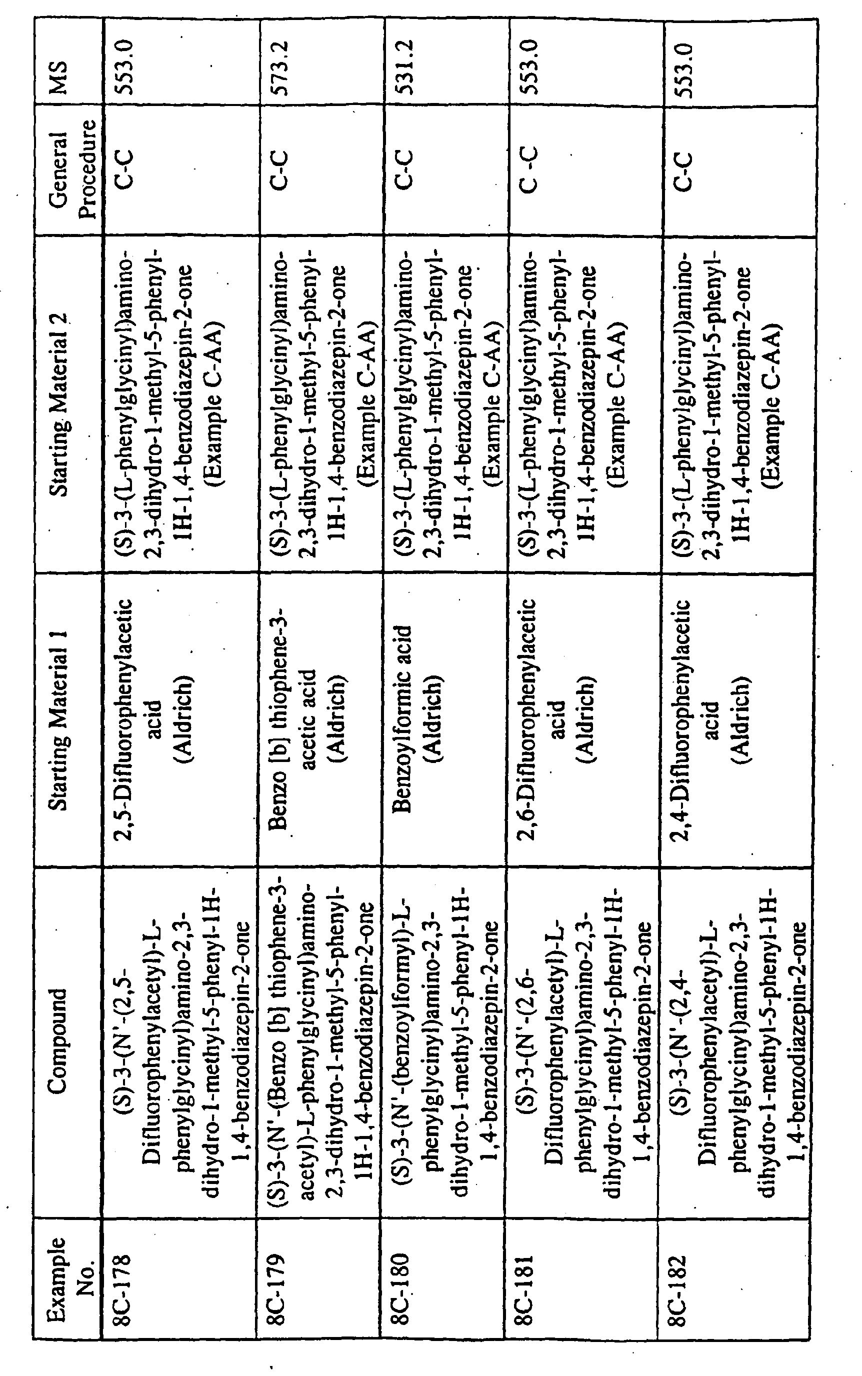

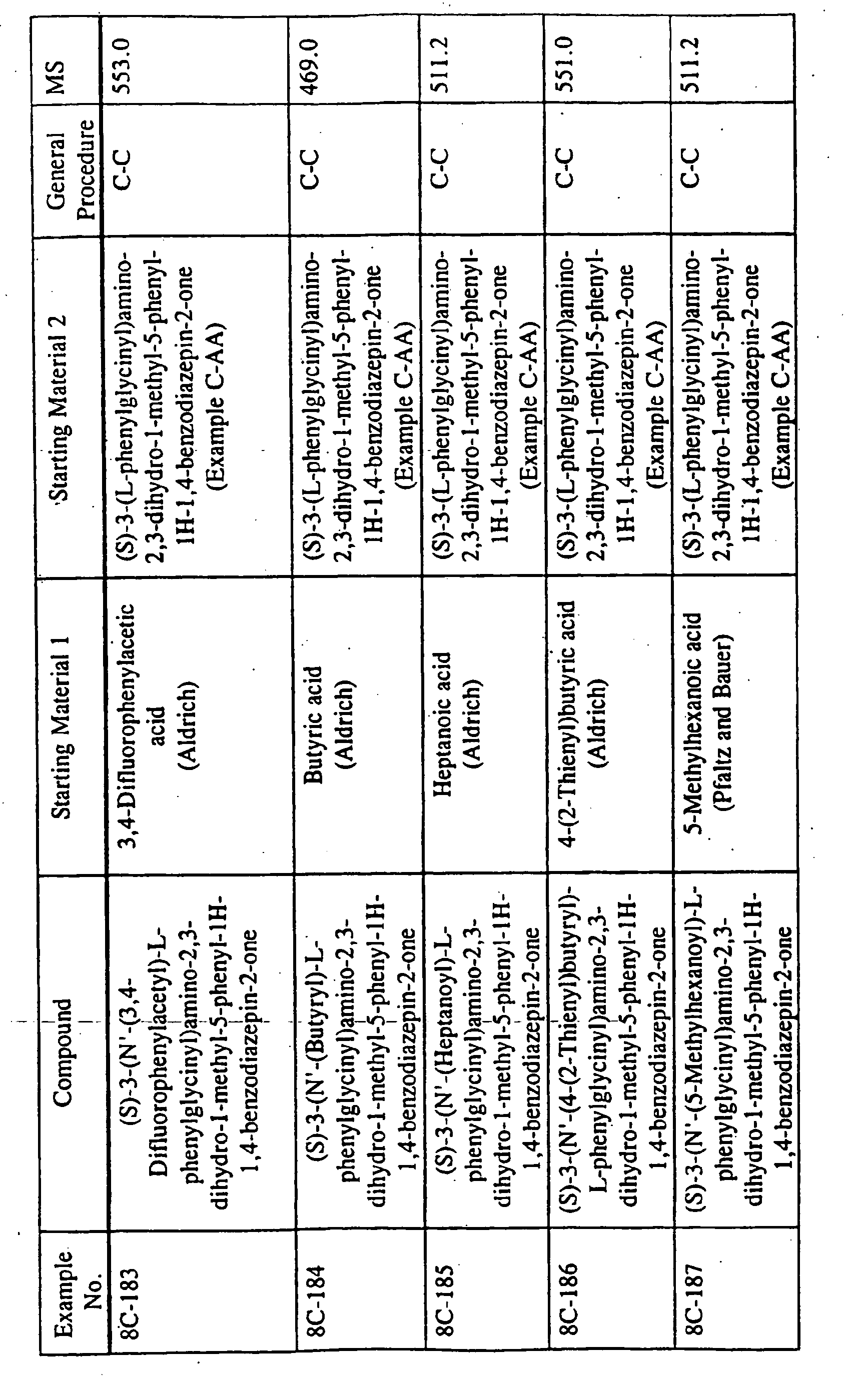

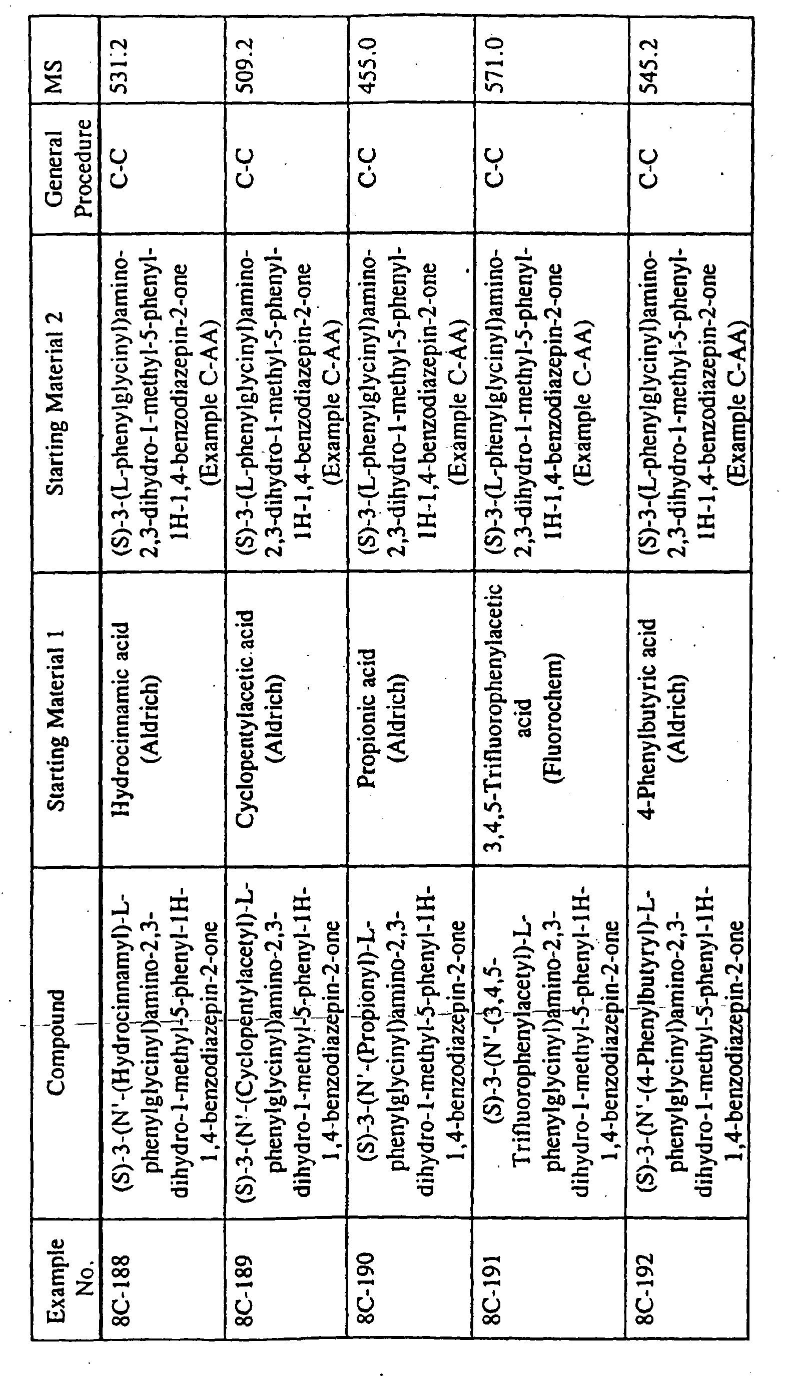

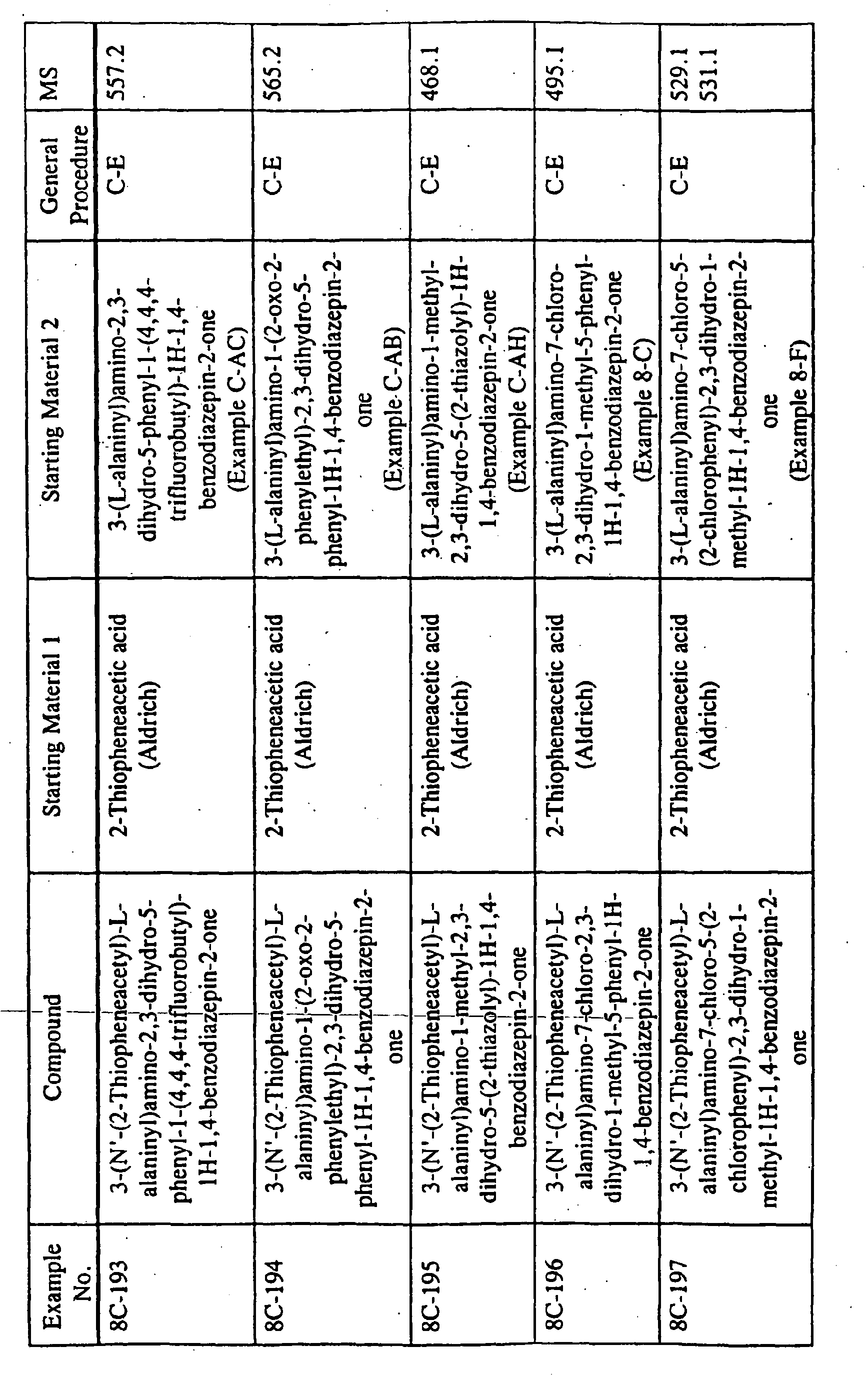

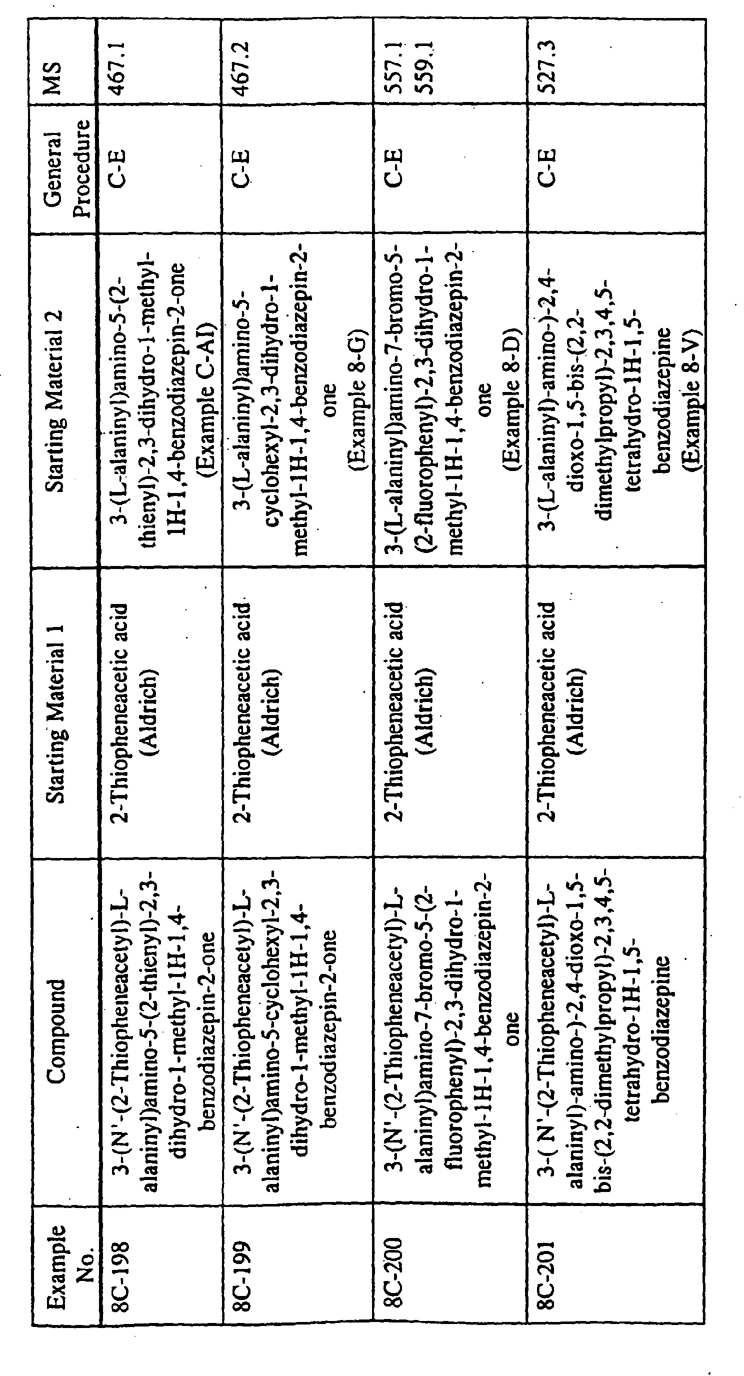

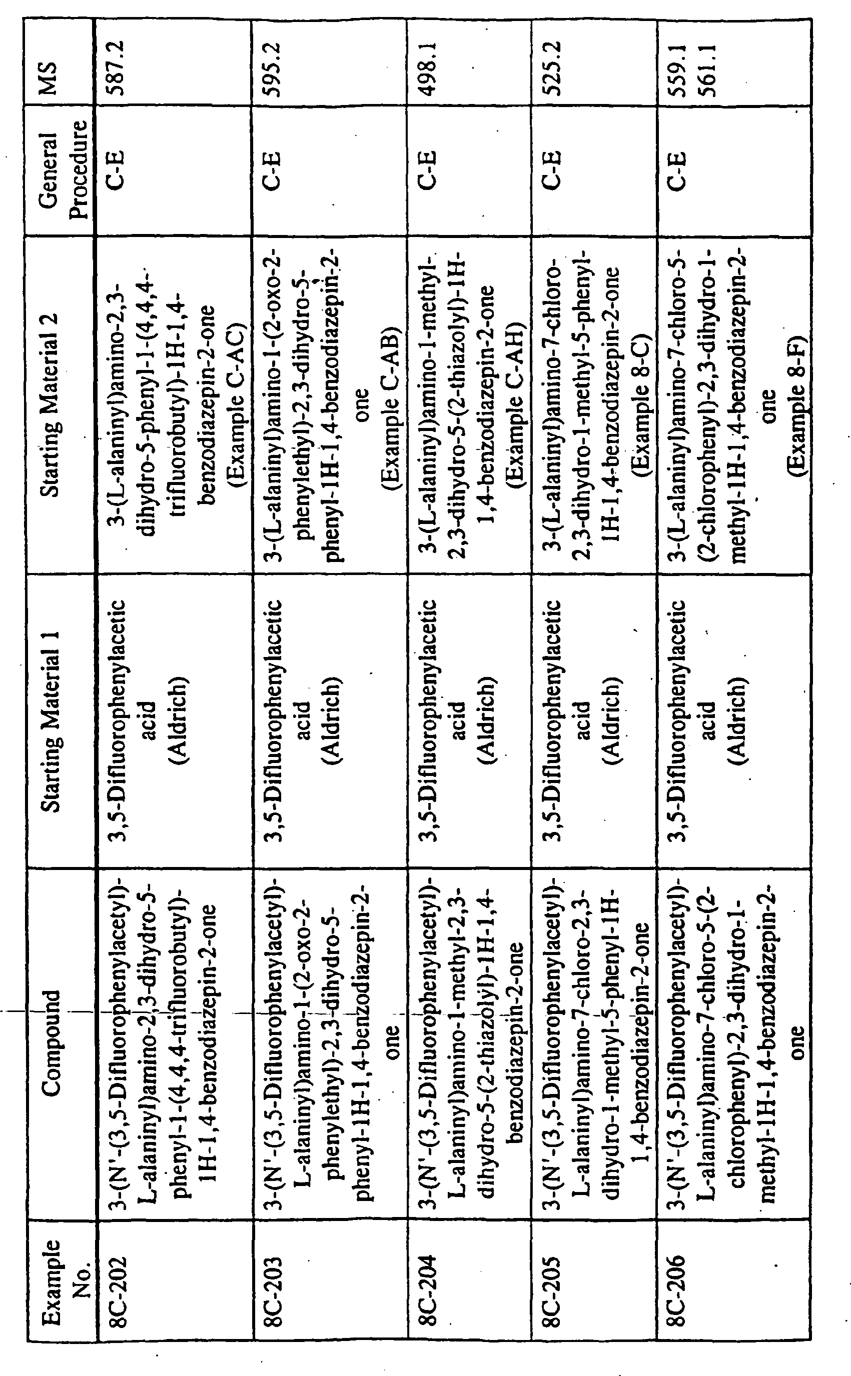

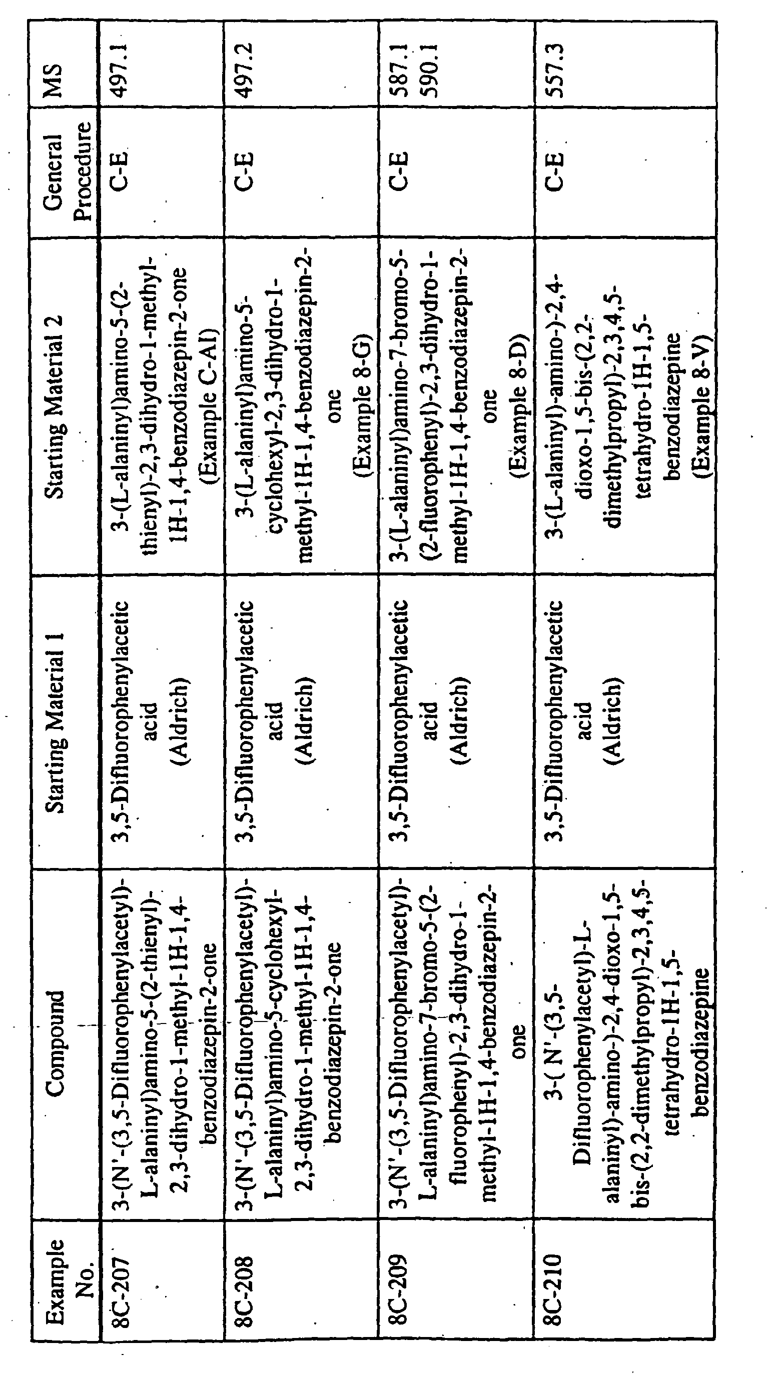

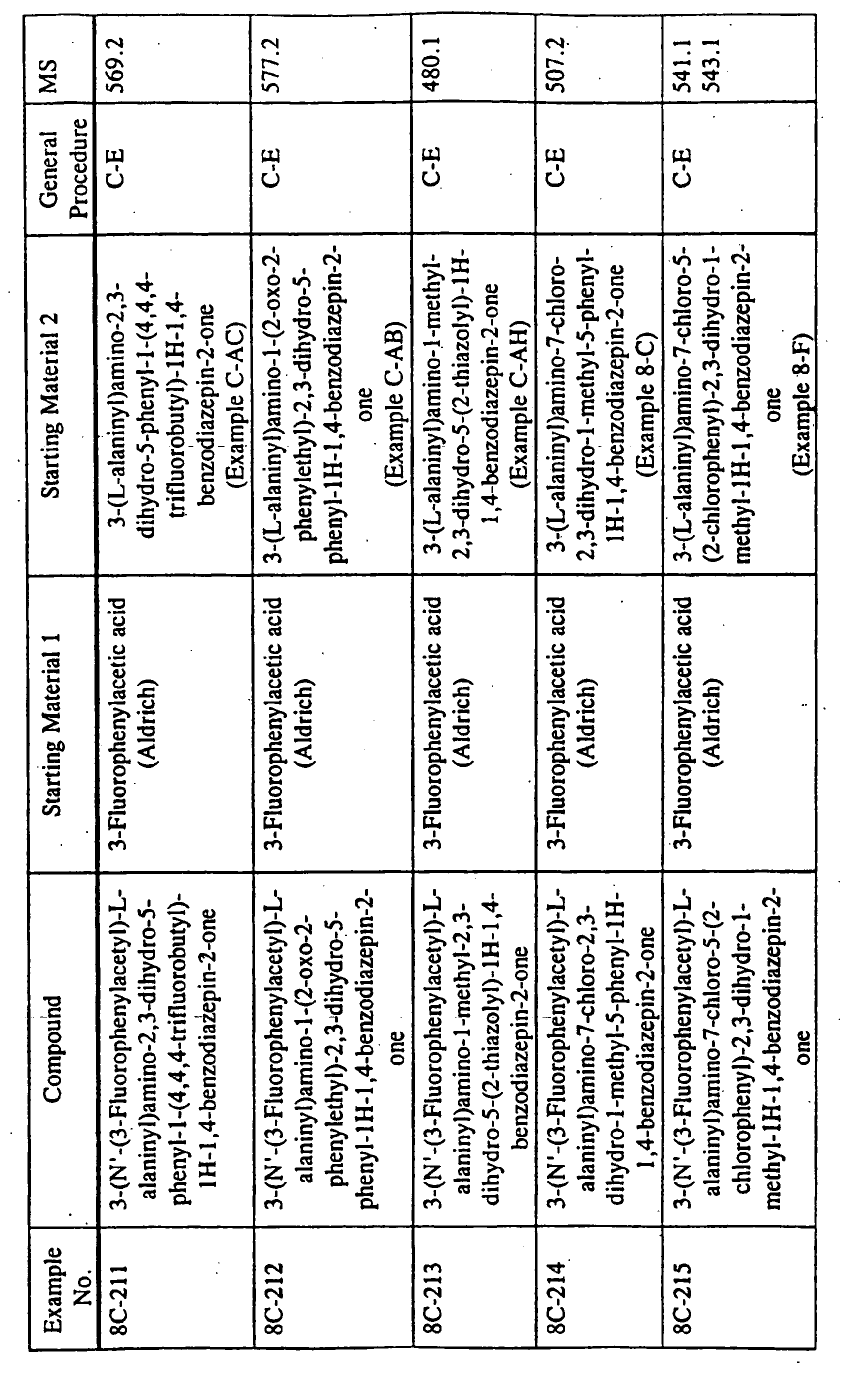

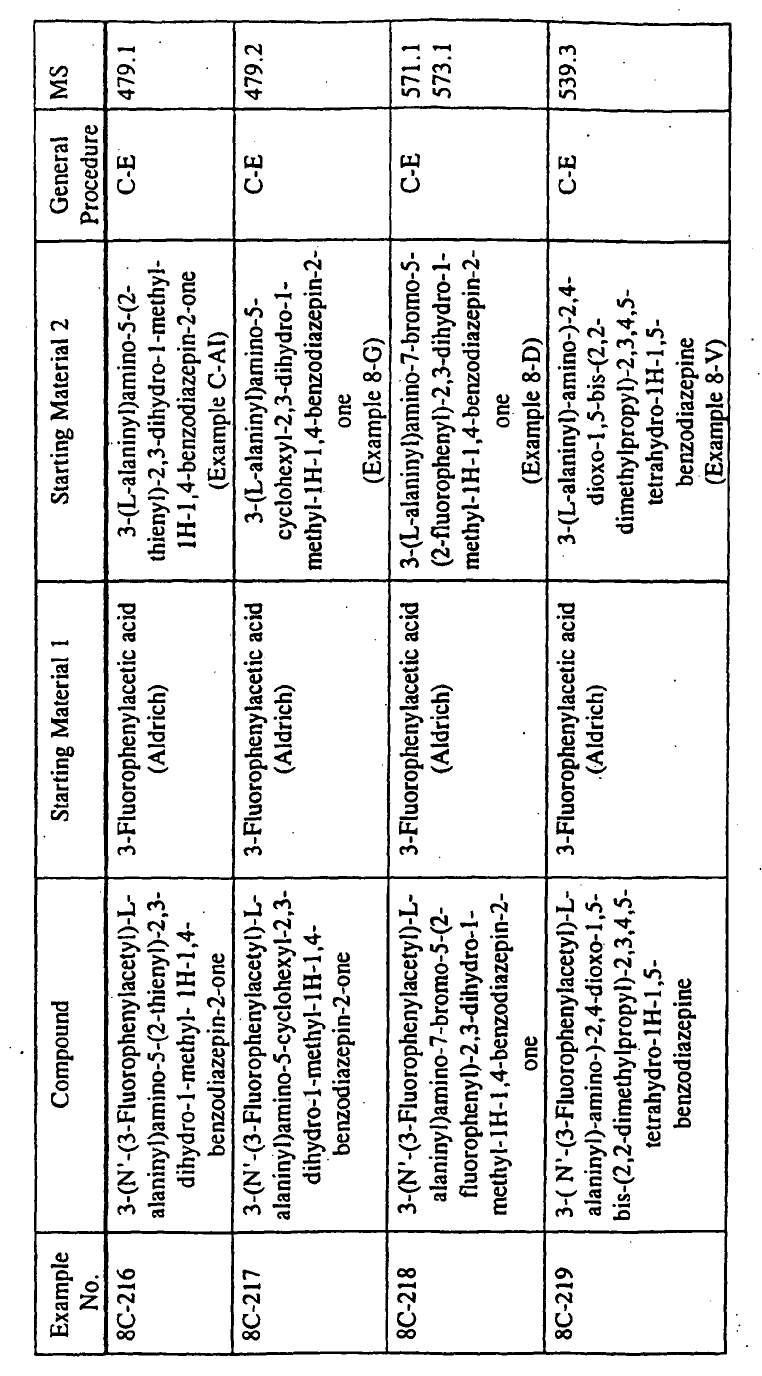

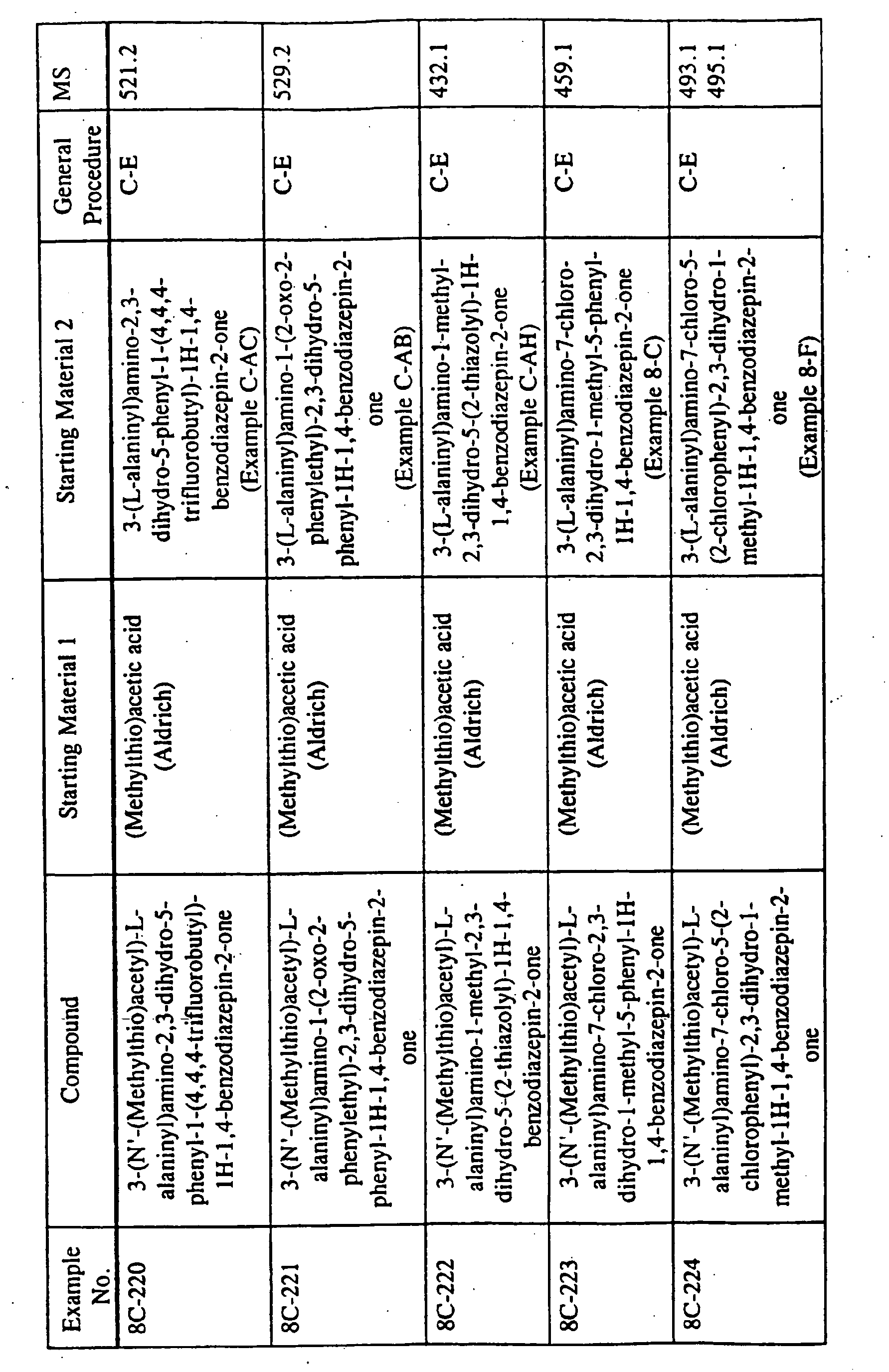

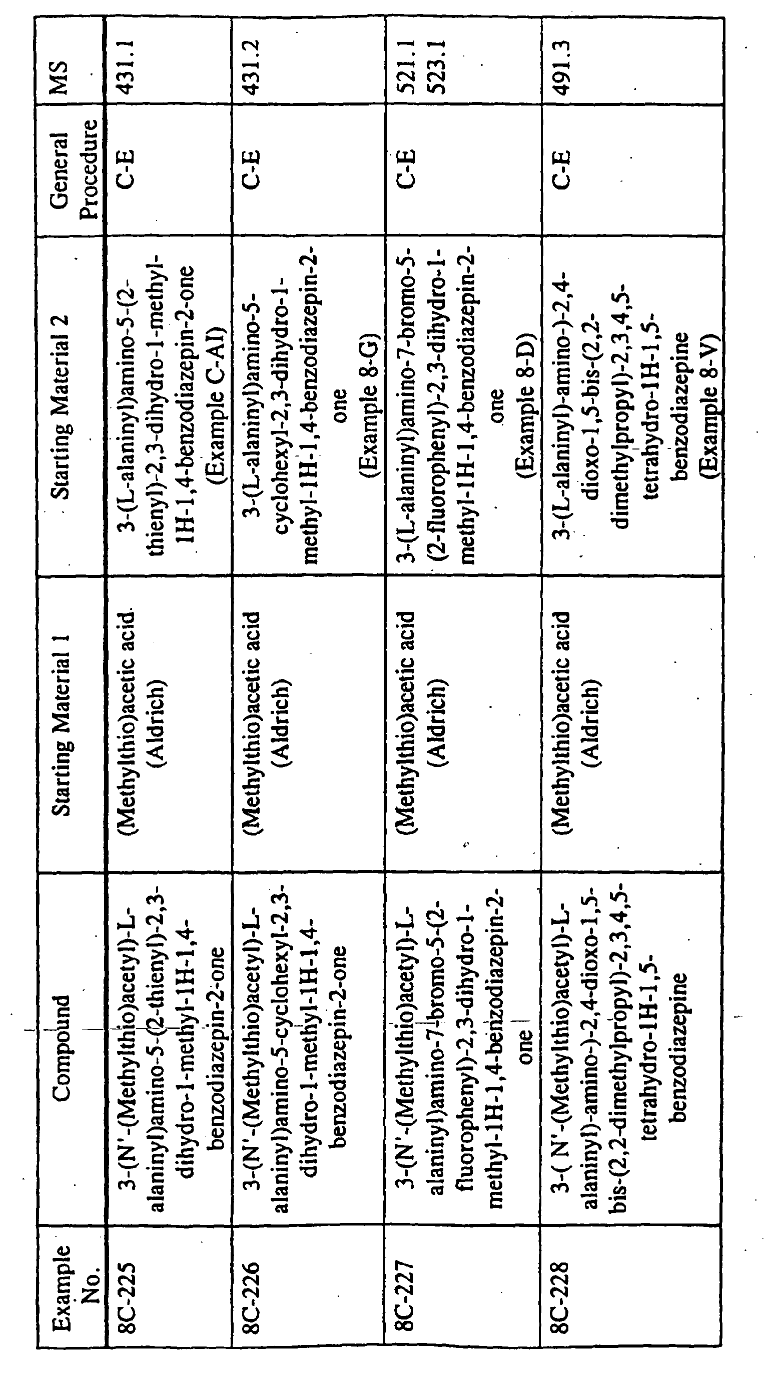

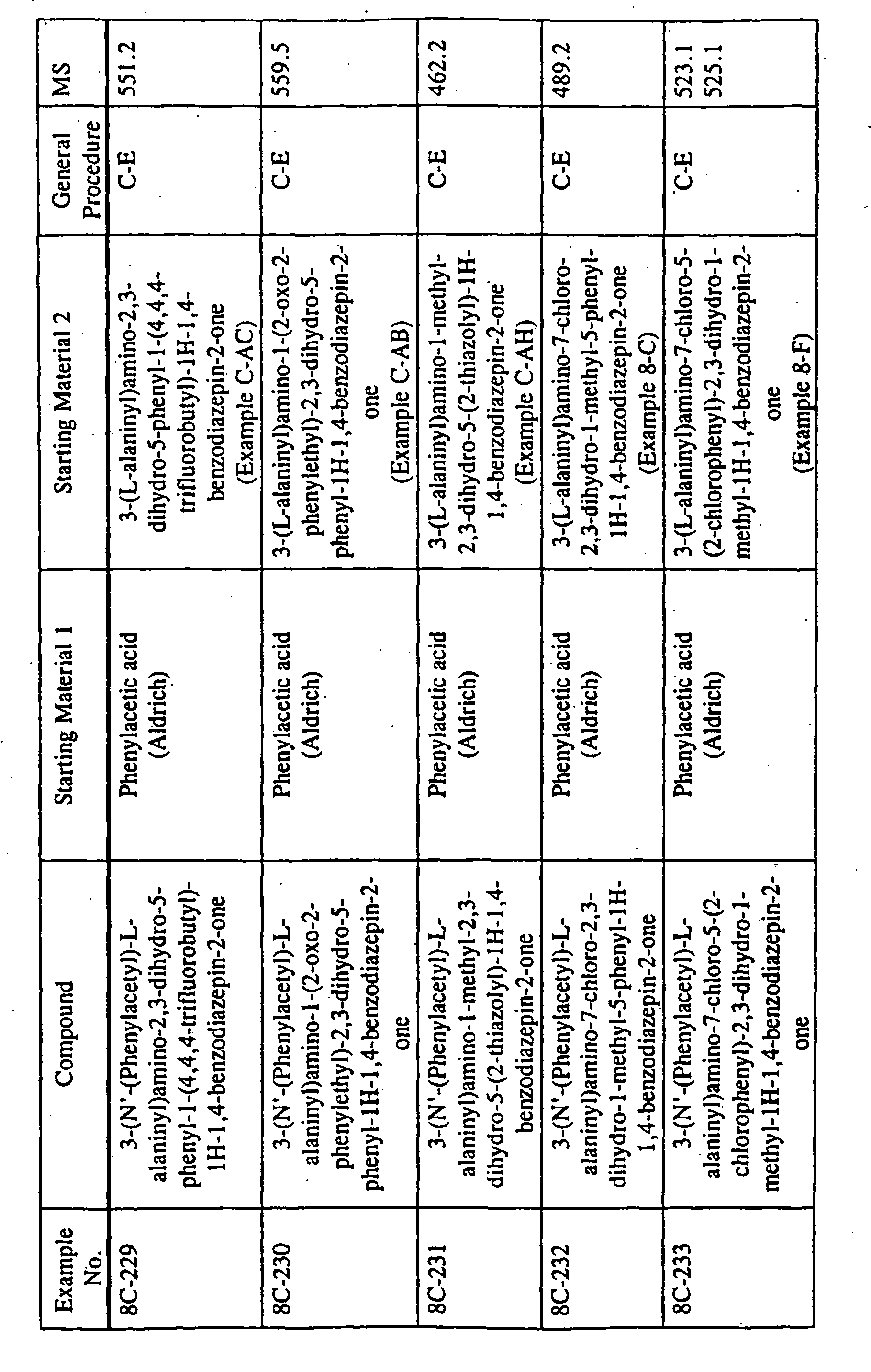

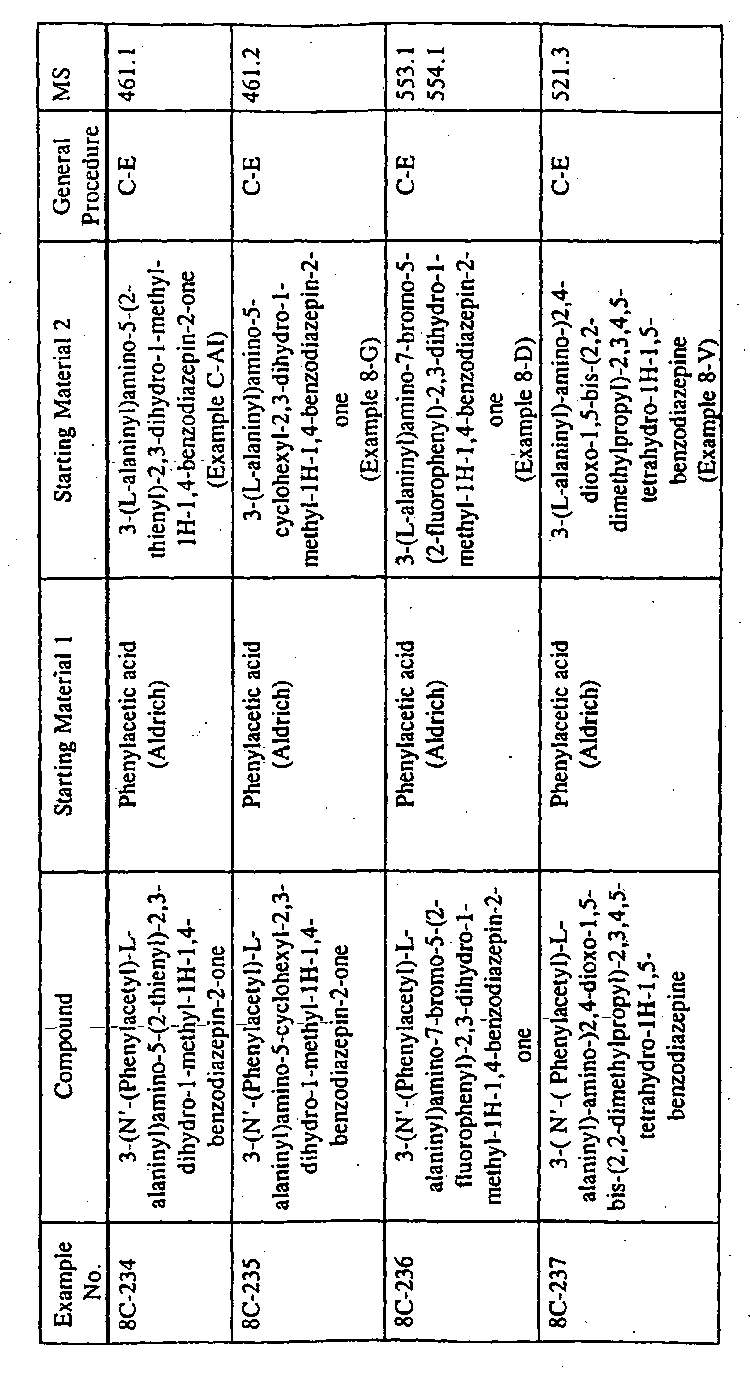

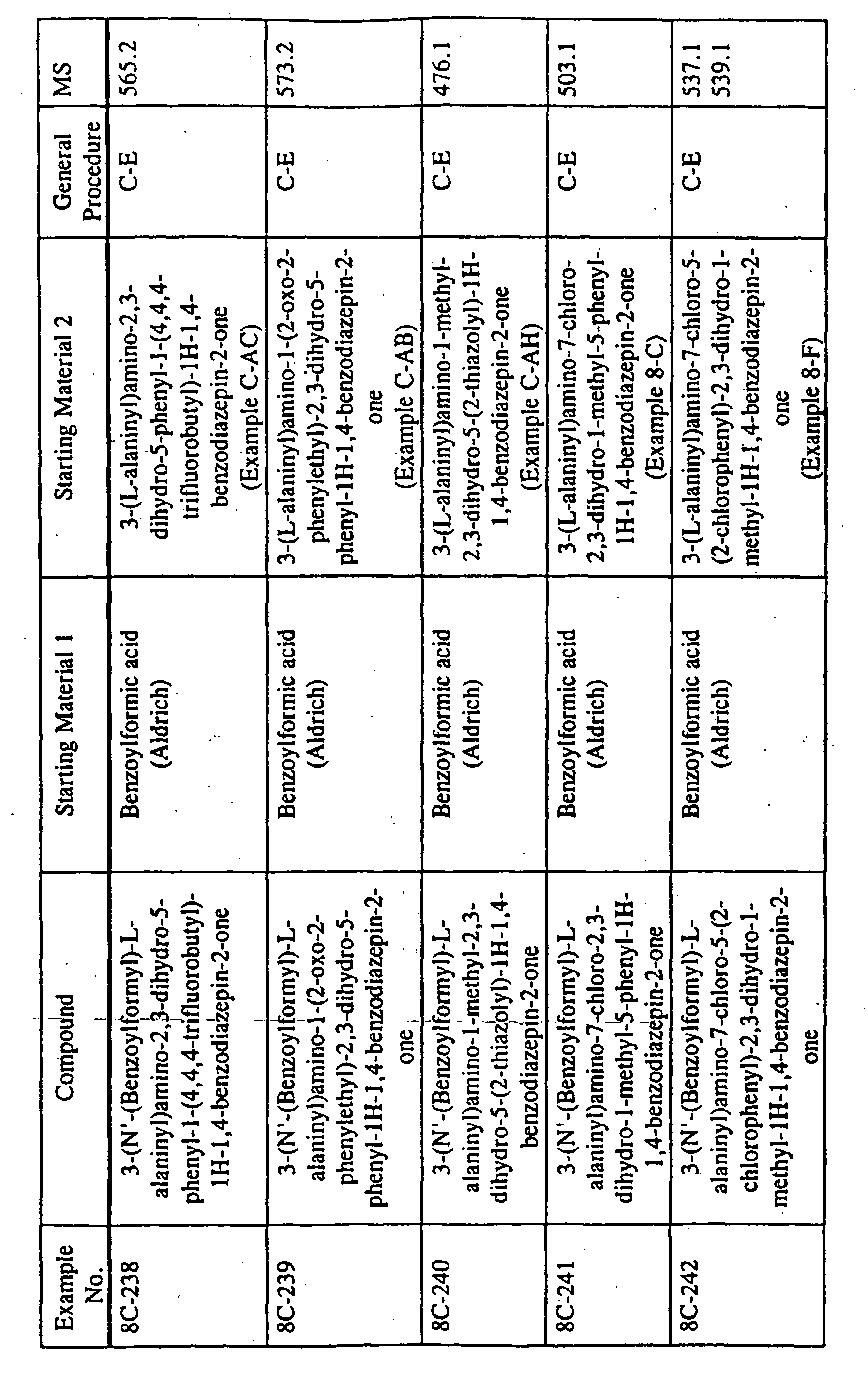

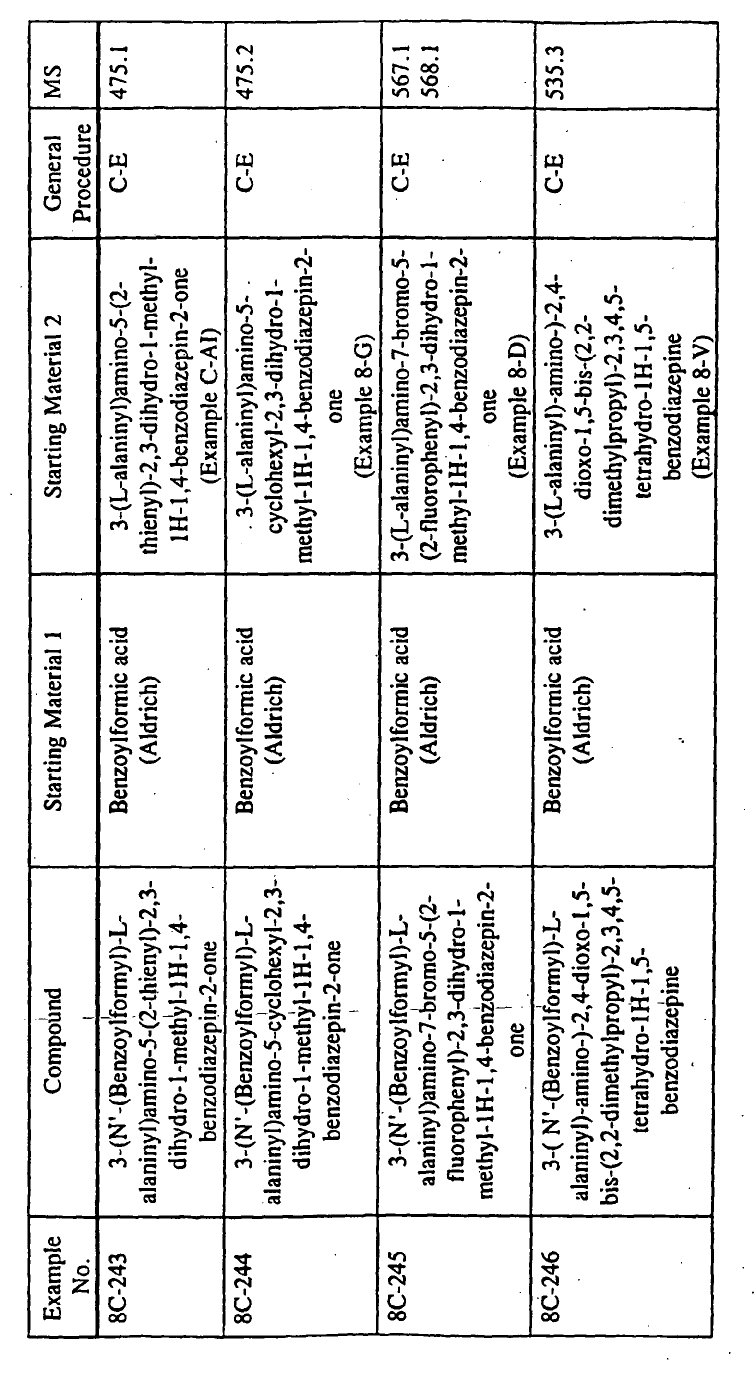

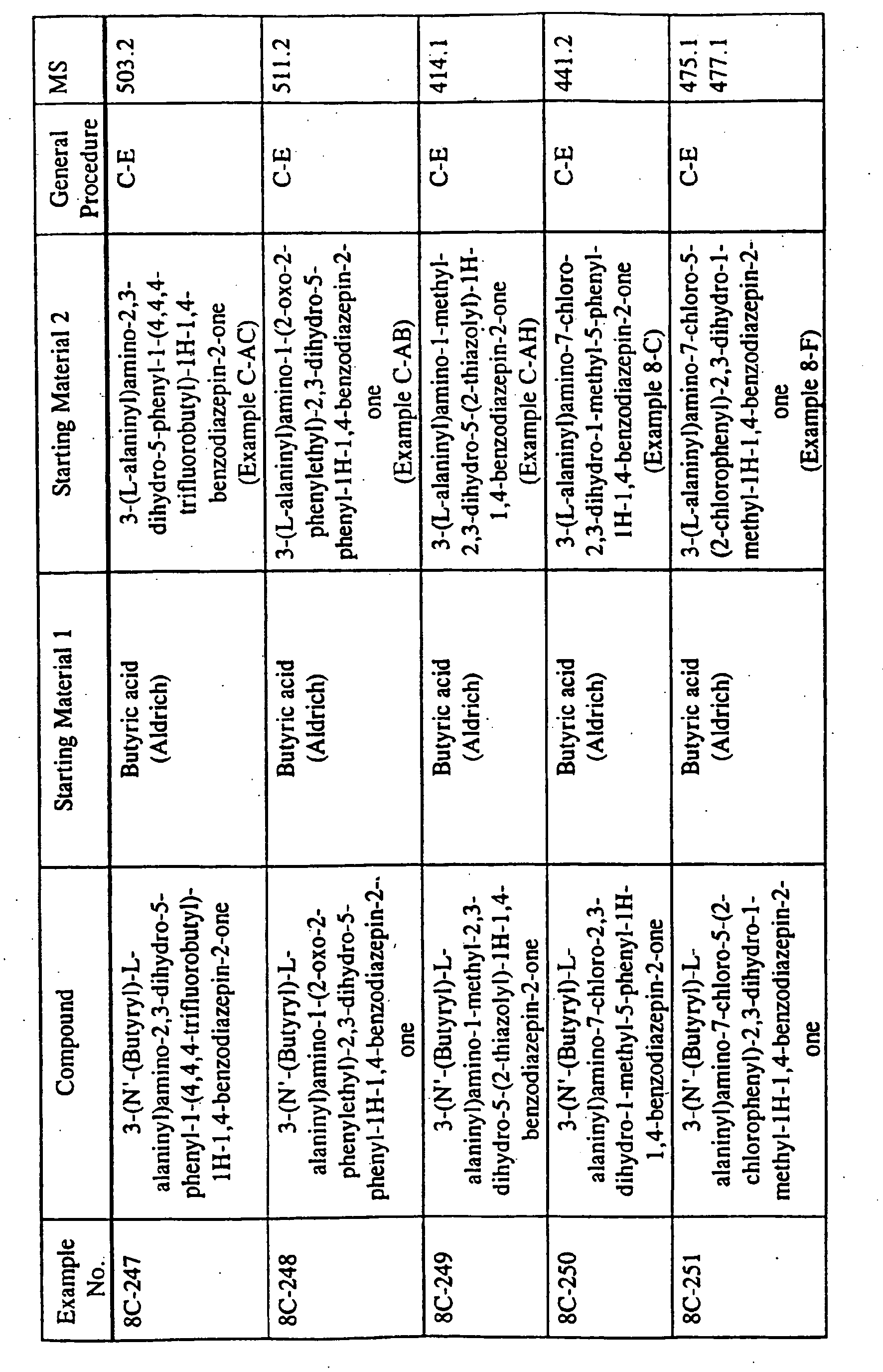

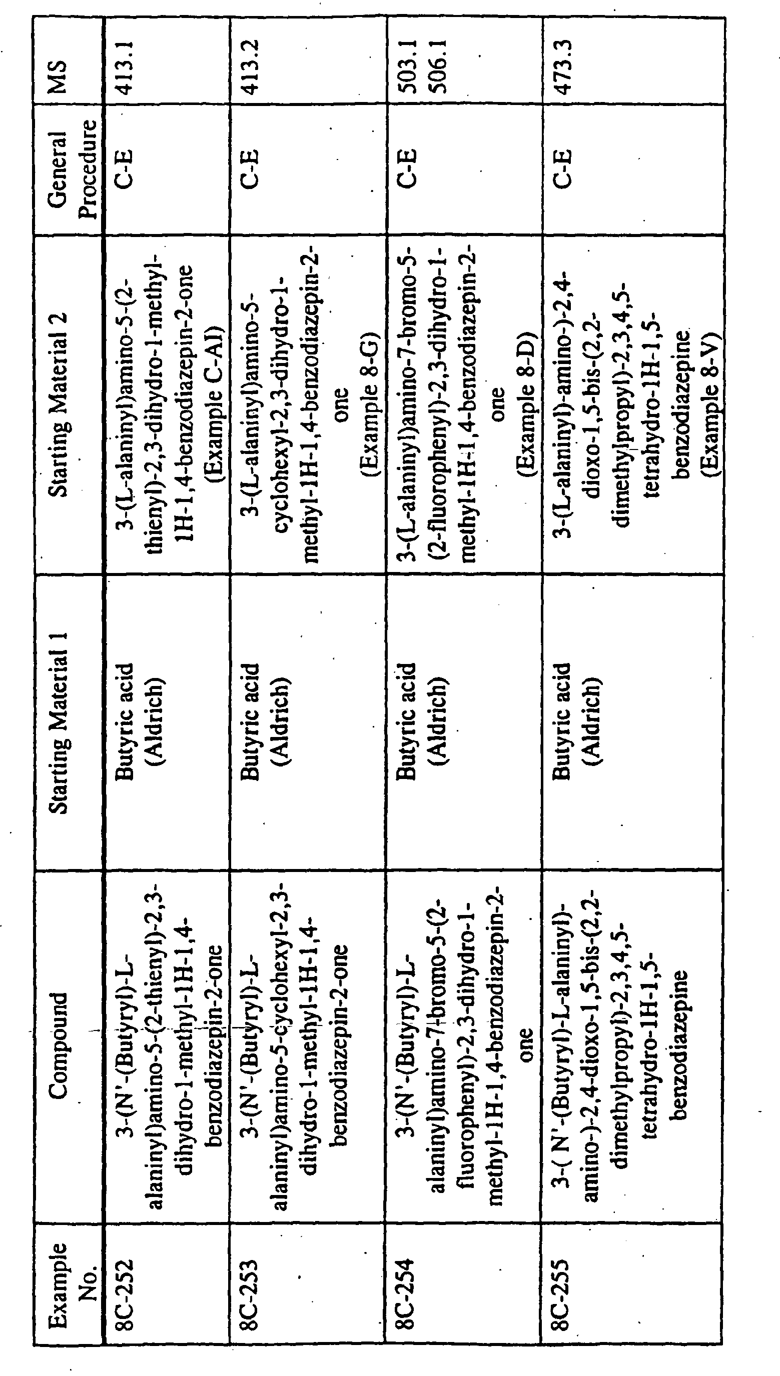

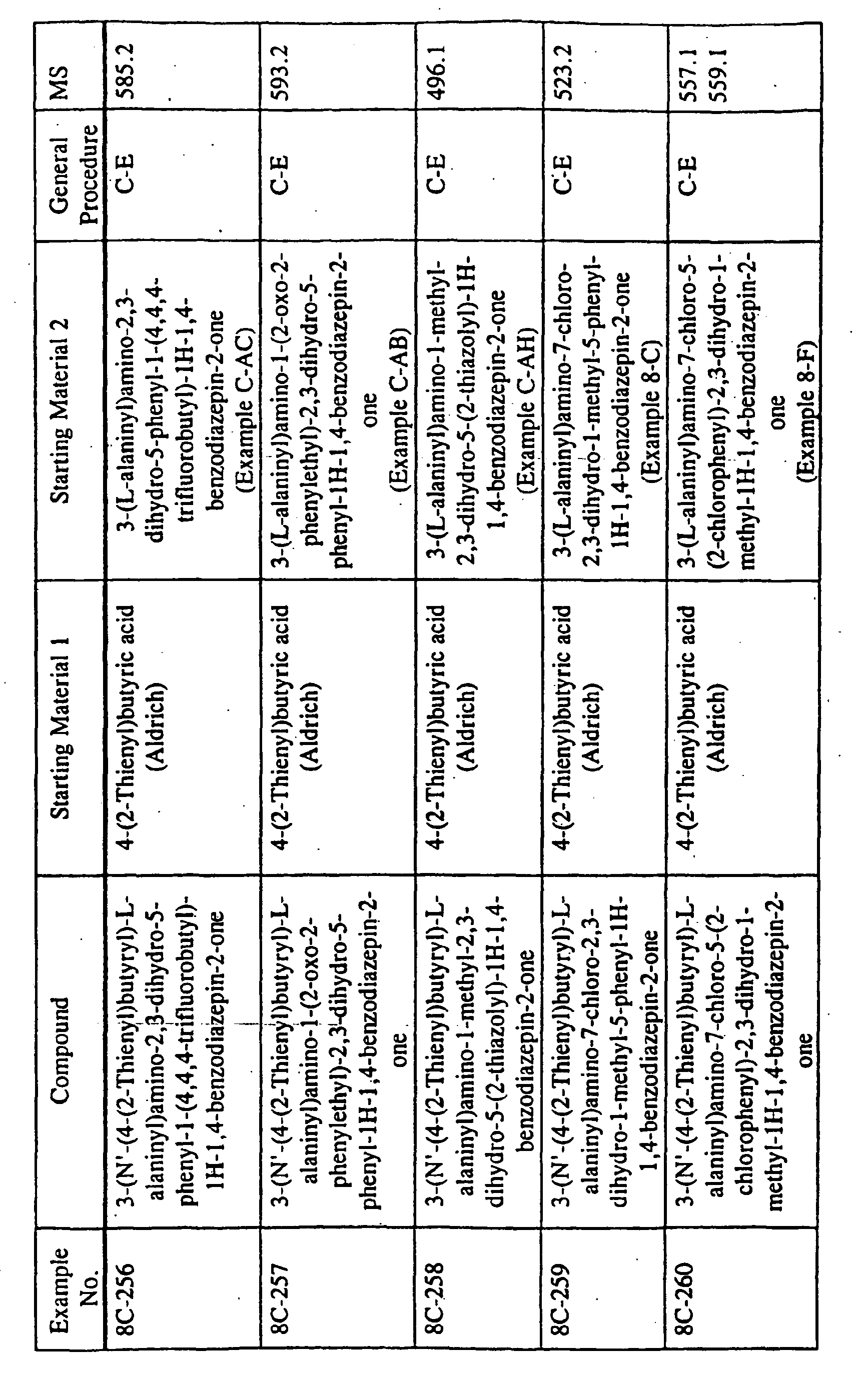

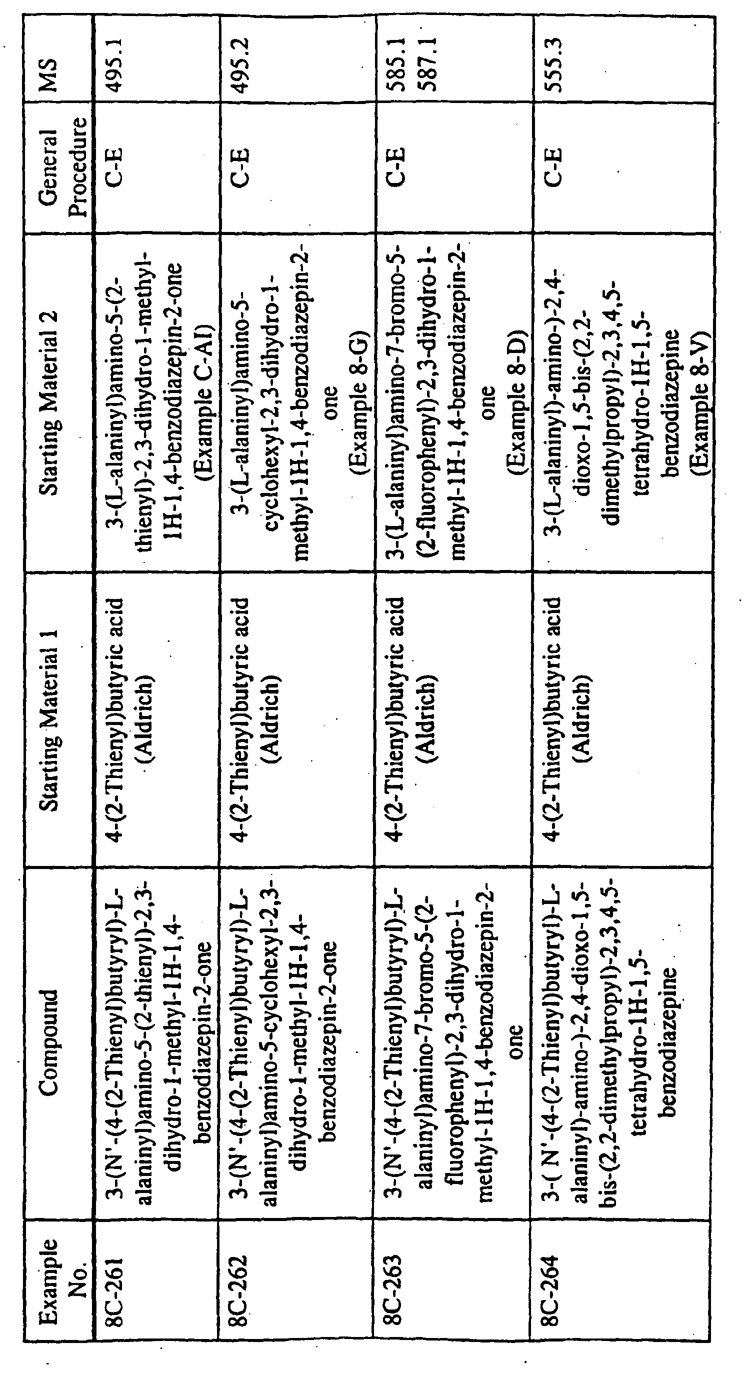

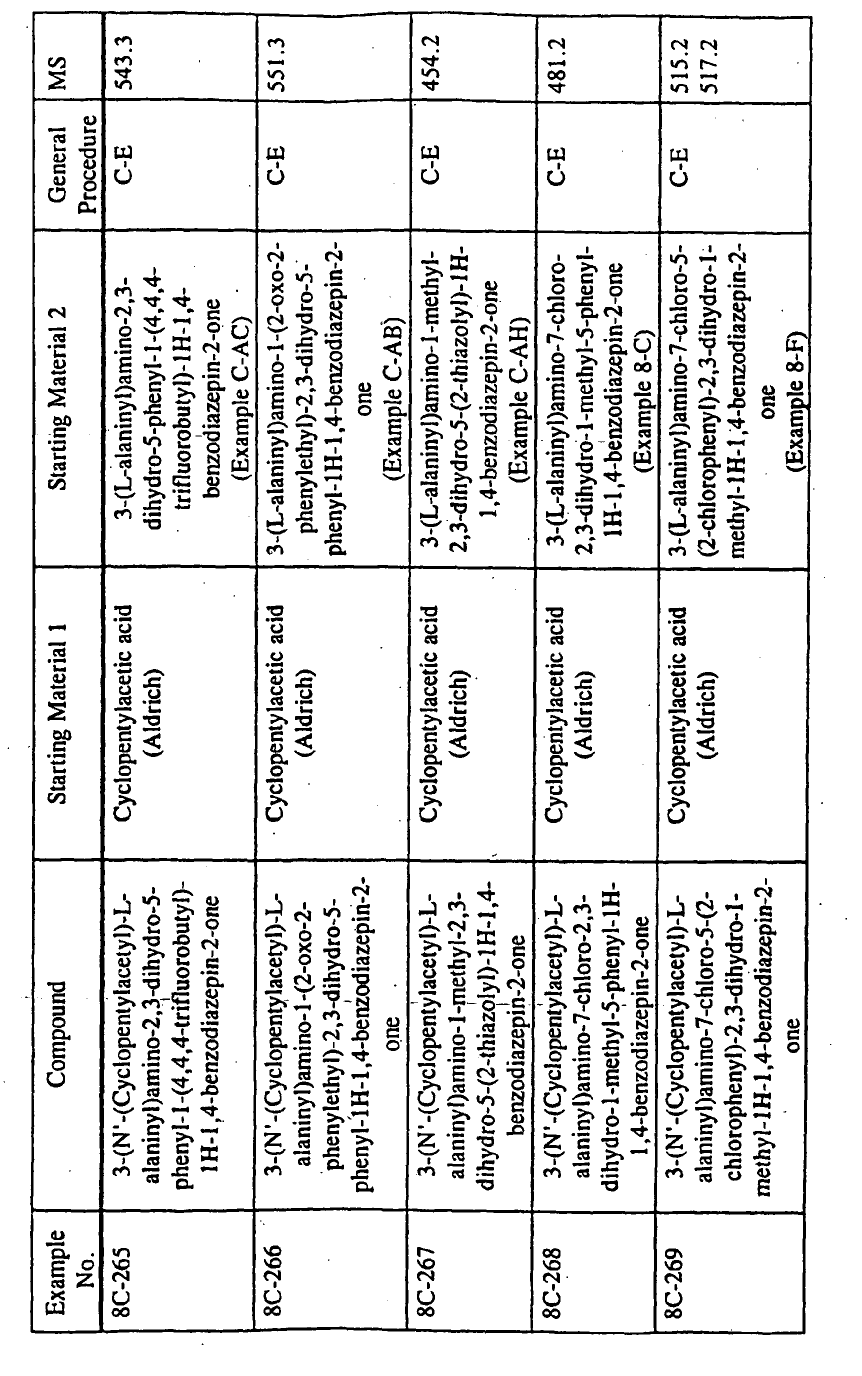

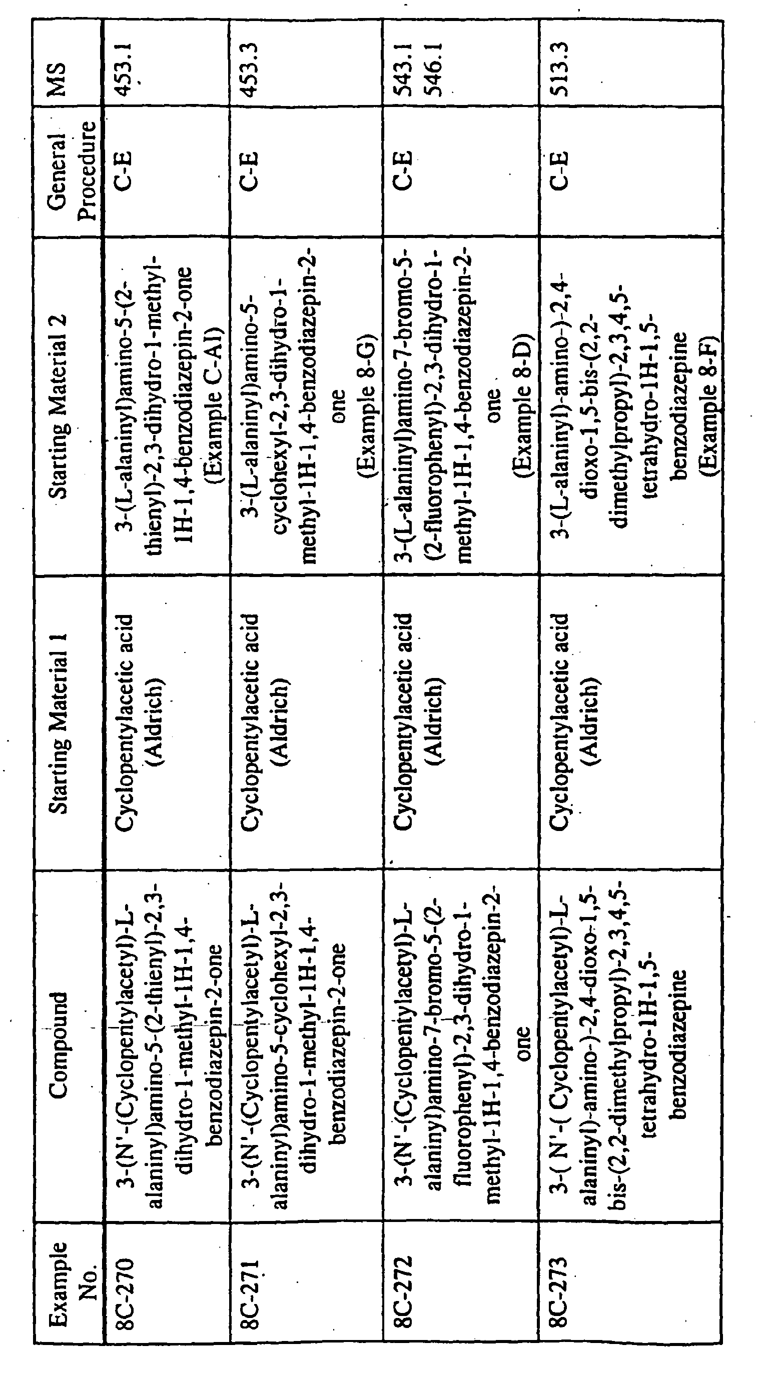

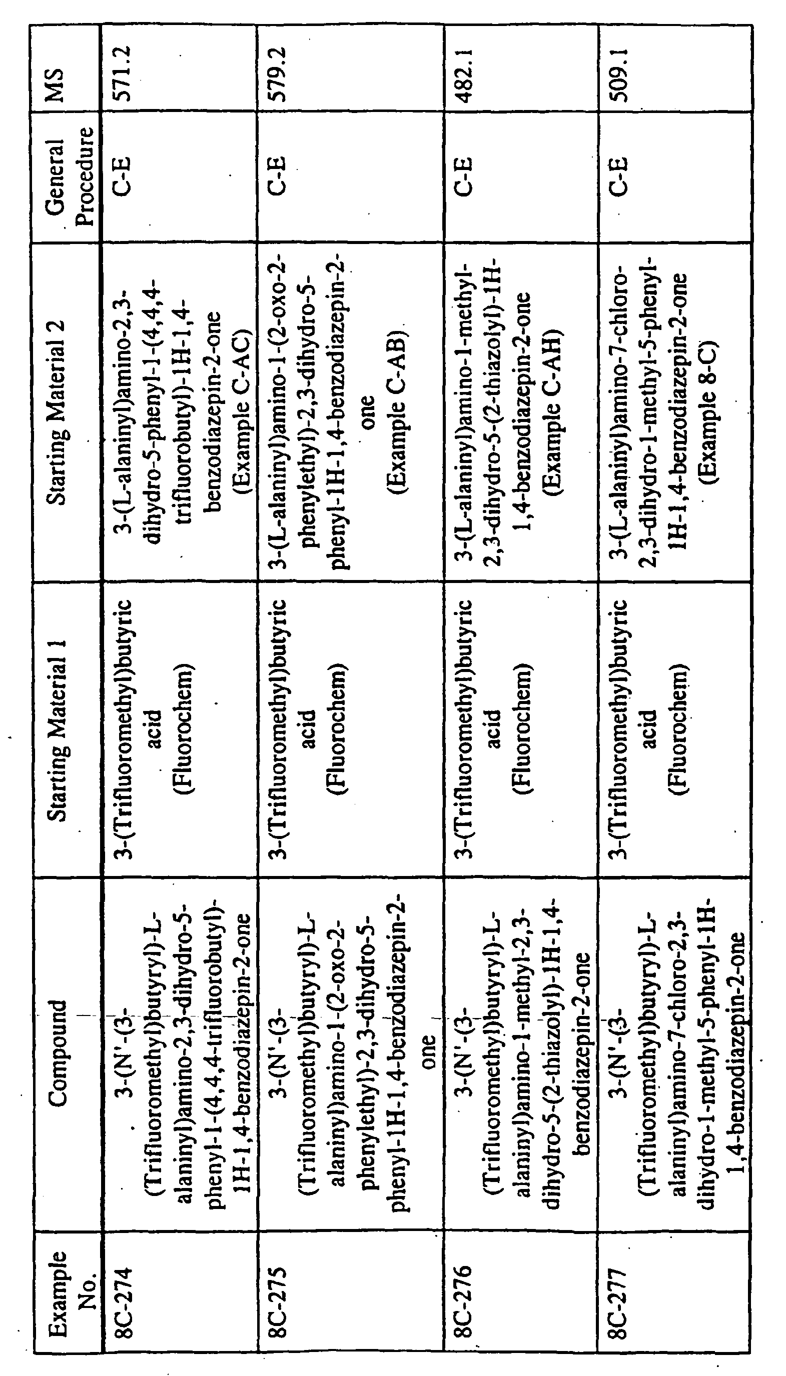

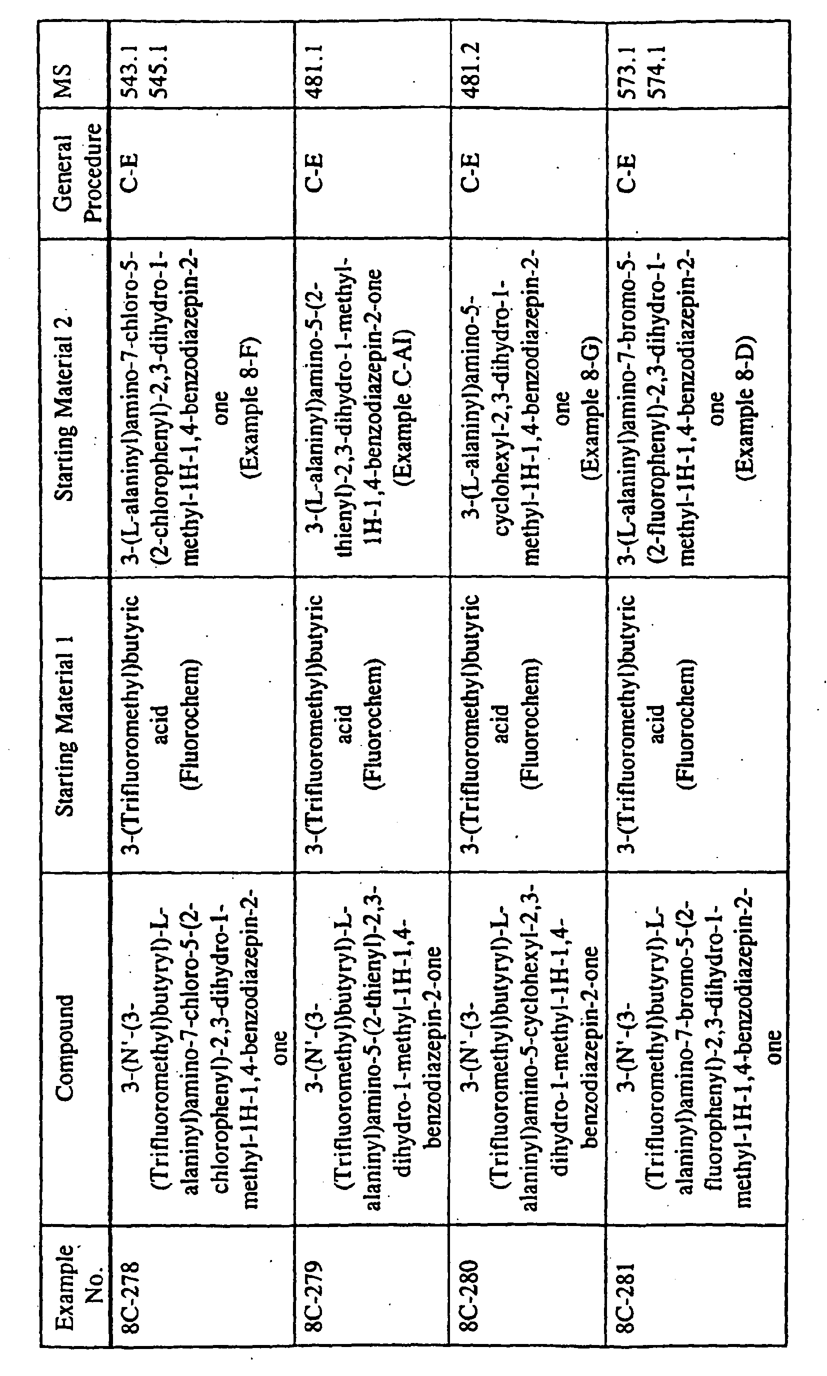

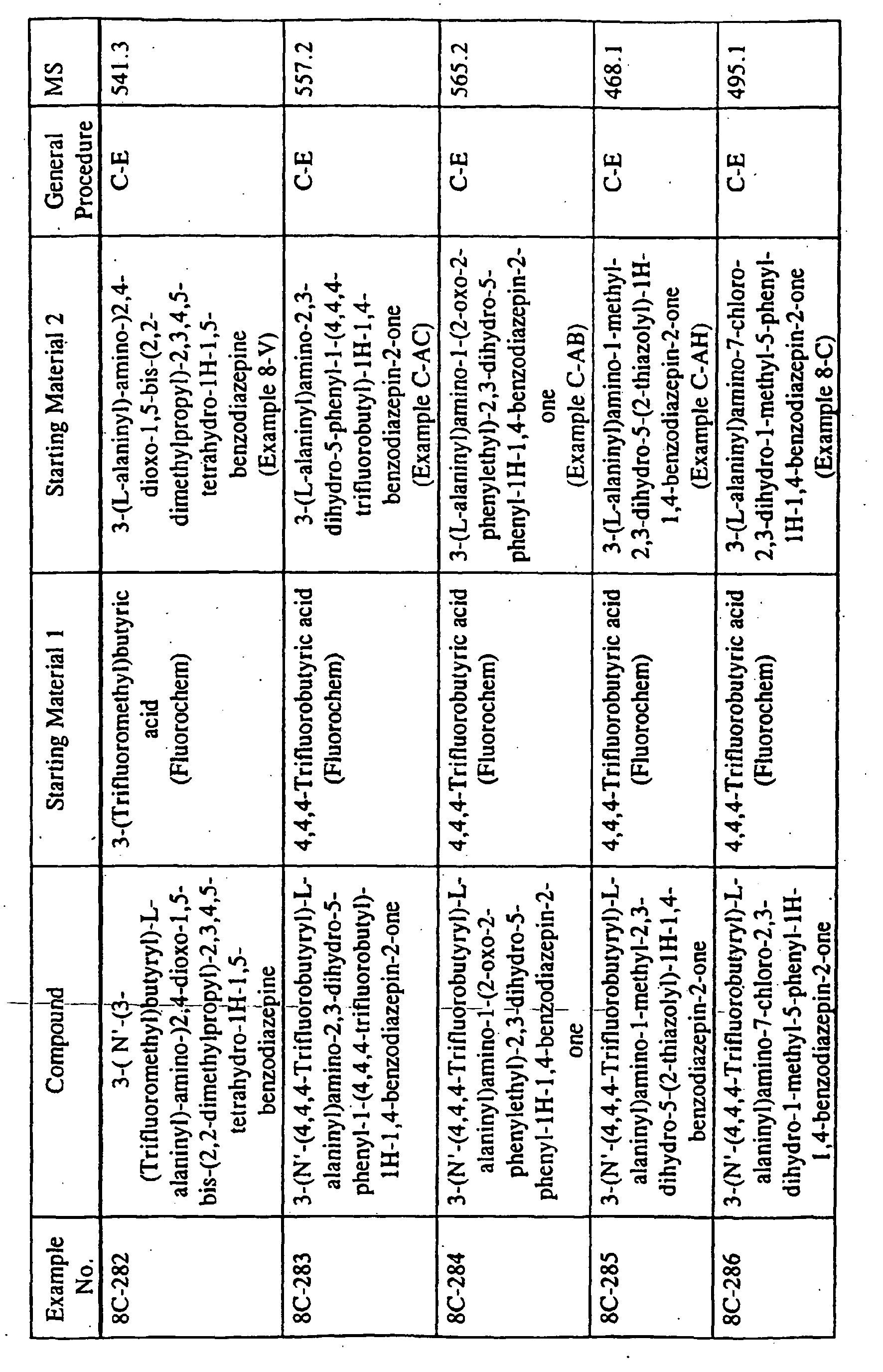

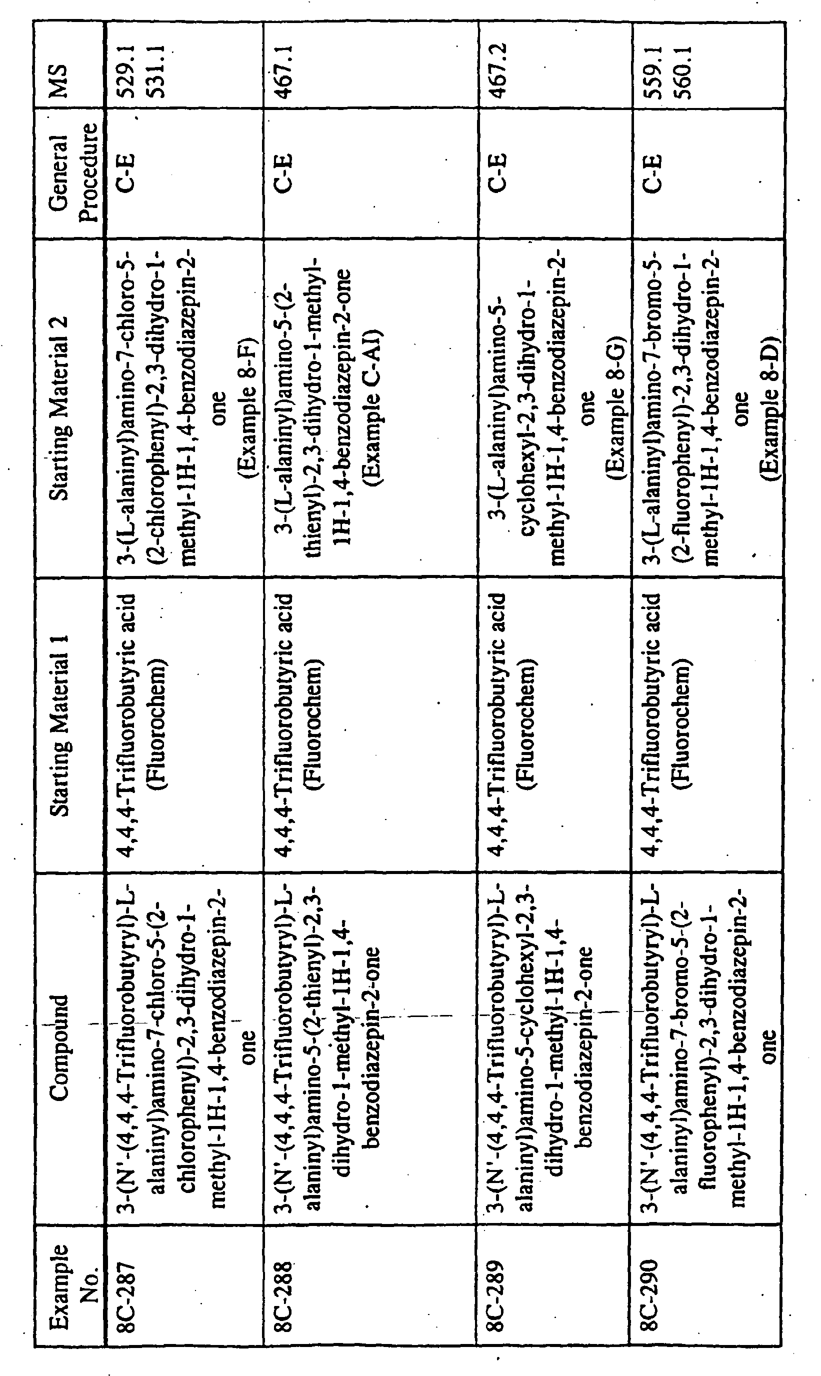

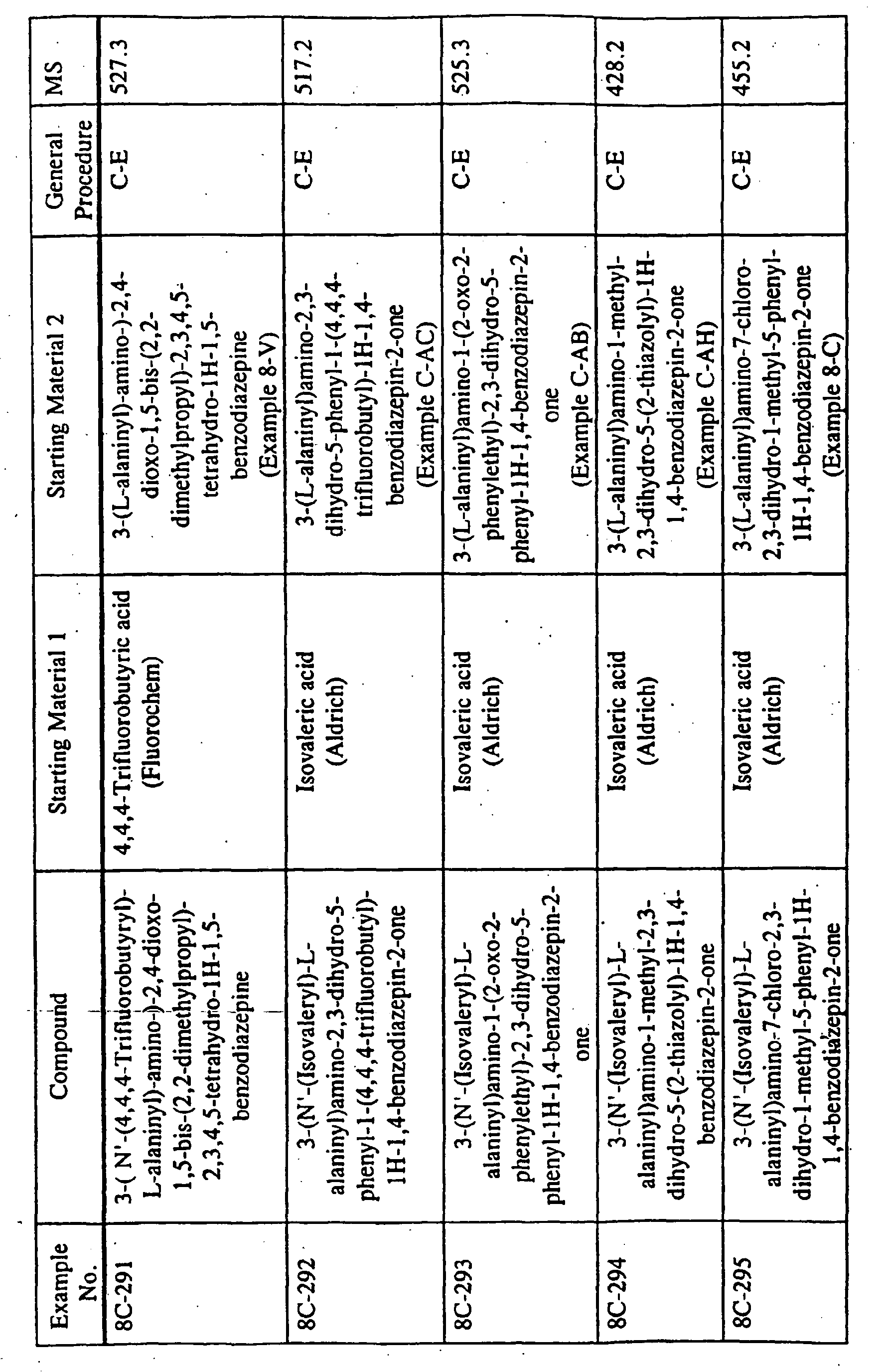

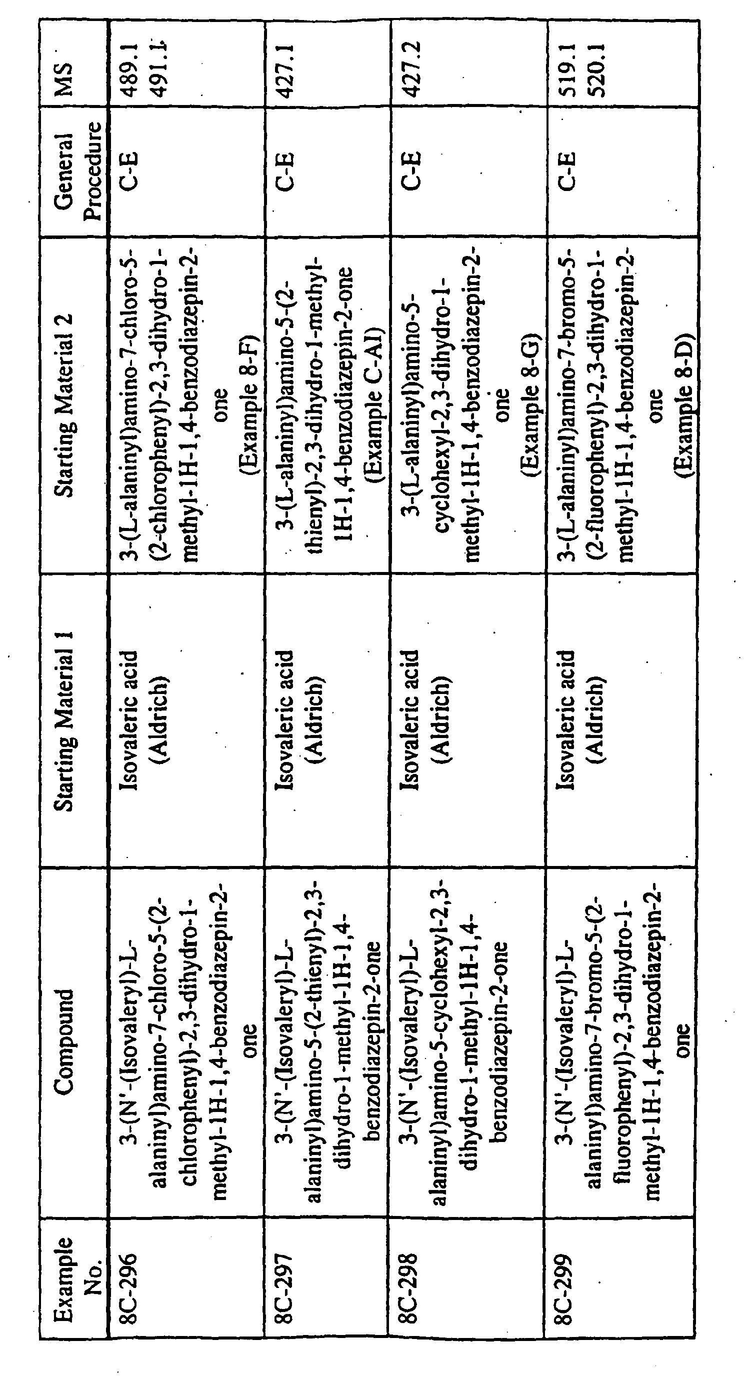

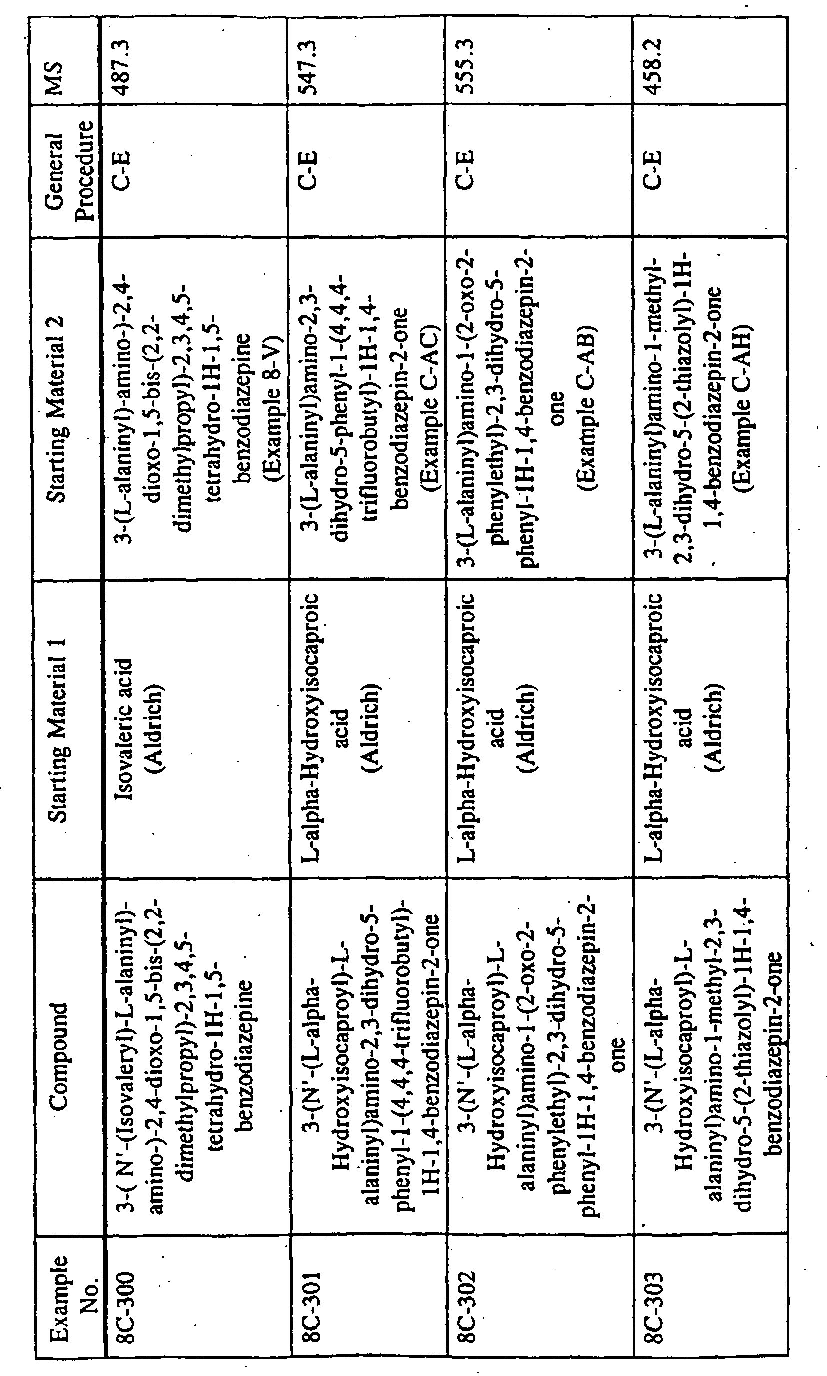

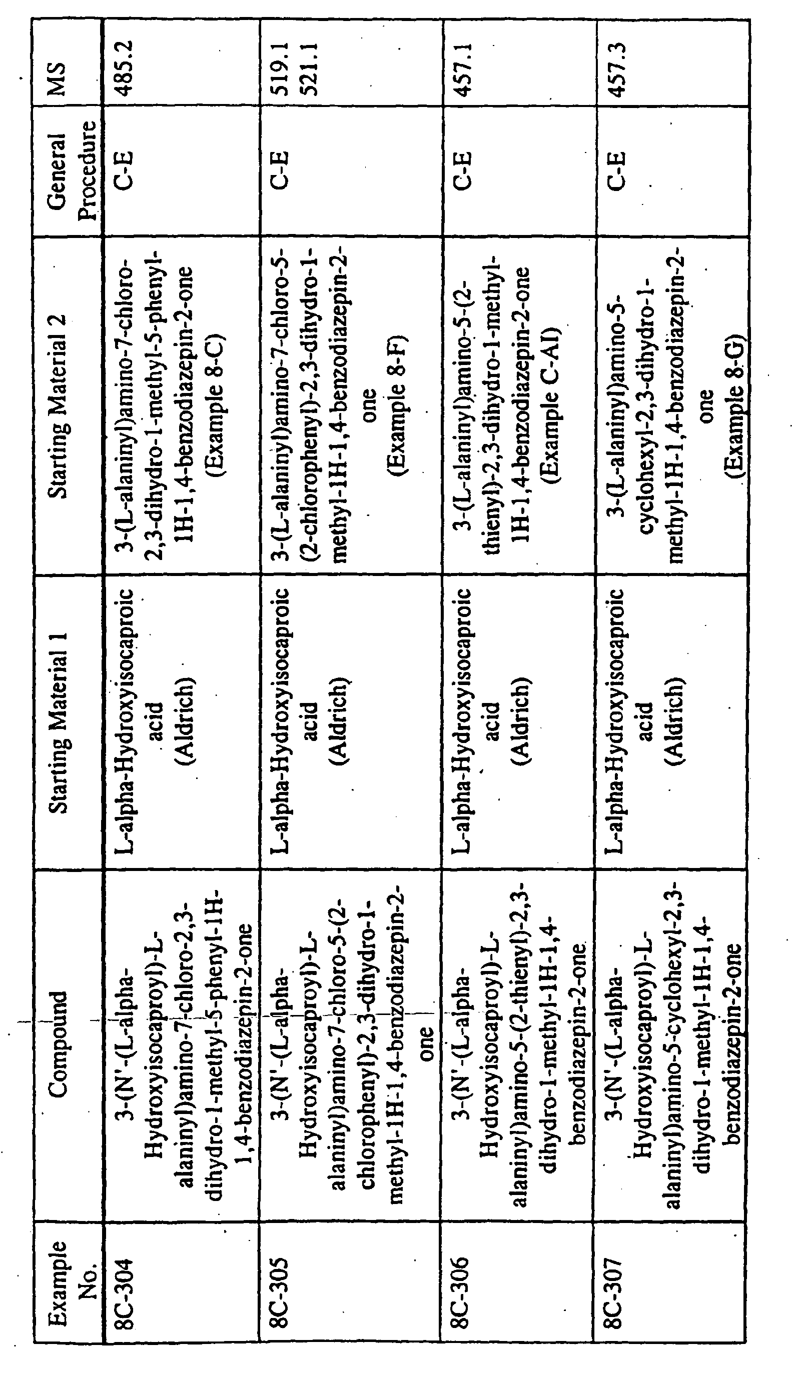

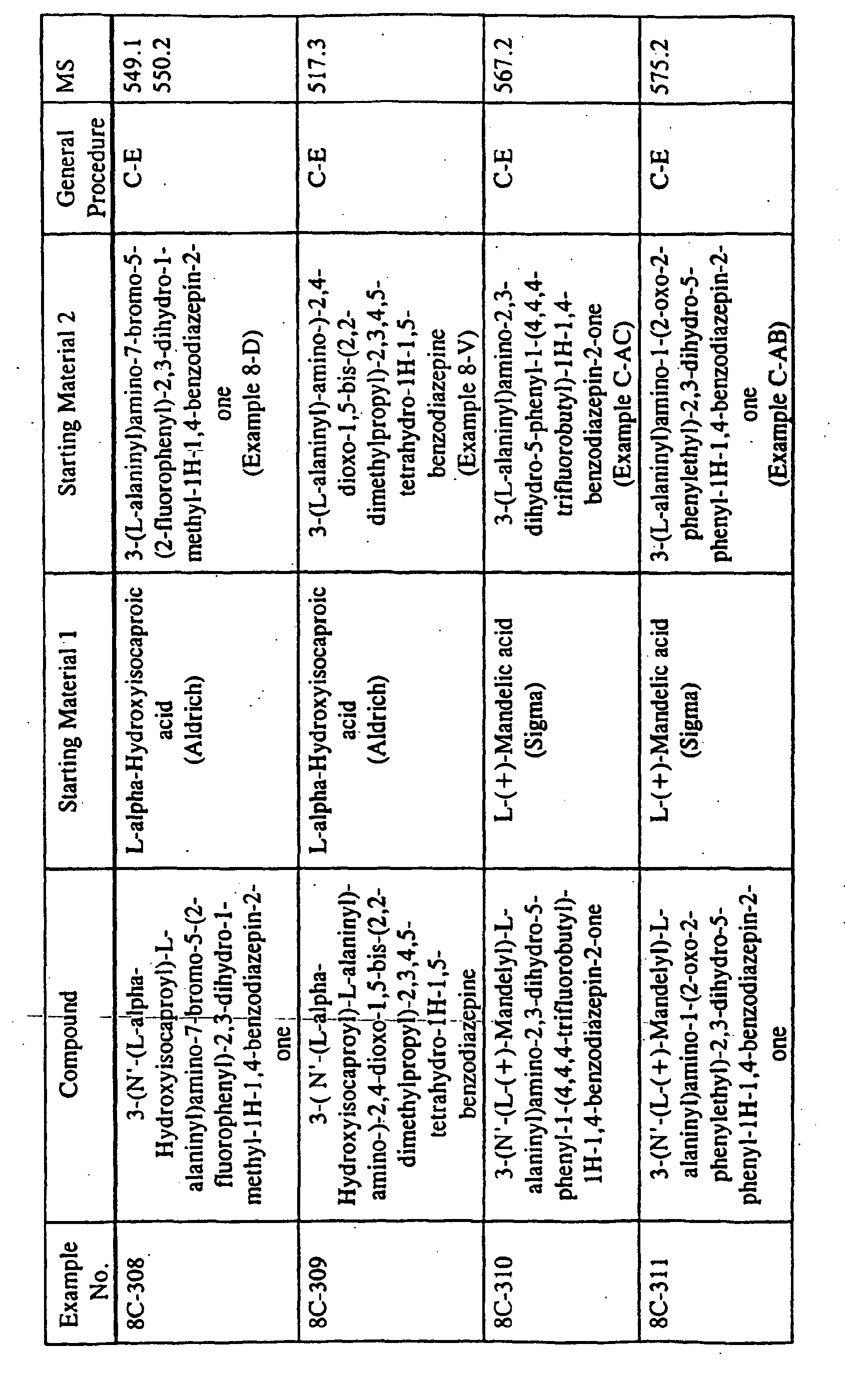

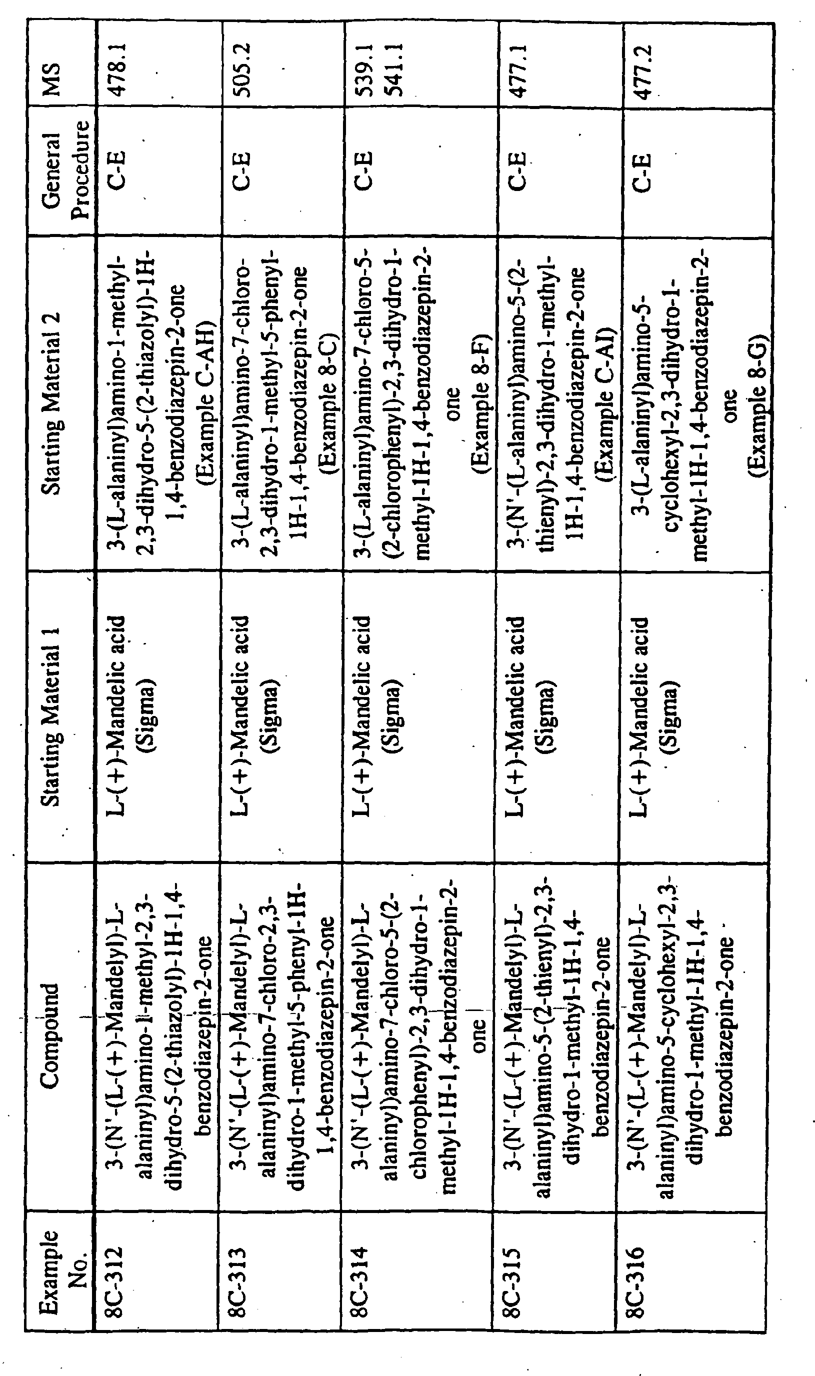

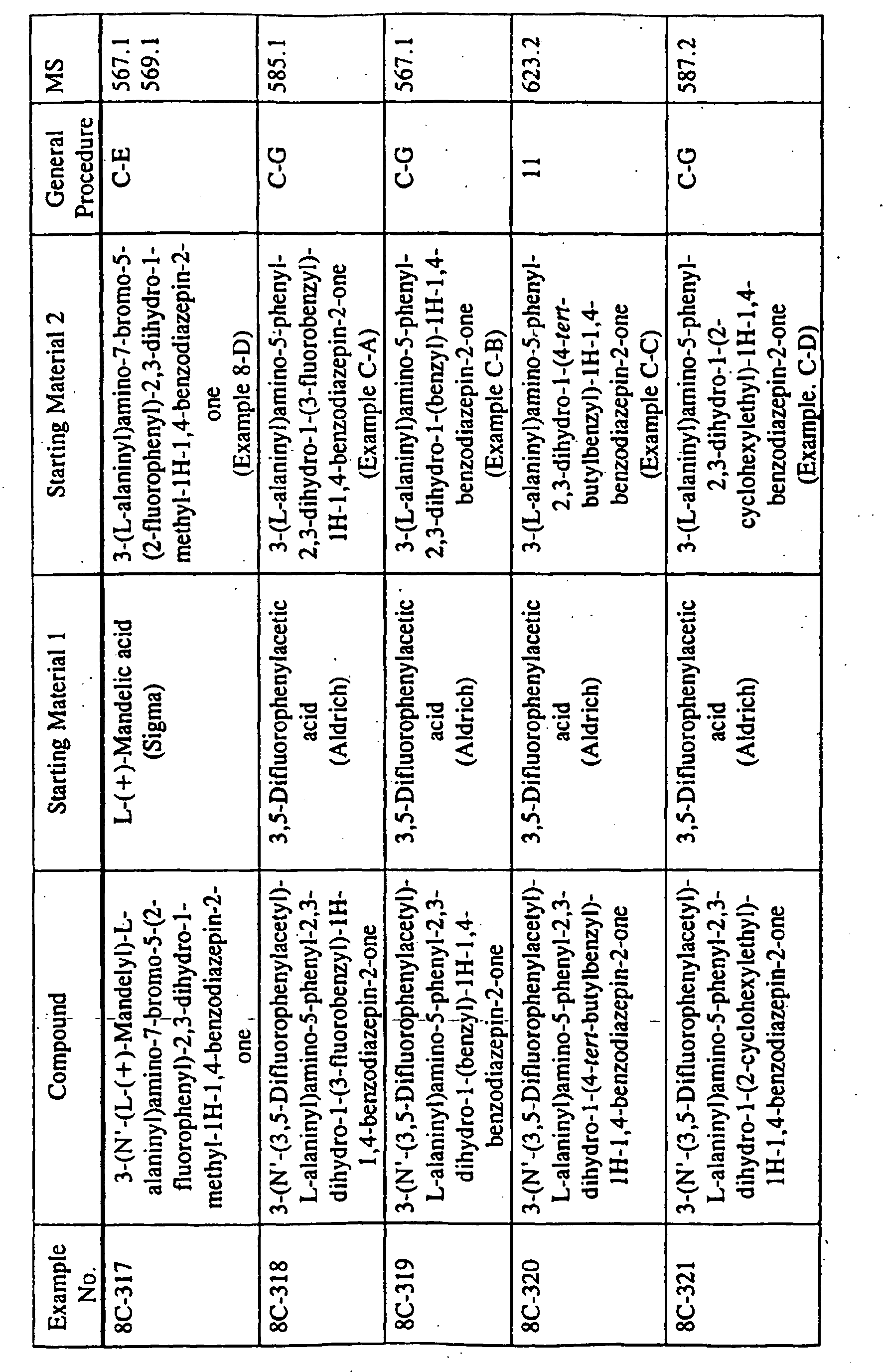

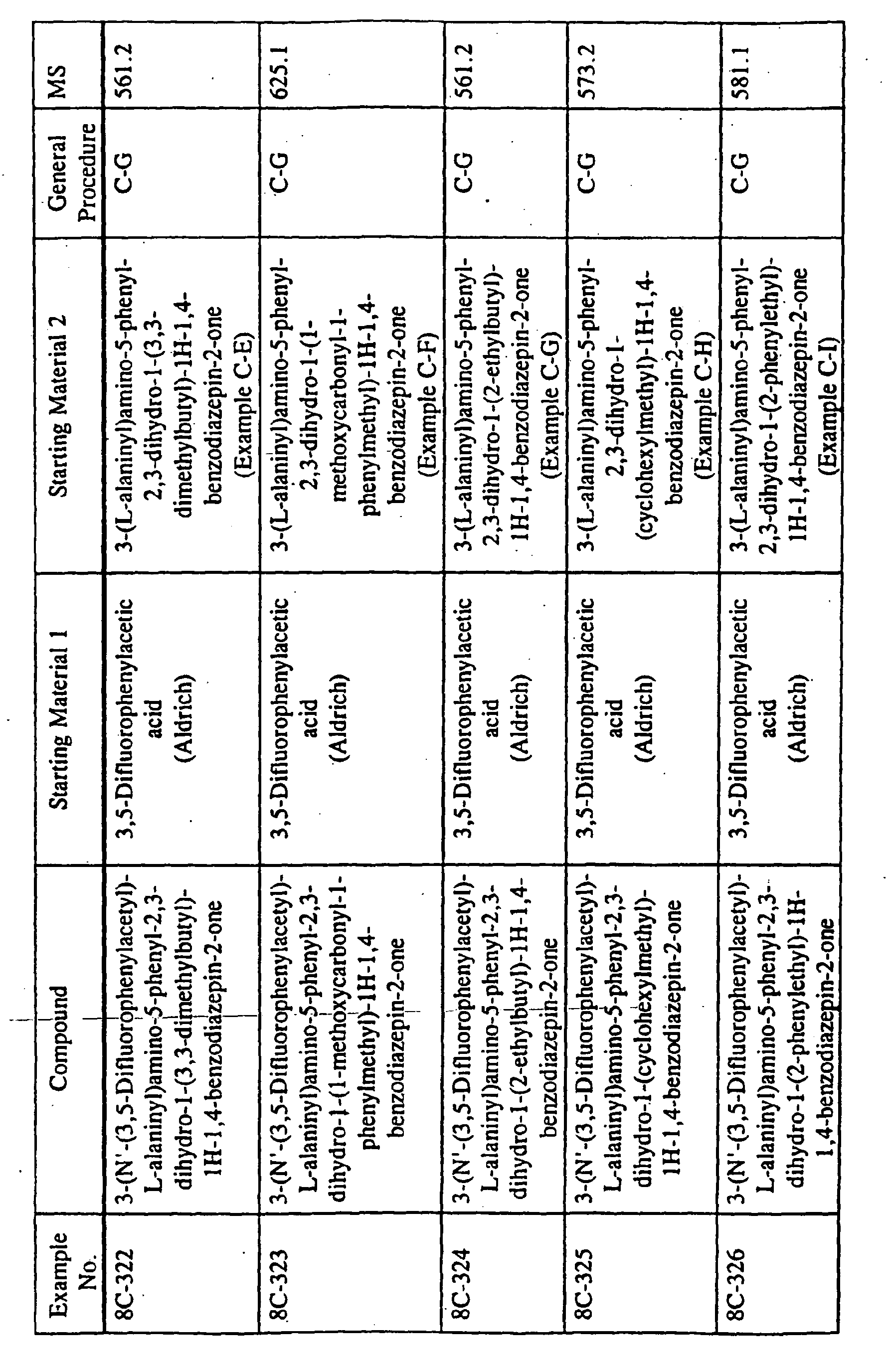

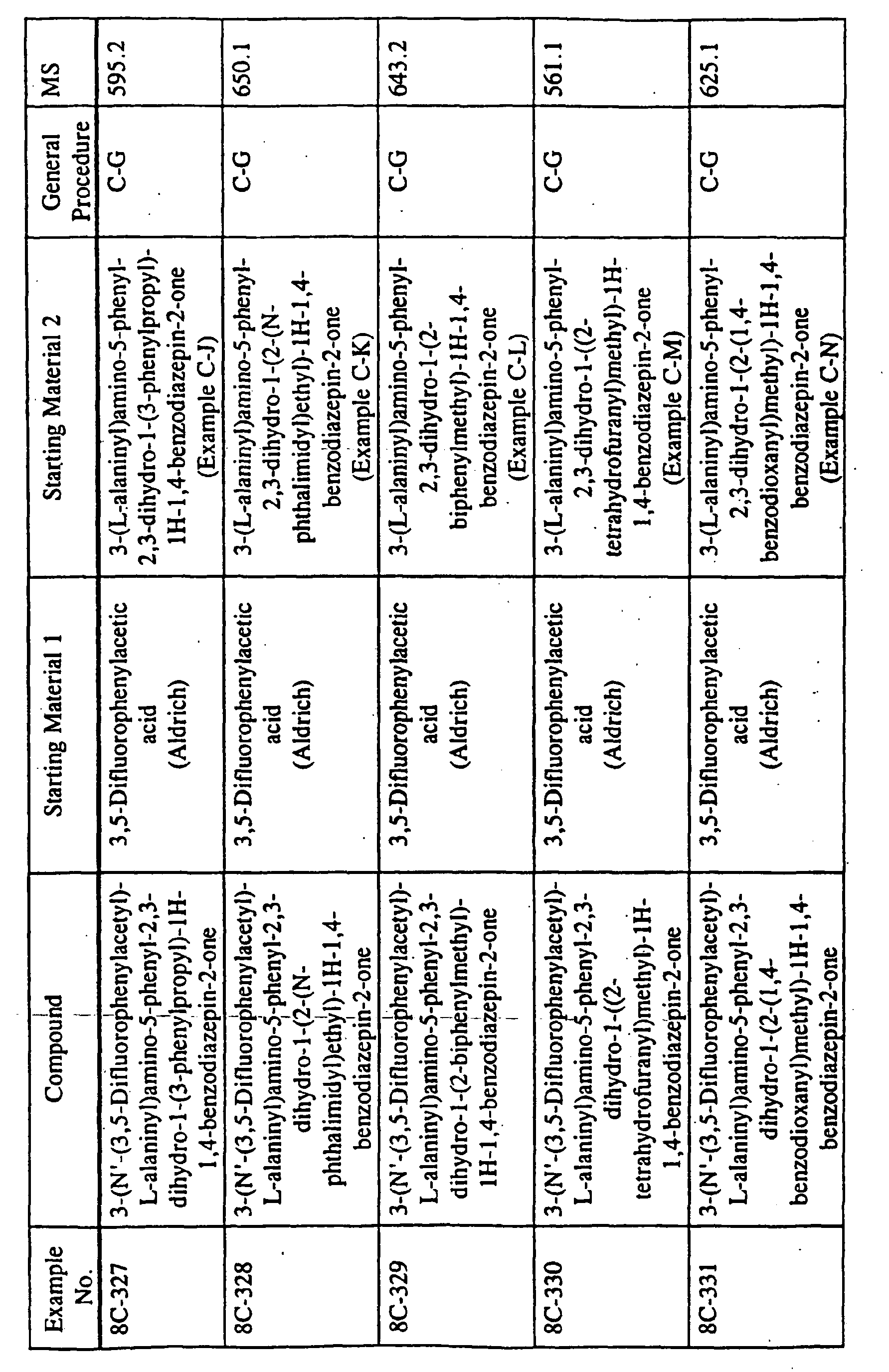

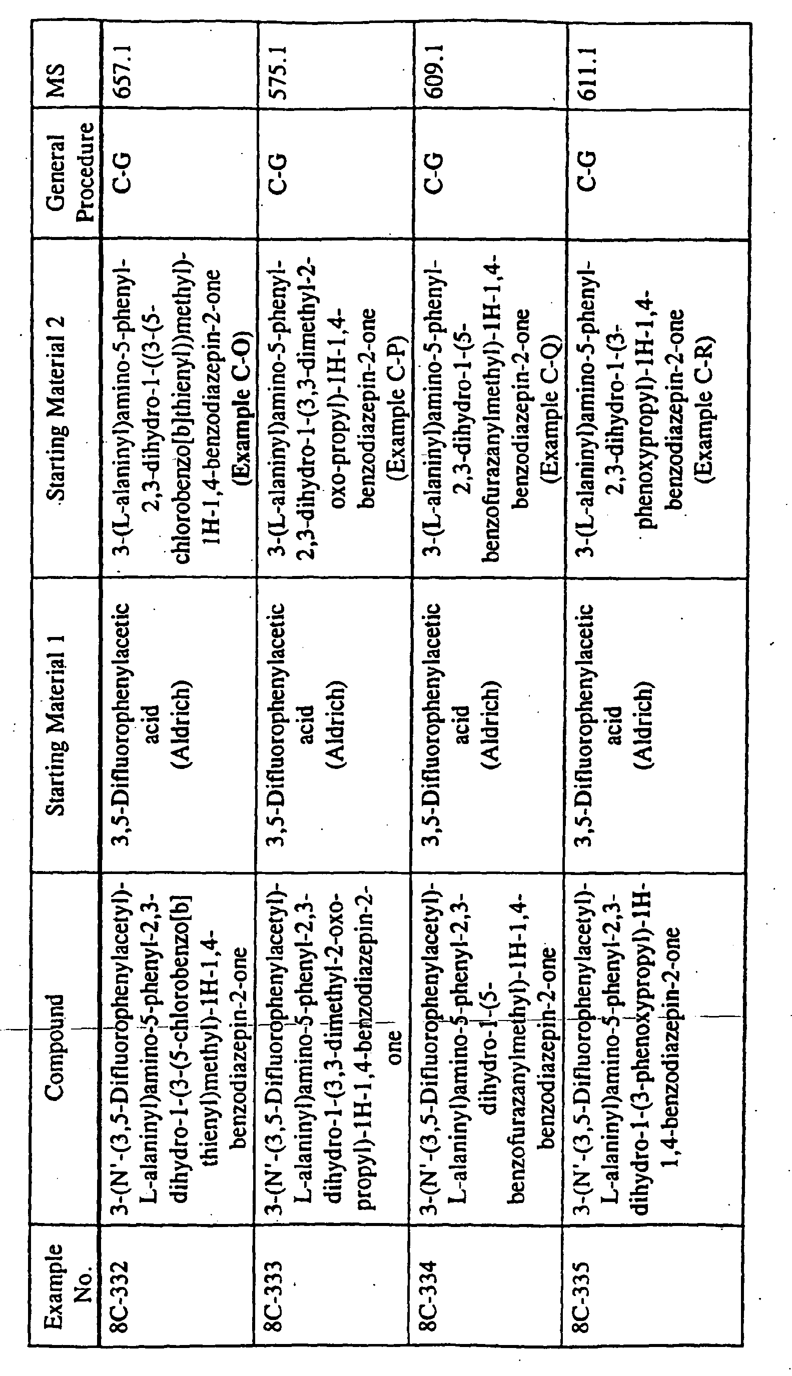

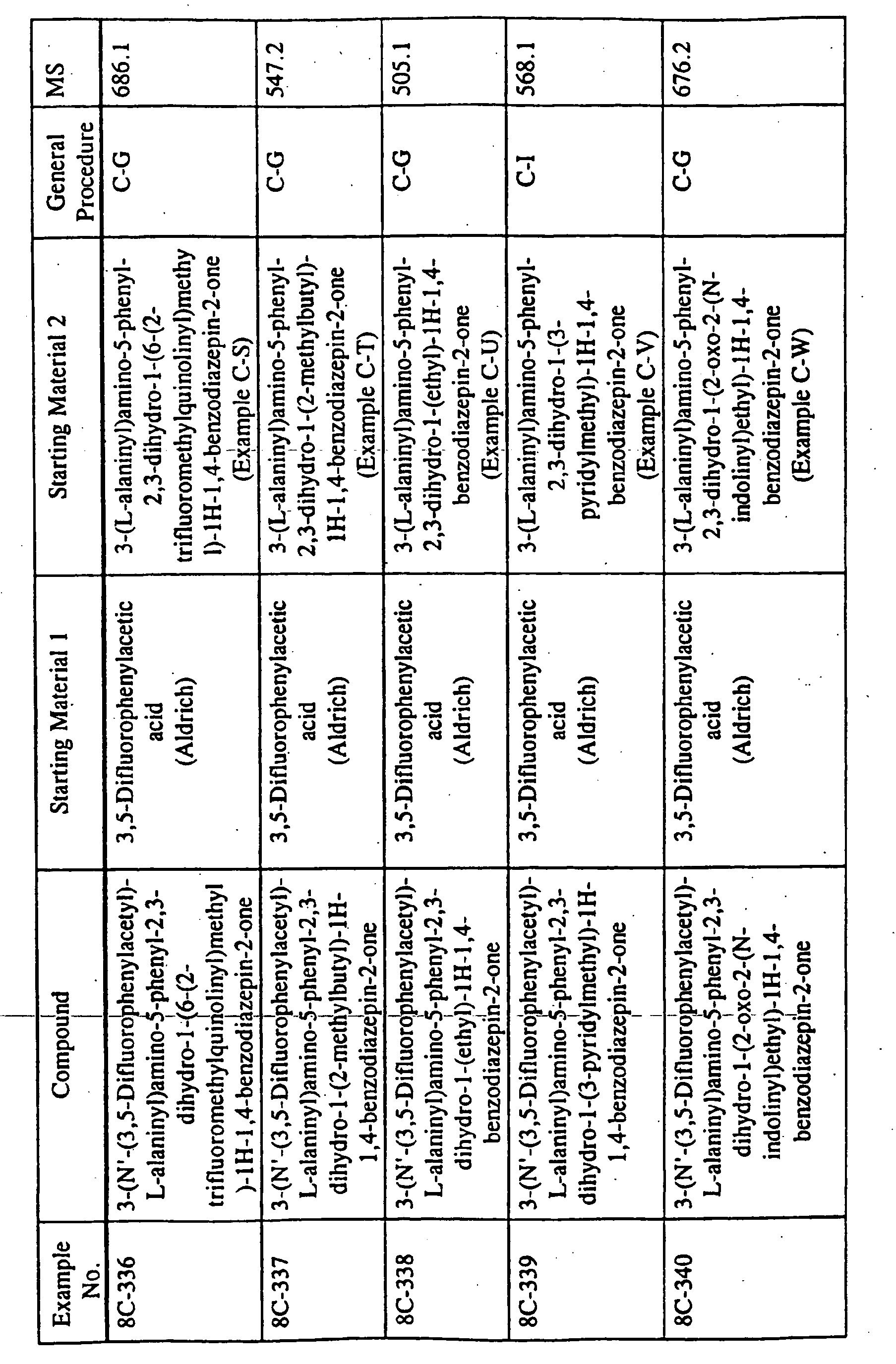

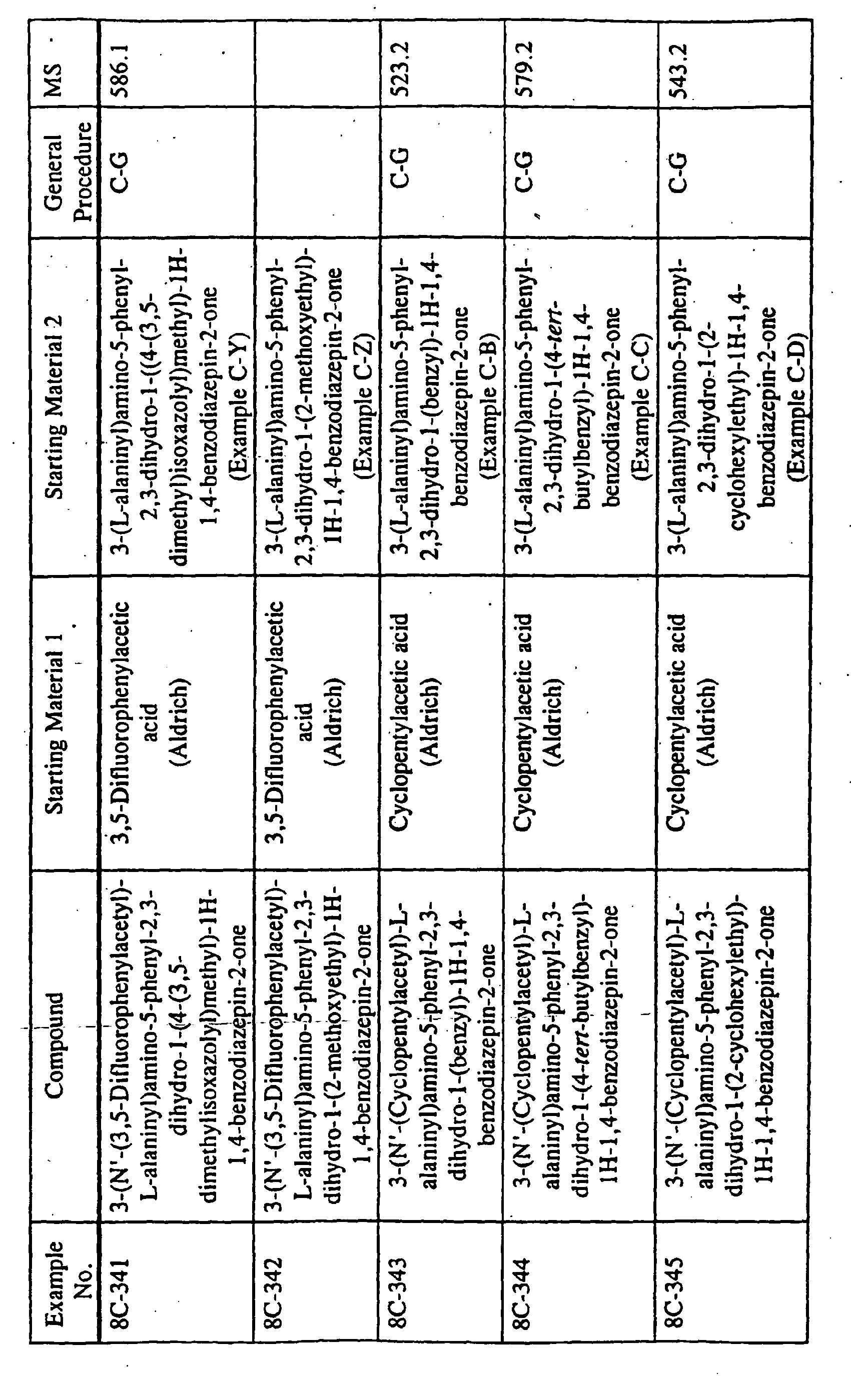

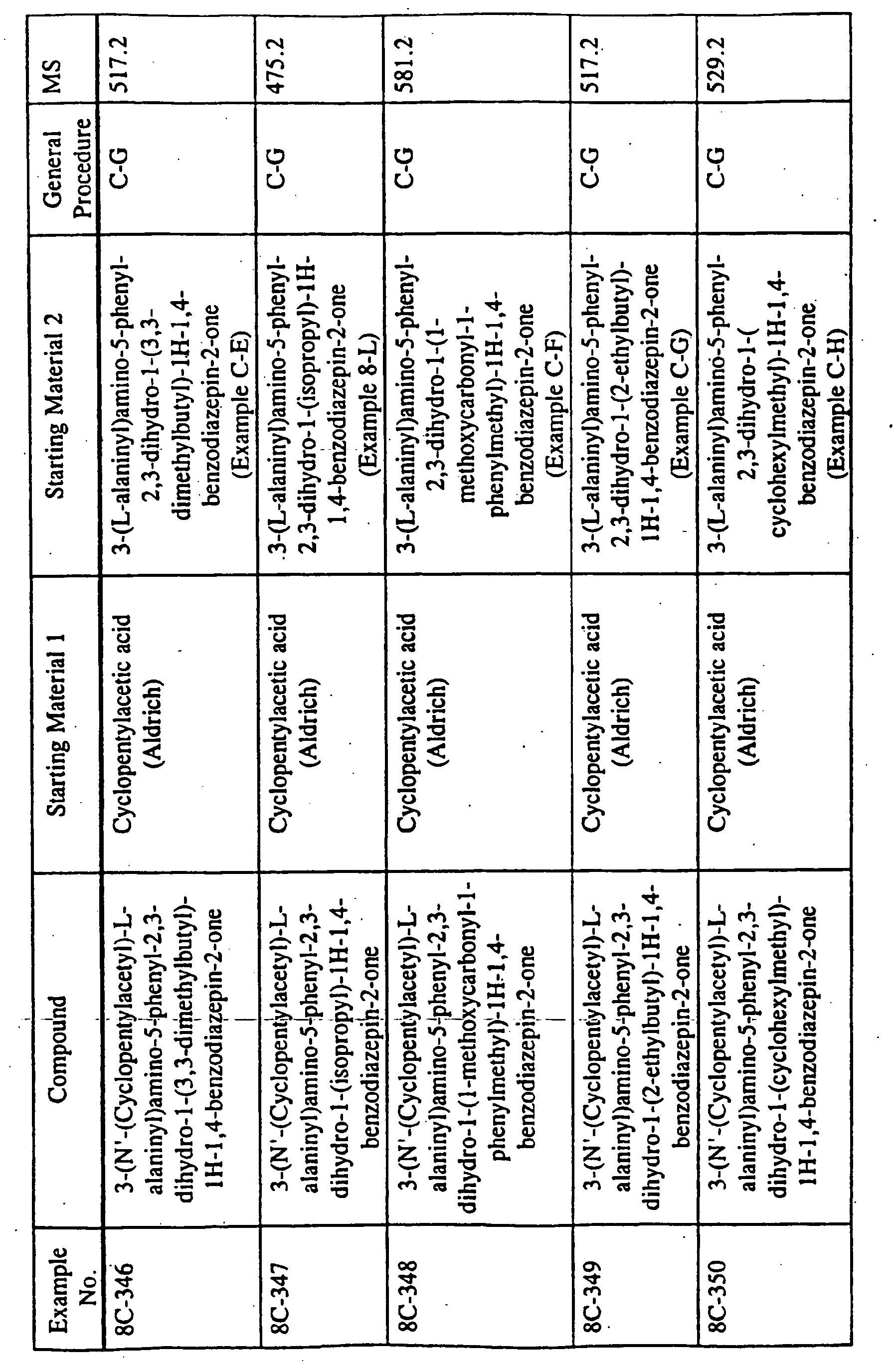

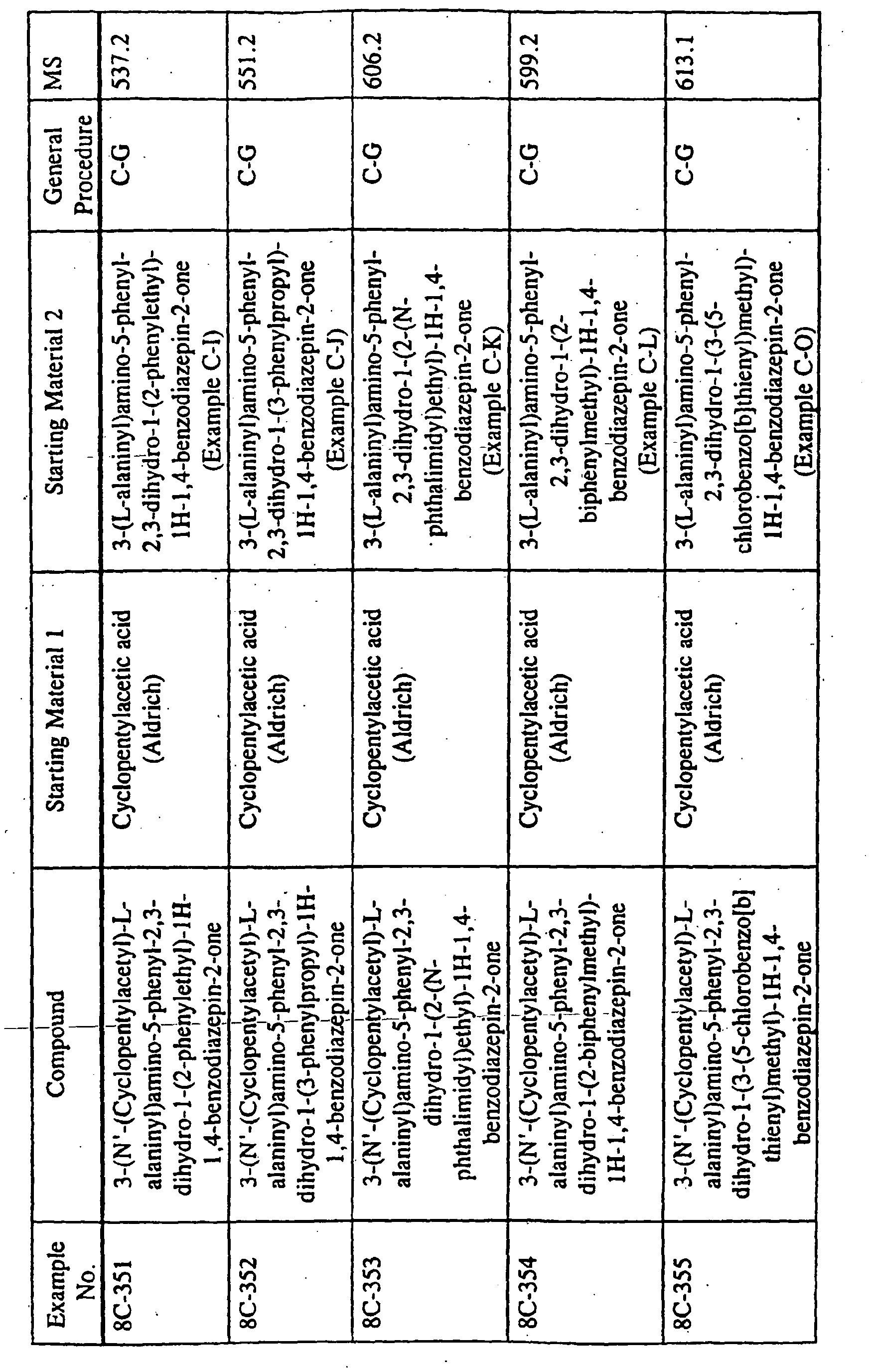

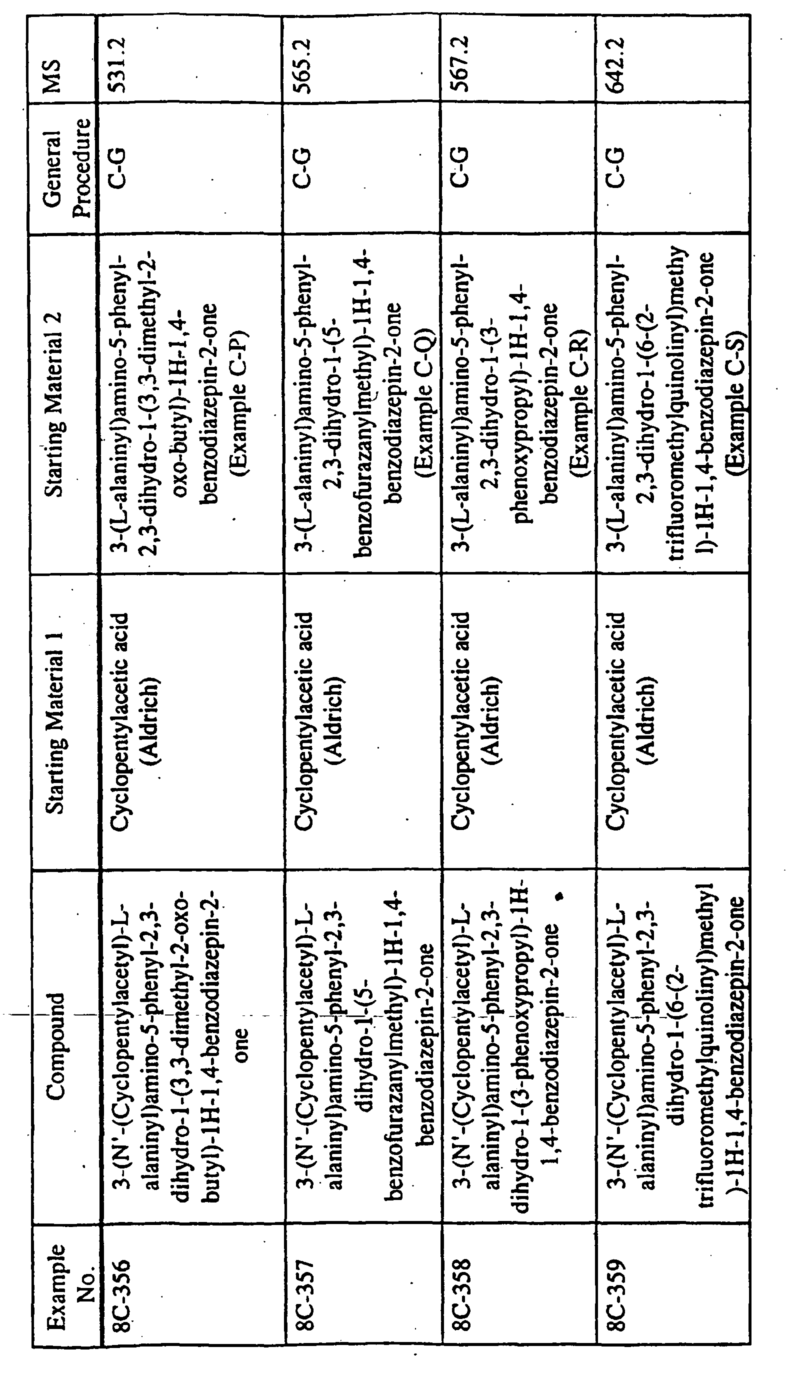

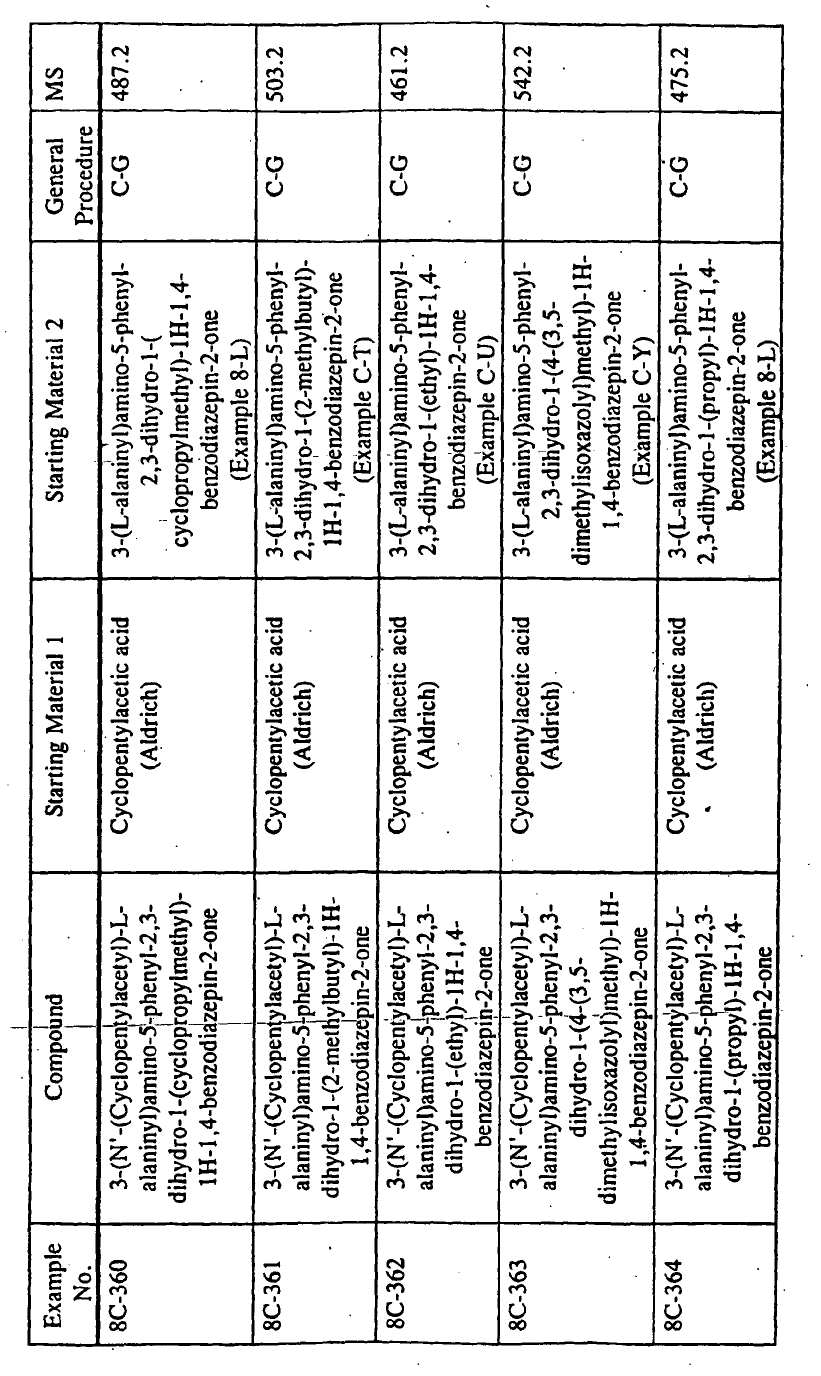

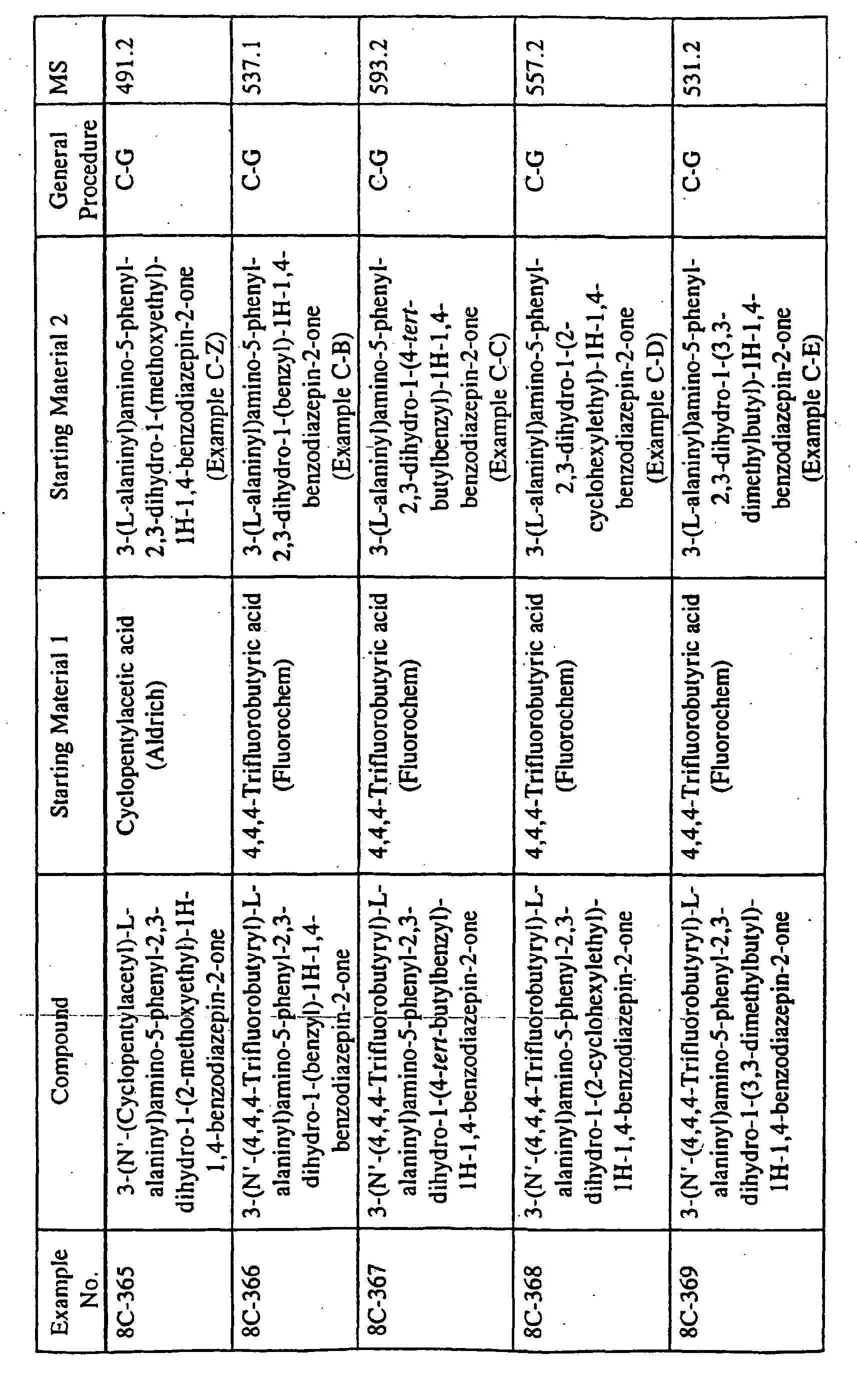

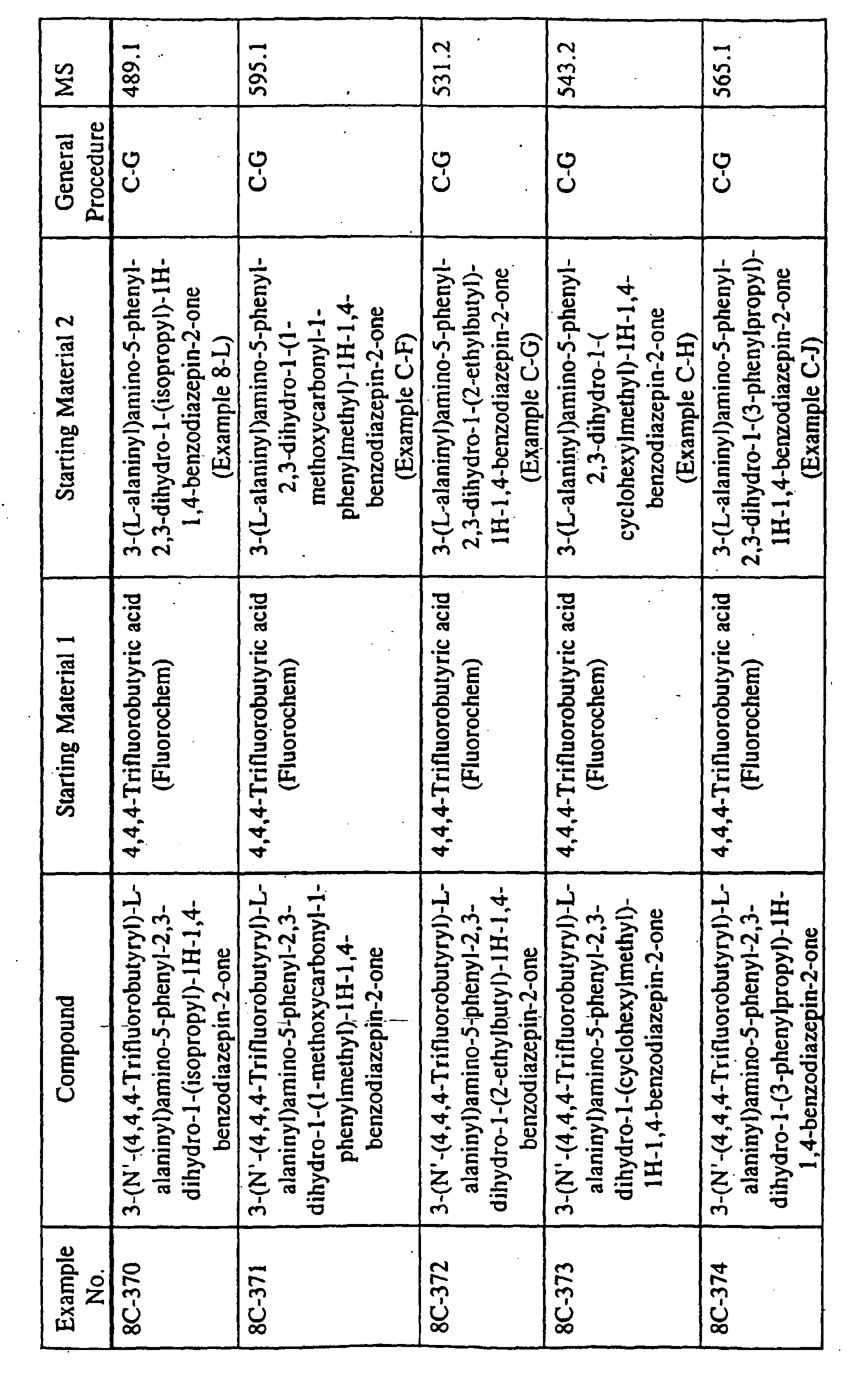

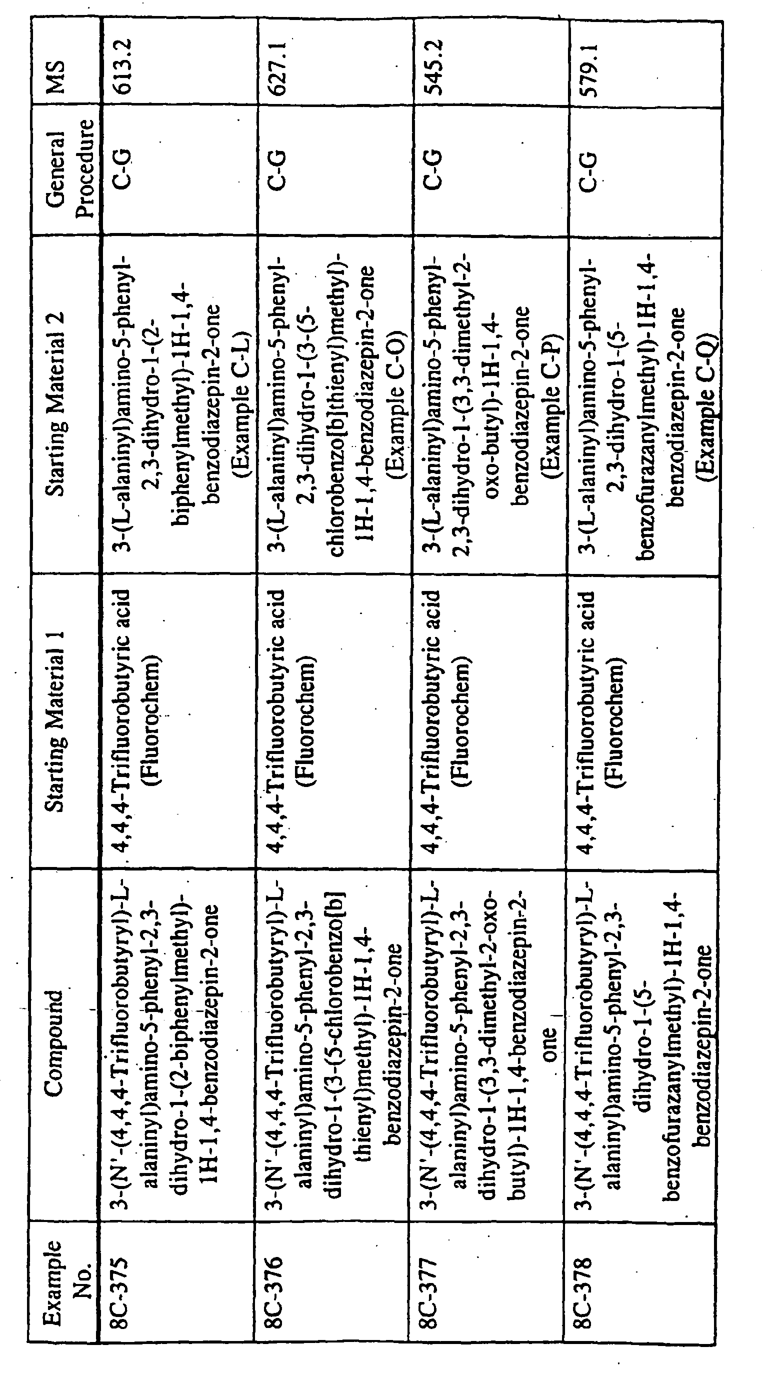

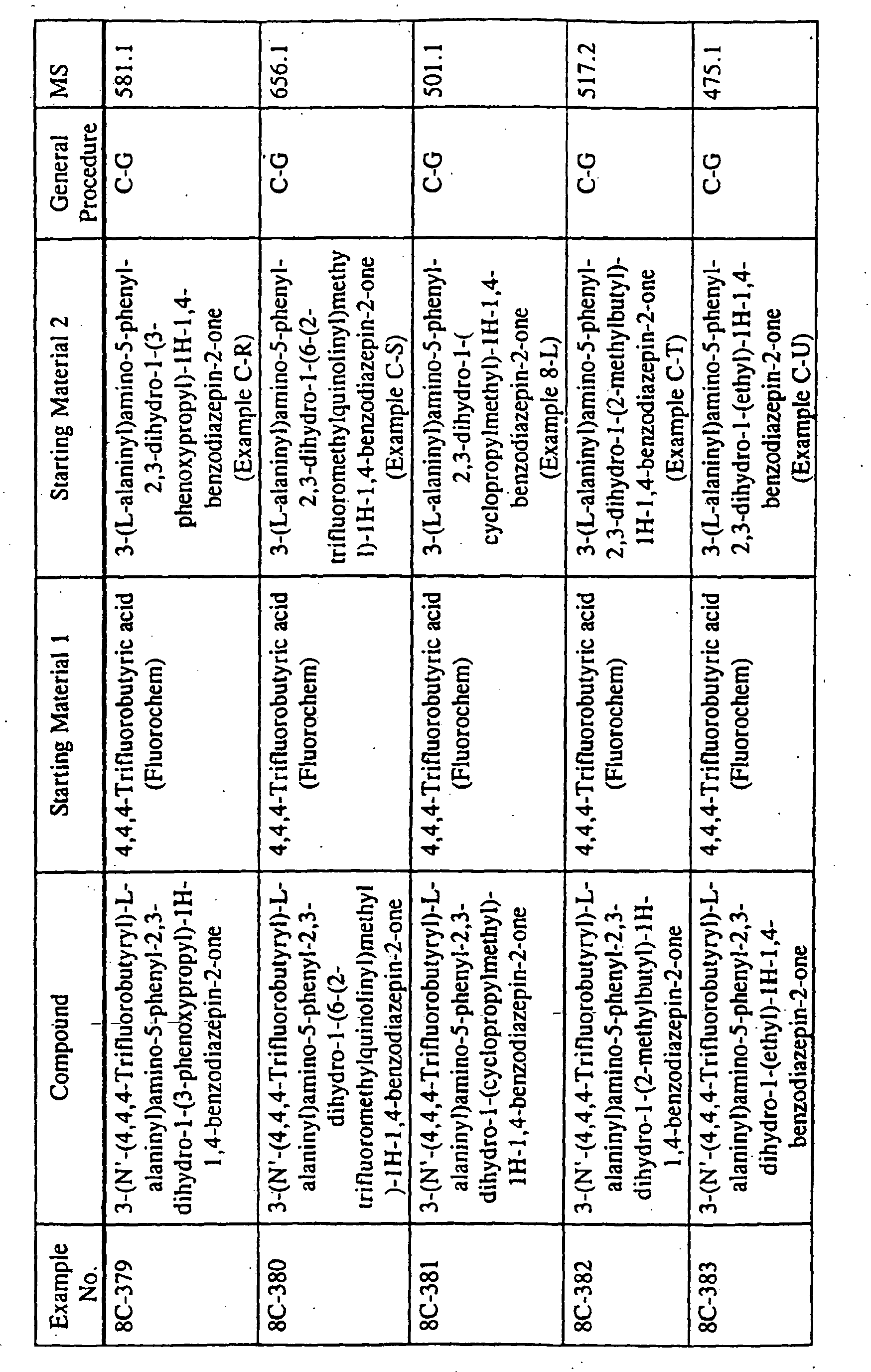

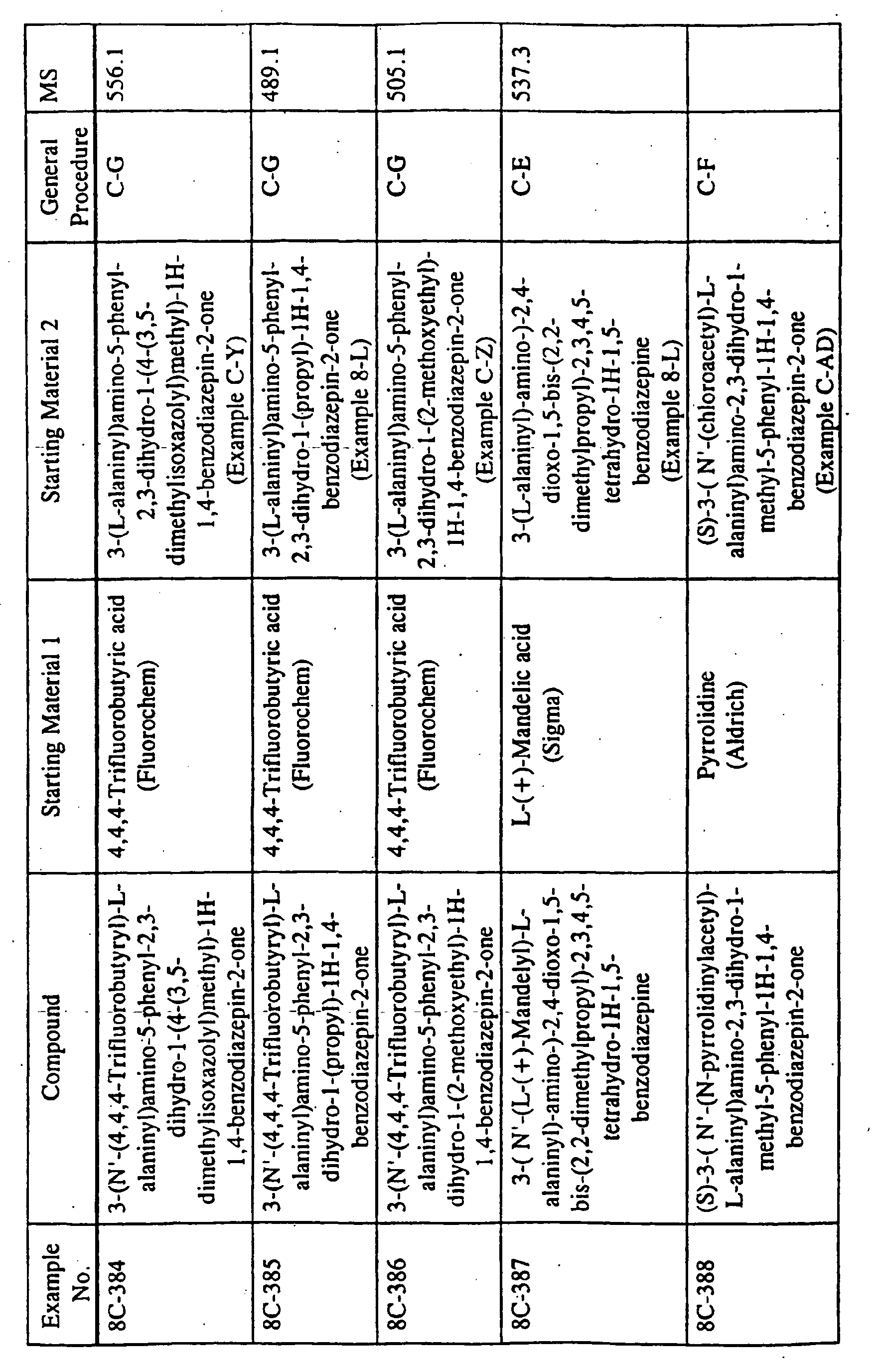

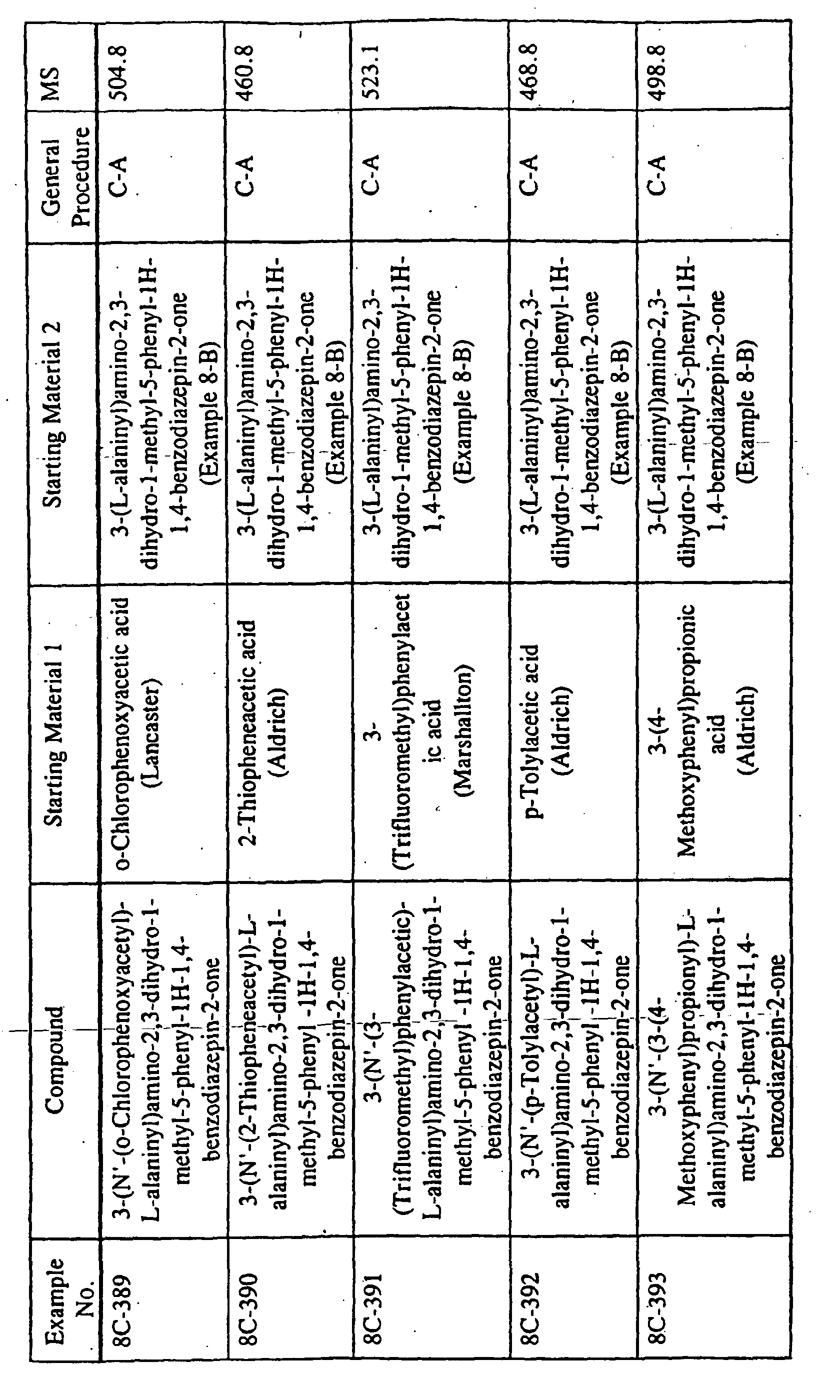

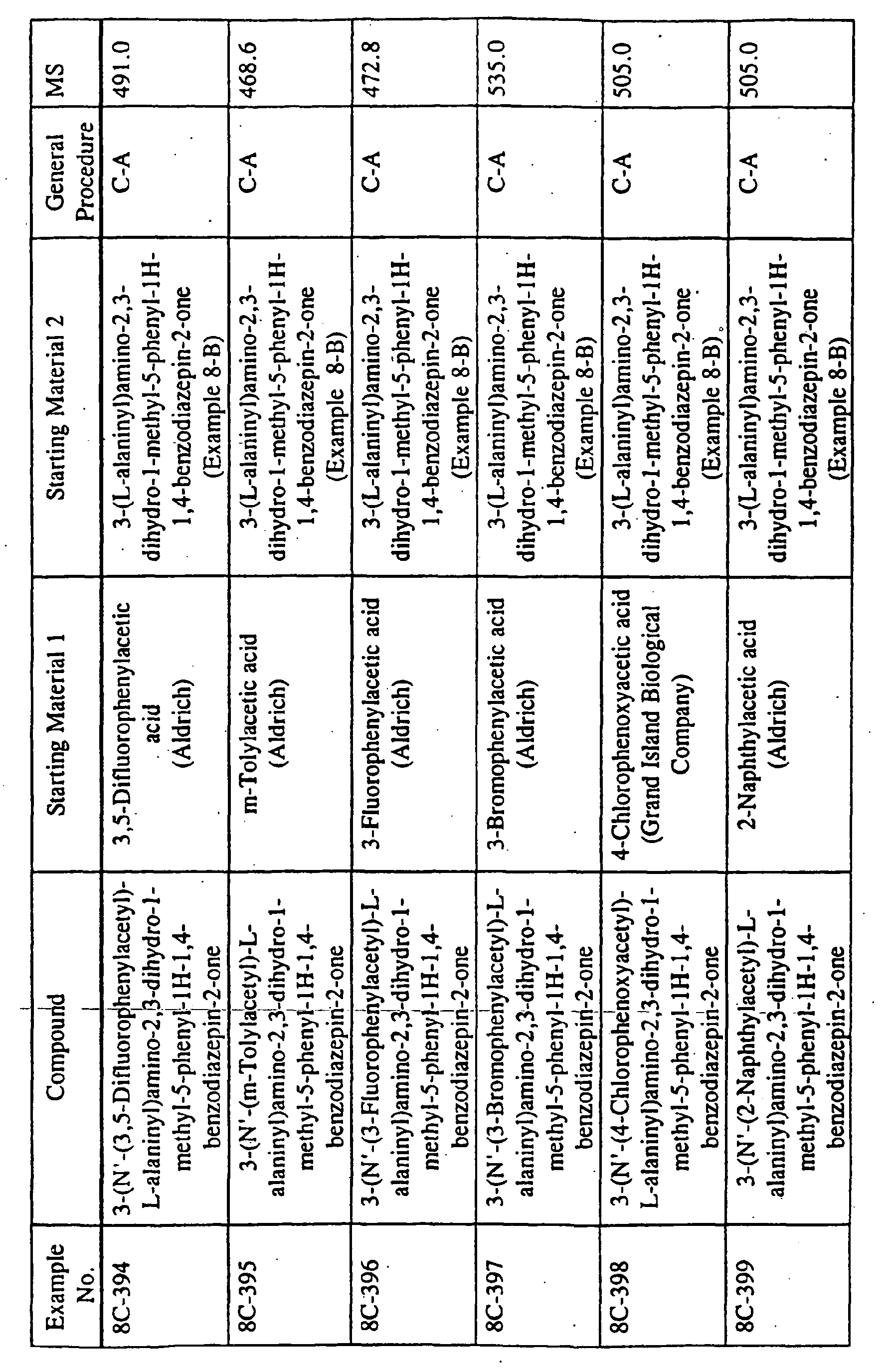

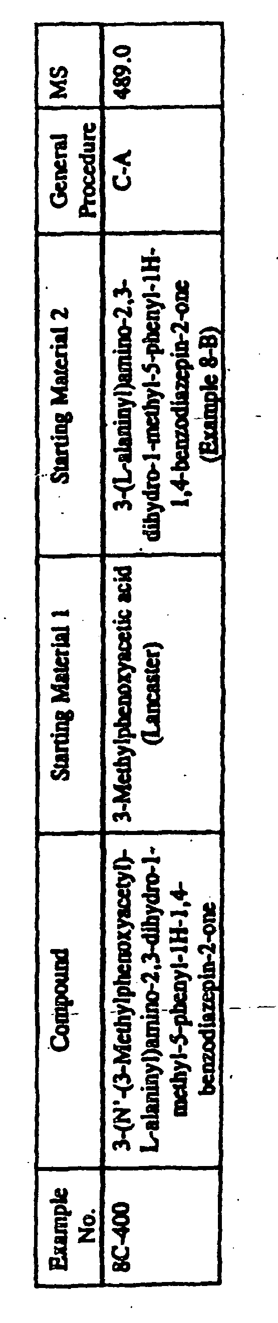

### GENERAL PROCEDURE C-P

A solution of the carboxylic acid (0.75 mL. 0.05 M in DCM) was reacted with L-alaninyl-5-amino-7-methyl-5,7-dihydro-6H-dibenz[b,d]azepin-6-one (0.75 mL, 0.06 M in DCM) (from Example 7-I), PP-HOBT (0.3 mL, 0.15 M in DMF, this reagent was used only with alpha substituted carboxylic acids), and EDC (0.3 mL, 0.15 M). The reaction was mixed for 18 hours, then purified on a Varian SCX column (500 mg column prewashed with MeOH (3 x 2.5 mL) and 20% MeOH: DCM (3 x 2.5 mL)) eluting with 2.5 mL of 20 % MeOH:DCM.

### GENERAL PROCEDURE C-Q

Step A: FMOC-Gly Wang resin (20 g, 10.8 mmole, Novabiochem A16415) was reacted with a 30% solution of piperidine in N-methylpyrrolidinone (NMP) for 30 minutes. The solution was drained and the resin washed with NMP (5 x 200 mL). Benzophenone imine (19.5 g, 108 mmole) in NMP (150 mL) was added to the resin followed by glacial acetic acid (5.6 g, 94 mmole) and the reaction was mixed overnight at room temperature. Reagents were drained and the resin washed with NMP (5 x·150 mL) followed by DCM (5 x 150 mL). The resin was dried under vacuum to afford (benzophenone imine)-Gly Wang resin with a theoretical loading of 0.56 mmole per.gram.
Step B: A suspension of the resin from Step A in NMP (9 mL) was reacted with an alkyl bromide (5.6 mL of 1 M solution ion NMP) selected from 1-bromo-2-ethylbutane, 1-bromo-3-methylbutane, cyclopropylmethyl bromide, 1-bromo-2-cyclohexylethane, 1-bromo-4-fluorobutane, and 1-bromo-2-methylbutane; and BEMP (5.6 mL of 1 M solution in NMP) and Bu₄NI (5.6 mL of 1 M solution in NMP) for 20 hours at room temperature. Reagents were drained and the resin washed with NMP (3 x 15 mL). To a mixture of the resin in THF (7 mL) was added hydroxylamine hydrochloride (2 mL of a 1.6 M solution in water) and the reaction was mixed for 20 hours at room temperature. Reagents were drained and the resin washed sequentially with THF (2 x 5 mL), 0.5 M solution of diisopropylethylamine in THF (5 mL), THF (5 mL), and NMP (3 x 5 mL).
Step C: The resin from Step B was divided into 12 equal reactions using an isopicnic solution in NMP:CH₂Cl2. To each reaction was added sequentially a carboxylic acid (0.75 mL of a 0.45 M solution in NMP), HOBT (0.75 mL of a 0.45 M solution in NMP) and DIC (0.75 mL of a 0.45 M solution in NMP). The reaction was mixed for 18 hours at room temperature. Reagents were drained and the resin washed with NMP (5 x 0.5 mL), and DCM (5 x. 0.5 mL). The resin was mixed with TFA:H₂O (95:5, 0.5 mL) for 4 hours. The filtrate was collected, resin washed with TFA:H₂O (95:5, 0.5 mL) and the filtrates combined. Solvents were evaporated to yield the N-acyl amino acid.

### GENERAL PROCEDURE C-R

Various acylated amino acids (approximately 0.02 mmole) (from General Procedure C-Q) in separate vials were reacted with 5-amino-7-methyl-5,7-dihydro-6H-dibenz[bd]azepin-6-one (0.1 mL, 0.3 M in DCM) (Example 7-A), PP-HOBT (0.2 mL, 0.15 M in DMF), and EDC-HCl (0.4 mL, 0.08 M in DCM). Reactions were mixed for 18 hours at room temperature. Reactions were diluted with 0.5 mL MeOH, loaded onto a Varian SCX column (500 mg, Varian Sample Preparations, pre-washed with MeOH (2.5 mL) and 10% MeOH:CHCl₃ (2.5 mL)), and eluted with 10% MeOH:CHCl₃ (2.5 mL). Solvents were evaporated from the products and the crude products purified by semi-prep reverse phase chromatography (gradient 0 to 100 %, 0.1 % TFA in H,O to 0.08 % TFA in CH₃CN). The correct molecular ion was detected for each product by ionspray mass spec and analytical reverse phase chromatography (gradient 0 to 100 %, 0.01 % TFA in H₂O to 0.08% TFA in CH₃CN) showed the products to be greater than 90% pure.

Using the procedures indicated, the compounds shown in Table C-4 were prepared. In this table, starting material 2 was prepared as described in Example 7-I.

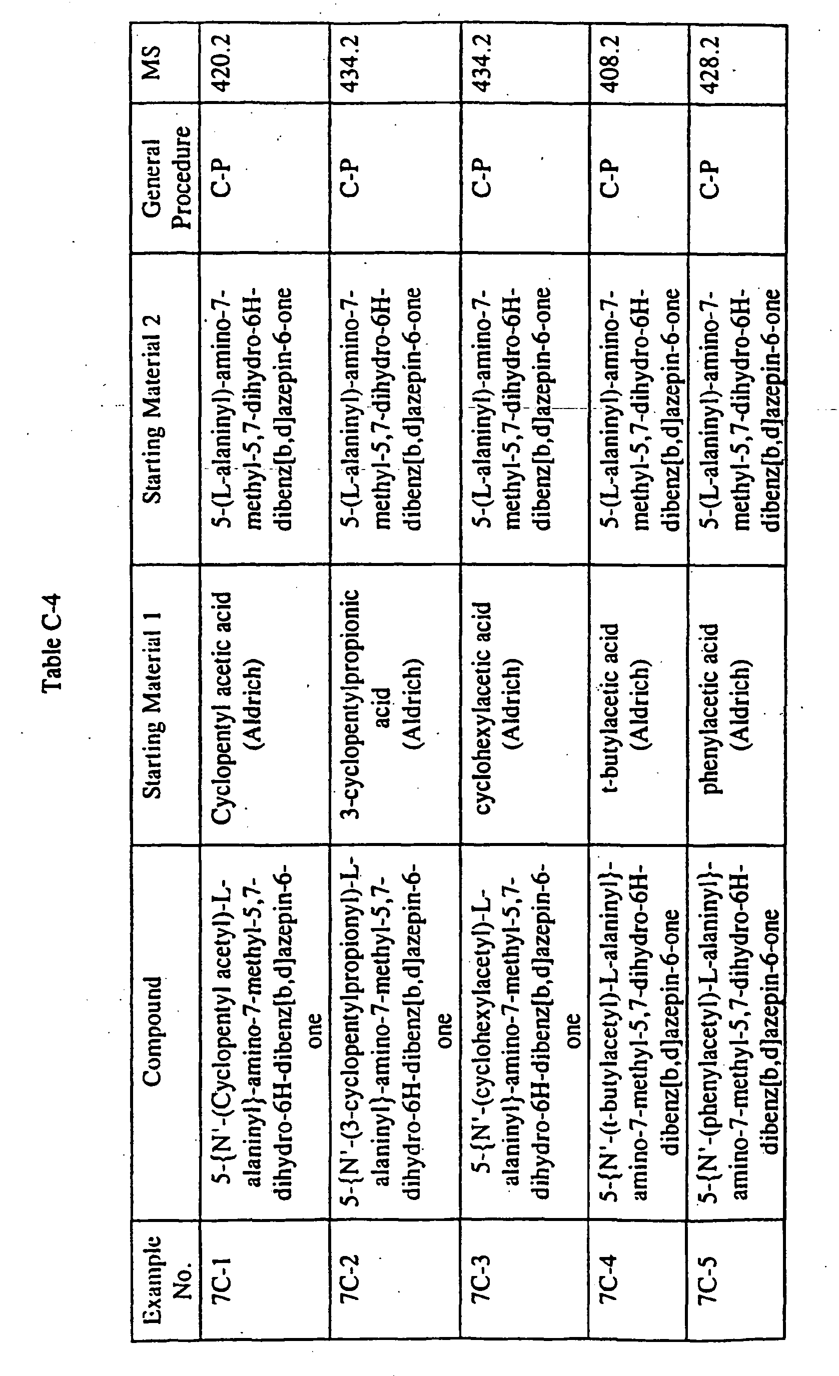

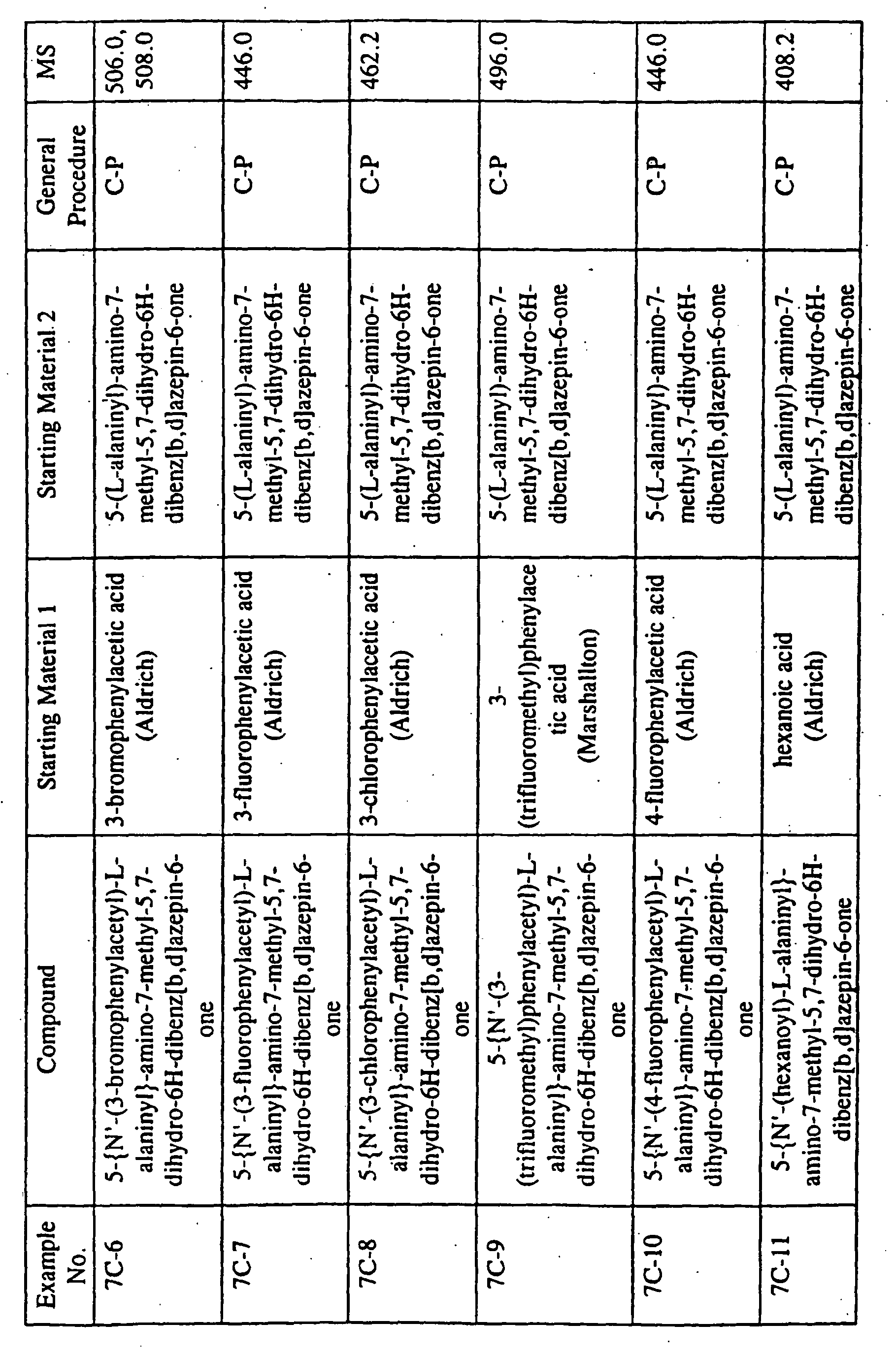

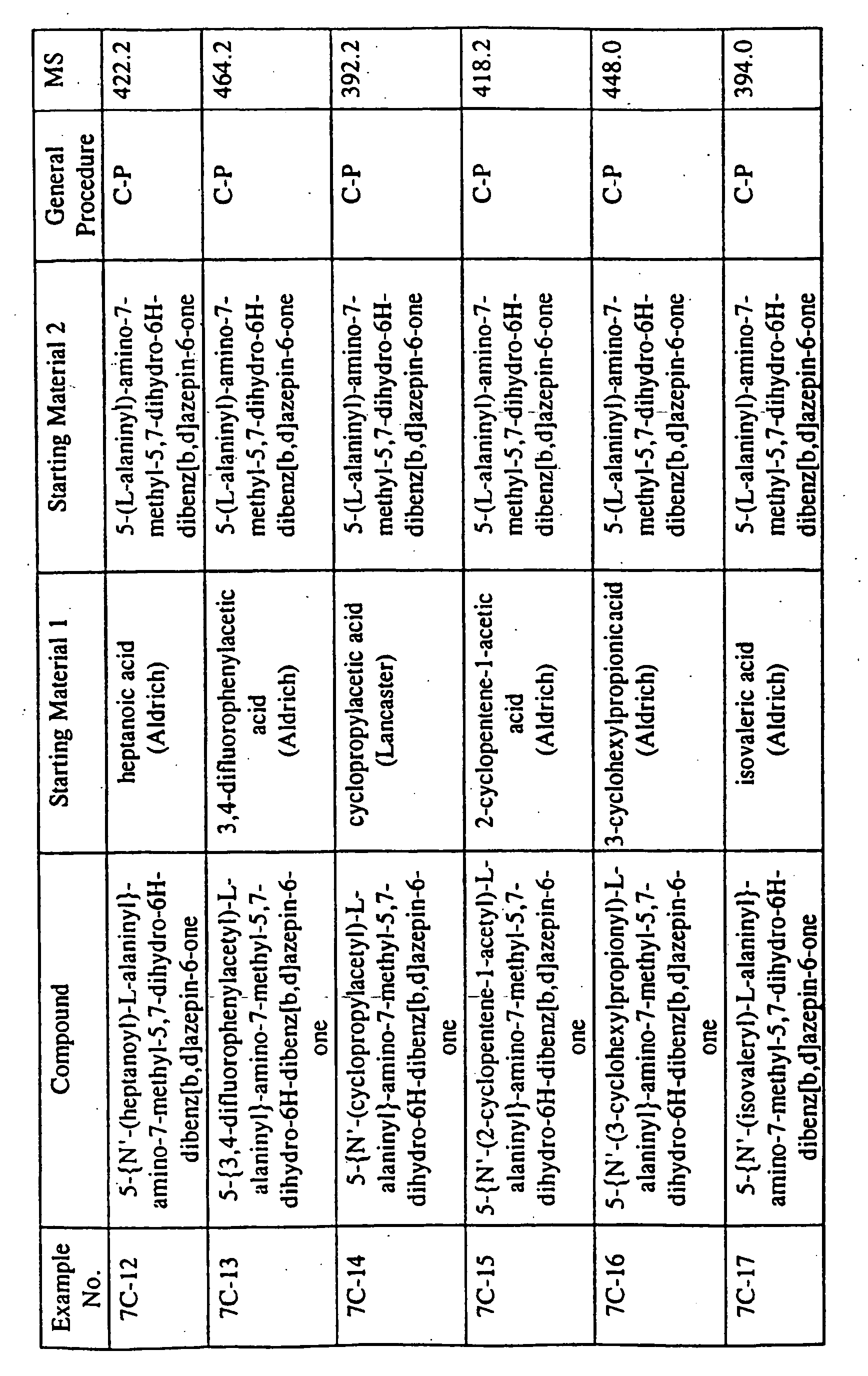

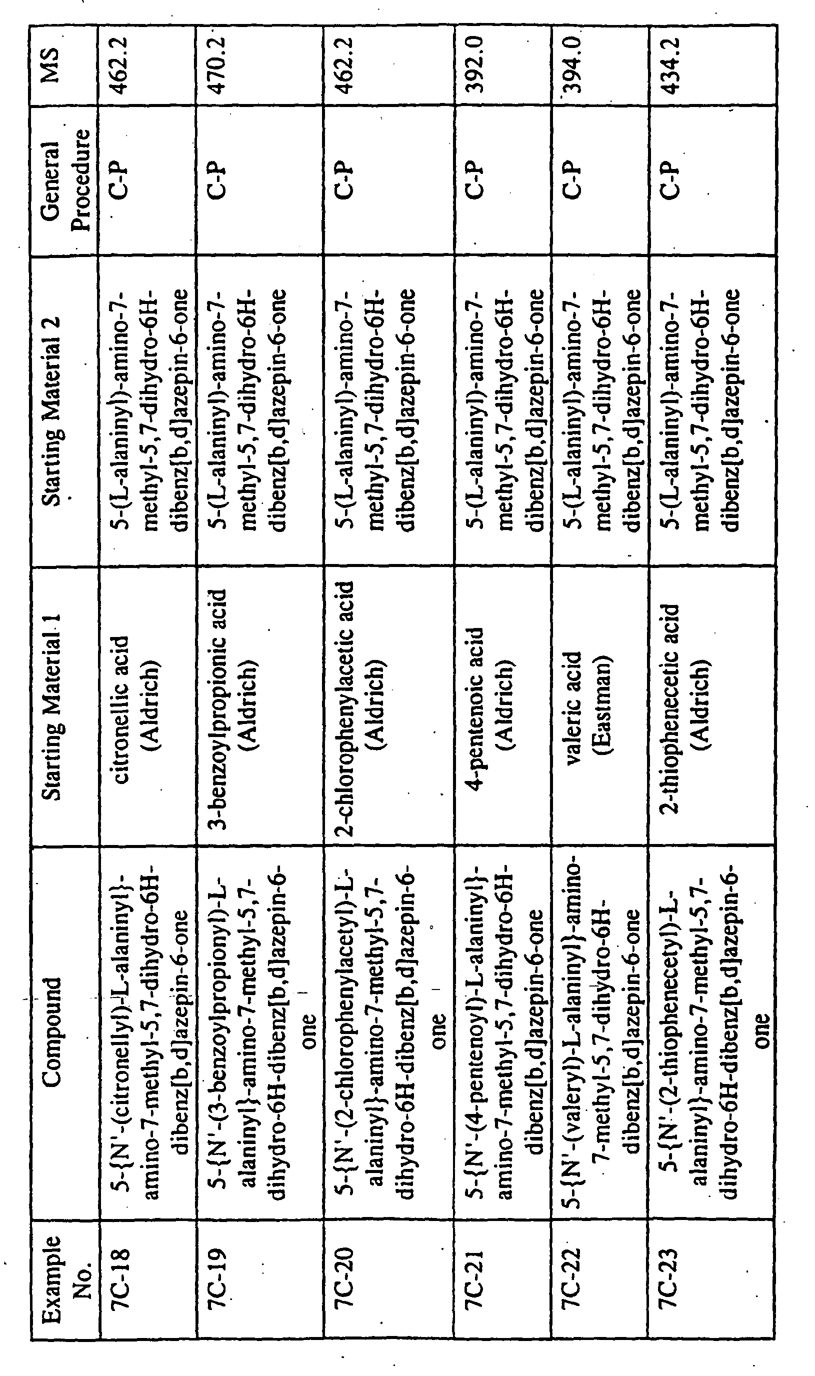

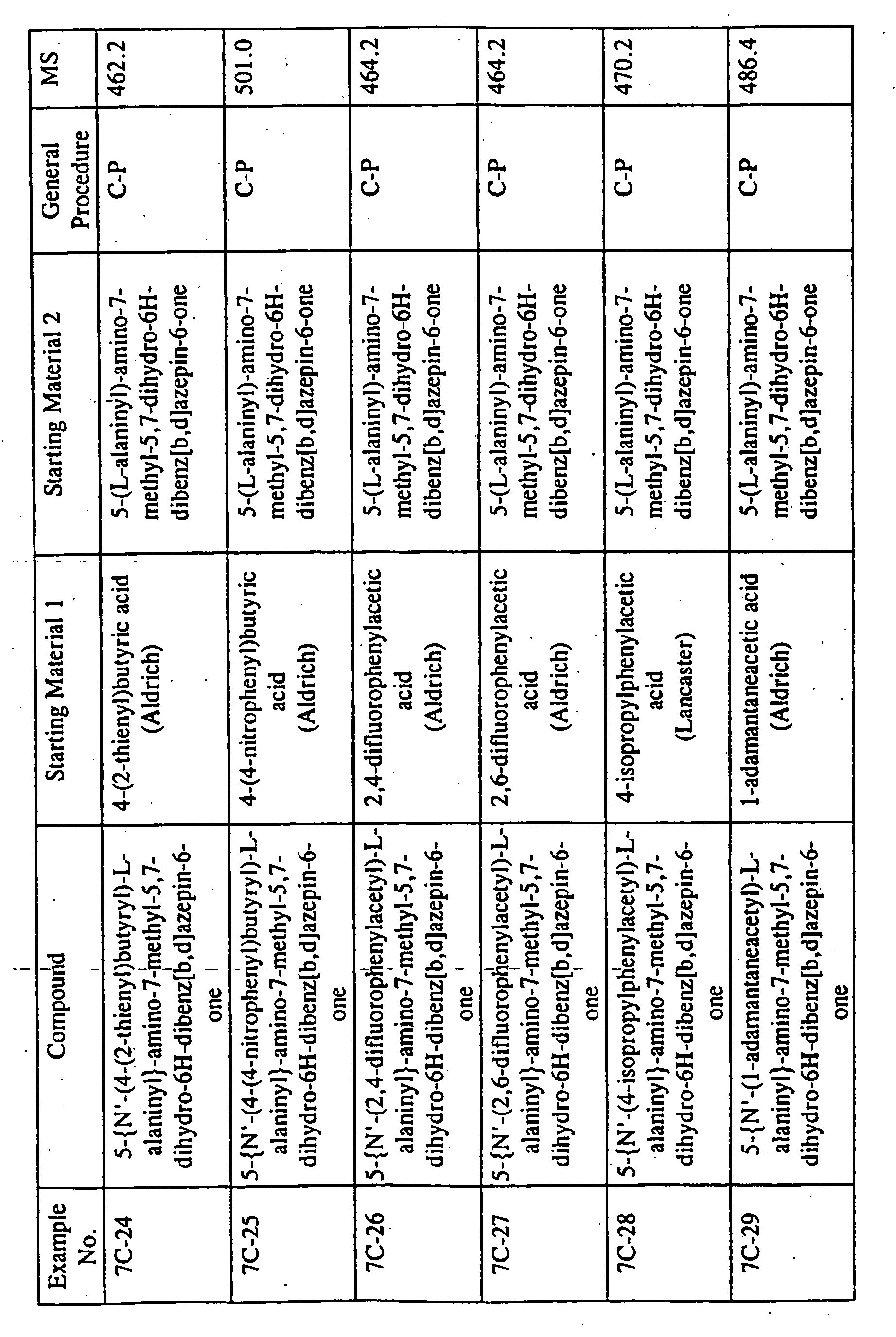

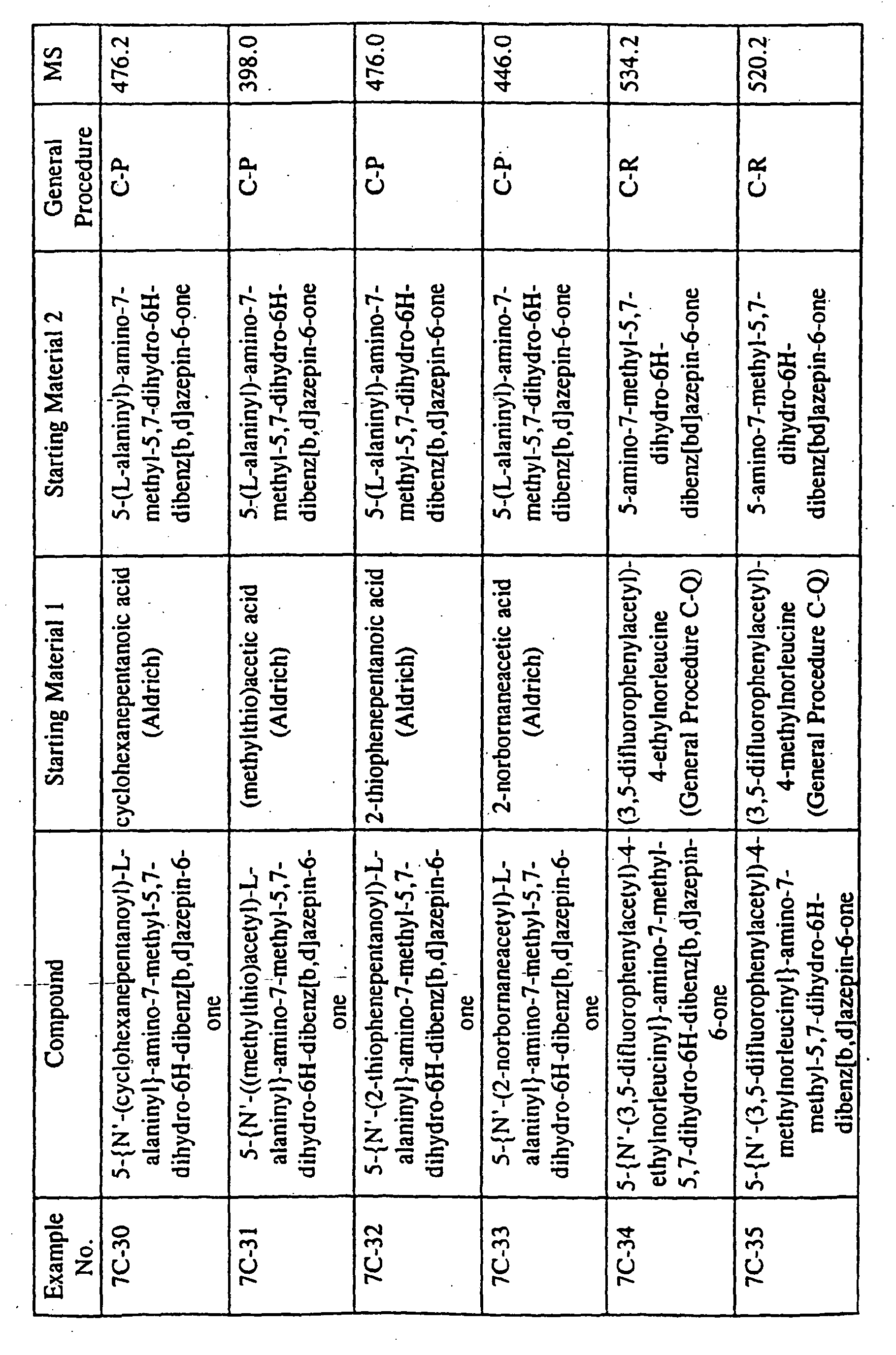

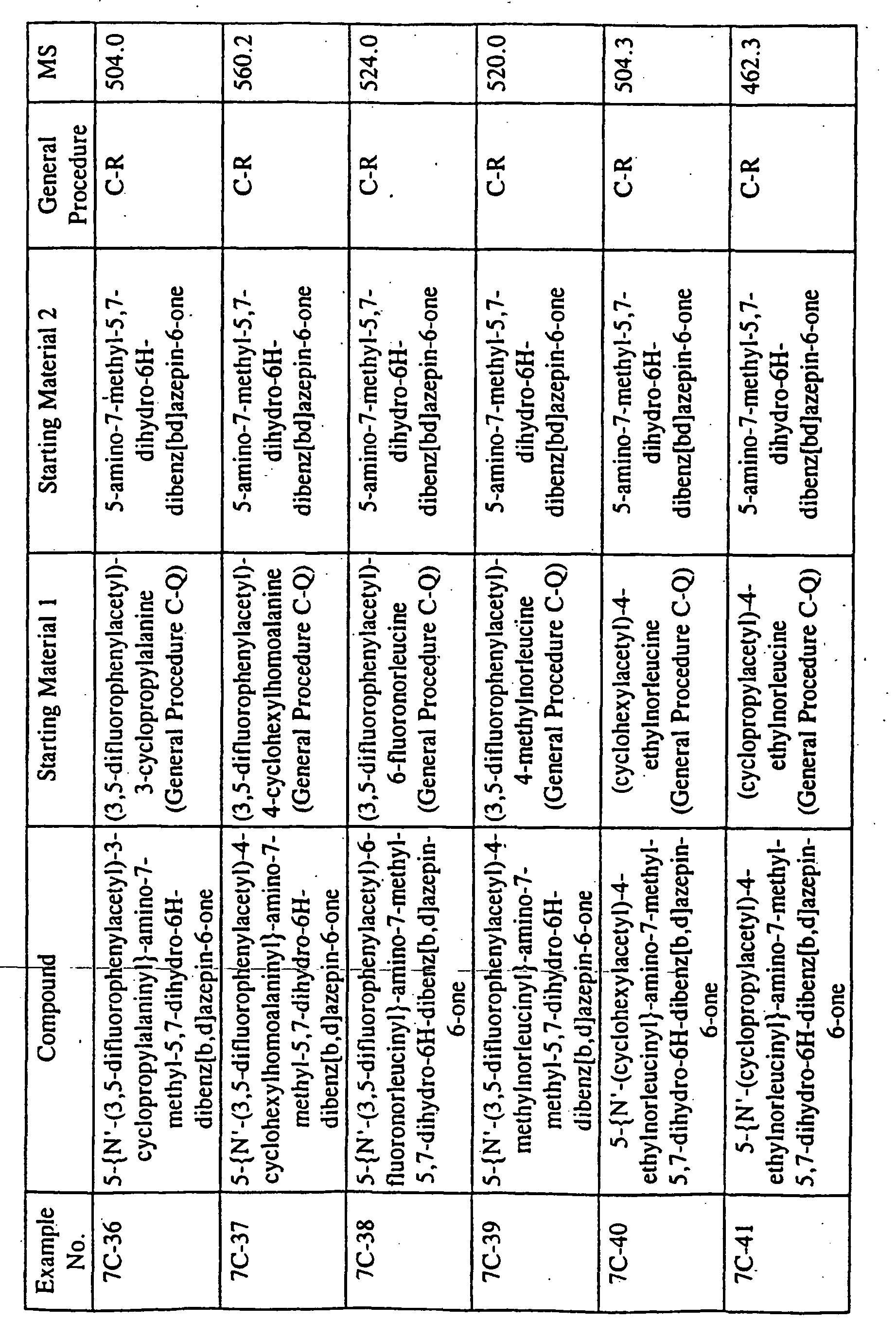

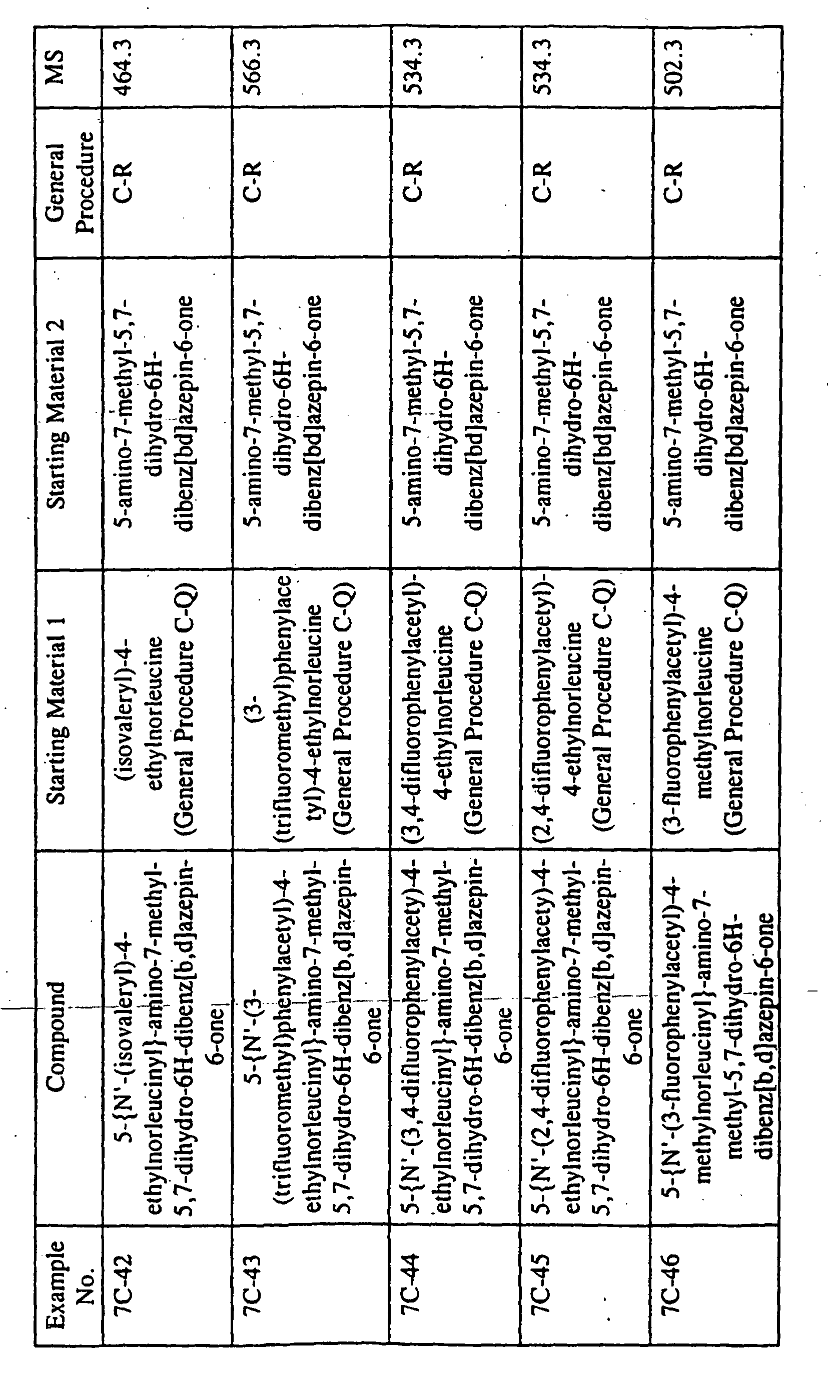

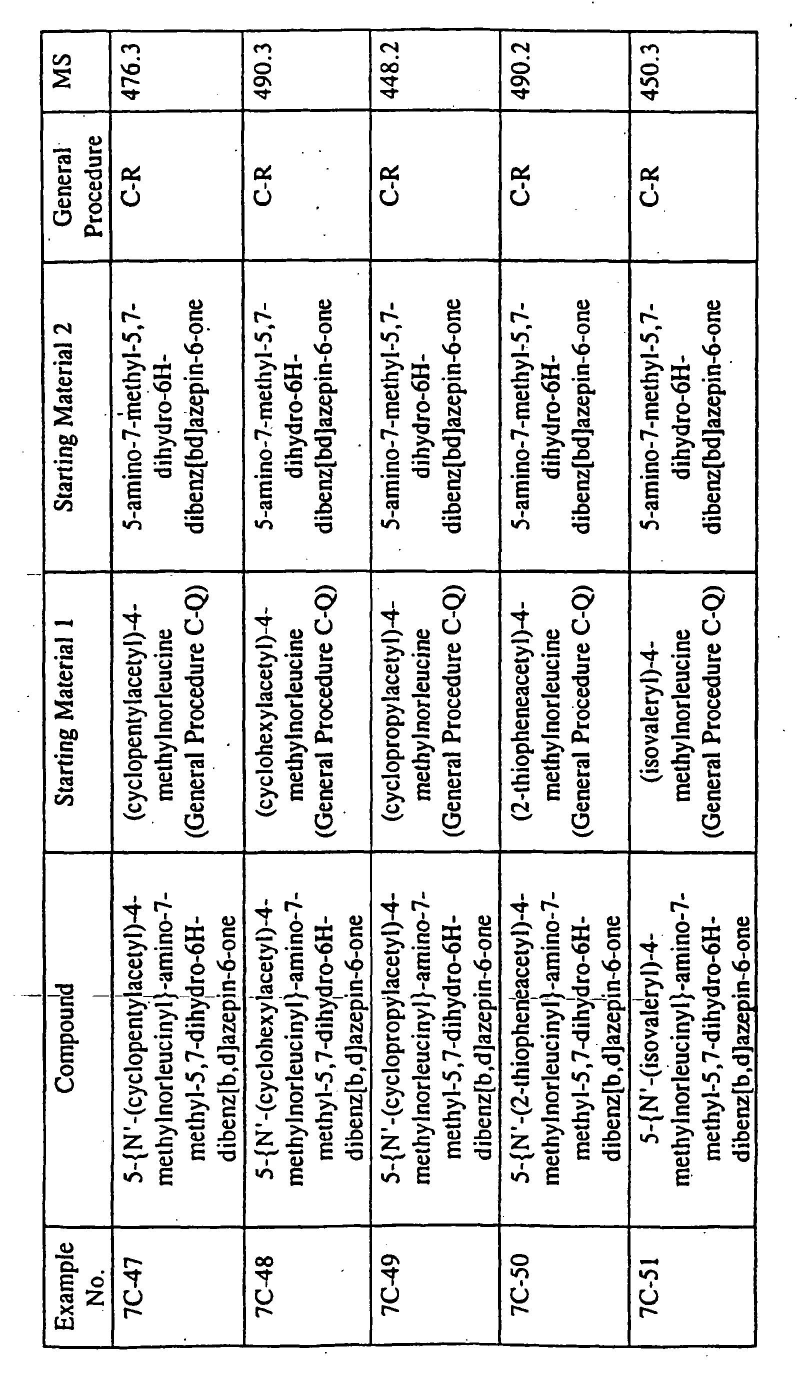

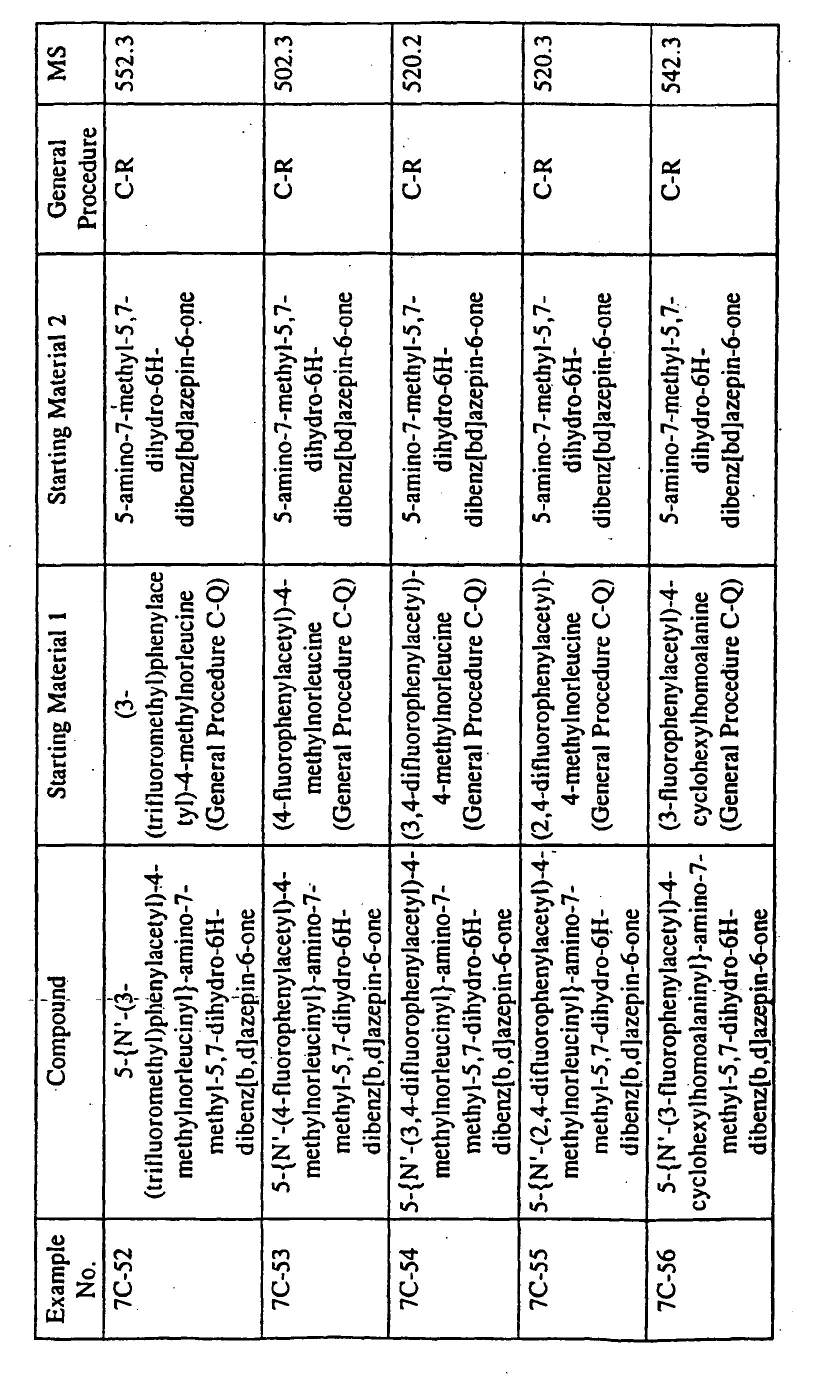

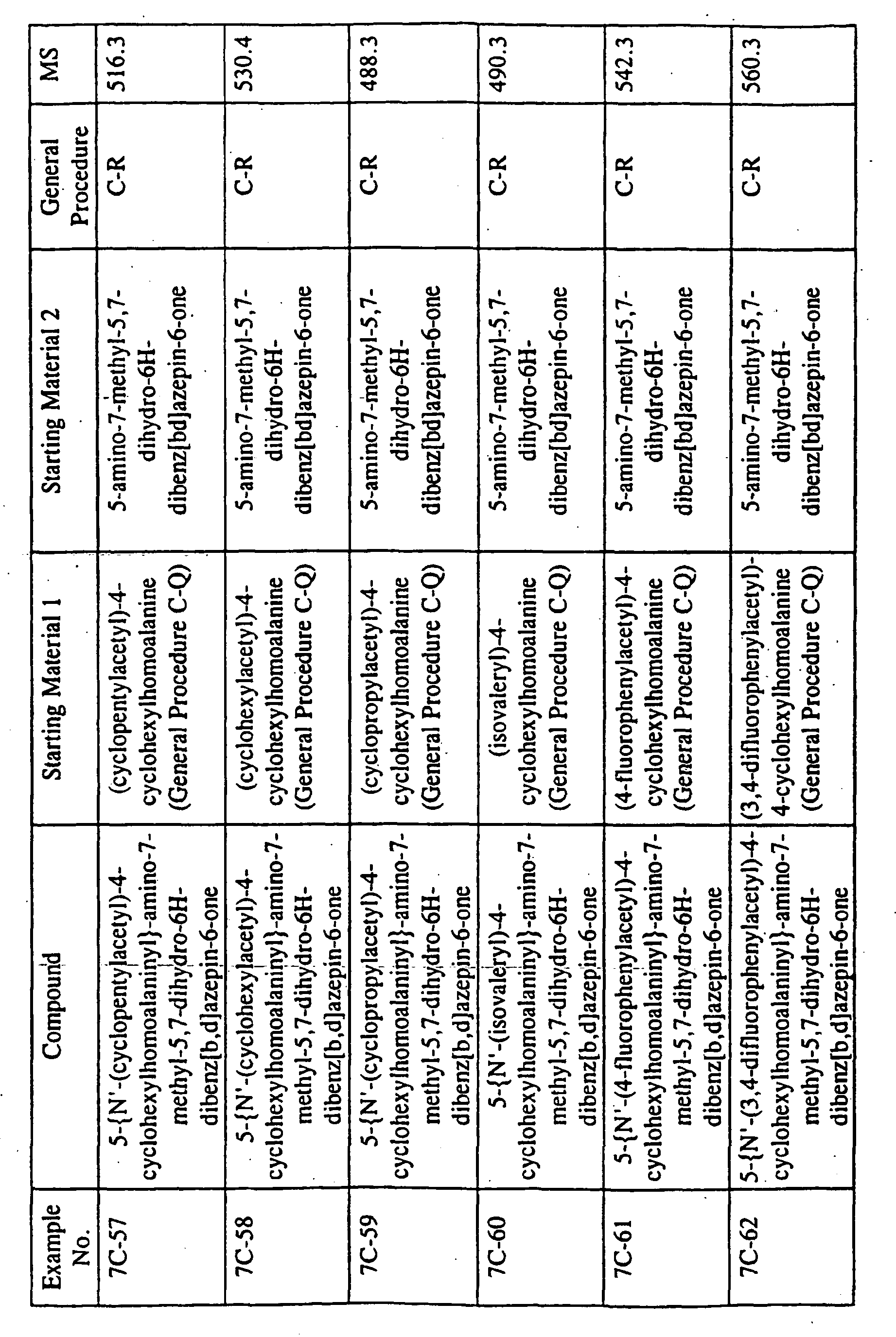

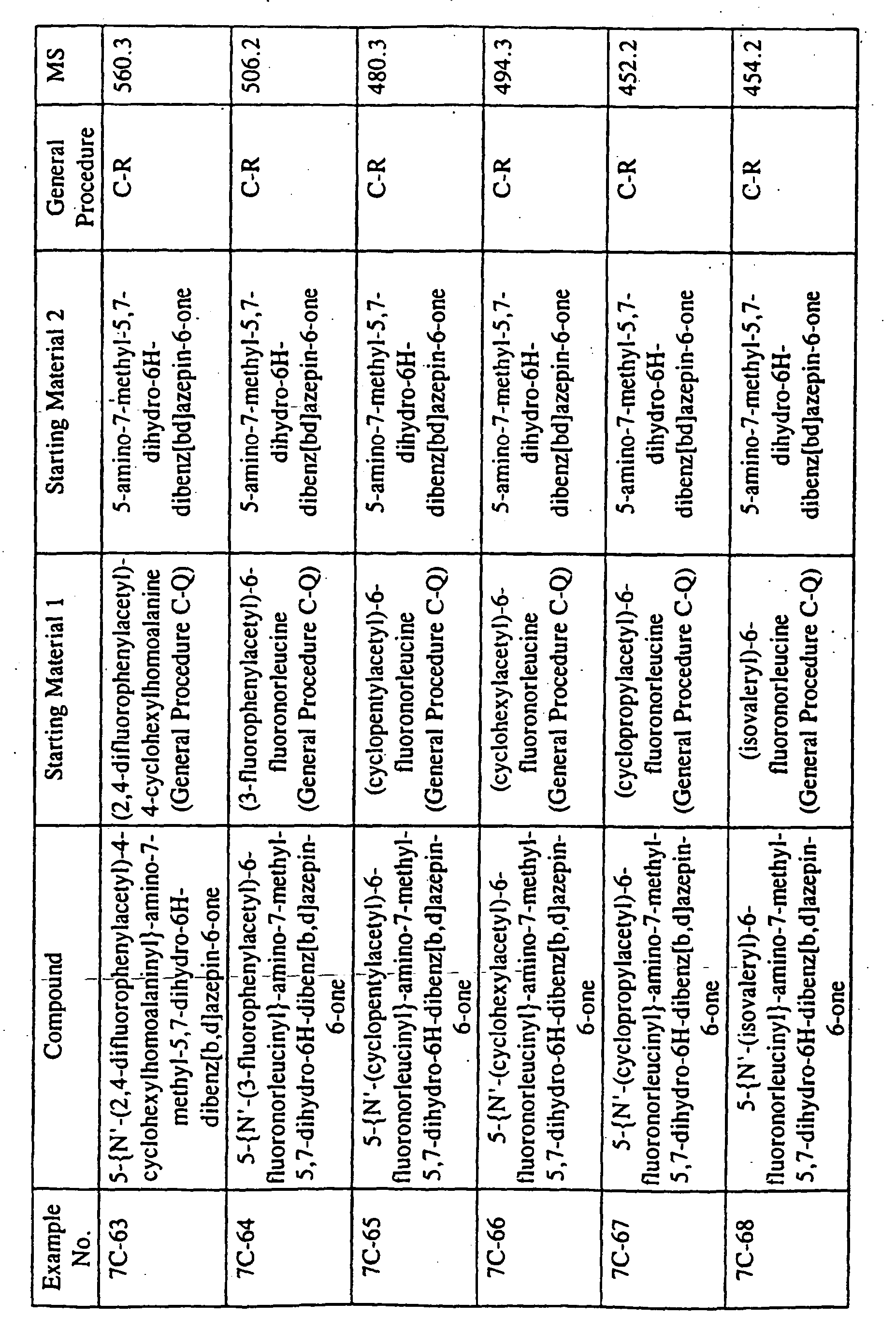

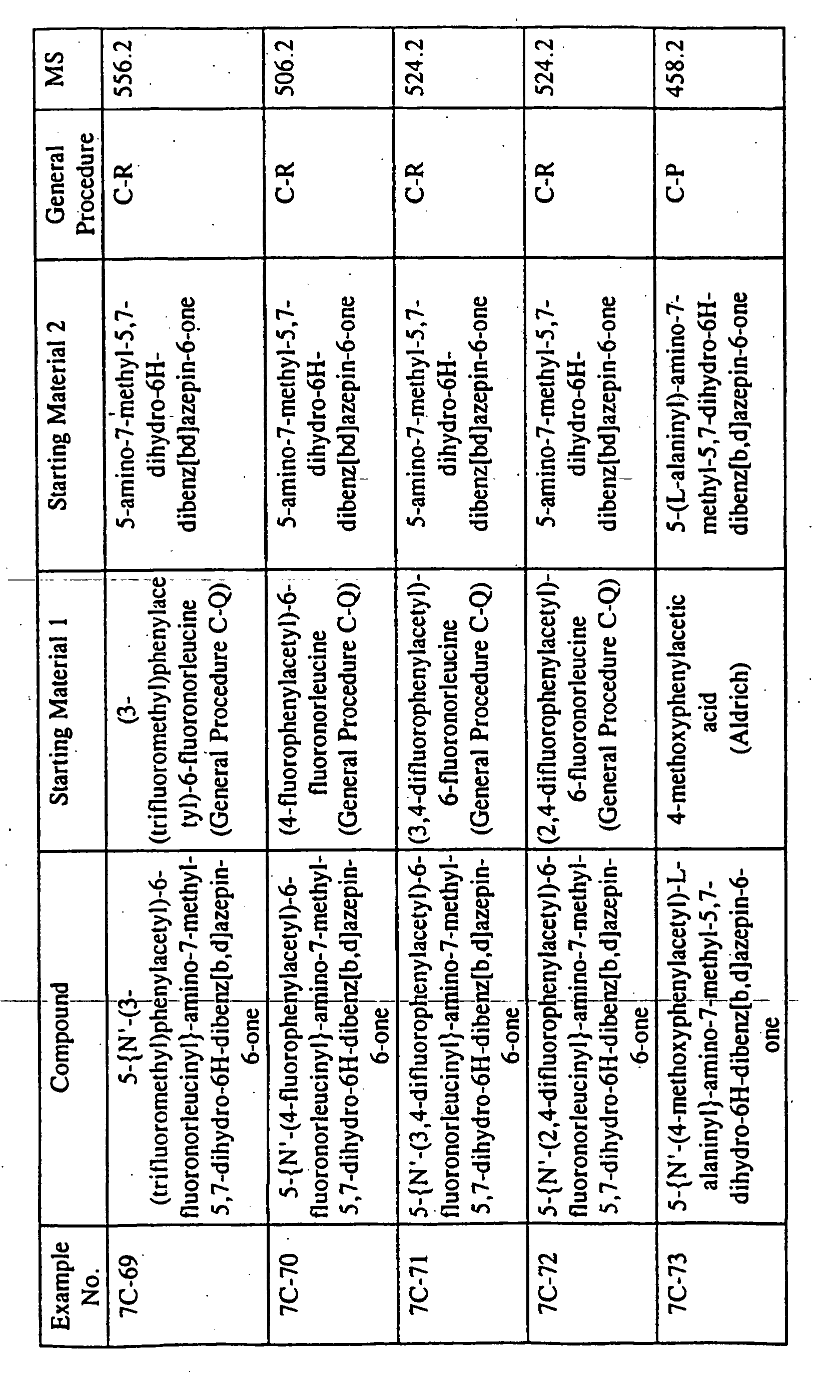

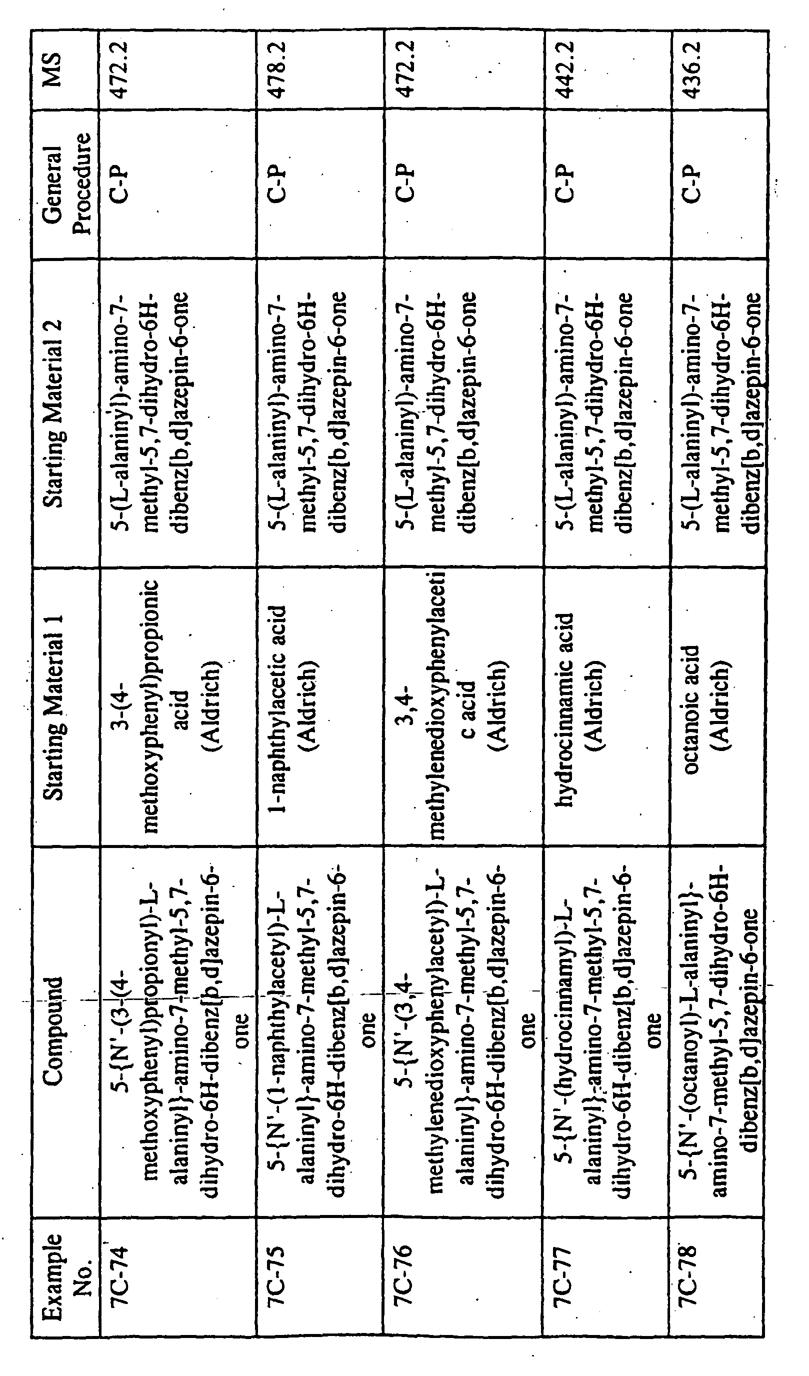

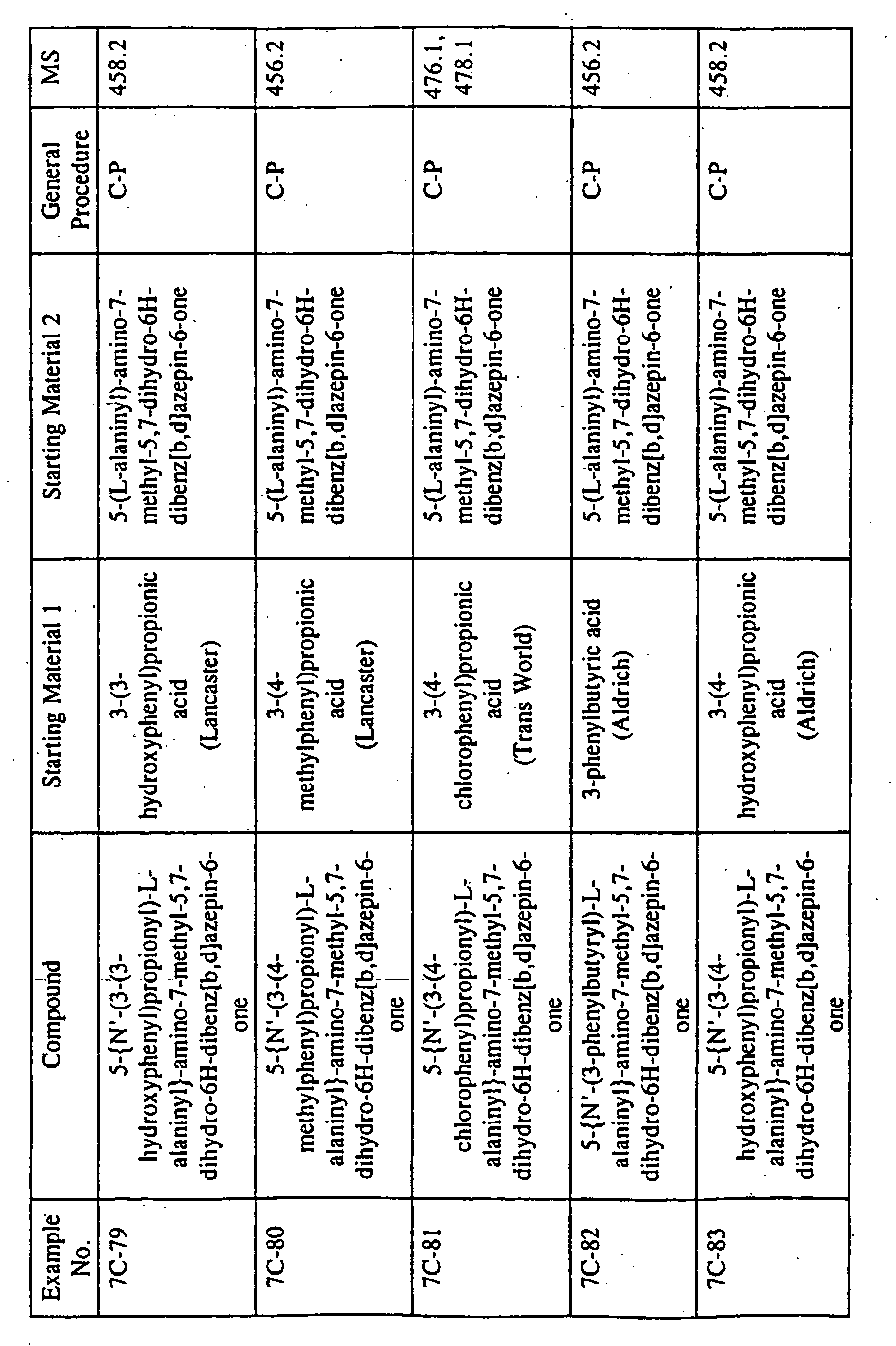

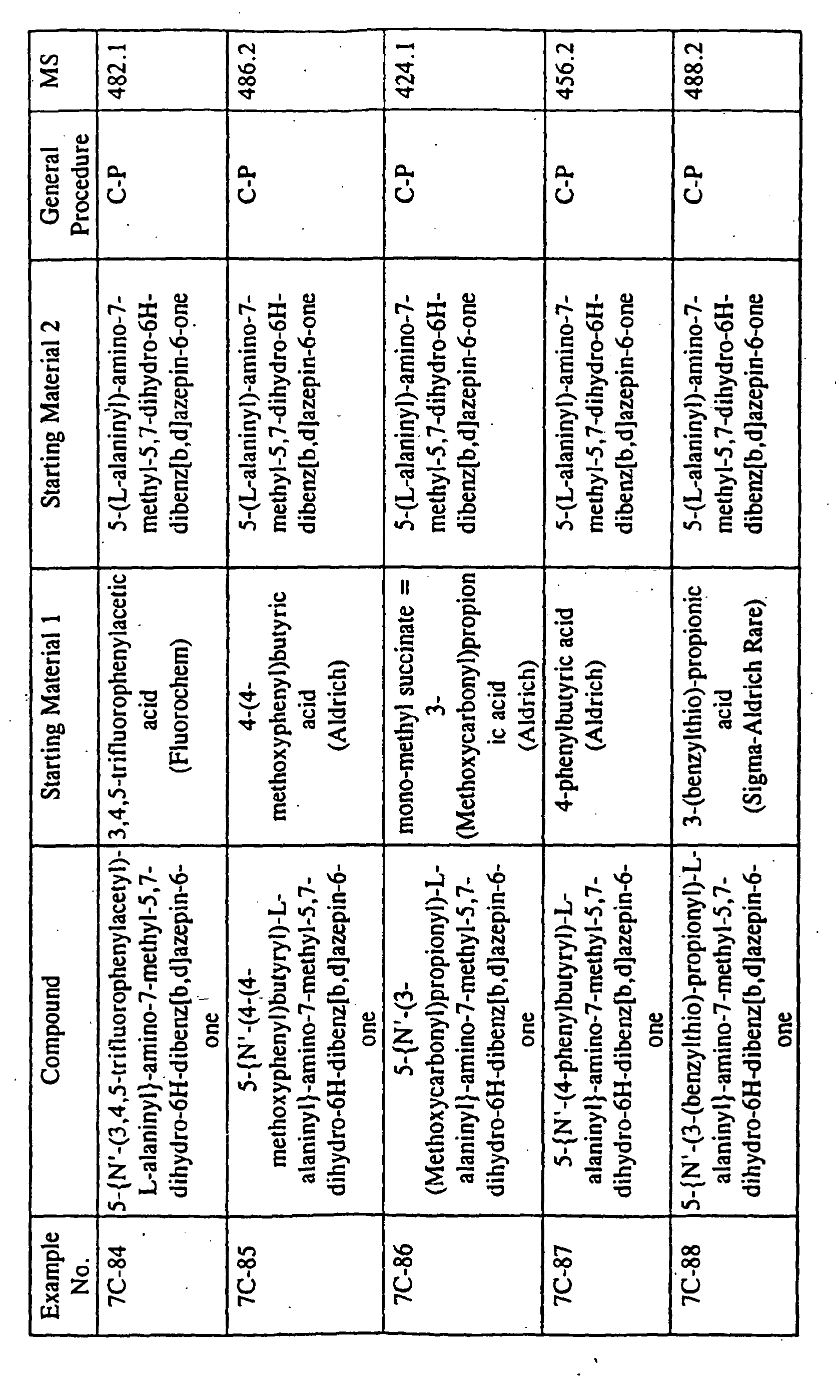

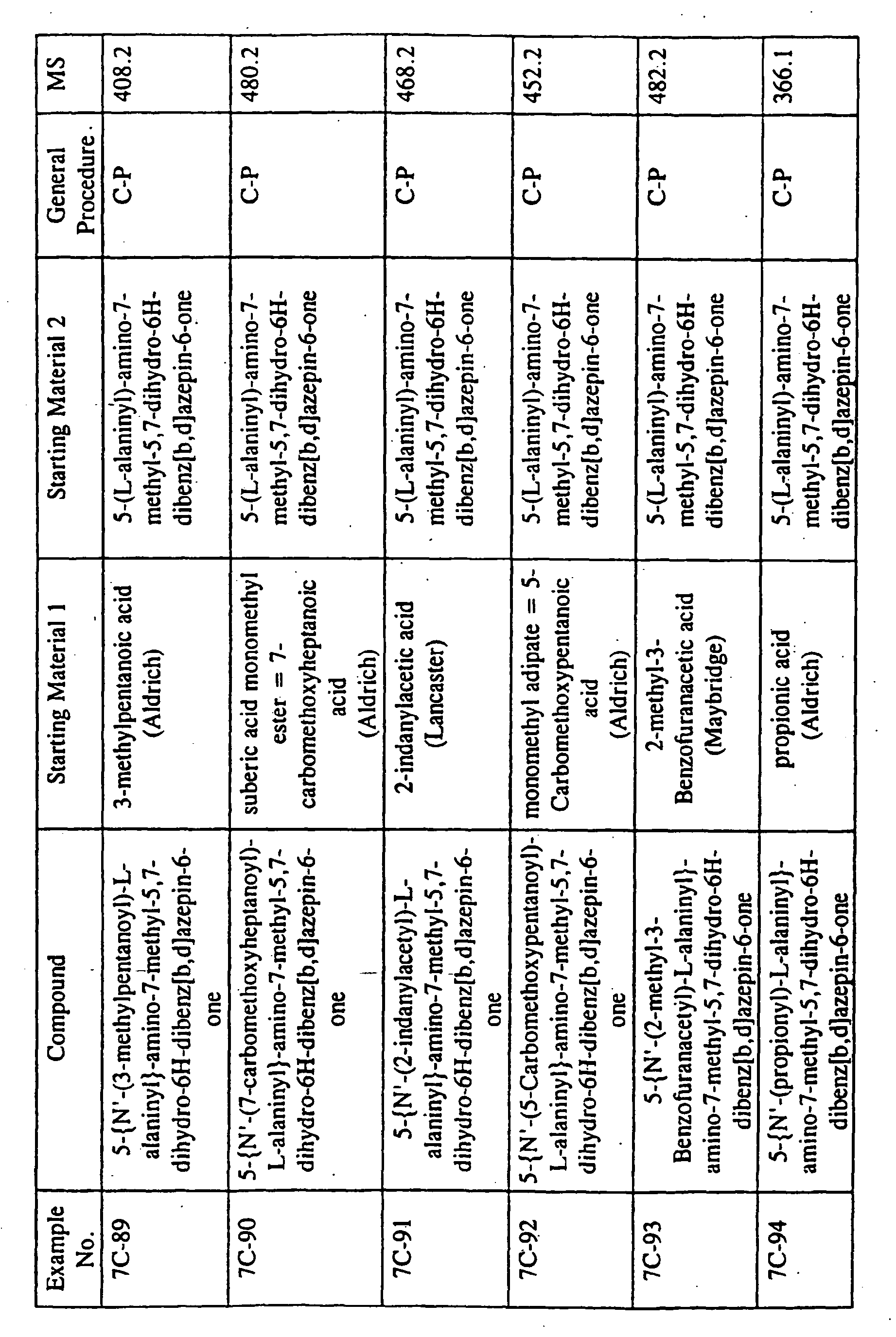

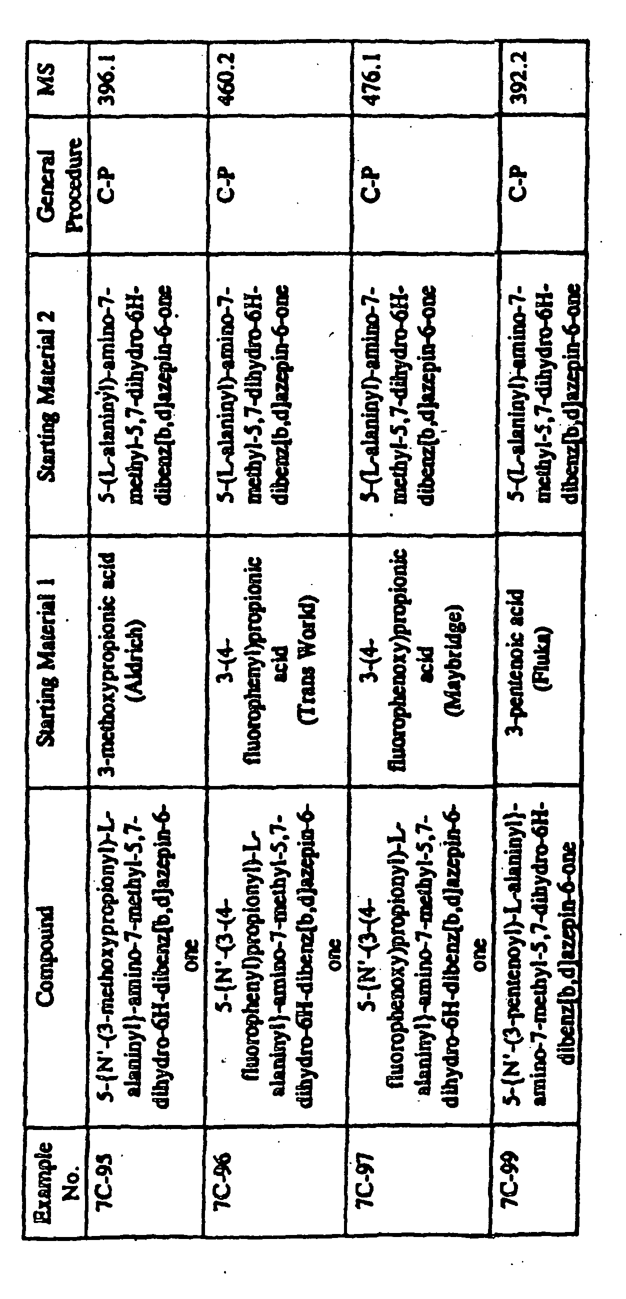

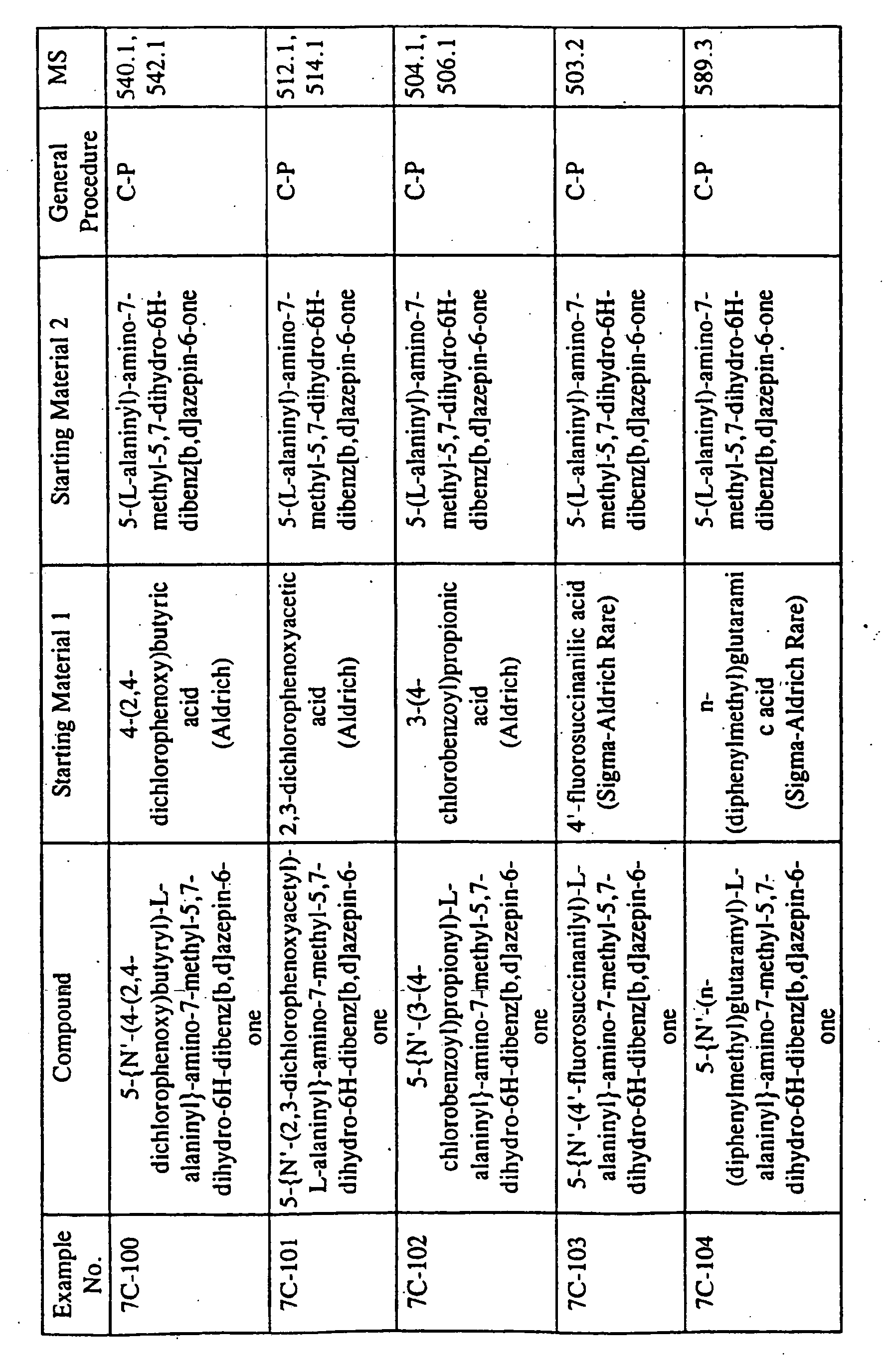

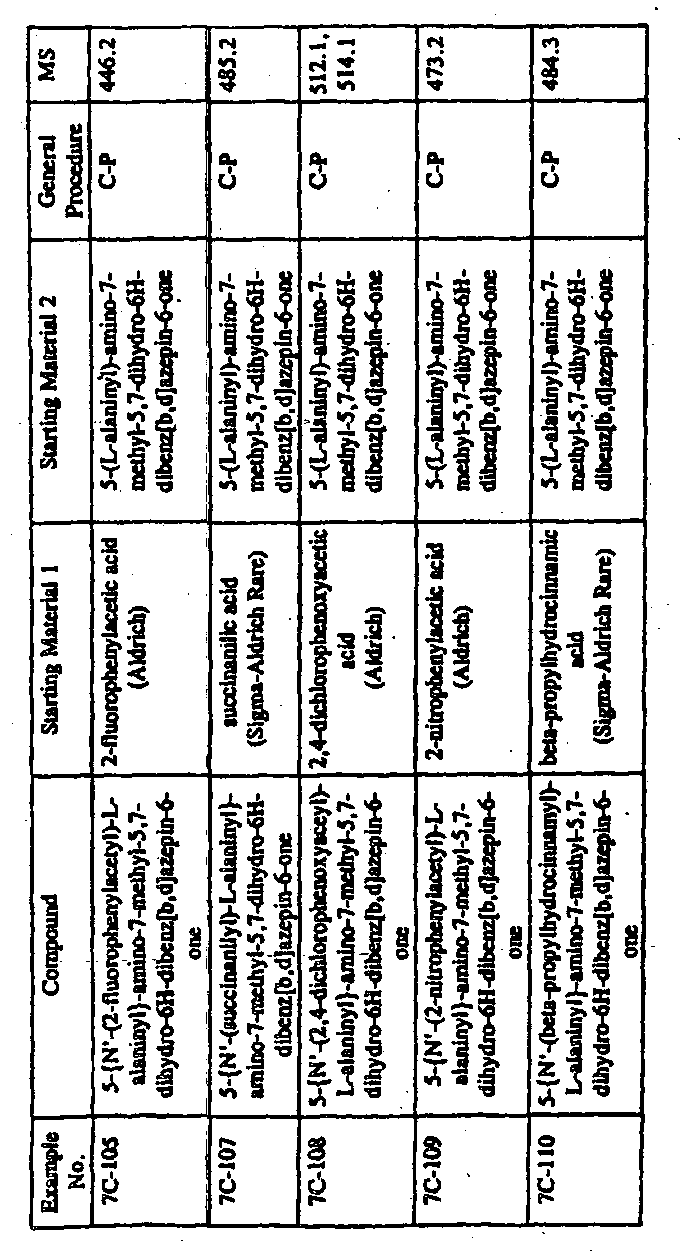

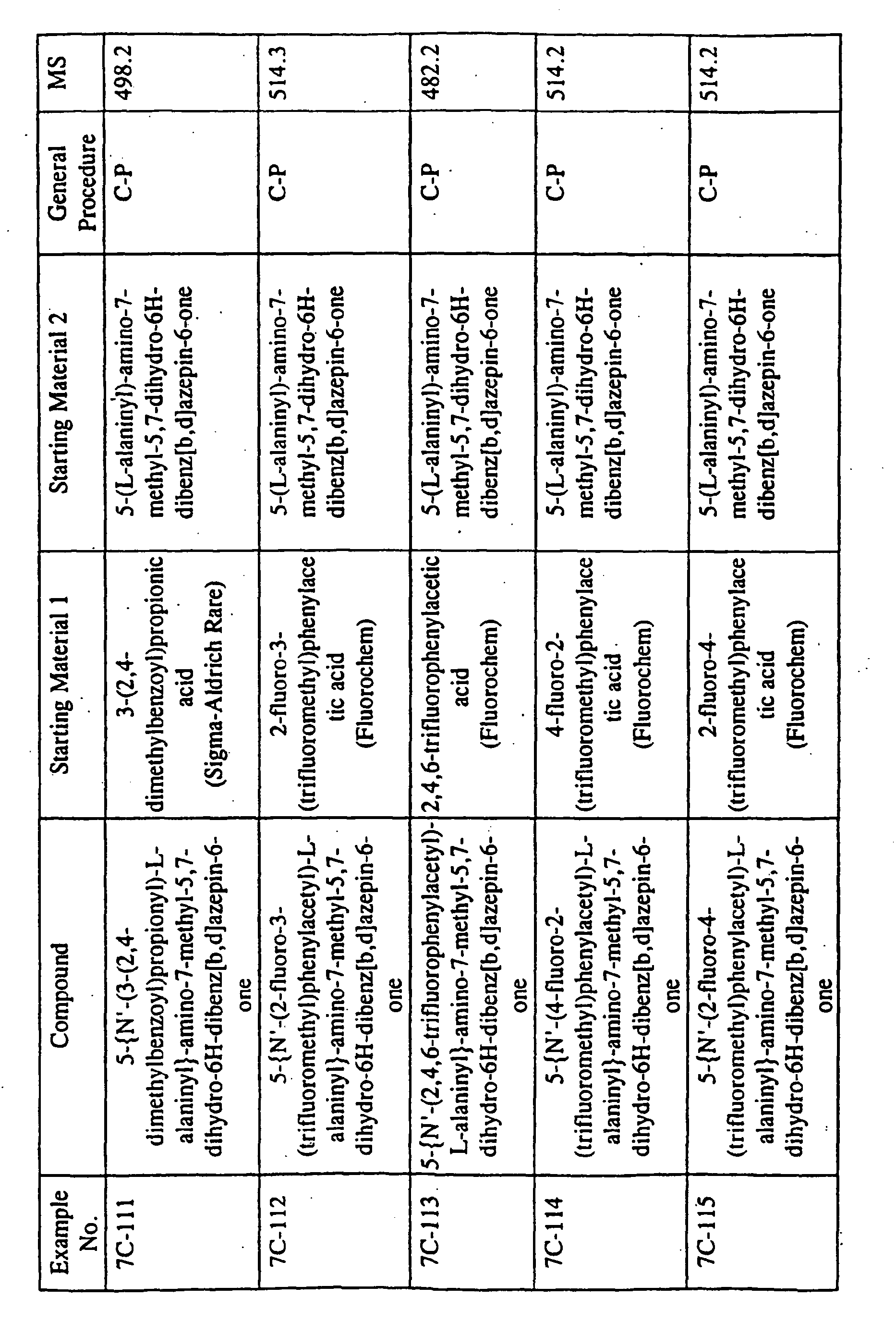

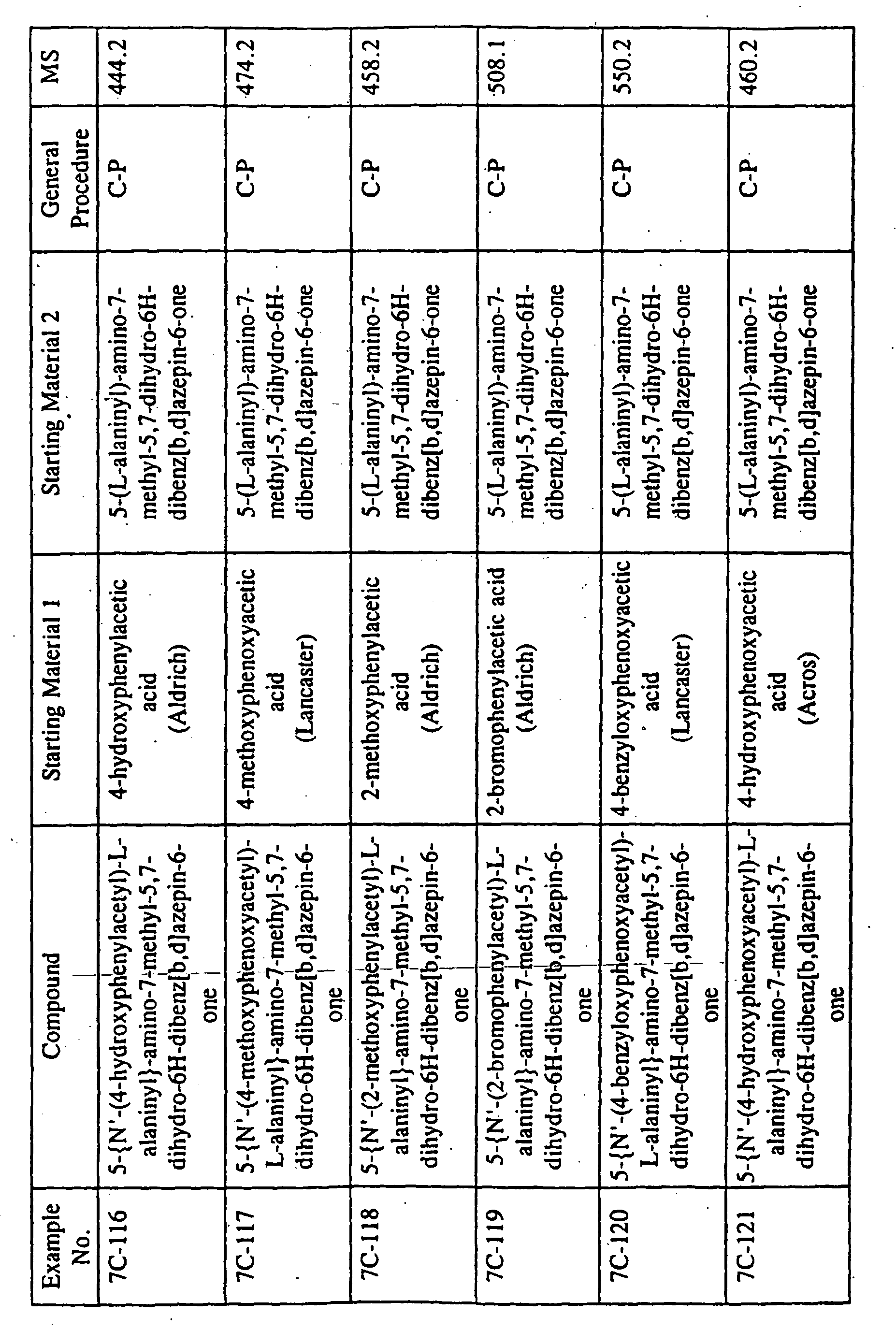

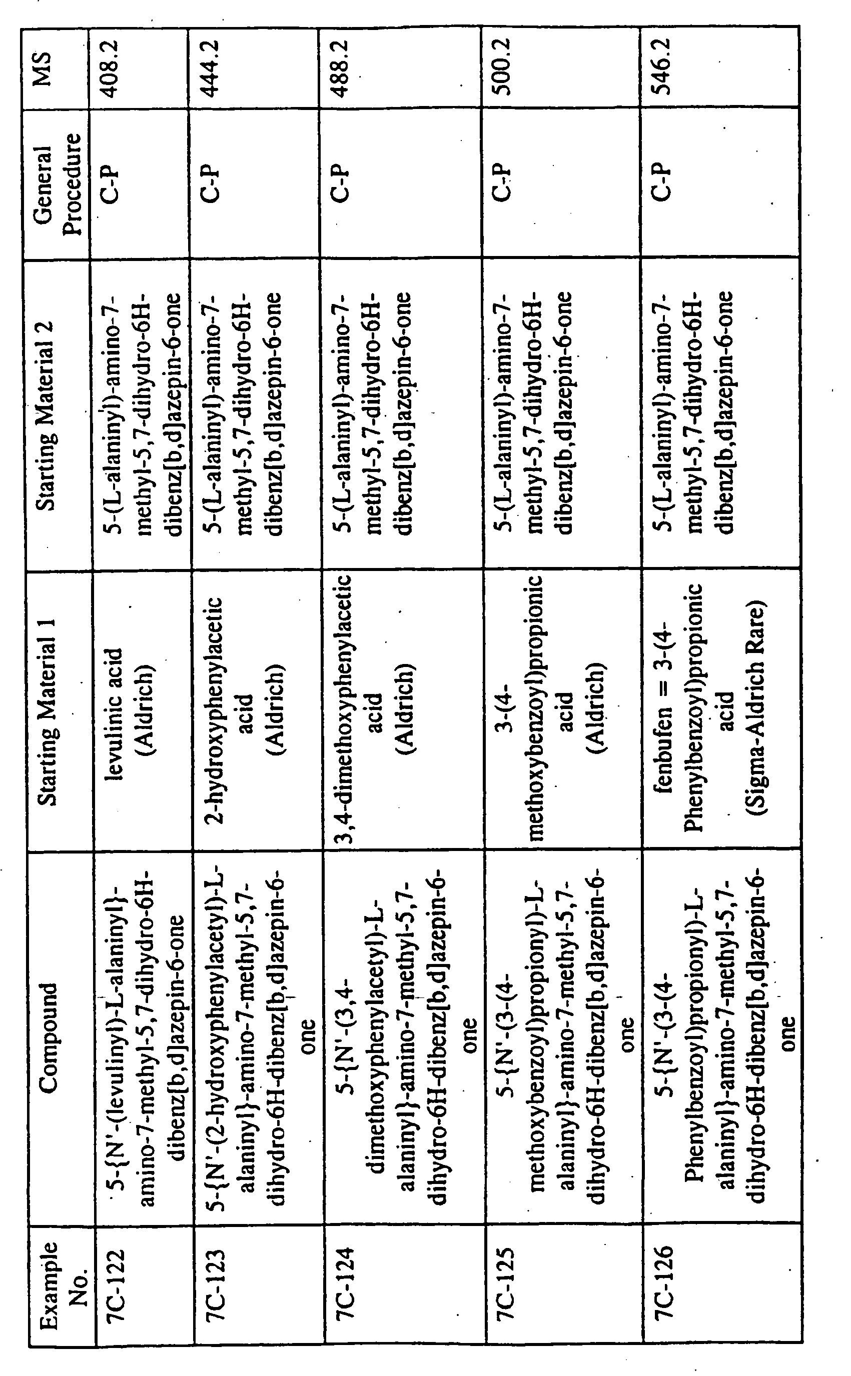

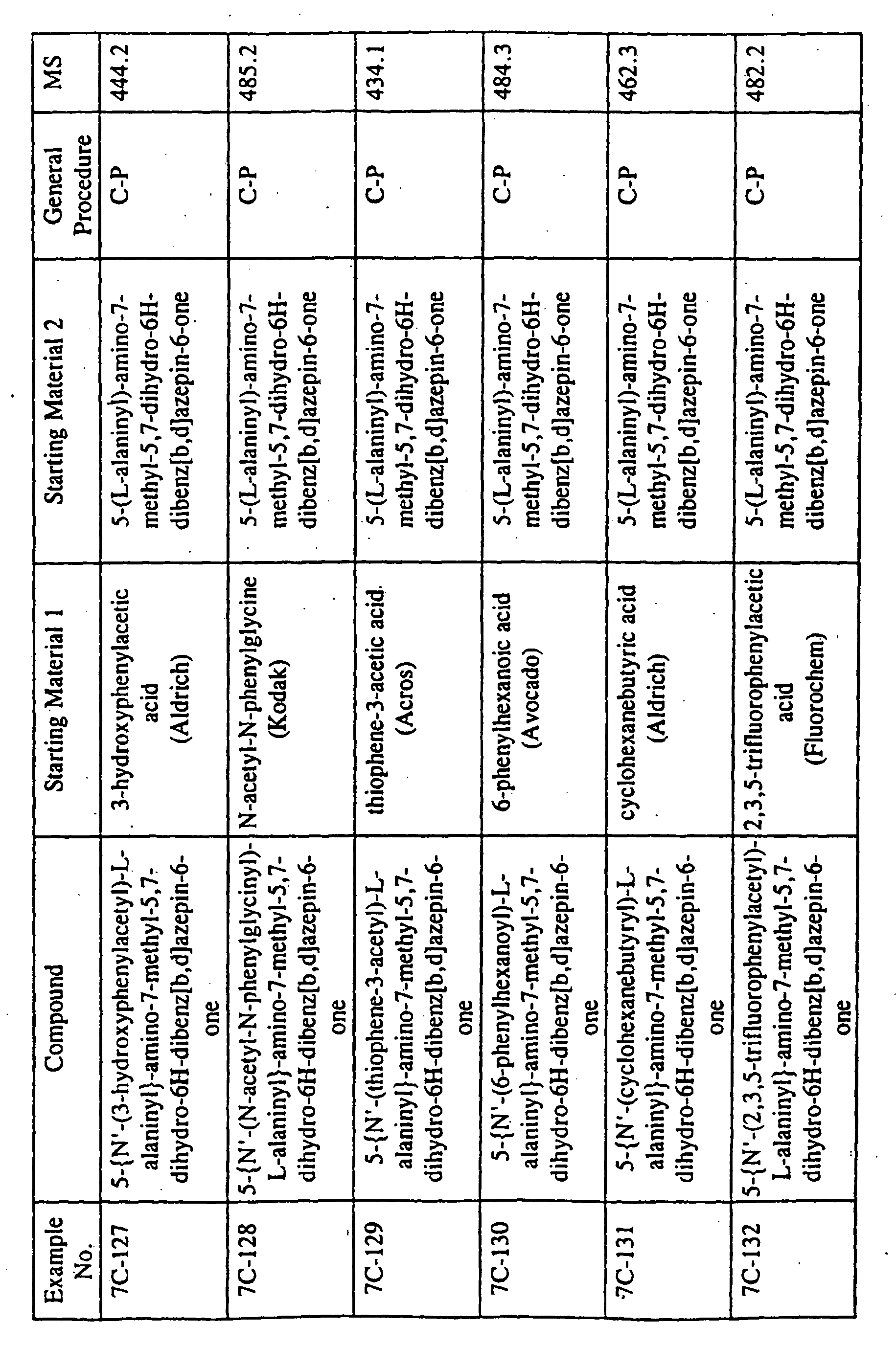

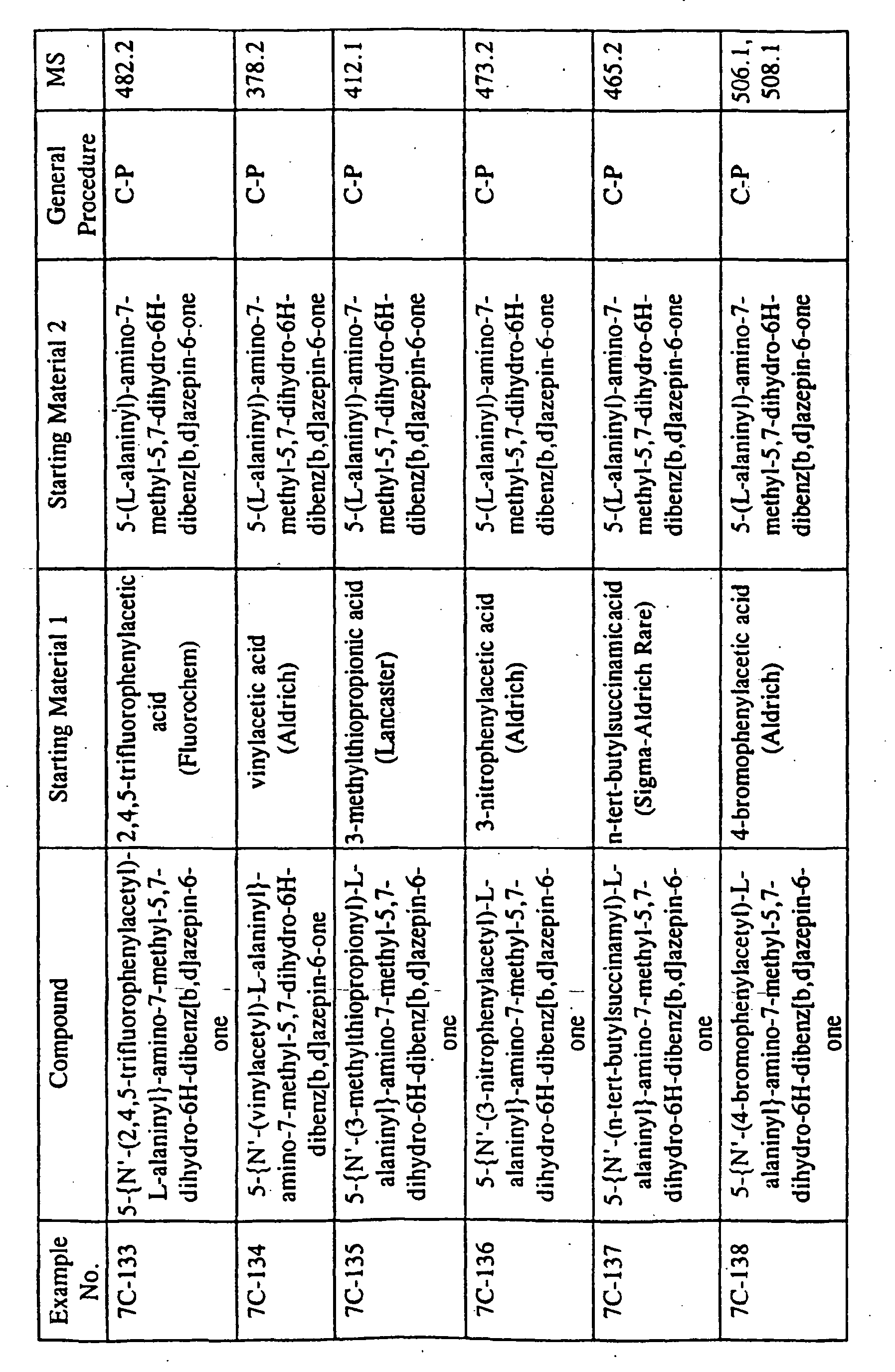

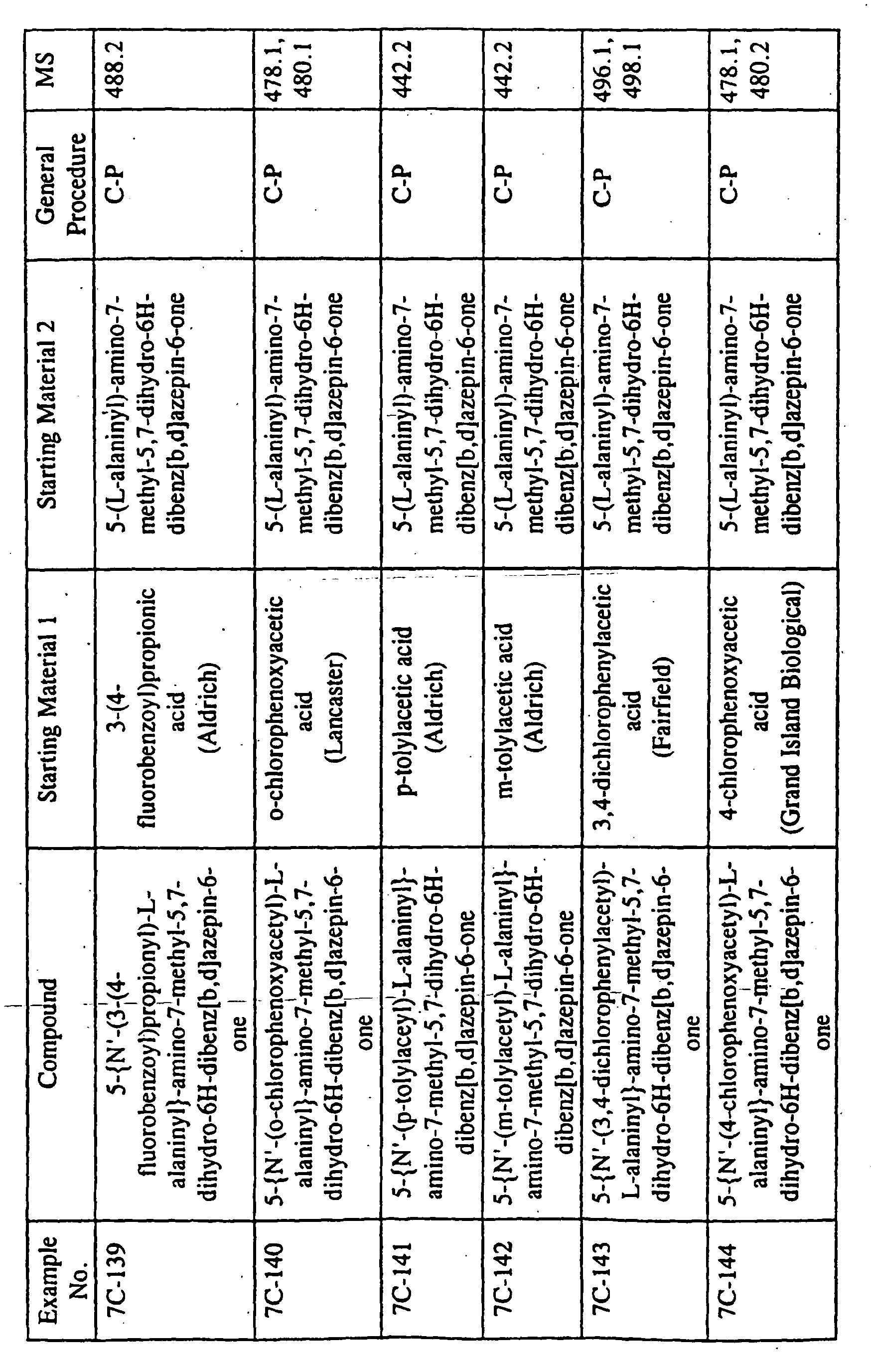

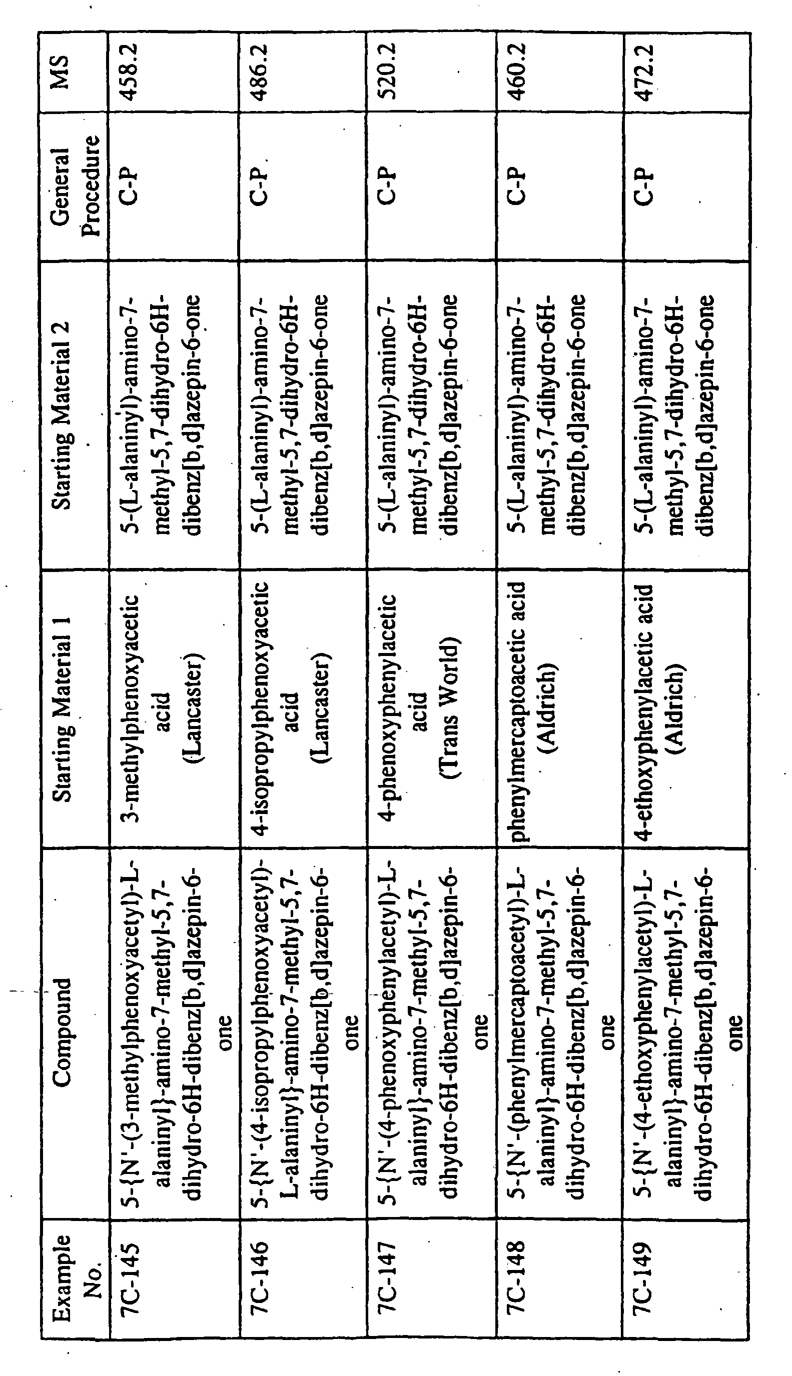

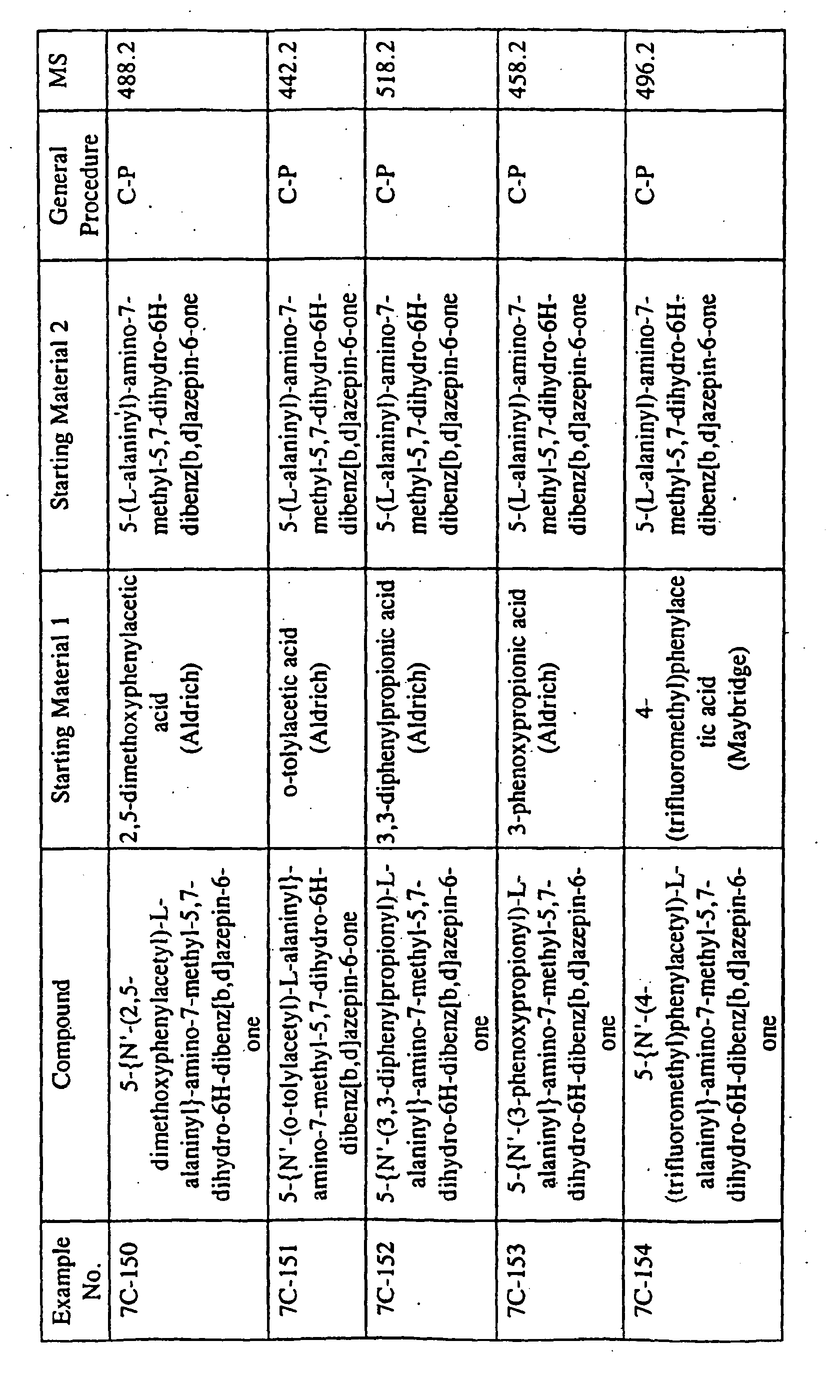

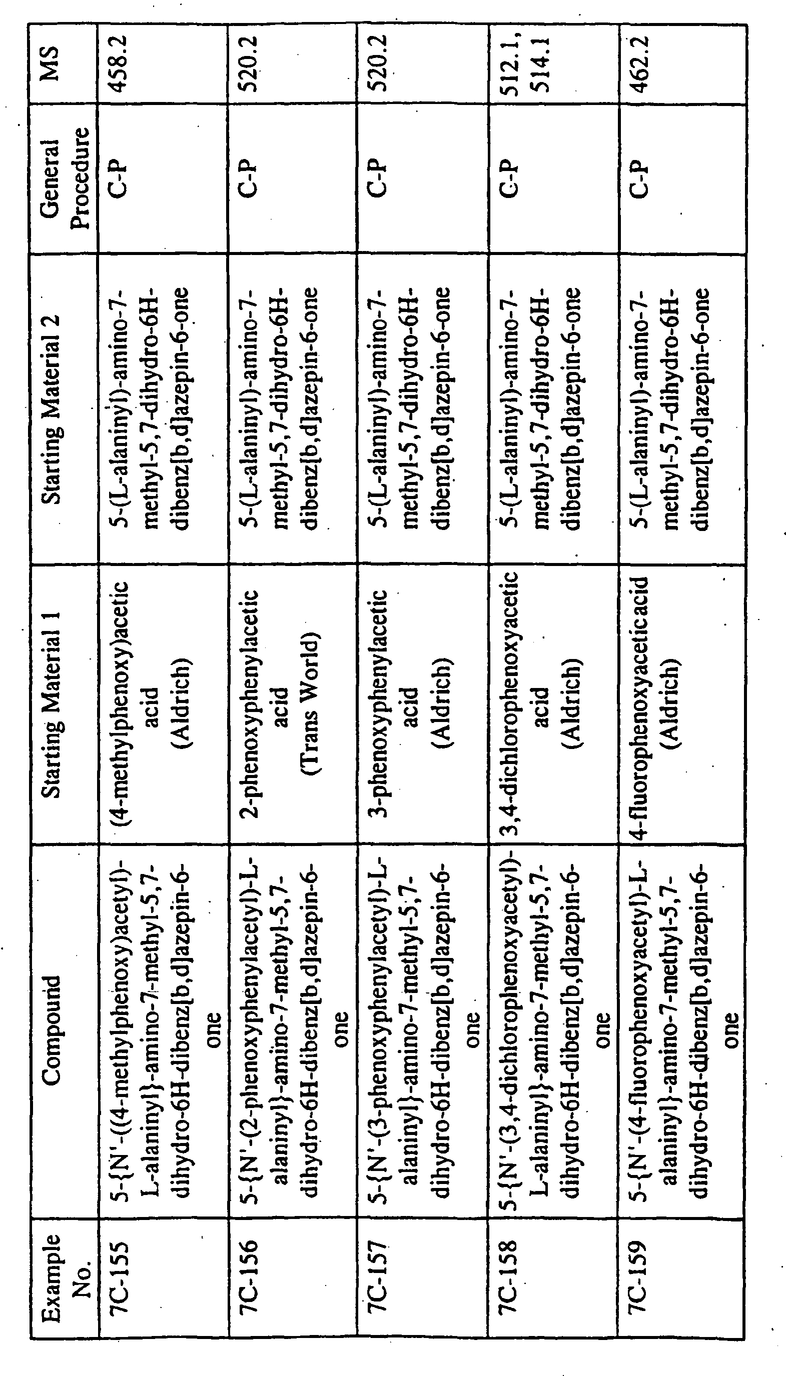

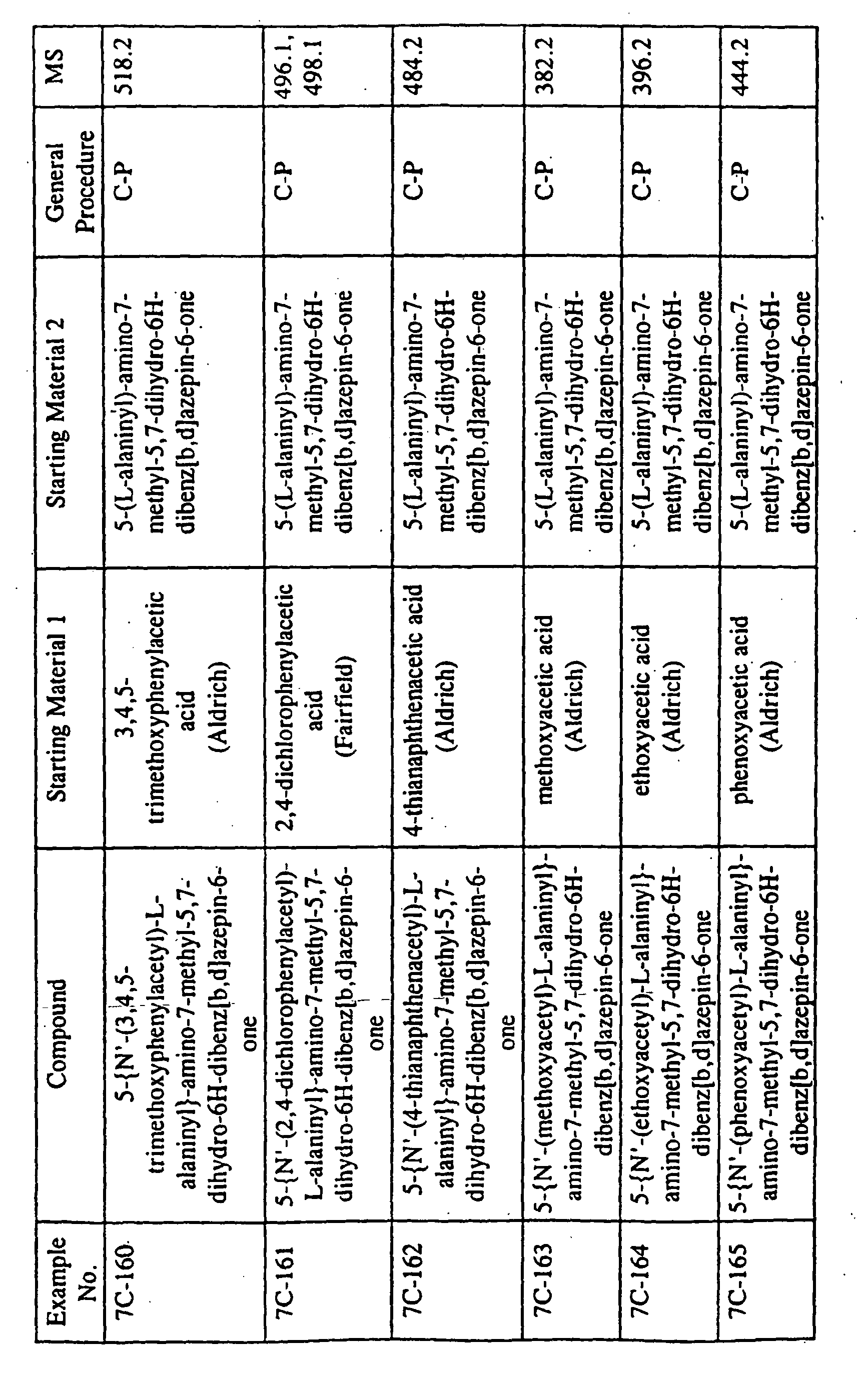

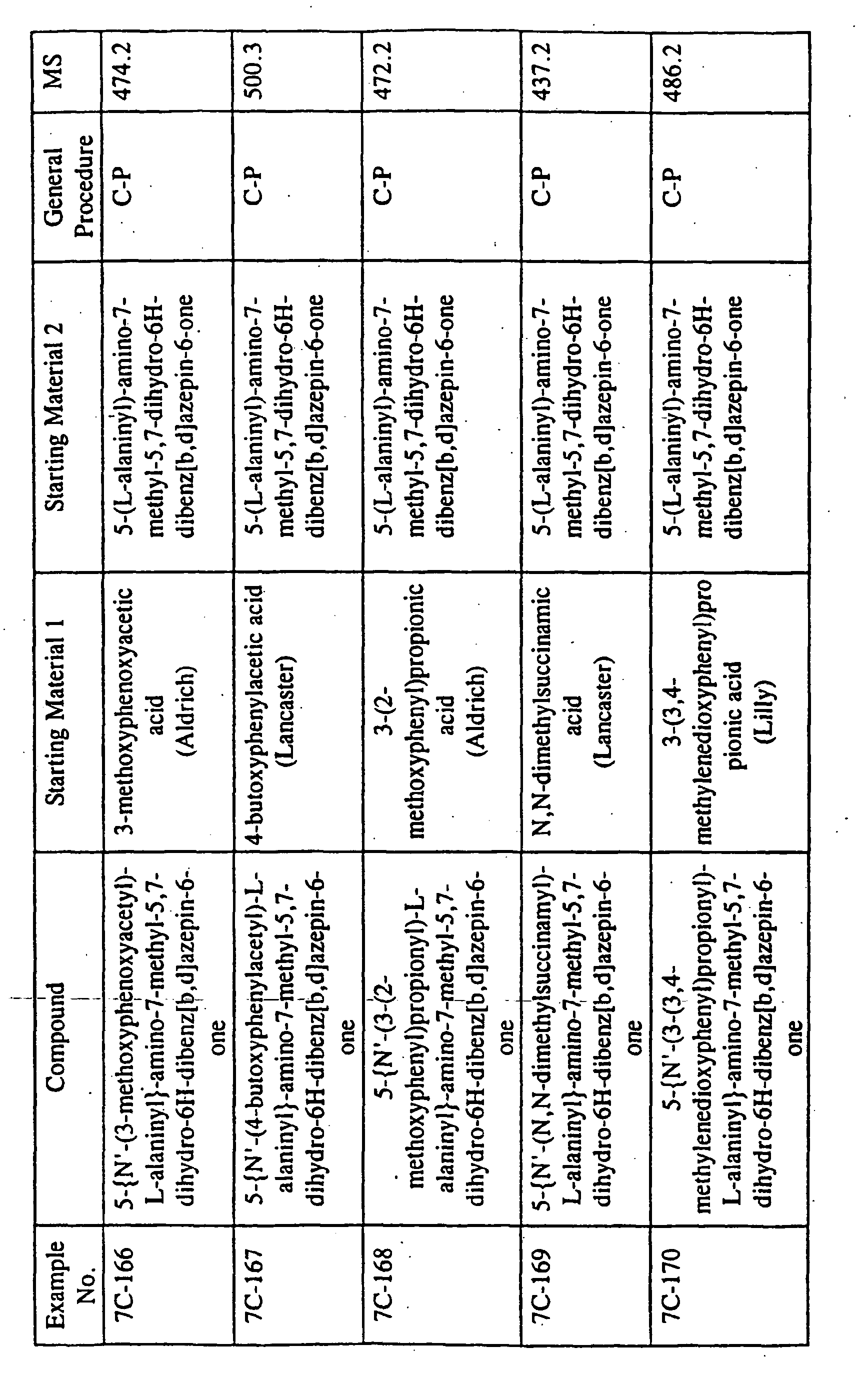

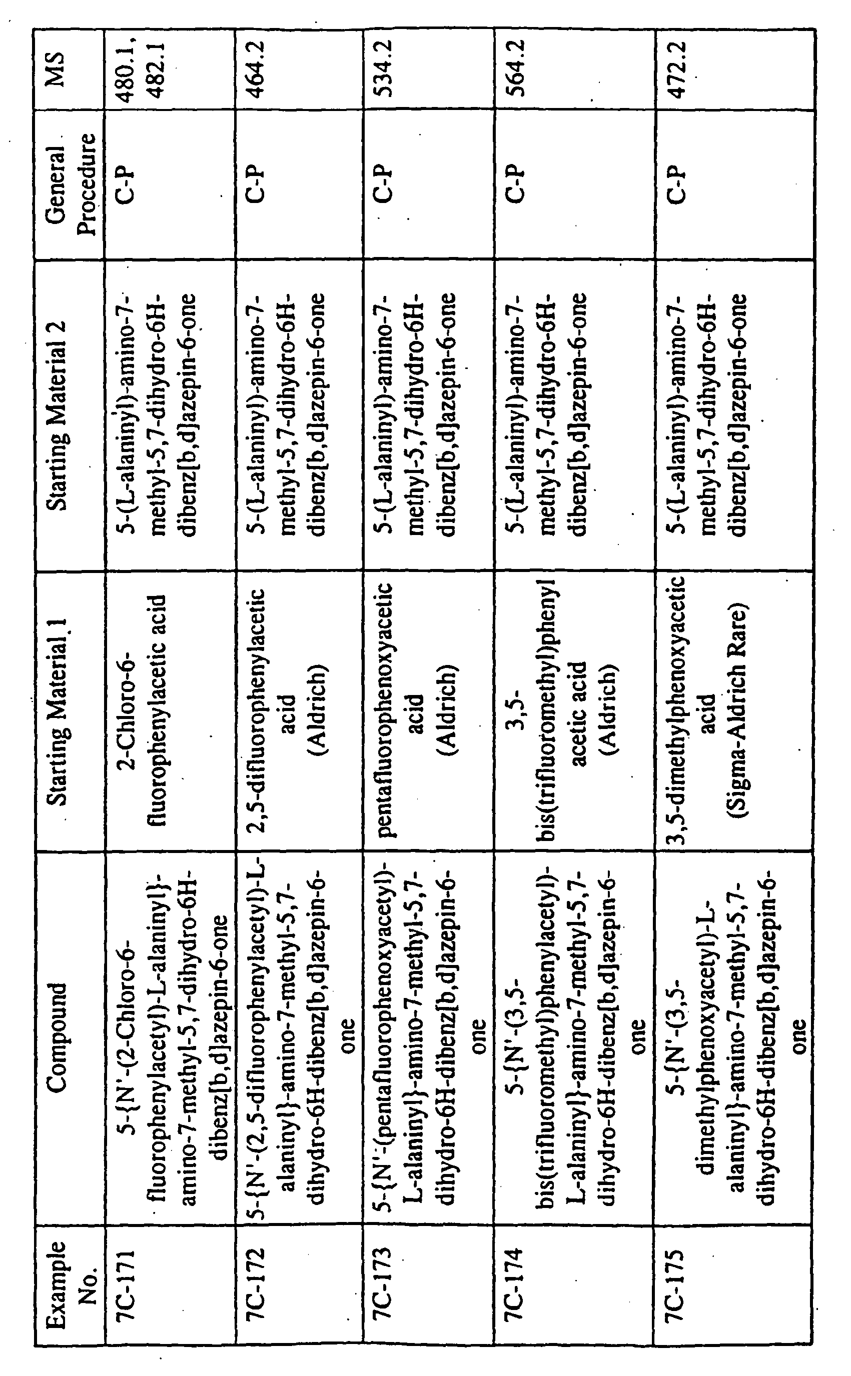

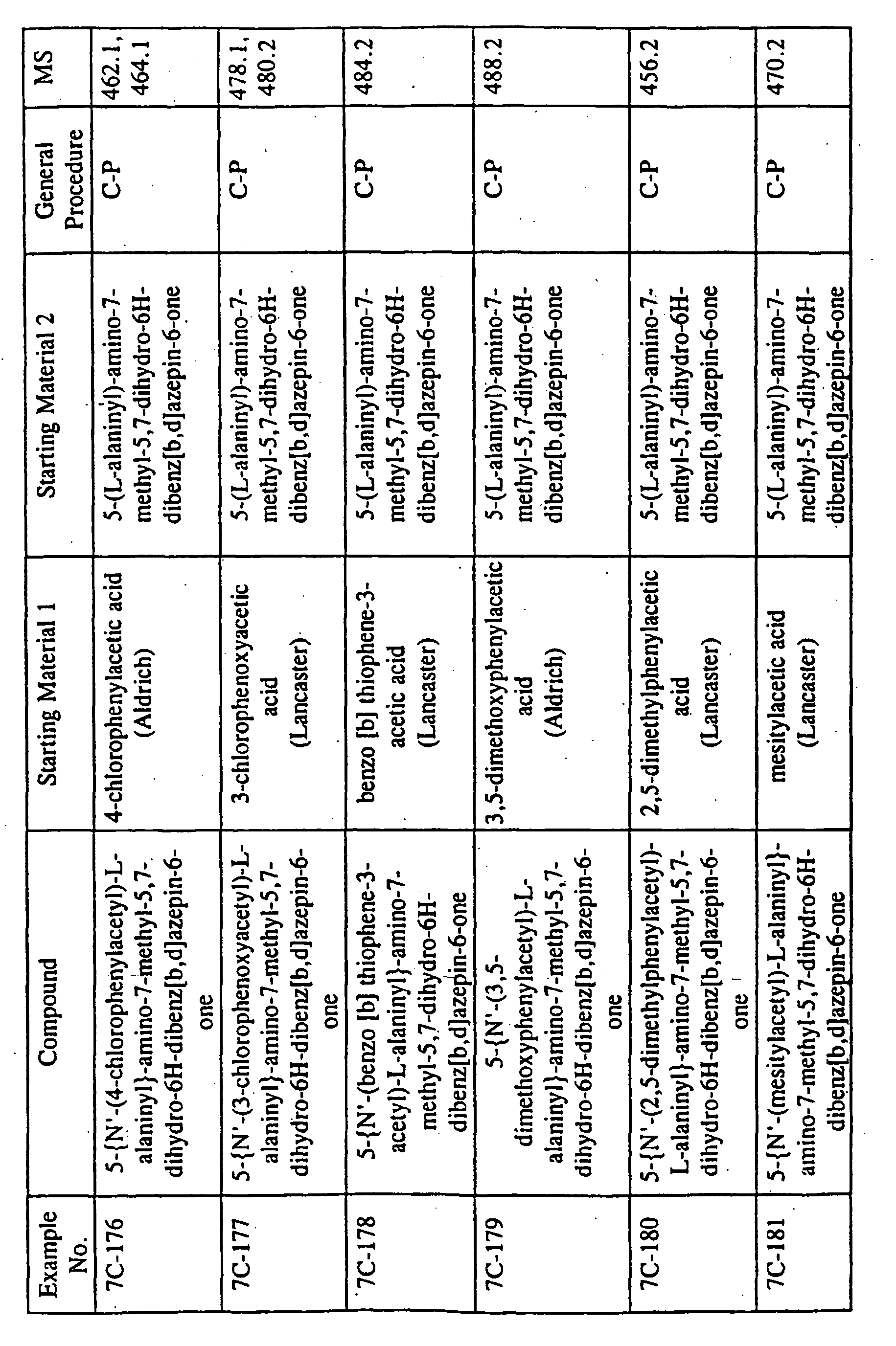

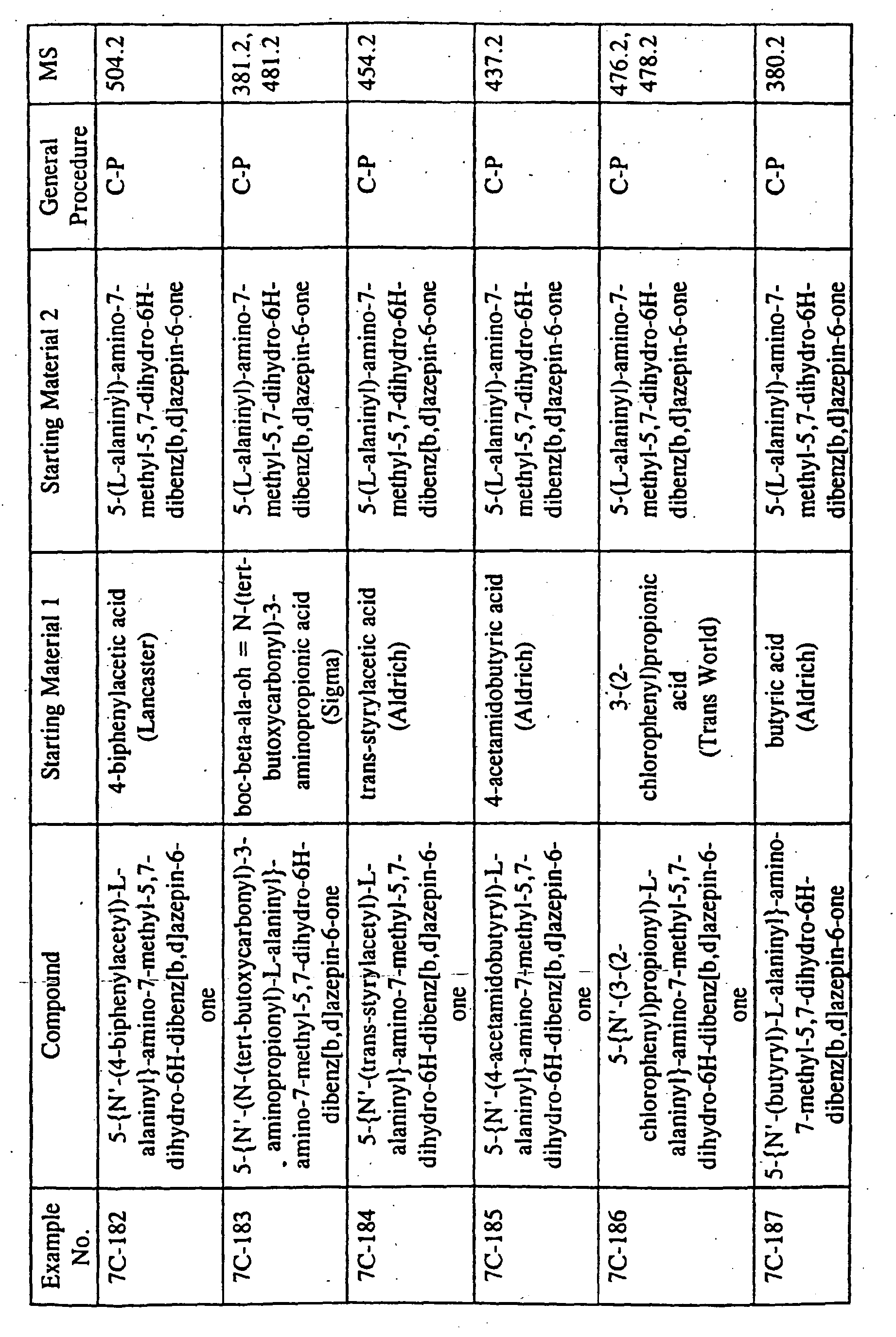

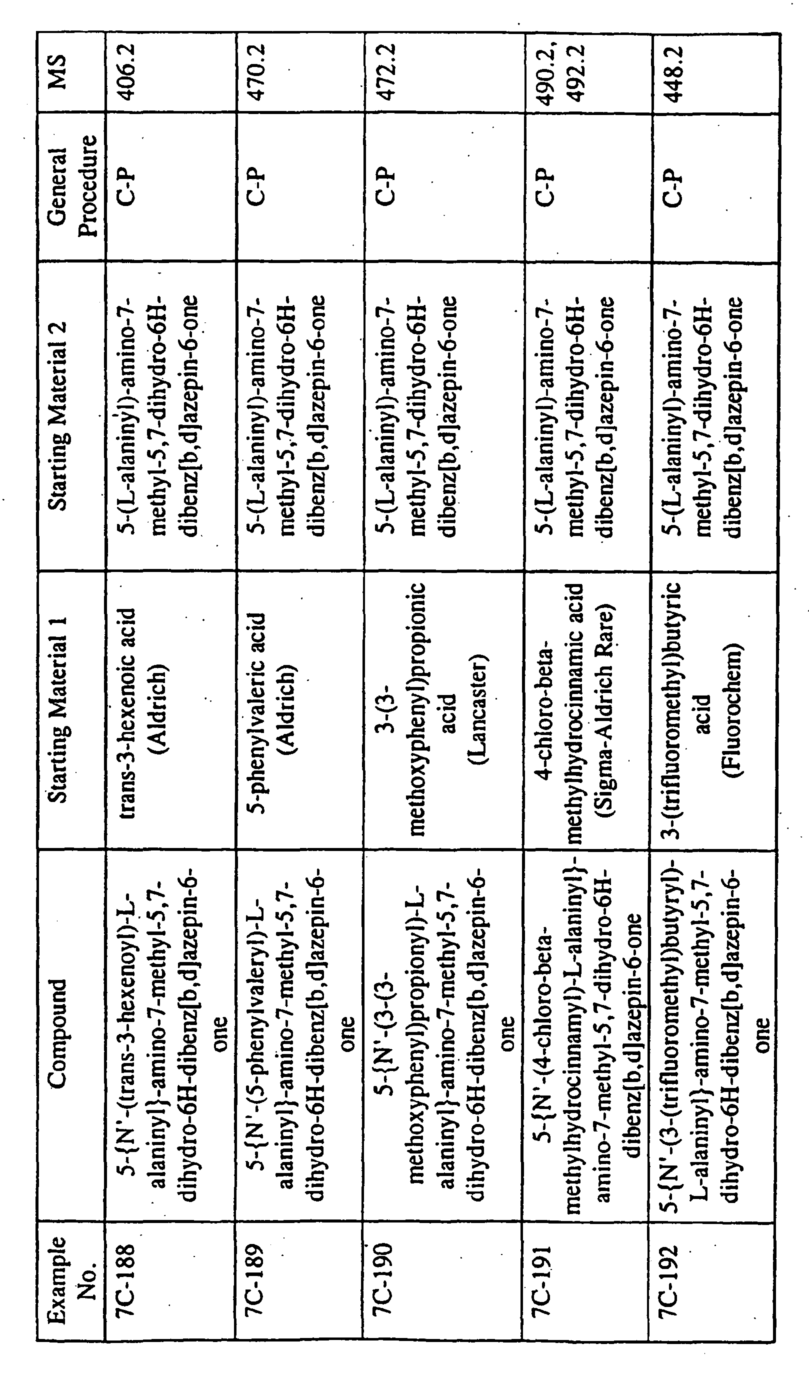

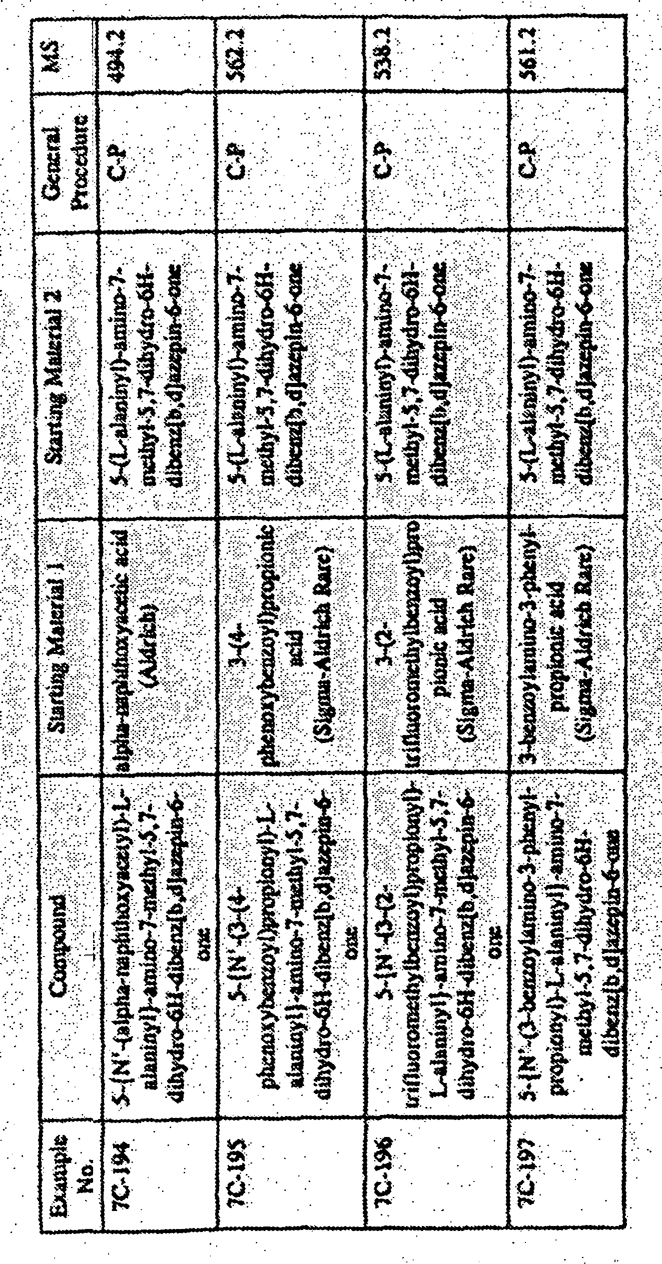

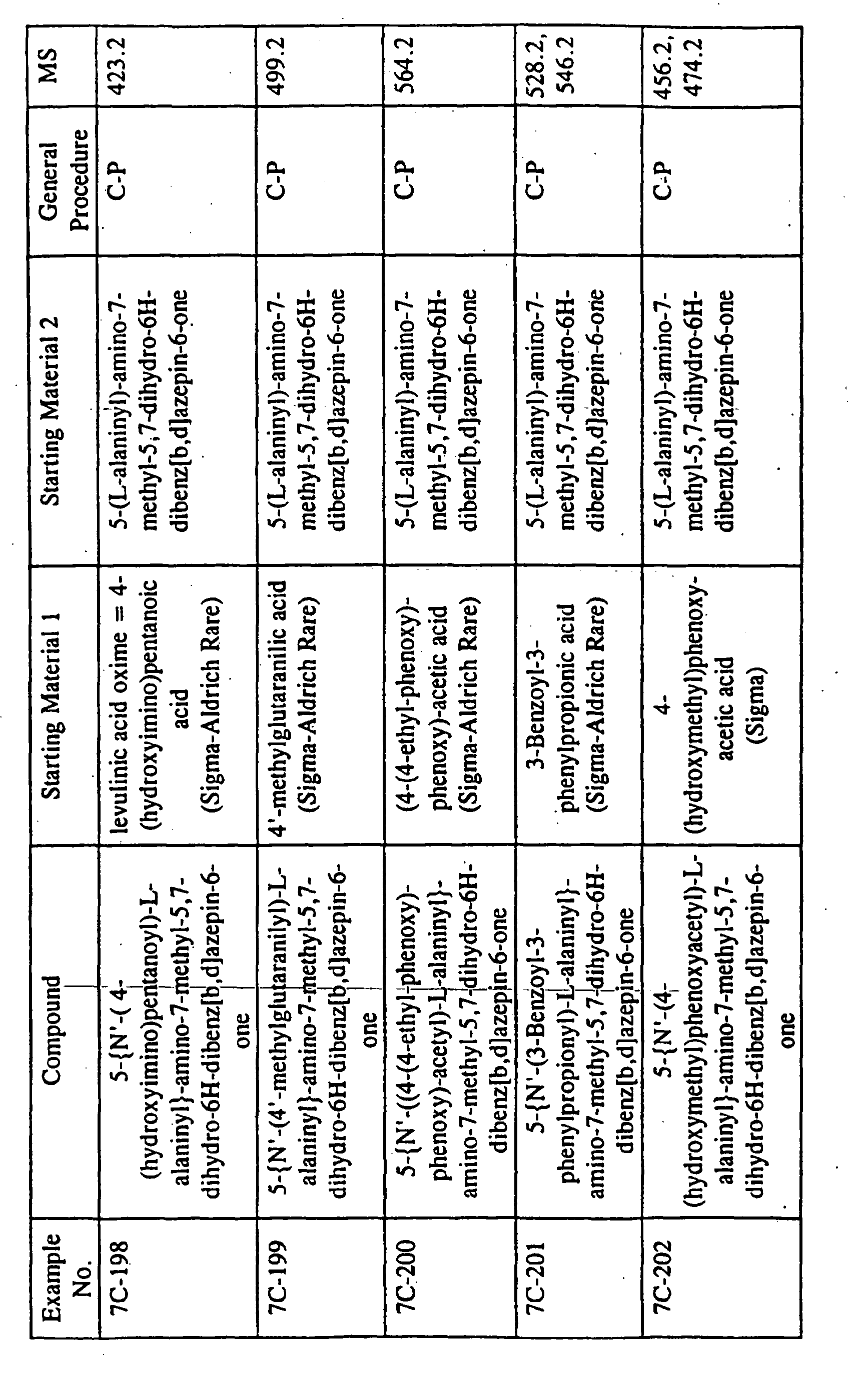

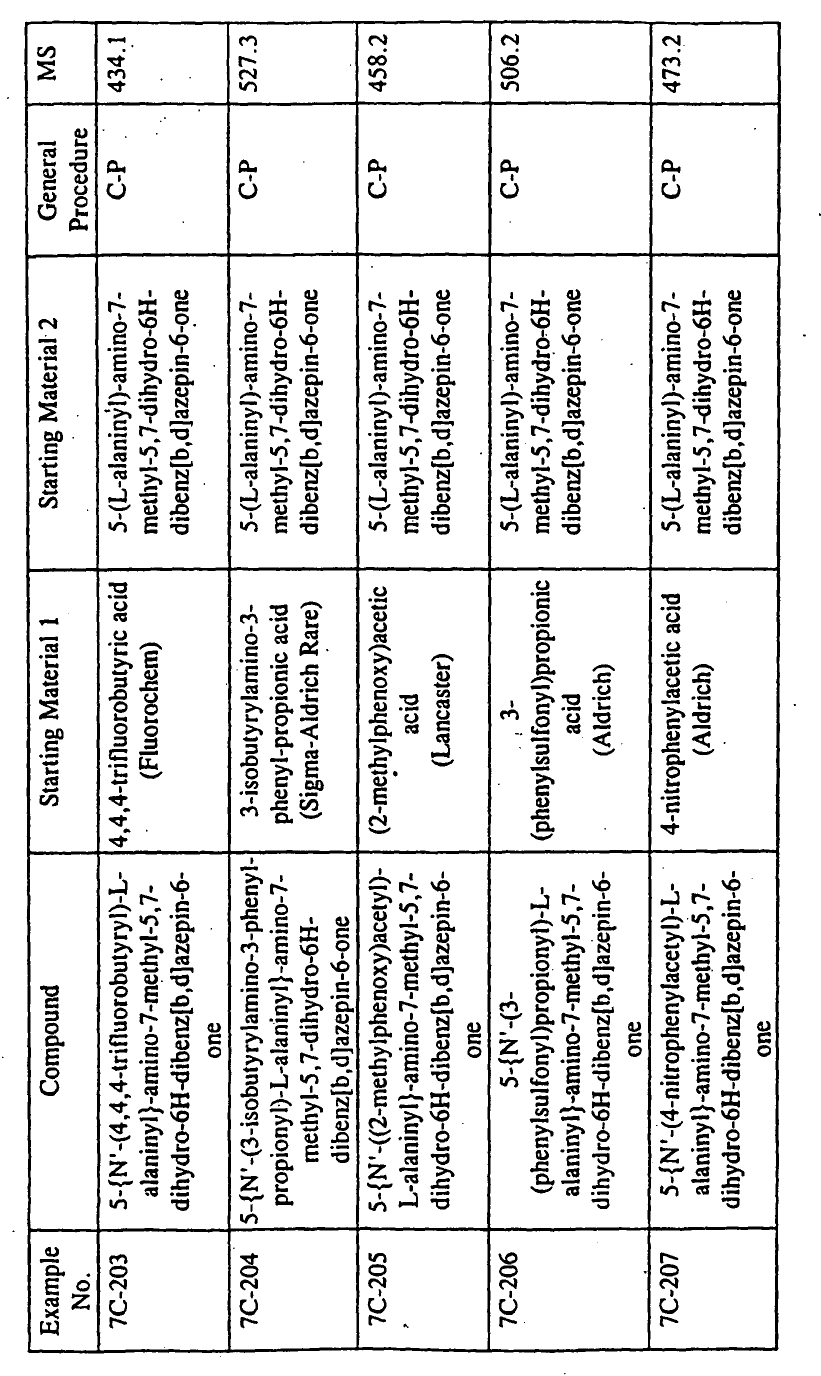

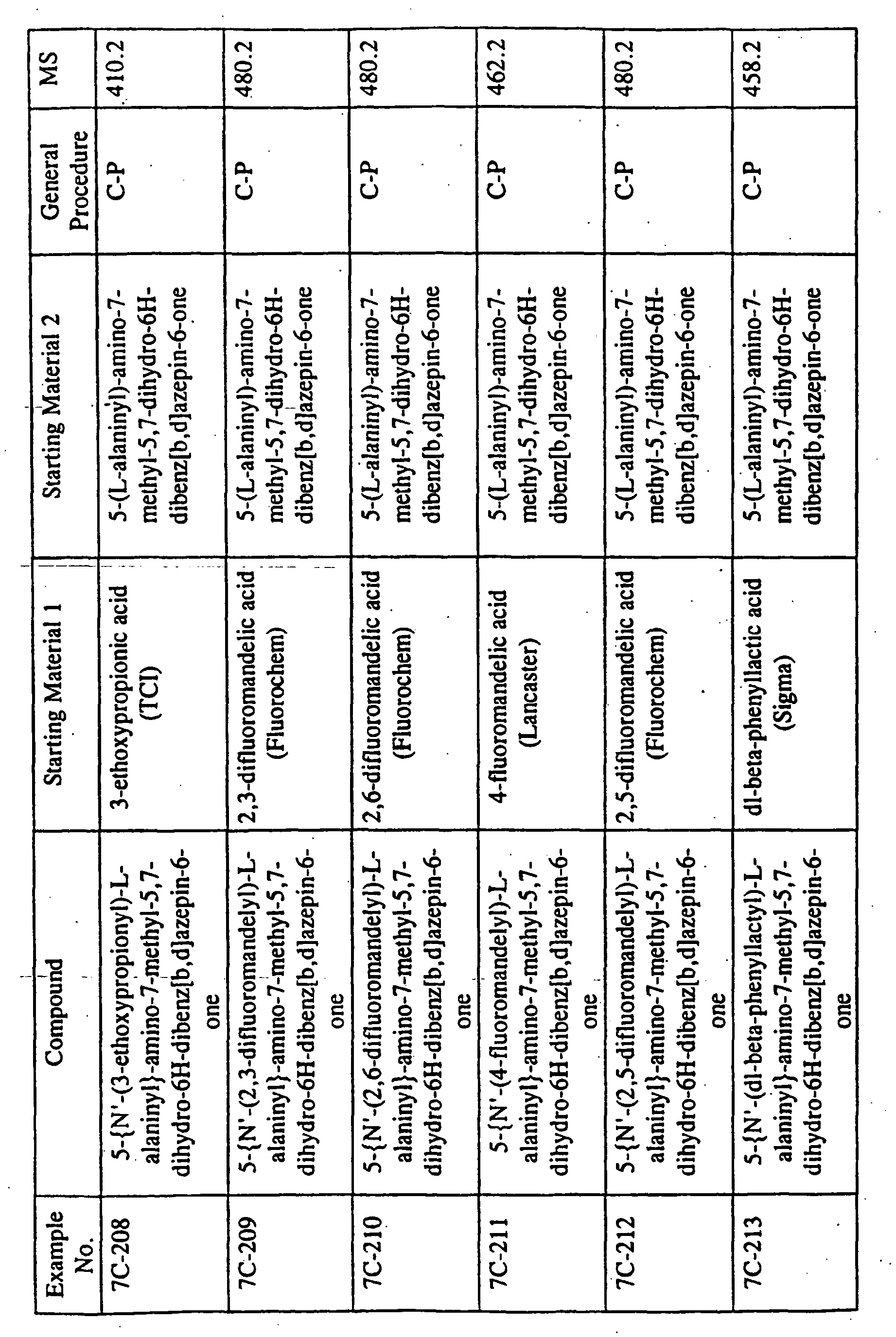

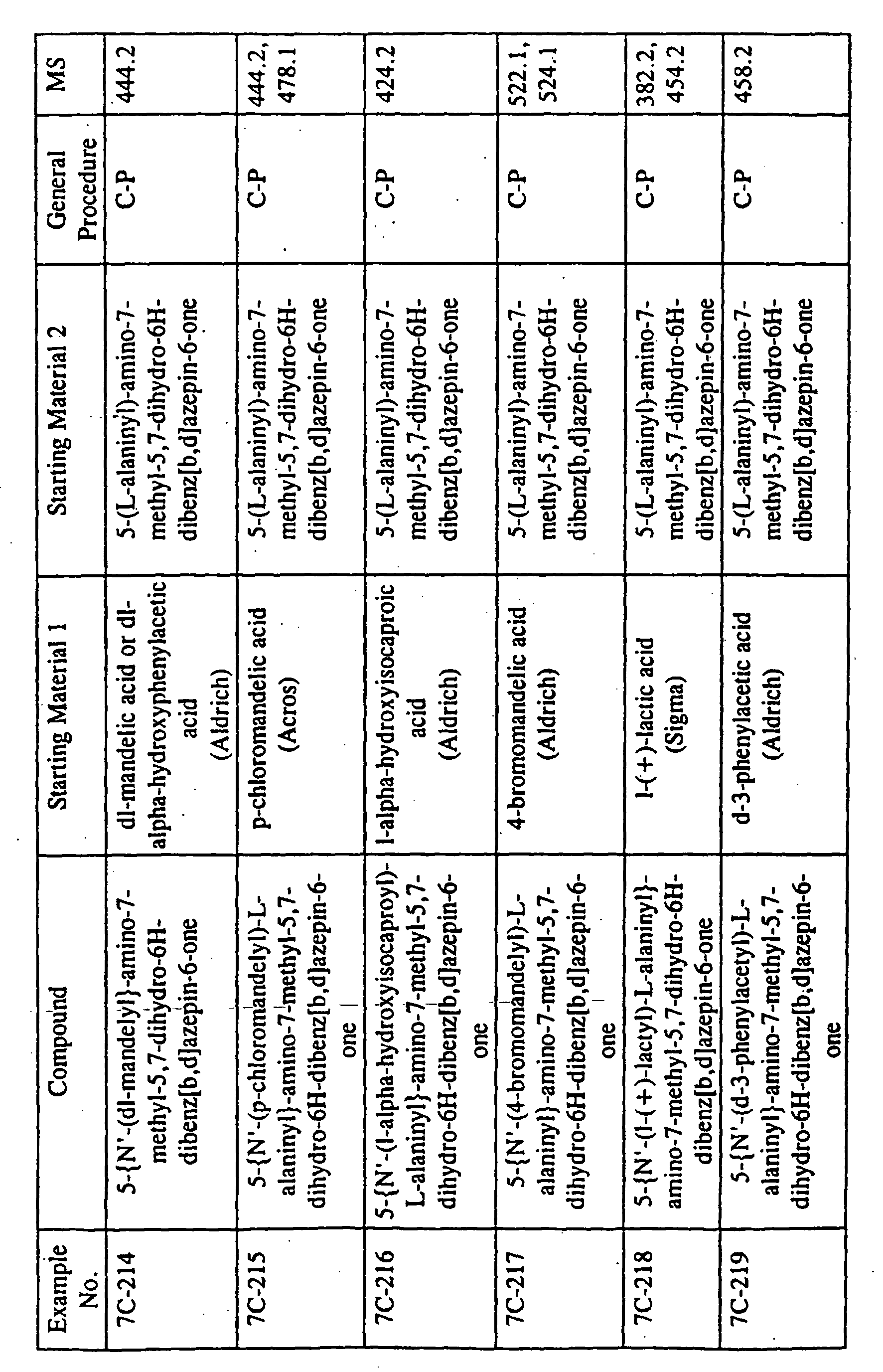

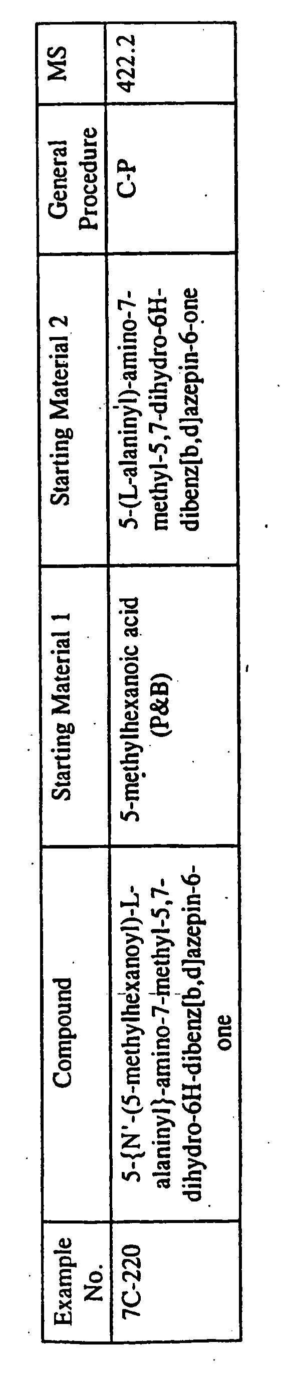

### GENERAL PROCEDURE C-S

Step A: Each amino acid (150 umol) was weighed into an 8-mL capacity vial and dissolved in 1.5 mL of 10% DMF in dichloromethane (DCM). To each vial was added 0.8 mL (175 umol) of a solution of 5-amino-7-methyl-5,7-dihydro-6H-dibenz[b,d]azepin-6-one hydrochloride (481 mg, 1.75 mmol) (from Example 7-A) and 670 mg (1.75 mmol) of PP-HOBT (from Example C-AF) dissolved in 7.5 mL DMF. This was followed by the addition to each vial of 2 mL (approximately 200 umol) of a solution of EDC hydrochloride in DCM (383 mg, 2.0 mmol in 20 mL DCM). After rocking the vials at room temperature for 14 hours, approximately 100-125 mg of polystyrene-piperidine resin (approximately 3.6 mmol/g, 350 umol, 2.33 eq.) was added to each vial and rocking continued for 15 minutes. Methanol (2.5 mL) was added to each vial and the material put on a 1 g SCX column (Varian) pre-equilibrated with 5 mL of MeOH and 5 mL of 10% MeOH/chloroform. After pushing the liquid through the column with nitrogen, the column was washed with 5 mL of 10 % MeOH/chloroform. The combined eluants (collected in 25 mL roundbottom flasks) were evaporated at reduced pressure with a warm water bath at 30-35 °C and then further evaported in a vacuum oven at 40-45°C. When the net weight of the residues was below 100 mg, 5 mL of dioxane and, if necessary, 1 mL of MeOH was added to redissolve the residue and solvent was again removed on the rotary evaporator and in the vacuum oven. After drying in the vacuum oven overnight, an HPLC was taken of each product. HPLC show primarily the desired product and with about 15% deblocked product (i.e., product with the BOC group removed).
Step B: To each round bottom flask was added 5 mL of 4 N HCl in dioxane. After sitting at room temperature for 2-3 hours, an HPLC was taken and was solvent removed on the rotary evaporator (bath temp 30-35°C) and in the vacuum oven overnight (at approximately 40°C). The HPLC of the t-butyl threonine adduct showed incomplete removal of the t-butyl group. An additional 5 mL of 4 N HCl in dioxane was added and the reaction (at room temperature) monitored by HPLC at 4 hours and approximately 20 hours. Complete removal of the t-butyl group was observed after 20 hours. All products were pure by HPLC with only a single peak or resolved diastereomeric peaks observed except for some trace impurities in the methione case. Yields varied from 80 to 100%. Each round bottom contained approximately 150 umoles of the amino acid linked to 5-amino-7-methyl-5,7-dihydro-6H-dibenz[b,d]azepin-6-one.
Step C: A stock solution of 567 mg (1.48 mmol) PP-HOBT in 8.5 mL DMF (approximately 0.175 M PP-HOBT in DMF) was prepared and 0.81 g (0.86 mL, 150 umol) of this PP-HOBT solution was added to each of the nine round-bottom vessels containing the products from Step B. Clear solutions were obtained for all, except where the linked amino acid was alpha amino isobutyric acid. In this case, an additional 0.86 mL of DMF was added but still the mixture remained heterogeneous. The contents of each of the nine round bottoms "n" (where n = 1 to 9) were divided into four equal portions (approximately 37 umols each) and placed in vials. Stock solutions (0.1 M) of the carboxylic acids were then made up in 10% DMF/DCM. The appropriate stock solution (0.3 mL, 30 umol) was then added to each of the vials. A 0.1 M stock solution (20 mL) of EDC hydrochloride in DMF was prepared. This stock solution (0.4 mL, 40 umol) was then added to each of the vials which were then capped and put on a rotator for 12 hours. Normal SCX workup and evaporation of solvent afforded products as white solids or clear to light caramel resins. Each of these products was taken up in methanol/chloroform and divided into three tared vials, plus a vial for MS and HPLC characterization. After evaporation of solvent, the final weights in each vial were determined. Product identity was verified by ionspray mass spec and purity assessed by reverse phase HPLC.

### Example C-AF

### Preparation of PP-HOBT

To a stirred solution of 7.68g (30 mmol) sulfonyl chloride in 120 mL of dichloromethane was added dropwise, over a 10 min period, 5.04g (30 mmol) of 4-piperidino-piperidine (Aldrich, 90%) and 3.6 g (36 mmol) of triethylamine in 30 mL of dichloromethane. A mildly exothermic reaction ensured. After stirring 2 hours at room temperature, the orange solution was diluted with 100 mL of dichloromethane and washed with 10% sodium bicarbonate solution (2 x 100 mL) and brine (1 x 100 mL). After drying over sodium sulfate, the solvents was removed at reduced pressure to afford 10.7 g of crude product as a light tan solid (R_{f} = 0.5, Silica, 10% MeOH/chloroform).

To this crude material was added 200 mL of 95 % EtOH/5 % MeOH followed by 60 mL of hydrazine hydrate. The mixture was refluxed for 3 hours. During the first 0.5 hour, the initially orange solution turned deep red-orange before turning orange again. After refluxing for 3 hours, most of the solvent, water and hydrazine was removed at reduced pressure. To the residue was added 50 mL of EtOH and solvent removed at reduced pressure. This was repeated 2 or more times to give a tan solid which was further dried in the vacuum oven to a constant weight of 13.5 g. To the flask containing this solid was added 250 mL of water. Almost all of the solid went into solution, then a fine light yellow precipitate formed. After stirring cooled in an ice bath for two hours, the solid was collected by vacuum filtration through a sintered glass filter, and rinsed with about 20 mL of cold water. Drying in the vacuum oven at 40°C overnight afforded 7.3 g (63% yield) of the title compound (PP-HOBT) as an off-white crunchy powder, mp 195-200°C (dec).

Using the procedures indicated, the compounds shown in Table C-5 were prepared. Starting material 2 used in these procedures was prepared as described in General Procedure C-S.

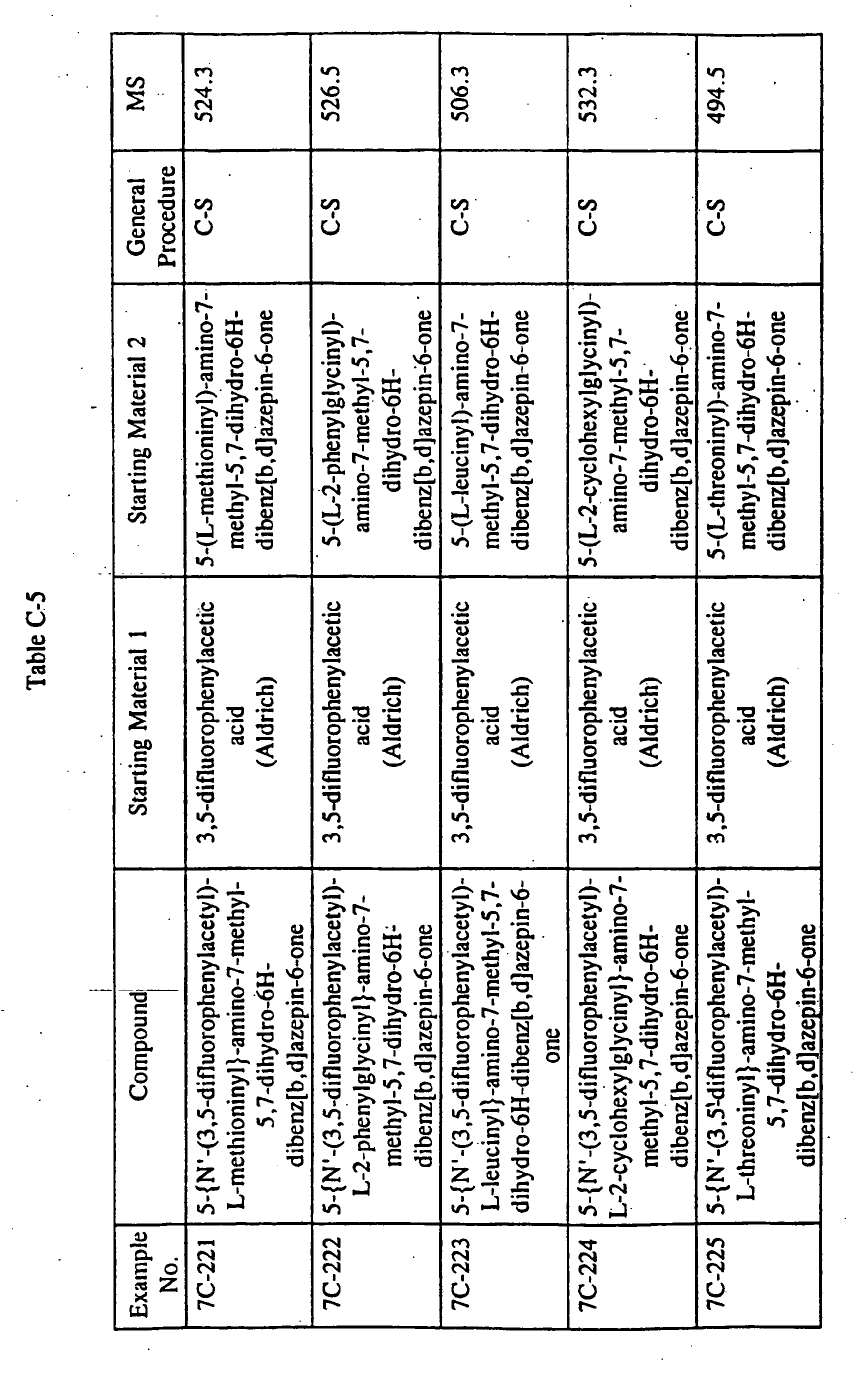

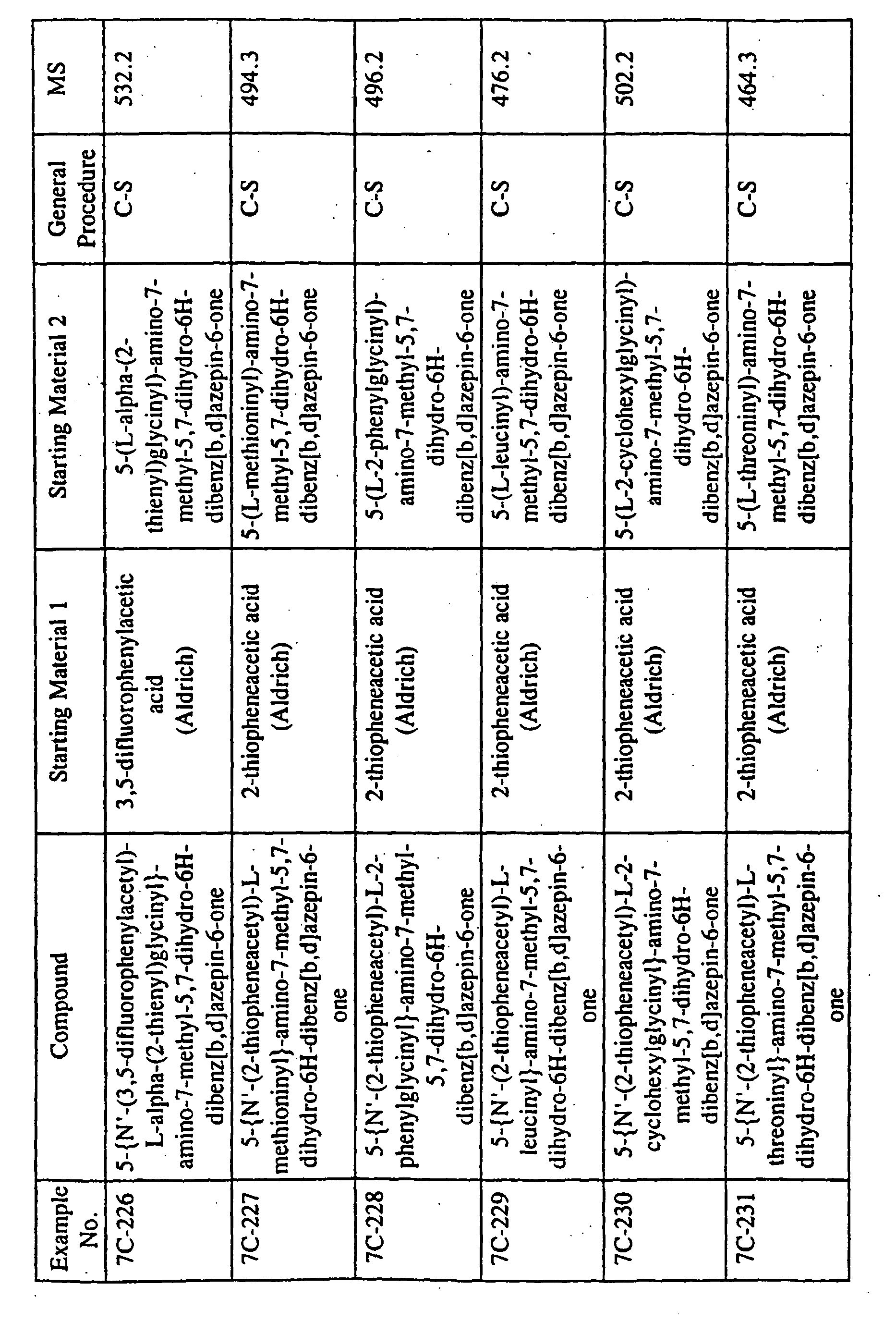

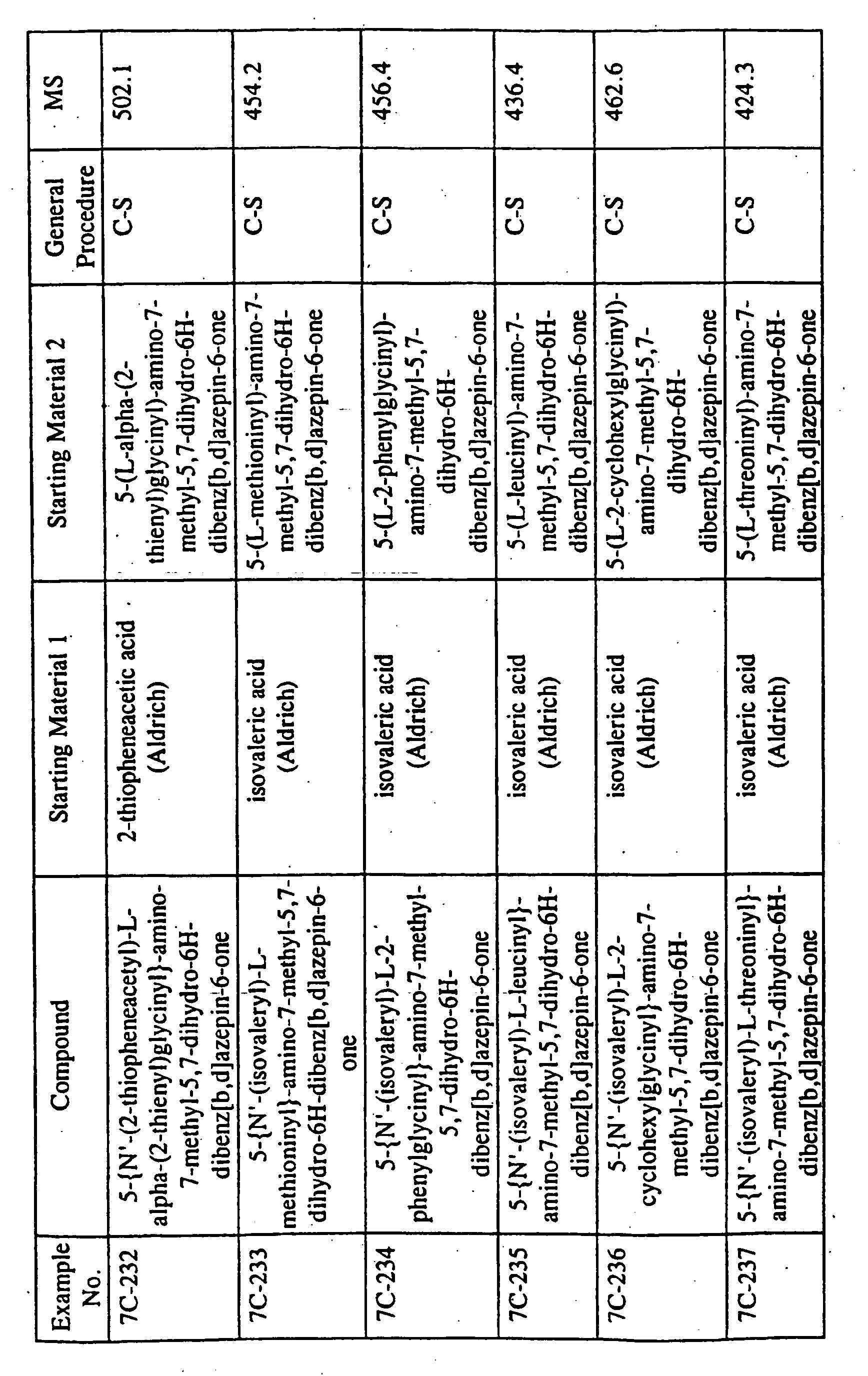

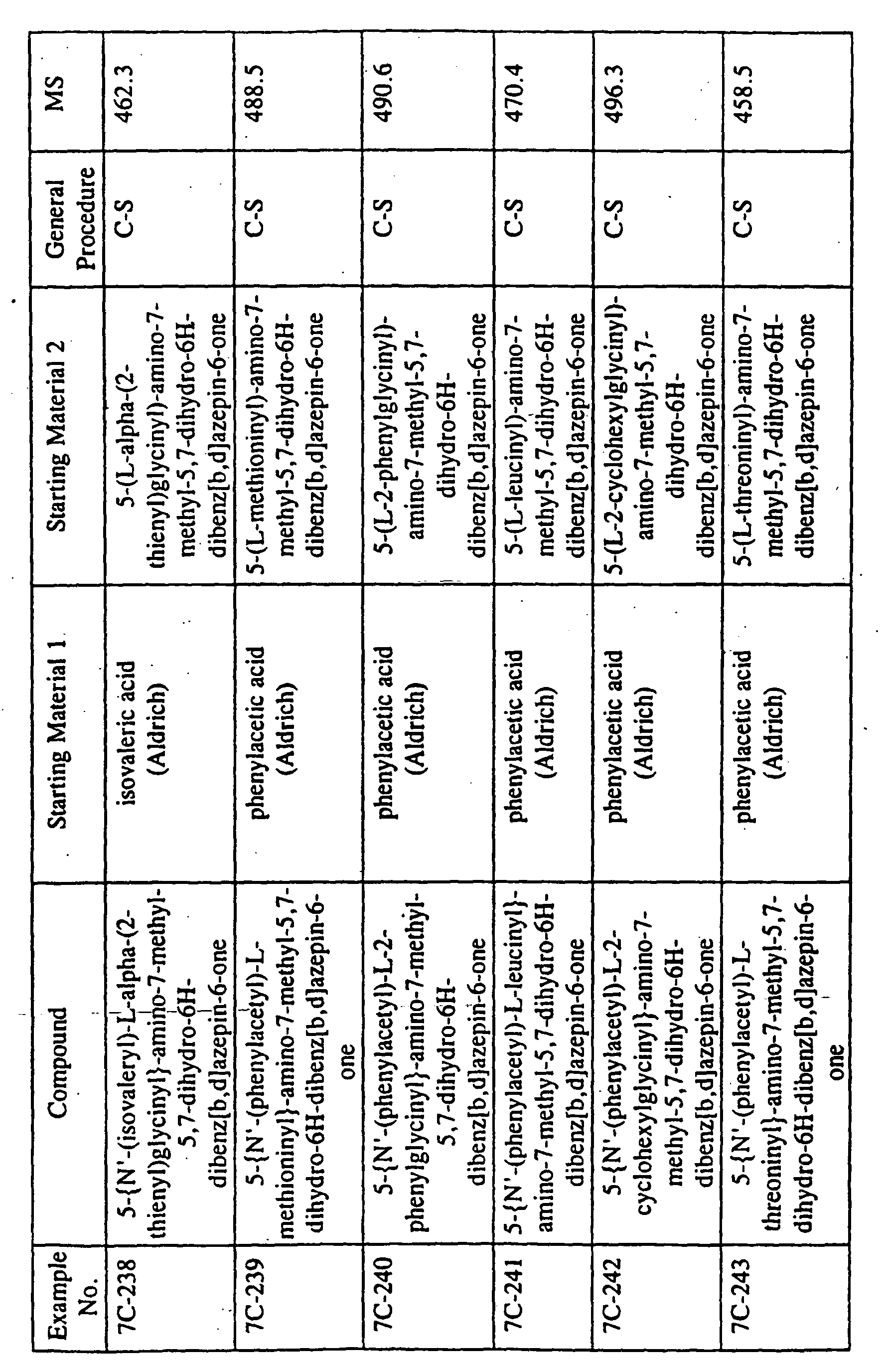

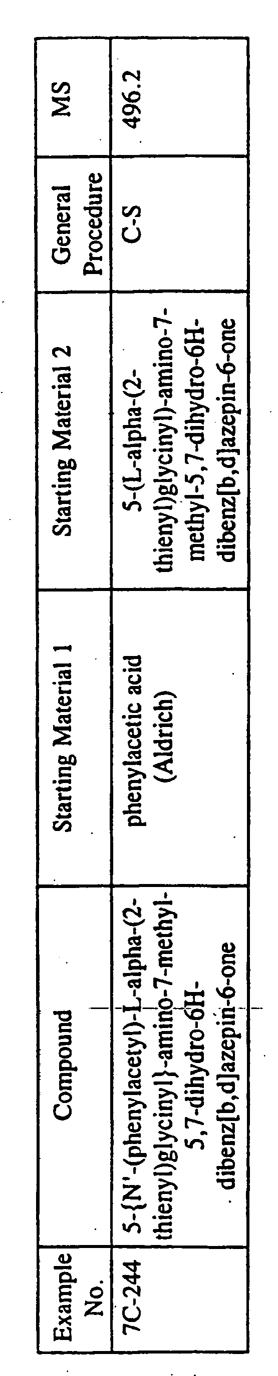

Additionally, the following procedures provide various carboxylic acid esters which can be hydrolyzed using General Procedures AC or BD below to afford the corresponding carboxylic acids. Coupling of the resulting carboxylic acids to the amines employed above using the General Procedures set forth above provides for additional compounds within the scope of this invention.

### GENERAL PROCEDURE AA

### Reductive Amination

To a solution of the arylamine in ethanol in a hydrogenation flask was added 1 equivalent of the 2-oxocarboxylic acid ester (e.g., pyruvate ester), followed by 10% palladium on carbon (25 weight percent based on the arylamine). The reaction was hydrogenated at 20 psi H₂ on a Parr shaker until complete reaction was indicated by tlc (30 minutes to 16 hours). The reaction mixture was then filtered through a pad of Celite 545 (available from Aldrich Chemical Company, Inc.) and stripped free of solvent on a rotary evaporator. The crude product residue was then further purified via chromatography.

### GENERAL PROCEDURE AB

### First Transesterification Technique

A solution of 1-5 equivalents of the desired alcohol was added to 1. equivalent of sodium hydride in toluene. After off-gassing had ceased, the compound to be transesterified, dissolved in toluene, was added. After 0.5 hours, the reaction was either heated to 40°C and placed under house vacuum (∼20 mmHg), or nitrogen was bubbled through the solution while it was heated at 90°C. The reaction was followed by tlc, and when the reaction was complete the solution was cooled and quenched with water or 1M HCl, and in smaller scale reactions diluted with ethyl acetate. The organic phase was extracted with saturated aqueous NaHCO₃, then washed with saturated aqueous NaCl and dried over MgSO₄. The solution was stripped free of solvent on a rotary evaporator, and the crude product residue was then further purified by chromatography. Alternatively, the reaction mixture was worked-up by evaporation of the solvents and direct chromatography of the crude mixture.

This procedure is particularly useful in the case of costly and/or high boiling alcohols.

### GENERAL PROCEDURE AC

### Second Transesterification Technique

The compound to be transesterified was placed in a large excess of the desired alcohol. A catalytic amount of dry NaH was added, and the reaction was followed by tlc until the presence of starting material was no longer detected. The reaction was quenched with a few milliliters of 1N HCl, and after a few minutes of stirring saturated aqueous NaHCO₃ was added. The organic phase was washed with saturated aqueous NaCl and dried over MgSO₄. The solution was stripped free of solvent on a rotary evaporator, and the crude product residue was then further purified by chromatography.

### GENERAL PROCEDURE AD

### Third Transesterification Technique

The compound to be transesterified was placed in a large excess of the desired alcohol. A catalytic amount of dry NaH was added, and the reaction was followed by tlc until the presence of starting material was no longer detected. The reaction was quenched with a few milliliters of 1N HCl, and after a few minutes of stirring saturated aqueous NaHCO₃ was added. The volume of the reaction mixture was reduced on a rotary evaporator until the excess alcohol was removed and then the remaining residue was taken up in ethyl acetate and additional water was added. The organic phase was washed with saturated aqueous NaCl and dried over MgSO₄. The solution was stripped free of solvent on a rotary evaporator, and the crude product residue was then further purified by chromatography.

This procedure is particularly employed in the case of low boiling, inexpensive alcohols, miscible with water.

### GENERAL PROCEDURE AE

### O-Alkylation Technique

To a carboxylic acid compound (prepared, for example, by reductive amination via General Procedure AA to provide for the *N*-aryl amino acid ester, followed by hydrolysis via Procedure AF) in DMF was added 1.5 equivalent K₂CO₃, followed by 1 equivalent of alkylating agent (e.g., *tert*-butyl - bromoacetate). The reaction was stirred at room temperature for 2 hours, then was quenched with water and extracted into ethyl acetate. The organic phase was washed with saturated aqueous NaHCO₃, water, and saturated aqueous NaCl, and was then dried over MgSO₄. The solution was stripped free of solvent on a rotary evaporator to yield the crude product.

### GENERAL PROCEDURE AF

### Ester Hydrolysis to Free Acid

To a carboxylic ester compound (prepared, for example, by reductive amination via General Procedure AA to provide for the *N*-aryl amino acid ester) in a 1:1 mixture of CH₃OH/H₂O was added 2-5 equivalents of K₂CO₃. The mixture was heated to 50°C for 0.5 to 1.5 hours until tlc showed complete reaction. The reaction was cooled to room temperature and the methanol was removed on a rotary evaporator. The pH of the remaining aqueous solution was adjusted to ∼2, and ethyl acetate was added to extract the product. The organic phase was then washed with saturated aqueous NaCl and dried over MgSO₄. The solution was stripped free of solvent on a rotary evaporator to yield the crude product.

### GENERAL PROCEDURE AG

### N-Heteroarylation of Alanine

A solution of 1.1 equivalents of L-alanine and 2 equivalents NaOH in DMSO was stirred at room temperature for 1 hour, then I equivalent of 2-chlorobenzothiazole was added. The mixture was heated to 100°C for 4 hours, then cooled to room temperature and poured onto ice. The pH of the resulting aqueous solution was adjusted to ∼2, and the precipitated solid was removed by filtration. This solid was then dissolved in 1N NaOH and the resulting solution was filtered through a pad of Celite 545, The pH of the filtrate was adjusted to ∼2, and the white precipitate was removed by filtration and washed with water to yield the crude product.

### GENERAL PROCEDURE AH

### EDC Coupling

To a 1:1 mixture of the desired acid and alcohol in CH₂Cl₂ at O°C was added 1.5 equivalents triethylamine, followed by 2.0 equivalents hydroxybenzotriazole monohydrate, then 1.25 equivalents of ethyl-3-(3-dimethylamino)-propyl carbodiimide· HCl (EDC). The reaction was stirred overnight at room temperature, then transferred to a separatory funnel and washed with water, saturated aqueous NaHCO₃, 1N HCl, and saturated aqueous NaCl, and was then dried over MgSO₄. The solution was stripped free of solvent on a rotary evaporator to yield the crude product.

### GENERAL PROCEDURE AI

### Oxime or Amine Coupling Technique

The trichlorophenyl ester (1 eq) of a carboxylic acid was stirred in DMF or THF. The oxime or amine (1.2 eq) was added and the mixture was stirred at ambient temperature for 1-4 hours. In cases where the hydrochloride salt form of an amine was used, a suitable base such as *N,N-*diisopropylethylamine (1.2 eq) was also added. The resulting mixture was concentrated under reduced pressure to yield a crude product which was used without purification or was purified by silica gel chromatography and/or crystallization.

### GENERAL PROCEDURE AJ

### Alkylation Technique

The amine (1 eq), the α-bromo ester (1.1 eq) and a suitable base (such as triethylamine) (2 eq) were stirred in chloroform. The resulting solution was heated at reflux for 4-12 hours. After cooling, the mixture was diluted with chloroform and washed with water. The organic portion was dried (sodium sulfate) and concentrated under reduced pressure. The crude product was purified by silica gel chromatography.

### GENERAL PROCEDURE AK

### Oxime or Alcohol Coupling Technique

The carboxylic acid (1 eq) was stirred in a suitable solvent (such as THF, dioxane or DMF). An alcohol or oxime (1-5 eq) was added. EDC hydrochloride (1.2 eq) and hydroxybenzotriazole hydrate (1 eq) were added. A suitable base (such as 4-methylmorpholine or triethylamine) (0-1 eq) was added. A catalytic amount (0.1 eq) of 4-dimethylaminopyridine was added. The mixture was stirred at ambient temperature and under a dry atmosphere of nitrogen. After 20 hours, the mixture was concentrated under reduced pressure. The resulting concentrate was partitioned between ethyl acetate and water. The organic portion was separated and washed witch aqueous sodium bicarbonate and brine. The organic portion was dried (sodium sulfate) and concentrated under reduced pressure. The crude product was used without purification or was purified by silica gel chromatography and/or crystallization.

### GENERAL PROCEDURE AL

### EDC Coupling

The carboxylic acid was dissolved in methylene chloride. The amino acid (1 eq.), N-methylmorpholine (5 eq.) and hydroxybenzotriazole monohydrate (1.2 eq.) were added in sequence. A cooling bath was applied to the round bottomed flask until the solution reached 0°C. At that time, 1.2 eq. of 1-(3-dimethylaminopropyl)-3-ethylcarbodiimide hydrochloride (EDC) was added. The solution was allowed to stir overnight and come to room temperature under nitrogen pressure. The reaction mixture was worked up by washing the organic phase with saturated aqueous sodium carbonate, 0.1M citric acid, and brine before drying with sodium sulfate. The solvents were then removed to yield crude product. Pure products were obtained by flash chromatography in an appropriate solvent.

### GENERAL PROCEDURE AM

### Triflate Displacement

To a 0°C solution of *iso*-butyl R-(+)-lactate in CH₂Cl₂, was added 1.1 equivalents of trifluoromethanesulfonic anhydride. After stirring at room temperature for 20 min, 1.1 equivalents of 2,6-lutidine was added and stirring was continued for 10 min. This solution was then transferred to a flask containing 1 equivalent the arylamine and 1 equivalent *N,N-*diisopropylethylamine in CH₂Cl₂ or CH₃NO₂ at 0°C. The reaction was held overnight at.room temperature and then stripped free of solvent on a rotary evaporator. The residue was dissolved in ethyl acetate, washed with 5% citric acid, followed by saturated aqueous NaCl, dried over magnesium sulfate or sodium sulfate and then the solution was stripped free of solvent on a rotary evaporator to yield the crude product, which was then purified by chromatography.

### GENERAL PROCEDURE AN

### BOC Removal

The BOC-protected compound was added to a 1:1 mixture of CH₂Cl₂ and trifluoroacetic acid, and was stirred until tlc indicated complete conversion, typically 2h. The solution was then stripped to dryness and the residue was taken up in ethyl acetate and extracted with dilute HCl. The acid reaction was neutralized and extracted with ethyl acetate. The organic phase was washed with saturated aqueous NaCl and dried over MgSO₄. The solution was stripped free of solvent on a rotary evaporator to yield the product.

### GENERAL PROCEDURE AO

### Synthesis of Pyruvate Esters

To a mixture of pyruvic acid (8.8 g, 0.1 mol) (Aldrich) in 100 mL of benzene was added *iso*-butanol (14.82 g, 0.2 mol) and a catalytic amount of *p*-toluenesulfonic acid. The mixture was then refluxed using a Dean Stark apparatus. After 4 hours, the reaction appeared to be complete with the isolation of 1.8 g (0.1 mol) of water. The benzene and *iso*-butanol were removed on a rotary evaporator. The residue (14 g, 0.1 mol), which was primarily the pyruvate *iso*-butyl ester by nmr [¹H-Nmr (CDCl₃): δ = 4.0 (d, 2H), 2.5 (s, 3H), 2.0 (m, 1H), 1.0 (d, 6H)]; was used without further purification. By substituting other alcohols in place of *iso*-butanol (e.g., ethanol, isopropanol, *n-*butanol, benzyl alcohol and the like), other esters of pyruvic acid can be prepared in a similar manner.

### GENERAL PROCEDURE AP

### Aromatic Nucleophilic Substitution of Fluorobenzenes

A mixture of 1.82 g (10 mmol) of D,L-alanine *iso*-butyl ester hydrochloride, the fluorobenzene (10 mmol) and 3 g of anhydrous potassium carbonate in 10 mL of DMSO was stirred at 120°C for 2-5 hours. The reaction mixture was then cooled to room temperature and diluted with 100 mL of ethyl acetate. The ethyl acetate extract was washed with water (3x), dried over MgSO₄ and evaporated to dryness to afford the crude product, which was further purified by column chromatography.

### GENERAL PROCEDURE AQ

### Fourth Transesterification Technique

The ester to be transesterified was dissolved in a large excess of the alcohol and 0.3 equivalents of titanium(IV) isopropoxide (Aldrich) was added. The reaction was followed by tlc until complete and then the volatiles were removed at reduced pressure. The resulting crude material was then chromatographed to obtain the desired product.

### GENERAL PROCEDURE AR

### Synthesis on N-BOC Anilines

To a solution of the aniline in THF was added dropwise 1 equivalent of di-*tert*-butyl dicarbonate (Aldrich) in THF and then 1.5 equivalents of 10N aqueous sodium hydroxide at 0°C. After stirring at room temperature for 16 hours, or heating at 80°C for 3 hours, if needed, the reaction mixture was diluted with ether and washed with NaHCO₃, brine, dried over sodium sulfate and potassium carbonate, concentrated at reduced pressure and chromatographed to afford the *N*-BOC aniline.

### GENERAL PROCEDURE AS

### Oxime Ester Formation

The trichlorophenyl ester (1 eq.) was stirred in DMF or THF. The oxime (1.2 eq.) was added and the mixture was stirred at ambient temperature for 1 to 4 hours. The resulting mixture was concentrated under reduced pressure and the residue was purified by silica gel chromatography and/or crystallization.

### Example AA.

### Synthesis of D,L-alanine iso-butyl ester hydrochloride

A mixture of 35.64 g (0.4 mol) of D,L-alanine (Aldrich), 44 mL (0.6 mol) of thionyl chloride (Aldrich) and 200 mL of *iso*-butanol was refluxed for 1.5 hours. The volatiles were removed at reduced pressure at 90°C under reduced pressure to give the title compound as an oil, which was used without further purification.
NMR data was as follows:
¹H-nmr (CDCl₃): δ = 8.72 (br s, 3H), 4.27 (q, J = 7.4 Hz, 1H), 3.95 (m, 2H), 1.96 (s, 1H), 1.73 (d, J = 7.2 Hz, 3H), 0.92 (d, J = 6.7 Hz, 6H).
¹³C-nmr (CDCl₃): δ = 170.0, 72.2, 49.2, 27.5, 18.9, 16.1.

### Example AB

### Synthesis of N-(3,4-dichlorophenyl)alanine

Using the procedure set forth in U.S. Patent No. 3.598,859, the disclosure of which is incorporated herein by reference in its entirety, N-(3,4-dichlorophenyl)alanine was prepared. Specifically, to a solution of 3,4-dichloroaniline (1 equivalent) (Aldrich) in isopropanol (about 500 mL per mole of 3,4-dichloroaniline) is added water (about 0.06 mL per mL of isopropanol) and 2-chloropropionic acid (2 equivalents) (Aldrich). This mixture is warmed to 40°C and sodium bicarbonate (0.25 equivalents) is added in successive portions before heating under reflux for 4-5 days. After cooling, the reaction mixture is poured into water and the unreacted 3,4-dichloroaniline is removed by filtration. The filtrate is acidified to pH 3-4 with concentrated hydrochloric acid and the resultant precipitate is filtered, washed and dried to yield the title compound, m.p. = 148-149°C.

Alternatively, following General Procedure AF above and using N-(3,4-dichlorophenyl)alanine ethyl ester (from Example A1 below), the title compound was prepared.

### Example AC

### Synthesis of N-(3,5-difluorophenyl)alanine

Using the procedure set forth in U.S. Patent No. 3,598,859, N-(3,5-difluorophenyl)alanine was prepared using 3,5-difluoroaniline (Aldrich) and 2-chloropropionic acid (Aldrich).

### Example AD

### Synthesis of Iso-butyl 2-bromopropionate

To a mixture of *iso*-butanol and 1.0 equivalent of pyridine, in dry diethyl ether was added dropwise 1.3 equivalents of 2-bromopropionyl bromide at 0°C. After stirring at room temperature for 16 hours, the reaction was diluted with diethyl ether, washed with 1N HCl, water, aqueous NaHCO₃, brine and dried over magnesium sulfate or sodium sulfate. Removal of the solvents at reduced pressure gave the title compound as a clear oil.

### Example AE

### Synthesis of N-(2-naphthyl)alanine 2,4,5-trichlorophenyl ester

N-(2-Naphthyl)alanine methyl ester (5.0 g, 20.6 mmol) (from Example A44 below) was dissolved in dioxane (100 mL). NaOH (30 mL, IN) was added and the resulting solution was stirred for 1 hour. The reaction mixture was concentrated under reduced pressure. The resulting solid was dissolved in water and the aqueous mixture was washed with ether. The aqueous portion was adjusted to pH 3 with 1N HCl and extracted with ethyl acetate. The organic extracts were dried over magnesium sulfate or sodium sulfate and concentrated under reduced pressure to yield a white solid (4.35 g, 98%).

The resulting solid (4.35 g, 20 mmol) was dissolved in dichloromethane (300 mL). 2,4,5-Trichlorophenol (4.9 g, 25 mmol) (Aldrich) was added followed by dicyclohexylcarbodiimide (25 mL, 1M in dichloromethane) (Aldrich). After stirring for 18 hours, the mixture was filtered and concentrated to provide an oil which was purified by chromatography on silica gel using chloroform as the eluant (R_{f} = 0.6). The title compound was obtained as a thick oil which slowly crystallized.

### Example A1

### Synthesis of N-(3,4-dichlorophenyl)alanine ethyl ester

Following General Procedure AA above and using 3,4-dichloroaniline (Aldrich) and ethyl pyruvate (Aldrich), the title compound was prepared as an oil. The reaction was monitored by tlc on silica gel (Rf = 0.4 in 25% EtOAc/hexanes) and purification was by preparative plate chromatography (silica gel using 25% EtOAc/hexanes as the eluant).
NMR data was as follows:
¹H-nmr (CDCl₃): δ = 7.2 (d, 1H); 6.7 (d, 1H,); 6.4 (dd, 1H); 4.30 (bs, 1H); 4.2 (q, 2H); 4.1 (q, 1H); 1.5 (d, 3H); 1.3 (t, 3H).
¹³C-nmr (CDCl₃): δ = 175; 146.7; 133; 131; 121; 114.9; 112.6; 72.0; 52.4; 28.3; 19.5.
C₁₁H₁₃Cl₂NO₂ (MW = 262.14).

### Example A2

### Synthesis of N-(3-trifluoromethyl-4-chloro phenyl)alanine ethyl ester

Following General Procedure AA above and using 4-chloro-3-(trifluoromethyl)aniline (Aldrich) and ethyl pyruvate (Aldrich), the title compound was prepared.
Analysis: Calc.: C, 48.74; H, 4.43; N, 4.74. Found: C, 48.48; H, 4.54; N, 4.94.
C₁₂H₁₃F₃ClNO₂ (MW= 295.69); mass spectroscopy (MH⁺) 295.

### Example A3

### Synthesis of N-(3,5-dichlorophenyl)alanine ethyl ester

Following General Procedure AA above and using 3,5-dichloroaniline (Aldrich) and ethyl pyruvate (Aldrich), the title compound was prepared.
Analysis: Calc.: C, 50.40; H, 5.00; N, 5.34. Found: C, 50.50; H, 5.06; N, 5.25.
C₁₁H₁₃Cl₂NO₂ (MW = 262.14); mass spectroscopy (MH⁺) NA.

### Example A4

### Synthesis of N-(3,4-difluorophenyl)alanine ethyl ester

Following General Procedure AA above and using 3,4-difluoroaniline (Aldrich) and ethyl pyruvate (Aldrich), the title compound was prepared. The reaction was monitored by tlc on silica gel (Rf = 0.4 in 25% EtOAc/hexanes) and purification was by preparative plate chromatography (silica gel using 25% EtOAc/hexanes as the eluant).
NMR data was as follows:
¹H-nmr (CDCl₃): δ = 7.4 (m, 1H), 6.8 (d, 1H), 6.5 (m, 1H), 4.30 (bs, 1H), 4.2 (q, 2H), 4.1 (q, 1H), 1.5 (d, 3H), 1.3 (t, 3H).
¹³C-nmr (CDCl₃): δ = 175, 146.7, 135, 132, 125, 116, 113, 72, 52, 28, 19.
C₁₁H₁₃F₂NO₂ (MW = 229.23); mass spectroscopy (MH⁻) 230.

### Example A5

### Synthesis of N-(3,4-dichlorophenyl)alanine benzyl ester

Following General Procedure AA above and using 3,4-dichloroaniline (Aldrich) and benzyl pyruvate (prepared by following General Procedure AO above using benzyl alcohol in place off *iso*-butanol), the title compound was prepared as an oil. The reaction was monitored by tlc on silica gel (Rf = 0.4 in 25% EtOAc/hexanes) and purification was by preparative plate chromatography (silica gel using 25% EtOAc/hexanes as the eluant).
NMR data was as follows:
¹H-nmr (CDCl₃): δ = 7.18 (d, 1H); 7.0 (m, 5H); 6.6 (d, 1H,); 6.4 (dd, 1H); 5.1 (s, 2H); 4.30 (bs, 1H); 4.08 (q, 1H); 1.94 (m, 1H); 1.47 (d, 3H); 0.91 (d, 6H).
¹³C-nmr (CDCl₃): δ = 174.5; 146.7; 133.5; 131.3; 121.3; 120.1; 114.9; 113.6; 72.0; 60.1; 52.4; 28.3; 19.5; 19.3.
C₁₆H₁₅C₁₂NO₂ (MW = 324.31); mass spectroscopy (MH⁺) 325.

### Example A6

### Synthesis of N-(3,4-dichlorophenyl)alanine iso-butyl ester

Following General Procedure AA above and using 3,4-dichloroaniline (Aldrich) and *iso*-butyl pyruvate (prepared by following General Procedure AO above), the title compound was prepared as an oil. The reaction was monitored by tlc on silica gel (Rf = 0.55 in 25% EtOAc/hexanes) and purification was by preparative plate chromatography (silica gel using 25% EtOAc/hexanes as the eluant).
NMR data was as follows:
¹H-nmr (CDCl₃): δ = 7.18 (d, 1H, J=8.7 Hz), 6.66 (d, 1H, J=2.7 Hz), 6.43 (dd, 1H, J = 8.7 Hz, J = 2.7 Hz), 4.30 (bs, 1H), 4.08 (q, 1H, J = 6.9 Hz), 1.94 (sept, 1H, J = 6.7 Hz), 1.47 (d, 3H, J = 6.9 Hz), 0.91 (d, 6H, J = 6.6 Hz).
¹³C-nmr (CDCl₃) δ = 174.5, 146.7, 133.5, 131.3, 121.3, 114.9, 113.6, 72.0, 52.4, 28.3, 19.5, 19.3.
C₁₃H₁₇Cl₂NO₂ (MW = 290.19); mass spectroscopy (MH⁻) 290.

### Example A7

### Synthesis of N-(3,4-dichlorophenyl)alanine iso-propyl ester

Following General Procedure AA above and using 3,4-dichloroaniline (Aldrich) and isopropyl pyruvate (prepared by following General Procedure AO above using isopropanol in place of *iso*-butanol), the title compound was prepared as an oil. The reaction was monitored by tlc on silica gel (Rf = 0.4 in 25% EtOAc/hexanes) and purification was by preparative plate chromatography (silica gel using 25% EtOAc/hexanes as the eluant).
NMR data was as follows:
¹H-nmr (CDCl₃): δ = 7.18 (d, 1H); 6.66 (d, 1H,); 6.43 (dd, 1H); 4.30 (bs, 1H); 4.08 (m, 1H); 1.94 (m, 1H); 1.47 (d, 3H); 0.91 (d, 6H).
¹³C-nmr (CDCl₃): δ = 174.5; 146.7; 133.5; 131.3; 121.3; 114.9; 113.6; 72.0; 52.4; 19.5.
C₁₂H₁₅Cl₂NO₂ (MW 276.16); mass spectroscopy (MH⁻) 277.

### Example A8

### Synthesis of N-(3,4-dichlorophenyl)alanine n-butyl ester

Following General Procedure AA above and using 3,4-dichloroaniline (Aldrich) and *n-*butyl pyruvate (prepared by following General Procedure AO above using *n-*butanol in place of *iso*-butanol), the title compound was prepared. The reaction was monitored by tic on silica gel (Rf = 0.7 in 25% EtOAc/hexanes) and purification was by preparative plate chromatography (silica gel using 25% EtOAc/hexanes as the eluant).
NMR data was as follows:
¹H-nmr (CDCl₃): δ = 7.18 (d, 1H); 6.66 (d, 1H,); 6.43 (dd, 1H); 4.30 (bs, 1H); 4.2 (m, 2H); 4.08 (q, 1H); 1.94 (m, 1H); 1.47 (m, 4H); 0.91 (t, 3H).
¹³C-nmr (CDCl₃): δ = 174.5; 146.7; 133.5; 131.3; 121.3; 114.9; 113.6; 72.0; 52.4; 28.3; 20.2; 19.5.
C₁₃H₁₇Cl₂NO₂ (MW = 290.19); mass spectroscopy (MH⁻) 291.

### Example A9

### Synthesis of N-(3,4-dichlorophenyl)alanine methyl ester (R,S isomers)

Following General Procedure AA above and using 3,4-dichloroaniline (Aldrich) and methyl pyruvate (Aldrich), the title compound was prepared as an oil. The reaction was monitored by tlc on silica gel (Rf = 0.55 in 25% EtOAc/hexanes) and purification was by flash chromatography (silica gel using 25% EtOAc/hexanes as the eluant).
NMR data was as follows:
¹H-nmr (CDCl₃): δ = 7.19 (d, J = 8.73 Hz, 1H), 6.66 (d, J = 2.75 Hz, 1H), 6.43 (dd, J = 8.73 Hz, 2.80 Hz, 1H), 4.25 (bd, J = 8.25 Hz, 1H), 4.08 (m, 1H), 3.76 (s, 3H), 1.47 (d, J = 6.90 Hz).
¹³C-nmr (CDCl₃) δ = 174.35, 145.96, 132.87, 130.70, 120.76, 114.38, 112.90, 52.43, 51.70, 18.67.
C₁₀H₁₁Cl₂NO₂ (MW = 248.11); mass spectroscopy (MH⁺) 247.

### Example A10

### Synthesis of N-(3,4-dichlorophenyl)alanine cyclopentyl ester

Following transesterification General Procedure AB above and using N-(3,4-dichlorophenyl)alanine methyl ester (from Example A9 above) and cyclopentanol (Aldrich), the title compound was prepared as an oil. The reaction was monitored by silica gel tlc (Rf = 0.66 in 25% EtOAc/hexanes). Purification was by preparative plate chromatography (silica gel using 25% EtOAc/hexanes as the eluant).
NMR data was as follows:
¹H-nmr (CDCl₃): δ = 7.19 (d, 1H, J = 8.7 Hz), 6:66 (d, 1H, J = 2.7 Hz), 6.43 (dd, 1H, J = 8.7 Hz, J = 2.7 Hz), 5.22 (m, 1H), 4.27 (d, 1H, J = 8.1 Hz), 4.02 (quint, 1H, J = 7.5 Hz), 1.74 (m, 8H), 1.43 (d, 3H, J = 6.9 Hz).
¹³C-nmr (CDCl₃): δ = 174.3, 146.7, 133.4, 131.2, 121.2, 114.9, 113.7, 78.9, 52.5. 33.2, 24.2, 24.1, 19.1.
C₁₄H₁₇Cl₂NO₂ (MW = 302.20); mass spectroscopy (MH⁺) 301.

### Example A11.

### Synthesis of N-(3,4-dichlorophenyl)alanine n-propyl ester

Following General Procedure AA above and using 3,4-dichloroaniline (Aldrich) and *n*-propyl pyruvate (prepared by following General Procedure AO above using n-propanol in place of *iso*-butanol), the title compound was - prepared as an oil. The reaction was monitored by tlc on silica gel (Rf = 0.5 in 25% EtOAc/hexanes) and purification was by preparative plate chromatography (silica gel using 25% EtOAc/hexanes as the eluant).
NMR data was as follows:
¹H-nmr (CDCl₃): δ = 7.2 (d, 1H); 6.6 (d, 1H); 6.4 (dd, 1H); 4.30 (bs, 1H); 4.2 (q, 2H); 4.08 (q, 1H); 1.94 (m, 2H); 1.5 (d, 3H); 0.95 (t, 3H).
¹³C-nmr (CDCl₃): δ = 178; 144.7; 130.2; 120.62; 115.11; 71.82; 52.90.
C₁₂H₁₅Cl₂NO₂ (MW = 276.16); mass spectroscopy (MH⁺) 277.

### Example A12

### Synthesis of N-(3,4-dichlorophenyl)alanine allyl ester

Following transesterification General Procedure AB above and using N-(3,4-dichlorophenyl)alanine methyl ester (from Example A9 above) and allyl alcohol (Aldrich), the title compound was prepared as an oil. The reaction was monitored by silica gel tlc (Rf = 0.62 in 25% EtOAc/hexanes). Purification was by preparative plate chromatography (silica gel using 25% EtOAc/hexanes as the eluant).
NMR data was as follows:
¹H-nmr (CDCl₃): δ = 7.19 (d, 1H, J = 8.7 Hz), 6.67 (d, 1H, J =2.8 Hz), 6.44 (dd, 1H, J = 8.7 Hz, J=2.8 Hz), 5.90 (m, 1H), 5.30 (m, 2H), 4.64 (m, 2H), 4.26 (m, 1H, 4.10 (m, 1H), 1.48 (d, 3H, J = 6.9 Hz).
¹³C-nmr (CDCl₃): δ = 174.1, 146.6, 133.5, 132.1, 131.3, 121.4, 119.6, 115.0, 113.6, 66.5, 52.4, 19.3.
C₁₂H₁₃Cl₂NO₂ (MW = 274.15); mass spectroscopy (MH⁺) 273.

### Example A 13

### Synthesis of N-(3,4-dichlorophenyl)alanine 4-methylpentyl ester

Following transesterification General Procedure AB above and using N-(3,4-dichlorophenyl)alanine methyl ester (from Example A9 above) and 4-methylpentanol (Aldrich), the title compound was prepared as an oil. The reaction was monitored by silica gel tlc (Rf = 0.70 in 25% EtOAc/hexanes). Purification was by preparative plate chromatography (silica gel using 25% EtOAc/hexanes as the eluant).
NMR data was as follows:
¹H-nmr (CDCl₃): δ = 7.18 (d, 1H, J = 8.7 Hz), 6.66 (d, 1H, J = 2.7 Hz), 6.43 (dd, 1H, J = 8.7 Hz, J = 2.7 Hz), 4.28 (m, 1H), 4.10 (m, 3H), 1.55 (m, 6H), 1.19 (m, 2H), 0.87 (d, 3H, J = 6.6 Hz).
¹³C-nmr (CDCl₃): δ = 174.6, 146.7, 133.4, 131.3, 121.3, 115.0, 113.6, 66.4, 52.4, 35.4, 28.2, 27.0, 23.0, 19.3.
C₁₅H₂₁Cl₂NO₂ (MW = 318.25); mass spectroscopy (MH⁻) 317.

### Example A14

### Synthesis of N-(3,4-dichlorophenyl)alanine 2,2-dimethyl-1,3-dioxolane-4-methyl ester

Following transesterification General Procedure AB above and using N'(3,4-dichlorophenyl)alanine methyl ester (from Example A9 above) and 2,2-dimethyl-1,3-dioxolane-4-methanol (solketal) (Aldrich), the title compound was prepared as a mixture of diastereomers. The reaction was monitored by silica gel tlc (Rf = 0.32 in 25% EtOAc/hexanes). Purification was by preparative plate chromatography (silica gel using 25% EtOAc/hexanes as the eluant).
NMR data was as follows:
¹H-nmr (CDCl₃): δ = 7.19 (d, 1H, J = 8.7 Hz), 6.66 (d, 1H, 2.7 Hz), 6.43 (dd, 1H, J = 8.7 Hz, J = 2.7 Hz), 4.22 (m, 6H), 3.70 (m, 1H), 1.43 (m, 9H).
¹³C-nmr (CDCl₃): δ = 174.34, 174.32, 146.5, 133.5, 131.3, 121.5, 115.0, 113.6, 110.52, 110.51, 73.97, 73.89, 66.6, 66.01, 65.95, 52.42, 52.37, 27.3, 25.8, 19.3.
C₁₅H₁₉Cl₂NO₄ (MW = 348.23); mass spectroscopy (MH⁺) 347.

### Example A15

### Synthesis of N-(3,4-dichlorophenyl)alanine cyclohexylmethyl ester

Following-transesterification General Procedure AB above and using N-(3,4-dichlorophenyl)alanine methyl ester (from Example A9 above) and cyclohexylmethanol (Aldrich), the title compound was prepared.
NMR data was as follows:
¹H-nmr (CDCl₃): δ = 7.19 (d, 1H), 6.68 (d, 1H), 6.45 (dd, 1H), 4.26 (bd, 1H), 4.10 (m, 1H), 3.95 (d, 2H), 1.70-1.55 (m, 6H), 1.50 (d, 3H), 1.35-0.85 (m, 5H).
¹³C-nmr (CDCl₃): δ = 174.58, 146.72, 133.48, 131.27, 121.34, 114.98, 113.72, 71.06, 52.52, 37.68, 30.10, 26.83, 26.17, 19.32.
C₁₅H₂₁Cl₂NO₂ (MW = 318.25); mass spectroscopy (MH⁺) 317.

### Example A16

### Synthesis of N-(3,4-dichlorophenyl)alanine tert-butyloxycarbonylmethyl ester

Following General Procedure AE above and using N-(3,4-dichlorophenyl)alanine (from Example AB above) and *tert*-butyl bromoacetate (Aldrich), the title compound was prepared as a solid. The reaction was monitored by silica gel tlc (Rf = 0.57 in 25% EtOAc/hexanes). Purification was by recrystallization from ethanol.
NMR data was as follows:
¹H-nmr (CDCl₃): δ = 7.19 (d, 1H), 6.68 (d, 1H), 6.45 (dd, 1H), 4.55 (m, 2H), 4.20 (m, 2H), 1.55 (d, 3H), 1.45 (s, 9H).
¹³C-nmr (CDCl₃): δ = 173.9, 166.9, 146.5, 133.5, 131.3, 115.1, 113.6, 83.4, 62.2, 52.2, 28.6, 19.3.
C₁₅H₁₉Cl₂NO₄ (MW = 348.23); mass spectroscopy (MH⁺) 347.

### Example A17

### Synthesis of N-(3,4-dichlorophenyl)leucine iso-butyl ester

Following General Procedure AA above and using 3,4-dichloroaniline (Aldrich) and *iso*-butyl 4-methyl-2-oxopentanoate (prepared by following General Procedure AO above using 4-methyl-2-oxovaleric acid (Fluka) and *iso*-butanol), the title compound was prepared as an oil. The reaction was monitored by tlc on silica gel (Rf = 0.6 in 25% EtOAc/hexanes) and purification was by preparative plate chromatography (silica gel using 25% EtOAc/hexanes as the eluant).
NMR data was as follows:
¹H-nmr (CDCl₃): δ = 7.2 (d, 1H); 6.5 (d, 1H); 6.4 (dd, 1H); 4.30 (bs, 1H); 4.08 (q, 1H); 3.8(m, 2H); 1.8 (m, 3H); 0.91 (m, 12H).
¹³C-nmr (CDCl₃): δ = 174.5; 146.7; 133.5; 131.3; 121.3; 114.9; 113.6; 72.0; 52; 28.3; 20.1; 19.5.
C₁₆H₂₃Cl₂NO₂ (MW = 332.27); mass spectroscopy (MH⁺) 333.

### Example A18

### Synthesis of 2-[N-(3,4-dichlorophenyl)amino]pentanoic acid iso-butyl ester

Following General Procedure AA above and using 3,4-dichloroaniline (Aldrich) and *iso*-butyl 2-oxopentanoate (prepared by following General Procedure AO above using 2-oxovaleric acid (Fluka) and *iso*-butanol), the title compound was prepared as an oil. The reaction was monitored by tlc on silica gel (Rf = 0.5 in 25% EtOAc/hexanes) and purification was by preparative plate chromatography (silica gel using 25% EtOAc/hexanes as the eluant).
NMR data was as follows:
¹H-nmr (CDCl₃): δ = 7.2 (d, 1H); 6.6 (d, 1H); 6.4 (dd, 1H); 4.3 (d, 1H); 3.8 (m, 3H); 1.9 (m, 6H); 1.0 (t, 3H), 0.9 (m, 6H).
¹³C-nmr (CDCl₃): δ = 178; 144.7; 130.2; 120.62; 115.11; 71.82; 52.90; 28.30; 19.53.
C₁₅H₂₁Cl₂NO₂ (MW = 318.3); mass spectroscopy (MH⁺) 319.

### Example A19

### Synthesis of N-(4-cyanophenyl)alanine iso-butyl ester

Following General Procedure AP above and using 4-fluorobenzonitrile (Aldrich) and D,L-alanine *iso*-butyl ester hydrochloride (from Example AA above), the title compound was prepared as an oil. The product was recovered by column chromatography on silica gel using 1:5 EtOAc/hexanes as the eluant.
NMR data was as follows:
¹H-nmr (CDCl₃): δ = 7.44 (d, J = 8.8 Hz, 2H), 6.57 (d, J = 8.8 Hz, 2H), 4.74 (d, J = 8.1 Hz, 1H), 4.18 (t, J = 7.4 Hz, 1H), 3.95 (m, 2H), 1.94 (m, 1H), 1.51 (d, J = 6.9 Hz, 3H), 0.91 (d, J = 6.7 Hz, 6H).
¹³C-nmr (CDCl₃): δ = 173.4, 149.7, 133.8, 120.1, 112.7, 99.8, 71.6, 51.2, 27.7, 18.9, 18.6.
C₁₄H₁₈N₂O₂ MW = 246.31; mass spectroscopy (MH⁺) 247.

### Example A20

### Synthesis of N-(3-chloro-4-cyanophenyl)alanine iso-butyl ester

Following General Procedure AP above and using 2-chloro-4-fluorobenzonitrile (Aldrich) and D,L-alanine *iso*-butyl ester hydrochloride (from Example AA above), the title compound was prepared. The product was recovered by column chromatography on silica gel using 1:5 EtOAc/hexanes as the eluant.
NMR data was as follows:
¹H-nmr (CDCl₃): δ = 7.40 (d, J = 8.5 Hz, 1H), 6.62 (d, J = 2.3 Hz, 1H), 6.48 (dd, J = 2.4, 8.6 Hz, 1H), 4.90 (d, J = 7.6 Hz, 1H), 4.16 (quintet, J = 7.1 Hz, 1H), 3.96 (dd, J = 2.2, 6.7 Hz, 2H), 1.97 (m, 1H), 1.51 (d, J = 7.0 Hz, 3H), 0.93 (d, J = 6.7 Hz, 6H).
¹³C-nmr (CDCl₃): δ = 173.0, 150.4, 138.3, 134.9, 117.3, 112.8, 111.3, 100.6, 71.7, 51.1, 27.7, 18.9, 18.4.
C₁₄H₁₇N₂O₂Cl MW = 280.76; mass spectroscopy (MH⁺) 281.

### Example A21

### Synthesis of N-(3,4-dichloro)alanine iso-butyl ester (S isomer)

Following General Procedure AM above and using 3,4-dichloroaniline (Aldrich) and *iso*-butyl R-(+)-lactate (Aldrich), the title compound was prepared as an oil. The reaction was monitored by silica gel tlc (Rf = 0.55 in 25% EtOAc/hexanes). Purification was column chromatography.
NMR data was as follows:
¹H-nmr (CDCl₃): δ = 7.19 (d, J = 8.73, 1H), 6.67 (d, J = 2.75, 1H), 6.45 (dd, J = 8.73, J = 2.75, 1H), 4.28 (bd, J = 8.36, 1H), 4.09 (quint, 1H), 3.94 (d, J = 6.66, 2H), 1.95 (hept, J = 6.71, 1H), 1.49 (d, J = 6.90, 3H), 0.92 (d, J = 6.04, 6H).
¹³C-nmr (CDCl₃): δ = 174.57, 146.67, 133.47, 131.28, 121.29, 114.93, 113.63, 71.01, 52.43, 28.30, 19.55, 19.33.
C₁₃H₁₇Cl₂NO₂ (MW = 290.19); mass spectroscopy (MH⁺) 290.

### Example A22

### Synthesis of N-(3,4-dichloro)alanine tetrahydrofuran-3-yl-methyl ester

Following transesterification General Procedure AB above and using N-(3,4-dichlorophenyl)alanine methyl ester (from Example A9 above) and tetrahydro-3-furanmethanol (Aldrich), the title compound was prepared as an oil. The reaction was monitored by silica gel tlc (Rf = 0.33 in 25% EtOAc/hexanes). Purification was by preparative plate chromatography (silica gel using 25% EtOAc/hexanes as the eluant).
NMR data was as follows:
¹H-nmr (CDCl₃): δ = 7.18 (d, 1H, J = 8.7 Hz), 6.65 (d, 1H, J = 2.7 Hz), 6.42 (dd, 1H, J = 8.7 Hz, J = 2.7 Hz), 4.30 (m, 1H), 4.09 (m, 3H), 3.78 (m, 3H), 3.53 (m, 1H), 2.56 (m, 1H), 1.94 (m, 1H), 1.58 (m, 1H), 1.46 (d, 3H, J = 6.9 Hz).
¹³C-nmr (CDCl₃): δ = 174.5, 146.6, 133.5, 131.3, 121.4, 114.9, 113.6, 70.86, 70.83, 68.2, 67.31, 67.29, 52.4, 38.7, 29.36, 29.33, 19.2.
C₁₄H₁₇Cl₂NO₃ (MW = 318.20); mass spectroscopy (MH⁺) 318.

### Example A23

### Synthesis of N-(3,5-dichlorophenyl)alanine n-propyl ester

Following General Procedure AA above and using 3,5-dichloroaniline (Aldrich) and *n*-propyl pyruvate (which can be prepared by following General Procedure AO above using n-propanol in place of *iso*-butanol), the title compound could be prepared.

### Example A24

### Synthesis of 2-[N-(3,4-dichlorophenyl)amino]butanoic acid iso-butyl ester

Following General Procedure AA above and using 3,4-dichloroaniline (Aldrich) and *iso*-butyl 2-oxobutanoate (prepared by following General Procedure AO above using 2-oxobutyric acid (Aldrich) and *iso*-butanol), the title compound was prepared as an oil. The reaction was monitored by tlc on silica gel (Rf = 0.3 in 25% EtOAc/hexanes) and purification was by preparative plate chromatography (silica gel using 25% EtOAc/hexanes as the eluant).
NMR data was as follows:
¹H-nmr (CDCl₃): δ = 7.2 (d, 1H); 6.6 (d, 1H); 6.4 (dd, 1H); 4.3 (d, 1H); 3.8 (m, 3H); 1.9 (m, 3H); 1.0 (t, 3H); 0.9(m, 6H).
¹³C-nmr (CDCl₃): δ = 178; 144.7; 130.2; 120.62; 115.11; 71.82; 52.90; 28.30; 20.5; 19.53.
C₁₄H₁₉Cl₂NO₂ (MW = 304.22); mass spectroscopy (MH⁺) 305.

### Example A25

### Synthesis of N-(4-chlorophenyl)alanine iso-butyl ester

Following General Procedure AA above and using 4-chloroaniline (Aldrich) and *iso*-butyl pyruvate (prepared by following General Procedure AO above), the title compound was prepared as an oil. The reaction was monitored by tlc on silica gel (Rf = 0.6 in 25% EtOAc/hexanes) and purification was by preparative plate chromatography (silica gel using 25% EtOAc/hexanes as the eluant).
NMR data was as follows:
¹H-nmr (CDCl₃): δ = 7.18 (d, 2H), 6.66 (d, 2H), 4.30 (bs, 1H), 4.08 (q, 1H), 1.94 (sept, 1H), 1.47 (d, 3H), 0.91 (d, 6H).
¹³C-nmr (CDCl₃): δ =174.5, 146.7, 133.5, 131.3, 121.3, 114.9, 113.6, 72.0, 52.4, 28.3, 19.5, 19.3.
C₁₃H₁₈ClNO₂ (MW = 255.75); mass spectroscopy (MH⁺) 256.

### Example A26

### Synthesis of N-(3,5-dichlorophenyl)alanine iso-butyl ester

Following General Procedure AA above and using 3,5-dichloroaniline (Aldrich) and *iso*-butyl pyruvate (prepared by following General Procedure AO above), the title compound was prepared as an oil. The reaction was monitored by tic on silica gel (Rf = 0.4 in 25% EtOAc/hexanes) and purification was by preparative plate chromatography (silica gel using 25% EtOAc/hexanes as the eluant).
NMR data was as follows:
¹H-nmr (CDCl₃): δ = 7.18 (d, 2H), 6.66 (m, 1H), 4.30 (bs, 1H), 4.08 (q, 1H), 1.94 (m, 1H), 1.47 (d, 3H), 0.91 (d, 6H).
¹³C-nmr (CDCl₃): δ = 175; 146.7; 133; 131; 121; 114.9; 112.6; 72.0; 52.4; 28.3; 19.5.
C₁₃H₁₇Cl₂NO₂ (MW = 290.2); mass spectroscopy (MH⁺) 291.

### Example A27

### Synthesis of N-(4-ethylphenyl)alanine methyl ester

A solution of 0.68 g (5 mmol) of 4'-aminoacetophenone (Aldrich), 0.60 mL of 90% methyl pyruvate (Aldrich) and 0.05 g (0.25 mmol) of *p*-toluenesulfonic acid in ethanol was hydrogenated in the presence of a catalytic amount of 10% Pd/C at from 30 to 15 psi of hydrogen for 16 hours. The catalyst was removed by filtering the reaction mixture through Celite and the solvent was evaporated to provide the crude product. The product was purified by column chromatography (silica gel using 1:9 EtOAc/hexanes as the eluant) to provide the title compound.
NMR data was as follows:
¹H-nmr (CDCl₃): δ = 1.19 (t, J = 7.6 Hz, 3H), 1.47 (d, J = 6.8 Hz, 3H), 2.54 (q, J = 7.6 Hz, 2H), 3.74 (s, 3H), 4.04 (bs, 1H), 4.13 (m, 1H), 6.57 (d, J = 8.5 Hz, 2H), 7.03 (d, J = 8.4 Hz, 2H).
¹³C-nmr (CDCl₃): δ = 15.8, 18.0, 27.9, 52.17, 52.19, 113.5, 128.6, 134.1, 144.4, 175.3.
C₁₂H₁₇NO₂ MW = 207.27; mass spectroscopy (MH⁻) 208.

### Example A28

### Synthesis of N-(4-(1-ethoxy)ethylphenyl)alanine methyl ester

Following the procedure for Example A27 above, the title compound was isolated as another reaction product by column chromatography (silica gel using 1:9 EtOAc/hexanes as the eluant).
NMR data was as follows:
¹H-nmr (CDCl₃): δ = 1.15 (t, J = 7.0 Hz, 3H), 1.40 (d, J = 6.5 Hz, 3H), 1.47 (d, J = 6.1 Hz, 3H), 3.31 (q, J = 5.1 Hz, 2H), 3.74 (s, 3H), 4.14 (m, 2H), 4.29 (q, J = 6.4 Hz, 1H), 6.57 (d, J = 8.5 Hz, 2H), 7.12 (d, J = 8.4 Hz, 2H).
¹³C-nmr (CDCl₃): δ = 15.4, 19.0, 23.9, 51.9, 52.2, 63.4, 77.3, 113.1, 127.3, 133.6. 145.8, 175.1.
C₁₄H₂₁NO₃ MW = 251.33; mass spectroscopy (MH⁺) 251.

### Example A29

### Synthesis of N-(3,4-dichloro)alanine 2,2-dimethylpropyl ester (R,S isomers)

Following transesterification General Procedure AQ above and using N-(3,4-dichlorophenyl)alanine methyl ester (from Example A9 above) and neopentyl alcohol (Aldrich), the title compound was prepared. The reaction was monitored by silica gel tlc (Rf = 0.72 in 25% EtOAc/hexanes). Purification was by flash chromatography (silica gel using 25% EtOAc/hexanes as the eluant).
NMR data was as follows:
¹H-nmr (CDCl₃): δ = 7.19 (d, 1H, J = 8.7 Hz), 6.68 (d, 1H, J = 2.7 Hz), 6.45 (dd, 1H, J = 8.7 Hz, J = 2.7 Hz), 4.29 (m, 1H), 4.11 .(m, 1H), 3.85 (m, 2H), 1.49 (d, 3H, J = 6.9 Hz), 0.93 (s, 9H).
¹³C-nmr (CDCl₃): δ = 174.6, 146.7, 133.5, 131.3, 121.3, 114.9, 113.7, 75.2, 52.4, 32.0, 26.9, 19.4.
C₁₄H₁₉Cl₂NO₂ (MW = 304.22); mass spectroscopy (MH⁺) 303.

### Example A30

### Synthesis of N-(3,4-dichlorophenyl)glycine iso-butyl ester

3,4-Dichloroaniline (Aldrich) was treated with di-*tert*-butyl dicarbonate (Aldrich) using conventional procedures to produce the N-BOC aniline. The N-BOC aniline was treated with sodium hydride in THF and then with *iso*-butyl 2-bromoacetate (from Example AD above) to produce the N-BOC N-(3,4-dichlorophenyl)glycine *iso*-butyl ester. The BOC group was then removed using General Procedure AN above to afford the title compound. The reaction was monitored by tic on silica gel (Rf = 0.78 in 50% EtOAc/hexanes) and purification was by preparative plate chromatography (silica gel using 50% EtOAc/hexanes as the eluant).
NMR data was as follows:
¹H-nmr (CDCl₃): δ = 7.19 (dd, J=4.1, 4.7, 3.4, 1H); 6.65 (d, J=2.7, 1H); 6.44 (dd, J=2.7, 4.5, 4.2, 1H): 4.4 (m, 1H): 3.97 (dd, J=3.6, 3.0, 2.3, 2H); 3.87 (s, 2H); 1.9 (m, 1H); 0.93 (d, J=6.7, 6H).
¹³C-nmr (CDCl₃): δ = 171.2, 147.0. 133.5, 131.3, 121.2, 114.5, 113.3, 72.2, 46.0, 28.2, 19.6.
C₁₂H₁₅Cl₂NO₂ (MW = 276); mass spectroscopy (MH⁺) 277.

### Example A31

### Synthesis of N-(3,4-dichlorophenyl)alanine 2-ethylbutyl ester

Following General Procedure AA above and using 3,4-dichloroaniline (Aldrich) and 2-ethylbutyl pyruvate (prepared by following General Procedure AO above using 2-ethylbutanol (Aldrich) in place of *iso*-butanol), the title compound was prepared as an oil. The reaction was monitored by tlc on silica gel (Rf = 0.6 in 25% EtOAc/hexanes) and purification was by preparative plate chromatography (silica gel using 25% EtOAc/hexanes as the eluant).
NMR data was as follows:
¹H-nmr (CDCl₃): δ = 7.2 (d, 1H); 6.6 (d, 1H); 6.4 (dd, 1H); 4.2 (t, 2H); 4.1 (q, 1H); 1.5 (d, 3H); 1.4 (m, 4H); 1.0 (m, 6H).
¹³C-nmr (CDCl₃): δ = 178; 144.7; 130.2; 120.62; 115.11; 70.7; 51.90; 26.3; 19.53, 18.5.
C₁₅H₂₁Cl₂NO₂ (MW = 318.25); mass spectroscopy (MH⁺) 319.

### Example A32

### Synthesis of N-(3-chloro-4-iodophenyl)alanine iso-butyl ester

Following General Procedure AR above and using 3-chloro-4-iodoaniline (Aldrich), *N*-BOC-3-chloro-4-iodoaniline was prepared. To a stirred slurry of 5.0 equivalents of sodium hydride in DMF was added 1.0 equivalent of *N*-BOC-3-chloro-4-iodoaniline and then 1.1 equivalents of *iso*-butyl 2-bromopropionate (from Example AD above) were slowly added. The reaction was heated to 100°C for 10 hours, cooled, diluted with dichloromethane and washed with cold 1N HCl, water and brine. The solvents were removed at reduced pressure and the residue was chromatographed to provide *N*-BOC-*N*-(3-chloro-4-iodophenyl)alanine *iso*-butyl ester as a clear oil. Following General Procedure AN above, the BOC group was removed from *N*-BOC-*N*-(3-chloro-4-iodophenyl)alanine *iso*-butyl ester to provide the title compound. The BOC-removal reaction was monitored by tlc on silica gel (Rf = 0.58 in 30% EtOAc/hexanes) and purification was by preparative plate chromatography (silica gel using 30% EtOAc/hexanes as the eluant). The compound was further purified by chromatography on an HPLC chiral column (Chiralcel OD).
NMR data was as follows:
¹H-nmr (CDCl₃): δ = 7.52 (d, J=8.7, 1H); 6.72 (d, J=2.7, 1H); 6.25 (dd, J=2.7, 5.9, 2.7, 1H); 4.35 (d, J=6.6, 1H): 4.08 (quintex, J=7.2, 6.7, 1H); 3.93 (d, J=6.7, 2H): 1.94 (m, 1H); 1.47 (d, J=6.9, 3H); 0.92 (d, J=6.9, 6H).
¹³C-nmr (CDCl₃): δ = 174.5, 148.3, 140.7, 139.5, 114.4, 114.3, 82.6, 72.0, 52.2, 28.3, 19.6, 19.3.
C₁₃H₁₇ClINO₂ (MW = 381.5); mass spectroscopy (MH⁻) 382.

### Example A33

### Synthesis of N-(4-azidophenyl)alanine iso-butyl ester

Following General Procedure AA above and using 4-azidoaniline (Aldrich) and *iso*-butyl pyruvate (prepared by following General Procedure AO above), the title compound was prepared as an oil. The reaction was monitored by tlc on silica gel (Rf = 0.3 in 25% EtOAc/hexanes) and purification was by preparative plate chromatography (silica gel using 25% EtOAc/hexanes as the eluant).
NMR data was as follows:
¹H-nmr (CDCl₃): δ = 7.3 (d, 2H), 6.8 (d, 2H), 4.30 (bs, 1H), 4.08 (q, 1H), 1.94 (sept, 1H), 1.47 (d, 3H), 0.91 (d, 6H).
¹³C-nmr (CDCl₃): δ = 174.5, 148.7, 131.5, 130.3, 121.3, 114.9, 113.6, 72.0, 52.4, 28.3, 19.5, 19.3.
C₁₃H₁₈N₄O₂ (MW = 262.31); mass spectroscopy (MH⁻) 263.

### Example A34

### Synthesis of N-[(4-phenylcarbonyl)phenyl]alanine iso-butyl ester

Following General Procedure AA above and using 4'-aminobenzophenone (Aldrich) and *iso*-butyl pyruvate (prepared by following General Procedure AO above), the title compound was prepared as an oil. The reaction was monitored by tlc on silica gel (Rf = 0.4 in 25% EtOAc/hexanes) and purification was by preparative plate chromatography (silica gel using 25% EtOAc/hexanes as the eluant).
NMR data was as follows:
¹H-nmr (CDCl₃): δ = 7.7 (d, 2H), 7.1 (m, 5H), 6.9 (d, 2H), 4.30 (bs, 1H), 4.08 (q, 1H), 1.94 (sept, 1H), 1.47 (d, 3H), 0.91 (d, 6H).
¹³C-nmr (CDCl₃): δ = 199, 178.5, 149.7, 131.5, 130.3, 126, 121.3, 114.9, 113.6, 72.0, 52.4, 28.3, 19.5, 19.3.
C₂₀H₂₃NO₃ (MW = 325.41); mass spectroscopy (MH⁻) 326.

### Example A35

### Synthesis of N-(3,5-difluorophenyl)alanine iso-butyl ester

Following General Procedure AH above and using N-(3,5-difluorophenyl)alanine (from Example AC above) and *iso*-butanol, the title compound was prepared as an oil. The reaction was monitored by tlc on silica gel (Rf = 0.9 in 3% methanol/methylene chloride) and purification was by preparative plate chromatography (silica gel using 3% methanol/methylene chloride as the eluant).
NMR data was as follows:
¹H-nmr (CDCl₃): δ = 6.1 (m, 3H), 4.5 (bs, 1H), 4.1 (d, 1H), 3.95 (m, 2H), 2.0 (m, 1H), 1.5 (d, J = 7 Hz, 3H), 0.95(d, J = 6 Hz, 6H).
¹³C-nmr (CDCl₃): δ = 174.44, 166.40, 166.19, 163.16, 162.95, 149.43, 96.73, 96.60, 96.48, 96.35, 94.06, 93.72, 93.37, 72.03, 52.30, 28.29, 19.47, 19.23.
C₁₃H₁₇F₂NO₂ (MW = 290.2); mass spectroscopy (MH⁻) 291.

### Example A36

### Synthesis of N-(3,4-dichlorophenyl)alanine O-acylacetamidoxime ester

Following General Procedure AK above and using N-(3,4-dichlorophenyl)alanine (from Example AB above) and acetamide oxime (prepared according to the procedures described in J. Org. Chem., 46, 3953 (1981)), the title compound was prepared as a semisolid. The reaction was monitored by tic on silica gel (Rf = 0.4 in ethyl acetate) and purification was by preparative plate chromatography (silica gel using ethyl acetate as the eluant).
NMR data was as follows:
¹H-nmr (DMSO-*d*₆): δ = 7.27 (d, 1H), 6.81 (s, 1H) 6.4 (broad s, 2H), 6.62 (d, 1H), 6.45 (d, 1H), 4.22 (m, 1H), 1.74 (s, 3H), 1.40 (d, 3H).
C₁₁H₁₃Cl₂N₃O₂ (MW = 290.15); mass spectroscopy (MH⁺) 291.

### Example A37

### Synthesis of N-(3,4-dichlorophenyl)alanine pyrrolyl amide

Following General Procedure AL above and using N-(3,4-dichlorophenyl)alanine (from Example AB above) and pyrrole (Aldrich), the title compound was prepared as an oil. The reaction was monitored by tic on silica gel (Rf = 0.28 in 10% ethyl acetate/hexanes) and purification was by preparative plate chromatography (silica gel using 10% ethyl acetate/hexanes as the eluant).
NMR data was as follows:
¹H-nmr (CDCl₃): δ = 7.36 (d, J=2.2, 2H); 7.20 (d, J=8.7, 1H); 6.71 (d, J=2.7, 1H); 6.5 (m, 1H); 6.38 (t, J=2.4, 2H); 4.8 (m, 1H); 4.57 (d, J=8.7, 1H); 1.59 (d, J=6.8, 3H).
¹³C-nmr (CDCl₃): δ = 171.9, 146.1, 133.6, 131.5, 121.9, 119.6, 115.4, 114.7, 113.8. 51.8, 20.2.
C₁₃H₁₂Cl₂N₂O (MW = 283); mass spectroscopy (MH⁻) 284.

### Example A38

### Synthesis of N-(3,4-dichlorophenyl)alanine O-acylbutyramideoxime ester

Following General Procedure AI above and using N-(3,4-dichlorophenyl)alanine 2,4,6-trichlorophenyl ester (prepared from N-(3,4-dichlorophenyl)alanine methyl ester (from Example A9) using essentially the same procedure as described in Example AE above) and butyramide oxime (prepared according to the procedures described in J. Org. Chem., 46, 3953 (1981)), the title compound was prepared as a semisolid. The reaction was monitored by tic on silica gel (Rf = 0.25 in 50% ethyl acetate/hexanes) and purification was by preparative plate chromatography (silica gel using 50% ethyl acetate/hexanes as the eluant).
NMR data was as follows:
¹H-nmr (*d₆*-DMSO): δ = 7.27 (d, 1H), 6.83 (s, 1H) 6.38 (broad s, 2H), 6.61 (d, 1H), 6.46 (d, 1H), 4.25 (m, 1H), 2.02 (t, 2H), 1.55 (m, 2H), 1.40 (d, 3H), 0.88 (t, 3H).
C₁₃H₁₇Cl₂N₃O₂ (MW = 318.20); mass spectroscopy (MH⁻) 319.

### Example A39

### Synthesis of 2-[N-(napth-2-yl)amino]butanoic acid ethyl ester

Following General Procedure AJ above and using 2-aminonaphthalene (Aldrich) and ethyl 2-bromobutyrate (Aldrich), the title compound was prepared as a solid, m.p. 81-83°C. The reaction was monitored by silica gel tlc (Rf = 0.5 in CHCl₃). Purification was by chromatography (silica gel using chloroform as the eluant).
NMR data was as follows: ¹H-nmr (d⁶-DMSO): δ = 7.63 (m, 2H), 7.54 (d, 1H), 7.31 (t, 1H), 7.12 (t, 1H), 7.03 (d, 1H), 6.62 (s, 1H), 6.32 (d, 1H), 4.15 (m, 3H), 1.42 (d, 3H), 1.19 (t, 3H).
C₁₆H₁₉NO₂ (MW = 257.34); mass spectroscopy (MH⁻) 258.

### Example A40

### Synthesis of N-(2-naphthyl)alanine iso-butyl ester

Following General Procedure AA above and using 2-aminonaphthalene (Aldrich) and *iso*-butyl pyruvate (prepared by following General Procedure AO above), the title compound was prepared as an oil. Purification was by preparative plate chromatography (silica gel using 25% EtOAc/hexanes as the eluant).
NMR data was as follows:
¹H-nmr (CDCl₃): δ = 7.65 (m, 3H), 7.38 (t, 1H, J = 6.9 Hz), 7.23 (t, 1H, J. = 6.9 Hz), 6.93 (m, 1H), 6.81 (d, 1H, J = 2.3 Hz), 4.31 (q, 1H, J =6.9 Hz), 3.95 J = 6.7 Hz, J = 1.6 Hz), 1.96 (sept, 1H, J = 6.7 Hz), 1.57 (d, 3H, J = 6.9 Hz), 0.93 (dd, 6H, J = 6.7 Hz, J = 1.6 Hz).
¹³C-nmr (CDCl₃) δ = 174.6, 144.2, 134.9, 129.1, 127.8, 127.6, 126.3, 126.0, 122.3, 118.1, 105.3, 71.2, 52.0, 27.7, 18.9, 18.8.

### Example A41

### Synthesis of N-(2-methylquinolin-6-yl)alanine iso-butyl ester

Following General Procedure AA above and using 6-amino-2-methylquinoline (Lancaster) and *iso*-butyl pyruvate (prepared by following General Procedure AO above), the title compound was prepared. The reaction was monitored by silica gel tlc (Rf = 0.44 in 50% EtOAc/hexanes). Purification was by flash chromatography (silica gel using 50% EtOAc/hexanes as the eluant).
NMR data was as follows:
¹H-nmr (CDCl₃): δ = 7.90 (m, 2H), 7.10 (m, 2H), 6.66 (d, 1H, J = 2.6), 4.50 (bd, 1H), 4.24 (m, 1H), 3.91 (d, 2H, J = 6.6 Hz), 2.64 (s, 3H), 1.91 (sept, 1H,J=6.7Hz), 1.52 (d, 3H, J = 6.9 Hz), 0.87 (d, 6H, J = 6.7 Hz).
¹³C-nmr (CDCl₃) δ = 175.0, 155.4, 144.6, 143.4, 134.9, 130.2, 128.4, 122.8, 121.8,104.9, 71.8, 52.7, 28.3, 25.4, 19.5, 19.4.
C₁₇H₂₂Cl₂N₂O₂ (MW = 286.38); mass spectroscopy (MH⁻) 287.

### Example A42

### Synthesis of N-(3,4-methylenedioxyphenyl)alanine iso-butyl ester

Following reductive amination General Procedure AA above and using 3,4-methylenedioxyaniline (Aldrich) and methyl pyruvate (Aldrich), N-(3,4-methylenedioxyphenyl)alanine methyl ester was prepared. The methyl ester was then transesterified following General Procedure AQ above and using *iso-*butanol to provide the title compound as an oil. The reaction was monitored by silica gel tlc (Rf = 0.61 in 25% EtOAc/hexanes). Purification was by preparative plate chromatography with silica gel using 25% EtOAc/hexanes as the eluant.
NMR data was as follows:
¹H-nmr (CDCl₃): δ = 6.63 (d, 1H, 8.3 Hz), 6.25 (d, 1H, J = 2.3 Hz), 6.04 (dd, 1H, J = 8.3 Hz, J = 2.3 Hz), 5.83 (s, 2H), 3.96 (m, 4H), 1.92 (sept, 1H, J = 6.7 Hz), 1.44 (d,3H,J= 6.9 Hz), 0.90 (d, 6H, J = 6.6 Hz).
¹³C-nmr (CDCl₃): δ = 175.4, 148.9, 142.9, 140.8, 109.2, 105.8, 101.2, 97.4, 71.6, 53.6, 28.3, 19.6, 19.5.
C₁₄H₁₉NO₄ (MW = 265.31); mass spectroscopy (MH⁻) 265.

### Example A43

### Synthesis of N-(3,4-ethylenedioxyphenyl)alanine iso-butyl ester

Following reductive amination General Procedure AA above and using 1,4-benzodioxa-6-amine (Aldrich) and methyl pyruvate (Aldrich), N-(3,4-ethylenedioxyphenyl)alanine methyl ester was prepared. The methyl ester was then transesterifed following General Procedure AQ above using *iso*-butanol to provide the title compound. Purification was by preparative plate chromatography.
NMR data was as follows:
¹H-nmr (CDCl₃): δ = 0.91 (d, J = 7Hz, 6H), 1.42 (d, J = 7Hz, 3H), 1.8-2.0 (m, 1H), 3.8-3.95 (m, 3H), 4.0-4.1 (m, 1H), 4.15-4.25 (m, 4H), 6.12-6.2 (m, 2H), 6.65-6.75 (m, 1H).
¹³ C-nmr (CDCl₃): δ = 19.55, 19.56, 19.67, 28.3, 53.4, 64.7, 65.3, 71.7, 103.1, 108.0, 118.3, 142.1, 144.6, 175.4.
C₁₅H₂₁NO₄ (MW = 279.34); mass spectroscopy (MH⁻) 280.

### Example A44

### Synthesis of N-(2-naphthyl)alanine methyl ester

Following reductive amination General Procedure AA above and using 2-aminonaphthalene (Aldrich) and methyl pyruvate (Aldrich), the title compound was prepared. The reaction was monitored by silica gel tlc (Rf = 0.50 in 25% EtOAc/hexanes). Purification was by flash chromatography with silica gel using 25% EtOAc/hexanes as the eluant.
NMR data was as follows:
¹H-nmr (CDCl₃): δ = 7.65 (m, 3H), 7.48 (m, 1H), 7.25 (m, 1H), 6.91 (m, 1H), 6.79 (m, 1H), 4.31 (m, 2H), 3.76 (s, 3H), 1.55 (d, 3H).
¹³C-nmr (CDCl₃): δ = 175.66, 144.78, 135.55, 129.78, 128.47, 128.22, 126.96, 126.67, 123.01, 118.66, 105.88, 52.95, 52.51, 19.45.
C₁₄H₁₅NO₂ (MW = 229.28); mass spectroscopy (MH⁺) 229.

### Example A45

### Synthesis of N-(benzothiazol-6-yl)alanine ethyl ester

To a solution of 6-aminobenzothiazole (Lancaster) in dichloromethane was added 1.2 equivalents of pyridine, followed by 1.5 equivalents of trifluoroacetic anhydride. The reaction was stirred at room temperature for 3 hours and then washed with 5% citric acid, dried over MgSO₄, and stripped free of solvent on a rotary evaporator to yield 6-trifluoroacetamidothiazole. This material was dissolved in THF and then added to a suspension of KH in THF at 0°C. A catalytic amount of 18-crown-6 was added, followed by ethyl 2-bromopropionate (Aldrich). The reaction was held at room temperature for 1 hour, and then heated to reflux for 24 hours, and then cooled to room temperature. The reaction mixture was stripped free of solvent on a rotary evaporator and the resulting residue was dissolved in ether. This solution was washed with water, saturated aqueous NaCl, and dried over MgSO₄. The solution was stripped free of solvent on a rotary evaporator and the title compound was obtained by chromatography of the residue using 5% methanol/dichloromethane (Rf = 0.59) as the eluant.
NMR data was as follows:
¹H-nmr (CDCl₃): δ = 8.69 (s, 1H), 7.90 (d, 1H, J = 8.8 Hz), 7.04 (d, 1H, J = 2.3 Hz), 6.84 (dd, 1H, J = 8.8 Hz, J = 2.4 Hz), 4.41 (bd, 1H, J = 7.5 Hz), 4.20 (m, 3H), 1.53 (d, 3H, J = 6.9 Hz), 1.27 (t, 3H, J = 7.1 Hz).
¹³C-nmr (CDCl₃): δ = 174.9, 150.2, 147.1, 145.6, 136.3, 124.6, 115.7, 103.5, 61.9, 52.9, 19.4, 14.8.
C₁₂H₁₄N₂O₂S (MW = 250.32); mass spectroscopy (MH⁺) 251.

### Example A46

### Synthesis of N-(indol-5-yl)alanine-iso-butyl ester (S isomer)

Following General Procedure AM and using 5-aminoindole (Aldrich) and *iso*-butyl R-(+)-lactate (Aldrich), the title compound was prepared as an oil. The reaction was monitored by silica gel tlc (Rf = 0.46 in 33% EtOAc/hexanes). Purification was by preparative plate chromatography with silica gel using 33% EtOAc/hexanes as the eluant.
NMR data was as follows:
¹H-nmr (CDCl₃): δ = 8.11 (bs, 1H), 7.07 (d, J=8.8 Hz, 1H), 6.98 (d, J=2.8 Hz, 1H), 6.83 (d, J=2.2 Hz, 1H), 6.61 (m, 1H), 6.32 (m, 1H), 4.18 (q, J=6.9 Hz, 1H), 3.95 (bs, 1H), 3.87 (d, J=6.7 Hz, 2H), 1.89 (hept, J=6.7 Hz, 1H), 1.48 (d, J=6.96 Hz, 3H), 0.86 (dd, J=6.7 Hz, J=1.6 Hz, 6H).
¹³C-nmr (CDCl₃): δ = 176.15, 141.06, 131.28, 129.24, 125.34, 113.34, 112.53, 104.21, 102.17, 71.65, 54.28, 28.36, 19.87, 19.62.
C₁₅H₂₀N₂O₂ (MW = 260.34); mass spectroscopy (MH⁺) 261.

### Example A47

### Synthesis of N-(naphth-2-yl)alanine O-acylacetamidoxime ester

Following General Procedure AI above using N-(naphth-2-yl)alanine 2,4,6-trichlorophenyl ester (from Example AE above) and acetamide oxime (prepared according to the procedures described in J. Org. Chem., 46, 3953 (1981)), the title compound was prepared as a semisolid. The reaction was monitored by tlc on silica gel (Rf = 0.4 in ethyl acetate) and purification was by preparative plate chromatography (silica gel using ethyl acetate as the eluant).
NMR data was as follows:
¹H-nmr (d⁶-DMSO): δ = 7.64 (t, 2H), 7.54 (d, 1H), 7.32 (t, 1H), 7.13 (t, 1H), 7.04 (d, 1H), 6.78 (s, 1H) 6.42 (broad s, 2H), 6.32 (d, 1H), 4.33 (m, 1H), 1.72 (s, 3H), 1.46 (d, 3H).
C₁₅H₁₇N₃O₂ (MW = 271.32); mass spectroscopy: 271.

### Example A48

### Synthesis of N-(2-naphthyl)alanine ethyl ester

Following reductive amination General Procedure AA above and using 2-aminonaphthalene (Aldrich) and ethyl pyruvate (Aldrich), the title compound was prepared as a solid having a melting point of 52-56°C. The reaction was monitored by silica gel tlc (Rf = 0.50 in 25% EtOAc/hexanes). Purification was by flash chromatography with silica gel using 25% EtOAc/hexanes as the eluant.
NMR data was as follows:
¹H-nmr (CDCl₃): δ = 7.65 (m, 3H), 7.48 (m, 1H), 7.25 (m, 1H), 6.91 (m, 1H), 6.79 (m, 1H), 4.31 (m, 2H), 3.76 (s, 3H), 1.55 (d, 3H).
¹³C-nmr (CDCl₃): δ = 175.66, 144.78, 135.55, 129.78, 128.47, 128,22, 126.96, 126.67, 123.01, 118.66, 105.88, 52.95, 52.51, 19.45.
C₁₄H₁₅NO₂ (MW = 229.28); mass spectroscopy (MH⁺) 229.

### Example A49

### Synthesis of N-(3,4-dichlorophenyl)alanine O-acylpropionamidoxime ester

Following General Procedure AI above using N-(3,4-dichlorophenyl)alanine 2,4,6-trichlorophenyl ester (prepared from N-(3,4-dichlorophenyl)alanine methyl ester (from Example A9) using essentially the same procedure as described in Example AE above) and propionamide oxime (prepared according to the procedures described in J. Org. Chem., 46, 3953 (1981)), the title compound was prepared as a semisolid. The reaction was monitored by tlc on silica gel (Rf = 0.2 in 50% ethyl acetate/hexane) and purification was by preparative plate chromatography (silica gel using 50% ethyl acetate/hexane as the eluant).
NMR data was as follows:
¹H-nmr (d°-DMSO): δ = 7.27 (d, 1H), 6.83 (s, 1H), 6.64 (d, 1H), 6.47 (d, 1H), 6.38 (broad s, 2H), 4.24 (m, 1H), 2.07 (q, 2H), 1.41 (d, 3H).
C₁₂H₁₅Cl₂N₃O₂ (MW = 304.17); mass spectroscopy (MH⁺) 305.

### Example A50

### Synthesis of N-(4-ethoxycarbonylphenyl)alanine iso-butyl ester (S isomer)

Following General Procedure AM and using ethyl 4-aminobenzoate (Aldrich) and *iso*-butyl R-(+)-lactate (Aldrich), the title compound was prepared as an oil. The reaction was monitored by silica gel tlc (Rf = 0.21 in 10% EtOAc/hexanes). Purification was by preparative plate thin layer chromatography using 25% EtOAc/hexanes as the eluant.
NMR data was as follows:
¹H-nmr (CDCl₃): δ = 7.82 (d, J = 8.73 Hz, 2H), 6.51 (d, J = 8.79 Hz, 2H), 4.81 (d, J = 7.82 Hz, 1H), 4.25 (q, J = 7.14 Hz, 2H), 4.15 (quint, J = 7.40 Hz, 1H), 3.87 (m, 2H), 1.87 (sept, J = 6.70 Hz, 1H), 1.43 (d, J = 6.95 Hz, 3H), 1.30 (t, J = 7.14 Hz, 3H), 0.84 (d, J = 6.71 Hz, 6H).
¹³C-nmr (CDCl₃): δ = 174.5, 167.3, 151.0, 132.0, 119.9, 112.5, 71.9, 60.8, 51.9, 28.2, 19.5, 19.2, 15.0.
C₁₆H₂₃NO₄ (MW = 293.37); mass spectroscopy (MH⁺) 294.

### Example A51

### Synthesis of N-[3,5-di(trifluoromethyJ)phenyl]alanine iso-butyl ester (S isomer)

Following General Procedure AM and using 3,5-di(trifluoromethyl)aniline (Aldrich) and *iso*-butyl R-(+)-lactate (Aldrich), the title compound was prepared as an oil. The reaction was monitored by silica gel tlc (Rf = 0.38 in 10% EtOAc/hexanes). Purification was by preparative plate thin layer chromatography using 10% EtOAc/hexanes as the eluant.
NMR data was as follows:
¹H-nmr (CDCl₃): δ = 7.13 (s, 1H), 6.91 (s, 2H), 4.97 (d, J = 8.24 Hz, 1H), 4.18 (m, 1H), 3.93 (d, J = 6.59 Hz, 2H), 1.93 (sept, J = 6.71 Hz, 1H), 1.49 (d, J = 7.02 Hz, 3H), 0.89 (d, J = 6.59 Hz, 6H).
¹³C-nmr (CDCl₃): δ = 174.4, 147.9, 133.6, 133.2, 132.7, 132.3, 129.4, 125.8, 122.2, 118.6, 112.81, 112.76, 111.42, 111.37, 111.32, 111.27, 111.22, 72.2, 52.0, 32.1, 28.24, 28.17, 23.2, 19.5, 19.3, 19.2, 18.9, 14.6.
C₁₅H₁₇F₆NO₂ (MW = 357.30); mass spectroscopy (MH⁺) 358.

### Example A52

### Synthesis of N-(3,5-dimethoxyphenyl)alanine iso-butyl ester

N-(3,5-dimethoxyphenyl)alanine (crude, 454 mg) (prepared according to the procedure described in U.S. Patent No. 3,598,859 using 3,5-dimethoxyaniline (Aldrich) and 2-chloropropionic acid (Aldrich)) was treated in dry *iso*-butanol (10 mL) with 0.1 mL of chlorotrimethylsilane and the reaction mixture refluxed overnight. The excess alcohol was removed at reduced pressure and the residue dissolved in ethyl acetate. The ethyl acetate solution was washed with saturated aqueous NaHCO₃, dried with Na₂SO₄ and the solvent removed to provide the title compound. The reaction was monitored by silica gel tlc (Rf = 0.3 in 20% EtOAc/hexanes). Purification was by preparative thin layer chromatography using 20% EtOAc/hexanes as the eluant.
NMR data was as follows:
¹H-nmr (CDCl₃): δ = 0.9 (d, J = 7, 6H), 1.47 (d, J = 7, 3H), 1.9-2.0 (m, 1H), 3.7 (s, 6H), 3.85-4.0 (m, 2H), 4.1-4.2 (m, 1H), 4.3 (brs, 1H), 5.8 (s, 2H), 5.9 (s, 1H).
¹³C-nmr (CDCl₃): δ = 19.49, 19.52, 19.54, 28.3, 52.5, 55.6, 71.7, 91.1, 92.7, 149.2, 162.3, 175.2.
C₁₅H₂₃NO₄ (MW = 281.35).

### Example A53

### Synthesis of N-(2-napthyl)alanine O-acylpropionamidoxime ester

Following General Procedure AS and using N-(2-naphthyl)alanine 2,4,5-trichlorophenyl ester (from Example AE above) and propionamide oxime (prepared according to the procedures described in J. Org. Chem., 46, 3953 (1981)), the title compound was prepared. The reaction was monitored by silica gel tlc (Rf = 0.5 in EtOAc). Purification was by silica gel chromatography using 1:1 EtOAc/hexanes as the eluant.
NMR data was as follows:
¹H-nmr (DMSO-*d₆*): δ = 1.03 (t, 3H), 1.45 (d, 3H).
C₁₆H₁₉N₃O₂ (MW = 285.35); mass spectroscopy (M⁺) 285.

### Example A54

### Synthesis of N-(2-napthyl)alanine O-acylbutyramidoxime ester

Following General Procedure AS and using N-(2-naphthyl)alanine 2,4,5-trichlorophenyl ester (from Example AE above) and butyramide oxime (prepared according to the procedures described in J. Org. Chem., 46, 3953 (1981)), the title compound was prepared as an oil. The reaction was monitored by silica gel tic (Rf = 0.6 in EtOAc). Purification was by silica gel chromatography using 1:1 EtOAc/hexanes as the eluant.
NMR data was as follows:
¹H-nmr (DMSO-*d₆*): δ = 0.86 (t, 3H), 1.46 (d, 3H).
C₁₇H₂₁N₃O₂ (MW = 299.37); mass spectroscopy (MH⁺) 299.

### Example A55

### Synthesis of N-(2-napthyl)alanine O-acylisovaleramidoxime ester

Following General Procedure AS and using N-(2-naphthyl)alanine 2,4,5-trichlorophenyl ester (from Example AE above) and isovaleramide oxime (prepared according to the procedures described in J. Org. Chem., 46, 3953 (1981)), the title compound was prepared as an oil. The reaction was monitored by silica gel tlc (Rf = 0.3 in 1:1 EtOAc/hexanes). Purification was by silica gel chromatography using 1:1 EtOAc/hexanes as the eluant.
NMR data was as follows:
¹H-nmr (DMSO-*d₆*): δ = 0.86 (t, 3H), 1.45 (d, 3H).
C₁₈H₂₃N₃O₂ (MW = 313.40); mass spectroscopy (MH⁺) 313.

### Example A56

### Synthesis of N-(2-napthyl)alanine O-acylbenzamidoxime ester

Following General Procedure AS and using N-(2-naphthyl)alanine 2,4,5-trichlorophenyl ester (from Example AE above) and benzamide oxime (prepared according to the procedures described in J. Org. Chem., 46, 3953 (1981)), the title compound was prepared as an oil. The reaction was monitored by silica gel tlc (Rf = 0.3 in 1:1 EtOAc/hexanes). Purification was by silica gel chromatography using 1:1 EtOAc/hexanes as the eluant.
NMR data was as follows:
¹H-nmr (DMSO-*d₆*): δ = 4.42 (m, 1H), 1.53 (d, 3H).
C₂₀H₁₉N₃O₂ (MW = 333.39); mass spectroscopy (MH⁺) 333.

### Example A57

### Synthesis of N-(2-napthyl)alanine O-acylcyclopropanecarboxamidoxime ester

Following General Procedure AS and using N-(2-naphthyl)alanine 2,4,5-trichlorophenyl ester (from Example AE above) and cyclopropanecarboxamide oxime (prepared according to the procedures described in J. Org. Chem., 46, 3953 (1981)), the title compound was prepared as an oil. The reaction was monitored by silica gel tlc (Rf = 0.3 in 1:1 EtOAc/hexanes). Purification was by silica gel chromatography using 1:1 EtOAc/hexanes as the eluant.
NMR data was as follows:
¹H-nmr (DMSO-*d₆*): δ = 0.85 (m, 4H), 1.43 (d, 3H).
C₁₇H₁₉N₃O₂ (MW = 297.36); mass spectroscopy (MH⁺) 297.

### Example A58

### Synthesis of N-(2-napthyl)alanine O-acylcyclopropylacetamidoxime ester

Following General Procedure AS and using N-(2-naphthyl)alanine 2,4,5-trichlorophenyl ester (from Example AE above) and cyclopropylacetamide oxime (prepared according to the procedures described in J. Org. Chem., 46, 3953 (1981)), the title compound was prepared as an oil. The reaction was monitored by silica gel tlc (Rf = 0.3 in 1:1 EtOAc/hexanes). Purification was by silica gel chromatography using 1:1 EtOAc/hexanes as the eluant.
NMR data was as follows:
¹H-nmr (DMSO-*d₆*): δ = 1.43 (d, 3H), 1.91 (d, 2H).
C₁₈H₂₁N₃O₂ (MW = 311.39); mass spectroscopy (MH⁺) 311.

### Example A59

### Synthesis of N-(2-napthyl)alanine-O-acylcyclopentanecarboxamidoxime ester

Following General Procedure AS and using N-(2-naphthyl)alanine 2,4,5-trichlorophenyl ester (from Example AE above) and cyclopentanecarboxamide oxime (prepared according to the procedures described in J. Org. Chem., 46, 3953 (1981)), the title compound was prepared as an oil. The reaction was monitored by silica gel tlc (Rf = 0.3 in 1:1 EtOAc/hexanes). Purification was by silica gel chromatography using 1:1 EtOAc/hexanes as the eluant.
NMR data was as follows:
¹H-nmr (DMSO-*d₆*): δ = 1.43 (d, 3H), 2.43 (m, 1H).
C₁₇H₁₉N₃O₂ (MW = 297.36).

### GENERAL PROCEDURE BA

### Coupling of R¹C(X')(X")C(O)Cl with H₂NCHCR²)C(O)XR³

To a stirred solution of (D,L)-alanine *iso*-butyl ester hydrochloride (from Example BB below) (4.6 mmol) in 5 mL of pyridine was added 4.6 mmol of an acid chloride. Precipitation occurred immediately. The mixture was stirred for 3.5 h, diluted with 100 mL of diethyl ether, washed with 10% HCl three times, brine once, 20% potassium carbonate once and brine once. The solution was dried over magnesium sulfate, filtered, and evaporated at reduced pressure to yield the product. Other amino acid esters may also be employed in this procedure.

### GENERAL PROCEDURE BB

### Coupling of R¹C(X')(X")C(O)OH with H₂NCH(R²)C(O)XR³

A solution of the acid (3.3 mmol) and CDI in 20 mL THF was stirred for 2 h. L-alanine *iso*-butyl ester hydrochloride (from Example BB below) (3.6 mmol) was added, followed by 1.5 mL (10.8 mmol) of triethylamine. The reaction mixture was stirred overnight. The reaction mixture was diluted with 100 mL of diethyl ether, washed with 10% HCI three times, brine once, 20% potassium carbonate once and brine once. The solution was dried over magnesium sulfate, filtered, and evaporated at reduced pressure to yield the product. Other amino acid esters may also be employed in this procedure.

### GENERAL PROCEDURE BC

### Esterification of R¹C(X')(X")C(O)NHCH(R²)C(O)OH With HOR³ ,

To a stirred solution of phenylacetylvaline (1.6470 g, 7.0 mmol) in 20 mL THF was added CDI (1.05 g, 6.5 mmol) and the mixture was stirred for 1.5 h. 2-Methylbutanol (0.53 g, 6 mmol) was added the mixture, followed by addition of NaH (0.16 g, 6.5 mmol). Bubbling occurred immediately. The reaction mixture was stirred overnight. The reaction mixture was diluted with 100 mL of diethyl ether, washed with 10% HCI three times, brine once, 20% potassium carbonate once and brine once. The solution was dried over magnesium sulfate, filtered, and evaporated at reduced pressure to yield the product. Other N-acyl amino acids and alcohols may also be employed in this procedure.

### GENERAL PROCEDURE BD

### Ester Hydrolysis to the Free Acid

Ester hydrolysis to the free acid was conducted by conventional methods. Below are two examples of such conventional de-esterification methods.

To the ester in a 1:1 mixture of CH₃OH/H₂O was added 2-5 equivalents of K₂CO₃. The mixture was heated to about 50°C for about 0.5 to 1.5 hours until tlc showed complete reaction. The reaction was cooled to room temperature and the methanol was removed at reduced pressure. The pH of the remaining aqueous solution was adjusted to about 2, and ethyl acetate was added to extract the product. The organic phase was then washed with saturated aqueous NaCl and dried over MgSO₄. The solution was stripped free of solvent at reduced pressure to yield the product.

The amino acid ester was dissolved in dioxane/water (4:1) to which was added LiOH (∼2 eq.) that was dissolved in water such that the total solvent after addition was about 2:1 dioxane:water. The reaction mixture was stirred until reaction completion and the dioxane was removed under reduced pressure. The residue was diluted with EtOAc, the layers were separated and the aqueous layer acidified to pH 2. The aqueous layer was back extracted with EtOAc, the combined organics were dried over Na₂SO₄ and the solvent was removed under reduced pressure after filtration. The residue was purified by conventional methods (e.g., recrystallization).

The following exemplifies this later example. The methyl ester of 3-NO₂ phenylacetyl alanine 9.27 g (0.0348 mols) was dissolved in 60 mL dioxane and 15 mL of H₂O and adding LiOH (3.06 g, 0.0731 mol) that has been dissolved in 15 mL of H₂O. After stirring for 4 hours, the dioxane was removed under reduced pressure and the residue diluted with EtOAc, the layers were separated and the aqueous layer acidified to pH 2. The aqueous layer was back extracted with EtOAc (4 X 100 mL), the combined organics were dried over Na₂SO₄ and the solvent was removed under reduced pressure after filtration. The residue was recrystallized from EtOAc/isooctane giving 7.5 g (85%) of 3-nitrophenylacetyl alanine. C₁₁H₁₂N₂O₅ requires C = 52.38, H = 4.80, and N = 11.11. Analysis found C = 52.54, H = 4.85, and N = 11.08. [α]₂₃ = - 29.9 @ 589 nm.

### GENERAL PROCEDURE BE

### Low Temperature BOP Coupling of Acid and Alcohol

A solution of methylene chloride containing the carboxylic acid (100M%) and N-methyl morpholine (150 M%) was cooled to -20°C under nitrogen. BOP (105 M%) was added in one portion and the reaction mixture was maintained at -20°C for 15 minutes. The corresponding alcohol (120 M%) was added and the reaction mixture was allowed to warm to room temperature and stirred for 12 hours. The reaction mixture was then poured into water and extracted with ethyl acetate (3x). The combined ethyl acetate portions were backwashed with saturated aqueous citric acid (2x), saturated aqueous sodium bicarbonate (2x), brine (1x), dried over anhydrous magnesium sulfate or sodium sulfate and the solvent removed under reduced pressure to yield the crude product.

### GENERAL PROCEDURE BF

### EDC Coupling of Acid and Amine

The acid derivative was dissolved in methylene chloride. The amine (1 eq.), N-methylmorpholine (5 eq.), and hydroxybenzotriazole monohydrate (1.2 eq.) were added in sequence. The reaction was cooled to about 0°C and then 1.2 eq. of 1-(3-dimethylaminopropyl)-3-ethylcarbodiimide hydrochloride was added. The solution was allowed to stir overnight and come to room temperature under N₂ pressure. The reaction mix was worked up by washing the solution with saturated, aqueous Na₂CO_{3,} 0.1M citric acid, and brine before drying with Na₂SO₄ and removal of solvents to yield crude product. Pure products were obtained by flash chromatography in an appropriate solvent.

### GENERAL PROCEDURE BG

### EDC Coupling of Acid and Amine

A round bottom flask was charged with carboxylic acid (1.0 eq.), hydroxybenzotriazole hydrate (1.1 eq.) and amine (1.0 eq.) in THF under nitrogen atmosphere. An appropriate amount (1.1 eq. for free amines and 2.2 eq. for hydrochloride amine salts) of base, such as Hunig's base was added to the well stirred mixture followed by EDC (1,1 eq.). After stirring from 4 to 17 hours at room temperature the solvent was removed at reduced pressure, the residue taken up in EtOAc (or similar solvent)/water. The organic layer was washed with saturated aqueous sodium bicarbonate solution, IN HCl, brine and dried over anhydrous sodium sulfate. In some cases, the isolated product was analytically pure at this stage while, in other cases, purification via chromatography and/or recrystallization was required prior to biological evaluation.

### GENERAL PROCEDURE BH

### Coupling of R¹C(X')(X")C(O)Cl with H₂NCH(R²)C(O)XR³

An excess of oxalyl chloride in dichloromethane was added to the acid derivative together with one drop of DMF. The resulting mixture was stirred for about 2 hours or until bubbling ceases. The solvent was then removed under reduced pressure and rediluted with dry methylene chloride. To the resulting solution was added about 1.1 eq. of the appropriate amino acid ester and triethylamine (1.1 eq. in methylene chloride). The system was stirred at room temperature for 2 hours and then the solvent was removed under reduced pressure. The residue was dissolved in ethyl acetate, washed with 1N HCl followed by 1N NaOH. The organic layer was dried over anhydrous soldium sulfate, filtered and the solvent removed under reduced pressure to provide for the desired product.

### GENERAL PROCEDURE BI

### P-EPC coupling

P-EPC coupling employs an amino acid ester and a substituted acetic acid compound. The acetic acid derivative is well known in the art and is typically commercially available. The amino acid ester is prepared by conventional methods from the known and typically commercially available N-BOC amino acid as described in GENERAL PROCEDURE BJ below.

Specifically, the appropriate amino ester free base (0.0346 mmols) and substituted phenylacetic acid (0.069 mmols) were dissolved in 2.0 mL CHCl₃ (EtOH free), treated with 150 mg of P-EPC (0.87 meq./g) and the reaction was mixed for 4 days at 23°C. The reaction was filtered through a plug of cotton, rinsed with 2.0 mL of CHCl₃ and the filtrate evaporated under a stream of nitrogen. The purity of each sample was determined by ¹H NMR and ranged from 50% to > 95%. Between 8.0 and 15.0 mg of final product was obtained from each reaction and was tested without additional purification.

### GENERAL PROCEDURE BJ

### Synthesis of Amino Acid Esters From the Corresponding N-BOC Amino Acid

### A. Esterification of the Acid.

The N-BOC amino acid was dissolved in dioxane and treated with an excess of alcohol (∼1.5 eq.) and catalytic DMAP (100 mg) at 0°C. Stirring was continued until reaction completion whereupon the product was recovered by conventional methods.

### B. Removal of N-BOC Group.

The N-BOC protected amino acid was dissolved in methylene chloride (0.05M) and treated with 10 eq. of TFA at room temperature under a nitrogen atmosphere. The reaction was monitored by tlc until starting material was consumed usually within 1-5 hours. An additional 10 eq. of TFA was added to the reaction if the starting material was still present after 5 hours. The reaction was carefully neutralized with Na₂CO₃, separated, the organic layer washed with brine and dried over anhydrous Na₂SO₄. The crude amine was then used without purification.

Specific exemplification of these procedures are as follows:
1. Racemic (+/-)-N-BOC-α-amino butyric acid (Aldrich) (9.29 g, 0.0457 mol) was dissolved in 100 mL of dioxane and treated with *iso*-butyl alcohol (6.26 mL, 0.0686 mol), EDC (8.72 g, 0.0457) and catalytic DMAP (100 mg) at 0°C. After stirring for 17 hours, the organics were evaporated at reduced pressure, the residue diluted with EtOAc washed with NaHCO₃, brine and dried over Na₂SO₄. Evaporation yields 8.42 g (71 %) of an oil. C₁₃H₂₅NO₄ requires: C = 60.21, H = 9.72, and N = 5.40. Anal found: C = 59.91, H = 9.89, and N = 5.67.
   The above N-BOC amino acid ester (8.00 g, 0.032 mol) was deprotected as above giving 3.12 g (61%) of the free base as a colorless oil which solidifies upon standing.
2. L-N-BOC-alanine (Aldrich) (8.97 g, 0.047 mol) was dissolved in 100 mL of CH₂Cl₂, *iso*-butyl alcohol (21.9 mL, 0.238 mol) and treated with DMAP (100 mg) and EDC (10.0 g, 0.52 mol) at 0°C. The mixture was stirred for 17 hours, diluted with H₂O, washed with 1.0 N HCI, NaHCO₃, then brine and the organics were dried over Na₂SO₄. Filtration and evaporation yields 11.8 g (quantitative) of L-N-BOC alanine *iso*-butyl ester which is contaminated with a small amount of solvent. A sample was vacuum dried for analytical analysis. C₁₂H₂₃NO₄ requires: C = 58.79, H = 9.38, and N = 5.71. Anal found: C = 58.73, H = 9.55, and N = 5.96.
   The above N-BOC amino acid ester (11.8 g, 0.0481 mol) was deprotected as above. The free base was converted to the corresponding HCI salt using saturated HCI (g)/EtOAc to give L-N-alanine *iso*-butyl ester hydrochloride. Obtained 4.2 g (48%) of a colorless solid. C₇H₁₅NO₂. HCI requires: C = 46.28, H = 8.88, and N = 7.71. Anal found: C = 46.01, H = 8.85, and N = 7.68.

### GENERAL PROCEDURE BK

### Methyl ester formation from amino acids

The amino acid (amino acid or amino acid hydrochloride) is suspended in methanol and chilled to 0°C. HCI gas is bubbled through this solution for 5 minutes. The reaction is allowed to warm to room temperature then stirred for 4 hours. The solvents are then removed at reduced pressure to afford the desired amino acid methyl ester hydrochloride. This product is usually used without further purification.

### Example BA

### Synthesis of free and polymer bound PEPC

### N-ethyl-N'-3-(1-pyrrolidinyl)propylurea

To a solution of 27.7 g (0.39 mol) ethyl isocyanate in 250 mL chloroform was added 50 g (0.39 mol) 3-(1-pyrrolidinyl)propylamine dropwise with cooling. Once the addition was complete, the cooling bath was removed and the reaction mixture stirred at room temperature for 4 hours. The reaction mixture was then concentrated under reduced pressure to give 74.5 g (96.4%) of the desired urea as a clear oil.

### 1-(3-(1-pyrrolidinyl)propyl)-3-ethylcarbodiimide (P-EPC)

To a solution of 31.0 g (0.156 mol) N-ethyl-N'-3-(1-pyrrolidinyl)propyl-urea in 500 mL dichloromethane was added 62.6.g (0.62 mol) triethylamine and the solution was cooled to 0°C. To this solution were then added 59.17 g (0.31 mol) 4-toluenesulfonyl chloride in 400 mL dichloromethane dropwise at such a rate as to maintain the reaction at 0-5°C. After the addition was complete, the reaction mixture was warmed to room temperature and then heated to reflux for 4 hours. After cooling to room temperature, the reaction mixture was washed with saturated aqueous potassium carbonate (3 x 150 mL). The aqueous phases were combined and extracted with dichloromethane. All organic phases were combined and concentrated under reduced pressure. The resultant orange slurry was suspended in 250 mL diethyl ether and the solution decanted off from the solid. The slurry/decantation process was repeated 3 more times. The ether solutions were combined and concentrated under reduced pressure to give 18.9 g (67%) of the desired product as a crude orange oil. A portion of the oil was distilled under vacuum to give a colorless oil distilling at 78-82°C (0.4 mm Hg)..

### Preparation of a polymer supported form of 1-(3-(1-pyrrolidinyl)propyl)-3-ethylcarbodiimide (P-EPC)

A suspension of 8.75 g (48.3 mmol) 1-(3-(1-pyrrolidin-yl)propyl)-3-ethylcarbodiimide and 24.17 g (24.17 mmol) Merrifield's resin (2 % crosslinked, 200-400 mesh, chloromethylated styrene/divinylbenzene copolymer, 1 meq. Cl/g) in dimethylformamide was heated at 100°C for 2 days. The reaction was cooled and filtered and the resulting resin washed sequentially with 1L DMF, 1L THF and 1L diethyl ether. The remaining resin was then dried under vacuum for 18 hours.

### Example BB

### Preparation of alanine iso-butyl ester hydrochloride

A mixture of 35.64 g (0.4 mol) of (D,L)-alanine (Aldrich) (or L-alanine (Aldrich)); 44 mL (0.6 mol) of thionyl chloride (Aldrich) and 200 mL of isobutanol was refluxed for 1.5 hours and the volatiles were removed completely on a rotavapor of 90°C under reduced pressure to give (D,L)-alanine iso-butyl ester hydrochloride (or L-alanine *iso*-butyl ester hydrochloride), which was pure enough to be used for further transformations.

### Example BC

### Preparation of 3,5-dicblorophenylacetic acid

To a solution of 3.5 g of 3,5-dichlorobenzyl alcohol (Aldrich) in 75 mL of dichloromethane at 0°C was added 1.8 mL of methane sulfonylchloride followed by 3.5 mL of triethylamine added dropwise. After 2 hours the solution was diluted to 150 mL with dichloromethane, washed with 3N HCl, saturated aqueous NaHCO₃ dried with Na₂SO₄ and the solvents removed to yield the desired 3,5-dichlorobenzyl methanesulfonate as a yellow oil that was used without purification.

The crude sulfonate was dissolved in 50 mL of DMF at 0°C and then 3 g of KCN was added. After 2 hours an additional 50 mL of DMF was added and the solution was stirred for 16 hours. The red solution was diluted with 1 L of H₂O and acidified to pH 3 with 3N HCI. The aqueous solution was extracted with dichloromethane. The combined organics were washed with 3N HCl, dried with Na₂SO₄ and the solvents removed at reduced pressure to yield crude 3,5-dichlorophenylacetonitrile which was used without purification.

The nitrile was added to a mixture of 40 mL of concentrated sulfuric acid and 50 mL H₂O and heated to reflux for 48 hours; cooled to room temperature and stirred for 48 hours. The reaction was diluted into 1 L of crushed ice, warmed to toom temperature and extracted with 2 x 200 mL of dichloromethane and 2 x 200 mL of ethylacetate. Both sets of organics were combined and washed with saturated aqueous NaHCO₃. The NaHCO₃ fractions were combined and acidified to pH 1 with 3N HCl. The white solid was too fine to filter and was extracted out with 2 X 200 mL of dichloromethane. The combined organics were dried with Na₂SO₄ and the solvents removed at reduced presure to yield crude 3,5-dichlorophenylacetic acid as a white solid. The solid was slurried with hexane and filtered to get 1.75g of white solid.
NMR (CDCl₃): (in ppm) 3.61 (s, 2H), 7.19 (s,1H), 7.30 (s, 1H)

### Example BD

### Synthesis of N-(3-chlorophenylacetyl)alanine

The title compound was prepared using L-alanine (Nova Biochem) and 3-chlorophenyl acetic acid (Aldrich) by following General Procedures BF or BG, followed by hydrolysis using General Procedure BD.

### Example B1

### Synthesis of N-(phenylacetyl)-D,L-alanine iso-butyl ester

Following General Procedure BA above and using phenylacetyl chloride (Aldrich) and D,L-alanine iso-butyl ester hydrochloride (from Example BB above), the title compound was prepared. The reaction was monitored by tlc on silica gel and purification was by extraction with Et₂O followed by washes with aqueous K₂CO₃ and aqueous HCl.
NMR data was as follows:
¹H-nmr (CDCl₃): δ = 7.23-7.36 (m, 5H), 6.18 (d, 1H), 4.58 (t, *J* = 7.3 Hz, - 1H), 3.87 (m, 2H), 3.57 (s, 2H), 1.90-(m, 1H), 1.34. (d, *J* = 7.2 Hz, 3H), 0.89 (d, *J* = 6.8 Hz, 6H).
¹³C-nmr (CDCl₃):δ = 172.7, 170.3, 134.5, 129.2, 128.8, 127.2, 71.3, 48.1, 43.4, 27.5, 18.8, 18.3.
C₁₅H₂₁NO₃ (MW = 263.34; Mass Spectroscopy (MH⁺ = 264))

### Example B2

### Synthesis of N-(3-phenylpropionyl)-D,L-alanine iso-butyl ester

Following General Procedure BA above and using 3-phenylpropionyl chloride (Aldrich) and D,L-alanine iso-butyl ester hydrochloride (from Example BB above), the title compound was prepared as a solid having a melting point of from 51°-54°C; The reaction was monitored by tic on silica gel and purification was by extraction with Et₂O followed by washes with aqueous K₂CO₃ and aqueous HCl.
NMR data was as follows:
¹H-nmr (CDCl₃): δ = 7.25 (m, 2H), 7.19 (m, 3H), 6.28 (d, *J* = 7.2 Hz, 1H), 4.58 (quint., *J* = 7.2 Hz, 1H), 3.89 (m, 2H), 2.95 (t, *J* = 7.7 Hz, 2H), 2.50 (m, 2H), 1.92 (m. 1H), 1.33 (d, *J* = 7.1 Hz, 3H), 0.91 (d, *J* = 6.7 Hz, 6H).
¹³C-nmr (CDCl₃): δ = 173.0, 171.5, 140.6, 128.3, 128.1, 126.0, 71.2, 47.8, 37.9. 31.4, 27.5, 18.79, 18.77, 18.3.
C₁₆H₂₃NO₃ (MW 277.37, Mass Spectroscopy (MH⁺ 278))

### Example B3

### Synthesis of N-(3-methylpentanoyl)-L-alanine iso-butyl ester

Following General Procedure BB and using 3-methylpentanoic acid (Aldrich) and L-alanine iso-butyl ester hydrochloride (from Example BB above), the title compound was prepared as an oil. The reaction was monitored by tlc on silica gel and purification was by extraction with Et₂O followed by washes with aqueous K₂CO₃ and aqueous HCl. NMR data was as follows:
¹H-nmr (CDCl₃): δ = 6.08 (d, *J* = 5.9 Hz, 1H), 4.62 (quint., *J* = 7.3 Hz, 1H), 3.92 (m, 2H), 2.22 (m, 1H), 1.84-2.00 (m, 3H), 1.40 (d, *J* = 7.2 Hz, 3H), 1.35 (m, 1H), 1.20 (m, 1H), 0.85-0.96 (m, 12H)..
¹³C-nmr (CDCl₃): δ = 173.3, 172.1, 71.4, 47.9, 43.9, 32.3, 29.38, 29.35, 27.6, 19.10, 19.06, 18.93, 18.91, 18.72, 18.67, 11.3.
C₁₃H₂₅NO₃ (MW = 243.35, Mass Spectroscopy (MH⁺ 244))

### Example B4

### Synthesis of N-[(4-chlorophenyl)acetyl]-L-alanine iso-butyl ester

Following General Procedure BB and using 4-chlorophenylacetic acid (Aldrich) and L-alanine iso-butyl ester hydrochloride (from Example BB above), the title compound was prepared as a solid having a melting point of 111°-113°C. The reaction was monitored by tlc on silica gel and purification was by extraction with Et₂O followed by washes with aqueous K₂CO₃ and aqueous HCl.
NMR data was as follows:
¹H-nmr (CDCl₃): δ = 7.30 (d, *J* = 8.2 Hz, 2H), 7.21 (d, *J* = 8.3 Hz, 2H), 6.18 (d, *J* = 5.5 Hz, 1H), 4.57 (quint., *J* = 7.2 Hz, 1H), 3.88 (m, 2H), 3.53 (s, 2H), 1.91 (m, 1H), 1.36 (d, *J* = 7.1 Hz, 3H), 0.90 (d, *J* = 6.8 Hz, 6H).
¹³C-nmr (CDCl₃): δ = 172.8, 169.8, 133.1, 133.0, 130.6, 128.9, 71.4, 48.2, 42.6. 27.6, 18.85, 18.82, 18.4.
C₁₅H₁₀NO₃Cl (MW = 297.78, Mass Spectroscopy (MH⁺ 298))

### Example B5

### Synthesis of N-[(3,4-dichlorophenyl)acetyl]-L-alanine iso-butyl ester

Following General Procedure BB and using 3,4-dichlorophenylacetic acid (Aldrich) and L-alanine *iso*-butyl ester hydrochloride (from Example BB above), the title compound was prepared as a solid having a melting point of 81°-83°C. The reaction was monitored by tlc on silica gel and purification was by extraction with Et₂O followed by washes with aqueous K₂CO₃ and aqueous HCl.
NMR data was as follows:
¹H-nmr (CDCl₃): δ = 0.90 (d, *J* = 6.8 Hz, 6H), 1.38 (d, *J=* 7.1 Hz, 3H), 1.91 (m, 1H), 3.50 (s, 2H), 3.90 (m, 2H), 4.57 (quint., *J* = 7.1 Hz, 1H), 6.31 (d, *J* = 4.9 Hz, 1H), 7.12 (m, 1H), 7.38 (m, 2H).
¹³C-nmr (CDCl₃): δ = 18.4, 18.8, 18.9, 27.6, 42.2, 48.3, 71.5, 128.6, 130.6, 131.2, 131.3, 132.6, 134.7, 169.2, 172.8.
C₁₅H₁₉NO₃Cl₂ (MW = 332.23, Mass Spectroscopy (MH⁺ 332))

### Example B6

### Synthesis of N-[(4-methylpbenyl)acetyl]-D,L-alanine iso-butyl ester

Following General Procedure BB and using 4-methylphenylacetic acid (Aldrich) and D,L-alanine *iso*-butyl ester hydrochloride (from Example BB above), the title compound was prepared as a solid having a melting point of 102°-104°C. The reaction was monitored by tlc on silica gel (Rf =0.6 in 33% ethyl acetate/hexanes) and purification was by extraction with Et₂O followed by washes with aqueous K₂CO₃ and aqueous HCl.
NMR data was as follows:
¹H-nmr (CDCl₃): δ = 0.90 (d, *J* = 6.7 Hz. 6H), 1.35 (d, *J* = 7.2 Hz, 3H), 1.91 (m, 1H). 2.34 (s, 3H), 3.55 (s, 2H), 3.88 (m, 2H), 4.58 (m, 1H), 6.05 (bd, 1H), 7.16 (s, 4H).
¹³C-nmr (CDCl₃): δ = 18.5, 18.85, 18.87, 21.0, 27.6, 43.1, 48.1, 71.3, 129.2, 129.6, 131.3, 136.9, 170.6, 172.8..
C₁₆H₂₃NO₃ (MW = 277.37, Mass Spectroscopy (MH⁺ 278))

### Example B7

### Synthesis of N-[(3-pyridyl)acetyl]-D,L-alanine iso-butyl ester

Following General Procedure BF and using 3-pyridylacetic acid hydrochloride (Aldrich) and D,L-alanine *iso*-butyl ester hydrochloride (from Example BB above), the title compound was prepared as a solid having a melting point of-62°-64°C. The reaction was monitored by tic on silica gel (Rf = 0.48 10% methanol/dichloromethane) and Purification was by silica gel chromatography.
NMR data was as follows:
¹H-nmr (CDCl₃): δ = 8.40 (d, *J* = 2.8, 2H); 7.6 (m, 1H): 7.16 (m, 2H); 4.5 (quint., *J* = 7.2, 7.2, 1H); 3.8 (m, 2H); 3.48 (s, 2H); 1.8 (m, 1H); 1.30 (d, *J* = 7.2, 3H); 0.81 (d, *J* = 6.7, 6H).
¹³C-nmr (CDCl₃): δ = 173.4, 170.1, 150.6, 148.8, 137.4, 131.4, 124.1, 71.9, 48.9, 40.6, 28.1, 19.5, 19.4, 18.6..
C₁₄H₂₀N₂O₃ (MW = 264, Mass Spectroscopy (MH⁺ 265))

### Example B8

### Synthesis of N-[(1-naphthyl)acelyl]-L-alanine iso-butyl ester

Following General Procedure BB and using 1-naphthylacetic acid (Aldrich) and L-alanine *iso*-butyl ester hydrochloride (from Example BB above), the title compound was prepared as a solid having a melting point of 69°-73°C. The reaction was monitored by tic on silica gel and purification was by extraction with Et₂O followed by washes with aqueous K₂CO₃ and aqueous HCl.
NMR data was as follows:
¹H-nmr (CDCl₃): δ = 0.83 (m, 6H), 1.25 (d, *J* = 7.1 Hz, 3H), 1.81 (m, 1H), 3.79 (m, 2H), 4.04 (2s, 2H), 4.57 (quint., *J* = 7.3 Hz, 1H), 5.99 (d, *J* = 7.1 Hz, 1H), 7.44 (m. 2H), 7.53 (m, 2H), 7.85 (m, 2H), 7.98 (m, 1H).
¹³C-nmr (CDCl₃): δ = 18.2, 18.81, 18.83, 27.5, 41.5, 48.2, 71.3, 123.7, 125.6, 126.1, 126.6. 128.2, 128.5, 128.7, 130.7, 132.0, 133.9, 170.3, 172.5.
C₁₉H₂₃NO₃ (MW = 313.40, Mass Spectroscopy. (MH⁺ 314))

### Example B9

### Synthesis of N-[(2-naphthyl)acetyl]-L-alanine iso-butyl ester

Following General Procedure BB and using 2-naphthylacetic acid (Aldrich) and L-alanine *iso*-butyl ester hydrochloride (from Example BB above), the title compound was prepared as a solid having a melting point of 128°-129°C. The reaction was monitored by tlc on silica gel and purification was by extraction with Et₂O followed by washes with aqueous K₂CO₃ and aqueous HCl.
NMR data was as follows:
¹H-nmr (CDCl₃): δ = 0.86 (m, 6H), 1.35 (d, *J* = 7.1 Hz, 3H), 1.78 (m, 1H), 3.76 (s, 2H), 3.87 (m, 2H), 4.62 (quint., *J* = 7.2 Hz, 1H), 6.13 (d, *J* = 7.1 Hz, 1H), 7.41 (m, 1H), 7.48 (m, 2H), 7.74 (s, 1H), 7.83 (m, 3H).
¹³C-nmr (CDCl₃): δ = 18.4, 18.82, 18.85, 27.6, 43.7, 48.2, 71.4, 125.9, 126.3, 127.2, 127.6. 127.7, 128.2, 128.7, 132.0, 132.5, 133.5, 170.3, 172.8.
C₁₉H₂₃NO₃ (MW = 313.40, Mass Spectroscopy (MH⁺ 314)).

### Example B10

### Synthesis of N-(4-phenylbutanoyl)-L-alanine iso-butyl ester

Following General Procedure BB and using 4-phenylbutanoic acid (Aldrich) and L-alanine *iso*-butyl ester hydrochloride (from Example BB above), the title compound was prepared as an oil. The reaction was monitored by tlc on silica gel and purification was by extraction with Et₂O followed by washes with aqueous K₂CO₃ and aqueous HCl.
NMR data was as follows:
¹H-nmr (CDCl₃): δ = 0.92 (d, *J* = 6.7 Hz, 6H), 1.38 (d, *J* = 7.1 Hz, 3H), 1.96 (m, 3H), 2.21 (t, *J* = 7.1 Hz, 2H), 2.64 (t, *J* = 7.3 Hz, 2H), 3.90 (m, 2H), 4.59 (quint., *J* = 7.2 Hz, 1H), 6.31 (d, 1H), 7.16 (m, 3H), 7.24 (m, 2H).
¹³C-nmr (CDCl₃): δ = 18.3, 18.75, 18.78, 26.8, 27.5, 34.9, 35.3, 47.8, 71.2, 125.7, 128.2, 128.3, 141.3, 172.1, 173.0.
C₁₇H₂₅NO₃ (MW = 291.39, Mass Spectroscopy (MH⁺ 292)).

### Example B11

### Synthesis of N-(5-phenylpentanoyl)-L-alanine iso-butyl ester

Following General Procedure BB and using 5-phenylpentanoic acid (Aldrich) and L-alanine *iso*-butyl ester hydrochloride (from Example BB above), the title compound was prepared as an oil. The reaction was monitored by tlc on silica gel and purification was by extraction with Et₂O followed by washes with aqueous K₂CO₃ and aqueous HCl.
NMR data was as follows:
¹H-nmr (CDCl₃): δ =.7.23 (m, 2H), 7.17 (m, 3H), 6.30 (d, 1H), 4.59 (quint., *J* = 7.3 Hz, 1H), 3.91 (m, 2H), 2.61 (t, *J* = 7.2 Hz, 2H), 2.22 (t, *J* = 7.2 Hz, 2H), 1.93 (m, 1H), 1.66 (m, 4H), 1.38 (d, *J* = 7.2 Hz, 3H), 0.92 (d, *J* = 6.7 Hz, 6H).
¹³C-nmr (CDCl₃): δ= 173.1, 172.3, 142.0, 128.2, 128.1, 125.6, 71.2, 47.8, 36.1, 35.5, 30.8, 27.5, 25.0, 18.80, 18.77, 18.4.
C₁₈H₂₇NO₃ (MW = 305.39, Mass Spectroscopy (MH⁺ 306)).

### Example B12

### Synthesis of N-[(4-pyridyl)acetyl]-D,L-alanine iso-butyl ester

Following General Procedure BF and using 4-pyridylacetic acid hydrochloride (Aldrich) and (D,L)-alanine iso-butyl ester hydrochloride (from Example BB above), the title compound was prepared as a solid having a melting point of 64°-66°C. The reaction was monitored by tlc on silica gel (Rf = 0.43 10% methanol/dichloromethane) and purification was by silica gel chromatography.
NMR data was as follows:
¹H-nmr (CDCl₃): δ = 8.51 (dd, *J* = 1.6, 2.8, 1.6, 2H); 7.23 (dd, *J* = 4.3, 1.6, 4.4, 2H); 6.71 (d, *J* = 6.8, 1H); 4.56 (quint., *J* = 7.3, 7.2, 1H); 3.88 (m, 2H); 3.53 (s, 2H); 1.89 (m, 1H); 1.36 (d, *J* = 7.2, 3H); 0.88 (d, *J* = 6.7, 6H).
¹³C-nmr (CDCl₃): δ = 173.5, 169.3, 150.5, 144.4, 125.1, 72.1, 48.9, 43.0, 28.2, 19.5, 19.5, 18.9.
C₁₄H₂₀N₂O₃ (MW = 264, Mass Spectroscopy (MH⁺ 265))

### Example B13

### Synthesis of N-(phenylacetyl)-L-alanine iso-butyl ester

Following General Procedure BB and using phenylacetyl chloride (Aldrich) and L-alanine *iso*-butyl ester hydrochloride (from Example BB above), the title compound was prepared as a solid having a melting point of 45°-47°C. The reaction was monitored by tlc on silica gel and purification was by extraction with Et₂O followed -by-washes with aqueous K₂CO₃ and aqueous HCl.
NMR data was as follows:
¹H-nmr (CDCl₃): δ = 7.24-7.39 (m, 5H), 6.14 (d, 1H), 4.58 (t, *J* = 7.3 Hz, 1H), 3.88 (m, 2H), 3.58 (s, 2H), 1.90 (m, 1H), 1.35 (d, *J* = 7.2 Hz, 3H); 0.89 (d, *J* = 6.7 Hz, 6H).
¹³C-nmr (CDCl₃): δ = 172.8, 170.4, 134.5, 129.3, 128.9, 127.2, 71.3, 48.1, 43.5, 27.5, 18.9, 18.8, 18.4.
C₁₅H₂₁NO₃ (MW = 263.34, Mass Spectroscopy (MH⁻ 264)).

### Example B14

### Synthesis of 2-[(3,4-dichlorophenyl)acetamido]butyric acid iso-butyl ester

Following General Procedure BI above and using 3,4-dichlorophenylacetic acid (Aldrich) and *iso*-butyl 2-aminobutyrate (prepared following General Procedure BJ above) the title compound was prepared. The reaction was monitored by tlc on silica gel and purification was by filtration as described in the general procedure.
NMR data was as follows:
¹H-nmr (CDCl₃): δ = 7.36 (m, 3H), 6.03 (bd, 1H), 4.54 (m, 1H), 3.87 (m, 2H), 3.49 (s, 2H), 1.93 (m, 2H), 1.72 (m, 1H), 0.88 (d, 6H), 0.80 (t, 3H).

### Example B15

### Synthesis of 2-[(3-methoxyphenyl)acetamido]butyric acid iso-butyl ester

Following General Procedure BI above and using 3-methoxyphenylacetic acid (Aldrich) and *iso*-butyl 2-aminobutyrate (prepared frollowing General Procedure BJ above), the title compound was prepared. The reaction was monitored by tlc on silica gel and purification was by filtration as described in the general procedure.
NMR data was as follows:
¹H-nmr (CDCl₃): δ = 6.75 (m, 4H), 5.93 (bd, 1H), 4.51 (m, 1H), 3.83 (m, 2H), 3.75 (s, 2H), 3.52 (s, 2H), 1.82 (m, 2H), 1.60 (m, 1H), 0.84 (d, 6H), 0.74 (t, 3H).
C₁₇H₂₅NO₄ (MW = 307.39, Mass Spectroscopy (MH⁻ 309)).

### Example B16

### Synthesis of 2-[(4-nitrophenyl)acetamido]butyric acid iso-butyl ester

Following General Procedure BI above and using 4-nitrophenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 8.16 (d, 2H), 7.44 (d, 2H), 6.04 (bd, 1H), 4.55 (m, 1H), 3.86 (m, 2H), 3.66 (s, 2H), 1,86 (m, 2H), 1.67 (m, 1H), 0.85 (d, 6H), 0.81 (t, 3H).
C₁₆H₂₂N₂O₅ (MW = 322.36, Mass Spectroscopy (MH⁺ 323)).

### Example B17

### Synthesis of 2-[(3,4-methylenedioxyphenyl)acetamido]butyric acid iso-butyl ester

Following General Procedure BI above and using 3,4-(methylenedioxy)-phenyl acetic acid (Aldrich) and iso-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 6.72 (m, 3H), 5.92 (bd, 1H), 4.54 (m, 1H), 3.86 (m, 2H), 3:66 (s, 2H), 1.86 (m, 2H), 1.66 (m, 1H), 0.89 (d; 6H), 0.79 (t, 3H).

### Example B18

### Synthesis of 2-[(thien-3-yl)acetamido]butyric acid iso-butyl ester

Following General Procedure BI above and using 3-thiopheneacetic acid (Aldrich) and *iso*-butyl 2-aminobutyrate (prepared following General Procedure BJ- above), the title compound was prepared. The reaction was monitored by tic on silica gel and purification was by filtration as described in the general procedure.
NMR data was as follows:
¹H-nmr (CDCl₃): δ = 7.37 (m, 1H), 7.16 (m, 1H), 7.04 (m, 1H), 6.05 (bd, 1H), 4.57 (m, 1H), 3.66 (s, 2H), 1.93 (m, 2H), 1.67 (m, 1H), 0.91 (d, 6H), 0.86 (t, 3H).

### Example B19

### Synthesis of 2-[(4-chlorophenyl)acetamido]butyric acid iso-butyl ester

Following General Procedure BI above and using 4-chlorophenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.22 (m, 2H), 7.11 (m, 2H), 5.80 (m. 1H), 4.44 (m, 1H), 3.78 (m, 2H), 3.43 (s, 2H), 1.77 (m, 2H), 1.56 (m, 1H), 0.83 (d, 6H) 0.71 (t, 3H).

### Example B20

### Synthesis of 2-[(3-nitrophenyl)acetamido]butyric acid iso-butyl ester

Following General Procedure BI above and using 3-nitrophenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 8.15 (m, 2H), 7.65 (m, 1H), 6.08 (m, 1H), 4.46 (m, 1H), 3.92 (m, 2H), 3.68 (s, 2H), 1.91 (m, 2H), 1.75 (m, 1H), 0.98 (d, 6H) 0.71 (t. 3H).

### Example B21

### Synthesis of 2-[(2-bydroxyphenyl)acetamido]butyric acid iso-butyl ester

Following General Procedure BI above and using 2-hydroxyphenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.14 (m, 1H), 7.01 (m, 1H), 6.93 (m, 1H), 6.79 (m, 1H), 6.46 (m, 1H), 4.5 (m, 1H), 3.87 (m, 2H), 3.57 (s, 2H), 2.01 (m, 2H), 1.75 (m, 1H), 0.89 (d, 6H), 0.85 (t, 3H).

### Example B22

### Synthesis of 2-[(2-napbthyl)acetamido]butyric acid iso-butyl ester

Following General Procedure BI above and using 2-naphthylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.83 (m, 7H), 5.95 (m, 1H), 4.58 (m, 1H), 3.84 (m, 2H), 3:75 (s, 2H), 1.89 (m, 2H), 1.63 (m, 1H), 0.91 (d, 6H), 0.81 (t, 3H). C₂₀H₂₅NO₃ (MW = 327.42, Mass Spectroscopy (MH⁻ 328)).

### Example B23

### Synthesis of 2-[(2,4-dichlorophenyl)acetamido]butyric acid iso-butyl ester

Following General Procedure BI above and using 2,4-dichlorophenylacetic acid-(Aldrich) and-*iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.49 (m, 1H), 7.22 (m, 2H) 5.98 (m, 1H), 4.52 (m, 1H), 3.86 (m, 2H), 3.61 (s, 2H), 1.84 (m, 2H), 1.62 (m, 1H) 0.87 (d, 6H), 0.80 (t. 3H).

### Example B24

### Synthesis of 2-[(4-bromophenyl)acetamido]butyric acid iso-butyl ester

Following General Procedure BI above and using 4-bromophenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.43 (d, 2H), 7.19 (d, 2H) 5.85 (m, 1H), 4.51 (m, 1H), 3.81 (m, 2H), 3.47 (s, 2H), 1.84 (m, 2H), 1.61 (m, 1H) 0.84 (d, 6H), 0.76 (t, 3H).
C₁₆H₂₂NO₃Br (MW = 356.26, Mass Spectroscopy (MH⁺ 358)).

### Example B25

### Synthesis of 2-[(3-chlorophenyl)acetamido])butyric acid iso-butyl ester

Following General Procedure BI above and using 3-chlorophenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.25 (m, 3H), 7.12 (m, 1H) 5.80-(m, 1H), 4.52 (m, 1H), 3.86 (m, 2H), 3.50 (s, 2H), 1.87 (m, 2H), 1.67 (m, 1H) 0.88 (d, 6H), 0.77 (t, 3H).
C₁₆H₂₂NO₃Cl (MW = 311.81 Mass Spectroscopy (MH⁺ 313)).

### Example B26

### Synthesis of 2-[(3-fluorophenyl)acetamido]butyric acid iso-butyl ester

Following General Procedure BI above and using 3-fluorophenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.31 (m, 1H), 7.01 (m, 3H) 5.95 (m, 1H), 4.54 (m, 1H), 3.84 (m, 2H), 3.54 (s, 2H), 1.88 (m, 2H), 1.65 (m, 1H) 0.87 (d, 6H), 0.81 . (t. 3H).
C₁₆H₂₂NO₃F (MW = 295.35 Mass Spectroscopy (MH⁺ 296)).

### Example B27

### Synthesis of 2-[(benzothiazol-4-yl)acetamido]butyric acid iso-butyl ester.

Following General Procedure BI above and using 4-benzothiazol-4-yl acetic acid (Chemservice) and iso-butyl 2-aminobutyrate (prepared following General Procedure BJ above), the title compound was prepared. The reaction was monitored by tic on silica gel and purification was by filtration as described in the general procedure.
NMR data was as follows:
¹H-nmr (CDCl₃): δ = 7.82 (m, 1H), 7.51-7.21 (m, 4H) 5.84 (m, 1H), 4.51 (m, 1H), 3.90 (s, 2H), 3.79 (m, 2H), 1.78 (m, 2H), 1.58 (m, 1H) 0.80 (d, 6H), 0.66 (t, 3H).

### Example B28

### Synthesis of 2-[(2-methylphenyl)acetamido]butyric acid iso-butyl ester

Following General Procedure BI above and using 2-methylphenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.18 (m, 4H), 5.79 (m, 1H), 4.54 (m, 1H), 3.85 (m, 2H), 3.59 (s, 2H), 3.29 (s, 3H), 1.81 (m, 2H), 1.59 (m, 1H) 0.87 (d, 6H), 0.77 (t, 3H).
C₁₇H₂₅NO₃. (MW = 291.39 Mass Spectroscopy (M⁺ 291)).

### Example B29

### Synthesis of 2-[(2-fluorophenyl)acetamido]butyric acid iso-butyl ester

Following General Procedure BI above and using 2-fluorophenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.28 (m, 1H), 7.09 (m, 3H) 6.03 (m, 1H), 4.54 (m, 1H), 3.87 (m, 2H), 3.57 (s, 2H), 1.89 (m, 2H), 1.64 (m., 1H) 0.88 (d, 6H), 0.80 (t, 3H).

### Example B30

### Synthesis of 2-[(4-fluorophenyl)acetamido]butyric acid iso-butyl ester

Following General Procedure BI above and using 4-fluorophenylacetic' acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.20 (m, 2H), 6.97 (m, 2H) 5.87 (m, 1H), 4.492 (m, 1H), 3.83 (m, 2H), 3.48 (s, 2H), 1.86 (m, 2H), 1.60 (m, 1H) 0.87 (d, 6H), 0.78 (t, 3H).
C₁₆H₂₂NO₃F (MW = 295.35 Mass Spectroscopy (MH⁺ 296)).

### Example B31

### Synthesis of 2-[(3-bromophenyl)acetamido]butyric acid iso-butyl ester

Following General Procedure BI above and using 3-bromophenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.45 (m, 2H), 7.23 (m, 2H) 5.95 (m, 1 H), 4.55 (m, 1H) 3.84 (m, 2H) 3.55 (s, 2H), 1:89 (m, 2H), 1.68 (m, 1H) 0.91 (d. 6H), 0.81 (t, 3H).
C₁₆H₂₂NO₃Br (MW = 356.26 Mass Spectroscopy (M⁺ 357)).

### Example B32

### Synthesis of 2-[(3-trifluoromethylphenyl)acetamido]butyric acid iso-butyl ester

Following General Procedure BI above and using 3-trifluoromethyl-phenylacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.52 (m, 1H), 7.47 (m, 2H) 6.01 (m, 1H), 4.56 (m, 1H), 3.86 (m, 2H), 3.61 (s, 2H), 1.84 (m, 2H), 1.62 (m, 1H) 0.87 (d, 6H), 0.80 (t, 3H).
C₁₇H₂₂NO₃F₃ (MW = 345.36 Mass Spectroscopy (MH⁻ 345)).

### Example B33

### Synthesis of 2-[(2-thienyl)acetamido]butyric acid iso-butyl ester

Following General Procedure BI above and using 2-thiopheneacetic acid (Aldrich) and *iso*-butyl 2-aminobutyrate (prepared following General Procedure BJ above), the title compound was prepared. The reaction was monitored by tlc on silica gel and purification was by filtration as described in the general procedure.
NMR data was as follows:
¹H-nmr (CDCl₃): δ = 6.89 (m, 3H), 6.07 (bd, 1H), 4.50 (m, 1H), 3.82 (m, 2H), 3.71 (s, 2H), 1.85 (m, 2H), 1.62 (m, 1H), 0.81 (d, 6H), 0.75 (t, 3H).
C₁₄H₂₁NO₃S (MW = 283.39, Mass Spectroscopy (MH⁺ 284)).

### Example B34

### Synthesis of 2-(phenylacetamido)butyric acid iso-butyl ester

Following General Procedure BH above and using phenylacetic acid (Aldrich) and *iso*-butyl 2-aminobutyrate (prepared following General Procedure BJ above), the title compound was prepared. The reaction was monitored by tlc on silica gel and purification was by chromatography on silica gel using 9:1 toluene:EtOAc as the eluant.
NMR data was as follows:
¹H-nmr (CDCl₃): δ = 7.17-7.28 (m, 5H), 6.23 (bd, 1H), 4.51 (m, 1H), 3.86 (m, 2H), 3.54 (s, 2H), 1.87 (m, 2H), 1.62 (m, 1H), 0.87 (d, 6H), 0.78 (t, 3H).
C₁₆H₂₃NO₃ (MW = 277.36, Mass Spectroscopy (MH⁺ 277)).

### Example B35

### Synthesis of N-(phenylacetyl)valine 2-methylbutyl ester

### Step A. Preparation of N-(phenylacetyl) valine

To a stirred solution of 5.15 g (44 mmol) of valine (Bachem) in 50 mL (100 mmol) of 2N NaOH cooled to 0°C was added dropwise 5.3 mL (40 mmol) of phenylacetyl chloride (Aldrich). A colorless oil precipitated. The reaction mixture was allowed to warm to room temperature and stirred for 18 hours, washed with 50 mL diethyl ether, acidified to pH 2-3 with aqueous HCl. The white precipitate formed was filtered off, washed thoroughly with water, followed by diethyl ether to give 7.1 g (30 mmol, 69% yield) of the title compound.
NMR data was as follows
¹H-nmr (DMSO-*d*₆): δ = 12.63 (s, 1H), 8.25 (d, *J* = 8.6 Hz, 1H), 7.27 (m, 5H), 4.15 (m, 1H), 3.56 (d, *J* = 13.8 Hz, 1H), 3.47 (d, *J* = 13.8 Hz, 1H), 2.05 (m, 1H), 0.87 (d, *J* = 6.8, Hz, 3H), 0.84 (d, *J* = 6.8 Hz, 3)
¹³C-nmr (DMSO-*d*₆): δ = 173.2, 170.4, 136.6, 129.0, 128.2, 126.3, 57.1, 41.9, 30.0, 19.2, 18.0
C₁₃H₁₇NO₃ (MW=235.29; Mass Spectroscopy (MH⁺ = 236))

### Step B. Synthesis of N-(phenylacetyl)valine 2-methylbutyl ester

Following General Procedure BC and using the N-(phenylacetyl) valine prepared in Step A above and 2-methylbutan-1-ol (Aldrich), the title compound was prepared as a diastereomeric mixture. The reaction was monitored by tlc on silica gel and purification was by filtration as described in the general procedure.
NMR data was as follows:
¹H-nmr (CDCl₃): δ = 7.25-7.40 (m, 5H), 5.95 (d, 1H), 4.56 (m, 1H), 3.84-4.00 (m, 2H), 3.61 (s, 2H), 2.10 (m, 1H), 1.68 (m, 1H), 1.38 (m, 1H), 1.15 (m 1H), 0.82-0.94 (m, 9H), 0.76 (d, 3H).
¹³C-nmr (CDCl₃): δ = 171.84, 171.81, 170.7, 134.6, 129.31, 129.27, 128.9, 127.3, 69.8, 57.0, 43.7, 33.9, 31.3, 25.9, 25.8,, 18.9, 17.4, 16.34, 16.27, 11.12, 11.07.
C₁₈H₂₇NO₃ (MW = 305.42, Mass Spectroscopy (MH 306)).

### Example B36

### Synthesis of N-(phenylacetyl)-L-methionine iso-butyl ester

L-Methionine (0.129g, 0.869 mmols) (Aldrich) was taken-up in dioxane (5.0 mL) and treated with a saturated solution of sodium bicarbonate (5.0 mL) followed by phenylacetyl chloride (Aldrich) (0.114 mL, 0.822 mmols). After stirring for 17 hours at room temperature the mixture was diluted with ethyl acetate, the layers separated and the aqueous layer acidified to pH 2 with 5N HCl. The crude product was extracted into ethyl acetate, dried over sodium sulfate, vacuum dried and used without further purification.

N-phenylacetyl-L-methionine (0.1285 g, 0.447 mmol) was dissolved in 3.0 mL dioxane and *iso*-butyl alcohol (0.2 mL) and treated with EDC (0.094 g, 0.492 mmol), and catalytic DMAP (0.015g). After stirring for 17 hours at 23°C, the mixture was evaporated at reduced pressure to an oil, the residue was diluted in EtOAc and washed with 0.1 N HCl and saturated sodium bicarbonate. Chromatography on silica gel using 98:2 CHCl₃/MeOH as eluant provided the pure product.
NMR data was as follows:
¹H-nmr (CDCl₃): δ = 7.4-7.23 (m, 5H), 6.14 (bd, 1H), 4.70 (m. 1H), 3.89 (d, 2H), 3.62 (s, 2H), 2.43 (m, 2H), 2.12 (m, 1H), 1.93 (m, 2H), 0.94 (d, 6H).
C₁₇H₂₅NO₃S (MW = 323.17, Mass Spectroscopy (M⁻ 323)

### Example B37

### Synthesis of N-(phenylacetyl)-L-leucine iso-butyl ester

L-Leucine (Aldrich) (0.114g, 0.869 mmols) was taken-up in dioxane (5.0 mL) and treated with a saturated solution of sodium bicarbonate (5.0 mL) followed by phenylacetyl chloride (Aldrich) (0.114 mL, 0.822 mmols). After stirring for 17 hours at room temperature the mixture was diluted with ethyl acetate, the layers separated and the aqueous layer acidified to pH 2 with 5N HCl. The crude product was extracted into ethyl acetate, dried over sodium sulfate, vacuum dried and used without further purification.
N-Phenylacetyl-L-leucine (0.0081 g, 0.038 mmol) was dissolved in 2.0 mL CHCl₃ (EtOH free) and *iso*-butyl alcohol (0.055 mL) and treated with P-EPC (100 mg, 0.87 milliequivalents). The mixture was rotated for 4 days, filtered through a plug of cotton and the filtrate evaporated at reduced pressure to an oil which was sufficiently pure for testing.
NMR data was as follows:
¹H-nmr (CDCl₃): δ = 7.22 (m, 5H), 5.57 (d, 1H), 4.35 (m, 1H), 3.35 (m, 3H), 1.35 (m, 4H), 0.68 (m, 9H).
C₁₈H₂₇NO₃ (MW = 305.40, Mass Spectroscopy (M⁺ 305)).

### Example B38

### Synthesis of N-[(3-chlorophenyl)acetyl]alanine 3-methylbut-2-enyl ester

Following General Procedure BC above and using N-(3-chlorophenylacetyl alanine (from Example BD above) and 3-methylbut-2-en-1-ol (Aldrich), the title compound can be prepared. The reaction was monitored by tlc on silica gel and purification was by liquid chromatography using 30% EtOAc/hexane as the eluant.
NMR data was as follows:
¹H-nmr (CDCl₃): δ = 7.39-7.16 (m, 4H), 6.06 (bd, 1H), 5.38-5.29 (m, 1H), 4.63 (d, *J* = 9Hz, 2H), 3.56 (s, 2H), 1.79 (s, 3H), 1.7 (s, 3H), 1.39 (d, *J* = 9Hz, 3H).

### Example B39

### Synthesis of N-[(3-chlorophenyl)acetyl]alanine cyclopropylmethyl ester

Following General Procedure. BC above, and using N-(3-chlorophenylacetyl alanine (from Example BD above) and cyclopropylmethanol (Aldrich), the title compound can be prepared, The reaction was monitored by tlc on silica gel and purification was by liquid chromatography using 3:7 EtOAc:hexane as the eluant.
NMR data was as follows:
¹H-nmr (CDCl₃): δ = 7.2-7.1 (m, 4H), 6.09 (bs, 1H), 4.6 (dq, *J*= 9 Hz, 1H), 3.96 (dd, *J* = 9Hz, 2H), 3.59 (s, 2H), 1.2 (d, *J* = 9Hz, 3H), 1.2-1.0 (m, 1H), 0.603-0.503 (m, 2H), 0.300-0.203 (m, 2H).

### Example B40

### Synthesis of N-[(3-chlorophenyl)acetyl]alanine 2-thienylmethyl ester

Following General Procedure BC above, and using N-(3-chlorophenylacetyl alanine (from Example BD above) and 2-thiophenemethanol (Aldrich) the title compound can be prepared. The reaction was monitored by tlc on silica gel and purification was by liquid chromatography using 3:7 EtOAc:hexane as the eluant.
NMR data was as follows:
¹H-nmr (CDCl₃): δ = 7.37-6.97 (m, 7H), 5.97 (q, *J* = 14 Hz, 2H), 4.6 (dq, *J* = 9 Hz, 1H), 3.76 (s, 2H), 1.38 (d, *J* = 9Hz, 3H).

### Example B41

### Synthesis of N-[(3-chlorophenyl)acetyl]alanine (1-methylcyclopropyl)methyl ester

Following General Procedure BC above, and using N-(3-chlorophenylacetyl alanine (from Example BD above) and (1-methylcyclopropyl)methanol (Aldrich) the title compound can be prepared. The reaction was monitored by tlc on silica gel and purification was by liquid chromatography using 3:7 EtOAc:hexane as the eluant.
NMR data was as follows:
¹H-nmr (CDCl₃): δ = 8.6 (bd, *J* = 9 Hz, 1H), 3.86 (q, *J* = 14 Hz, 2H), 3.4 (s, 2H), 2.29 (q, *J* = 9 Hz, 1H), 1.3 (d, *J* = 9Hz, 3H), 1.03 (s, 3H), 0.5-0.4 (m, 2H), 0.4-0.28 (m, 2H).

### Example B42

### Synthesis of N-[(3-chlorophenyl)acetyl]alanine 3-thienylmethyl ester

Following General Procedure BC above, and using N-(3-chlorophenylacetyl alanine (from Example BD above) and 3-thiophenemethanol (Aldrich) the title compound can be prepared. The reaction was monitored by tlc on silica gel and purification was by liquid chromatography using 3:7 EtOAc:hexane as the eluant.
NMR data was as follows:
¹H-nmr (CDCl₃): δ = 8.03 (bd, *J* = 9 Hz, 1H), 7.56-7.5 (m, 1H), 7.47 (bs, 1H),7.4-7.17 (m, 4H), 7.06 (d, *J* = 9 Hz, 1H), 5.1 (s, 2H), 4.3 (dq, 1H), 1.3 (d, *J* = 9 Hz, 3H).

### Example B43

### Synthesis of N-[(3-chlorophenyl)acetyl]alanine 2-methylcyclopentyl ester

Following General Procedure BC above, and using N-(3-chlorophenylacetyl alanine (from Example BD above) and 2-methylcyclopentanol (Aldrich) the title compound can be prepared. The reaction was monitored by tlc on silica gel and purification was by liquid chromatography using 3:7 EtOAc:hexane as the eluant.
NMR data was as follows:
¹H-nmr (CDCl₃): δ = 7.39-7.16 (m, 4H), 6.3 (bd, 1H), 4.79-4.7 (m, 1H), 4.6-4.25 (m, *J* = 9 Hz, 1H), 3.577 (s, 2H), 2.09-1.8 (m, 2H), 1.74-1.6 (m, 2H), 1.39 (dd, *J* = 9 Hz, 3H), 1.2 (dt, *J* = 9 Hz, 1H), 0.979 (dd, *J* = 9 Hz, 2H)
C₁₇H₂₂NO₃Cl (MW = 323.82, Mass Spectroscopy (MH⁻ 323).

### Example B44

### Synthesis of N-[(3-chlorophenyl)acetyl]alanine 2-methylprop-2-enyl ester

Following General Procedure BC above, and using N-(3-chlorophenylacetyl alanine (from Example BD above) and 2-methylprop-2-en-1-ol (Aldrich) the title compound can be prepared. The reaction was monitored by tlc on silica gel and purification was by liquid chromatography using 3:7 EtOAc:hexane as the eluant.
NMR data was as follows:
¹H-nmr (CDCl₃): δ = 7.39-7.16 (m, 4H), 6.03 (bs, 1H), 4.77 (s, 2H), 4.7-4.29 (m, 3H), 2.59 (s, 2H), 1.73 (s, 3H), 1.43 (d, *J* = 9 Hz, 3H)
C₁₅H₁₈NO₃Cl (MW = 295.76, Mass Spectroscopy (MH⁻ 295)).

### Example B45

### Synthesis of N-[(3-chlorophenyl)acetyl]alanine cyclohex-2-enyl ester

Following General Procedure BC above, and using N-(3-chlorophenylacetyl alanine-(from Example BD above) and cyclohex-2-en-1-ol (Aldrich) the title compound can be prepared. The reaction was monitored by tlc on silica gel and purification was by liquid chromatography using 3:7 EtOAc:hexane as the eluant.
NMR data was as follows:
¹H-nmr (CDCl₃): δ = 8.6 (bd, *J* = 9 Hz, 1H), 7.4-7.2 (m, 4H), 6.0-5.8 (m, 1H), 5.7-5.5 (m, 1H), 5.1 (bs, 1H), 4.13-4.29 (m, 1H), 3.5 (s, 2H), 2.1-1.9 (m, 2H), 1.8-1.69 (m, 1H), 1.69-1.49 (m, 4H), 1.3 (dd, *J* = 9 Hz, 3H)
C₁₇H₂₀NO₃Cl (MW = 321.8, Mass Spectroscopy (MH⁺ 32.1.2)).

### Example B46

### Synthesis of N-[(2-phenylbenzoxazol-5-yl)acetyl]alanine iso-butyl ester

Following General Procedure BI above, and using 5-(2-phenylbenzoxazol)-yl-acetic acid (CAS# 62143-69-5) and alanine *iso*-butyl ester (prepared following General Procedure BJ above), the title compound was prepared.
NMR data was as follows:
¹H-nmr (CDCl₃): δ = 8:24 (m, 3H), 7.68 (m, 1H), 7.51 (m, 5H), 6.04 (m, 1H), 4.58 (m, 1H), 3.85 (m, 2H). 3.68 (s, 2H), 1.9 (m, 1H), 1.35 (d, 3H), 0.87 (d, 6H).
C₂₂H₂₄N₂O₄ (MW = 380, Mass Spectroscopy (MH⁺ 381)).

### Example B47

### Synthesis of N-[(3-methylthiophenyl)acetyl]alanine iso-butyl ester

Following General Procedure BI above, and using 3-methylthiophenylacetic acid (CAS# 18698-73-2) and alanine *iso*-butyl ester (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.14 (m, 2H), 7.01 (m, 1H), 4.56 (m, 1H), 3.88 (m, 2H), 3.54 (s, 2H), 2.46 (s, 3H), 1.89 (m, 1H), 1.35 (d, 3H) 0.85 (d, 6H).
C₁₆H₂₃NO₃S (MW = 309, Mass Spectroscopy (MH⁺ 310)).

### Example B48

### Synthesis of N-4-[(2-furyl)acetyl]alanine iso-butyl ester

Following General Procedure BI above, and using 2-furylacetic acid (CAS# 2745-26-8) and alanine *iso*-butyl ester (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.36 (m, 1H), 6.34 (m, 1H), 6.21 (m, 1H), 4.56 (m, 1H), 3.91 (m, 2H), 3.61 (s, 2H), 1.92 (m, 1H), 1.38 (d, 3H) 0.89 (d. 6H).
C₁₃H₁₉O₄ (MW = 253, Mass Spectroscopy (MH⁺ 254)).

### Example B49

### Synthesis of N-[(benzofuran-2-yl)acetyl]alanine iso-butyl ester

Following General Procedure BI above, and using benzofuran-2-ylacetic acid (Maybridge) and alanine *iso*-butyl ester (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.51 (m, 1H), 7.44 (m, 1H),7.25 (m, 2H), 6.67 (s, 1H), 4:60 (m, 1H), 3.87 (m, 2H), 3.77 (s, 2H), 1.88 (m, 1H), 1.38 (d, 3H), 0.87 (d, 6H).
C₁₇H₂₁NO₄ (MW = 303, Mass Spectroscopy (MH⁺ 304)).

### Example B50

### Synthesis of N-[(benzothiophen-3-yl)acetyl]alanine iso-butyl ester

Following General Procedure BI above, and using thianaphthen-3-ylacetic acid (Lancaster) and alanine *iso*-butyl ester (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.89 (m, 1H), 7.76 (m, 1H), 7.38 (m, 3H), 6.07 (m, 1H), 4.57 (m, 1H), 3.92 (m, 2H), 3.82 (s. 4H), 1.84 (m, 1H), 1.32 (d, 3H) 0.85 (d, 6H).
C₁₇H₂₁NO₃S (MW = 319, Mass Spectroscopy (MH⁺ 320)).

### Example B51

### Synthesis of N-[(2-chloro-5-thienyl)acetyl]alanine iso-butyl ester

Following General Procedure BI above, and using 5-chloro-2-thienyl)acetic acid (CAS# 13669-19-7) and alanine *iso*-butyl ester (prepared following General Procedure BJ above), the title compound was prepared. The reaction was monitored by tlc on silica gel and purification was by filtration as described in the general procedure.
NMR data was as follows
¹H-nmr (CDCl₃): δ = 6.77 (m, 1H), 6.68 (d, 1H), 6.31 (bm, 1H), 4.59 (m, 1H), 3.91 (m, 2H), 3.38 (s, 2H), 1.90 (m, 1H), 1.39 (d, 3H) 0.89 (d, 6H).
C₁₃H₁₈NO₃SCl (MW = 303, Mass Spectroscopy (MH⁺ 303)).

### Example B52

### Synthesis of N-[(3-methylisoxazol-5-yl)acetyl]alanine iso-butyl ester

Following General Procedure BI above, and using (3-methyl-isoxazol-5-yl)acetic acid (CAS# 19668-85-0) and alanine *iso*-butyl ester (prepared following- General Procedure BJ above), the title compound was prepared. The reaction was monitored by tlc on silica gel and purification was by filtration as described in the general procedure.
NMR data was as follows:
¹H-nmr (CDCl₃): δ = 6.07 (s, 2H), 4.56 (m, 1H), 3.92 (m, 2H), 3.68 (s, 2H), 2.29 (s, 3H), 1.94 (m, 1H), 1.89 (d, 3H) 0.91 (d, 6H).
C₁₃H₂₀N₂O₄ (MW = 268, Mass Spectroscopy (MH⁺ 269)).

### Example B53

### Synthesis of N-[(2-phenylthiothienyl)acetyl]alanine iso-butyl ester

Following General Procedure BI above, and using (2-phenyl-thiothienyl)acetic acid and alanine *iso*-butyl ester (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.21-7.11 (m, 6H), 6.92 (d, 1H), 4.56(m, 1H), 3.87 (m, 2H), 3.72 (s, 2H), 1.94 (m, 1H), 1.38 (d, 3H) 0.89 (d, 6H).
C₁₉H₂₃NO₃S₂ (MW = 377, Mass Spectroscopy (MH⁺ 378)).

### Example B54

### Synthesis of N-[(6-methoxybenzothiophen-2-yl)acetyl]alanine iso-butyl ester

Following General Procedure BI above, and using (6-methoxythianaphthen-2-yl)acetic acid and alanine *iso*-butyl ester (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.59 (d, 1H), 7.33 (d, 1H), 7.16 (s, 1H), 7.03 (dd, 1H), 4:56 (m, 1H), 3.87(s, 3H), 3.84 (m, 2H), 3.76 (s, 2H),1.85 (m, 1H), 1.30 (d, 3H) 0.86 (d, 6H).
C₁₈H₂₃NO₄S (MW = 349, Mass Spectroscopy (MH⁺ 350)).

### Example B55

### Synthesis of N-[(3-phenyl-1,2,4-thiadiazol-5-yl)acetyl]alanine iso-butyl ester

Following General Procedure BI above, and using (3-phenyl-1,2,4-thiadiazol-5-yl)acetic acid (CAS# 90771-06-5) and alanine *iso*-butyl ester (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.47 (m, 5H), 4.66 (m, 1H), 4.16 (s, 2H), 3.91 (m, 2H), 1.93 (m, 1H), 1.48 (d, 3H) 0.93 (d, 6H).
C₁₇H₂₁N₃O₃S (MW = 347, Mass Spectroscopy (MH⁺ 348)).

### Example B56

### Synthesis of N-[2-phenyloxazol-4-yl)acetyl]alanine iso-butyl ester

Following General Procedure BI above, and using (2-phenyloxazol-4-yl)acetic acid (CAS# 22086-89-1) and alanine *iso*-butyl ester (prepared following General Procedure BJ above), the title compound was prepared. The reaction was monitored by tlc on silica gel and purification was by filtration as described in the general procedure.

### Example B57

### Synthesis of N-[(3-methylphenyl)acetyl]alanine iso-butyl ester

Following General Procedure BI above, and using 3-methylphenylacetic acid (Aldrich) and alanine *iso*-butyl ester (prepared following General Procedure BJ above), the title compound was prepared. The reaction was monitored by tic on silica gel and purification was by filtration as described in the general procedure.
NMR data was as follows:
¹H-nmr (CDCl₃): δ = 7.21 (m, 1H), 7.07 (m, 3H), 4.54 (m, 1H), 3.83 (m, 2H), 3.52 (s, 2H), 2.35 (s, 3H), 1.87 (m,1H), 1.32 (d, 3H), 0.88 (d, 6H).
C₁₆H₂₃NO₃ (MW = 277, Mass Spectroscopy (MH⁺ 278)).

### Example B58

### Synthesis of N-[(2,5-difluorophenyl)acetyl]alanine iso-butyl ester

Following General Procedure BI above, and using 2,5-difluorophenylacetic acid (Aldrich) and alanine *iso*-butyl ester (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.08-6.94 (m, 3H), 4.57 (m, 1H), 3.91 (m, 2H), 3.56 (s, 2H), 1.92 (m, 1H), 1.41 (d, 3H) 0.91 (d, 6H).
C₁₅H₁₉NO₃F₂ (MW = 299, Mass Spectroscopy (MH⁺ 300)).

### Example B59

### Synthesis of N-[(3,5-diflurophenyl)acetyl]alanine iso-butyl ester

Following General Procedure BI above, and using 3,5-difluorophenylacetic acid (Aldrich) and alanine *iso*-butyl ester (prepared following General Procedure BJ above), the title compound was prepared. The reaction was monitored by tlc on silica gel.and purification was by filtration as described in the general procedure.
NMR data was as follows:
¹H-nmr (CDCl₃): δ = 6.81 (m, 2H), 6.74 (m, 1H), 6.06 (m, 1H), 4.57 (m, 1H), 3.92 (m, 2H), 3.51 (s, 2H), 1.94 (m, 1H), 1.36 (d, 3H) 0.87 (d, 6H).
C₁₅H₁₉NO₃F₂ (MW = 299, Mass Spectroscopy (MH⁺ 300)).

### Example B60 .

### Synthesis of N-[(3-thienyl)acetyl]alanine iso-butyl ester

Following General Procedure BI above, and using 3-thiopheneacetic acid (Aldrich) and alanine *iso*-butyl ester (prepared following General Procedure BJ above), the title compound was prepared. The reaction was monitored by tlc on silica gel and purification was by filtration as described in the general procedure.
NMR data was as follows:
¹H-nmr (CDCl₃): δ = 7.33 (m, 1H), 7.14 (m, 1H), 7.01 (m, 1H), 6.09 (m, 1H), 4.58 (m, 1H), 3.88 (m, 2H), 3.60 (s, 2H), 1.91 (m, 1H), 1.37 (d, 3H) 0.92 (d, 6H).
Optical Rotation: [α]₂₃ -52 (c 1 MeOH) @ 589 nm.
C₁₃H₁₉NO₃S (MW = 269, Mass Spectroscopy (MH⁺ 269)).

### Example B61

### Synthesis of N-[(4-methylphenyl)acetyl]-L-alanine iso-butyl ester

Following General Procedure BI above, and using 4-methylphenylacetic acid (Aldrich) and L-alanine *iso*-butyl ester (prepared following General Procedure BJ above), the title compound was prepared. The reaction was monitored by tic on silica gel and purification was by filtration as described in the general procedure.
NMR data was as follows:
¹H-nmr (CDCl₃): δ = 7.11 (s, 4H), 5.93 (m, 1H), 4.58 (m, 1H), 3.88 (m, 2H), 3.54 (s, 2H), 2:33 (s, 3H), 1.89 (m, 1H), 1.32 (d, 3H), 0.89 (d, 6H).
C₁₆H₂₃NO₃ (MW = 277.35, Mass Spectroscopy (MH⁺ 278)).

### Example B62

### Synthesis of N-(phenylacetyl)-L-alanine S-1-(methoxycarbonyl) iso-butyl ester

Following General Procedure BK and using (S)-(+)-2-hydroxy-2-methylbutyric acid (Aldrich) in place of the amino acid, methyl (S)-(+)-2-hydroxy-2-methylbutyrate was prepared.

Methyl (S)-(+)-2-hydroxy-2-methylbutyrate was then coupled with carbobenzyloxy-L-alanine (Aldrich) using General Procedure BE to provide carbobenzyloxy-L-alanine S-1-(methoxycarbonyl) *iso*-butyl ester.

Carbobenzyloxy-L-alanine S-1-(methoxycarbonyl) *iso*-butyl ester (1.0 g) was then dissolved in 20 mL of methanol and 6N HCl (0.5 mL) and 10% palladium on carbon (0.1 g) were added. This reaction mixture was hydrogenated at 40 psi of hydrogen on a Parr apparatus for 5 hours at room temperature and then filtered through a pad of Celite. The filtrate was concentrated at reduced pressure to provide L-alanine S-1-(methoxycarbonyl) *iso*-butyl ester hydrochloride (98% yield).

L-Alanine S-1-(methoxycarbonyl) *iso*-butyl ester hydrochloride was then coupled to phenylacetic acid using General Procedure BG to provide the title compound.
NMR data was as follows:
¹H-nmr (CDCl₃): δ = 7.35 - 7.20 (m, 5H), 6.22 (bd, 1H), 4.83 (d, 1H), 4.65 (p, 1H), 3.68 (s, 3H), 3.55 (s, 2H), 2.21 (m, 1H), 1.40 (d, 3H), 0.97 (d, 3H), 0.93 (d, 3H).
¹³C-nmr (CDCl₃): δ = 173.25, 171.18, 170.22, 135.11, 129.94, 129.50, 127.88, 52.67, 48.49, 43.98, 30.53, 19.21, 18.75, 17.58.

### Example B63

### Synthesis of N-[(3-nitropbenyl)acetyl)-L-alanine iso-butyl ester

Following General Procedure BH above and using 3-nitrophenylacetic acid (Aldrich) and L-alanine *iso*-butyl ester hydrochloride (from Example BB above), the title compound was prepared. The reaction was monitored by tlc on silica gel and purification was by recrystallization from butyl chloride.
NMR data was as follows:
¹H-nmr (CDCl₃): δ = 8.17 (m, 2H), 7.68 (d, 1H), 7.52 (t, 1H), 6.18 (m, 1H), 4.48 (m, 1H), 3.94 (m, 2H), 3.67 (s, 2H), 1.93 (m, 1H), 1.42 (d, 3H), 0.91 (d, 3H).
Optical Rotation: [α]₂₃ -49 (c 5, MeOH).

### Example B64

### Synthesis of N-[(3,5-difluorophenyl)acetyl]alanine ethyl ester

Following General Procedure BG and using 3,5-difluorophenylacetic acid (Aldrich) and alanine ethyl ester (Aldrich), the title compound was prepared as a solid with a melting point of 93°-95°C. The reaction was monitored by tlc on silica gel (Rf = 0.8 in EtOAC) and purification was by chromatography on silica gel using EtOAc as the eluant followed by recrystallization from 1-chlorobutane.
NMR data was as follows:
¹H-nmr (DMSO-*d*₆): δ = 1.30 (d, 3H); 3.52 (s, 2H).
C₁₃H₁₅NO₃F₂ (MW =271.26, Mass Spectroscopy (MH⁺ 271)).

### Example B65

### Synthesis of N-[(3-nitrophenyl)acetyl]methionine ethyl ester

Following General Procedure BG above and using 3-nitrophenylacetic acid (Aldrich) and methionine ethyl ester hydrochloride (Aldrich), the title compound was prepared. The reaction was monitored by tlc on silica gel and purification was by recrystallization from butyl chloride.
NMR data was as follows:
¹H-nmr (CDCl₃): δ = 8.18 (s, 1H), 8.15 (d, 1H) 7.66 (d, 1H), 7.48 (t, 1H), 6.30 (m, 1H), 4.67 (m, 1H), 4.21 (t, 2H), 3.67 (s, 2H), 2.47 (t, 2H), 2.12 (m, 2 H), 2.08 (s, 3H), 1.27 (t, 3H).
Optical Rotation: [α]₂₃ -30 (c 5, MeOH).

### Example B66

### Synthesis of N-[(3-chlorophenyl)acetyl]alanine iso-butyl ester

Following General Procedure BG above and using 3-chlorophenylacetic acid (Aldrich) and alanine *iso*-butyl ester (prepared following General Procedure BJ above), the title compound was prepared. The reaction was monitored by tlc on silica gel.
NMR data was as follows:
¹H-nmr (CDCl₃): δ = 7.29 (m, 3H); 7.18 (m, 1H), 6.0 (m, 1H), 4.56 (m, 1H), 3.89 (m, 2H), 3.53 (s, 2H), 1.91 (m, 1H), 1.39 (d, 3 H), 0.91 (d, 3H).
Optical Rotation: [α]₂₃ -45 (c 5, MeOH).
C₁₅H₂₀NO₃Cl (MW = 297.78, Mass Spectroscopy (MH⁺ 297)).

### Example B67

### Synthesis of N-[(3-chlorophenyl)acetyl]alanine 2-(N,N-dimethylamino)ethyl ester

Following General Procedure BC above, and using N-(3-chlorophenylacetyl)alanine (from Example BD above) and 2-(N,N-dimethyl amino) ethanol (Aldrich), the title compound can be prepared. The reaction was monitored by tlc on silica gel and purification was by liquid chromatography using 0.1:2:0.79 NH₄OH:EtOH:CHCl₃ as the eluant.
NMR data was as follows:
¹H-nmr (CDCl₃): 7.37 (s, 1H), 7.33-7.2 (m, 3H), 4.675-4.6 (m, 1H), 4.5-4.37 (m, 1H), 4.25-4.13 (m, 1H), 3.6 (d, *J* = 7 Hz 2H), 2.86 (bs, 2H), 2.3 (s, 6H), 1.23 (d, *J* = 9 Hz, 3H).
C₁₅H₂₁N₂O₃Cl (MW = 313.799, Mass Spectroscopy (M⁺ 313)).

### Example B68

### Synthesis of 2-[(3,5-dichlorophenyl)acetamido]hexanoic acid methyl ester

Following General Procedure BF above, an using 3.5-dichlorophenylacetic acid (from Example BC above) and L-norleucine methyl ester hydrochloride (Bachem), the title compound was prepared as a solid having a melting point of 77°-78°C. The reaction was monitored by tlc on silica gel (Rf = 0.70 in 40% EtOAC/hexanes) and purification was by flash chromatography on silica gel using 40% EtOAc/hexanes as the eluant.
NMR data was as follows:
¹H-nmr (CDCl₃): δ = 7.20 (s), 7.18 (s), 6.6 (m), 4.55 (m), 3.7 (s), 3.5 (s), 3.4 (s), 2.0 (s), 1.8 (m), 1.6 (m), 1.2 (m), 0.8 (t).
¹³C-nmr (CDCl₃): δ = 173.54, 169.67, 138.43, 135.72, 128.33, 128.07, 78.04, 77.62, 77.19, 53.04, 52.90, 43.14, 32.57, 27.87, 22.81, 14.41.

### Example B69

### Synthesis of N-[(3,5-diclorophenyl)acetyl]-L-alanine iso-butyl ester

Following General Procedure BF above, and using 3,5-dichlorophenylacetic acid (from Example BC above) and L-alanine *iso*-butyl ester hydrochloride (from Example BB above), the title compound was prepared as a solid having a melting point of 115°-116°C. The reaction was monitored by tlc on silica gel (Rf = 0.40 in 3% methanol/dichloromethane) and purification was by flash chromatography on silica gel using 3% methanol/dichloromethane as the eluant.
NMR data was as follows:
¹H-nmr (CDCl₃): δ = 7.27 (d, *J* = 2 Hz, 1H), 7.19 (s, 2H), 6.22 (d, *J* = 6 Hz, 1H), 4.59 (quint., *J* = 7 Hz, 1H), 3.9 (q, *J* = 4 Hz, 2H), 3.5 (s, 2H), 1.9 (m, 1H), 1.4 (d, *J* = 7 Hz, 3H), 0.91 (d, *J* = 7 Hz, 6H).
¹³C-nmr (CDCl₃): δ = 173.45, 169.37, 138.31, 135.75, 128.39, 128.11, 78.04, 77.61, 77.19, 72.19, 54.03, 48.97, 43.12, 28.24, 19.52, 19.49, 19.09.
C₁₅H₁₉NO₃Cl₂ (MW = 331.9, Mass Spectroscopy (MH⁺ 332)).

### Example B70

### Synthesis of N-(cyclohexylacetyl)-L-alanine iso-butyl ester

Following General Procedure BB above, and using cyclohexylacetic acid (Aldrich) and L-alanine *iso*-butyl ester hydrochloride (from Example BB above), the title compound was prepared as a solid having a melting point of 92°C-93°C. The reaction was monitored by tlc on silica gel (Rf = 0.39 in 1:3 EtOAc:hexane) and purification was by extraction with Et₂O followed by washes with aqueous K₂CO₃ and aqueous HCl.
NMR data was as follows:
¹H-nmr (CDCl₃): δ = 0.93 (d, J = 6.7 Hz, 6H), 0.85-1.01 (m, 2H), 1.05-1.35 (m. 3H), 1.40 (d, *J* = 7.1 Hz, 3H), 1.60-1.85 (m, 6H), 1.95 (m, 1H), 2.06 (d, *J* 7.0 Hz, 2H), 3.92 (m, 2H), 4.61 (m, 1H), 6.08 (bd, 1H).
¹³C-nmr (CDCl₃): δ = 18.7, 18.9, 26.0, 26.1, 27.6, 33.0, 35.3, 44.6, 47.9, 71.4, 171.8, 173.3.
C₁₅H₂₇NO₃ (MW = 269.39, Mass Spectroscopy (MH⁺ 270)).

### Example B71

### Synthesis of N-(cyclopentylacetyl)-L-alanine iso-butyl ester

- Following General Procedure BB above, and using cyclopentylacetic acid (Aldrich) and L-alanine *iso*-butyl ester hydrochloride (from Example BB above), the title compound was prepared as a solid having a melting point of 62°C-64°C. The reaction was monitored by tlc on silica gel (Rf = 0.37 in 1:3 EtOAc:hexane) and purification was by extraction with Et₂O followed by washes with aqueous K₂CO₃ and aqueous HCl.
NMR data was as follows:
¹H-nmr (CDCl₃): δ = 0.87 (d, *J =* 6.8 Hz, 6H), 1.01-1.17 (m, 2H), 1.34 (d, *J* = 7.2 Hz, 3H), 1.40-1.62 (m, 4H), 1.70-1.83 (m, 2H), 1.89 (m, 1H), 2.15 (m, 3H), 3.86 (m, 2H), 4.55 (m, 1H), 6.30 (d, *J* = 7.1 Hz, 1H).
¹³C-nmr (CDCl₃): δ = 18.4, 18.78, 18.80, 24.8 (very high), 27.5, 32.27, 32.32, 36.9, 42.5, 47.7, 71.2, 172.2, 173.2.
Elemental Analysis-Calc (%): C, 65.85; H, 9.87; N, 5.49; Found (%): C, 66.01; H, 10.08; N, 5.49.
C₁₄H₂₅NO₃ (MW = 255.36, Mass Spectroscopy (MH⁺ 256)).

### Example B72

### Synthesis of N-[(cyclohex-1-enyl)acetyl]-L-alanine iso-butyl ester

Following General Procedure BB above, and using cyclohex-1-enyl acetic acid (Alfa) and L-alanine iso-butyl ester hydrochloride (from Example BB above), the title compound was prepared as a solid having a melting point of 49°C-51°C. The reaction was monitored by tic on silica gel (Rf = 0.40 in 1:3. EtOAc:hexane) and purification was by extraction with Et₂O followed by washes with aqueous K₂CO₃ and aqueous HCl.
NMR data was as follows:
¹H-nmr (CDCl₃): δ = 0.91 (d. *J =* 4.5 Hz, 3H), 0.93 (d, *J* = 6.7 Hz. 3H), 1.40 (d. *J* = 7.2 Hz, 3H), 1.52-1.70 (m, 4H), 1.97 (m, 3H), 2.06 (bs, 2H), 2.89 (s, 2H), 3.92 (m, 2H), 4.59 (m, 1H), 5.65 (s, 1H), 6.33 (d, *J =* 6.6 Hz. 1H).
¹³C-nmr (CDCl₃): δ = 18.7, 18.91, 18.93, 21.9, 22.7, 25.3, 27.6, 28.3, 46.1, 47.9, 71.4, 127.1, 132.5, 170.6, 173.1.
Elemental Analysis-Calc (%): C, 67.38; H, 9.42; N, 5.24; Found (%): C, 67.34; H, 9.54; N, 5.16.
C₁₅H₂₅NO₃ (MW = 267.37, Mass Spectroscopy (MH⁻ 268)).

### Example B73

### Synthesis of N-[(3-chlorophenyl)acetyl]alanine 3-methylbut-2-enyl thioester

Following General Procedure BC above, and using *N*-[(3-chlorophenyl)acetyl] alanine and 3-methyl-2-butene thioester (TCI), the title compound can be prepared. The reaction was monitored by tlc on silica gel and purification was by liquid chromatography using 3:7 EtOAc:Hexane as the eluant.
NMR data was as follows:
¹H-nmr (DMSO-*d*₆): δ = 5.2-5.075 (m, 1H), 4.37 (dq, *J*= 9 Hz, 1H), 3.56 (s), 3.43 (d, *J* = 12 Hz, 2H), 1.266 (d, *J* = 12 Hz, 6H) 1.3 (d, *J* = 9 Hz 3H).
C₁₆H₂₀NO₂ClS (MW = 325.86, Mass Spectroscopy (M⁺ 325)).

### Example B74

### Synthesis of N [(2-phenyl)-2-fluoroacetyl]alanine ethyl ester

Following General Procedure BF above, and using α-fluorophenyl acetic acid (Aldrich) and alanine ethyl ester (Aldrich), the title compound was prepared. The reaction was monitored by tlc on silica gel (Rf = 0.75 in 1:1 EtOAc:hexane) and purification was by chromatography on silica gel using 1:2 ethyl acetate/hexanes as the eluent.
NMR data was as follows:
¹H-nmr (DMSO-*d*₆): δ = 1.14 (q, 3H), 1.34 (d, 3H), 4.07 (m, 2H), 4.33 (m, 1H), 5.84 (d, 1H), 6.01 (d, 1H), 7.40-7.55 (m, 5H), 8.87 (m, 1H).
C₁₃H₁₆NO₃F (MW = 253.27, Mass Spectroscopy (MH⁺ 253)).

### Example B75

### Synthesis of N-3,5-difluorophenylacetyl)-L-phenylglycine methyl ester

Following General Procedure BF above, and using 3,5-difluorophenylacetic acid (Aldrich) and L-phenylglycine methyl ester hydrochloride (Bachem), the title-compound was prepared.
NMR data was as follows:
¹H-nmr (CDCl₃): δ =7.4-7.3 (m, 5H), 6.9-6.7 (m, 3H), 6.55 (d 1H, 7.1 Hz), 5.56 (d 1H 7 Hz), 3.72 (s 3H), 3.57 (s 2H)
¹³C-nmr (CDCl₃): δ = 197.6, 177.6, 171.8, 169.3, 136.7, 129.6, 129.3, 127.8, 113.0, 112.9, 112.7, 111.4, 103.8, 103.5, 65.1, 57.2, 53.5, 45.1, 43.3, 43.3
C₁₇H₁₅NO₃F₂ (MW = 319.31, Mass Spectroscopy (MH +320)).

### Example B76

### Synthesis of N-(3,5-difluorophenylacetyl)-L-phenylglycine iso-butyl ester

The 3,5-difluorophenylacetic acid (Aldrich) was EDC coupled to L-phenylglycine methyl ester hydrochloride (Bachem) via General Procedure BF above.

The resulting compound was placed in a large excess of the desired alcohol. A catalytic amount of dry NaH was added, and the reaction was followed by tlc until the presence of starting material was no longer detected. The reaction was quenched with a few milliliters of 1N HCl, and after a few minutes of stirring saturated aqueous NaHCO₃ was added. The volume of the reaction mixture was reduced on a rotary evaporator until the excess alcohol was removed and then the remaining residue was taken up in ethyl acetate and additional water was added. The organic phase was washed with saturated aqueous NaCl and dried over MgSO₄. The solution was stripped free of solvent on a rotary evaporator, and the crude product residue was then further purified by chromatography.
NMR data was as follows:
¹H-nmr (CDCl₃): δ = 7.35-7.3 (m 5H), 6.8-6.7 (m 3H) 6.60 (d 1H, 7 Hz), 5.55 (d 1H 7.1 Hz), 3.9 (m 2H), 3.60 (s 2H), 1.85 (m 1H 7 Hz), 0.8 (q 6H 7 Hz)
¹³C-nmr (CDCl₃): δ = 171.3, 169.3, 165.4, 138.5, 137.0, 129.5, 129.2, 127.6, 113.1, 113.0, 112.8, 112.7, 103.8, 103.5, 103.2, 75.5, 57.2, 43.4, 43.3, 28.2, 19.3
C₂₀H₂₁NO₃F₂ (MW = 361.39, Mass Spectroscopy (MH +362)).

### Example B77

### Synthesis of N-(cyclopentylacetyl)-L-phenylglycine methyl ester

Following General Procedure BD above, and using cyclopentylacetic acid (Aldrich) with L-phenylglycine methyl ester hydrochloride (Bachem) the title compound was prepared.
NMR data was as follows:
¹H-nmr (CDCl₃): δ = 7.35 (s, 5H), 6.44 (bd, 1H), 5.6 (d, 1H), 3.72 (s, 3H), 2.24 (bs, 3H), 1.9-1.4 (m, 6H), 1.2-1.05 (m, 2H)
¹³C-nmr (CDCl₃): δ = 172.3, 171.7, 136.7, 129.0, 128.6, 127.3, 56.2, 52.7, 42.5, 36.9, 32.40, 32.38, 24.8

### Example B78

### Synthesis of N-(cyclopentylacetyl)-L-alanine methyl ester

Following General Procedure BD above, and using cyclopentylacetic acid (Aldrich) with L-alanine methyl ester hydrochloride (Sigma) the title compound was prepared.
NMR data was as follows:
¹H-nmr (CDCl₃): δ = 6.38 (d, 1H), 4.50 (m, 1H), 3.65 (s, 3H), 2.13 (bs, 3H), 1.80-1.00 (m (includes d at 1.30, 3H), 11H)
¹³C-nmr (CDCl₃): δ = 173.7, 172.5, 52.1, 47.6, 42.3, 36.8, 32.15, 32.14, 18.0
C₁₁H₁₉NO₃ (MW = 213.28, Mass Spectroscopy (MH⁺ 214)).

### Example B79

### Synthesis of N-(cyclopropylacetyl)-L-phenylglycine methyl ester

Following General Procedure BD above, and using cyclopropylacetic acid (Aldrich) with L-phenylglycine methyl ester hydrochloride (Bachem), the title compound was prepared.
NMR data was as-follows:
¹H-nmr (CDCl₃): δ = 7.35 (m, 5H) 6.97 (bd, *J* = 7.2 Hz, 1H) 5.59 (d, *J* = 7.8 Hz, 1H), 3.71 (s, 3H), 2.17 (m, 2H), 1.05-0.95 (m, 1H), 0.62 (m, 2H), 0.02 (m, 2H)
¹³C-nmr (CDCl₃): δ = 171.9, 174.6, 136.6, 129.0, 128.5, 127.2, 56.1, 52.7, 41.0, 6.9, 4.37, 4.33

### Example B80

### Synthesis of N-(cyclopropylacetyl)-L-alanine methyl ester

Following General Procedure BD above, and using cyclopropylacetic acid (Aldrich) with L-alanine methyl ester hydrochloride (Sigma), the title compound was prepared.
NMR data was as follows:
¹H-nmr (CDCl₃): δ = 6.60 (d, 1H), 4.55 (m, 1H), 3.69 (s, 3H), 2.10 (m, 2H), 1.34 (d, 3H), 0.95 (m, 1H), 0.58 (m, 2H) 0.15 (m, 2H)
¹³C-nmr (CDCl₃): δ = 173.7, 172.3, 52.3, 47.7, 41.0, 18.2, 6.7, 4.27, 4.22

### Example B81

### Synthesis of N-[(3-nitrophenyl)acetyl]-L-methionine iso-butyl ester

Following General Procedure BH above, and using nitrophenylacetic acid (Aldrich) and L-methionine (Aldrich), the title compound was prepared as a tan oil. The reaction was monitored by tlc on silica gel.
NMR data was as follows:
¹H-nmr (CDCl₃): δ = 8.16 (m, 2H) 7.67 (d,1H) 7.32 (t, 1H), 6.31 (bd, 1H), 4.69 (m, 1H), 3.90 (d, 2H), 3.68 (s, 2H), 2.47 (t, 2H), 2.15 (m, 1H), 2.02 (s, 3H), 1.90 (m, 2H), 0.91 (d, 6H).
C₁₇H₂₄N₂O₅S (MW = 368.4, Mass Spectroscopy (MH⁻ 368)).

### Example Bio-1

### Cellular Screen for the Detection of Inhibitors of β-Amyloid Production

Numerous compounds of formula I above were assayed for their ability to inhibit β-amyloid production in a cell line possessing the Swedish mutation. This screening assay employed cells (K293 = human kidney cell line) which were stably transfected with the gene for amyloid precursor protein 751 (APP751) containing the double mutation Lys₆₅₁Met₆₅₂ to Asn₆₅₁Leu₆₅₂ (APP751 numbering) in the manner described in International Patent Application Publication No. 94/10569⁸ and Citron et al.¹². This mutation is commonly called the Swedish mutation and the cells, designated as "293 751 SWE", were plated in Corning 96-well plates at 2-4 x 10⁴ cells per well in Dulbecco's minimal essential media (Sigma, St. Louis, MO) plus 10% fetal bovine serum. Cell number is important in order to achieve β-amyloid ELISA results within the linear range of the assay (∼0.2 to 2.5 ng per mL).

Following overnight incubation at 37°C in an incubator equilibrated with 10% carbon dioxide, media were removed and replaced with 200 µL of a compound of formula I (drug) containing media per well for a two hour pretreatment period and cells were incubated as above. Drug stocks were prepared in 100% dimethyl sulfoxide such that at the final drug concentration used in the treatment, the concentration of dimethyl sulfoxide did not exceed 0.5% and, in fact, usually equaled 0.1%.

At the end of the pretreatment period, the media were again removed and replaced with fresh drug containing media as above and cells were incubated for an additional two hours. After treatment, plates were centrifuged in a Beckman GPR at 1200 rpm for five minutes at room temperature to pellet cellular debris from the conditioned media. From each well, 100 µL of conditioned media or appropriate dilutions thereof were transferred into an ELISA plate precoated with antibody 266 [P. Seubert, Nature (1992) 359:325-327] against amino acids 13-28 of β-amyloid peptide as described in International Patent Application Publication No. 94/10569⁸ and stored at 4°C overnight. An ELISA assay employing labelled antibody 3D6 [P. Seubert, Nature (1992) 359:325-327] against amino acids 1-5 of β-amyloid peptide was run the next day to measure the amount of β-amyloid peptide produced.

Cytotoxic effects of the compounds were measured by a modification of the method of Hansen, et al.¹³. To the cells remaining in the tissue culture plate was added 25 µL of a 3-(4,5-dimethylthiazol-2-yl)-2,5-diphenyltetrazolium bromide (MTT) (Sigma, St. Louis, MO) stock solution (5 mg/mL) to a final concentration of 1 mg/mL. Cells were incubated at 37°C for one hour, and cellular activity was stopped by the addition of an equal volume of MTT lysis buffer (20% w/v sodium dodecylsulfate in 50% dimethylformamide, pH 4.7). Complete extraction was achieved by overnight shaking at room temperature. The difference in the OD₅₆₂ₙₘ and the OD₆₅₀ₙₘ was measured in a Molecular Device's UVₘₐₓ microplate reader as an indicator of the cellular viability.

The results of the β-amyloid peptide ELISA were fit to a standard curve and expressed as ng/mL β-amyloid peptide. In order to normalize for cytotoxicity, these results were divided by the MTT results and expressed as a percentage of the results from a drug free control. All results are the mean and standard deviation of at least six replicate assays.

The test compounds were assayed for β-amyloid peptide production inhibition activity in cells using this assay. The results of this assay demonstrate that the compounds of formula I inhibit β-amyloid peptide production by at least 30% as compared to control.

### Example Bio-2

### In Vivo Suppression of β-Amyloid Release and/or Synthesis

This example illustrates how the compounds of this invention could be tested for in *vivo* suppression of β-amyloid release and/or synthesis. For these experiments, 3 to 4 month old PDAPP mice are used [Games et al., (1995) Nature 373-523-527]. Depending upon which compound is being tested, the compound is usually formulated at between 1 and 10 mg/mL. Because of the low solubility factors of the compounds, they may be formulated with various vehicles, such as corn oil (Safeway, South San Francisco, CA); 10% ethanol in corn oil; 2-hydroxypropyl-β-cyclodextrin (Research Biochemicals International, Natick MA); and carboxy-methyl-cellulose (Sigma Chemical Co., St. Louis MO).

The mice are dosed subcutaneously with a 26 gauge needle and 3 hours later the animals are euthanized via CO₂ narcosis and blood is taken by cardiac puncture using a 1 cc 25G 5/8" tuberculin syringe/needle coated with solution of 0.5 M EDTA, pH 8.0. The blood is placed in a Becton-Dickinson vacutainer tube containing EDTA and spun down for 15 minutes at 1500 xg at 5°C. The brains of the mice are then removed and the cortex and hippocampus are dissected out and placed on ice.

### 1. Brain Assay

To prepare hippocampal and cortical tissue for enzyme-linked immunosorbent assays (ELISAs) each brain region is homogenized in 10 volumes of ice cold guanidine buffer (5.0 M guanidine-HCl, 50 mM Tris-HCl, pH 8.0) using a Kontes motorized pestle (Fisher, Pittsburgh PA). The homogenates are gently rocked on a rotating platform for three to four hours at room temperature and stored at -20°C prior to quantitation of β-amyloid.

The brain homogenates are diluted 1:10 with ice-cold casein buffer [0.25% casein, phosphate buffered saline (PBS), 0.05% sodium azide, 20 µg/ml aprotinin, 5 mM EDTA, pH 8.0, 10 µg/ml leupeptin], thereby reducing the final concentration of guanidine to 0.5 M, before centrifugation at 16,000 xg for 20 minutes at 4°C. Samples are further diluted, if necessary, to achieve an optimal range for the ELISA measurements by the addition of casein buffer with 0.5 M guanidine hydrochloride added. The β-amyloid standards (1-40 or 1-42 amino acids) were prepared such that the final composition equaled 0.5 M guanidine in the presence of 0.1 % bovine serum albumin (BSA).

The total β-amyloid sandwich ELISA, quantitating both β-amyloid (aa 1-40) and β-amyloid (aa 1-42) consists of two monoclonal antibodies (mAb) to β-amyloid. The capture antibody, 266 [P. Seubert, Nature (1992) 359:325-327], is specific to amino acids 13-28 of β-amyloid. The antibody 3D6 [Johnson-Wood et al., PNAS USA (1997) 94:1550-1555], which is specific to amino acids 1 - 5 of β-amyloid, is biotinylated and served as the reporter antibody in the assay. The 3D6 biotinylation procedure employs the manufacturer's (Pierce, Rockford IL) protocol for NHS-biotin labeling of immunoglobulins except that 100 mM sodium bicarbonate, pH 8.5 buffer is used. The 3D6 antibody does not recognize secreted amyloid precursor protein (APP) or full-length APP but detects only β-amyloid species with an amino terminal aspartic acid. The assay has a lower limit of sensitivity of -50 pg/ml (11 pM) and shows no cross-reactivity to the endogenous murine β-amyloid peptide at concentrations up to 1 ng/ml.

The configuration of the sandwich ELISA quantitating the level of β-amyloid (aa 1-42) employs the mAb 21F12 [Johnson-Wood et al., PNAS USA (1997) 94:1550-1555] (which recognizes amino acids 33-42 of β-amyloid) as the capture antibody. Biotinylated 3D6 is also the reporter antibody in this assay which has a lower limit of sensitivity of ~125 pg/ml (28 pM).

The 266 and 21F12 capture mAbs are coated at 10 µg/ml into 96 well immunoassay plates (Costar, Cambidge MA) overnight at room temperature. The plates are then aspirated and blocked with 0.25% human serum albumin in PBS buffer for at least 1 hour at room temperature, then stored desiccated at 4°C until use. The plates are rehydrated with wash buffer (Tris-buffered saline, 0.05% Tween 20) prior to use. The samples and standards are added to the plates and incubated overnight at 4°C. The plates are washed ≥ 3 times with wash buffer between each step of the assay. The biotinylated 3D6, diluted to 0.5 µg/ml in casein incubation buffer (0.25% casein, PBS, 0.05% Tween 20, pH 7.4) is incubated in the well for 1 hour at room temperature. Avidin-HRP (Vector, Burlingame CA) diluted 1:4000 in casein incubation buffer is added to the wells for 1 hour at room temperature. The colorimetric substrate, Slow TMB-ELISA (Pierce, Cambridge MA), is added and allowed to react for 15 minutes, after which the enzymatic reaction is stopped with addition of 2 N H₂SO₄. Reaction product is quantified using a Molecular Devices Vₘₐₓ (Molecular Devices, Menlo Park CA) measuring the difference in absorbance at 450 nm and 650 nm.

### 2. Blood Assay

The EDTA plasma is diluted 1:1 in specimen diluent (0.2 gm/l sodium phosphate•H₂O (monobasic), 2.16 gm/l sodium phosphate•7H₂O (dibasic), 0.5gm/l thimerosal, 8.5 gm/l sodium chloride, 0.5 ml Triton X-405, 6.0 g/l globulin-free bovine serum albumin; and water). The samples and standards in specimen diluent are assayed using the total β-amyloid assay (266 capture/3D6 reporter) described above for the brain assay except the specimen diluent was used instead of the casein diluents described.

Formulations other than those described above can also be used for oral delivery and intravenous delivery to a mammal. For oral delivery, the compound can be mixed with either 100% corn oil or, alternatively, in a solution comtaining 80% corn oil, 19.5% oleic acid and 0.5% labrafil. The compound can be mixed with the above solutions in concentrations ranging from 1 mg/mL to 10 mg/mL. The compound in solution is preferably administered orally to the mammal at a dose volume of 5 mL/kg of body weight. For IV delivery, the compound is preferably mixed with a solution of 3% ethanol, 3% solutol HS-15 and 94% saline. The compound is preferably mixed with the above solution in concentrations ranging from 0.25 mg/mL to 5 mg/mL. The compound in solution is preferably administered by IV to the mammal at a dose volume of 2 mL/kg of body weight.

From the foregoing description, various modifications and changes in the composition and method will occur to those skilled in the art. All such modifications coming within the scope of the appended claims are intended to be included therein.

## Claims

1. A compound of formula 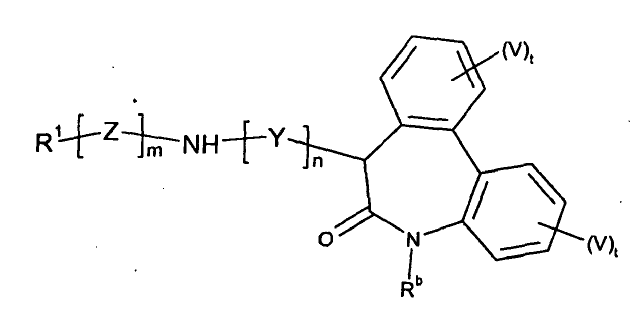
wherein R¹ is selected from the group consisting of alkyl, alkenyl, alkynyl, cycloalkyl, cycloalkenyl, aryl, heteroaryl and heterocyclic;
Y is represented by the formula: 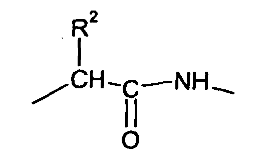
R² is selected from the group consisting of methyl, ethyl, *n*-propyl, *iso*-propyl, *n-*butyl, *iso*-butyl, *sec*-butyl, *tert*-butyl, -CH₂CH(CH₂CH₃)₂, 2-methyl-*n*-butyl, 6-fluoro-*n*-hexyl, phenyl, benzyl, cyclohexyl, cyclopentyl, cycloheptyl, allyl, *iso*-but-2-enyl, 3-methylpentyl, -CH₂-cyclopropyl, -CH₂-cyclohexyl, -CH₂CH₂-cyclopropyl, - CH₂CH₂-cyclohexyl, -CH₂-indol-3-yl, *p*-(phenyl)phenyl, *o*-fluorophenyl, *m-*fluorophenyl, *p*-fluorophenyl, *m*-methoxyphenyl, *p*-methoxyphenyl, phenethyl, benzyl, *m*-hydroxybenzyl, *p*-hydroxybenzyl, *p*-nitrobenzyl, *m*-trifluoromethylphenyl, *p*-(CH₃)₂NCH₂CH₂CH₂O-benzyl, *p*-(CH₃)₃COC(O)CH₂O-benzyl, *p*-(HOOCCH₂O)-benzyl, 2-aminopyrid-6-yl, *p*-(N-morpholino-CH₂CH₂O)-benzyl, -CH₂CH₂C(O)NH₂, -CH₂-imidazol-4-yl, -CH₂-(3-tetrahydrofuranyl), -CH₂-thiophen-2-yl, -CH₂(1-methyl)cyclopropyl, -CH₂-thiophen-3-yl, thiophen-3-yl, thiophen-2-yl, -CH₂-C(O)O-*t*-butyl, -CH₂-C(CH₃)₃, -CH₂CH(CH₂CH₃)₂, 2-methylcyclopentyl, cyclohex-2-enyl, -CH[CH(CH₃)₂)COOCH₃, -CH₂CH₂N(CH₃)₂, -CH₂C(CH₃)=CH₂, -CH₂CH=CHCH₃ (cis and trans), -CH₂OH, -CH(OH)CH₃, -CH(O-*t*-butyl)CH₃, - CH₂OCH₃, -(CH₂)₄NH-Boc, -(CH₂)₄NH₂, -CH₂-pyridyl, pyridyl, -CH₂-naphthyl, -CH₂-(N-morpholino), *p*-(N-morpholino-CH₂CH₂O)-benzyl, benzo[b]thiophen-2-yl, 5-chlorobenzo[b]thiophen-2-yl, 4,5,6,7-tetrahydrobenzo[b]thiophen-2-yl, benzo[b]thiophen-3-yl, 5-chlorobenzo[b]thiophen-3-yl, benzo[b]thiophen-5-yl, 6-methoxynaphth-2-yl, -CH₂CH₂SCH₃, thien-2-yl, and thien-3-yl;
Z is represented by the formula -T-CX'X"C(O)- where T is selected from the group consisting of a bond covalently linking R¹ to -CX'X"-, oxygen, sulfur, -NR⁵ where R⁵ is hydrogen, acyl, alkyl, aryl or heteroaryl group;
X' is hydrogen, hydroxy or fluoro,
X' is hydrogen, hydroxy or fluoro, or X' and X" together form an oxo group;
*m* is an integer equal to 0 or 1;
*n* is an integer equal to 0, 1 or 2;
wherein the term "Alkyl" refers to monovalent alkyl groups preferably having from 1 to 10 carbon atoms;
the term "Alkaryl" refers to -alkylene-aryl groups preferably having from 1 to 8 carbon atoms in the alkylene moiety and from 6 to 10 carbon atoms in the aryl moiety;
the term "Alkoxy" refers to the group "alkyl-O-";
the term "Alkenyl" refers to alkenyl groups preferably having from 2 to 10 carbon atoms;
the term "Alkynyl" refers to alkynyl groups preferably having from 2 to 10 carbon atoms;
the term "Aryl" refers to phenyl or naphthyl, wherein such aryl groups can optionally be substituted with from 1 to 5 substituents selected from the group consisting of alkyl, alkoxy, halo, cyano, nitro, trihalomethyl, and thioalkoxy;
the term "Aryloxy" refers to the group aryl-O- wherein the aryl group is as defined above including optionally substituted aryl groups;
the term "Carboxyalkyl" refers to the group "-C(O)Oalkyl" where alkyl is as defined above;
the term "Cycloalkyl" refers to cyclic alkyl groups of from 3 to 12 carbon atoms having a single cyclic ring or multiple condensed rings;
the term "Cycloalkenyl" refers to cyclic alkenyl groups of from 4 to 8 carbon atoms having a single cyclic ring and at least one point of internal unsaturation;
the term "Heteroaryl" refers to an aromatic carbocyclic group of from 1 to 15 carbon atoms and 1 to 4 heteroatoms selected from oxygen, nitrogen and sulfur within at least one ring;
the term "heterocyclic" refers to a monovalent saturated or unsaturated group having a single ring or multiple condensed rings, from 1 to 15 carbon atoms and from 1 to 4 hetero atoms selected from nitrogen, sulfur or oxygen within the ring;
the term "Thioalkoxy" refers to the group -S-alkyl;
each V is independently selected from the group consisting of hydroxy, acyl, acyloxy, alkyl, alkoxy, alkenyl, alkynyl, amino, aminoacyl, alkaryl, aryl, aryloxy, carboxyl, carboxylalkyl, cyano, halo, nitro, heteroaryl, thioalkoxy and trihalomethyl;
R^{b} is selected from the group consisting of hydrogen, alkyl, alkenyl, alkynyl, acyl, aryl, heteroaryl, and heterocyclic;
t is an integer from 0 to 4.

2. A compound of formula 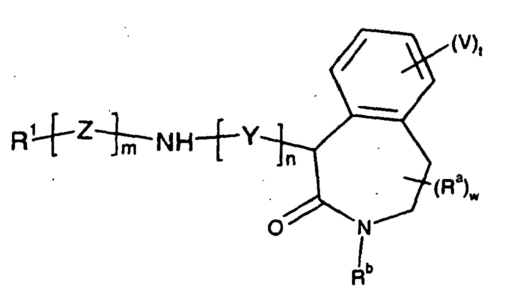
wherein R¹, Z, Y, *m, n,* V, R^{b} and t have the values as defined in claim 1 and
wherein R^{a} is selected from the group consisting of alkyl, alkoxy, amino, carboxyl, carboxyl alkyl, cyano, and halo;
w is an integer from 0 to 3; and
wherein the terms "Alkyl" "Alkoxy" and "Carboxyl alkyl" have the meaning defined in claim 1.

3. A compound of formula I: 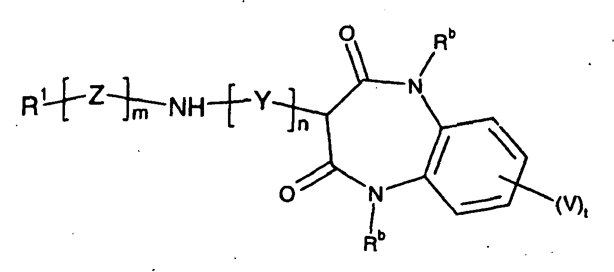
wherein R¹, Z, Y, *m, n,* V, R^{b} and t have the values as defined in claim 1.

4. A compound selected from the group consisting of:
- compounds of formula: 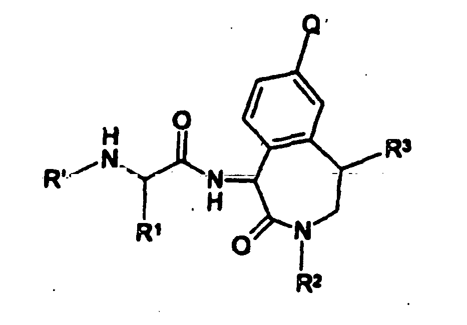
wherein R', R¹, R², R³ and Q have the values defined in the table below: 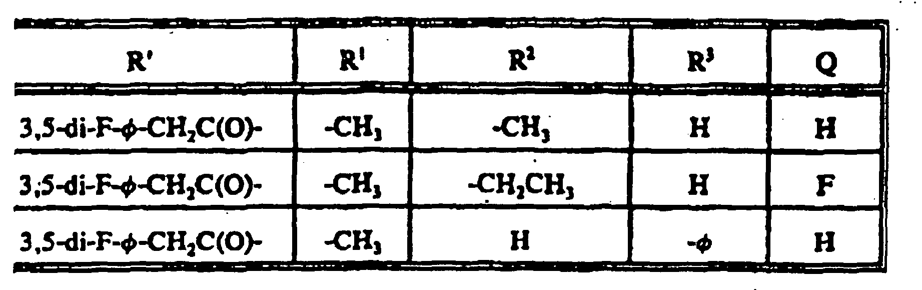
- compounds of formula: 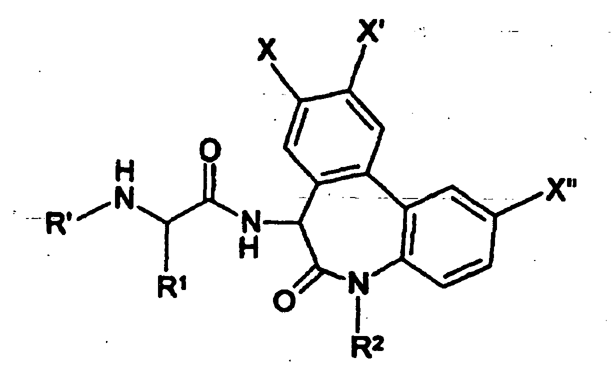
wherein R' R¹, R², X, X' and X" have the values defined in the table below: 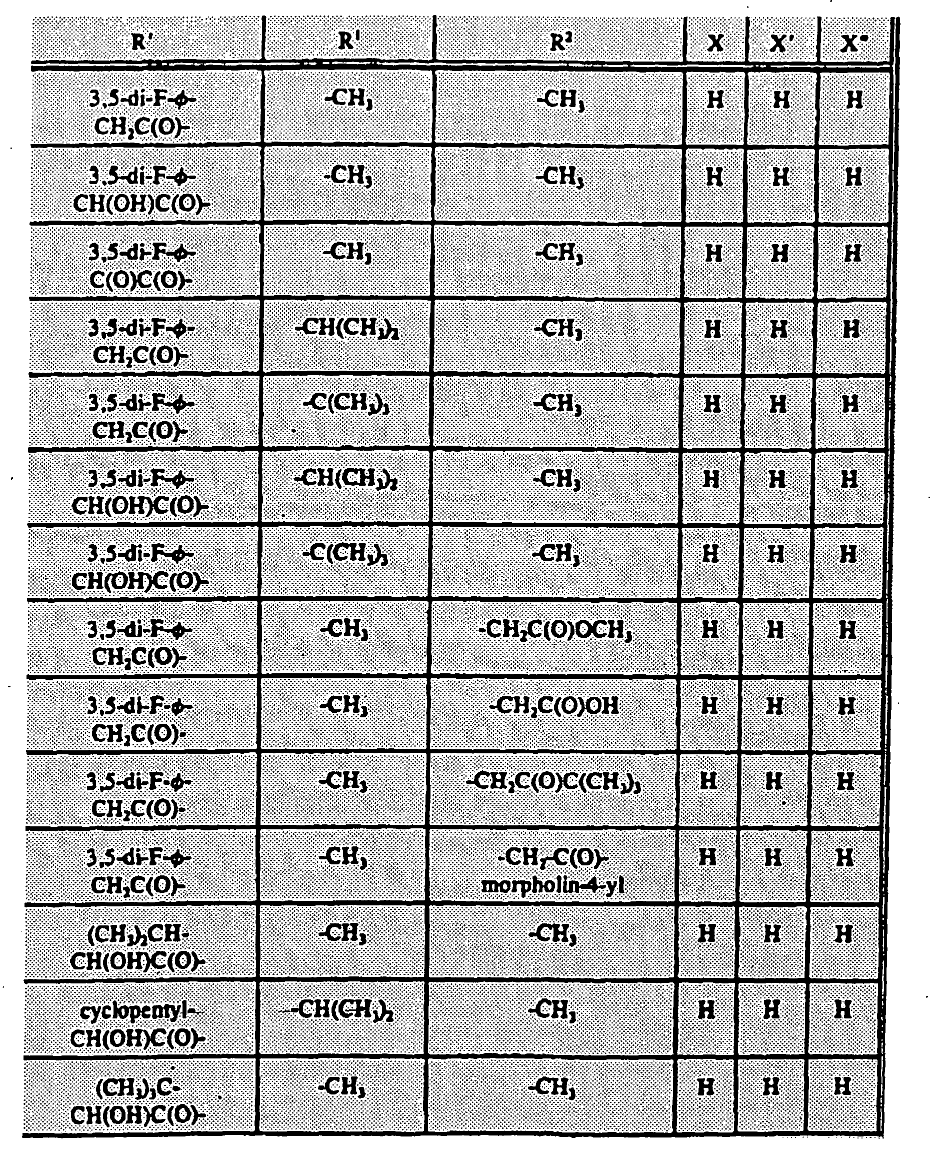 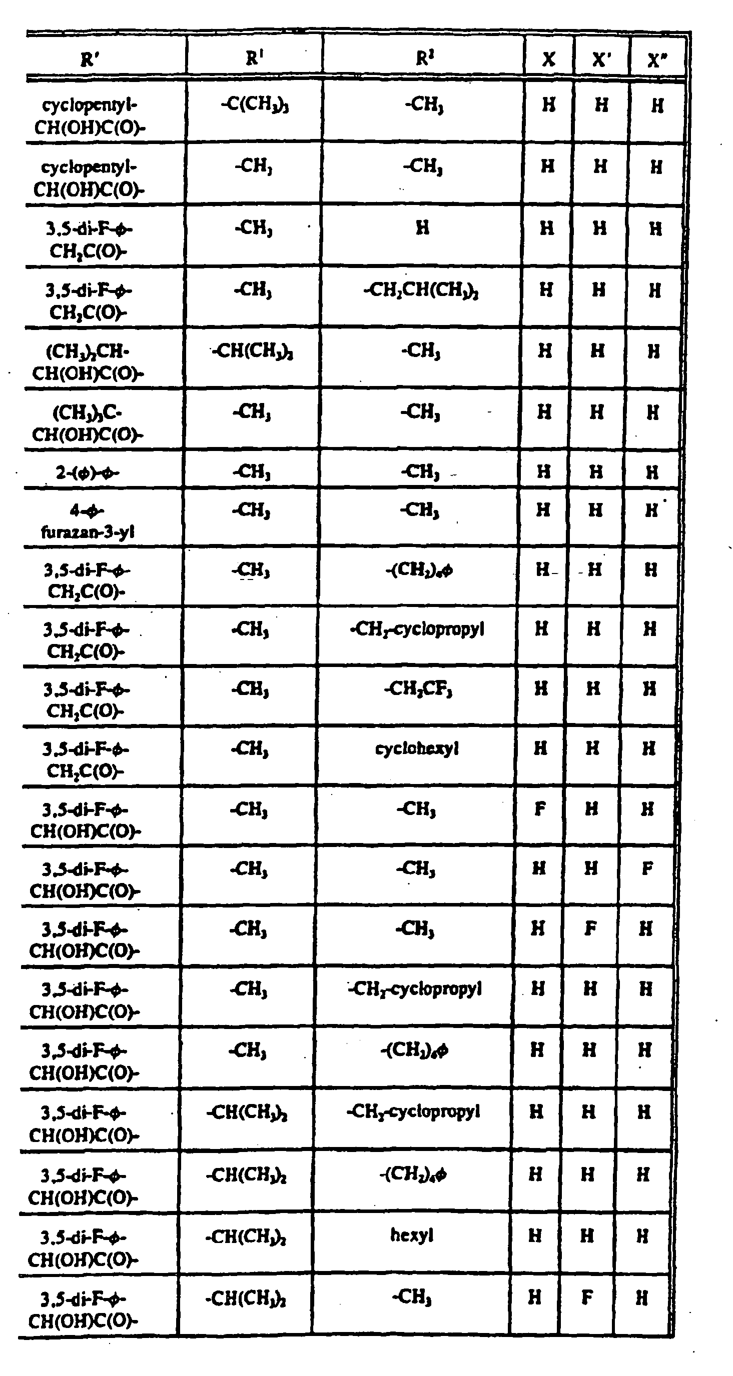 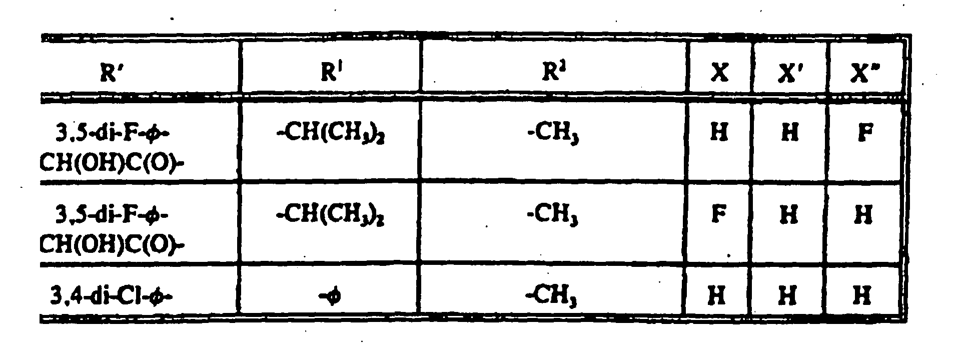
- compounds of formula: 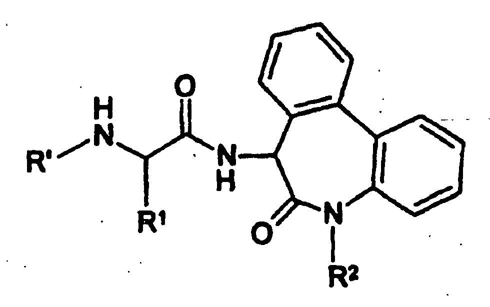
wherein R', R¹ and R² have the values defined in the table below: 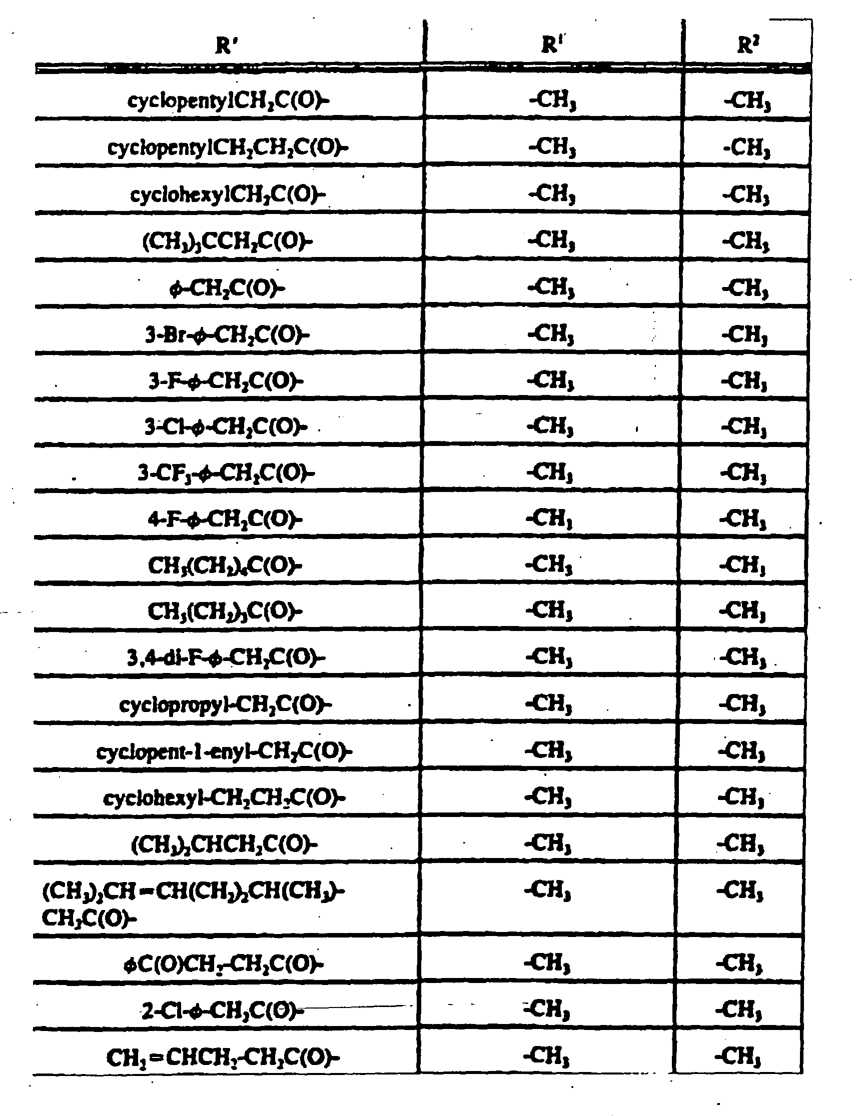 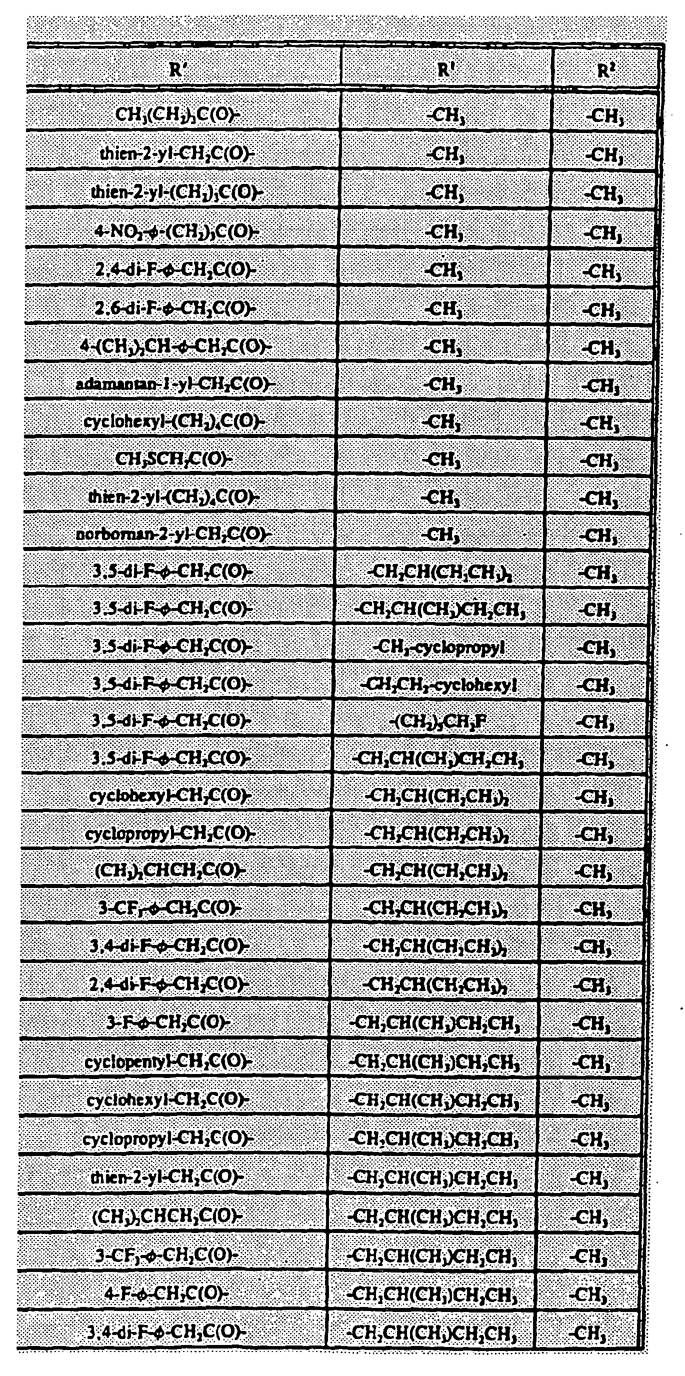 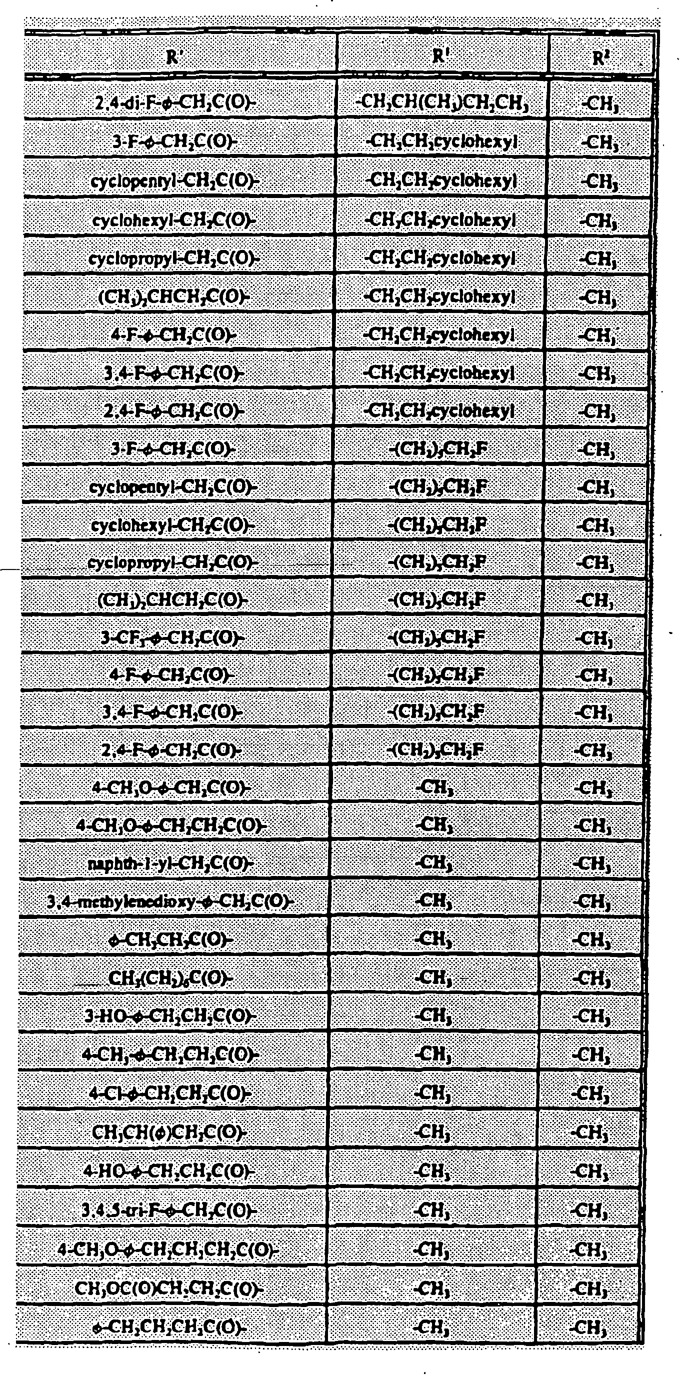 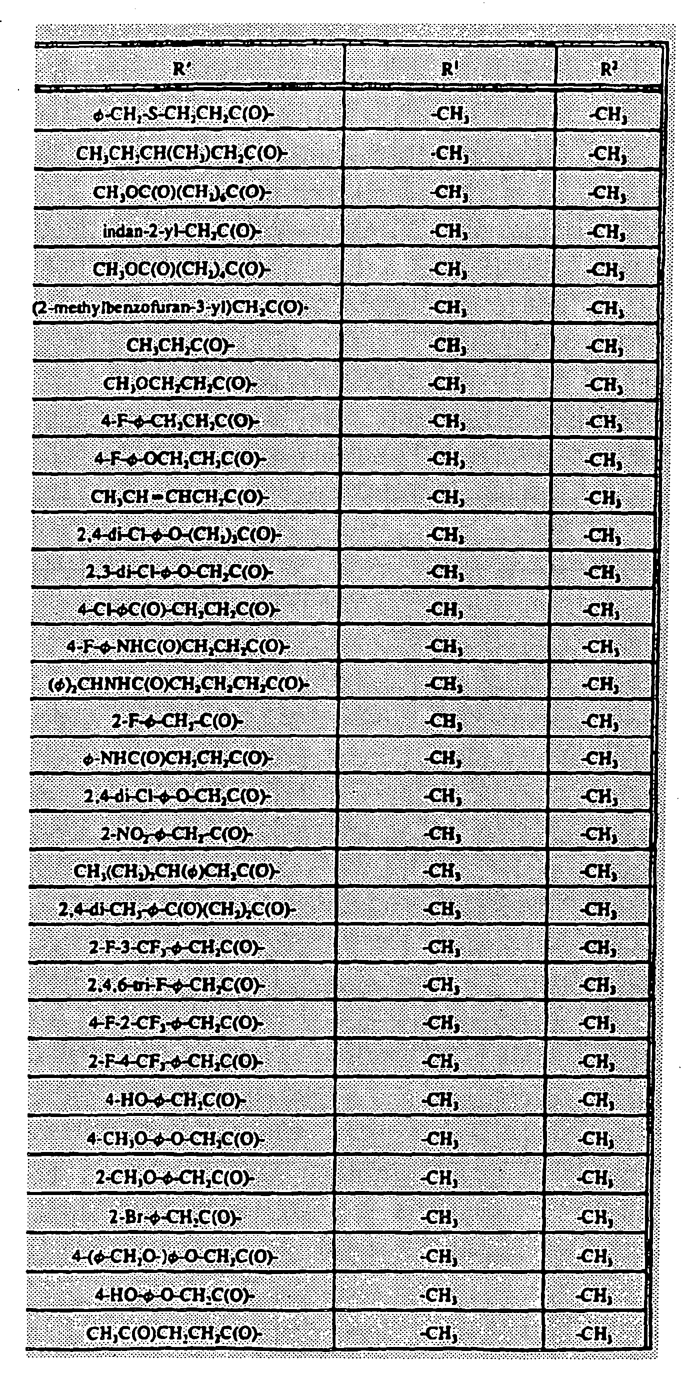 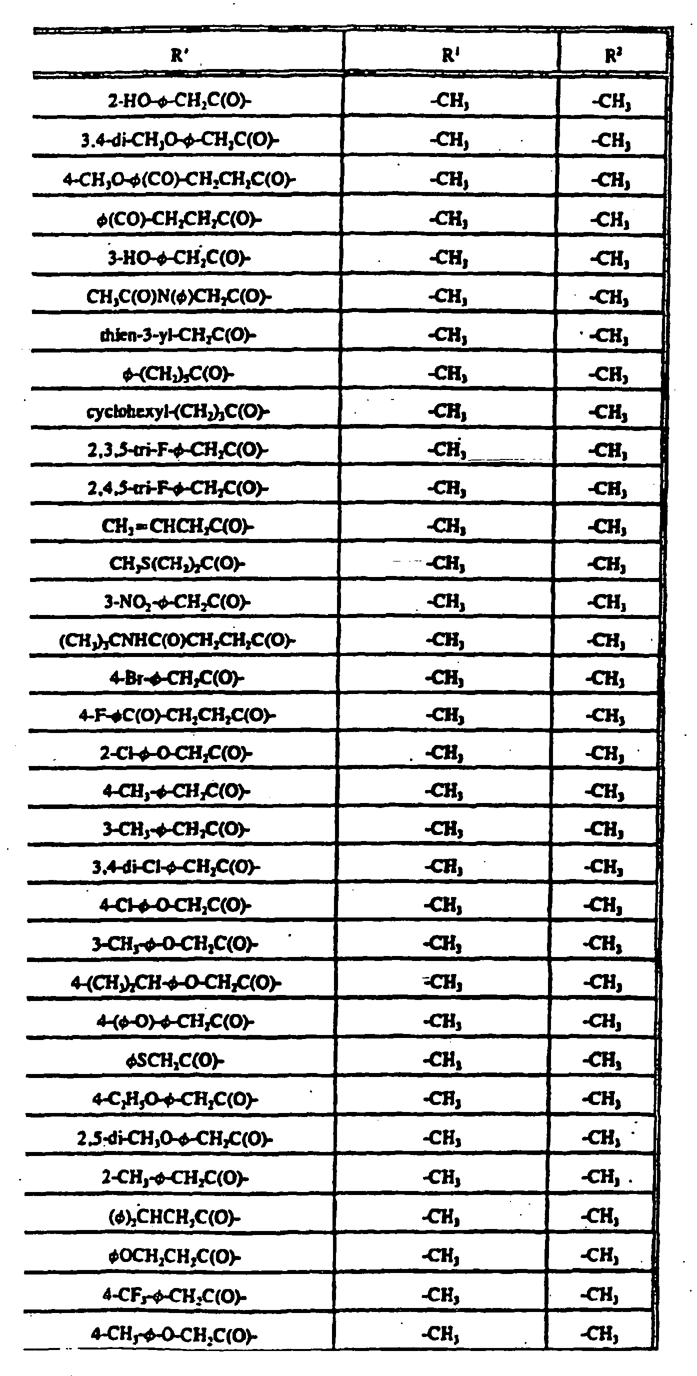 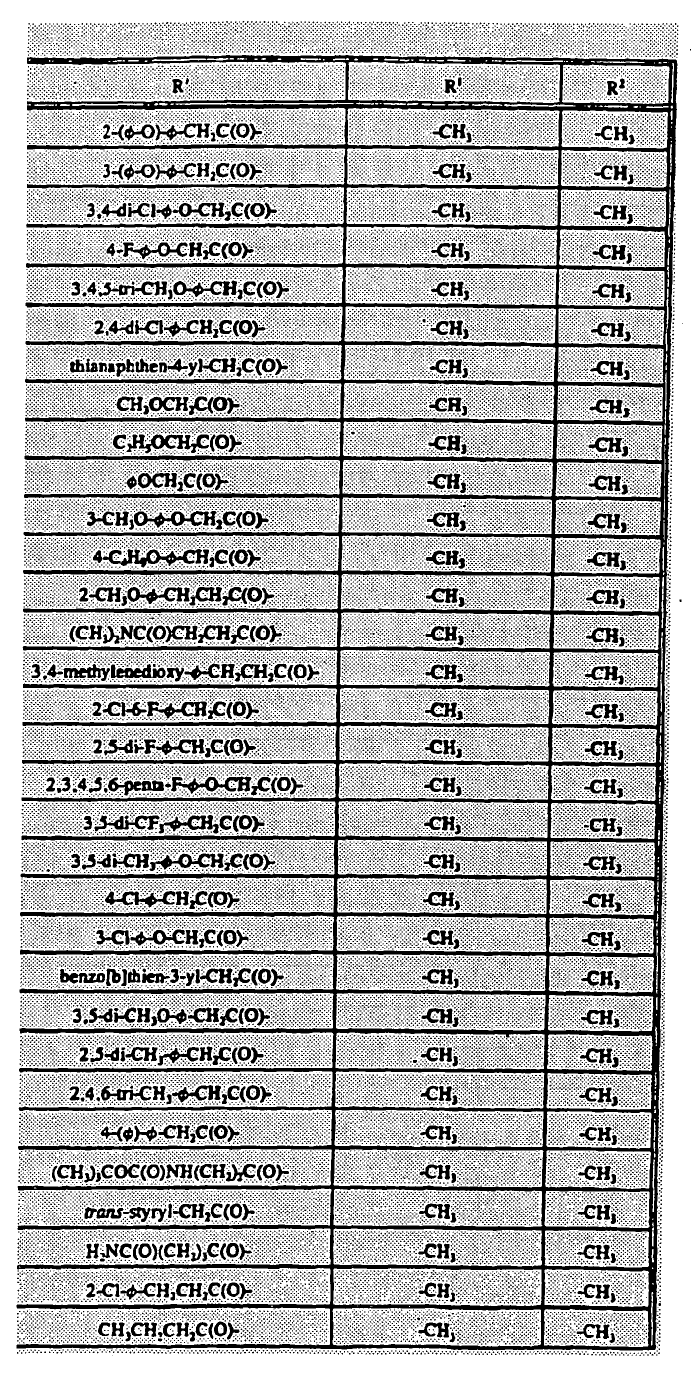 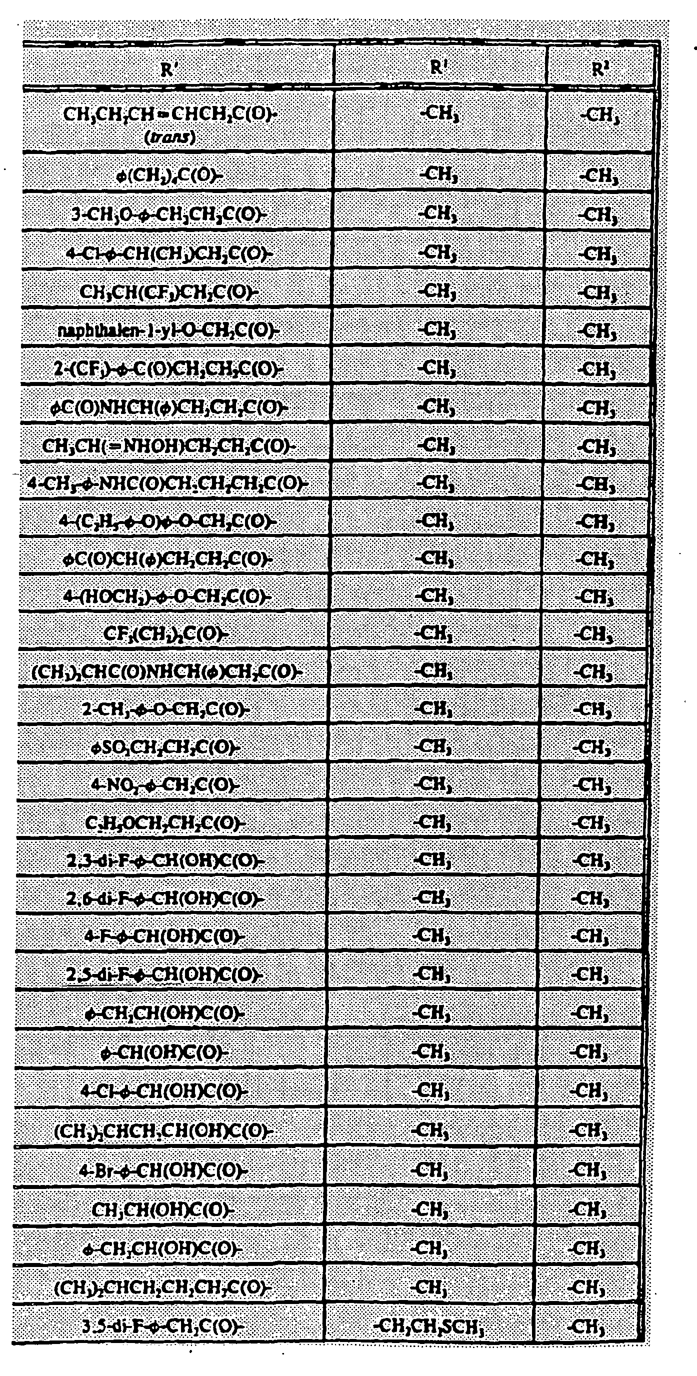 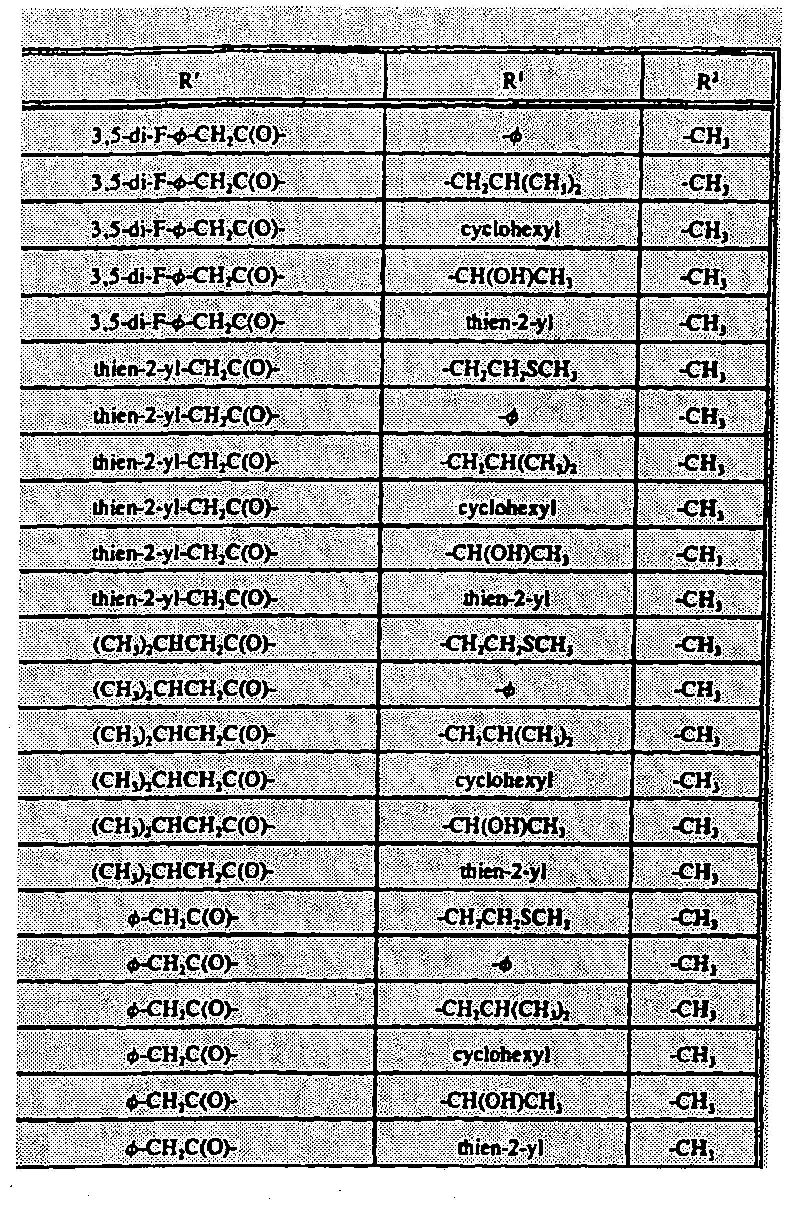
- compounds of formula: 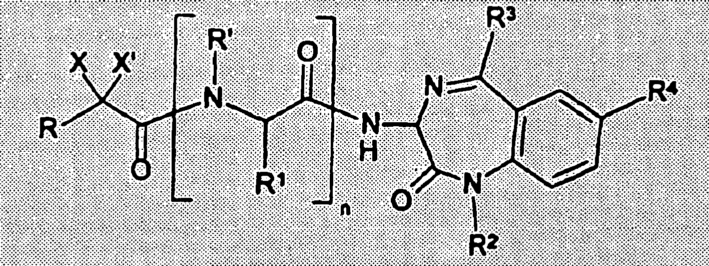
wherein R, R', R¹ to R⁴, X, X' and n have the values defined in the table below: 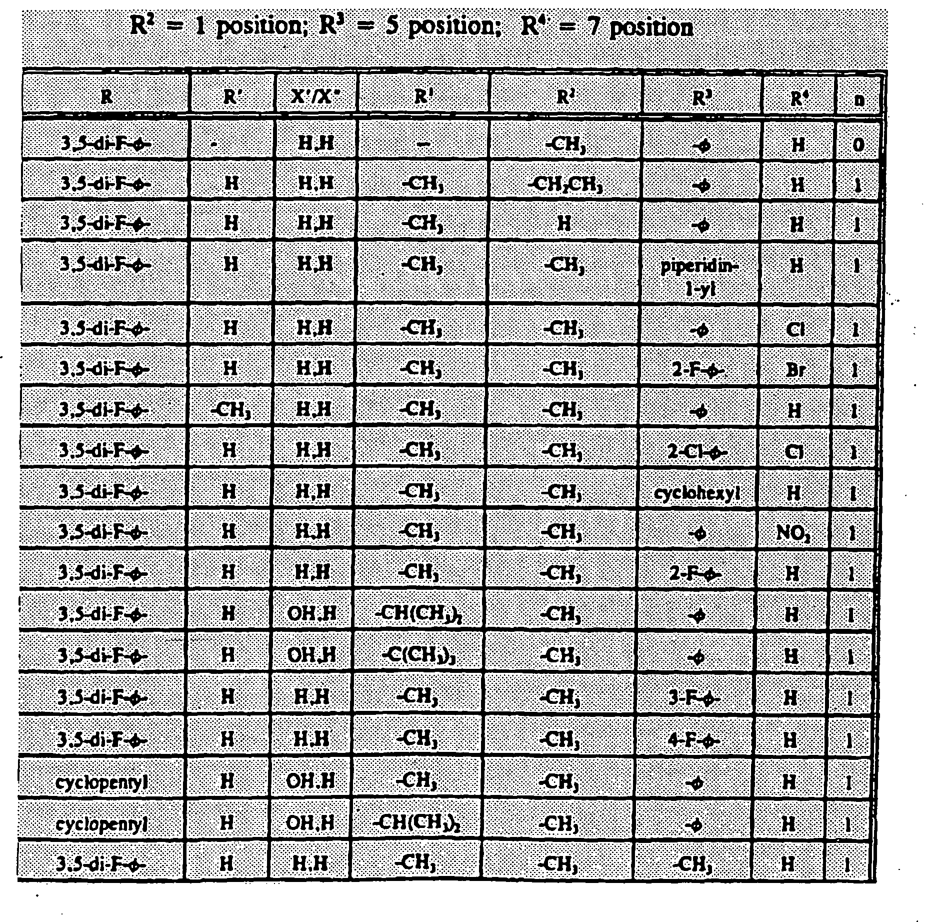 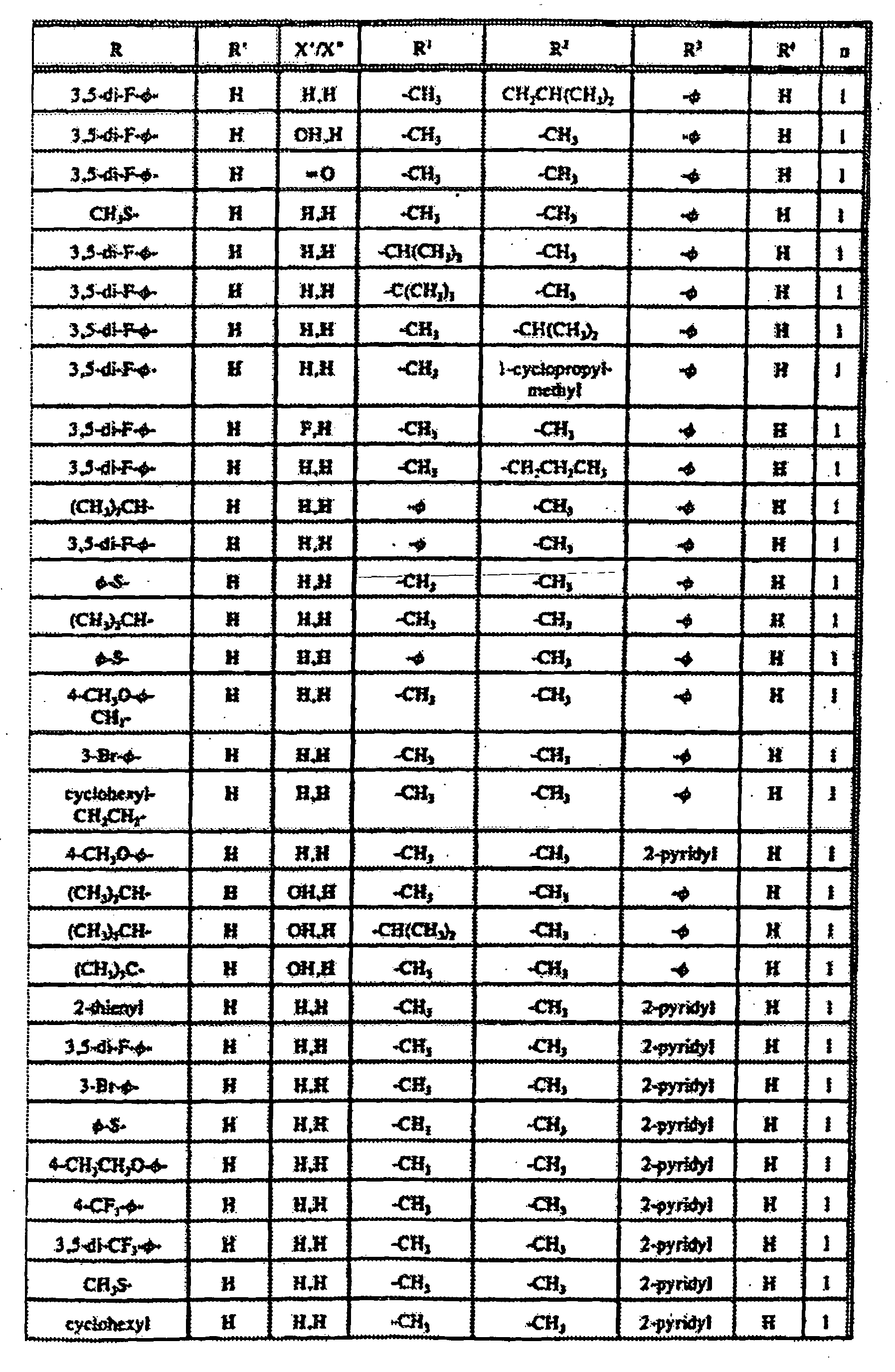 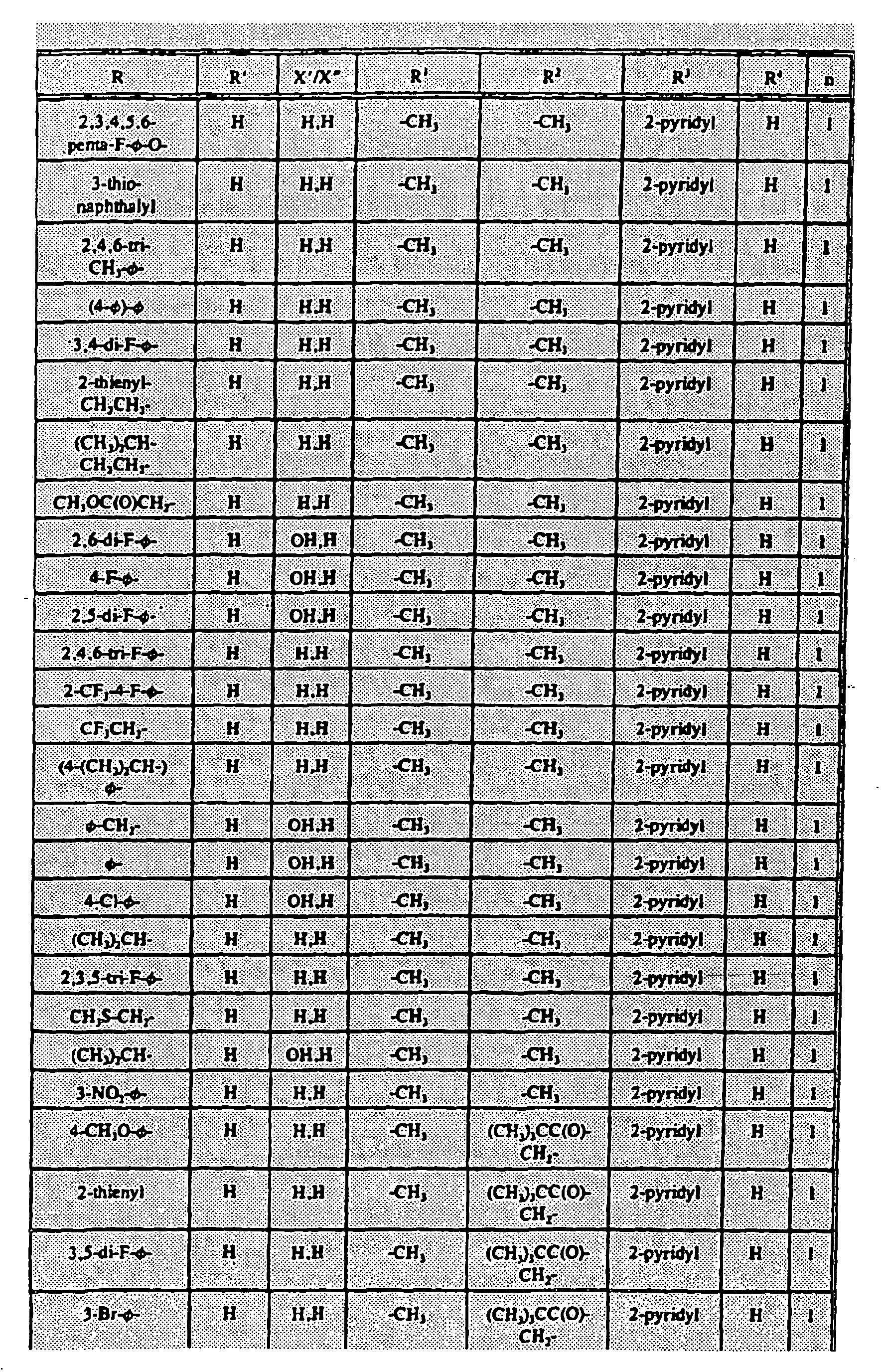 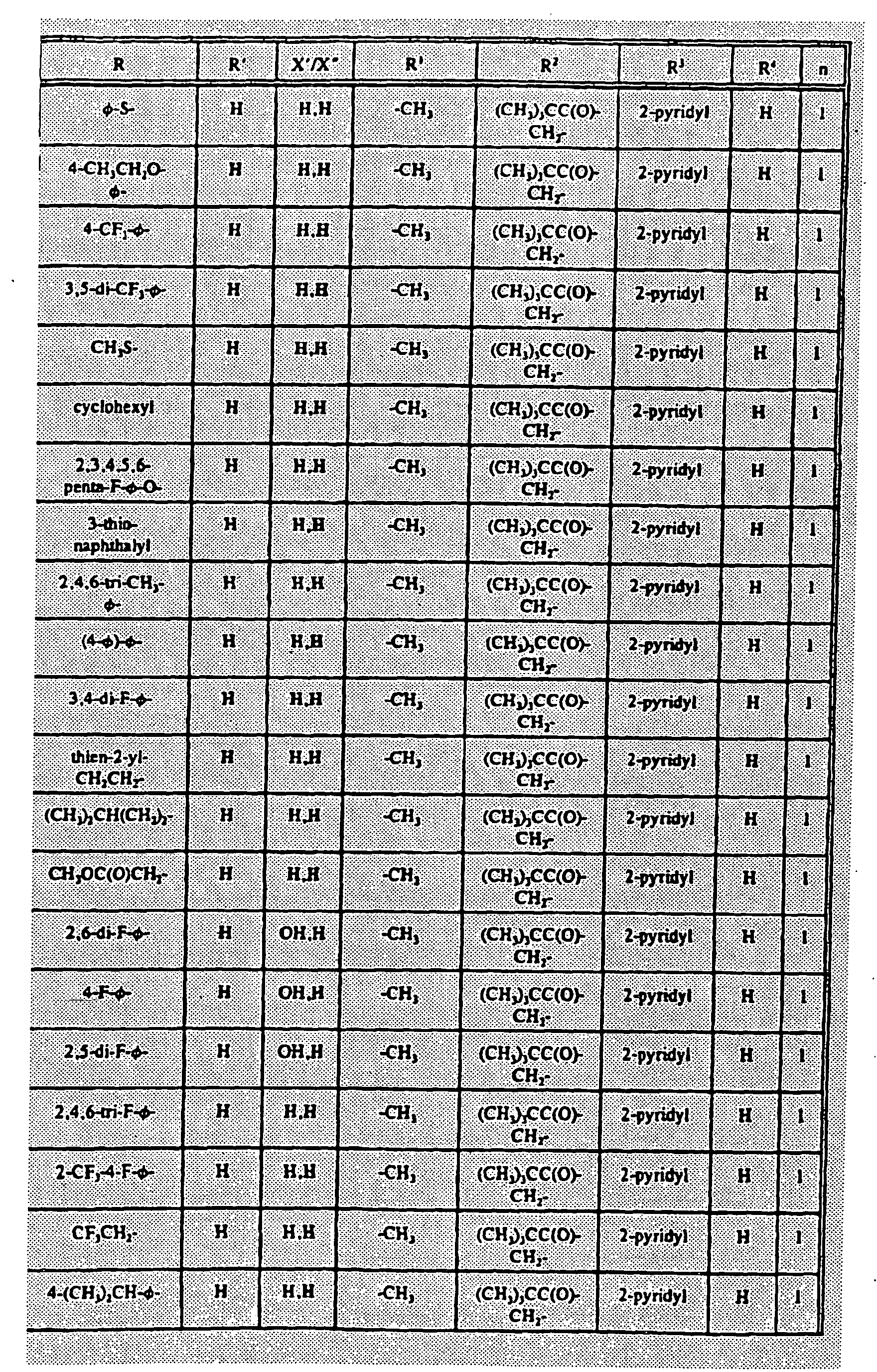 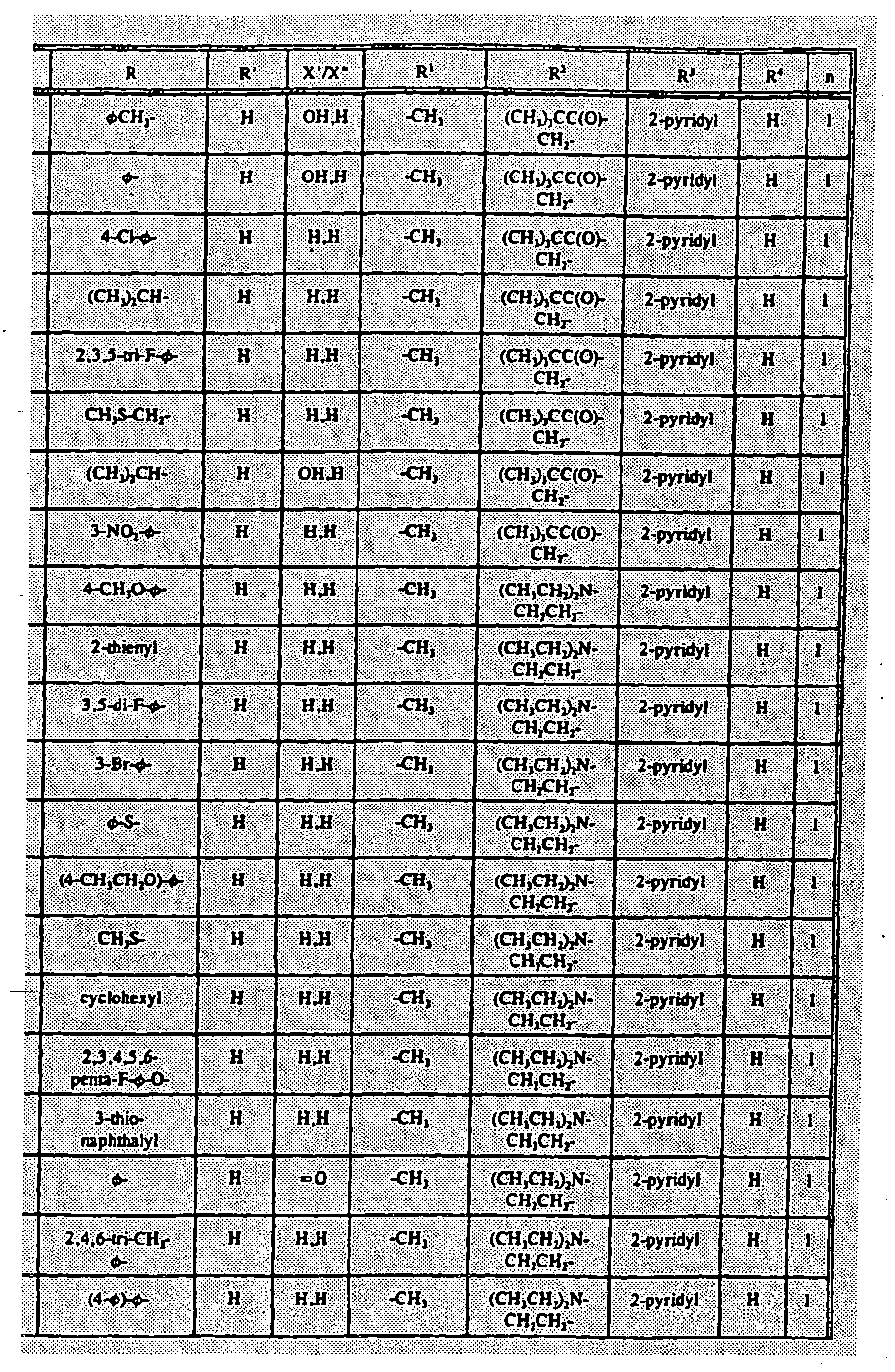 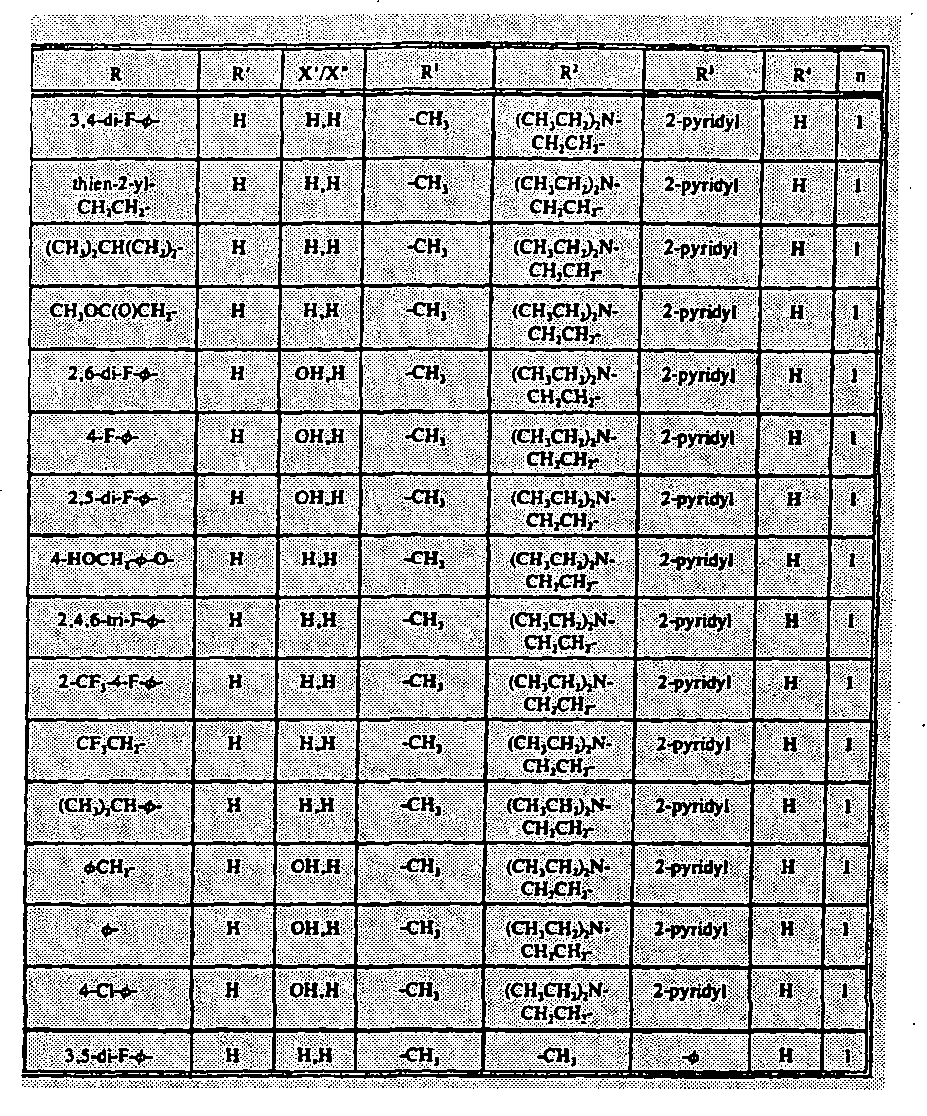
- compounds of formula: 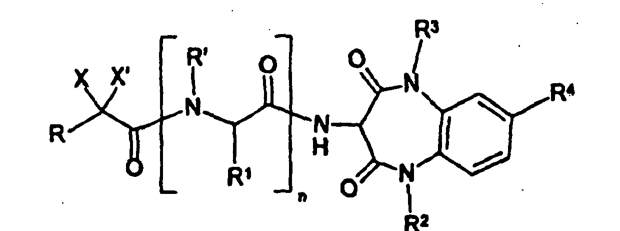
wherein R, X, X', R', R¹ to R⁴ and n have the values defined in the table below: 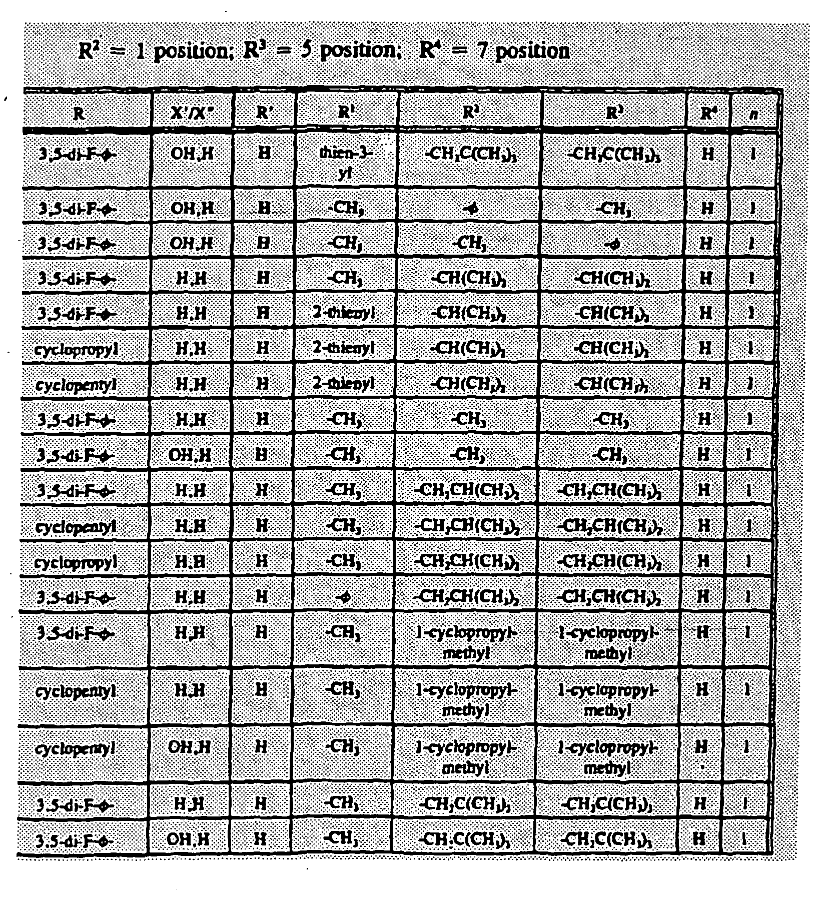 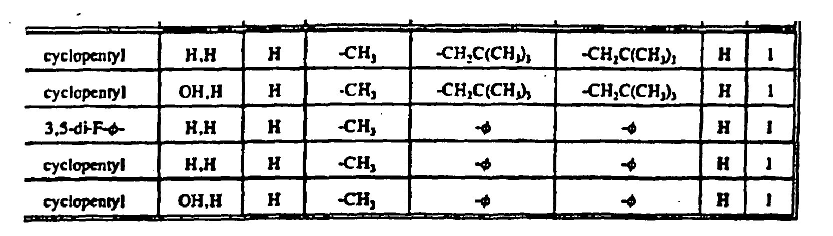
- and compounds of formula: 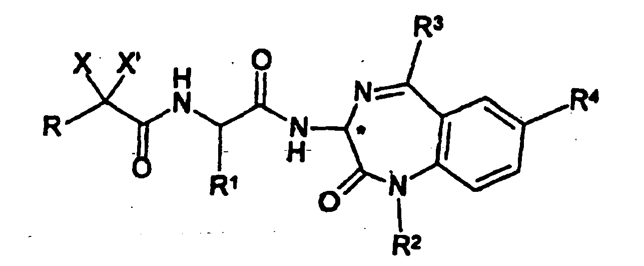
wherein R, R¹ to R⁴, X and X' have the values defined in the table below:

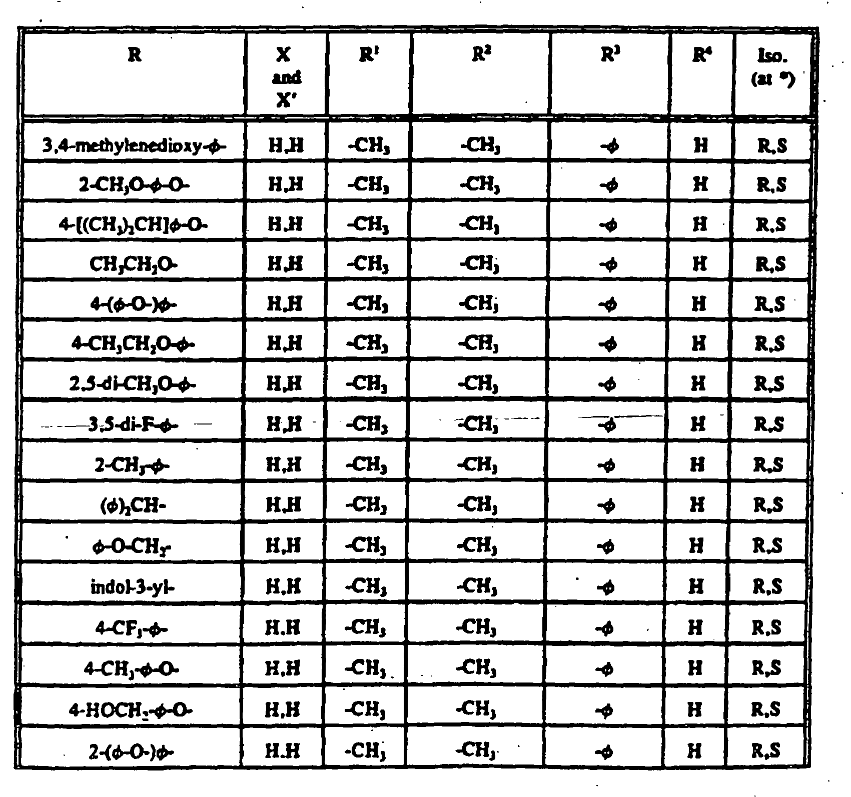

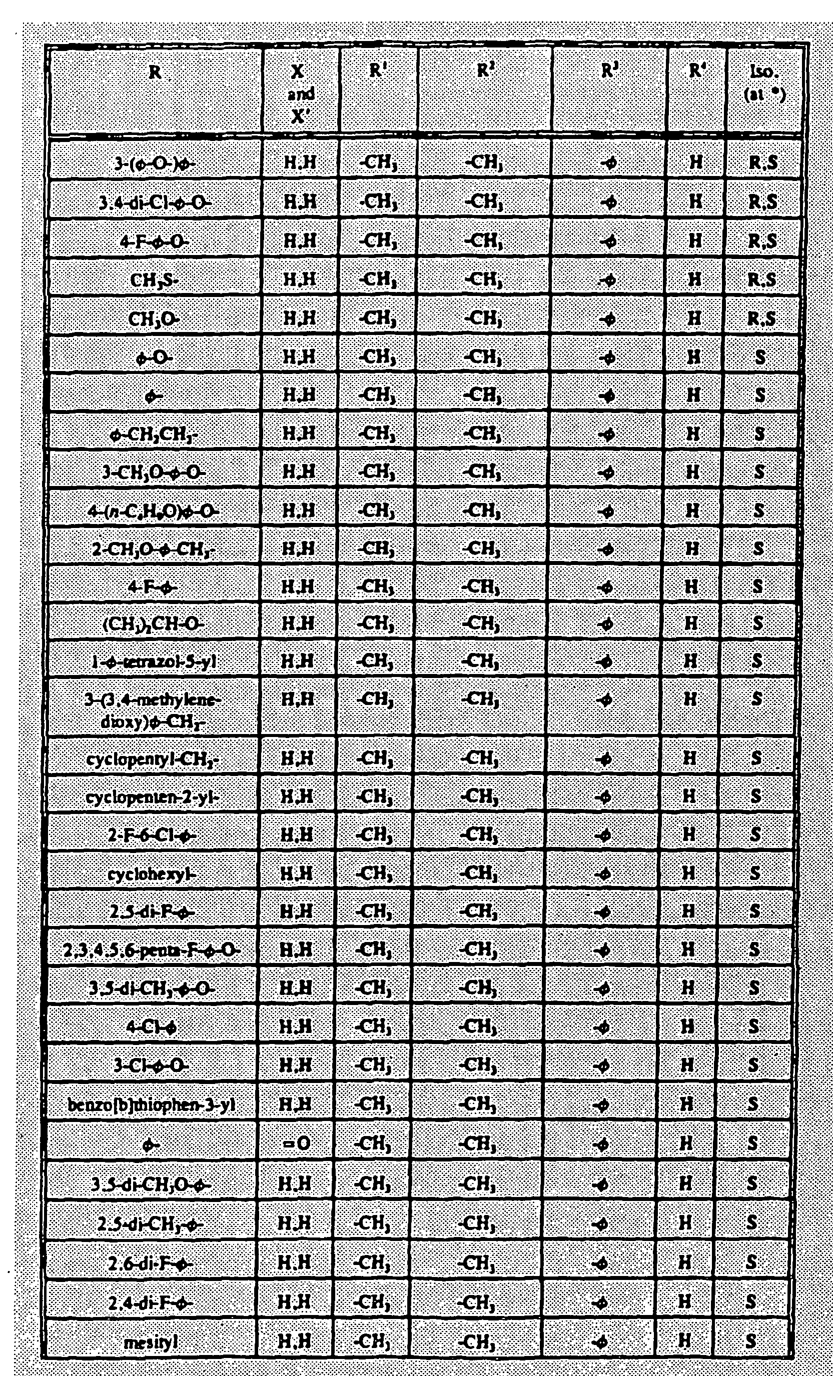

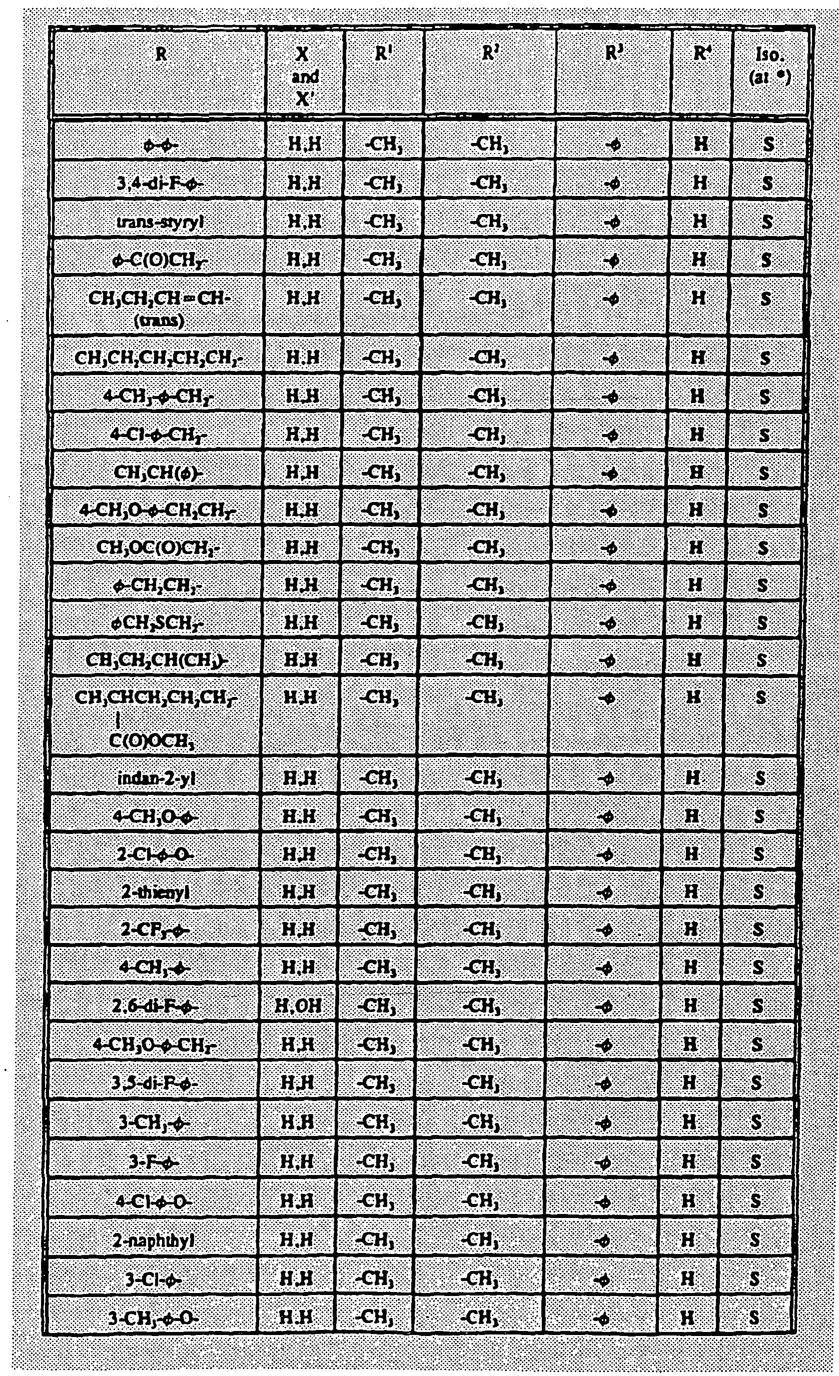

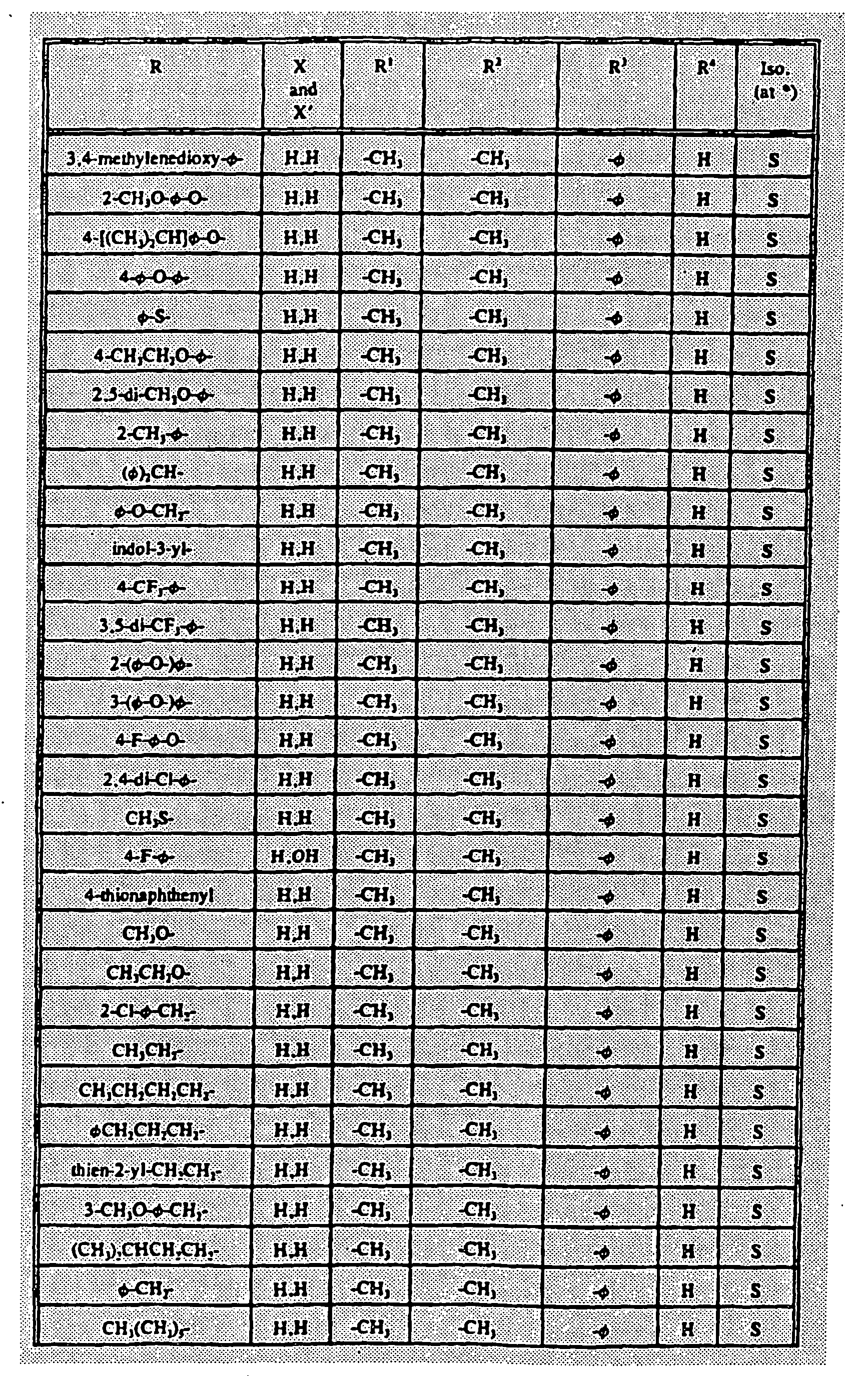

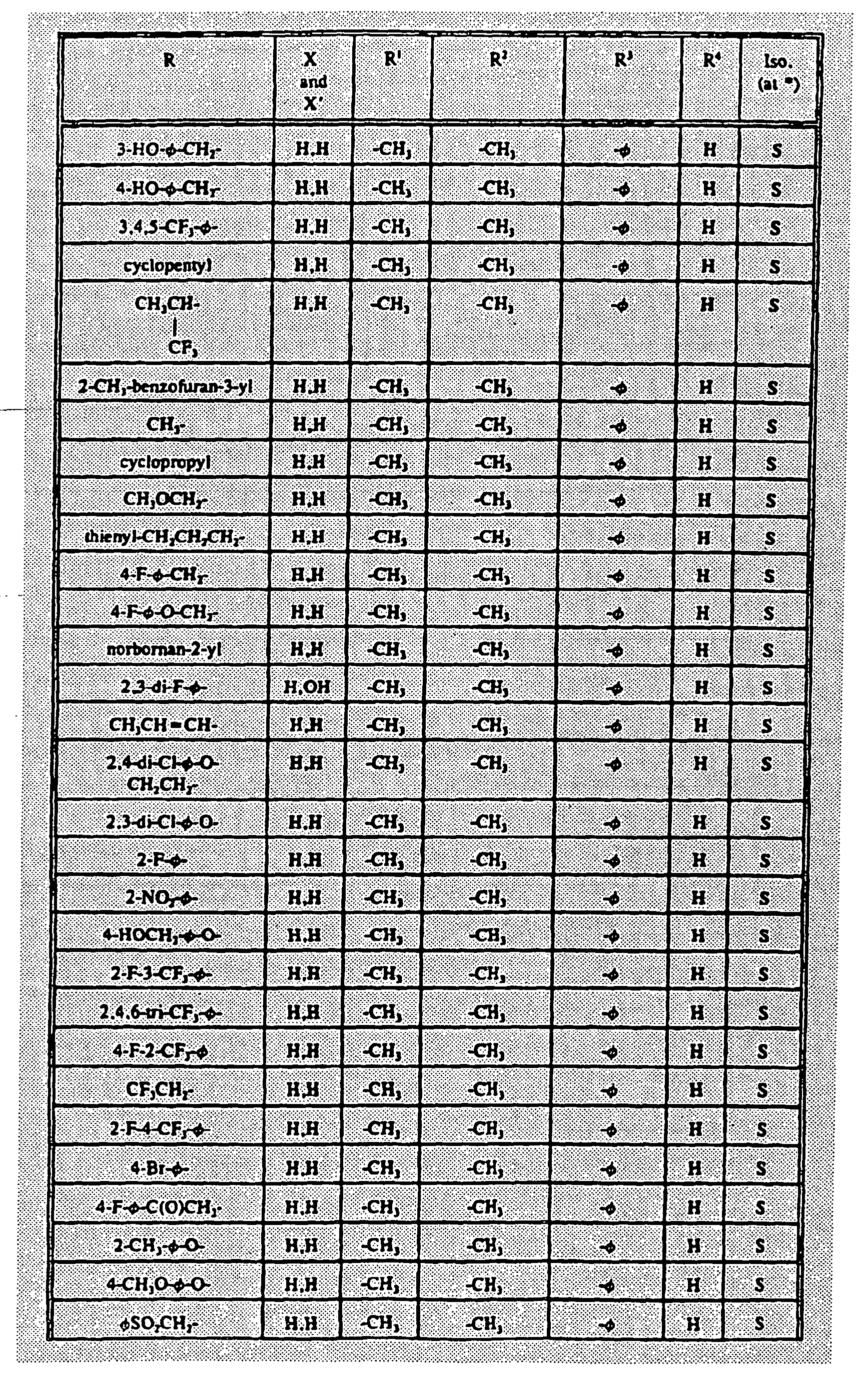

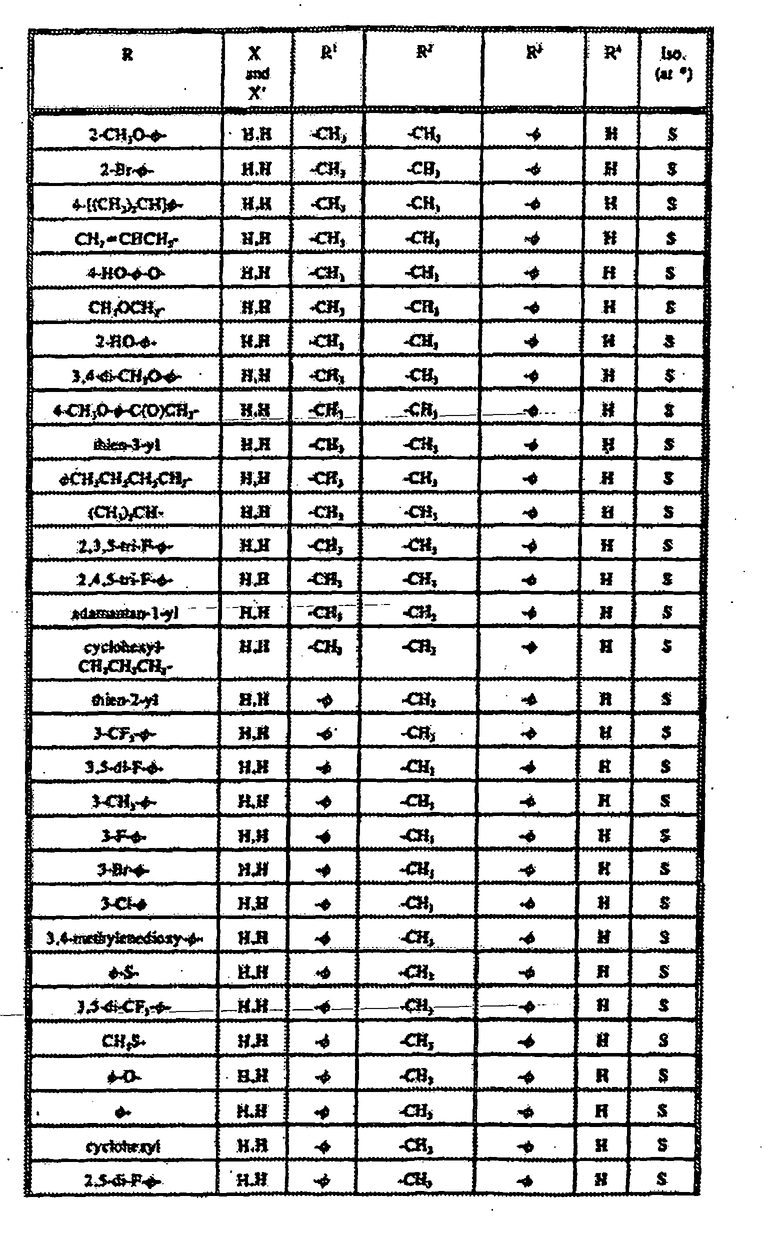

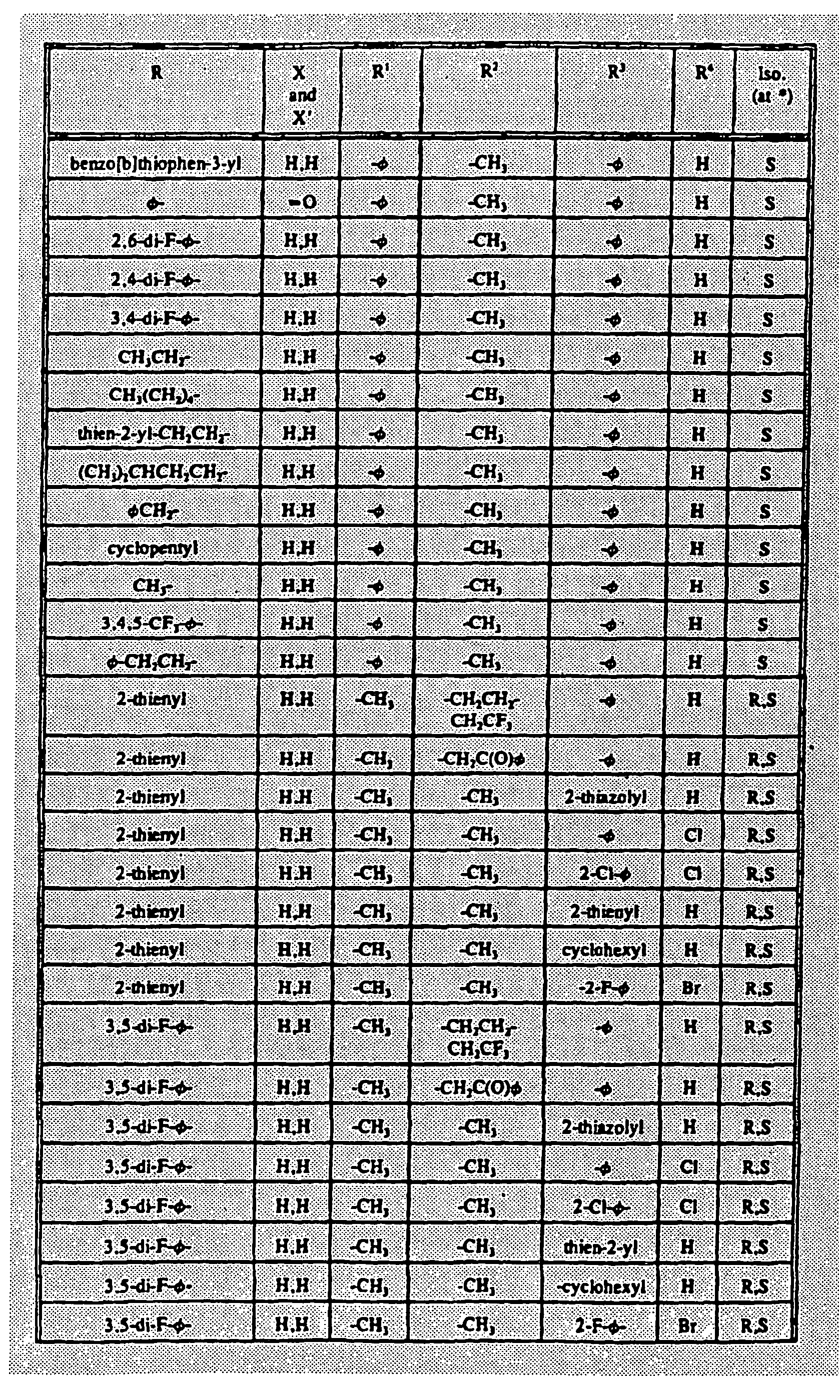

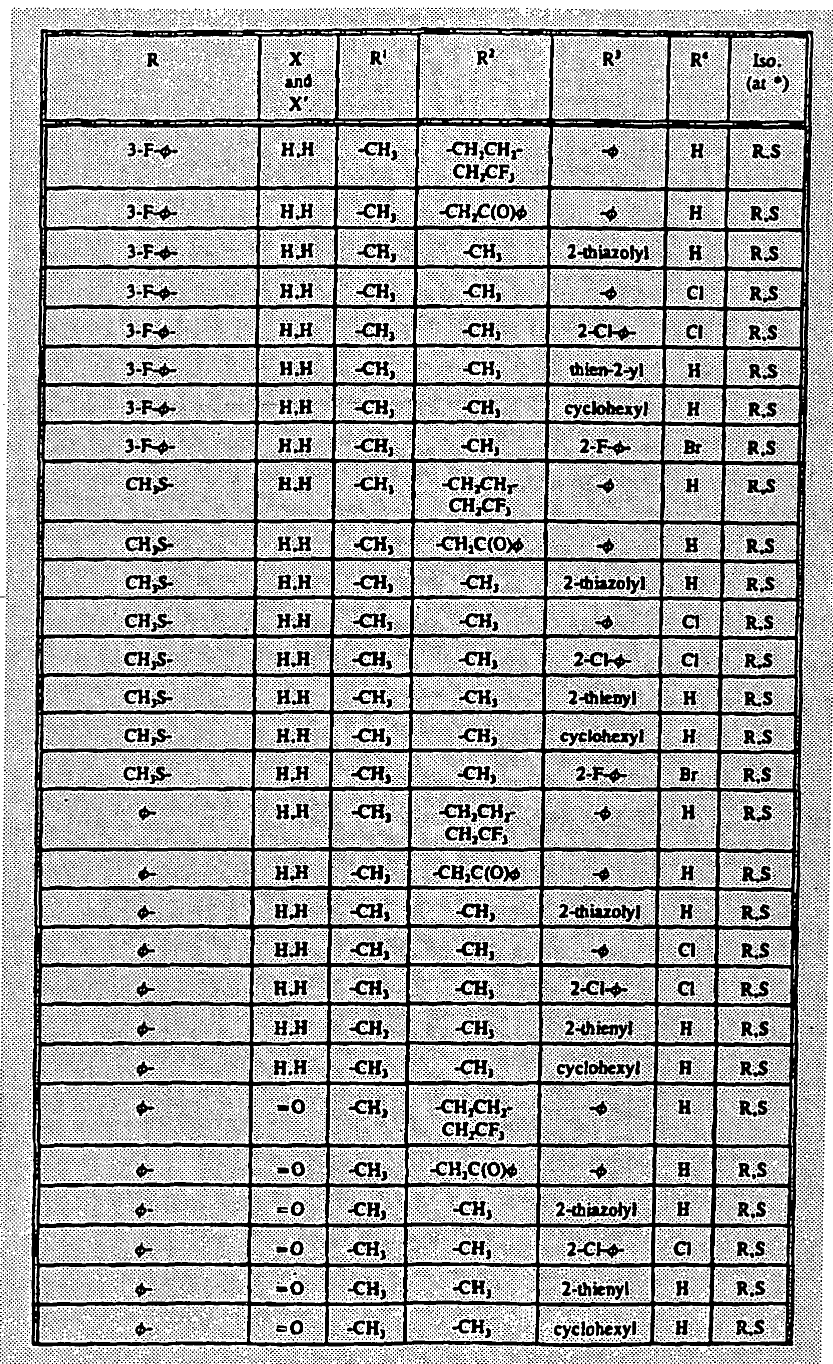

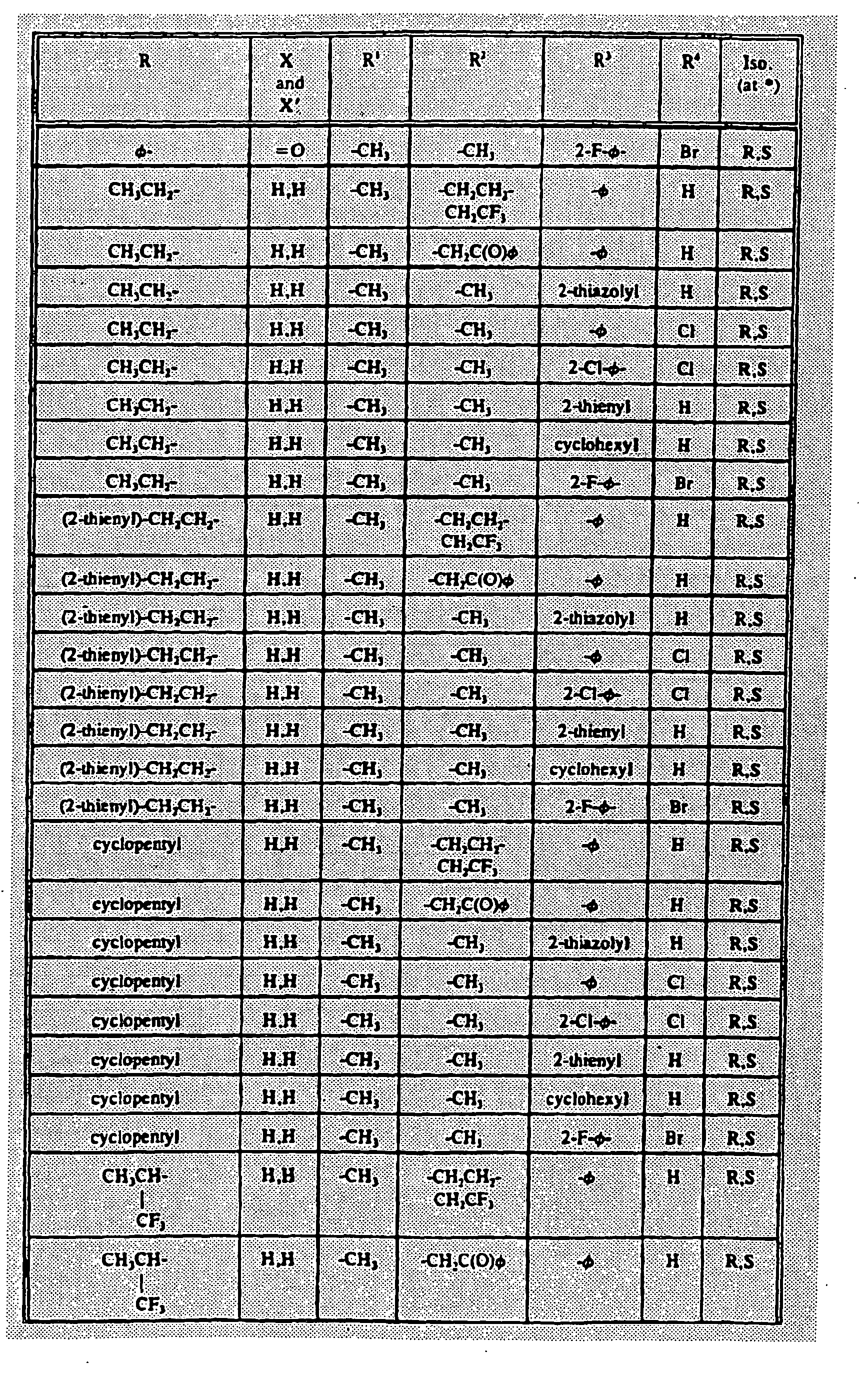

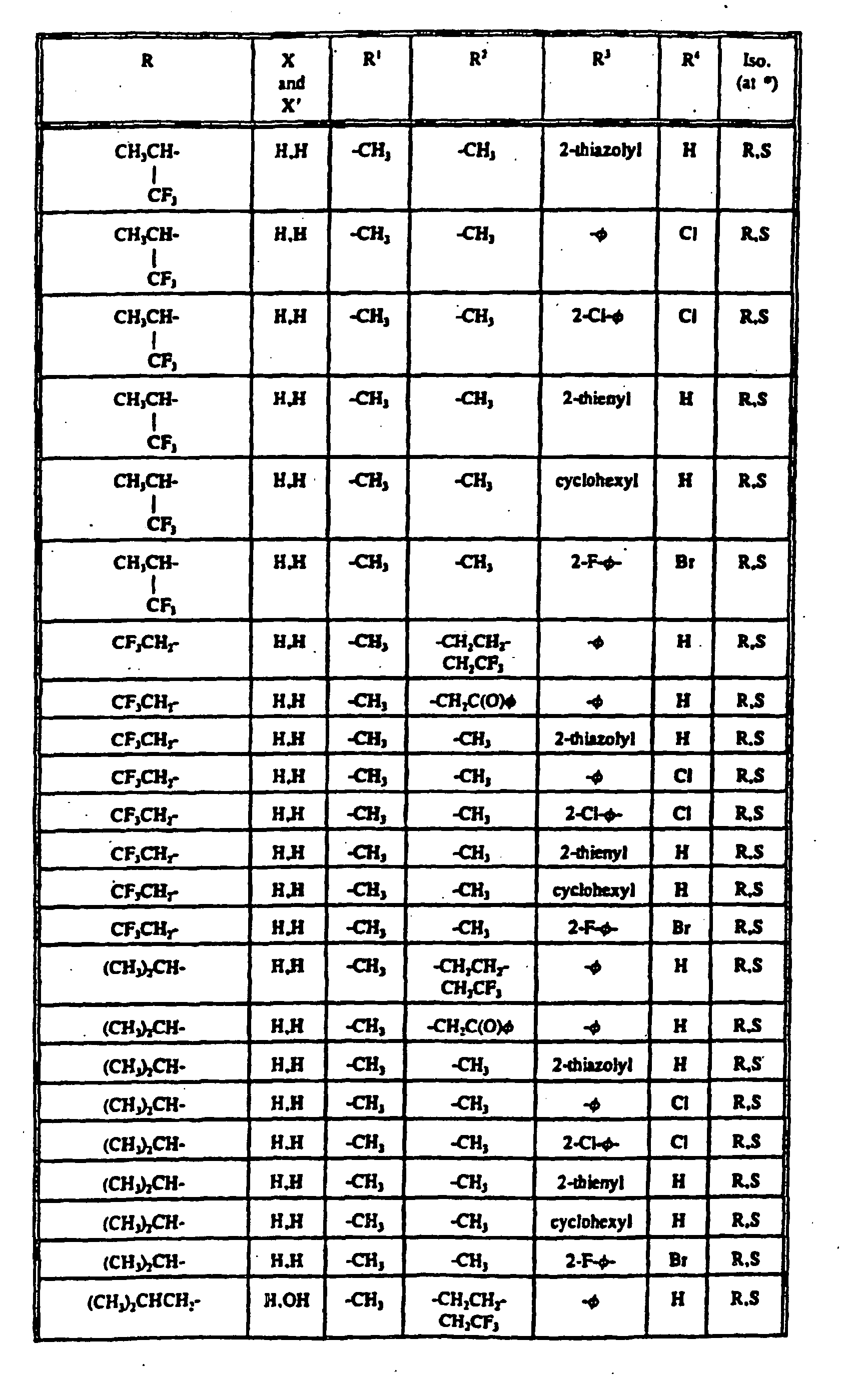

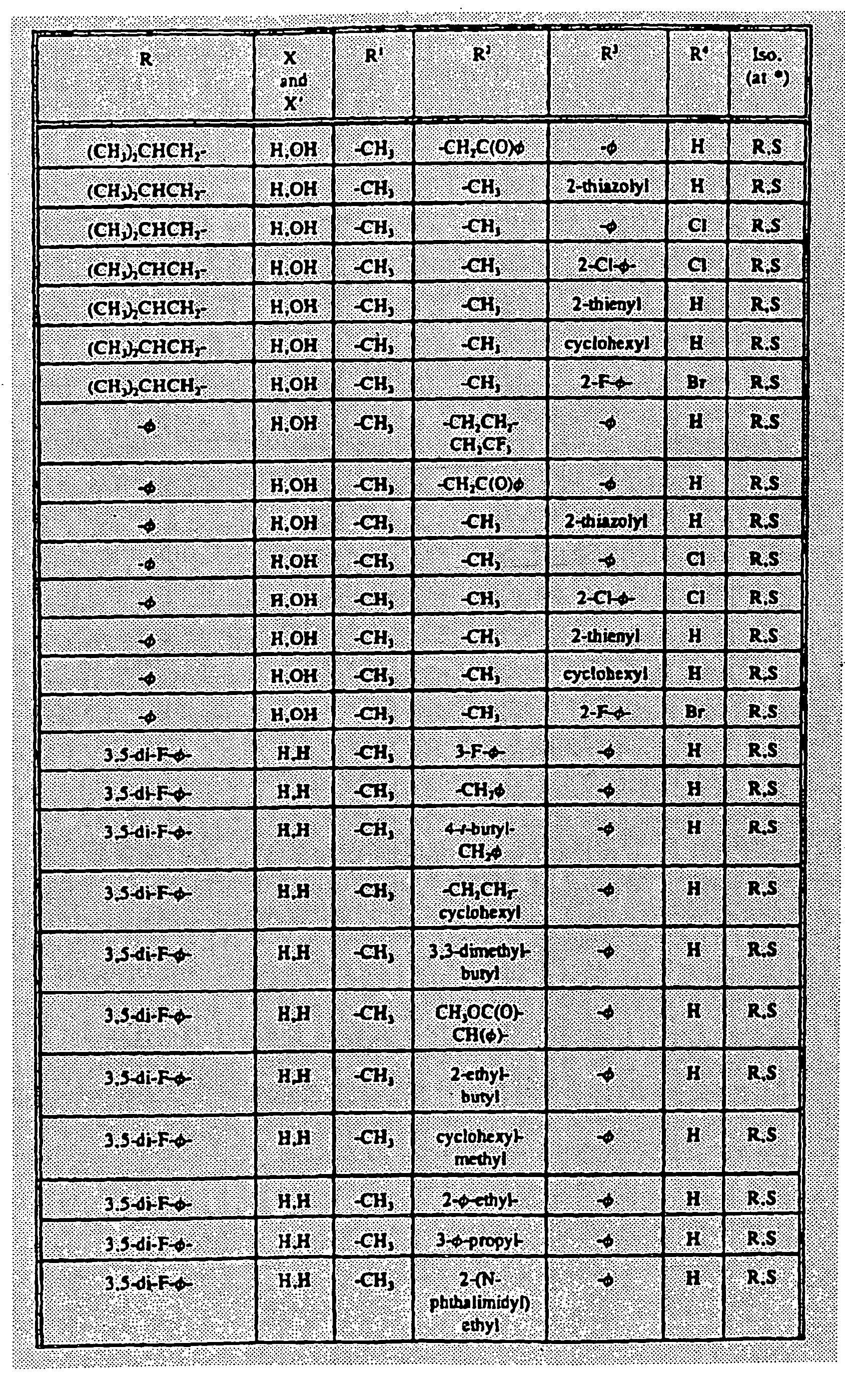

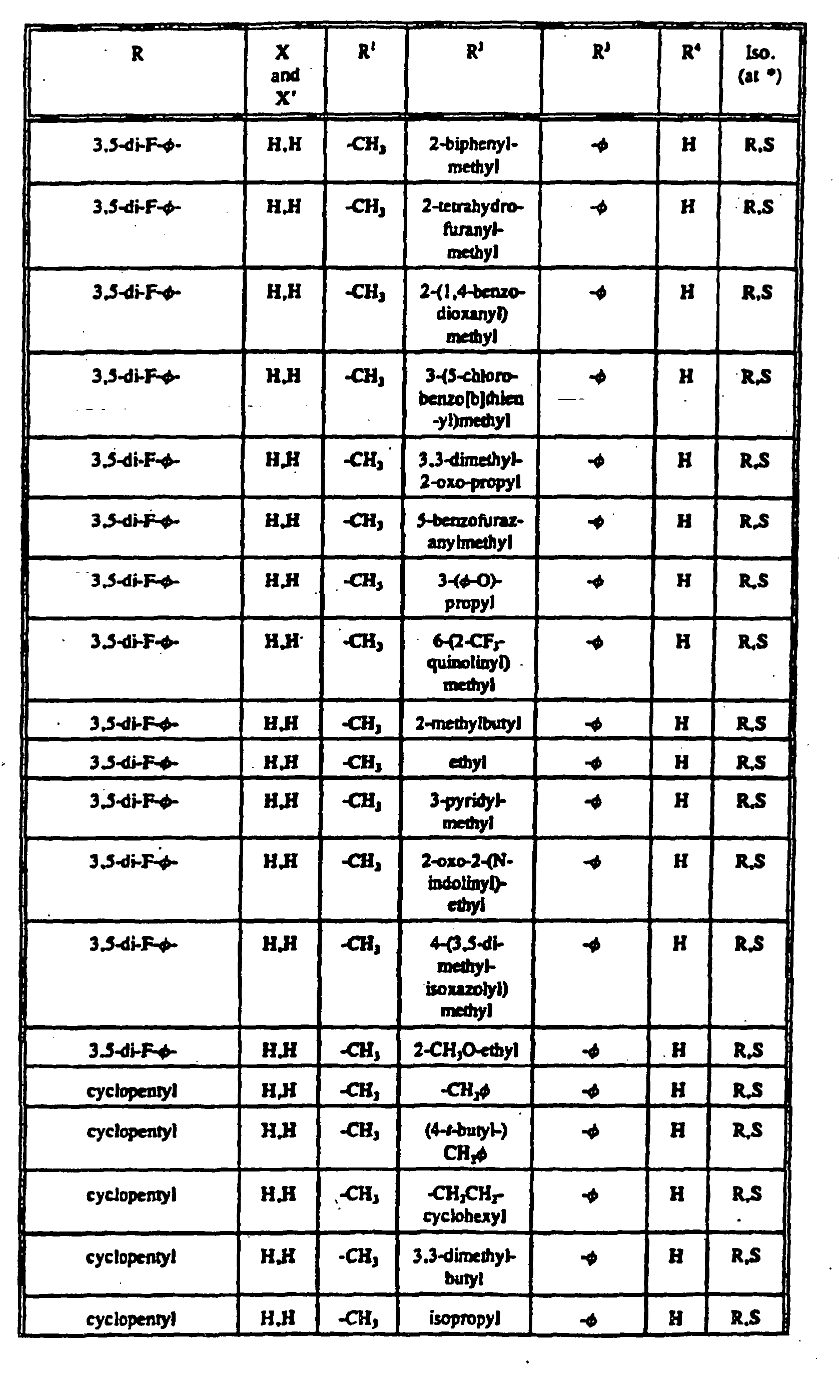

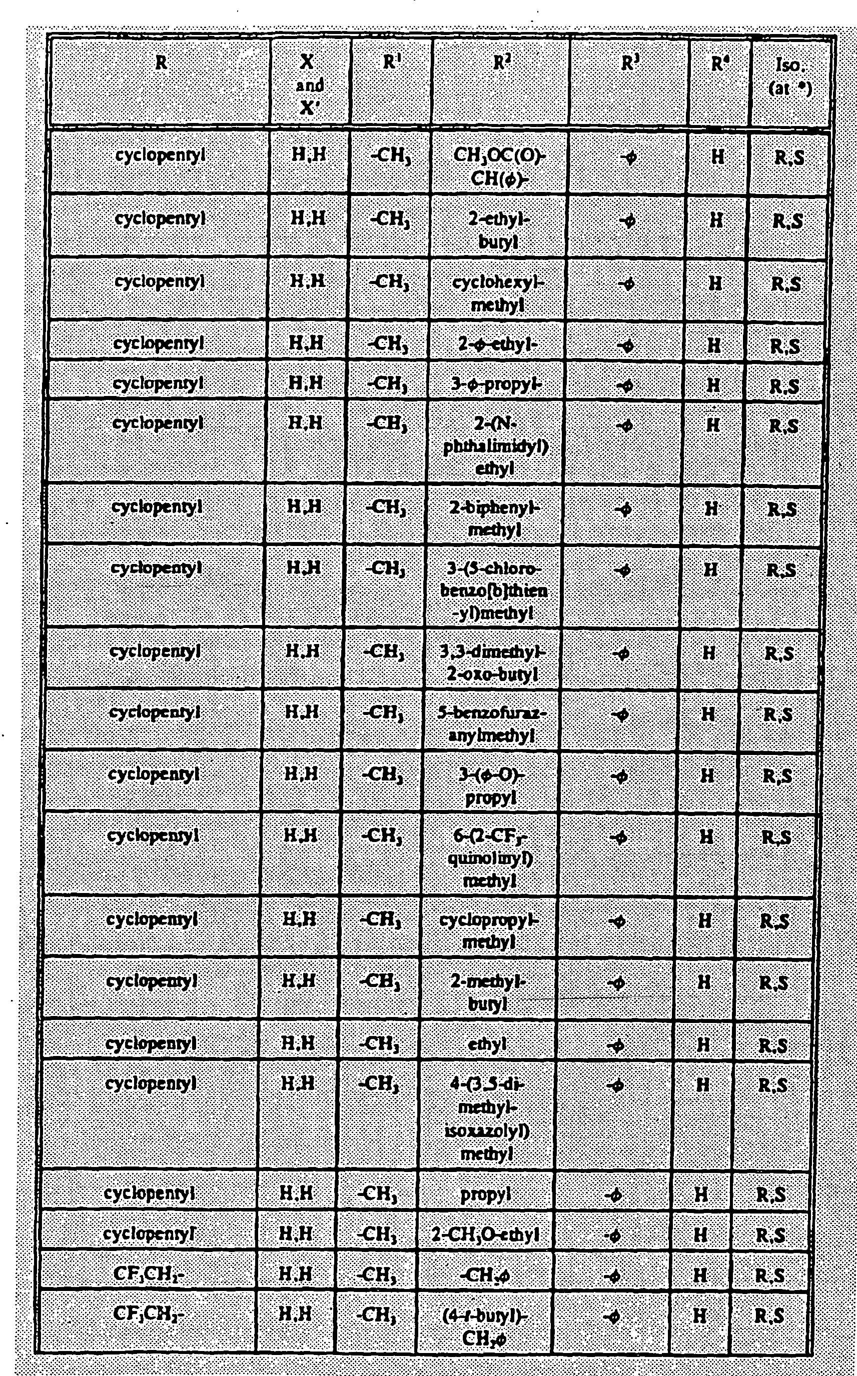

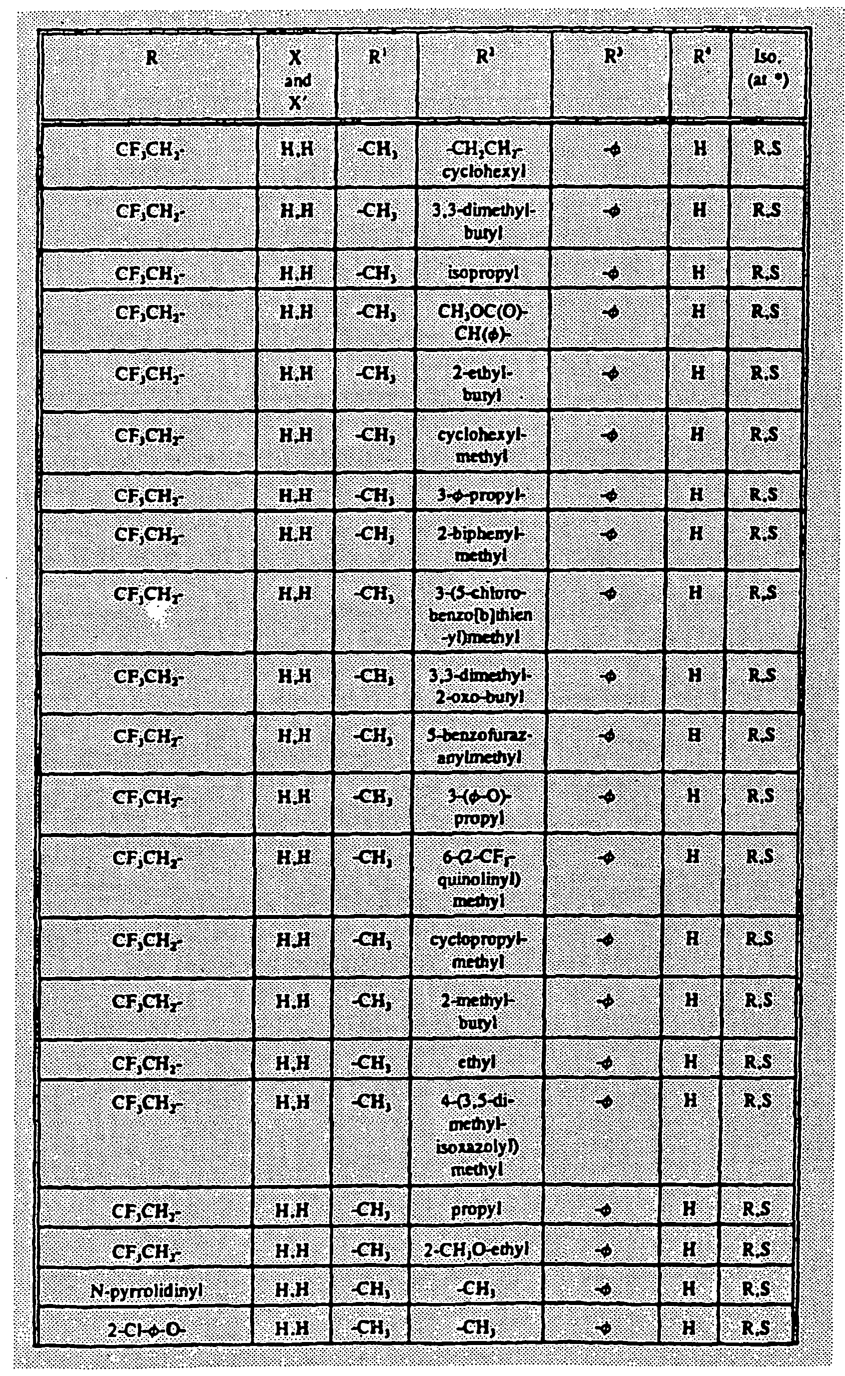

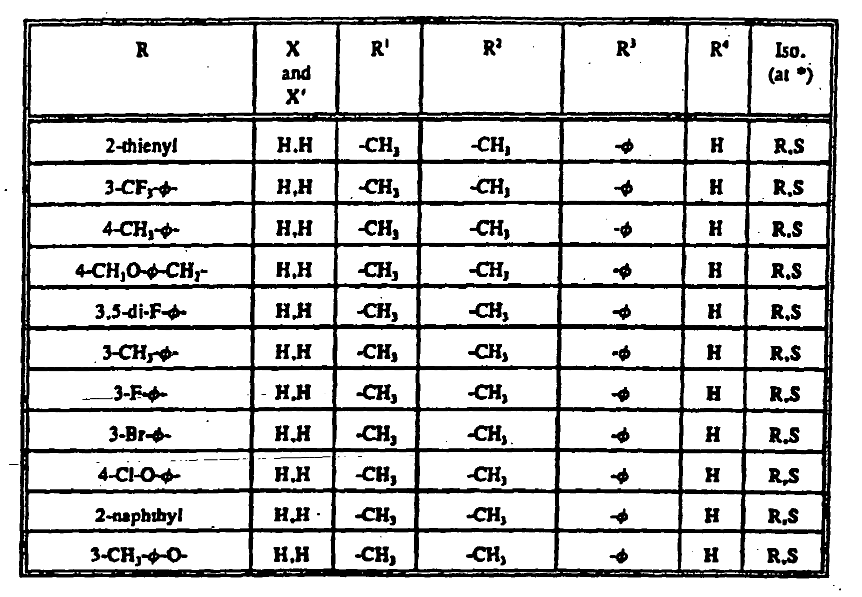

5. A pharmaceutical composition comprising a pharmaceutically inert carrier and a pharmaceutically effective amount of a compound according to claims 1 to 4.

6. A compound according to claims 1 to 4 for use in therapy.

7. A compound according to claims 1 to 4 for use in the treatment of Alzheimer's disease.

8. Use of a compound according to claims 1 to 4 for the manufacture of a medicament for the treatment of Alzheimer's disease.

## Patentansprüche

1. Verbindung der Formel 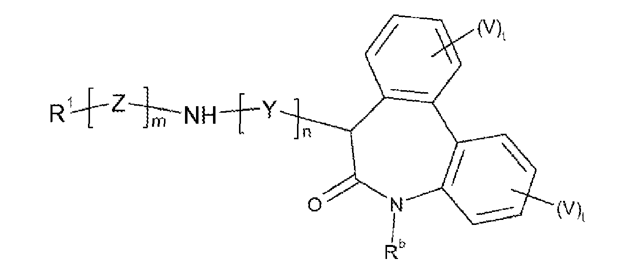
worin R¹ ausgewählt ist aus der Gruppe, die besteht aus Alkyl, Alkenyl, Alkinyl, Cycloalkyl, Cycloalkenyl, Aryl, Heteroaryl und Heterocyclyl,
Y für die folgende Formel steht 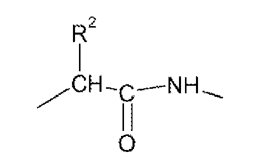
worin R² ausgewählt ist aus der Gruppe, die besteht aus Methyl, Ethyl, n-Propyl, Isopropyl, n-Butyl, Isobutyl, sec-Butyl, tert-Butyl, -CH₂CH(CH₂CH₃)₂, 2-Methyl-n-butyl, 6-Fluor-n-hexyl, Phenyl, Benzyl, Cyclohexyl, Cyclopentyl, Cycloheptyl, Allyl, iso-But-2-enyl, 3-Methylpentyl, -CH₂-Cyclopropyl, -CH₂-Cyclohexyl, -CH₂CH₂-Cyclopropyl, -CH₂CH₂-Cyclohexyl, -CH₂-lndol-3-yl, p-(Phenyl)-phenyl, o-Fluorphenyl, m-Fluorphenyl, p-Fluorphenyl, m-Methoxyphenyl, p-Methoxyphenyl, Phenethyl, Benzyl, m-Hydroxybenzyl, p-Hydroxybenzyl, p-Nitrobenzyl, m-Trifluormethylphenyl, p-(CH₃)₂NCH₂CH₂CH₂O-Benzyl, p-(CH₃)₃COC(O)CH₂O-Benzyl, p-(HOOCCH₂O)-Benzyl, 2-Aminopyrid-6-yl, p-(N-Morpholino-CH₂CH₂O)-benzyl, -CH₂CH₂C(O)NH₂, -CH₂-Imidazol-4-yl, -CH₂-(3-Tetrahydrofuranyl), -CH₂-Thiophen-2-yl, -CH₂(1-Methyl)-cyclopropyl, -CH₂-Thiophen-3-yl, Thiophen-3-yl, Thiophen-2-yl, -CH₂-C(O)O-t-Butyl, -CH₂-C(CH₃)₃, -CH₂CH(CH₂CH₃)₂, 2-Methylcyclopentyl, Cyclohex-2-enyl, -CH[CH(CH₃)₂]COOCH₃, -CH₂CH₂N(CH₃)₂, -CH₂C(CH₃)=CH₂, -CH₂CH=CHCH₃ (cis und trans), -CH₂OH, -CH(OH)CH₃, -CH(O-t-Butyl)CH₃, -CH₂OCH₃, -(CH₂)₄NH-Boc, -(CH₂)₄NH₂, -CH₂-Pyridyl, Pyridyl, -CH₂-Naphthyl, -CH₂-(N-Morpholino), p-(N-Morpholino-CH₂CH₂O)-benzyl, Benzo[b]thiophen-2-yl, 5-Chlorbenzo[b]thiophen-2-yl, 4,5,6,7-Tetrahydrobenzo[b]thiophen-2-yl, Benzo[b]thiophen-3-yl, 5-Chlorbenzo[b]thiophen-3-yl, Benzo[b]thiophen-5-yl, 6-Methoxynaphth-2-yl, -CH₂CH₂SCH₃, Thien-2-yl und Thien-3-yl, Z für die Formel -T-CX'X"C(O)- steht, worin T ausgewählt ist aus der Gruppe, die besteht aus einer Bindung, die R¹ kovalent bindet an -CX'X"-, Sauerstoff, Schwefel oder -NR⁵,
worin R⁵ für Wasserstoff, Acyl, Alkyl, Aryl oder Heteroaryl steht,
X' für Wasserstoff, Hydroxy oder Fluor steht,
X" für Wasserstoff, Hydroxy oder Fluor steht, oder X' oder X" zusammen eine Oxogruppe bilden, m für 0 oder 1 steht,
n für 0, 1 oder 2 steht,
worin
der Ausdruck Alkyl sich bezieht auf monovalente Alkylgruppen mit vorzugsweise 1 bis 10 Kohlenstoffatomen,
der Ausdruck Alkylaryl sich bezieht auf Alkylenarylgruppen mit vorzugsweise 1 bis 8 Kohlenstoffatomen im Alkylenteil und 6 bis 10 Kohlenstoffatomen im Arylteil,
der Ausdruck Alkoxy sich bezieht auf die Gruppe Alkyl-O-,
der Ausdruck Alkenyl sich bezieht auf Alkenylgruppen mit vorzugsweise 2 bis 10 Kohlenstoffatomen,
der Ausdruck Alkinyl sich bezieht auf Alkinylgruppen mit vorzugsweise 2 bis 10 Kohlenstoffatomen,
der Ausdruck Aryl sich bezieht auf Phenyl oder Naphthyl, worin diese Arylgruppen optional substituiert sein können mit 1 bis 5 Substituenten, die aus der Gruppe ausgewählt sind, die besteht aus Alkyl, Alkoxy, Halogen, Cyano, Nitro, Trihalogenmethyl und Thioalkoxy,
der Ausdruck Aryloxy sich bezieht auf die Gruppe Aryl-O-, worin die Arylgruppe wie oben definiert ist, unter Einschluss optional substituierter Arylgruppen,
der Ausdruck Carboxyalkyl sich bezieht auf die Gruppe -CH(O)O-Alkyl, worin Alkyl wie oben definiert ist,
der Ausdruck Cycloalkyl sich bezieht auf Cycloalkylgruppen mit 3 bis 12 Kohlenstoffatomen, die einen einzelnen cyclischen Ring oder multiple kondensierte Ringe haben,
der Ausdruck Cycloalkenyl sich bezieht auf Cycloalkenylgruppen mit 4 bis 8 Kohlenstoffatomen, die einen einzelnen cyclischen Ring und wenigstens einen Punkt einer inneren Ungesättigtheit haben,
der Ausdruck Heteroaryl sich bezieht auf eine aromatische Carbocyclylgruppe mit 1 bis 15 Kohlenstoffatomen und 1 bis 4 Heteroatomen, die ausgewählt sind aus Sauerstoff, Stickstoff und Schwefel innerhalb wenigstens eines Rings,
der Ausdruck Heterocyclyl sich bezieht auf eine monovalente gesättigte oder ungesättigte Gruppe, die einen einzelnen Ring oder multiple kondensierte Ringe hat, mit 1 bis 15 Kohlenstoffatomen und 1 bis 4 Heteroatomen, die ausgewählt sind aus Stickstoff, Schwefel oder Sauerstoff innerhalb des Rings,
der Ausdruck Thioalkoxy sich bezieht auf die Gruppe -S-Alkyl,
V jeweils unabhängig ausgewählt ist aus der Gruppe, die besteht aus Hydroxy, Acyl, Acyloxy, Alkyl, Alkoxy, Alkenyl, Alkinyl, Amino, Aminoacyl, Alkaryl, Aryl, Aryloxy, Carboxyl, Carboxylalkyl, Cyano, Halogen, Nitro, Hetereoaryl, Thioalkoxy und Trihalogenmethyl,
R^{b} ausgewählt ist aus der Gruppe, die besteht aus Wasserstoff, Alkyl, Alkenyl, Alkinyl, Acyl, Aryl, Heteroaryl und Heterocyclyl, und
t für 0 bis 4 steht.

2. Verbindung der Formel 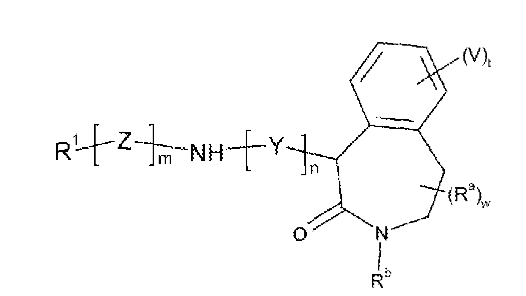
worin R¹, Z, Y, m, n, V, R^{b} und t die im Anspruch 1 definierten Bedeutungen haben und
worin R^{a} ausgewählt ist aus der Gruppe, die besteht aus Alkyl, Alkoxy, Amino, Carboxyl, Carboxylalkyl, Cyano und Halogen,
w für 0 bis 3 steht, und
worin die Ausdrücke Alkyl, Alkoxy und Carboxyalkyl die im Anspruch 1 angegebenen Bedeutungen haben.

3. Verbindung der Formel I 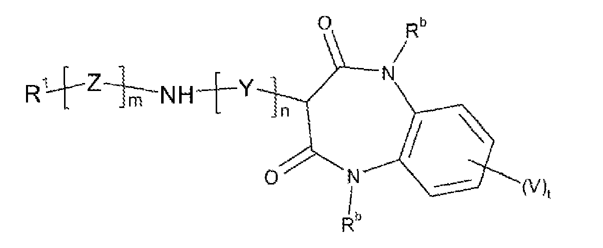
worin R¹, Z, Y, m, n, V, R^{b} und t die im Anspruch 1 definierten Bedeutungen haben.

4. Verbindung, die ausgewählt ist aus der Gruppe, die besteht aus
- Verbindungen der Formel 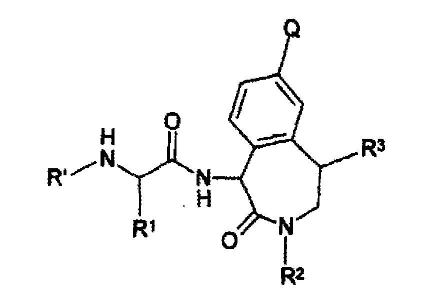
worin R', R¹, R², R³ und Q die in der folgenden Tabelle definierten Bedeutungen haben: 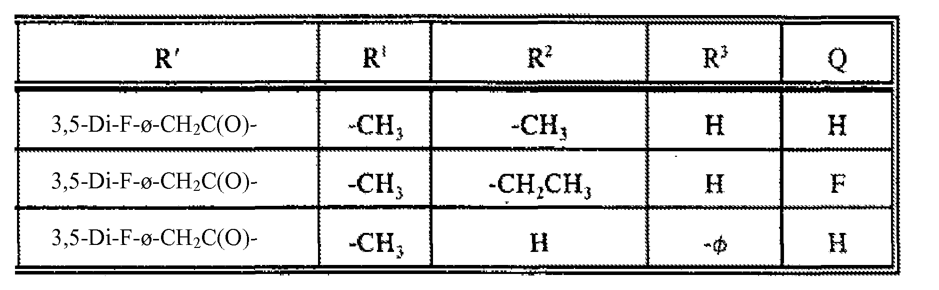
- Verbindungen der Formel: 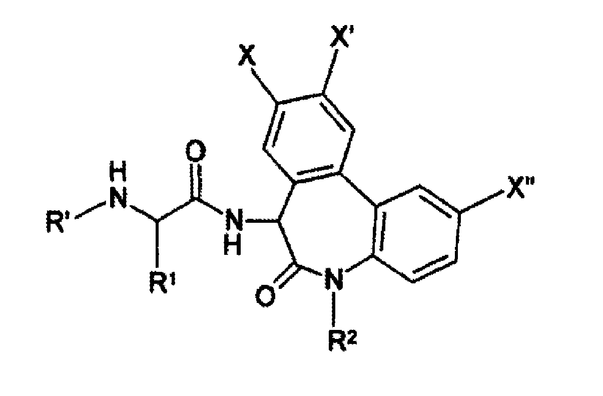
worin R', R¹, R², X, X' und X" die in der folgenden Tabelle definierten Bedeutungen haben: 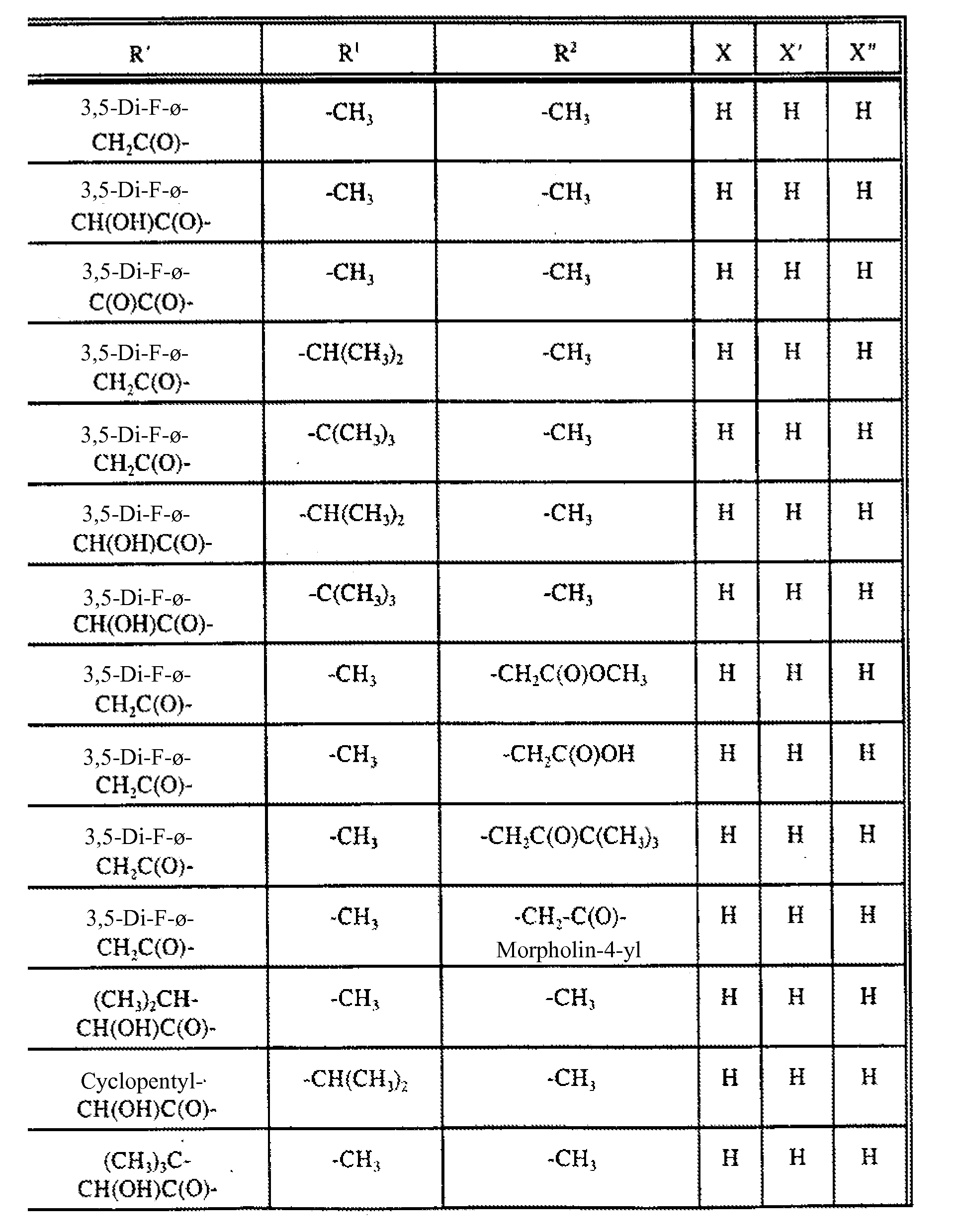 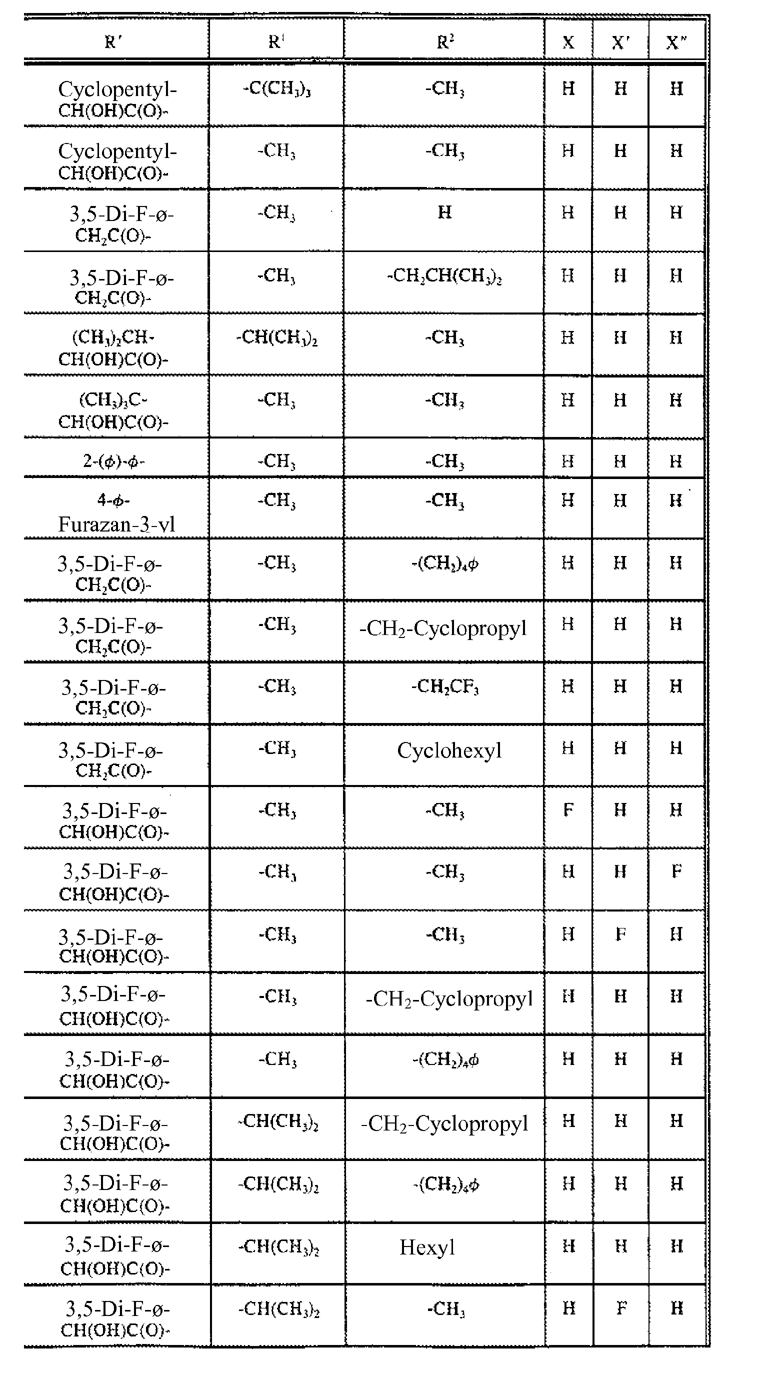 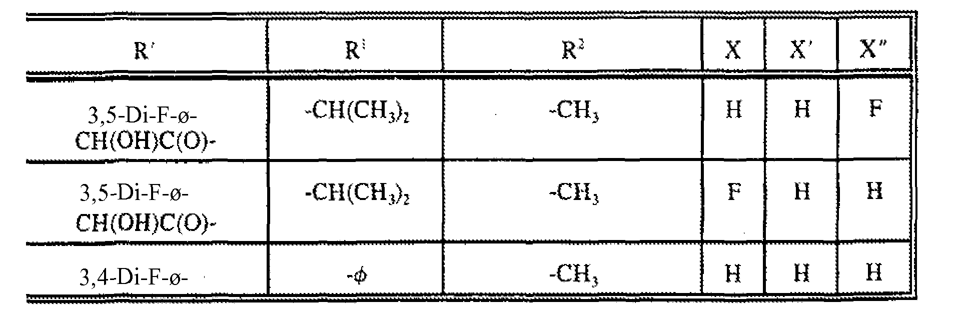
- Verbindungen der Formel: 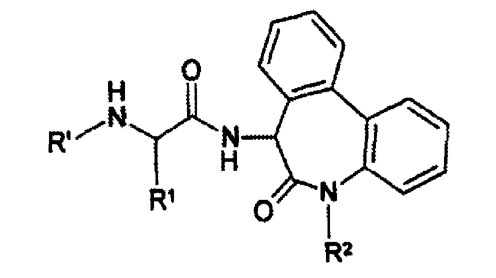
worin R', R¹ und R² die in der folgenden Tabelle definierten Bedeutungen haben: 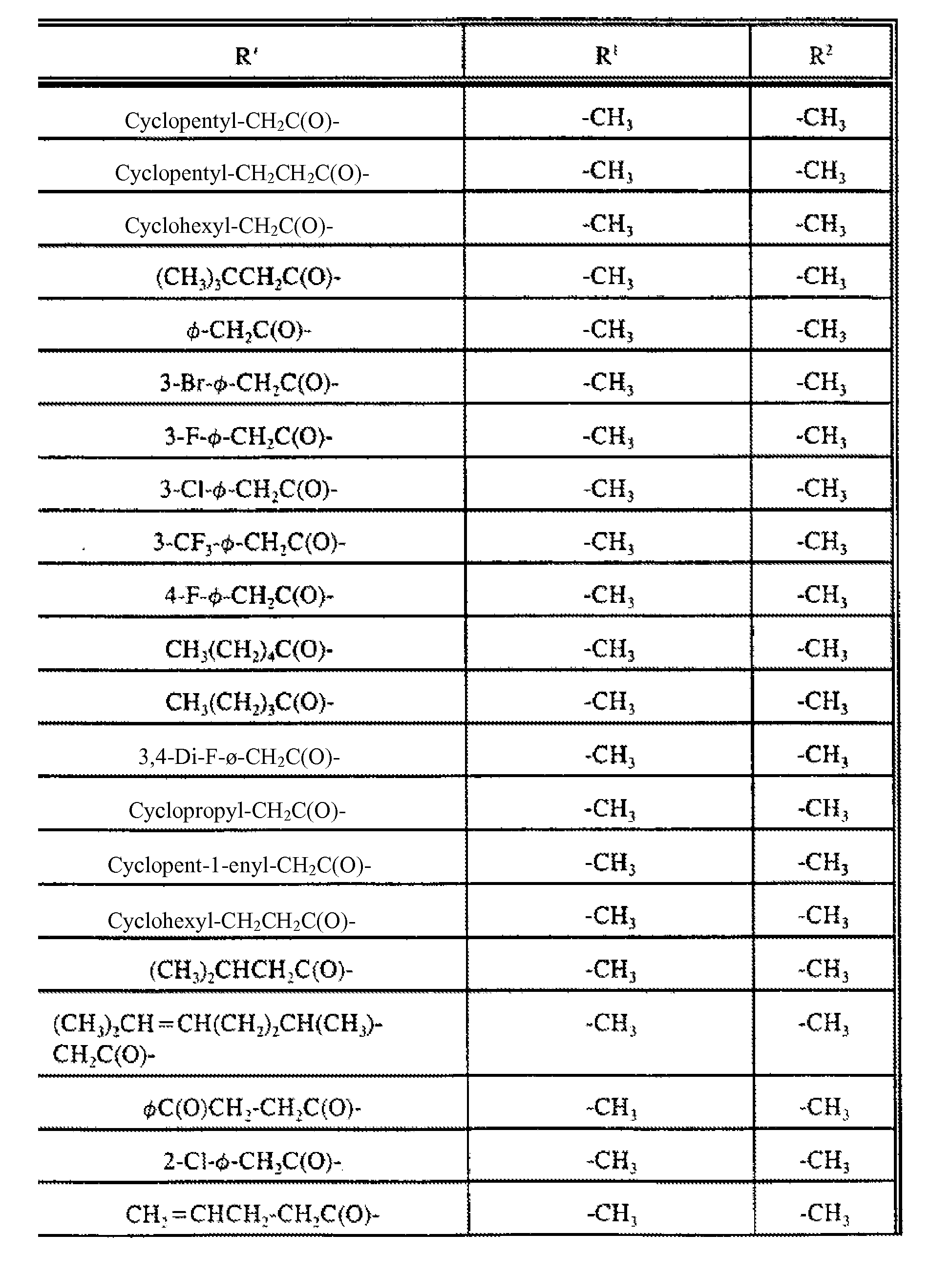 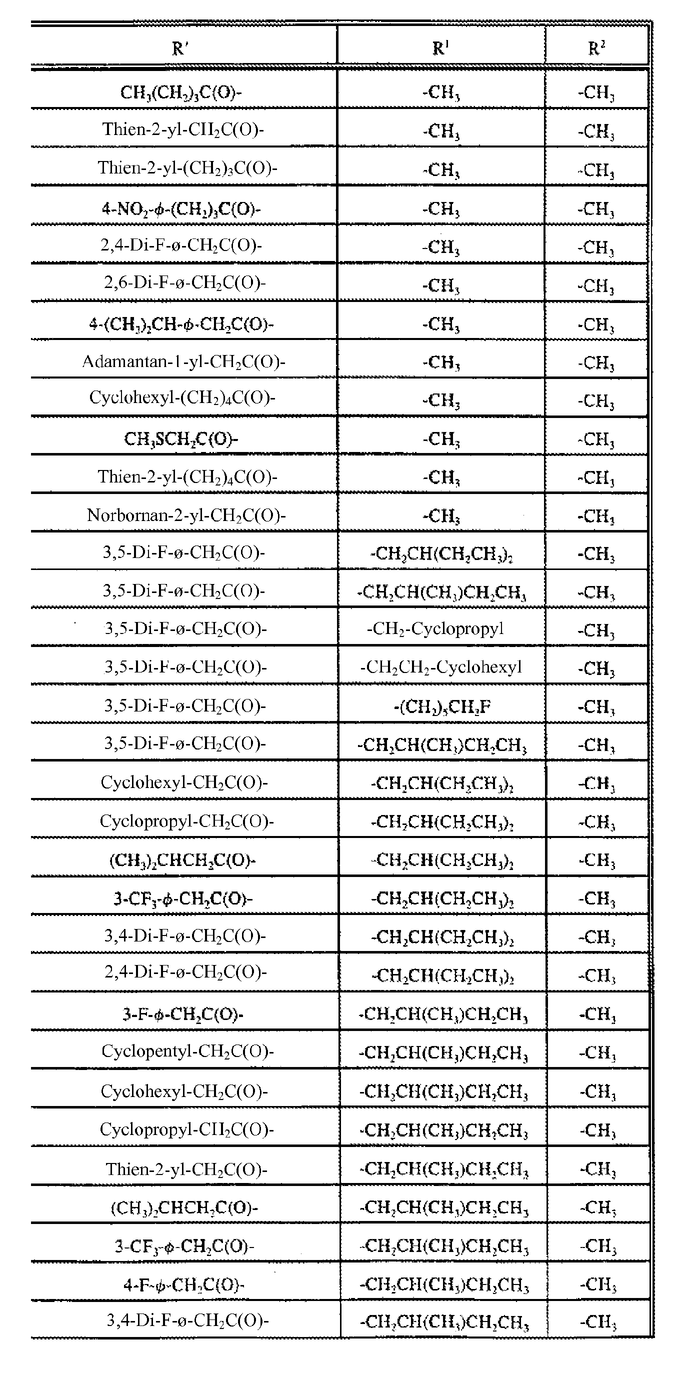 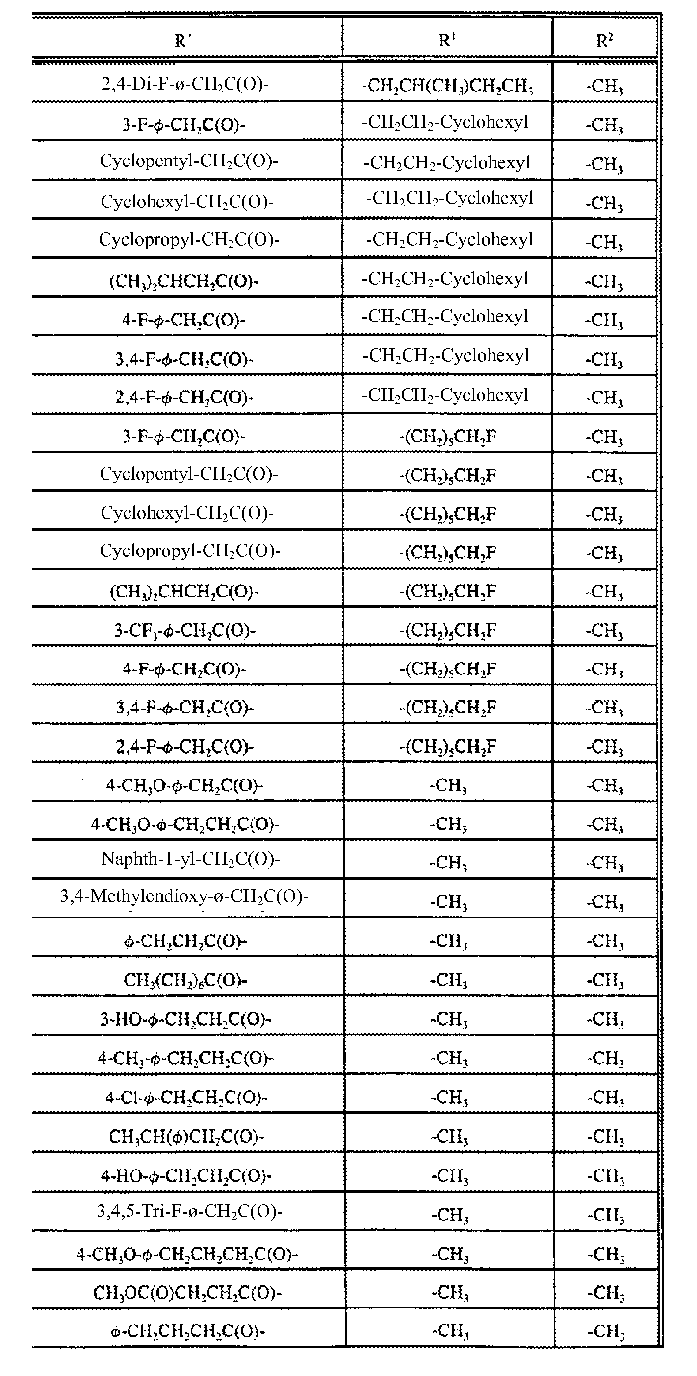 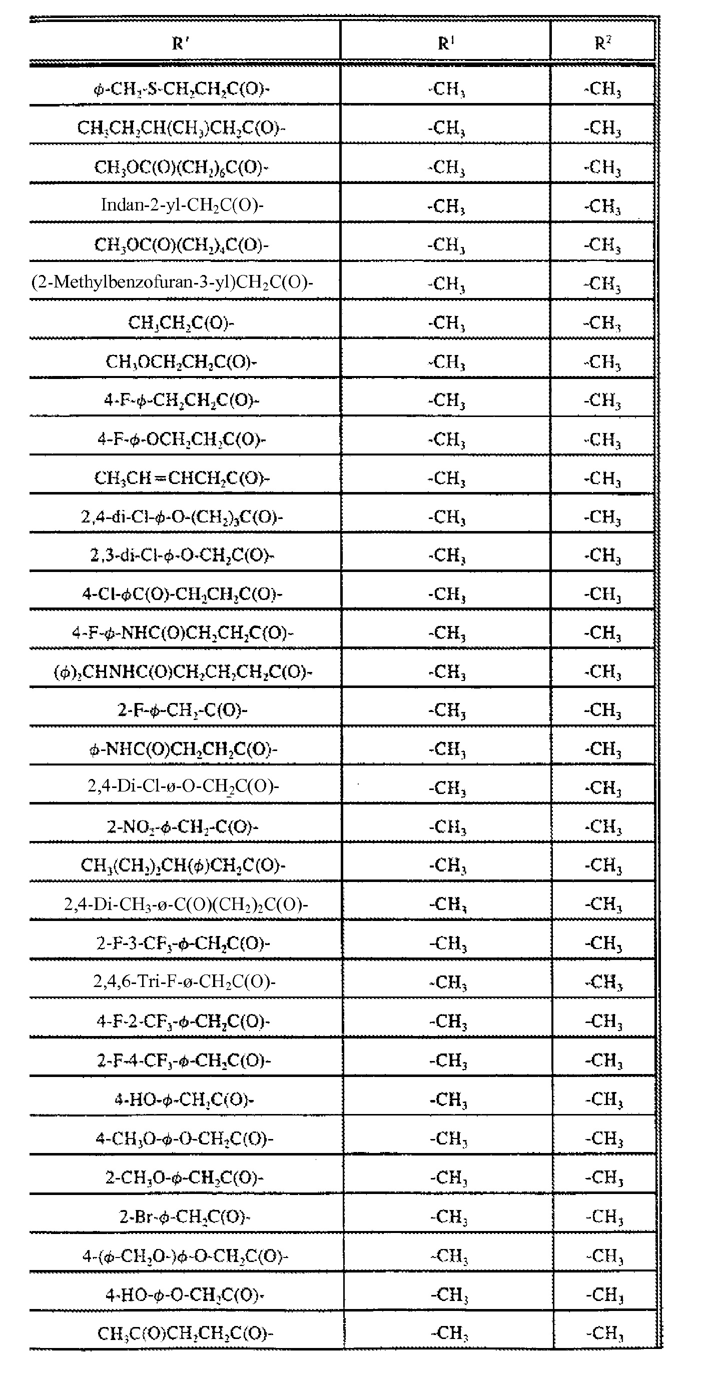 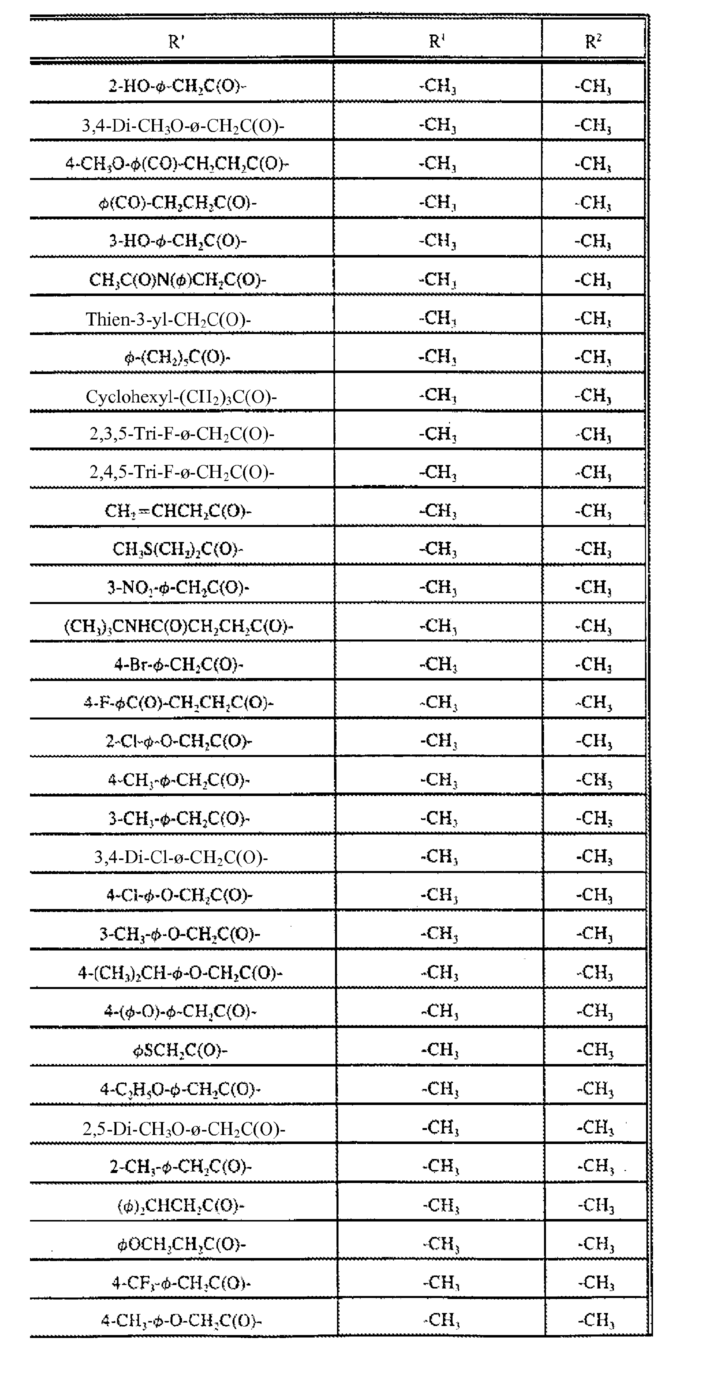 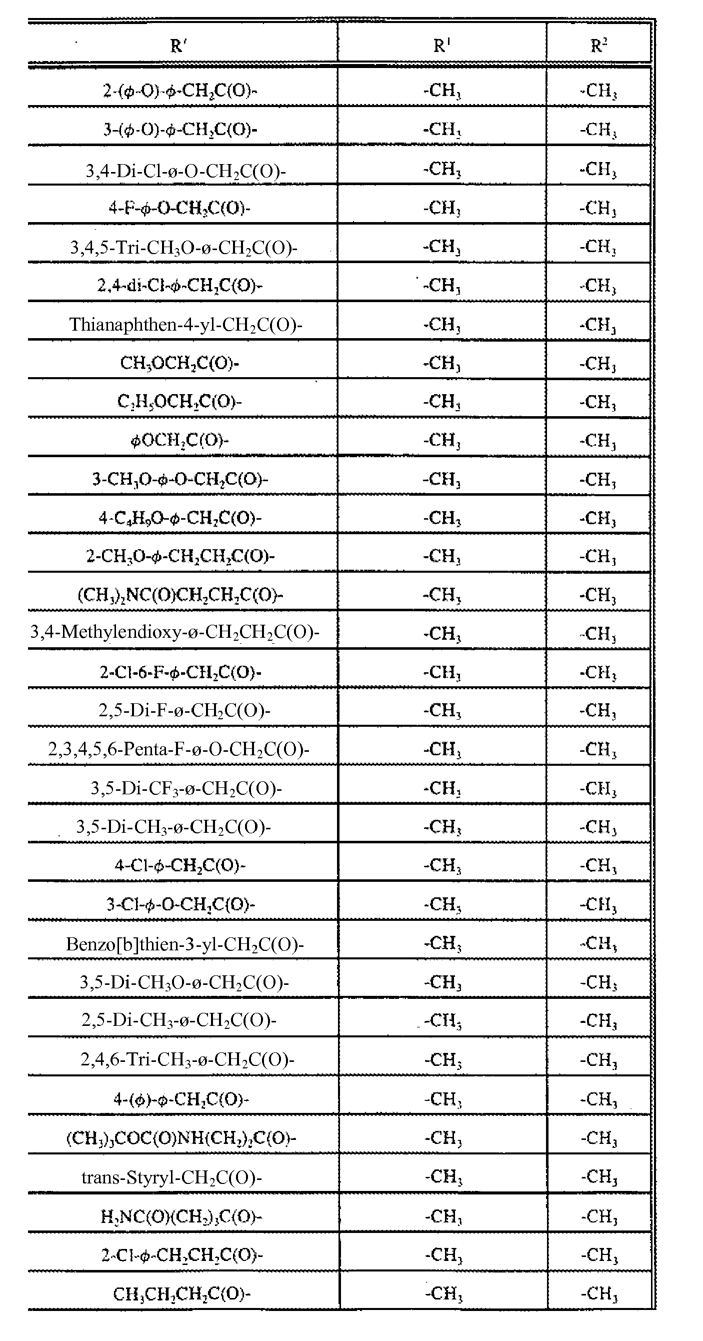 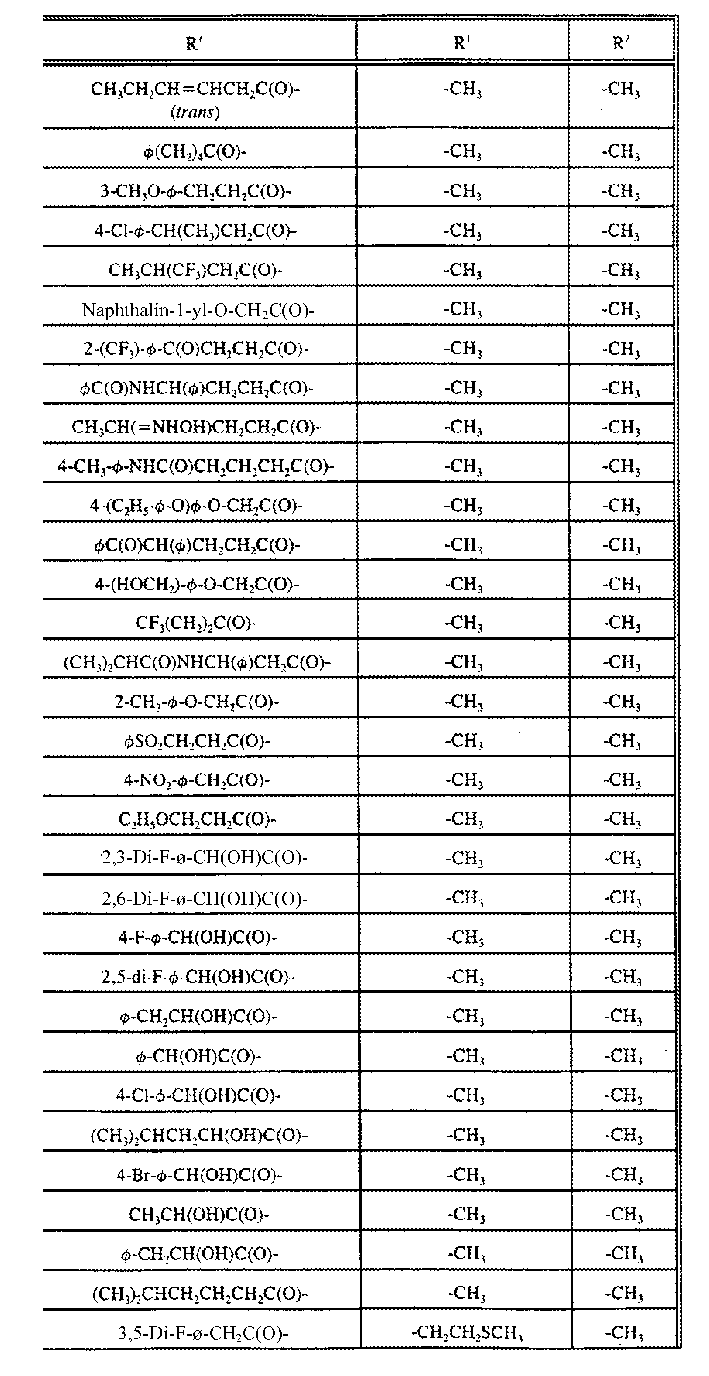 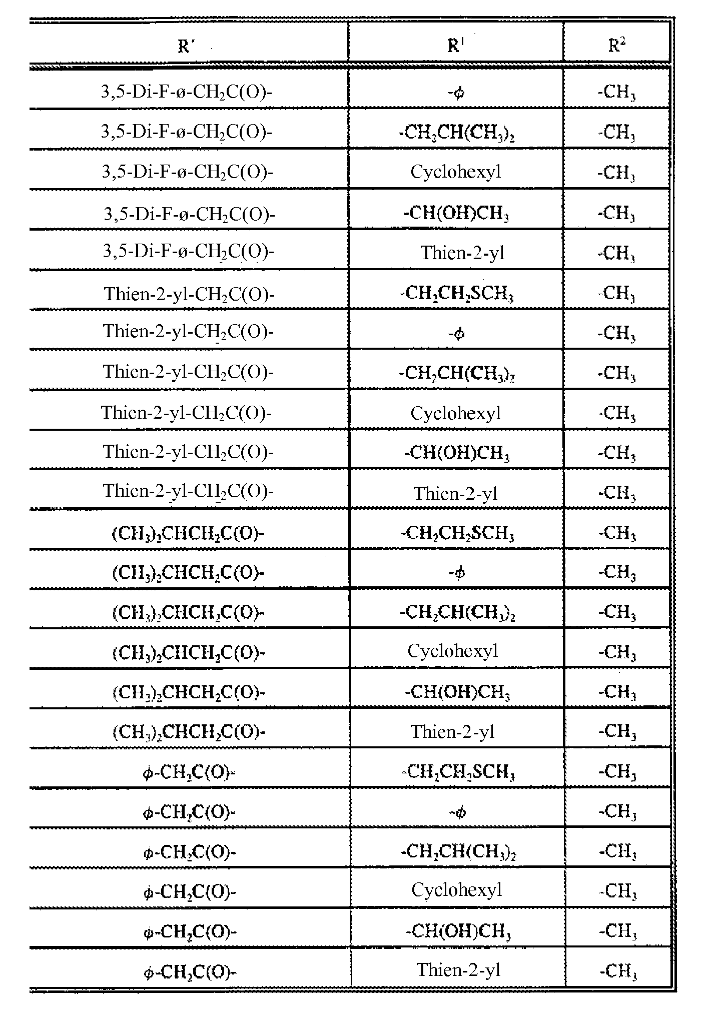
- Verbindungen der Formel: 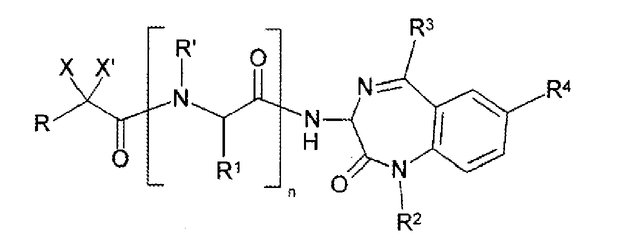
worin R, R', R¹ bis R⁴, X, X' und n die in der folgenden Tabelle definierten Bedeutungen haben: 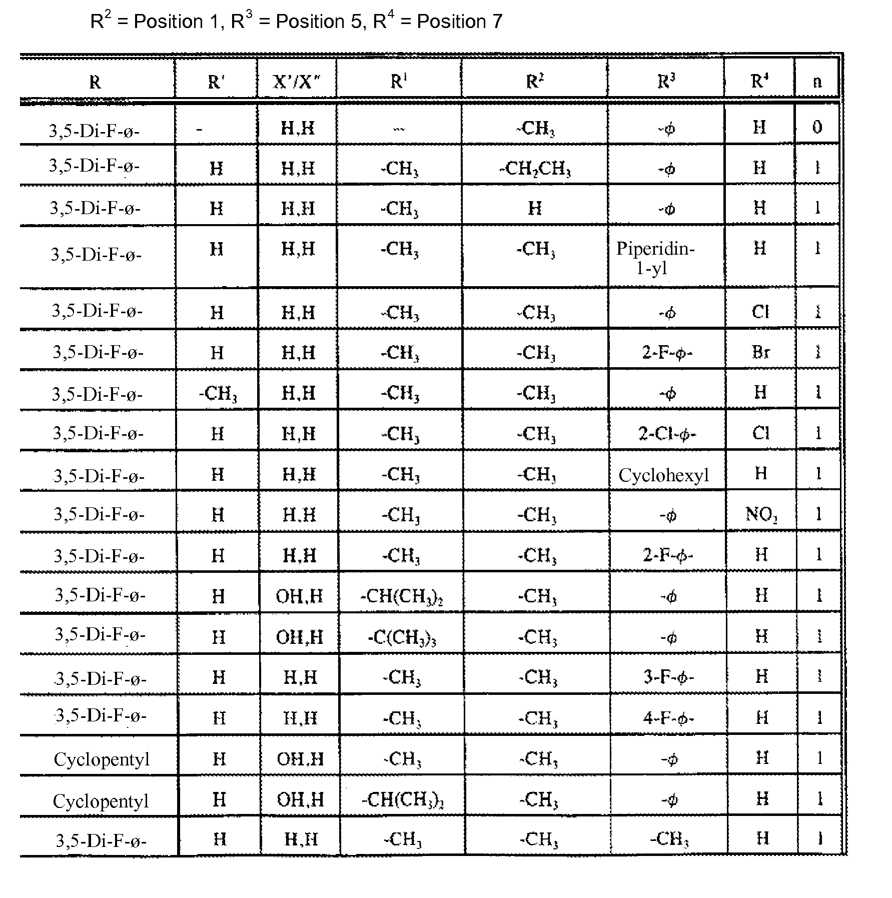 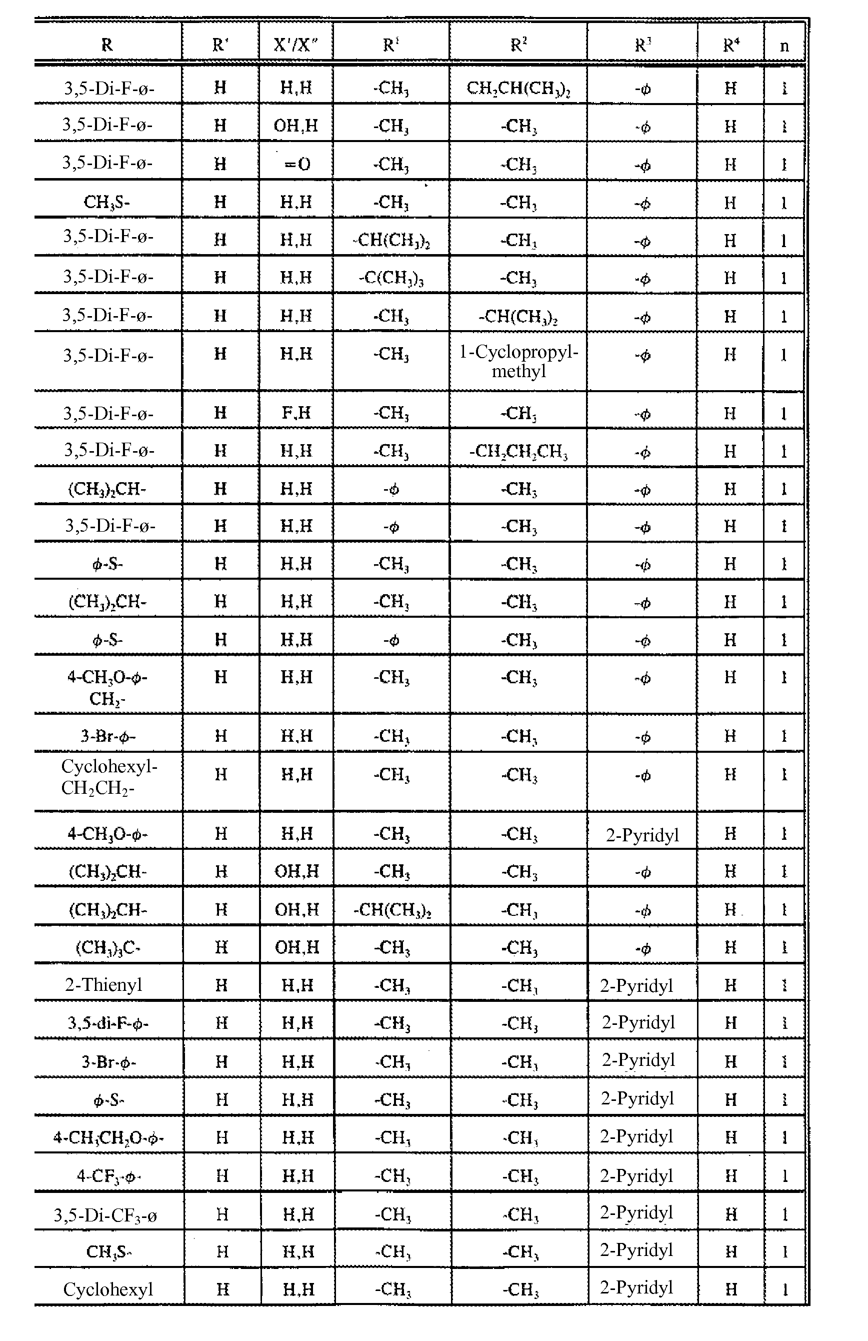 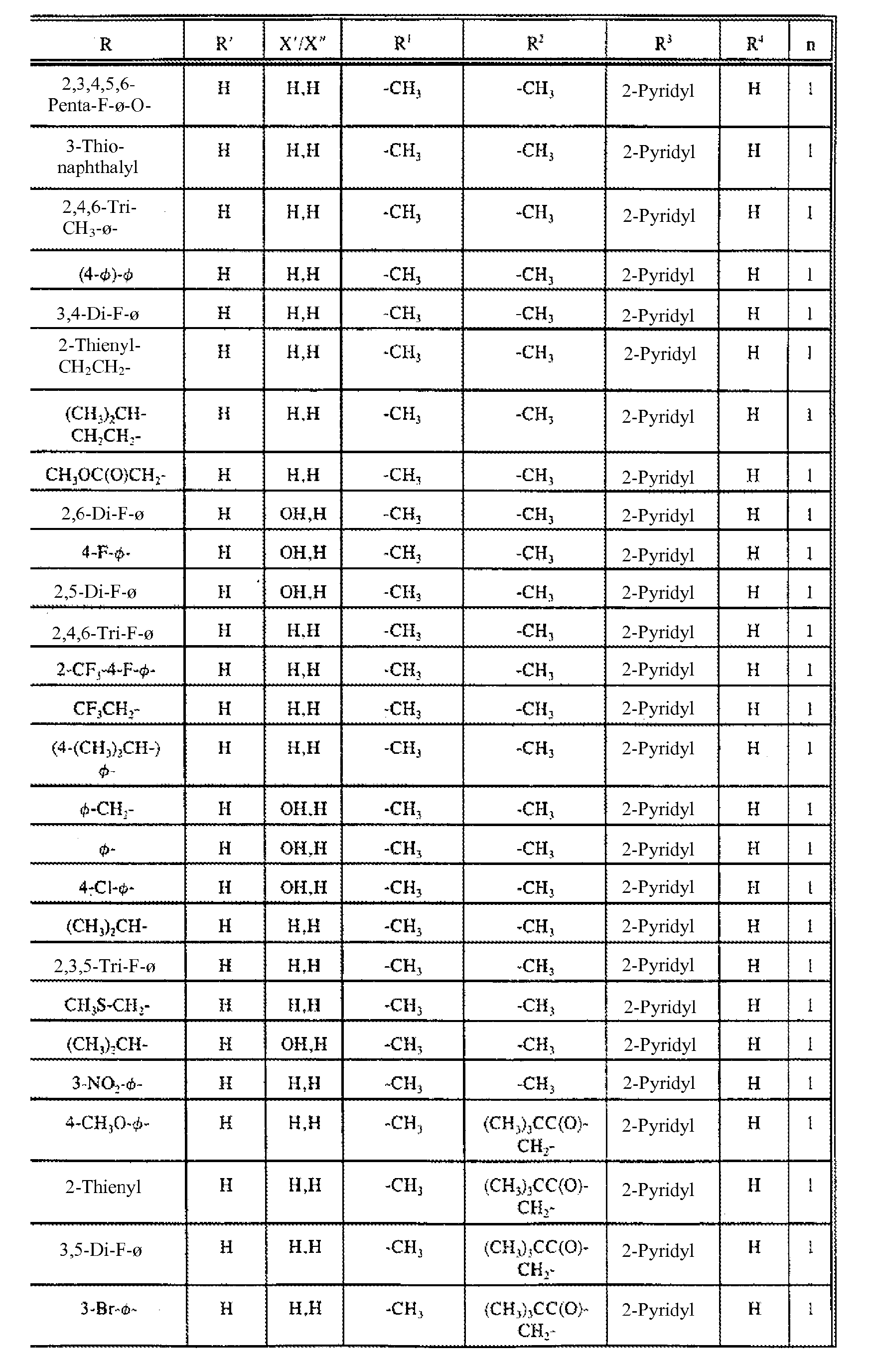 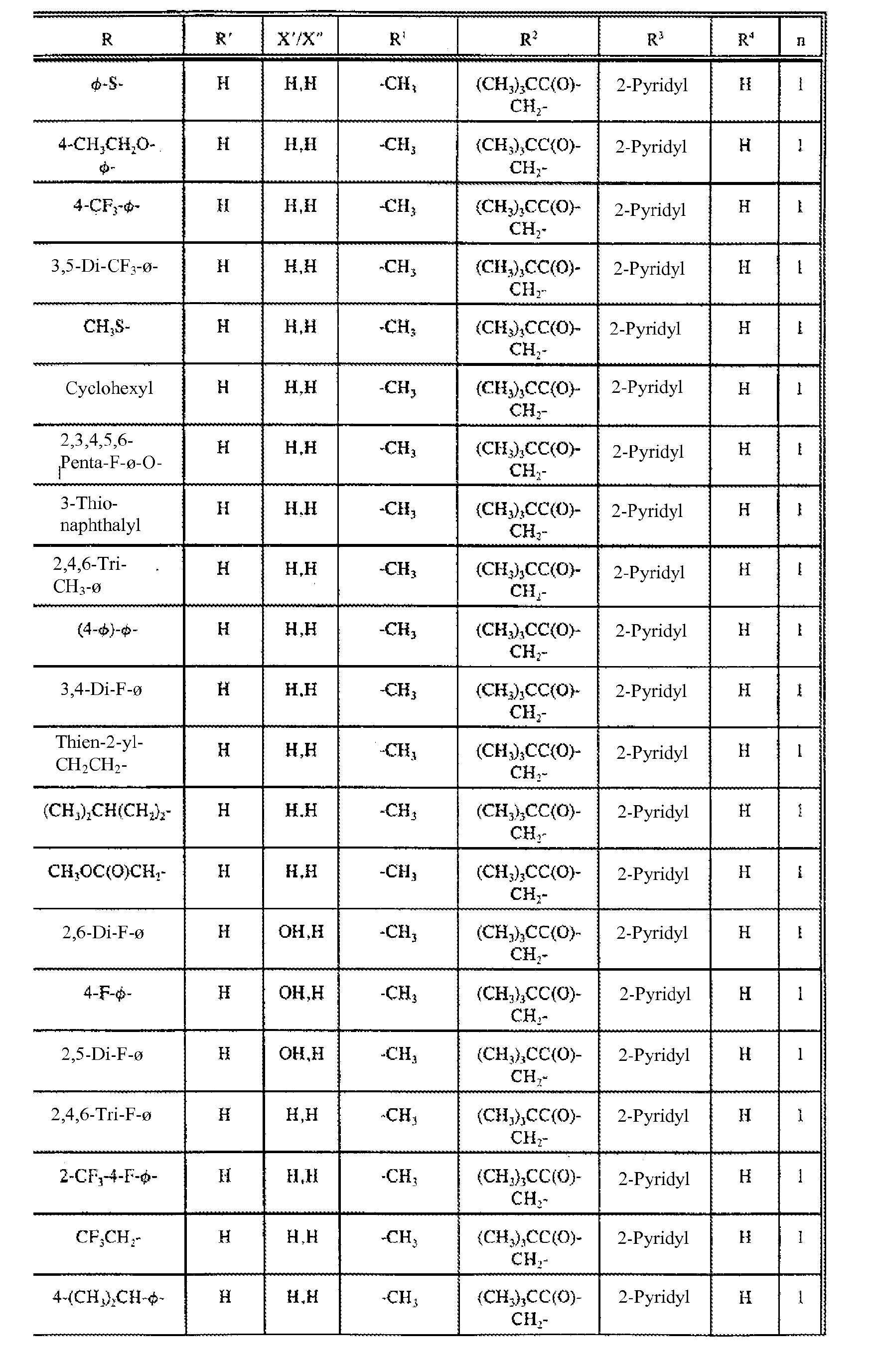 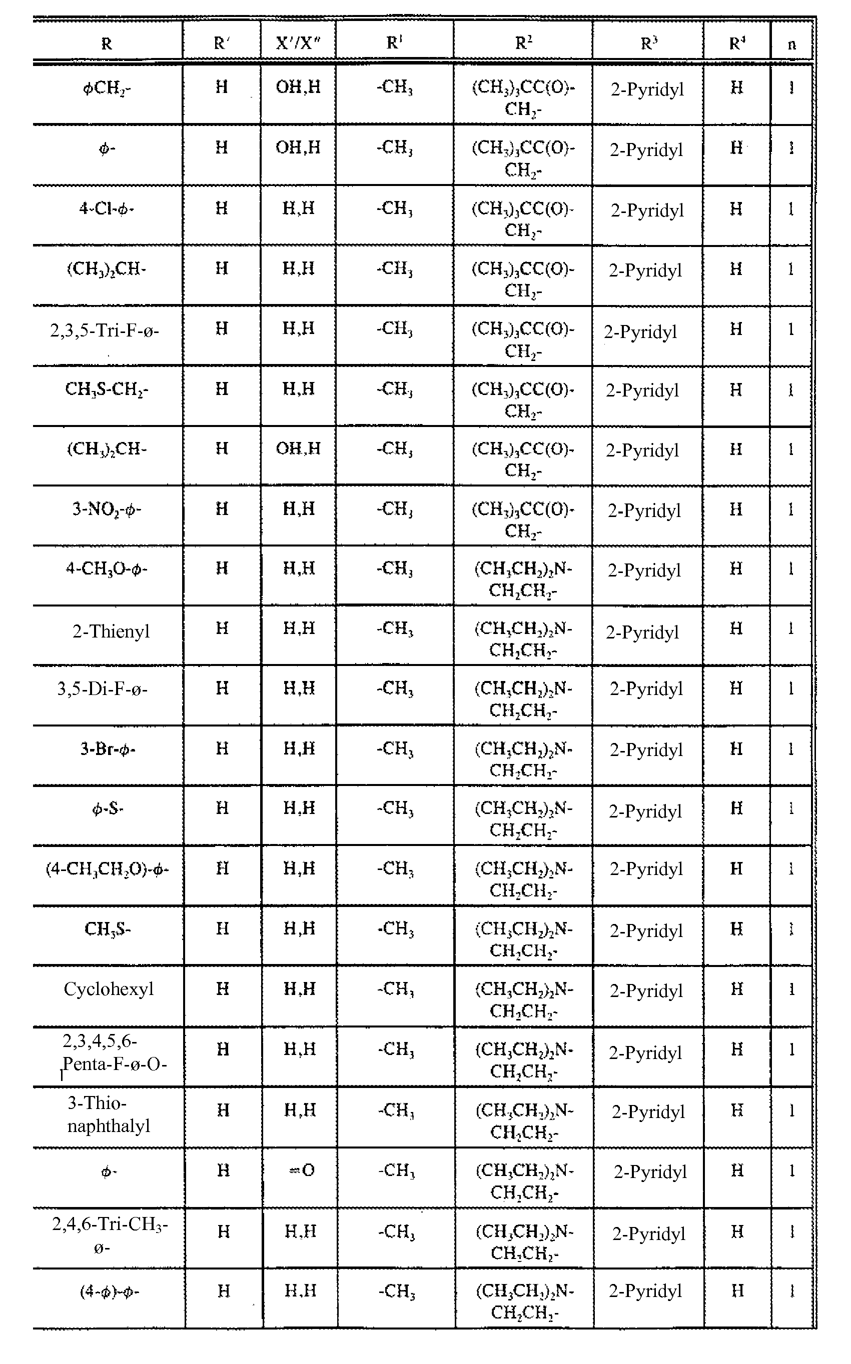 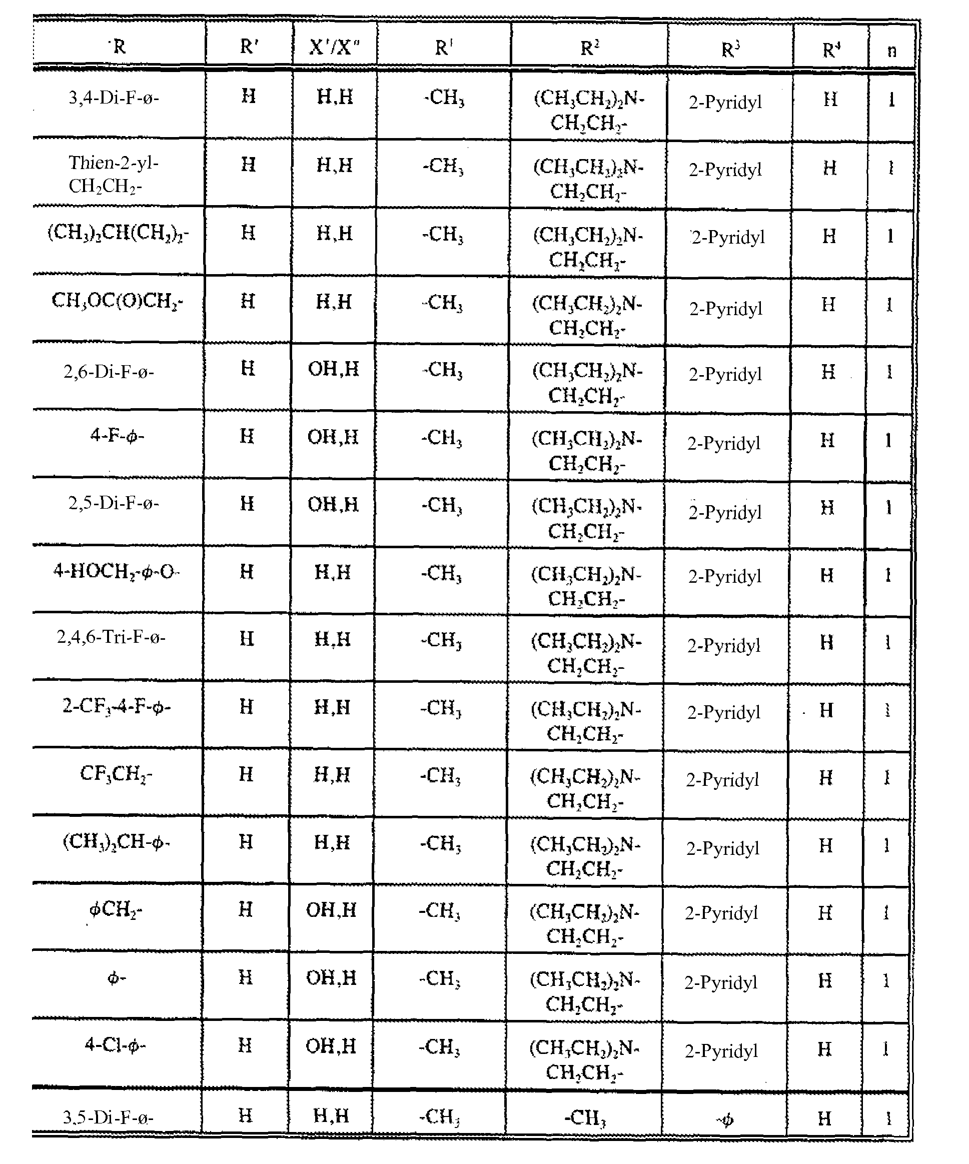
- Verbindungen der Formel 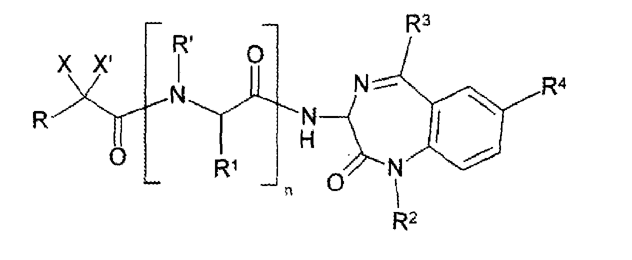
worin R, X, X', R', R¹ bis R⁴ und n die in der folgenden Tabelle definierten Bedeutungen haben: 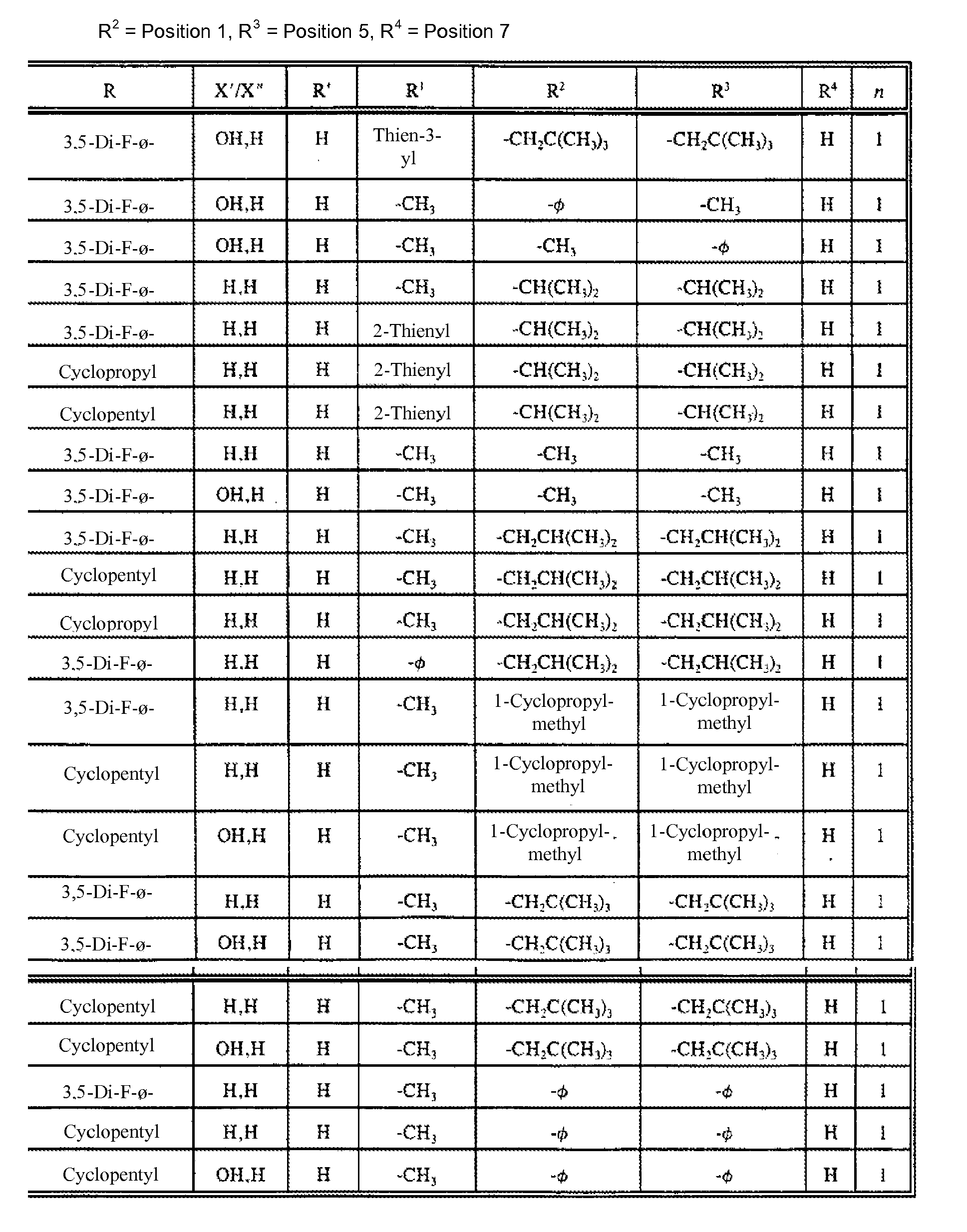
- und Verbindungen der Formel: 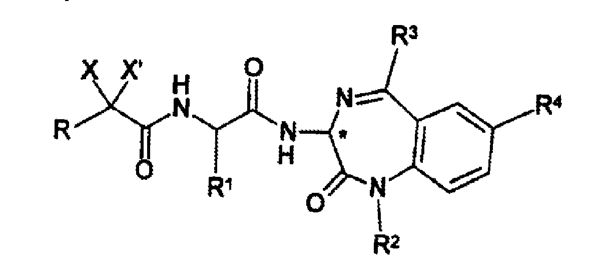
worin R, R¹ bis R⁴, X und X' die in der folgenden Tabelle definierten Bedeutungen haben:

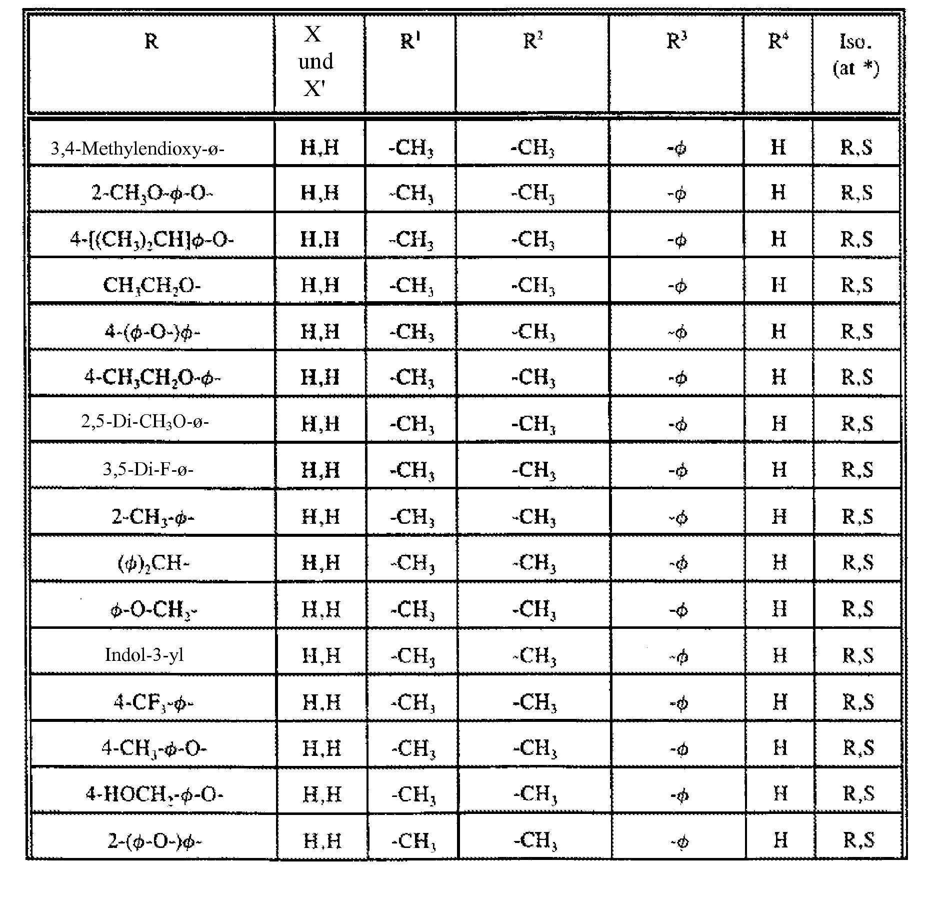

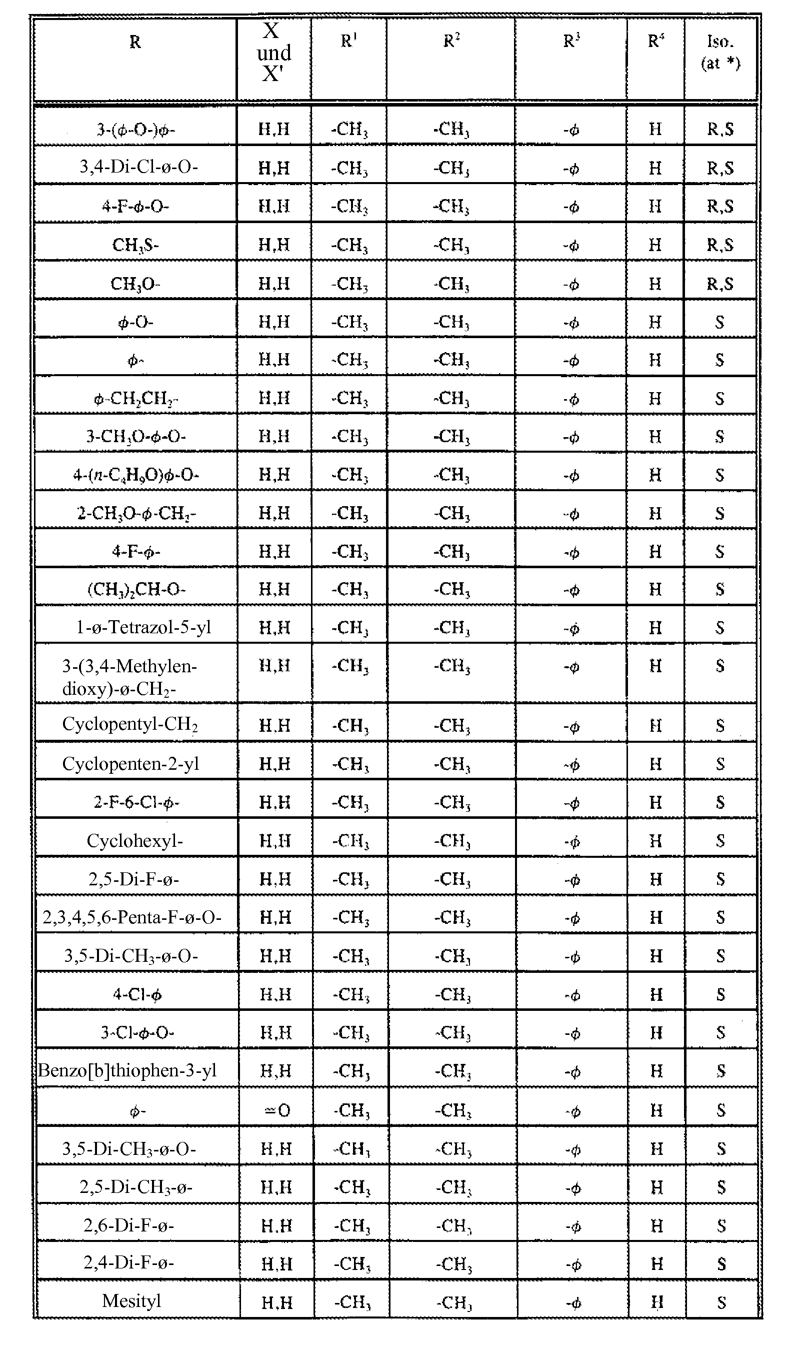

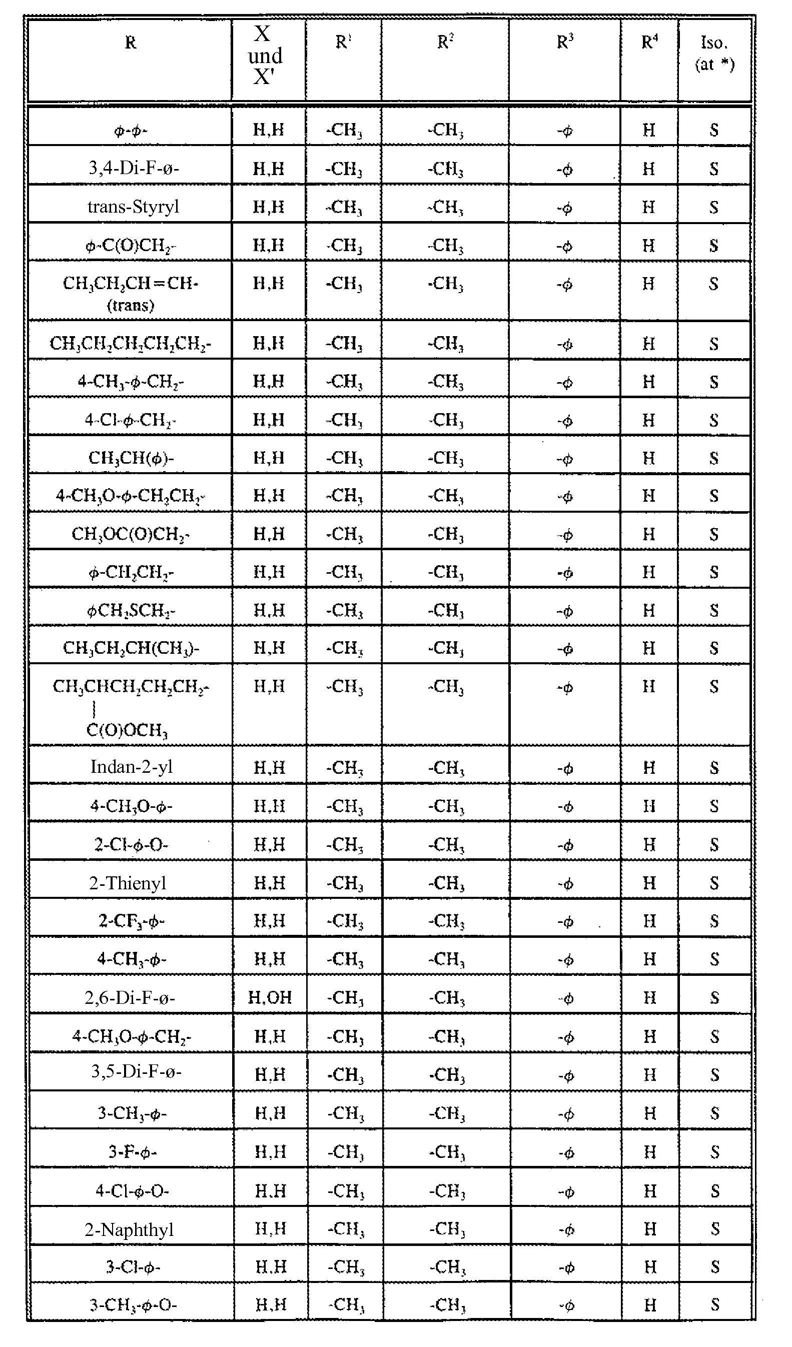

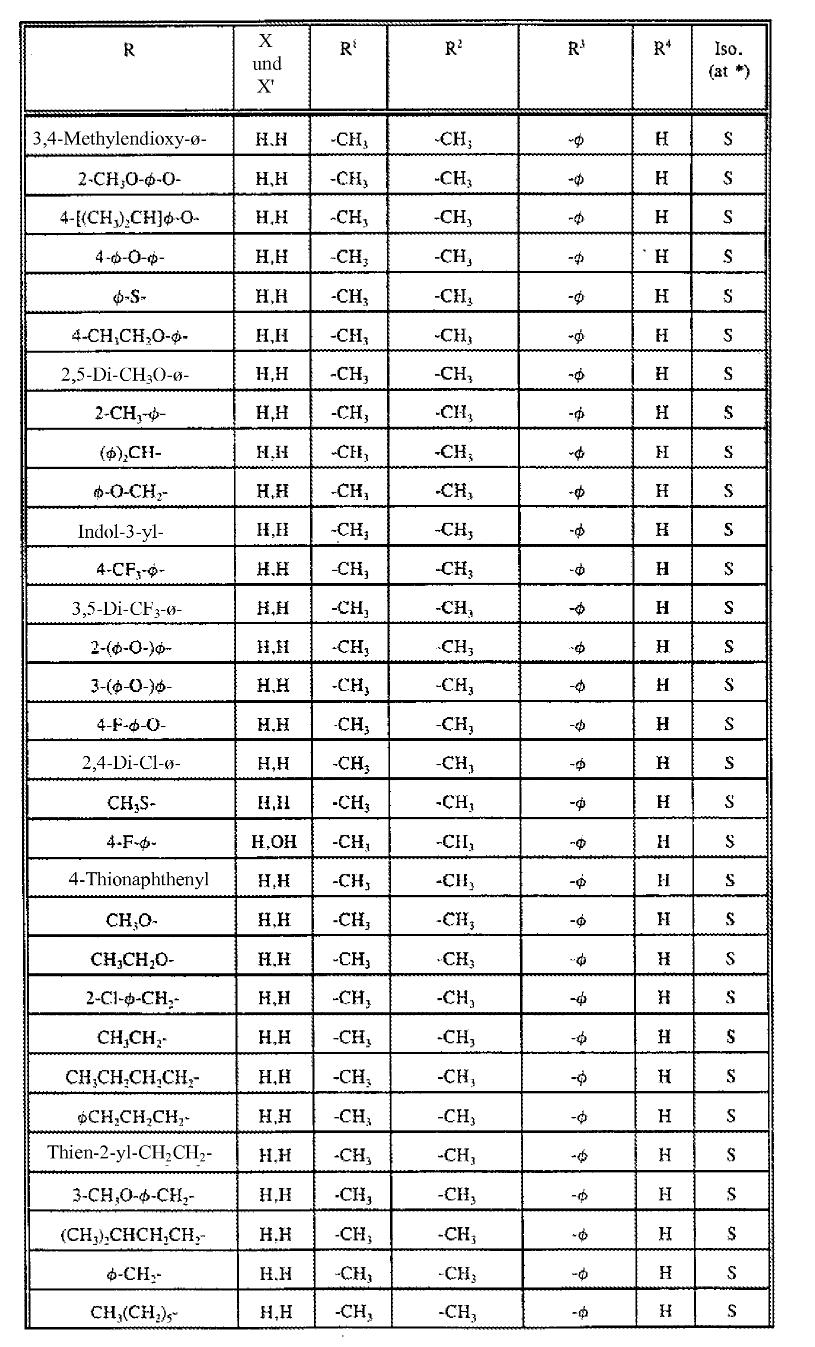

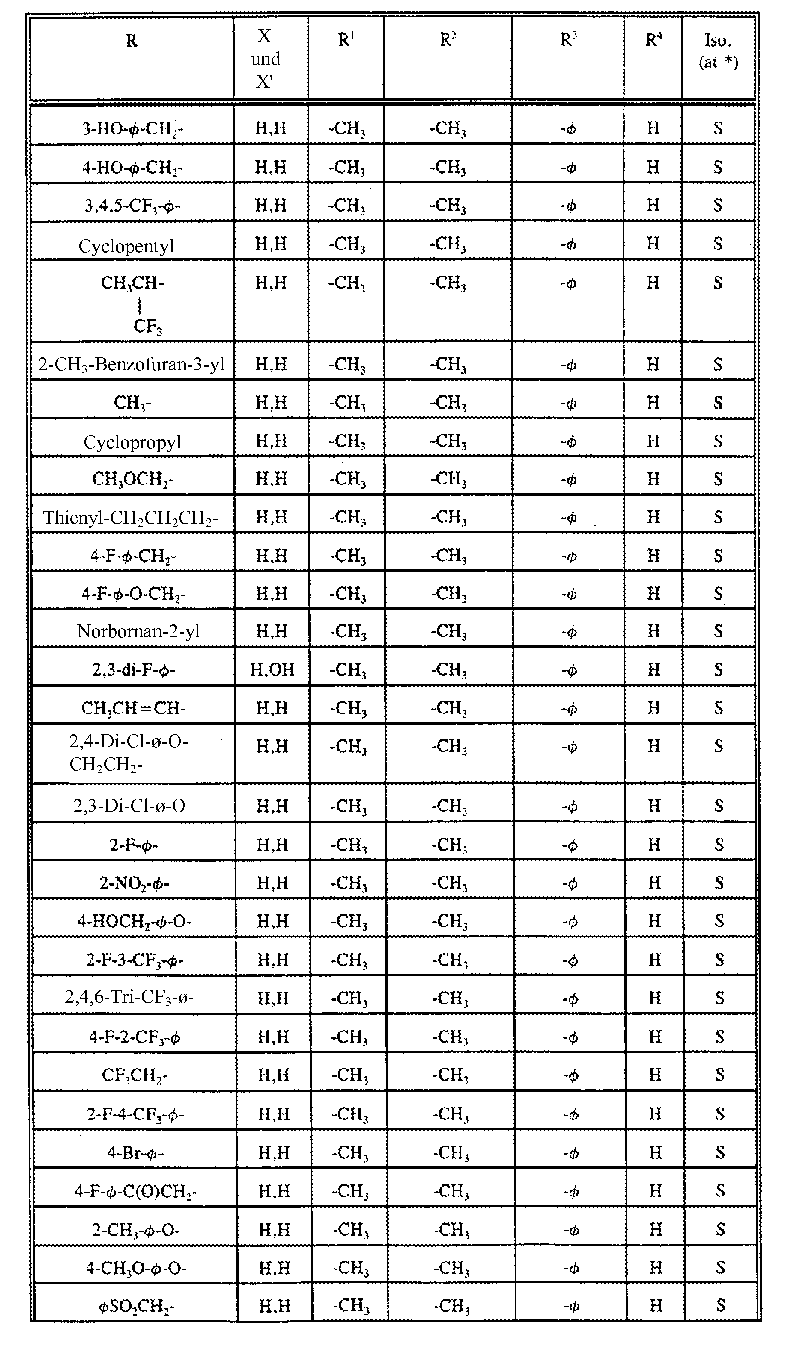

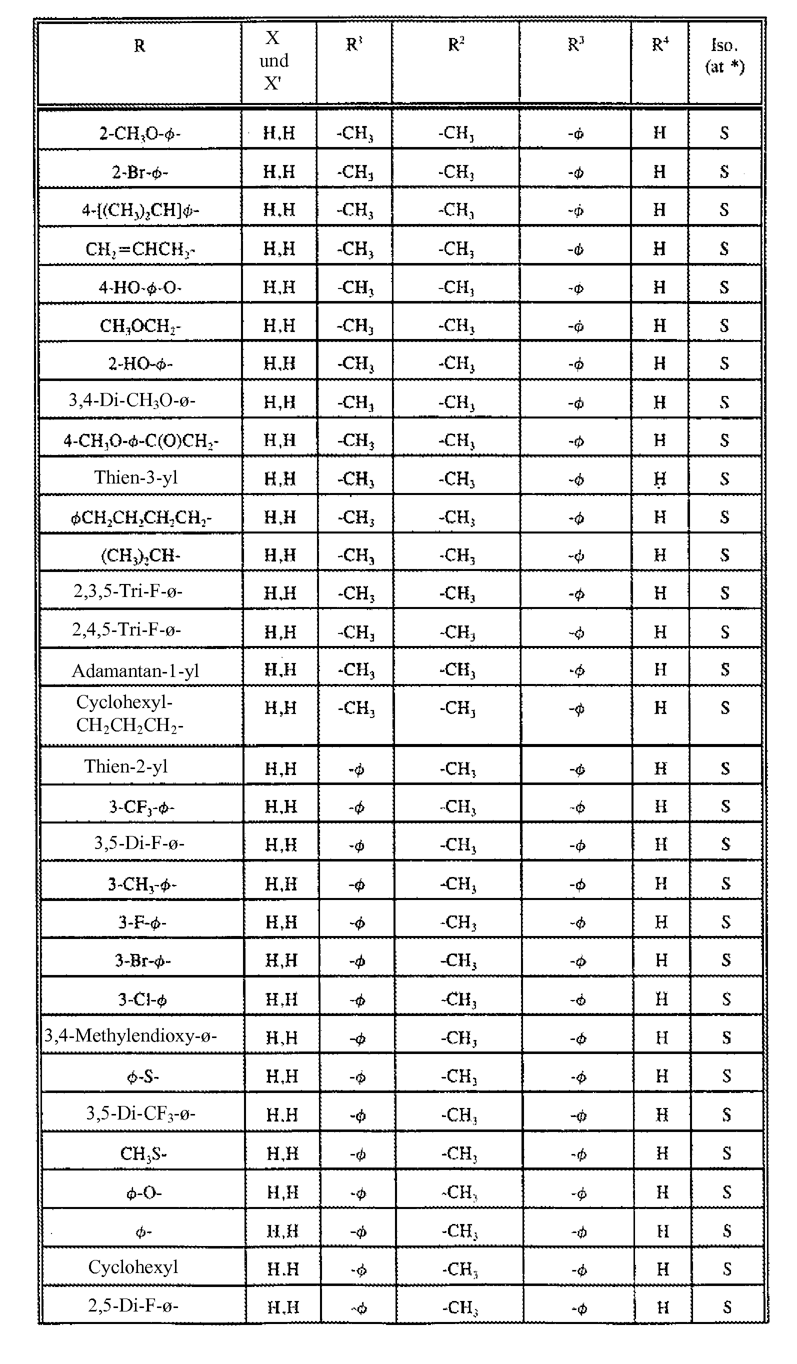

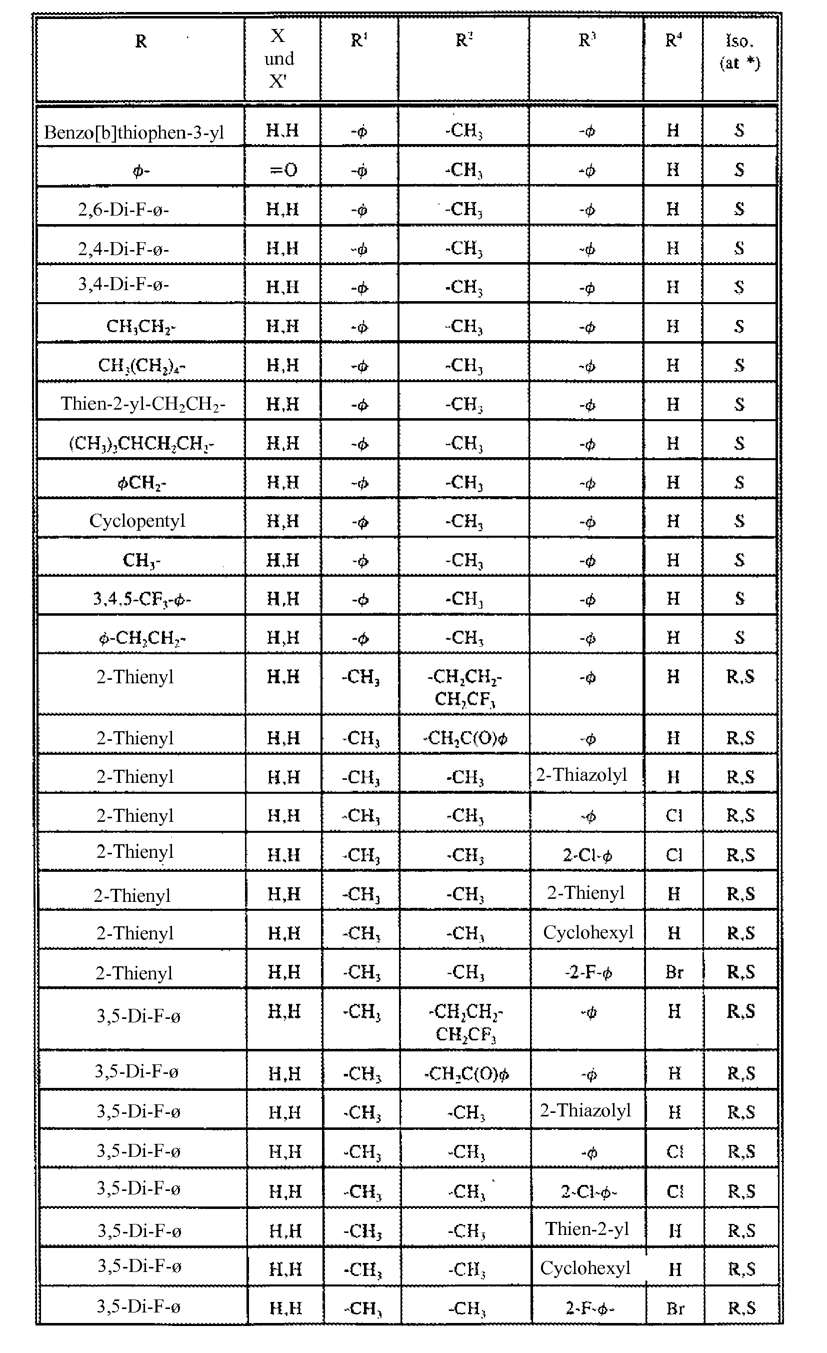

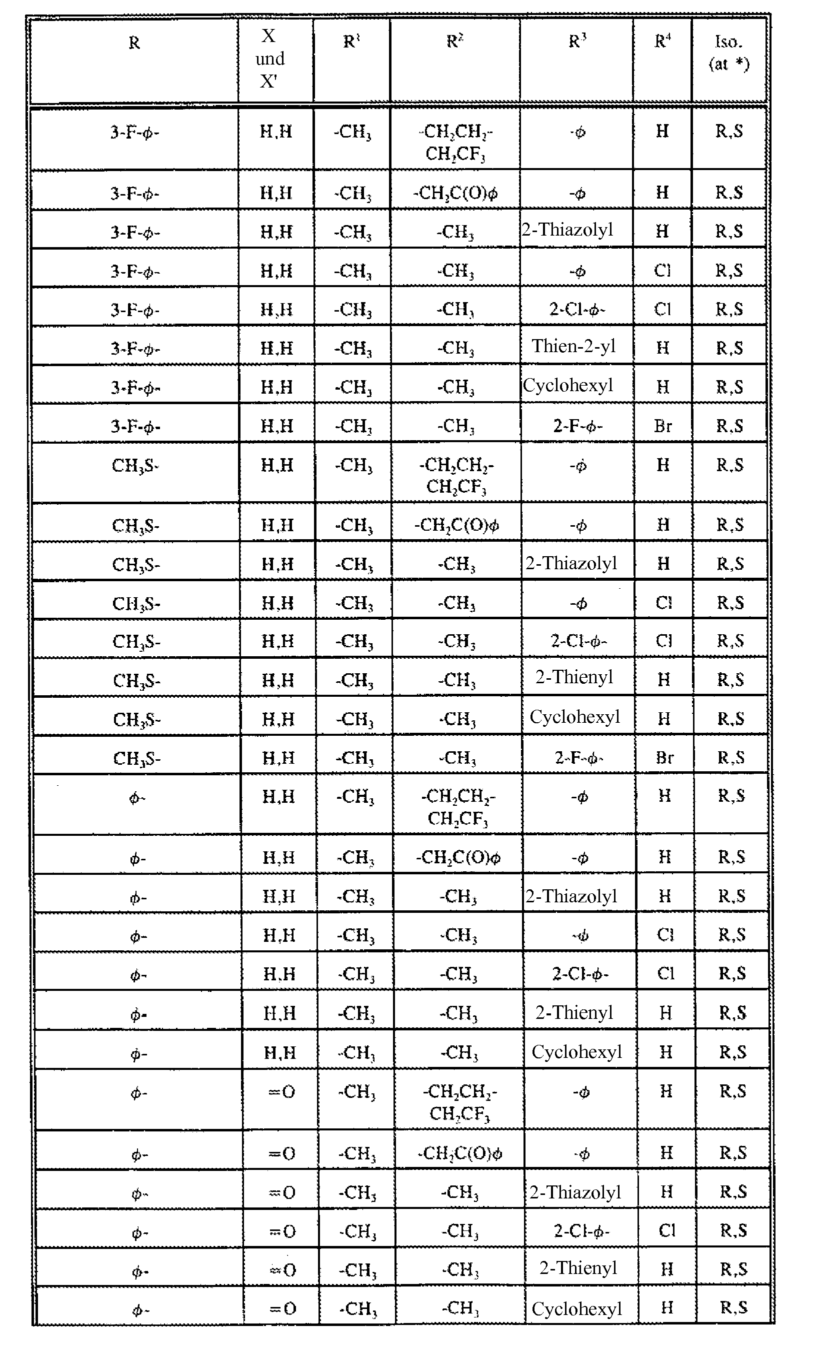

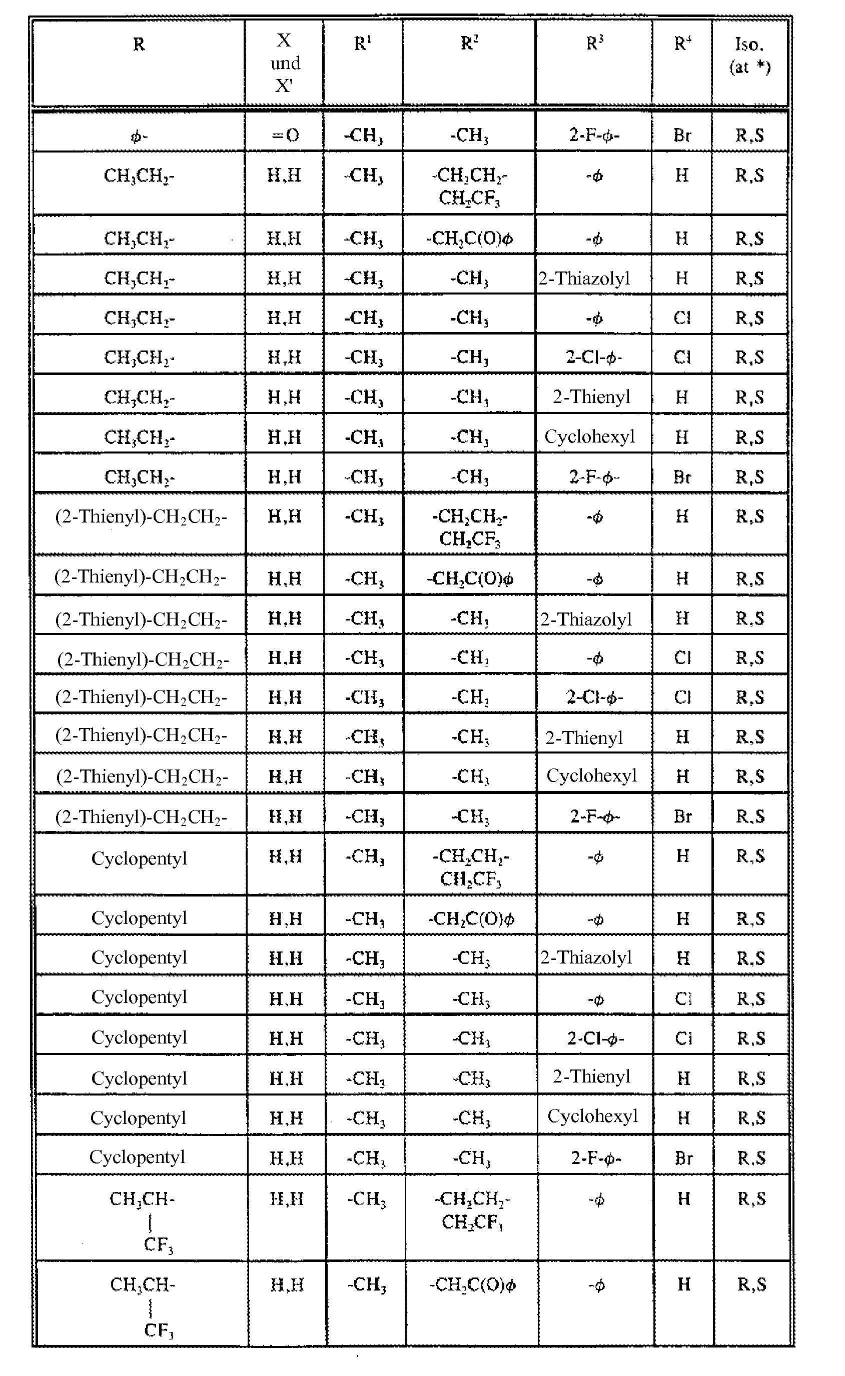

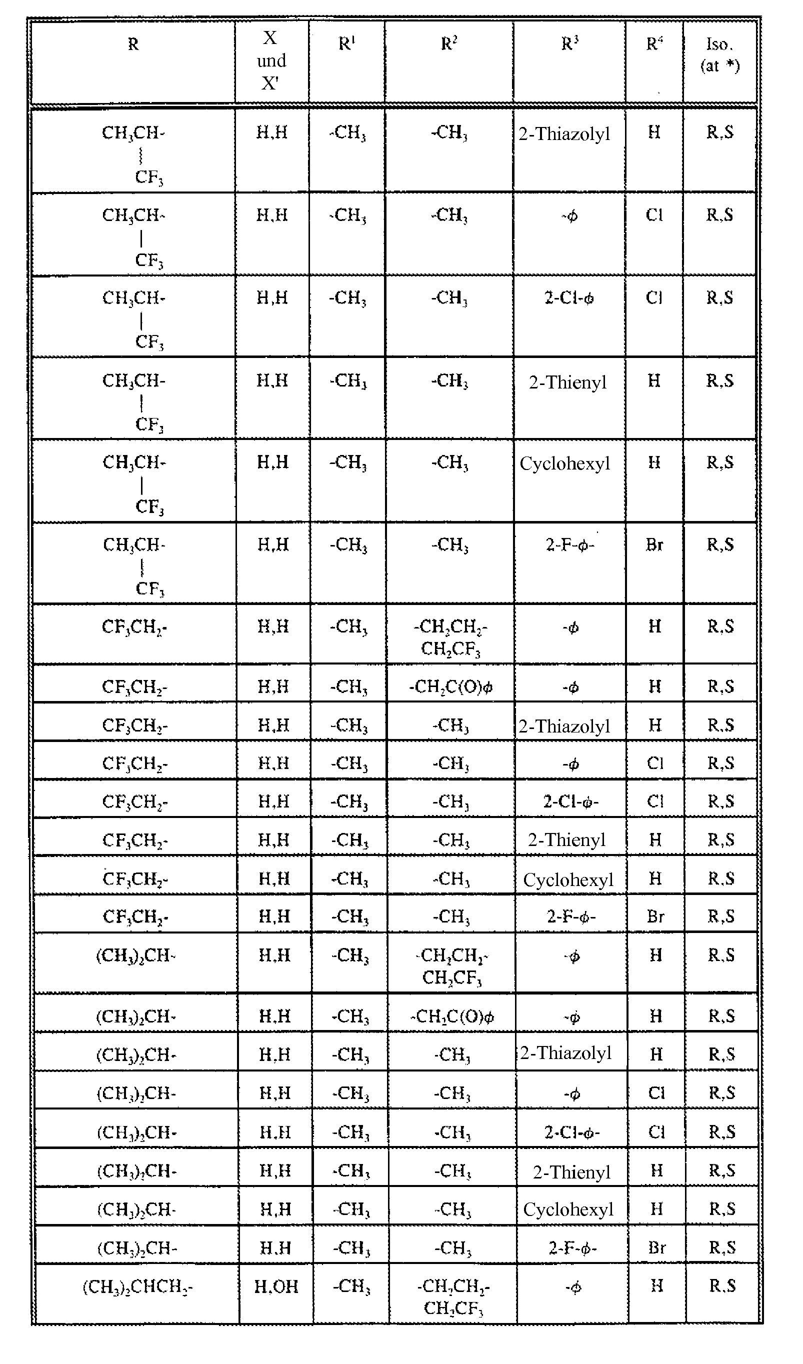

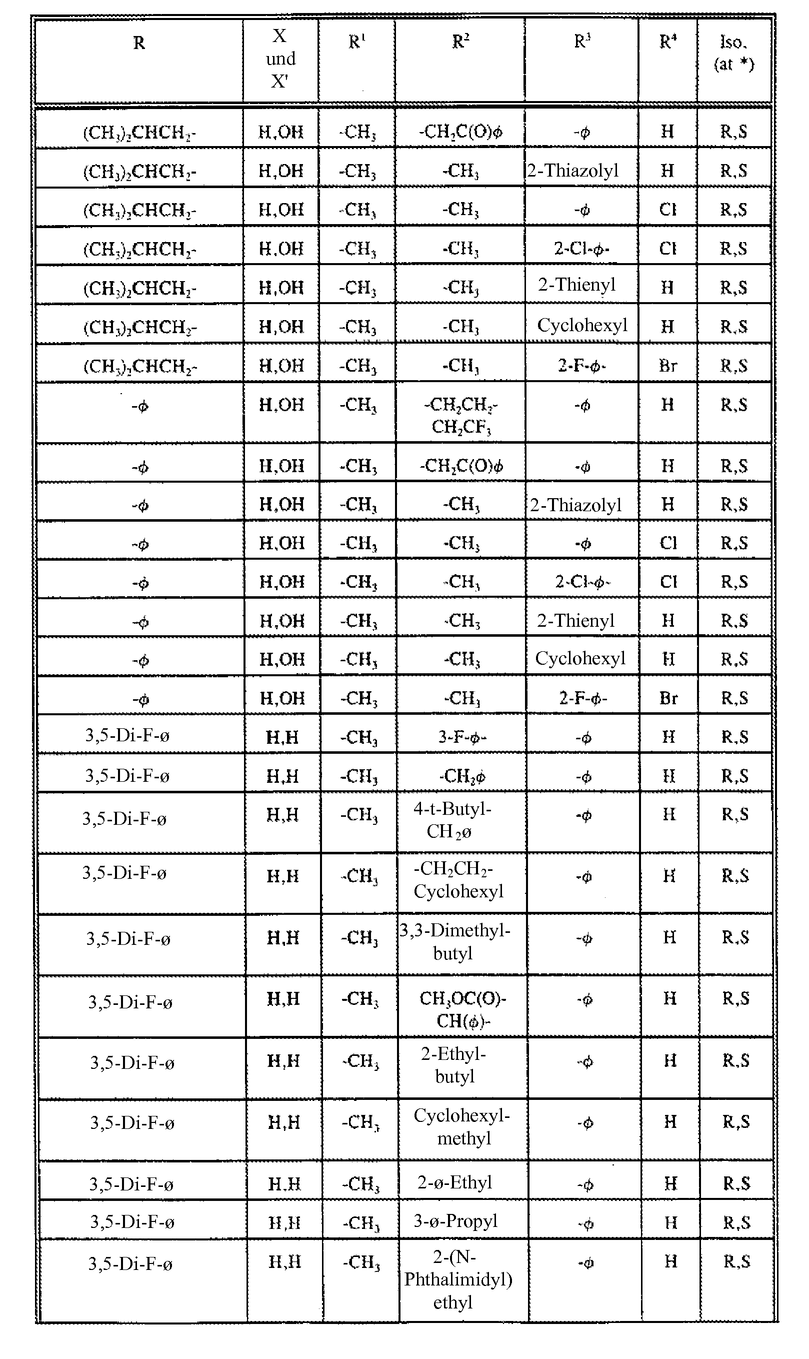

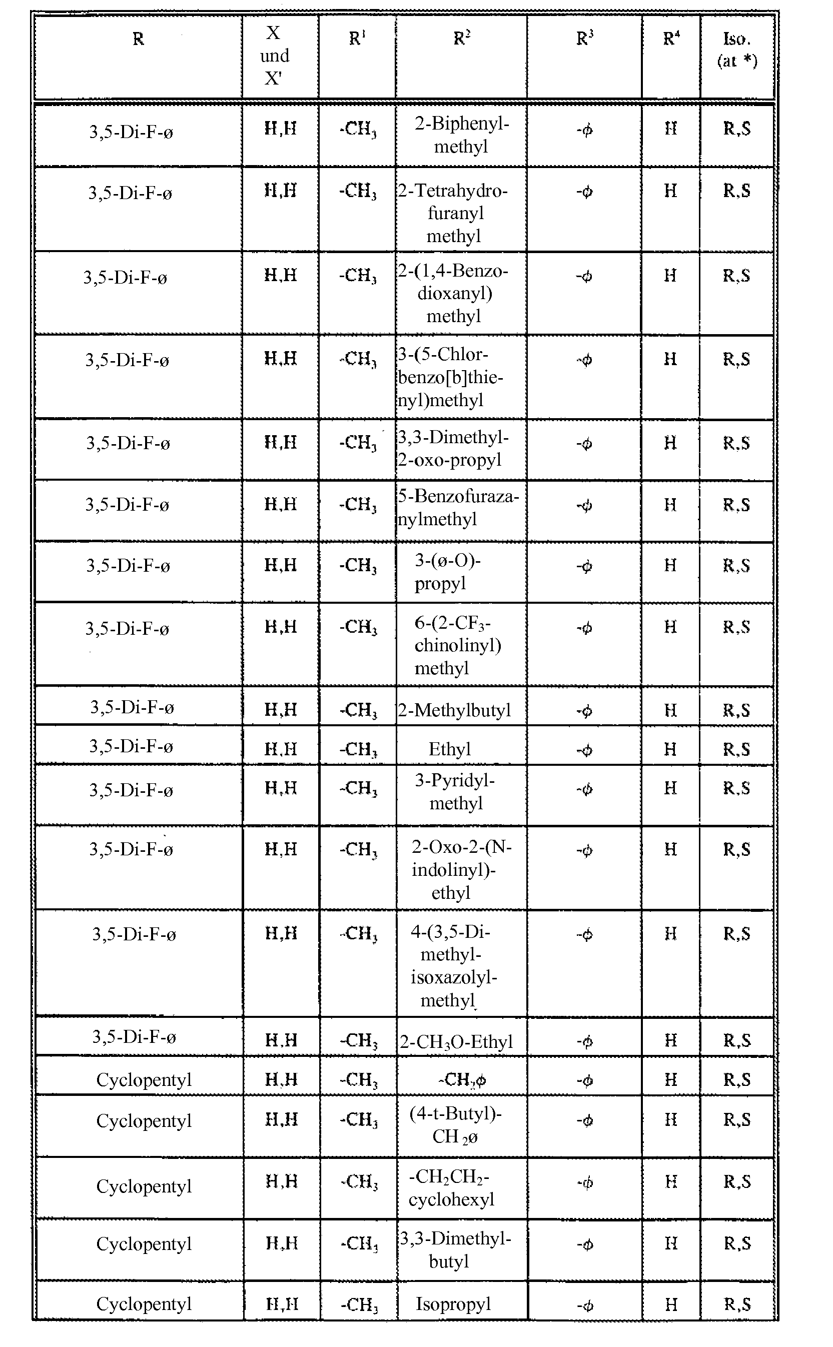

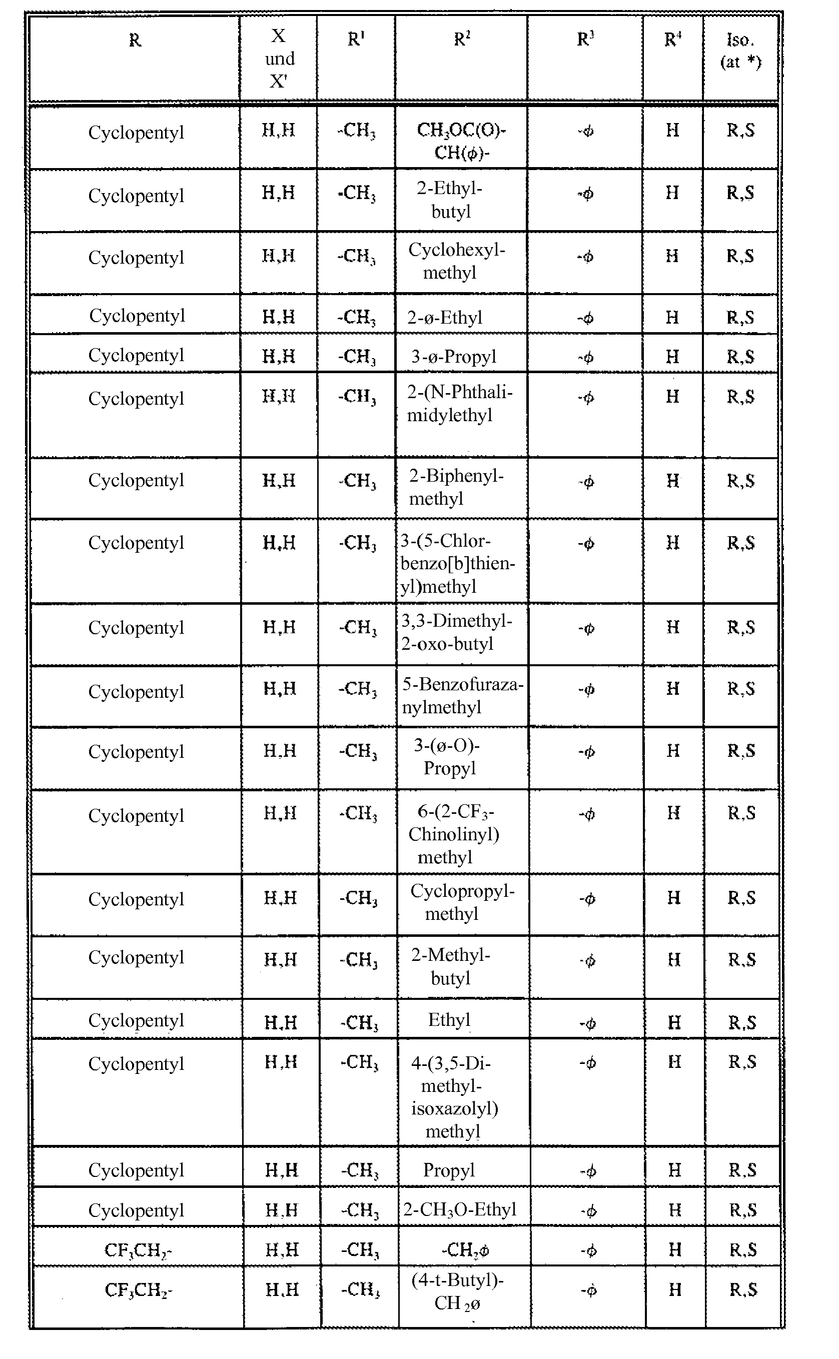

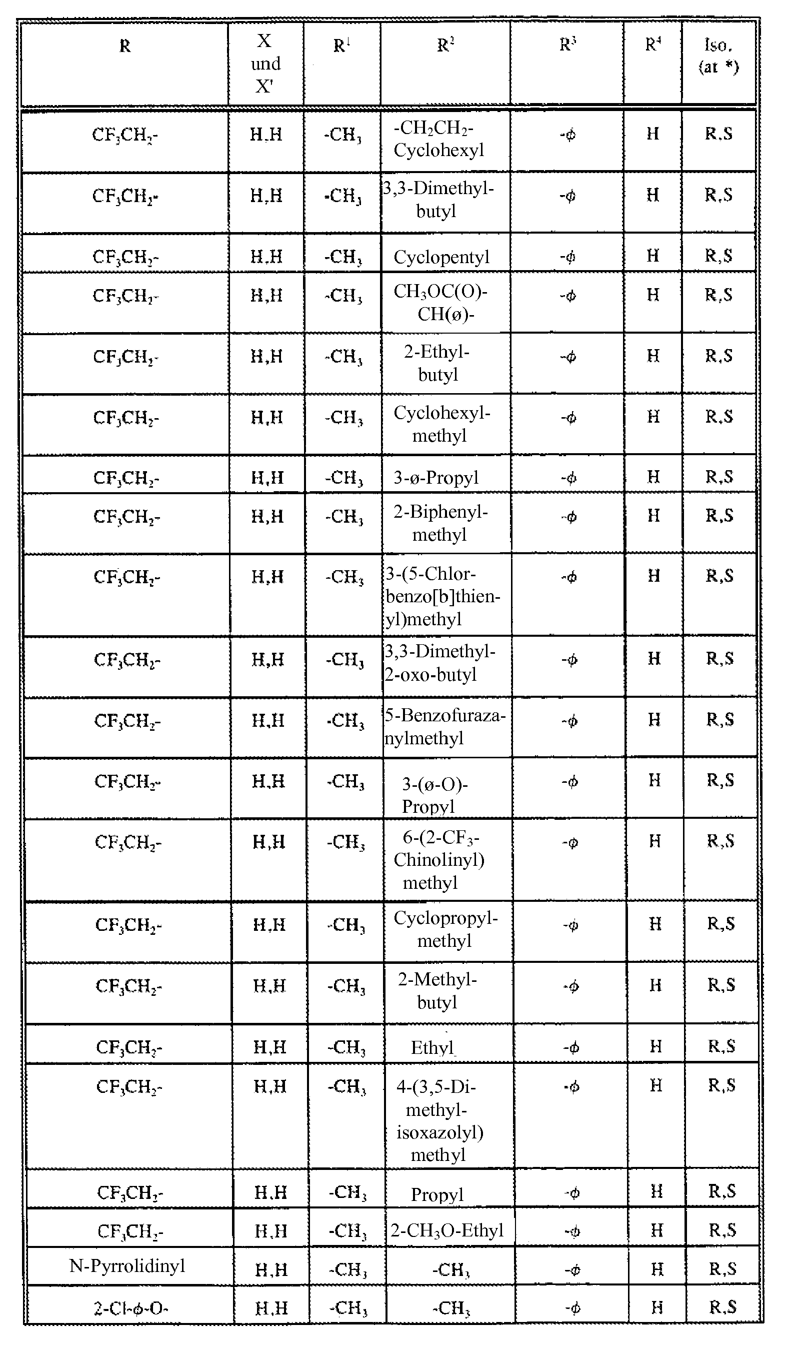

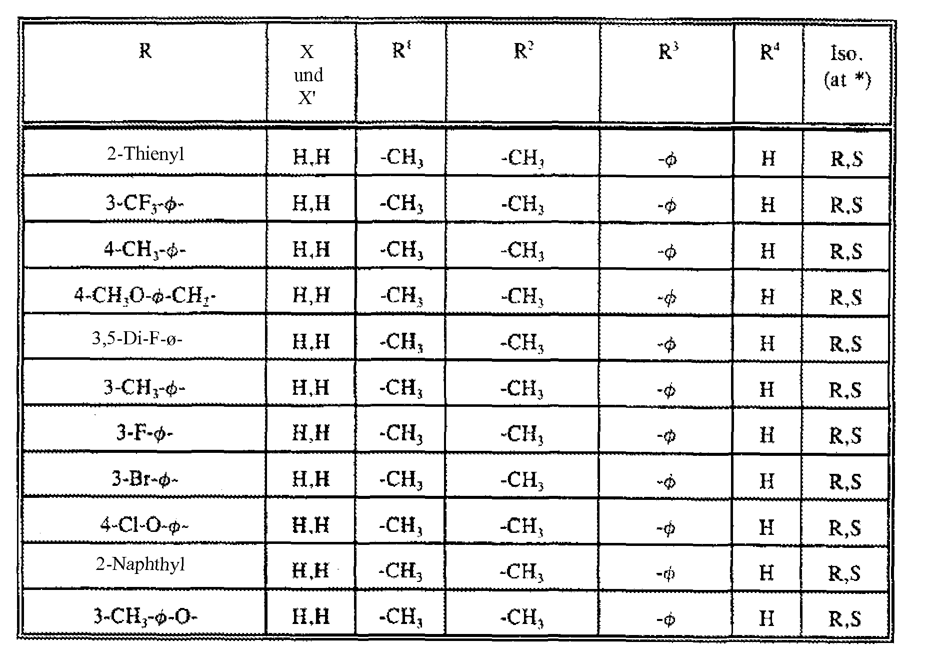

5. Pharmazeutische Zusammensetzung, umfassend einen pharmazeutisch inerten Träger und eine pharmazeutisch wirksame Menge einer Verbindung nach Anspruch 1 bis 4.

6. Verbindung nach Anspruch 1 bis 4 für die Verwendung in der Therapie.

7. Verbindung nach Anspruch 1 bis 4 für die Verwendung zur Behandlung von Alzheimer-Krankheit.

8. Verwendung einer Verbindung nach Anspruch 1 bis 4 zur Herstellung eines Arzneimittels für die Behandlung von Alzheimer-Krankheit.

## Revendications

1. Composé répondant à la formule 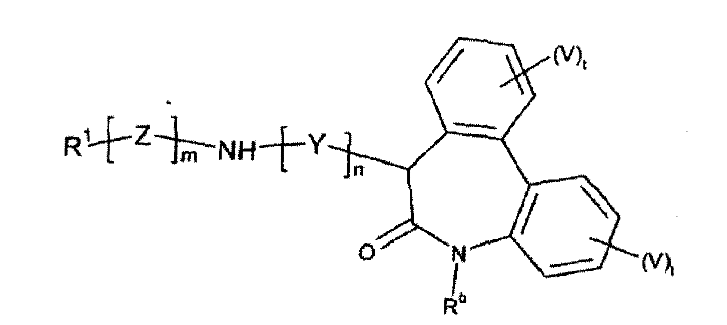
dans laquelle R¹ est choisi parmi le groupe constitué par un groupe alkyle, un groupe alcényle, un groupe alcynyle, un groupe cycloalkyle, un groupe cycloalcényle, un groupe aryle, un groupe hétéroaryle et un groupe hétérocyclique ;
Y répond à la formule : 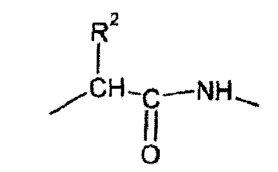
dans laquelle R² est choisi parmi le groupe constitué par un groupe méthyle, un groupe éthyle, un groupe n-propyle, un groupe iso-propyle, un groupe n-butyle, un groupe iso-butyle, un groupe sec-butyle, un groupe tert-butyle, un groupe -CH₂CH(CH₂CH₃)₂, un groupe 2-méthyl-n-butyle, un groupe 6-fluoro-n-hexyle, un groupe phényle, un groupe benzyle, un groupe cyclohexyle, un groupe cyclopentyle, un groupe cycloheptyle, un groupe allyle, un groupe iso-but-2-ényle, un groupe 3-méthylpentyle, un groupe -CH₂-cyclopropyle, un groupe -CH₂-cyclohexyle, un groupe -CH₂CH₂-cyclopropyle, un groupe -CH₂CH₂-cyclohexyle, un groupe -CH₂-indol-3-yle, un groupe p-(phényl)phényle, un groupe o-fluorophényle, un groupe m-fluorophényle, un groupe p-fluorophényle, un groupe m-méthoxyphényle, un groupe p-méthoxyphényle, un groupe phénéthyle, un groupe benzyle, un groupe m-hydroxybenzyle, un groupe p-hydroxybenzyle, un groupe p-nitrobenzyle, un groupe m-trifluorométhylphényle, un groupe p-(CH₃)₂NCH₂CH₂CH₂O-benzyle, un groupe p-(CH₃)₃COC(O)CH₂O-benzyle, un groupe p-(HOOCCH₂O)-benzyle, un groupe 2-aminopyrid-6-yle, un groupe p-(N-morpholino-CH₂CH₂O)-benzyle, un groupe -CH₂CH₂C(O)NH₂, un groupe -CH₂-imidazol-4-yle, un groupe -CH₂-(3-tétrahydrofuranyle), un groupe -CH₂-thiophén-2-yle, un groupe -CH₂(1-méthyl)cyclopropyle, un groupe -CH₂-thiophén-3-yle, un groupe thiophén-3-yle, un groupe thiophén-2-yle, un groupe -CH₂-C(O)O-*t-*butyle, un groupe -CH₂-C(CH₃)₃, un groupe -CH₂CH(CH₂CH₃)₂, un groupe 2-méthylcyclopentyle, un groupe cyclohex-2-ényle, un groupe -CH[CH(CH₃)₂]COOCH₃, un groupe -CH₂CH₂N(CH₃)₂, un groupe -CH₂C(CH₃)=CH₂, un groupe -CH₂CH=CHCH₃ (cis et trans), un groupe -CH₂OH, un groupe -CH(OH)CH₃, un groupe -CH(O-*t* butyl)CH₃, un groupe -CH₂OCH₃, un groupe -(CH₂)₄NH-Boc, un groupe -(CH₂)₄NH₂, un groupe -CH₂-pyridyle, un groupe pyridyle, un groupe -CH₂-naphtyle, un groupe -CH₂-(N-morpholino), un groupe p-(N-morpholino-CH₂CH₂O)-benzyle, un groupe benzo[b]thiophén-2-yle, un groupe 5-chlorobenzo[b]thiophén-2-yle, un groupe 4,5,6,7-tétrahydrobenzo[b]thiophén-2-yle, un groupe benzo[b]thiophén-3-yle, un groupe 5-chlorobenzo[b]thiophén-3-yle, un groupe benzo[b]thiophén-5-yle, un groupe 6-méthoxynapht-2-yle, un groupe -CH₂CH₂SCH₃, un groupe thién-2-yle, et un groupe thién-3-yle ;
Z répond à la formule -T-CX'X"C(O)- où T est choisi parmi le groupe constitué par une liaison reliant de manière covalente R¹ au groupe -CX'X"-, un atome d'oxygène, un atome de soufre, un groupe -NR⁵ où R⁵ représente un atome d'hydrogène, un groupe acyle, un groupe alkyle, un groupe aryle ou un groupe hétéroaryle ;
X' représente un atome d'hydrogène, un groupe hydroxyle ou un groupe fluoro
X" représente un atome d'hydrogène, un groupe hydroxyle ou un groupe fluoro ; ou bien X' et X" forment ensemble un groupe oxo ;
m représente un entier égal à 0 ou 1 ;
n représente un entier égal à 0, 1 ou 2 ;
le terme « groupe alkyle » désignant des groupes alkyle monovalents possédant de préférence de 1 à 10 atomes de carbone ;
le terme « groupe alkaryle » désignant des groupes alkylène-aryle possédant de préfèrence de 1 à 8 atomes de carbone dans le radical alkylène et de 1 à 10 atomes de carbone dans le radical aryle ;
le terme « groupe alcoxy » désignant le groupe « alkyl-O- » ;
le terme « groupe alcényle » désignant des groupes alcényle possédant de préférence de 2 à 10 atomes de carbone ;
le terme « groupe alcynyle » désignant des groupes alcynyle possédant de préférence de 2 à 10 atomes de carbone ;
le terme « groupe aryle désignant un groupe phényle ou un groupe naphtyle, ledit groupe aryle pouvant de manière facultative être substitué avec un nombre de 1 à 5 substituants choisis parmi le groupe constitué par un groupe alkyle, un groupe alcoxy, un atome d'halogène, un groupe cyano, un groupe nitro, un groupe trihalogénométhyle et un groupe thioalcoxy ;
le terme « groupe aryloxy » désignant le groupe « aryl-O-» dans lequel le groupe aryle est tel que défini ci-dessus, y compris des groupes aryle facultativement substitués ;
le terme "groupe carboxyalkyle" désignant le groupe « -C(O)O-alkyle » dans lequel le groupe alkyle est tel que défini ci-dessus ;
le terme « groupe cycloalkyle » désignant des groupes alkyle cycliques contenant de 3 à 12 atomes de carbone, possédant un noyau cyclique unique ou de multiples noyaux condensés ;
le terme « groupe cycloalcényle » désignant des groupes alcényle cycliques contenant de 4 à 8 atomes de carbone, possédant un noyau cyclique unique et au moins un point d'insaturation interne ;
le terme « groupe hétéroaryle » désignant un groupe carbocyclique aromatique contenant de 1 à 15 atomes de carbone et de 1 à 4 hétéroatomes choisis parmi un atome d'oxygène, un atome d'azote et un atome de soufre, au sein d'au moins un noyau ;
le terme « groupe hétérocyclique désignant un groupe saturé ou insaturé monovalent possédant un noyau unique ou de multiples noyaux condensés, de 1 à 15 atomes de carbone et de 1 à 4 hétéroatomes choisis parmi un atome d'azote, un atome de soufre ou un atome d'oxygène au sein du noyau ;
le terme « groupe thioalcoxy » désignant le groupe « -S-alkyle » ;
chaque V étant choisi de manière indépendante parmi le groupe constitué par un groupe hydroxyle, un groupe acyle, un groupe acyloxy, un groupe alkyle, un groupe alcoxy, un groupe alcényle, un groupe alcynyle, un groupe amino, un groupe aminoacyle, un groupe alkaryle, un groupe aryle, un groupe aryloxy, un groupe carboxyle, un groupe carboxylalkyle, un groupe cyano, un atome d'halogène, un groupe nitro, un groupe hétéroaryle, un groupe thioalcoxy et un groupe trihalogénométhyle ;
R^{b} est choisi parmi le groupe constitué par un atome d'hydrogène, un groupe alkyle, un groupe alcényle, un groupe alcynyle, un groupe acyle, un groupe aryle, un groupe hétéroaryle, et un groupe hétérocyclique ;
t représente un entier de 0 à 4.

2. Composé répondant à la formule 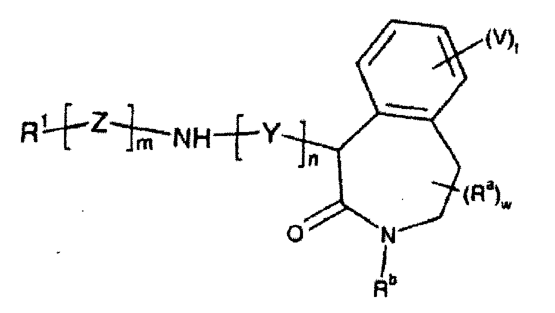
dans laquelle R¹, Z, Y, *m*, n, V, R^{b} et t ont les valeurs telles que définies à la revendication 1 ; et
dans laquelle R^{a} est choisi parmi le groupe constitué par un groupe alkyle, un groupe alcoxy, un groupe amino, un groupe carboxyle, un groupe carboxyalkyle, un groupe cyano et un atome d'halogène ;
w représente un entier de 0 à 3 ; et
dans laquelle les termes « groupe alkyle », « groupe alcoxy » et « groupe carboxyalkyle » ont la signification définie à la revendication 1.

3. Composé répondant à la formule I : 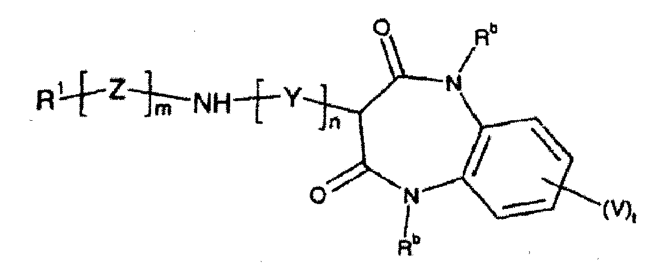 dans laquelle R¹, Z, Y, *m*, *n*, V, R^{b} et t ont les valeurs telles que définies à la revendication 1.

4. Composé choisi parmi le groupe constitué par :
- des composés répondant à la formule : 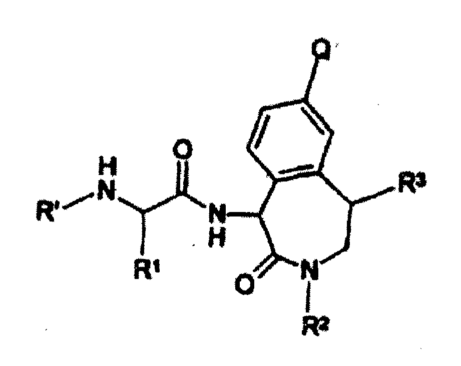
dans laquelle R', R¹, R², R³ et Q ont les valeurs définies dans le tableau ci-dessous : 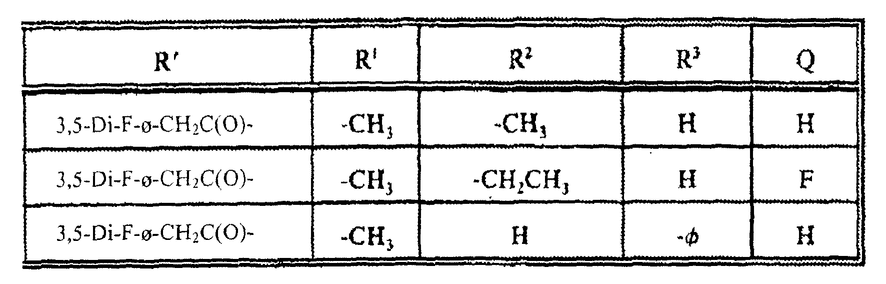
- des composés répondant à la formule : 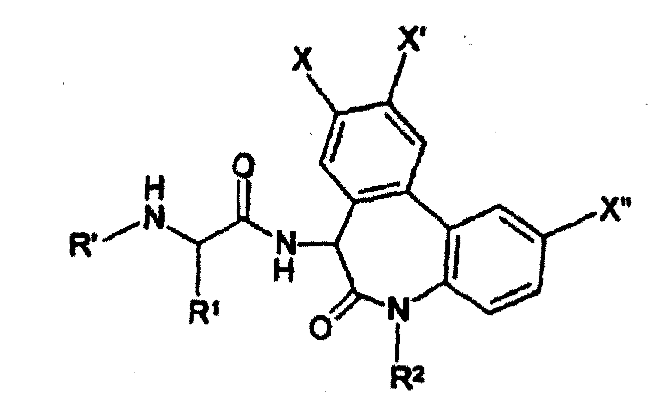
dans laquelle R', R¹, R², X, X' et X" ont les valeurs définies dans les tableaux ci-dessous : 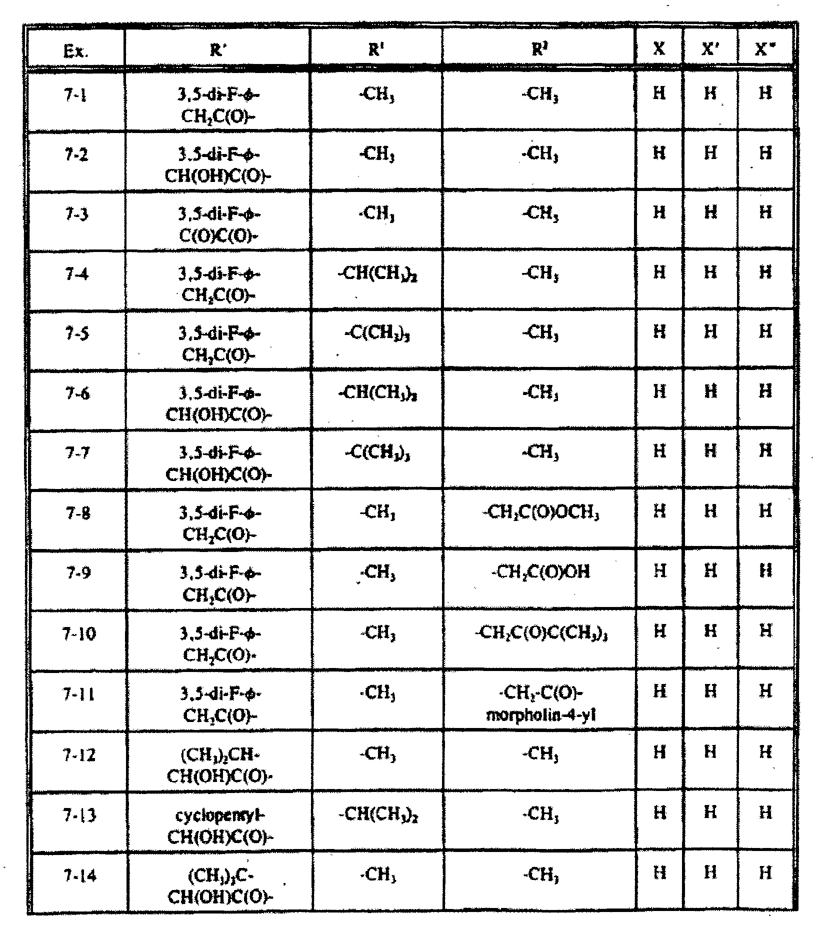 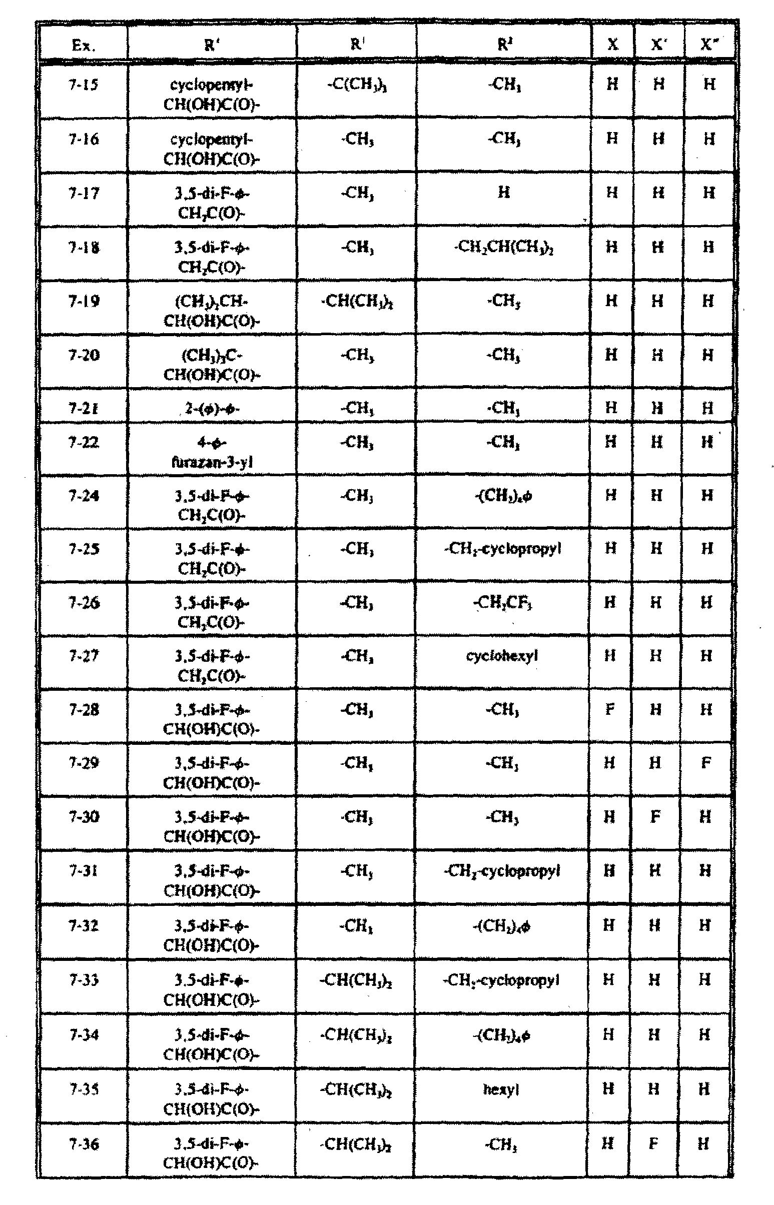 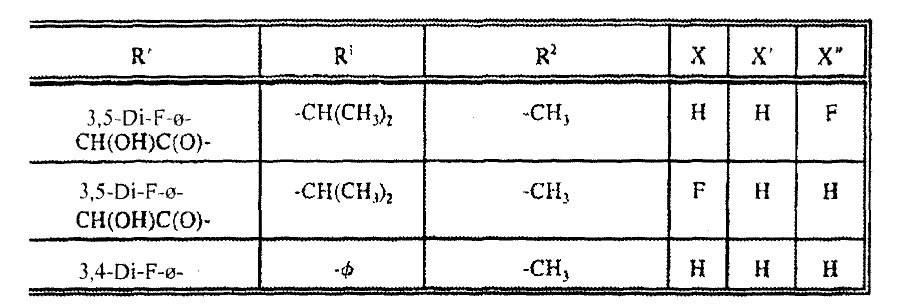
- des composés répondant à la formule : 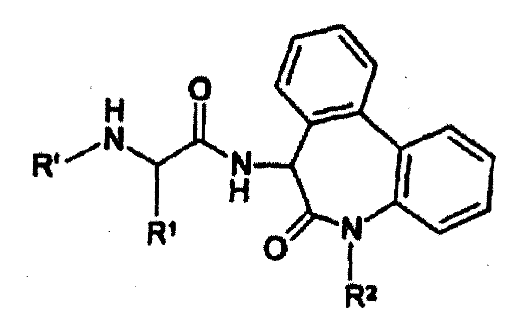
dans laquelle R', R¹ et R² ont les valeurs définies dans les tableaux ci-dessous : 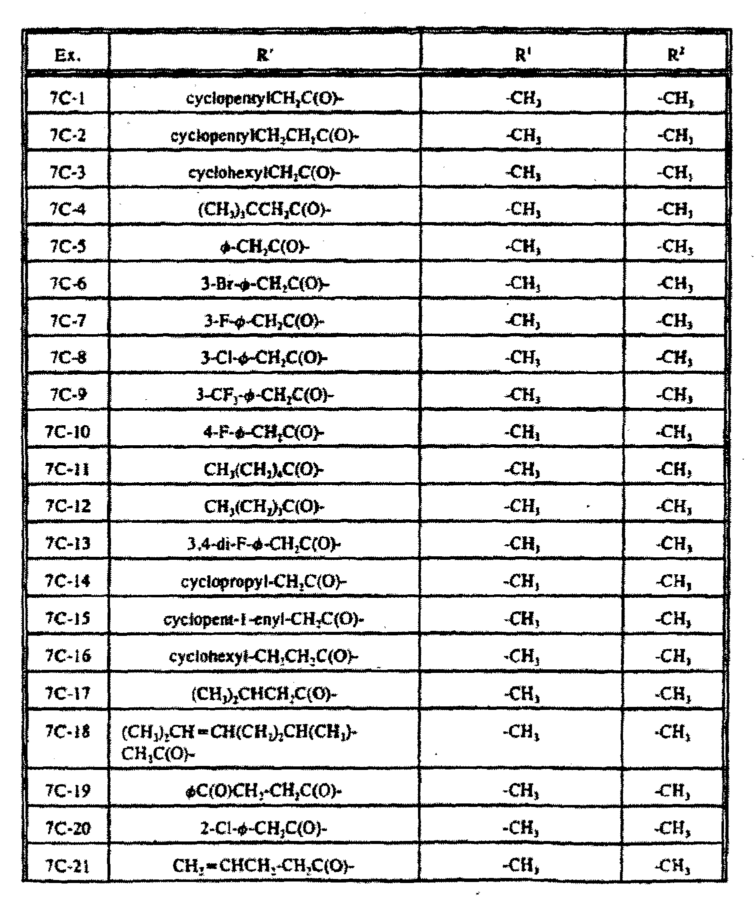 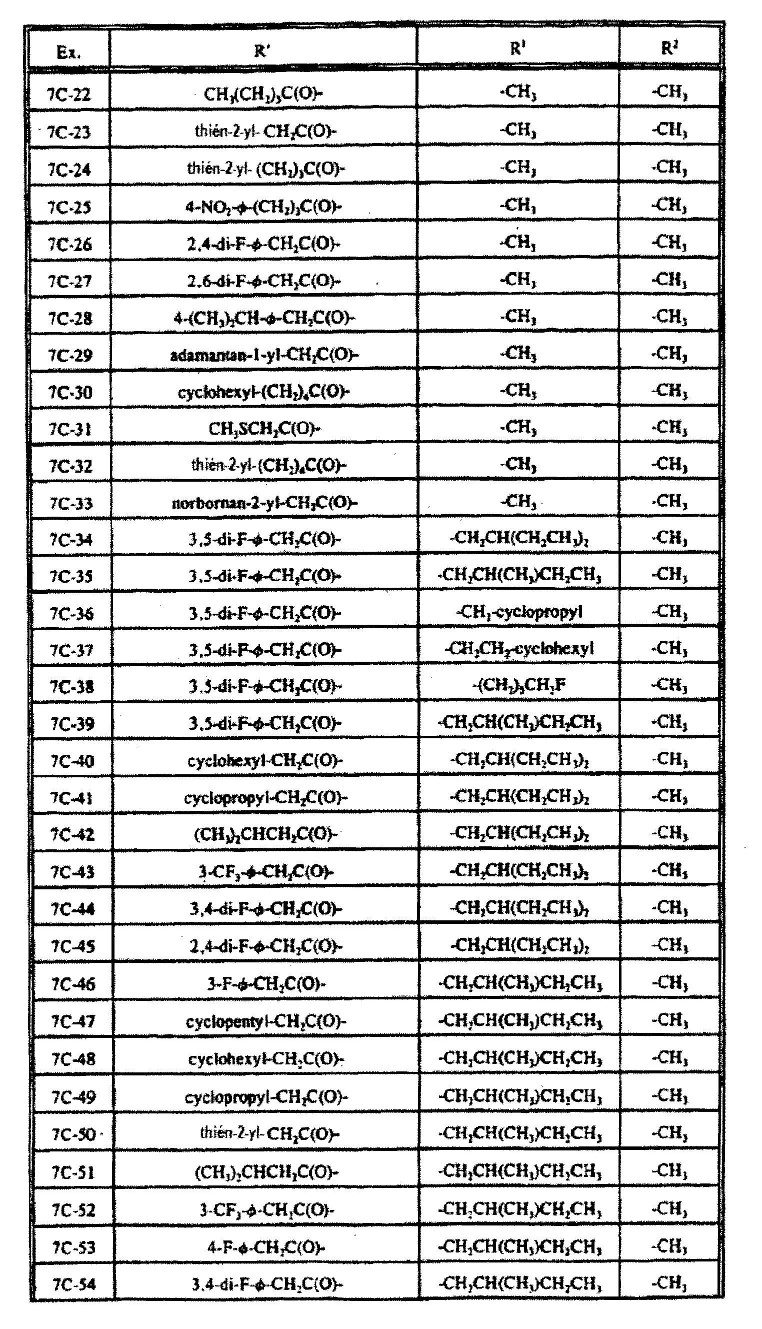 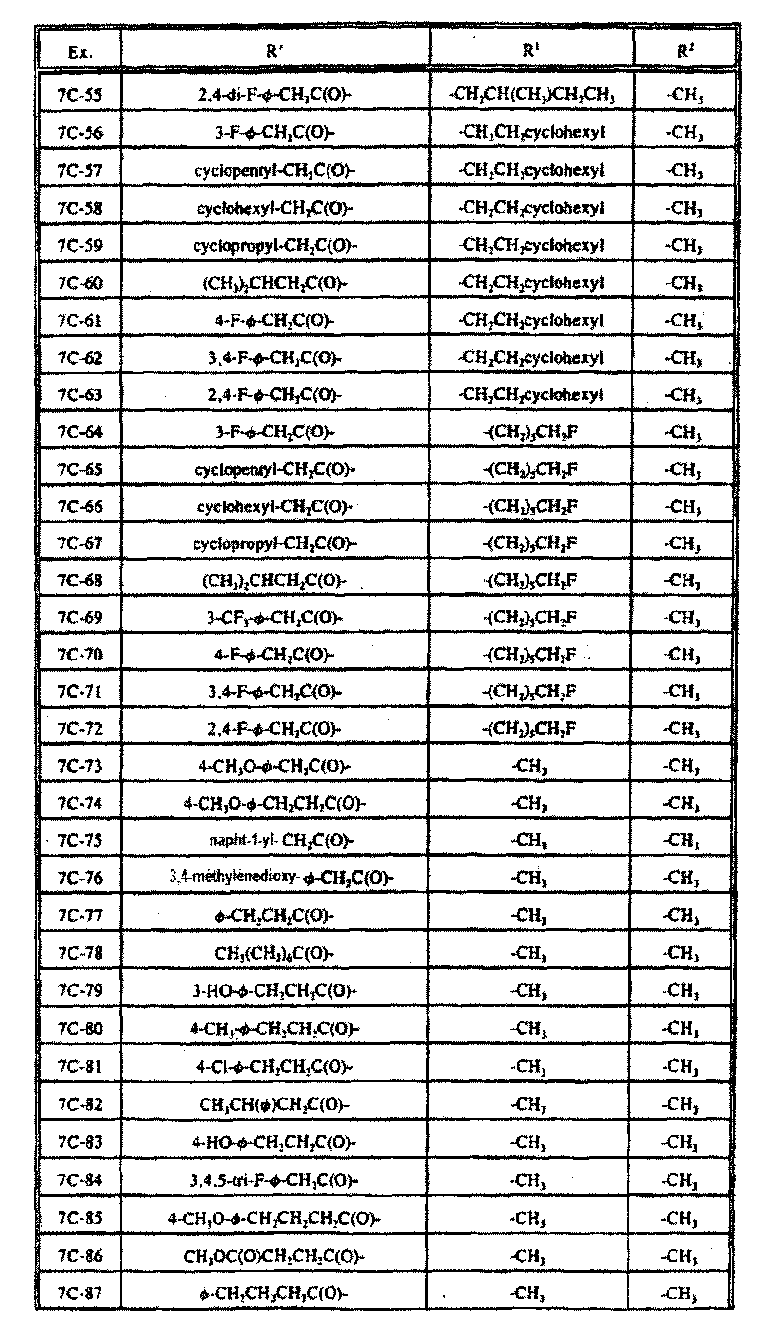 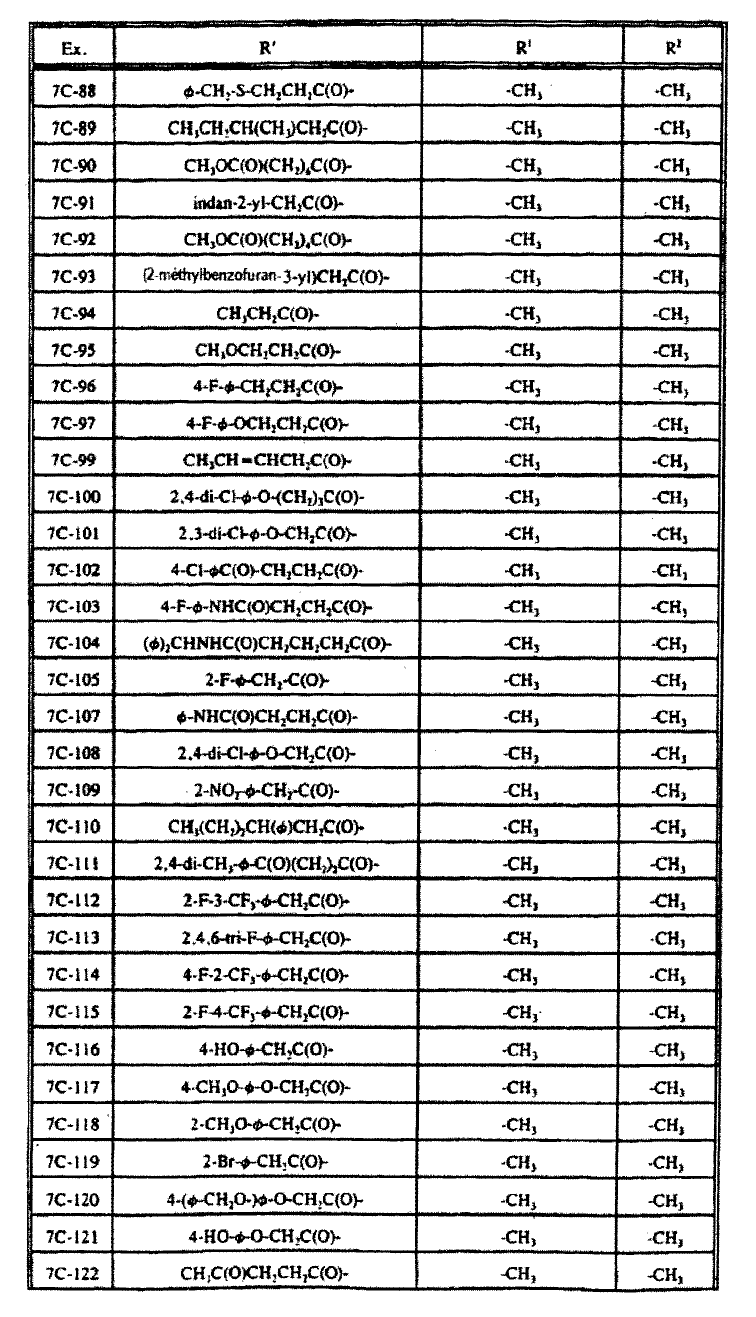 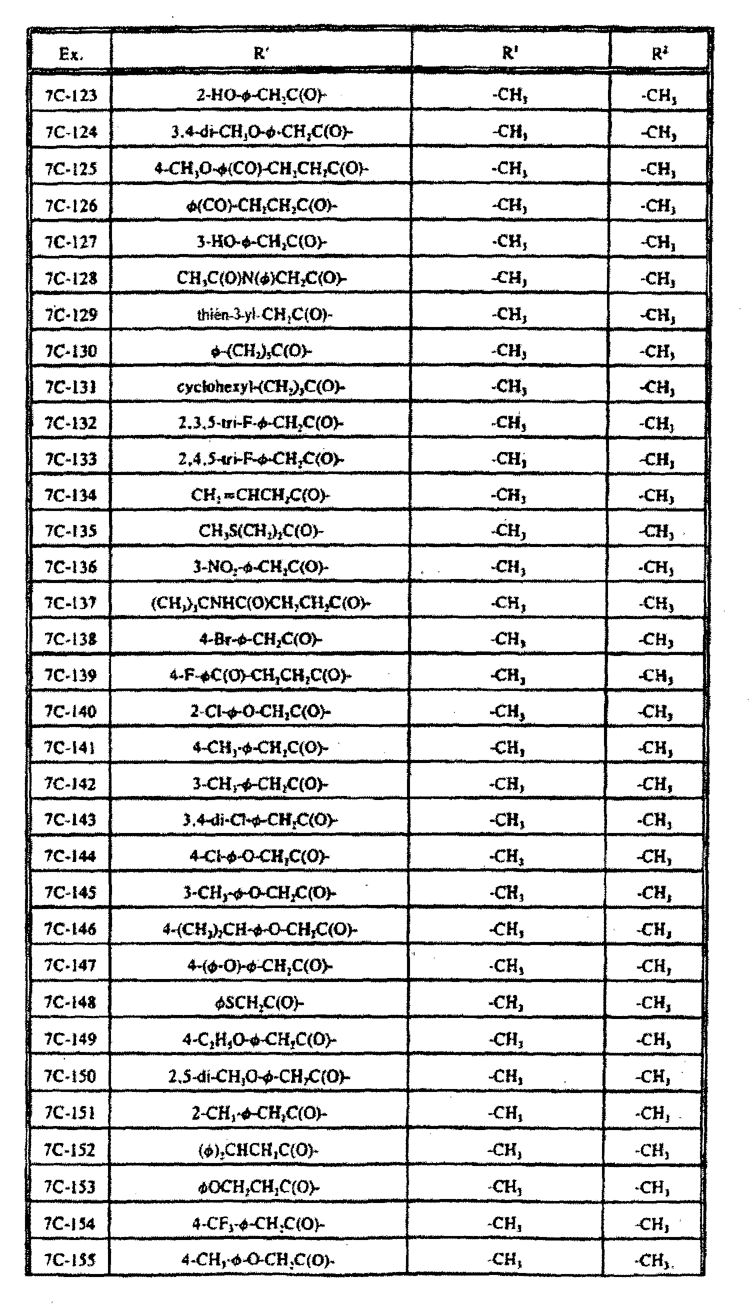 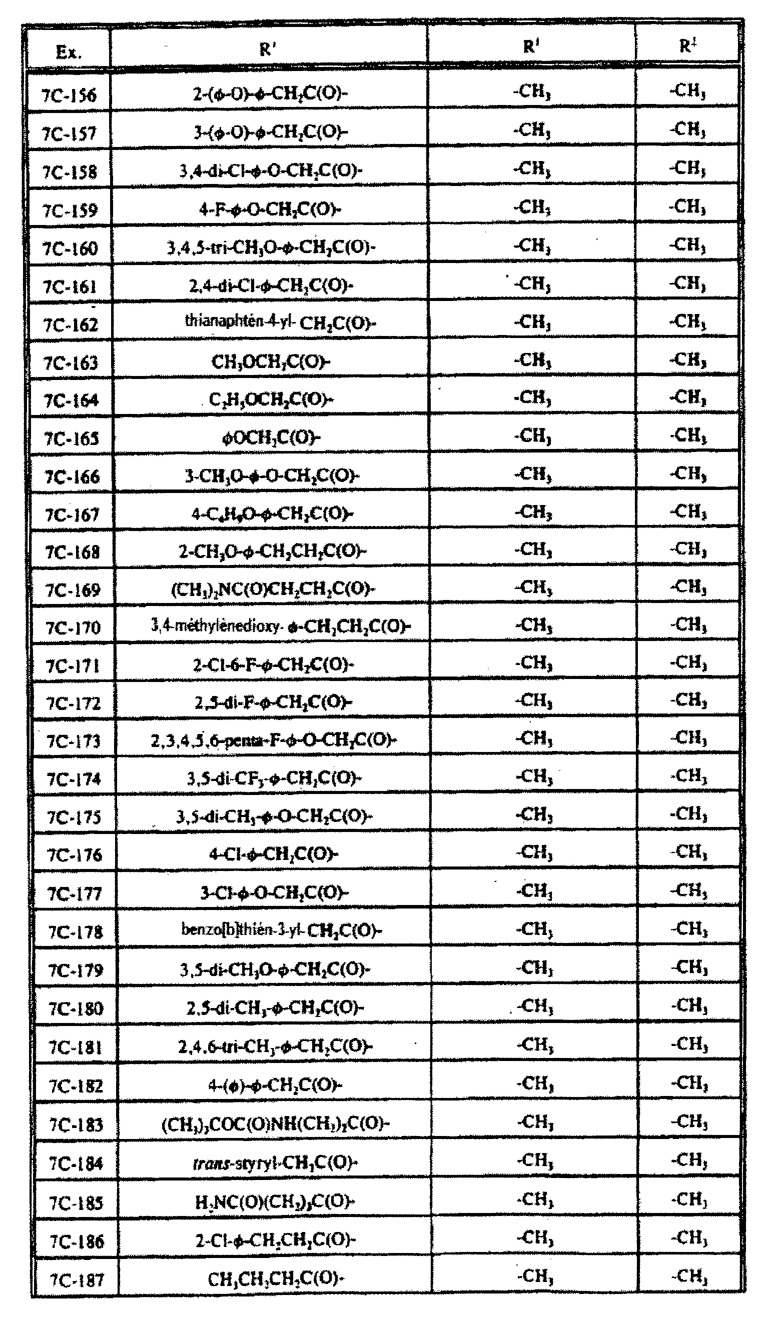 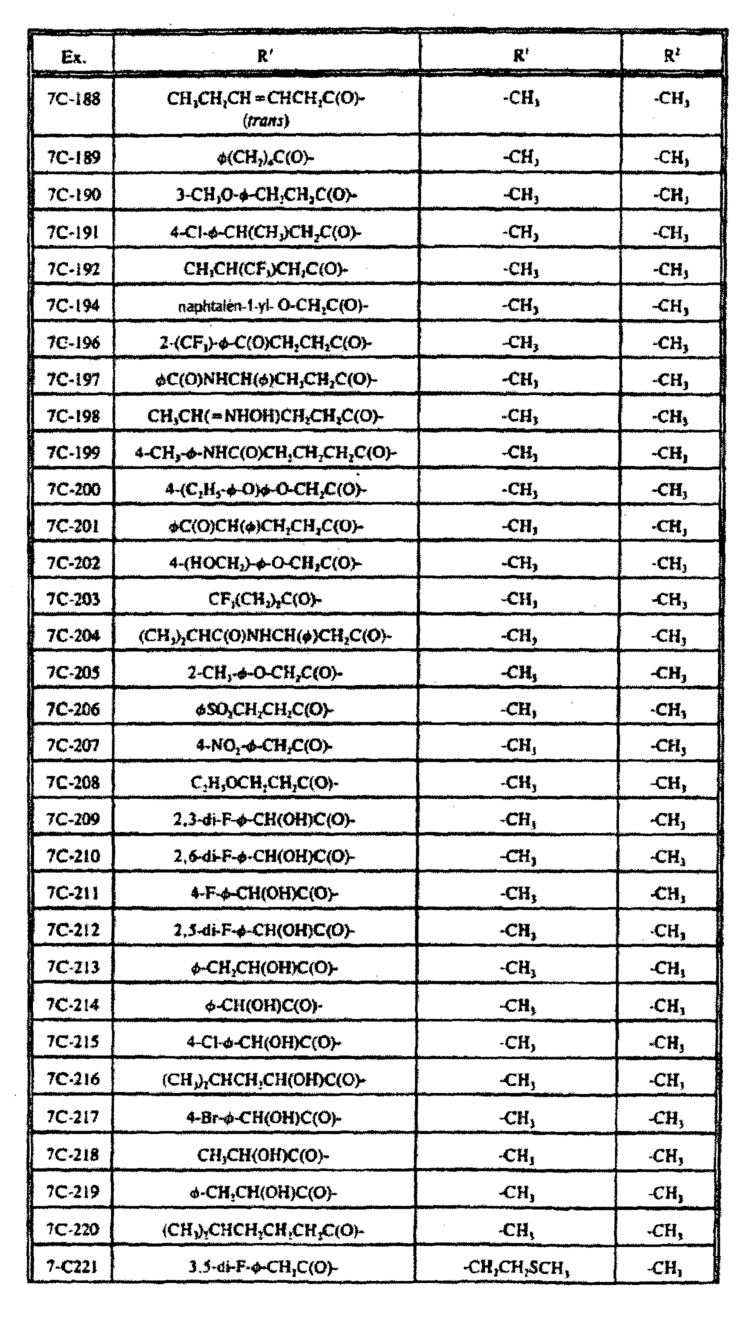 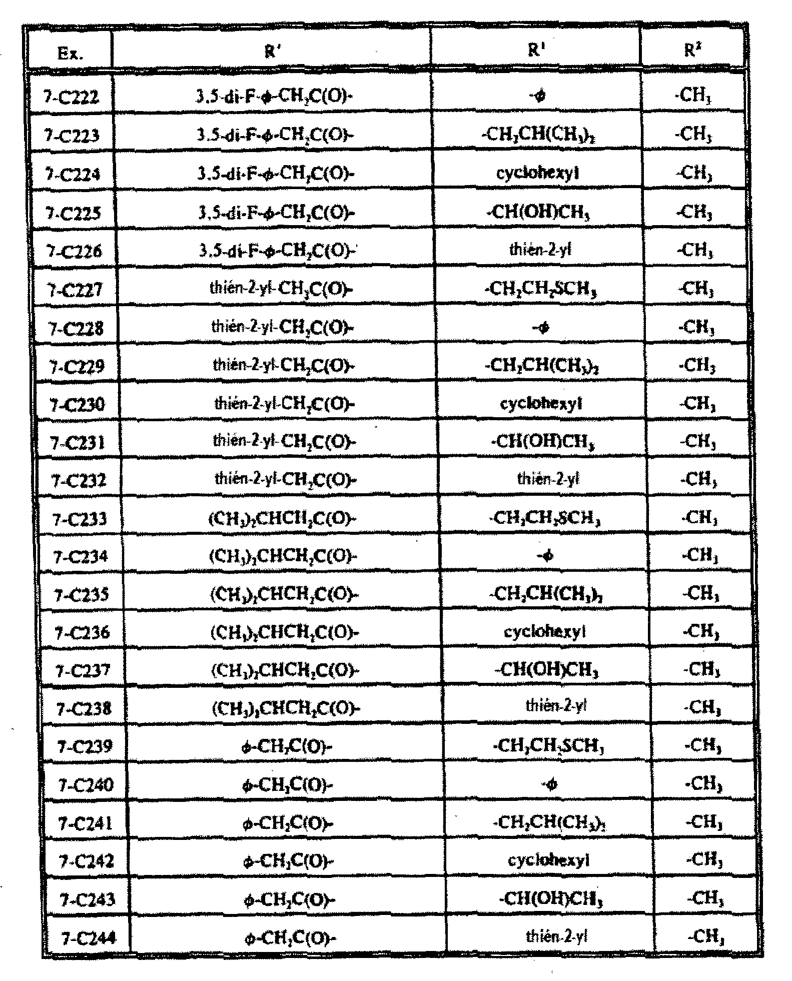
- des composés répondant à la formule : 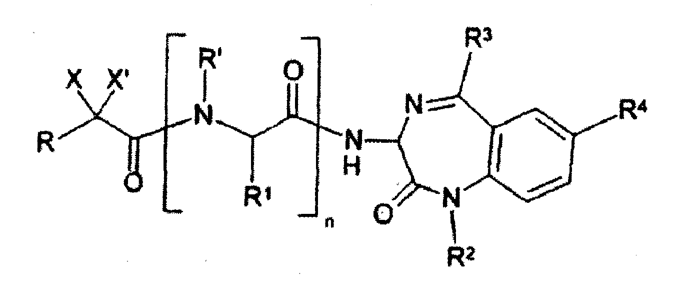
dans laquelle R, R', R¹ à R⁴, X, X' et n ont les valeurs définies dans les tableaux ci-dessous : 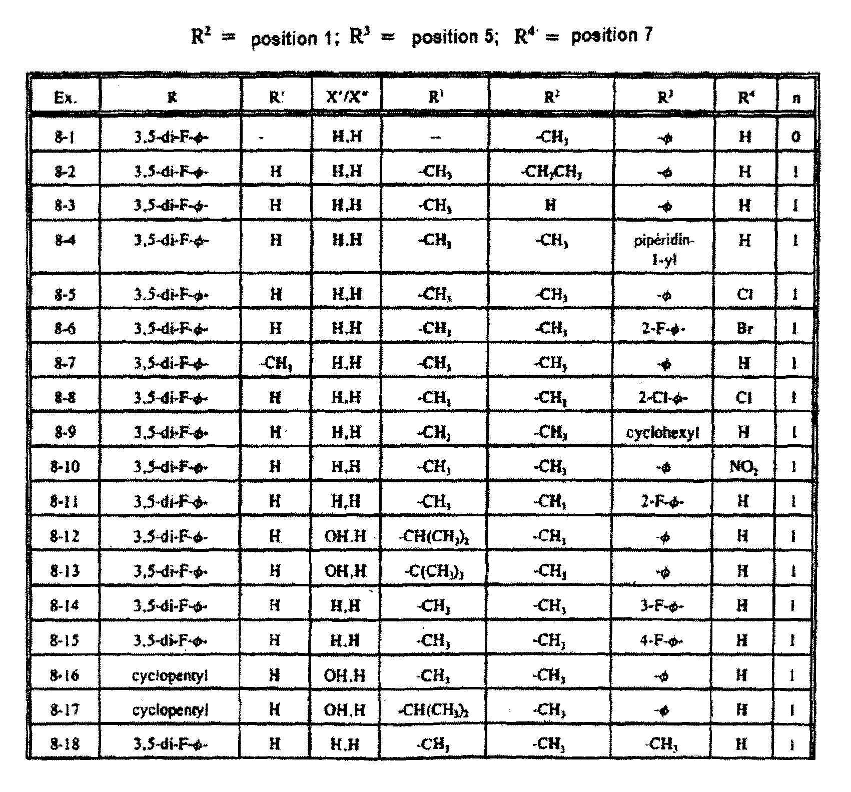 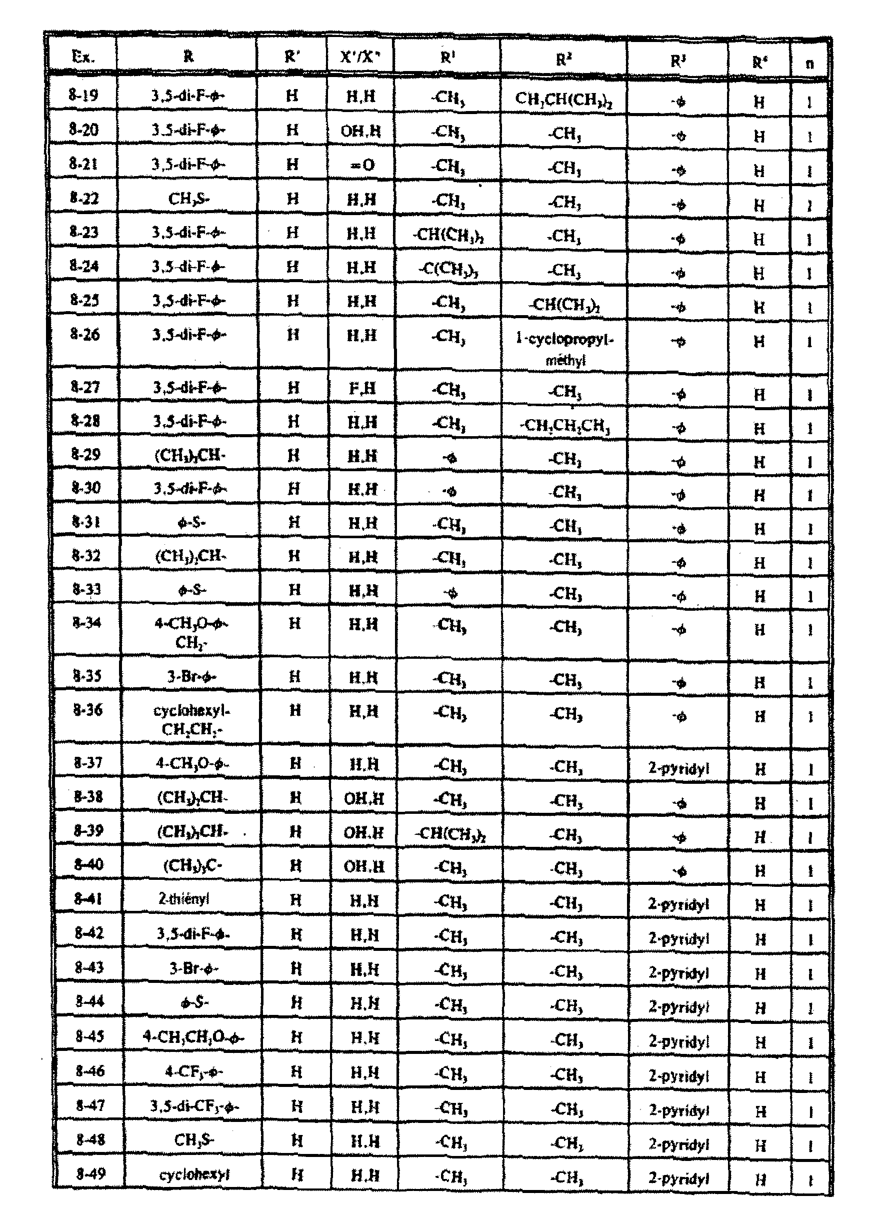 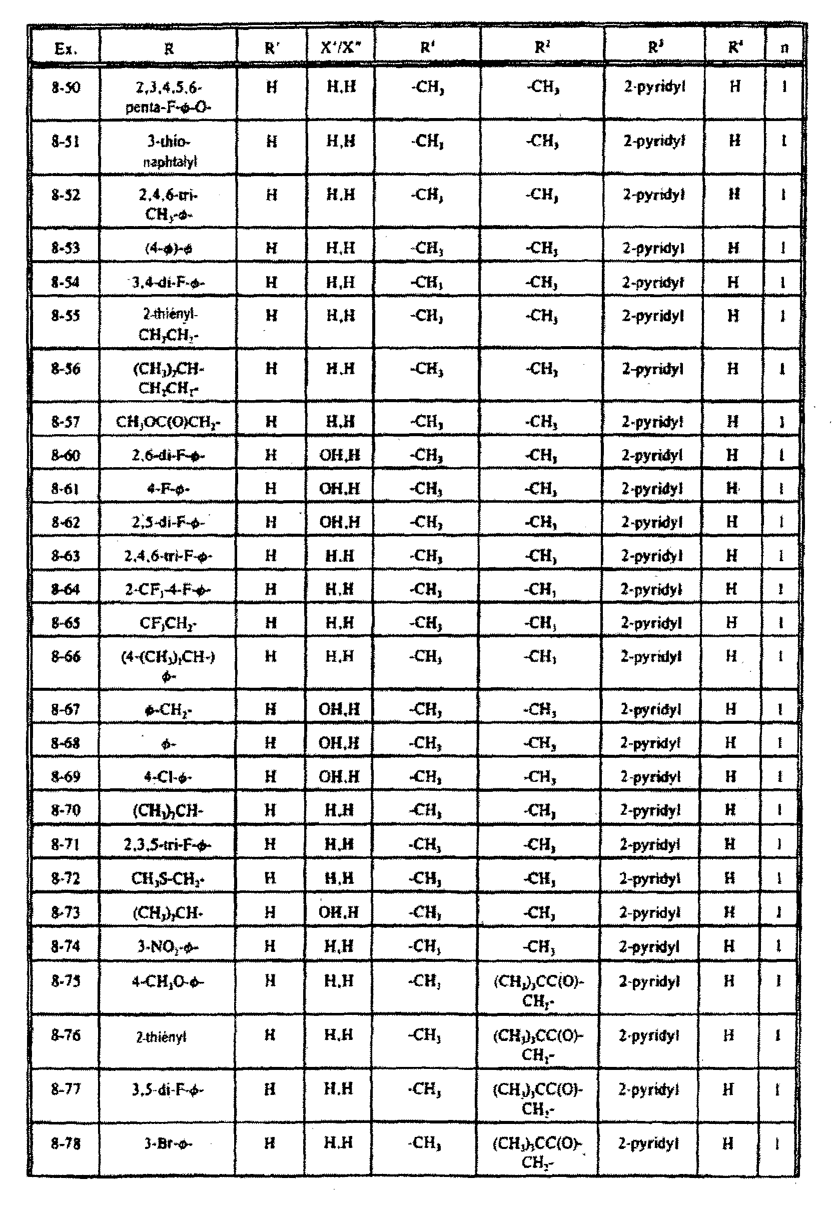 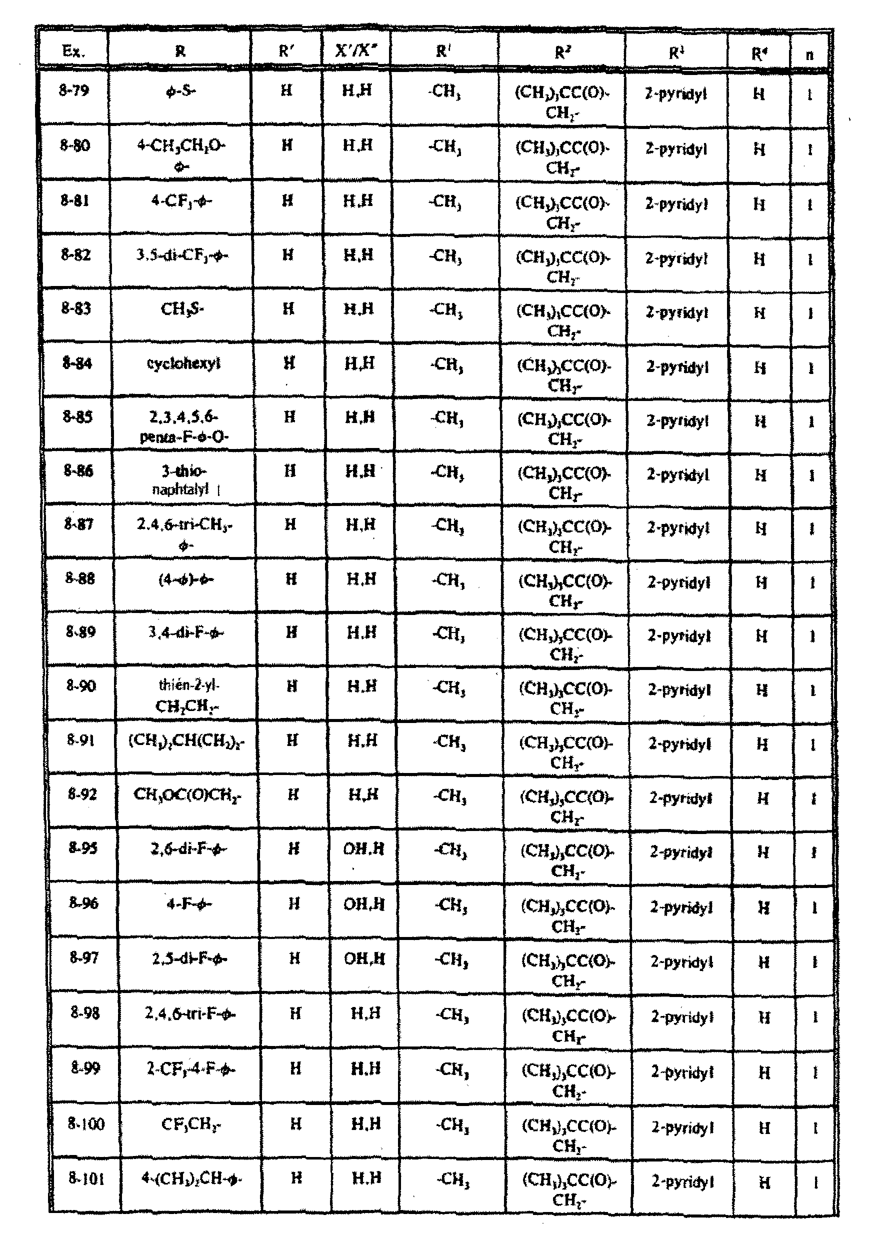 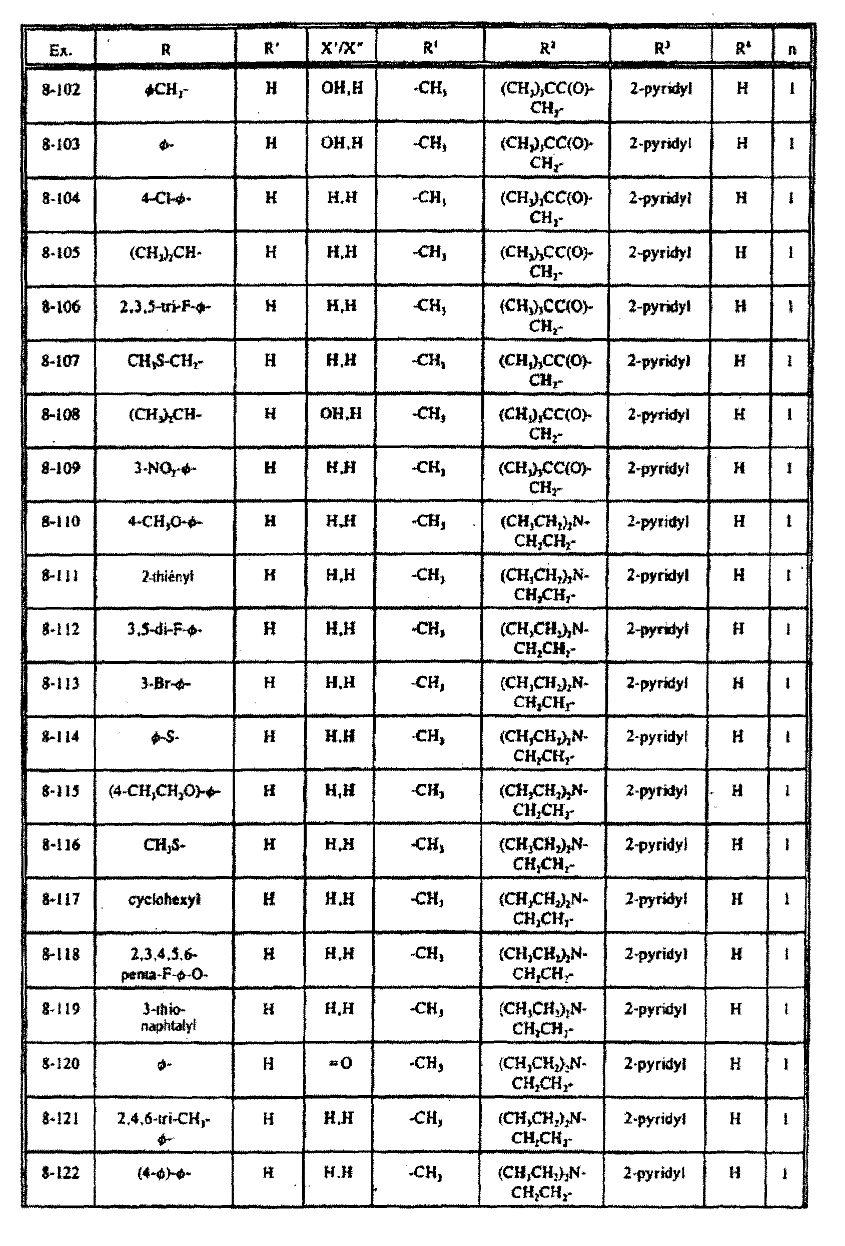 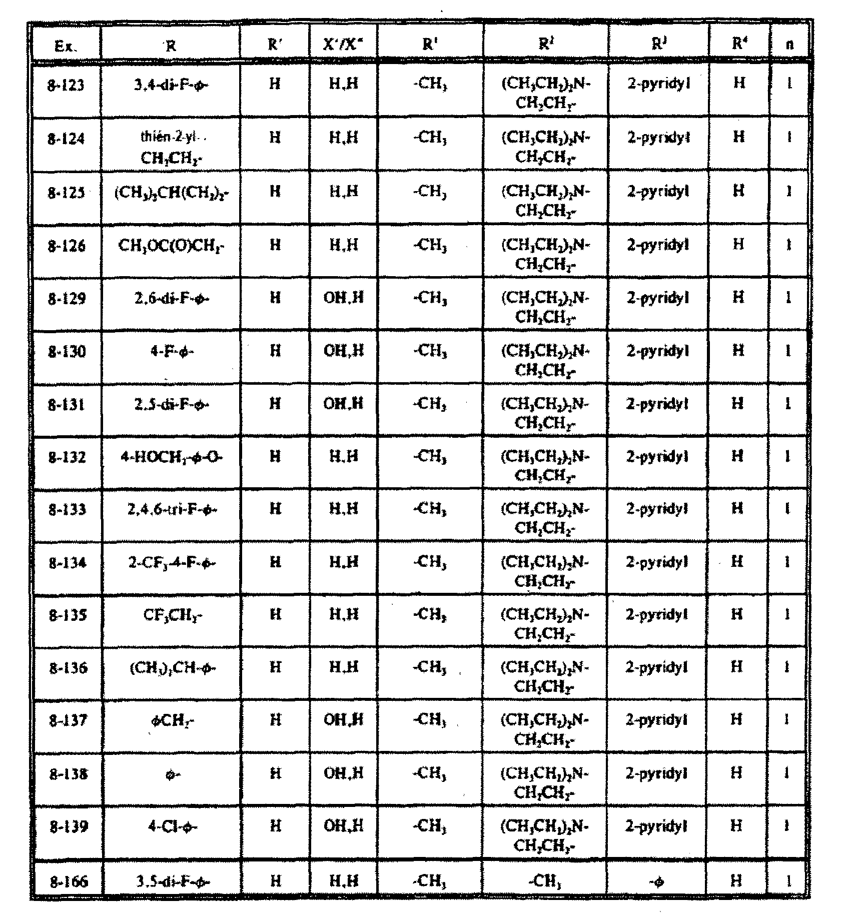
- des composés répondant à la formule : 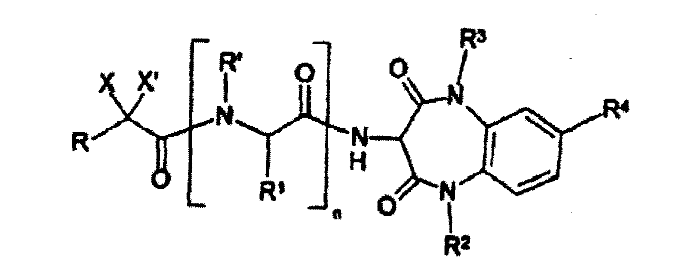
dans laquelle R, X, X', R', R¹ à R⁴ et n ont les valeurs définies dans les tableaux ci-dessous : 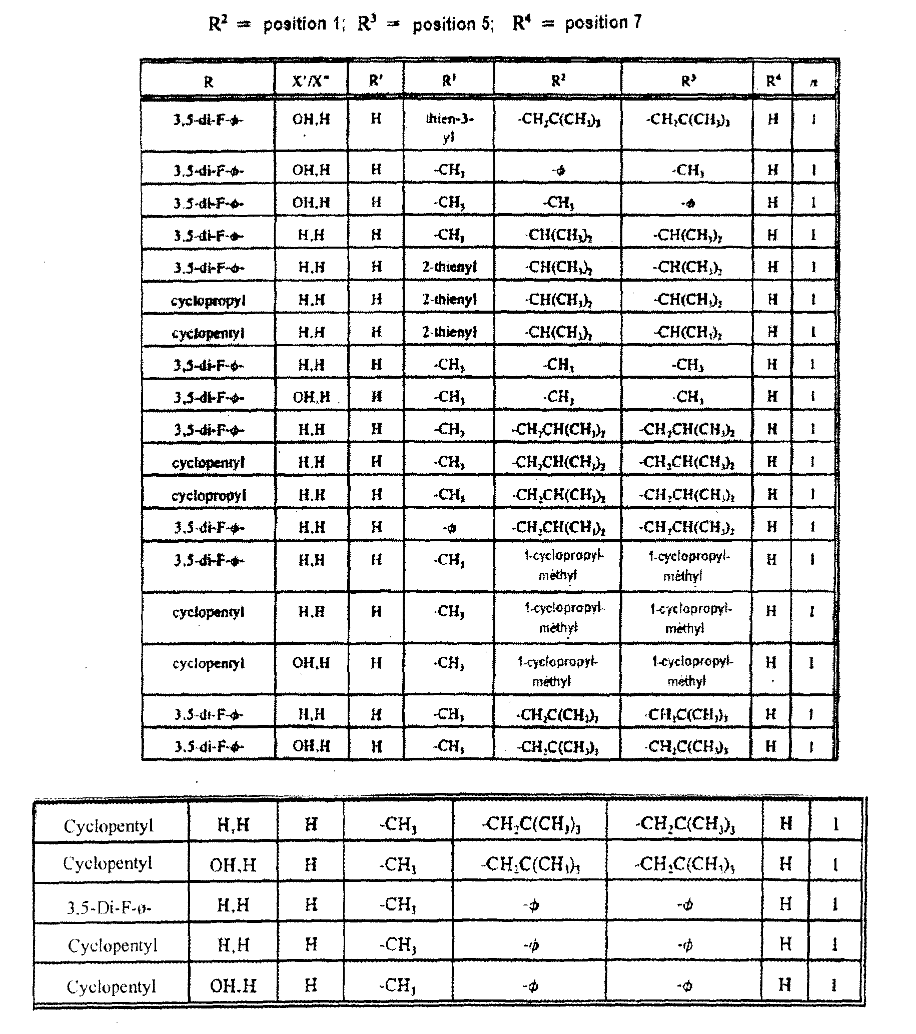
- et des composés répondant à la formule : 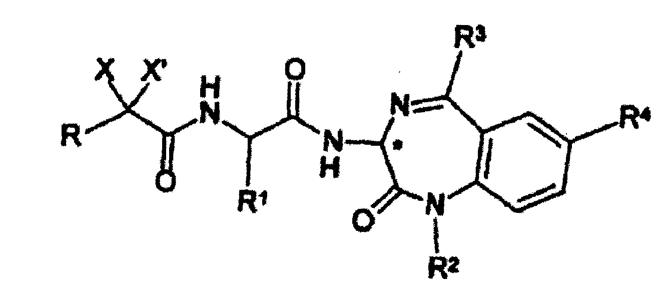
dans laquelle R, R¹ à R⁴, X et X' ont les valeurs définies dans les tableaux ci-dessous :

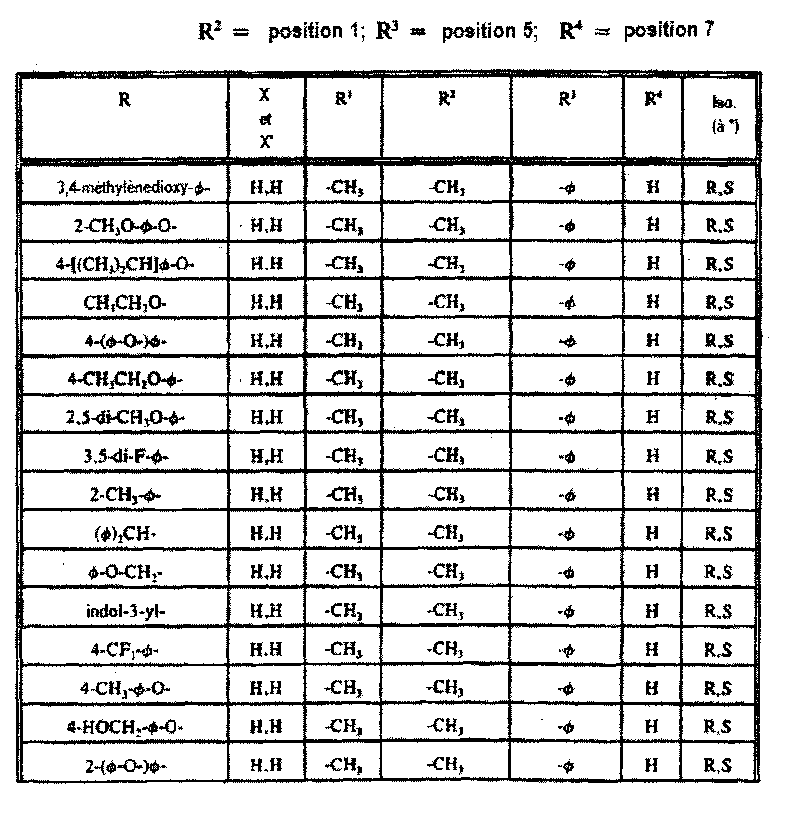

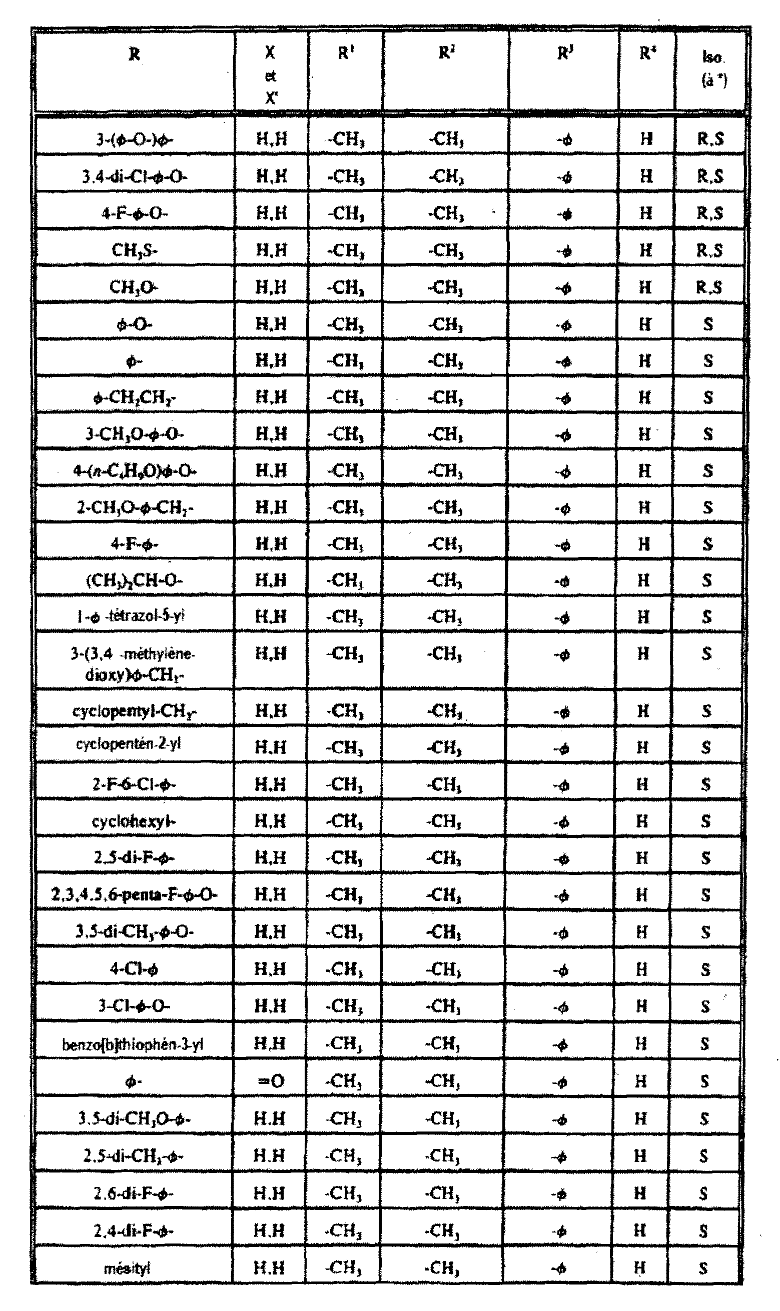

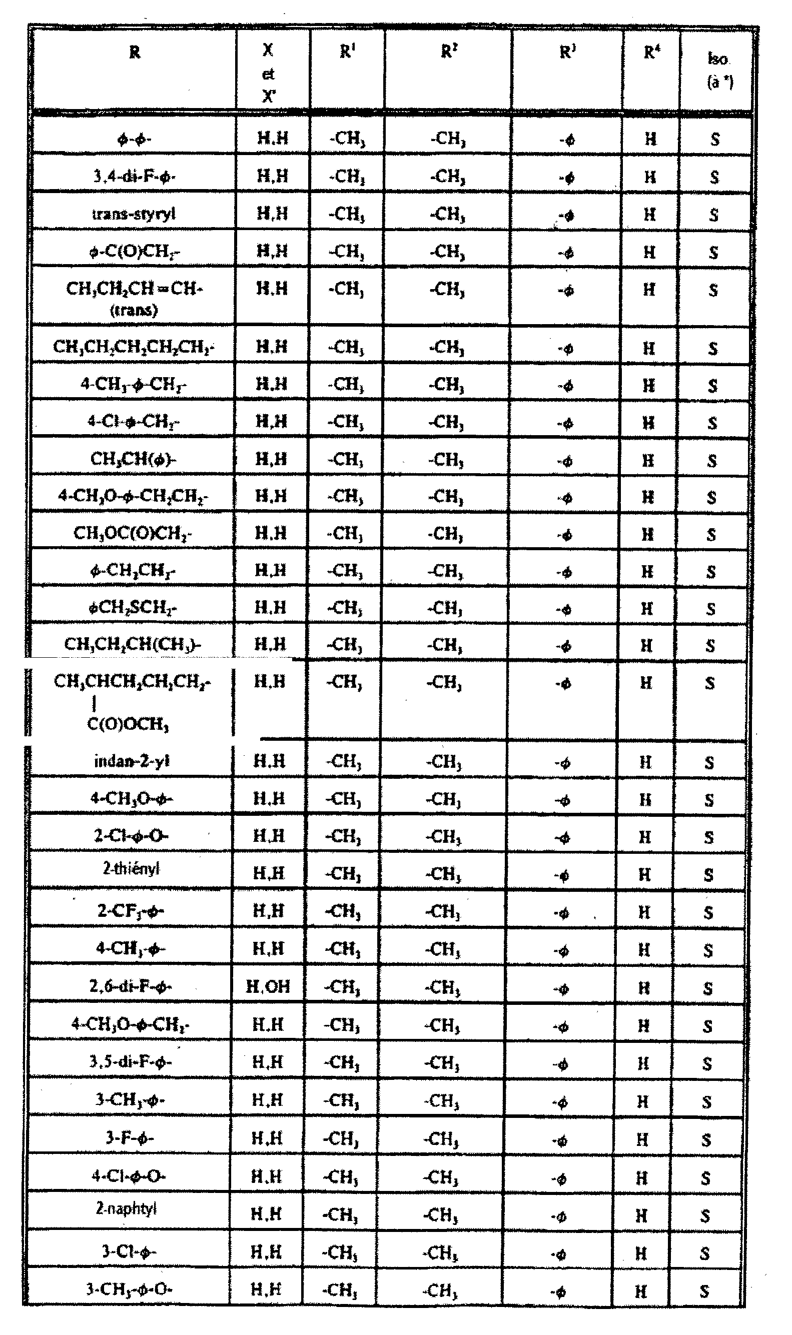

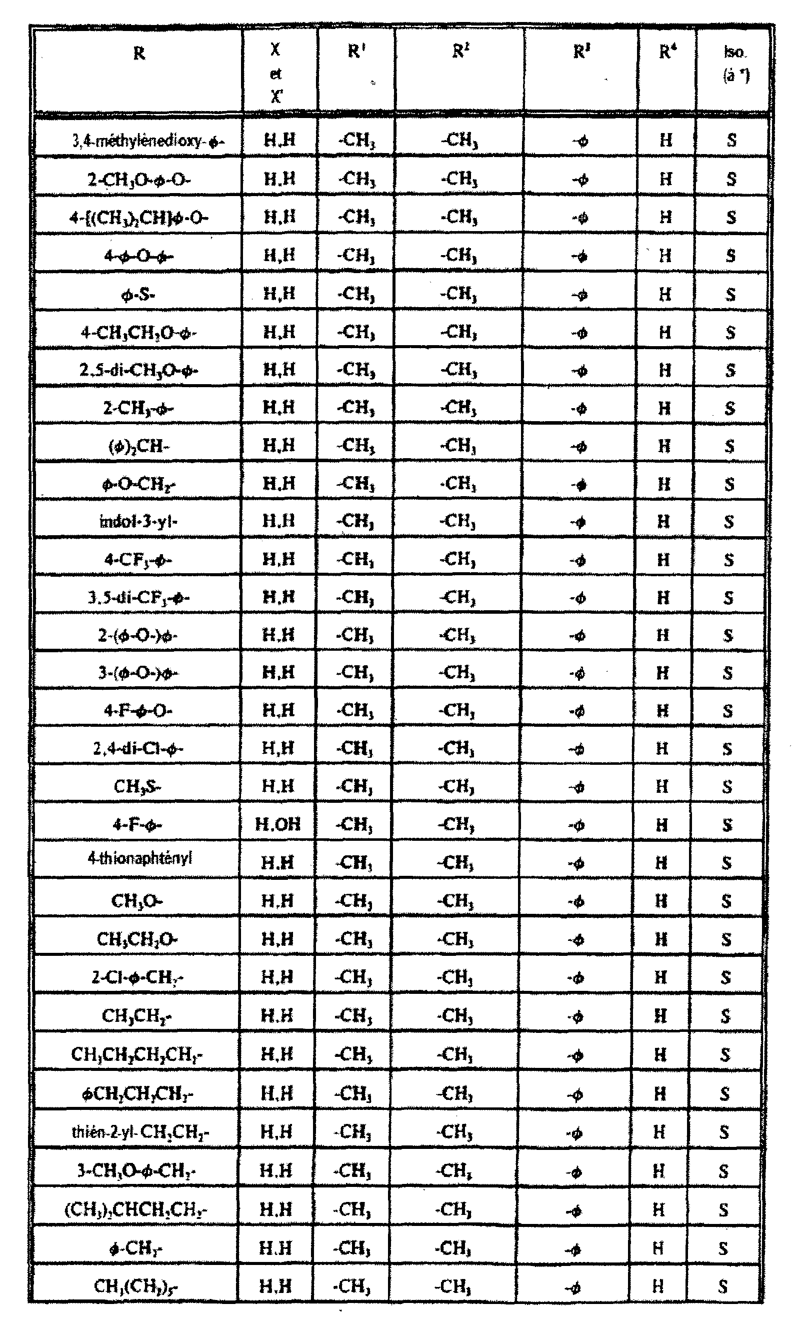

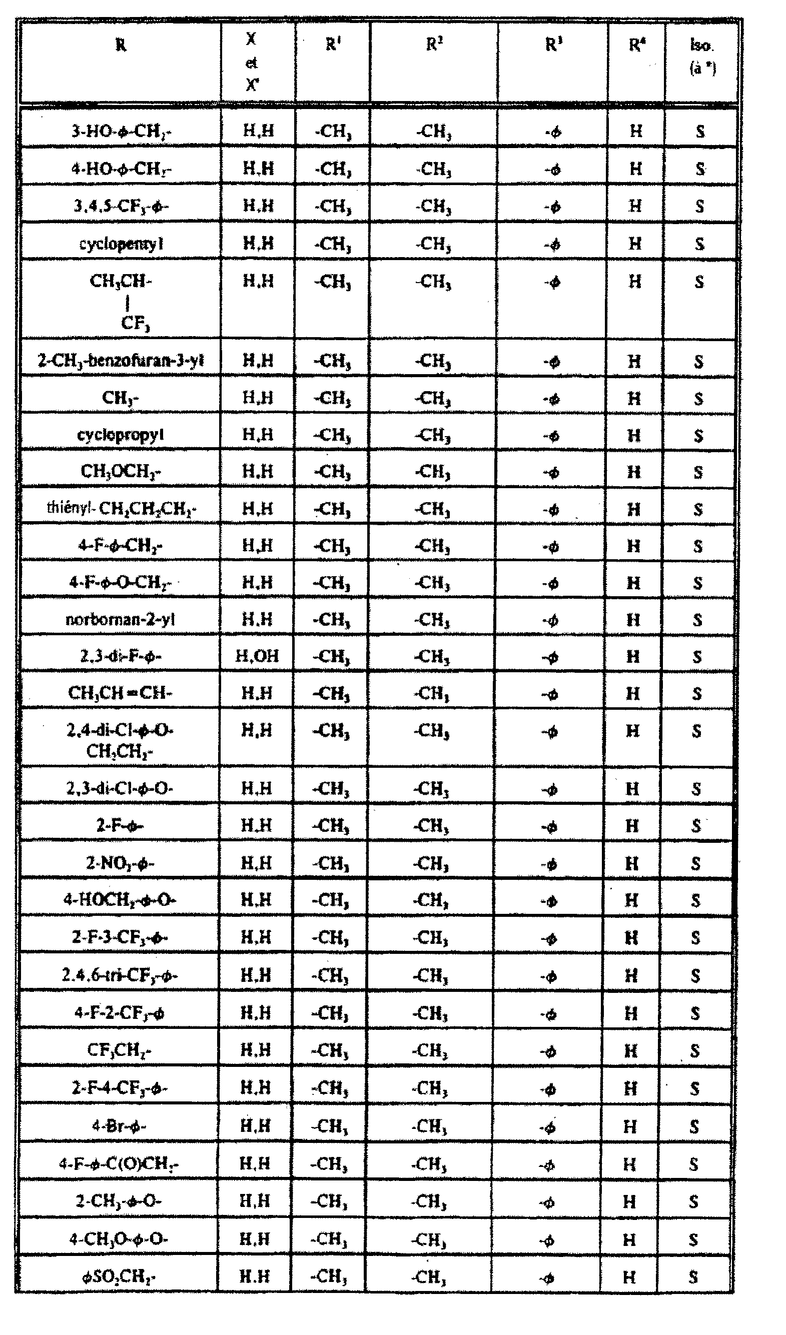

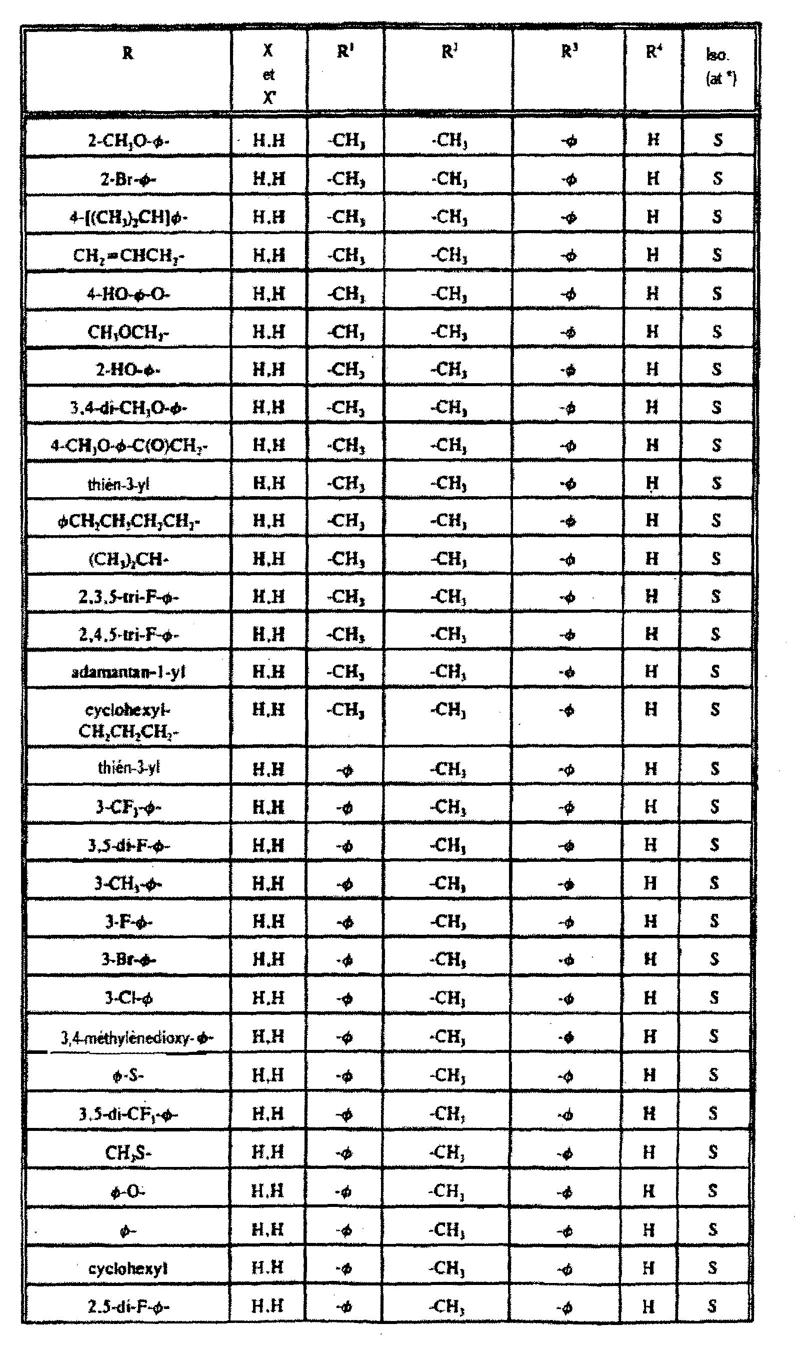

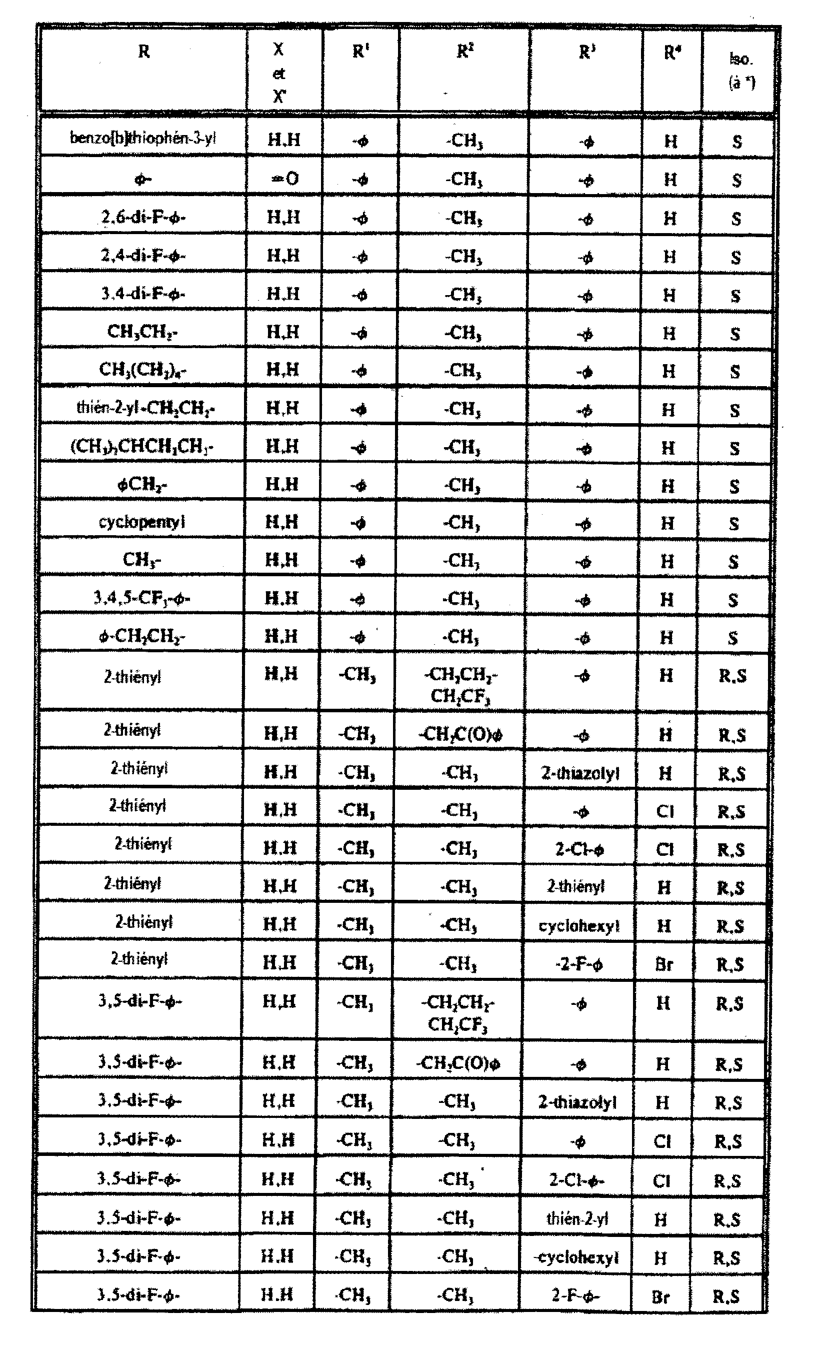

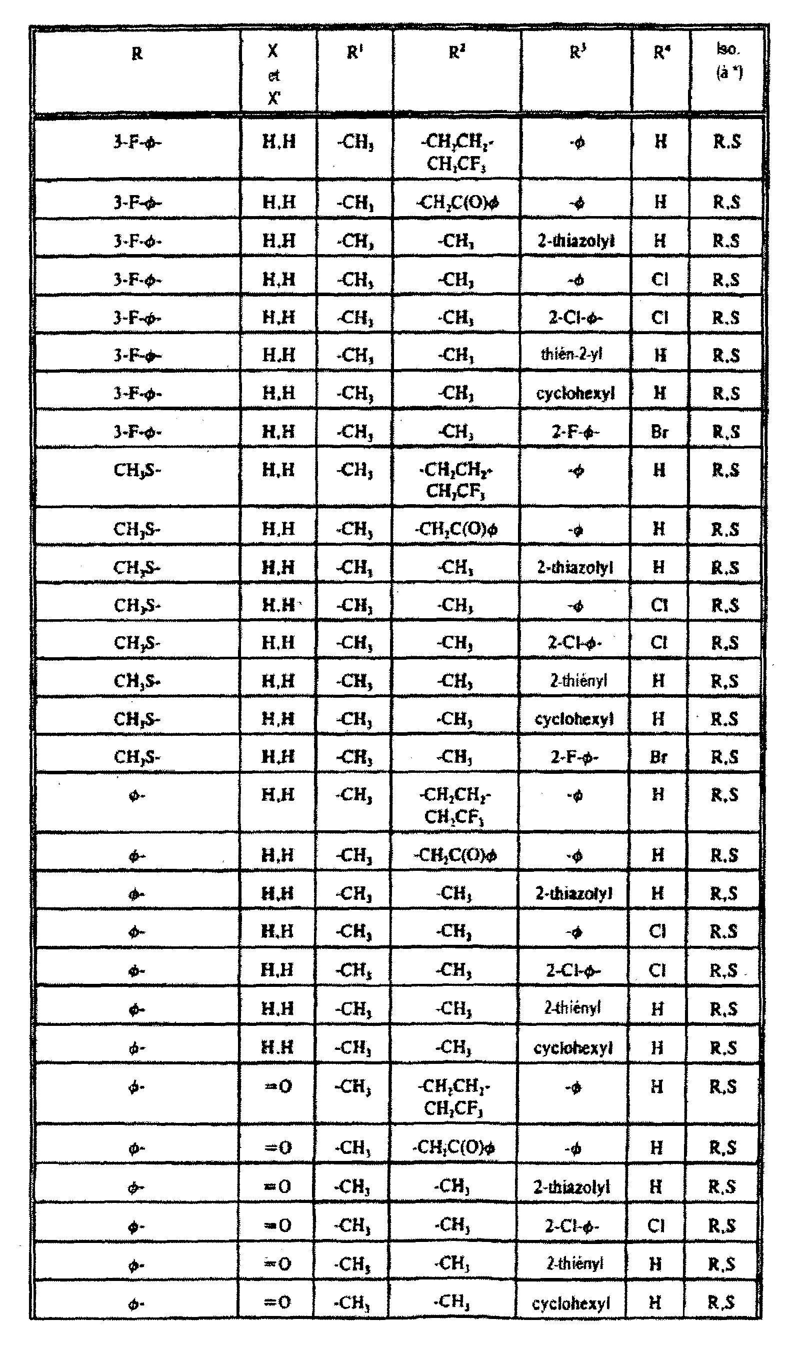

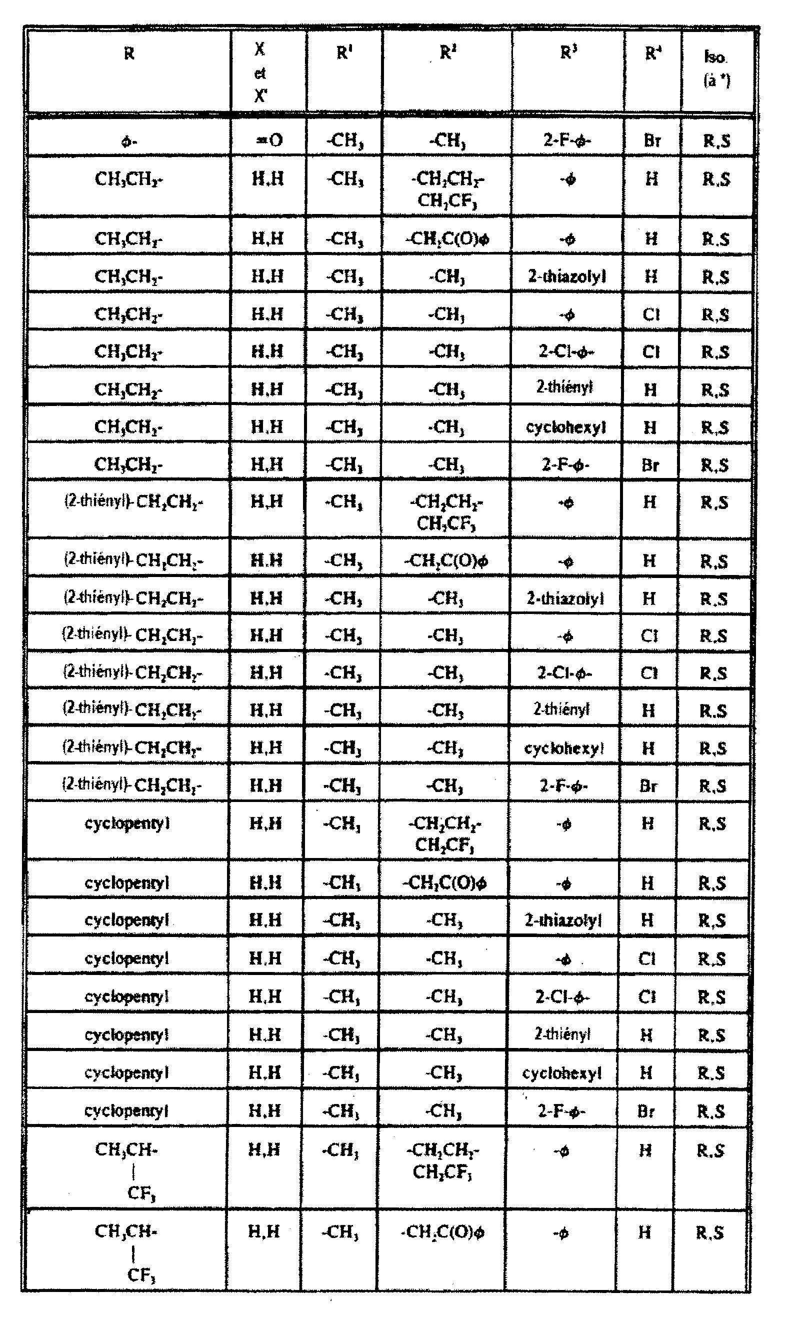

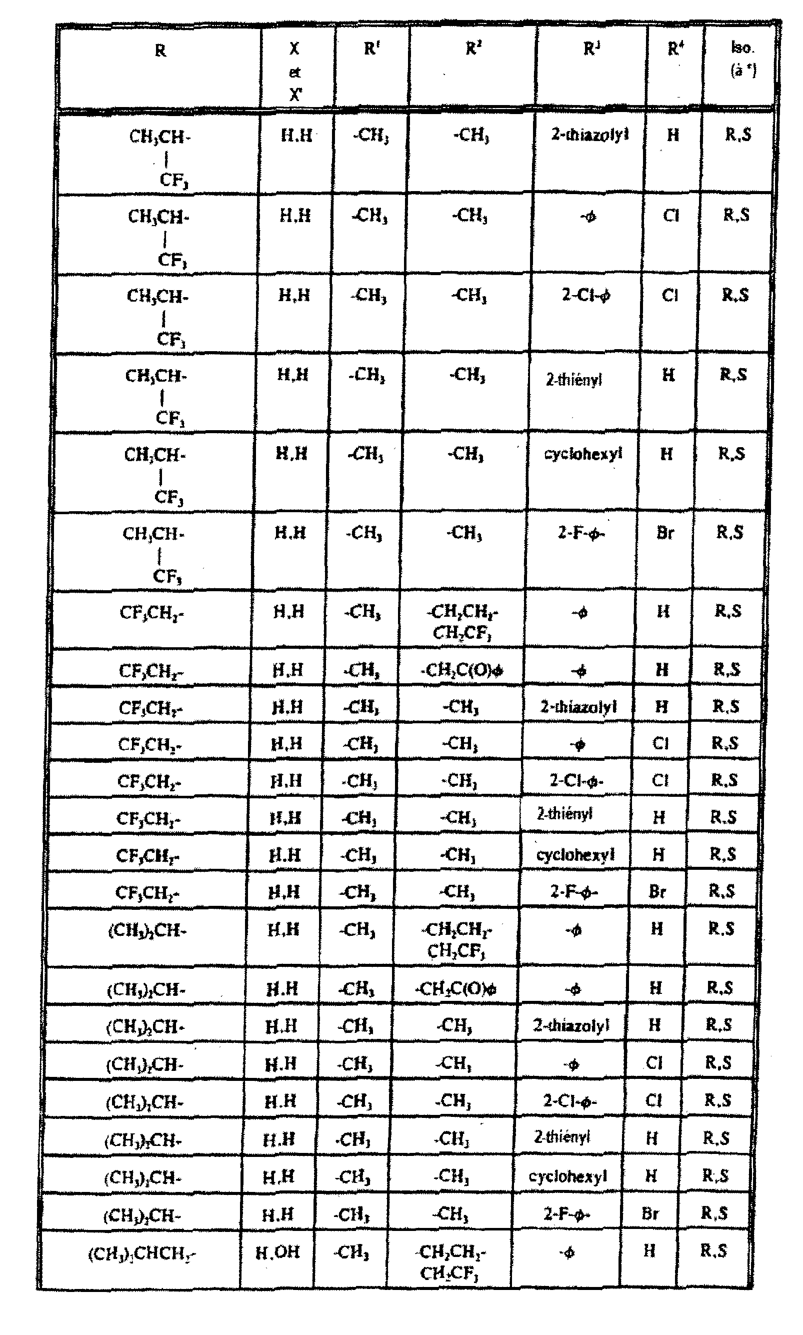

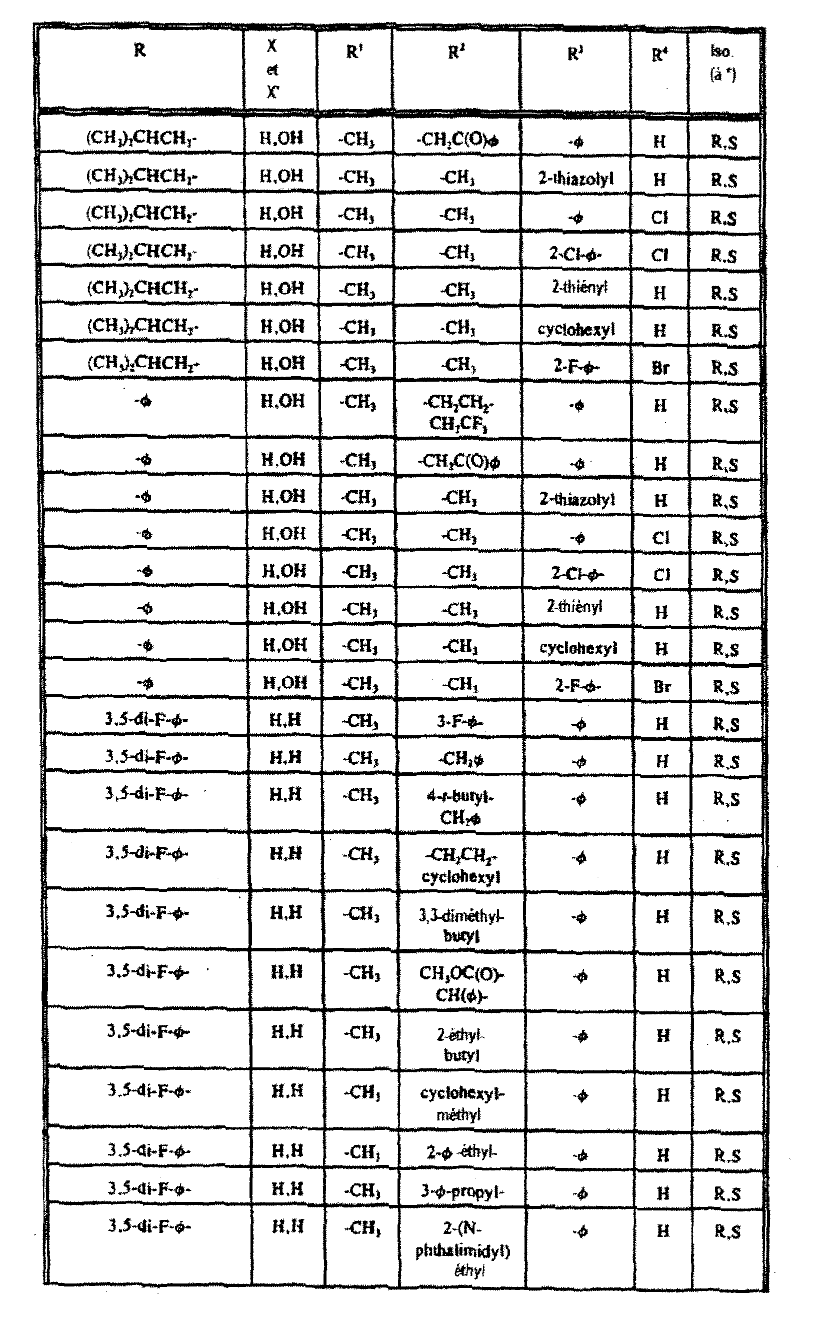

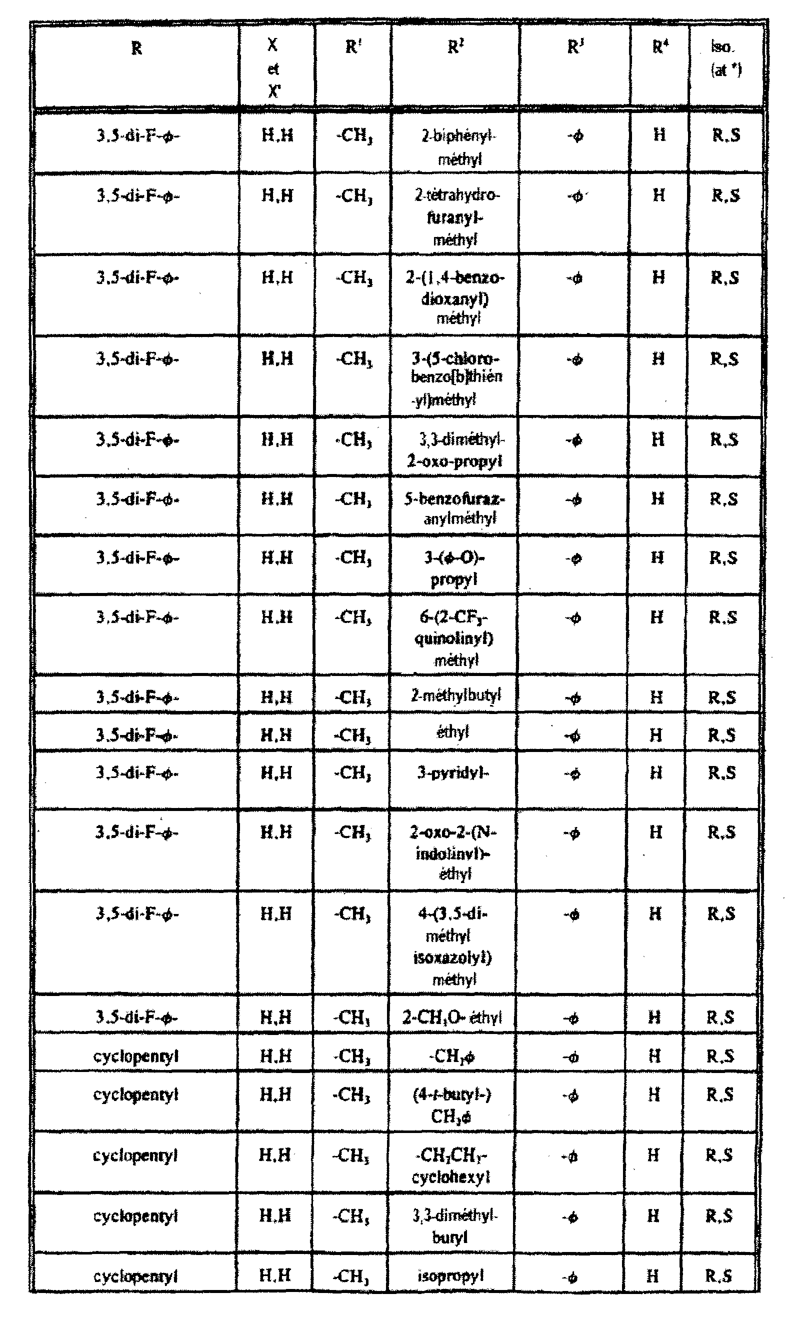

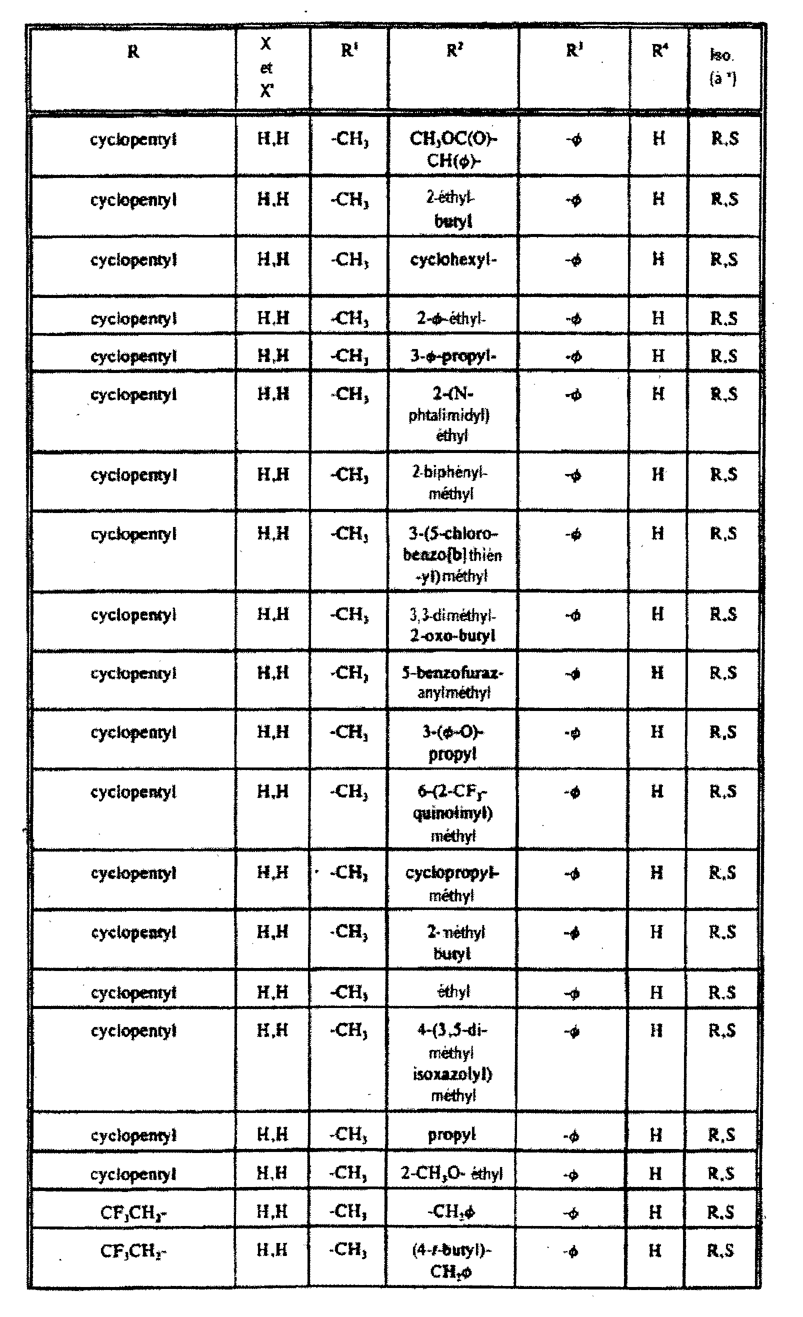

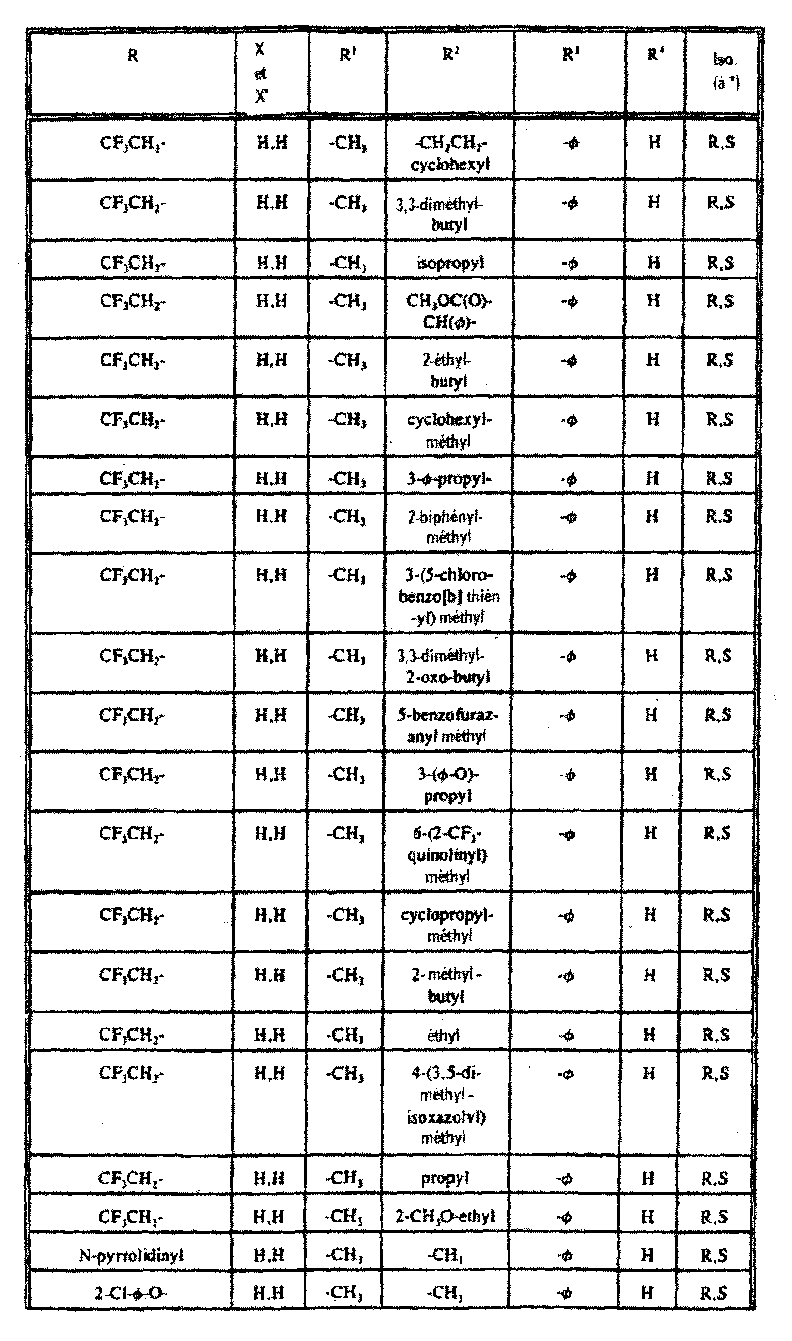

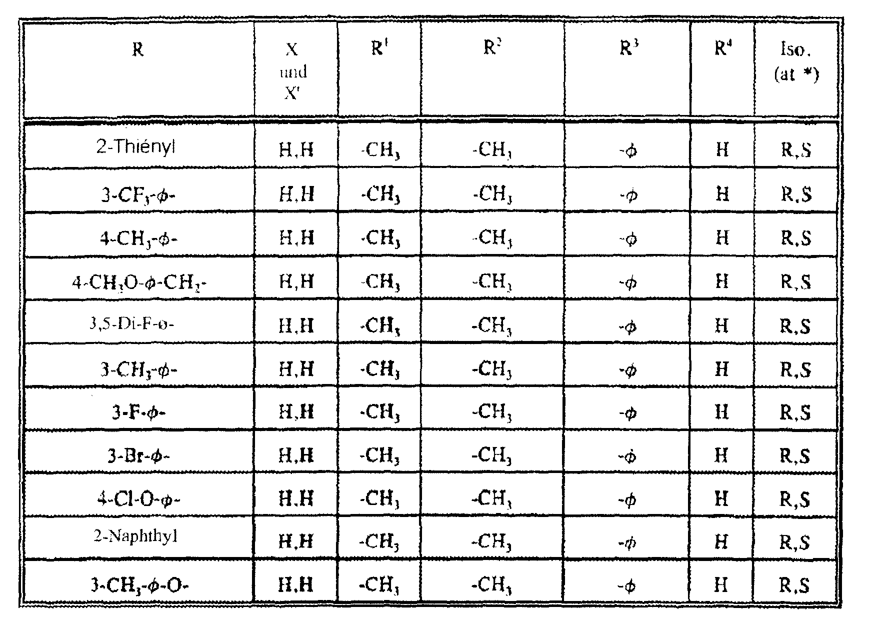

5. Composition pharmaceutique comprenant un support pharmaceutiquement inerte et une quantité pharmaceutiquement efficace d'un composé selon les revendications 1 à 4.

6. Composé selon les revendications 1 à 4, à utiliser en thérapie.

7. Composé selon les revendications 1 à 4, à utiliser dans le traitement de la maladie d'Alzheimer.

8. Utilisation d'un composé selon les revendications 1 à 4, pour la préparation d'un médicament destiné au traitement de la maladie d'Alzheimer.
